(19) Europäisches Patentamt
European Patent Office
Office européen des brevets

(11) **EP 4 548 937 A1**

(12) **EUROPEAN PATENT APPLICATION**
published in accordance with Art. 153(4) EPC

(43) Date of publication:
**07.05.2025 Bulletin 2025/19**

(21) Application number: **23857427.1**

(22) Date of filing: **24.08.2023**

(51) International Patent Classification (IPC):
***A61K 47/68*** (2017.01) ***A61K 31/498*** (2006.01)
***A61K 47/54*** (2017.01) ***C07D 401/04*** (2006.01)
***C07D 401/14*** (2006.01) ***C07D 405/14*** (2006.01)
***C07D 471/04*** (2006.01) ***C07D 491/048*** (2006.01)

(52) Cooperative Patent Classification (CPC):
**A61K 47/6803; A61K 31/498; A61K 47/6849; A61K 47/6853; A61K 47/6883; A61K 47/6889; C07D 401/04; C07D 401/14; C07D 405/14; C07D 471/04; C07D 491/048**

(86) International application number:
**PCT/JP2023/030603**

(87) International publication number:
**WO 2024/043319 (29.02.2024 Gazette 2024/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **26.08.2022 US 202263373646 P**
**28.09.2022 US 202263377518 P**

(71) Applicant: **Eisai R&D Management Co., Ltd.**
**Tokyo 112-8088 (JP)**

(72) Inventors:
- **MIYANO Masayuki**
  **Tsukuba-shi, Ibaraki 300-2635 (JP)**
- **NAKAZAWA Yuya**
  **Tsukuba-shi, Ibaraki 300-2635 (JP)**
- **ISO Kentaro**
  **Tsukuba-shi, Ibaraki 300-2635 (JP)**
- **YABE Yuki**
  **Tsukuba-shi, Ibaraki 300-2635 (JP)**
- **UMIHARA Hirotatsu**
  **Tsukuba-shi, Ibaraki 300-2635 (JP)**
- **TAGUCHI Junichi**
  **Tsukuba-shi, Ibaraki 300-2635 (JP)**
- **INOUE Satoshi**
  **Tsukuba-shi, Ibaraki 300-2635 (JP)**
- **TSUKAMOTO Shuntaro**
  **Tsukuba-shi, Ibaraki 300-2635 (JP)**
- **KOGAI Hiroyuki**
  **Tsukuba-shi, Ibaraki 300-2635 (JP)**
- **YAMAGUCHI Atsumi**
  **Tsukuba-shi, Ibaraki 300-2635 (JP)**
- **AKAGI Tsuyoshi**
  **Tsukuba-shi, Ibaraki 300-2635 (JP)**
- **MUKAI Yohei**
  **Kobe-shi, Hyogo 650-0047 (JP)**
- **HIRAYAMA Toshifumi**
  **Kobe-shi, Hyogo 650-0047 (JP)**
- **KATO Masaki**
  **Kamisu-shi, Ibaraki 314-0255 (JP)**
- **MOCHIZUKI Toshiki**
  **Tsukuba-shi, Ibaraki 300-2635 (JP)**
- **YAMAMOTO Akihiko**
  **Tsukuba-shi, Ibaraki 300-2635 (JP)**
- **YAMAMOTO Yuji**
  **Tsukuba-shi, Ibaraki 300-2635 (JP)**
- **SAKURADA Takato**
  **Tsukuba-shi, Ibaraki 300-2635 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

(54) **ANTIBODY-DRUG COMPLEX**

(57) An antibody-drug conjugate represented by formula (I):

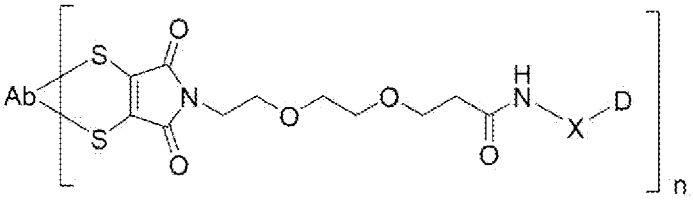
(I)

(where
Ab is an antibody,
X is a group represented by formula (X-1), formula (X-2) or formula (X-3):

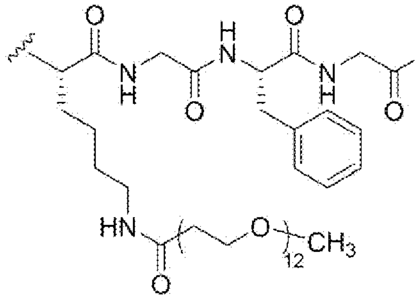
(X-1)

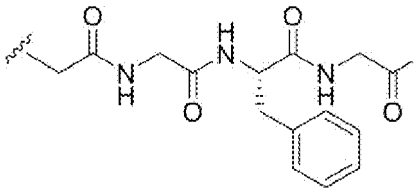
(X-2)

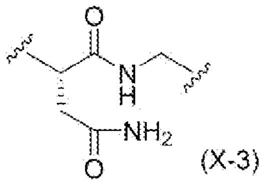
(X-3)

(where
at the left represents the binding site with NH and
at the right represents the binding site with D),
D is a group represented by formula (D-1) or formula (D-2):

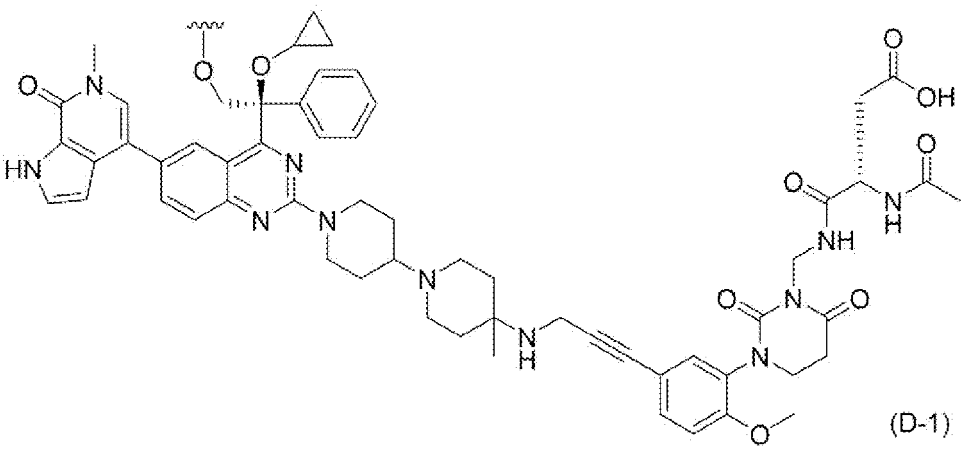
(D-1)



(Cont. next page)

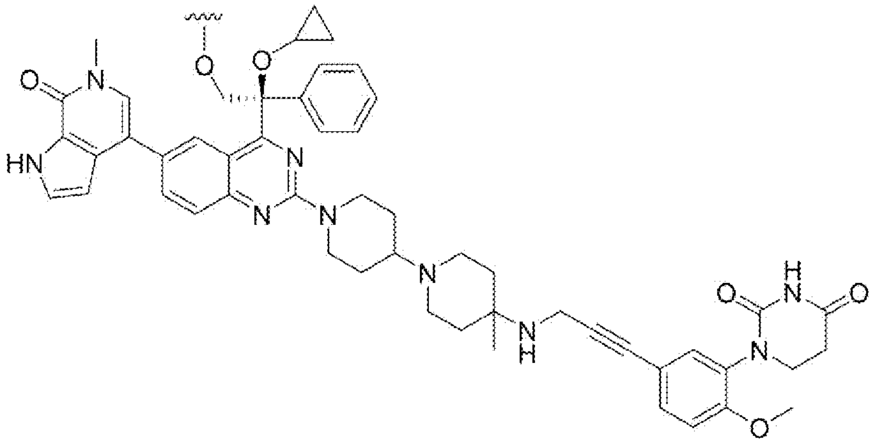

(D-2)

(where
represents the binding site with X), and
n is in the range of about 1 to about 8).

***Fig.1***

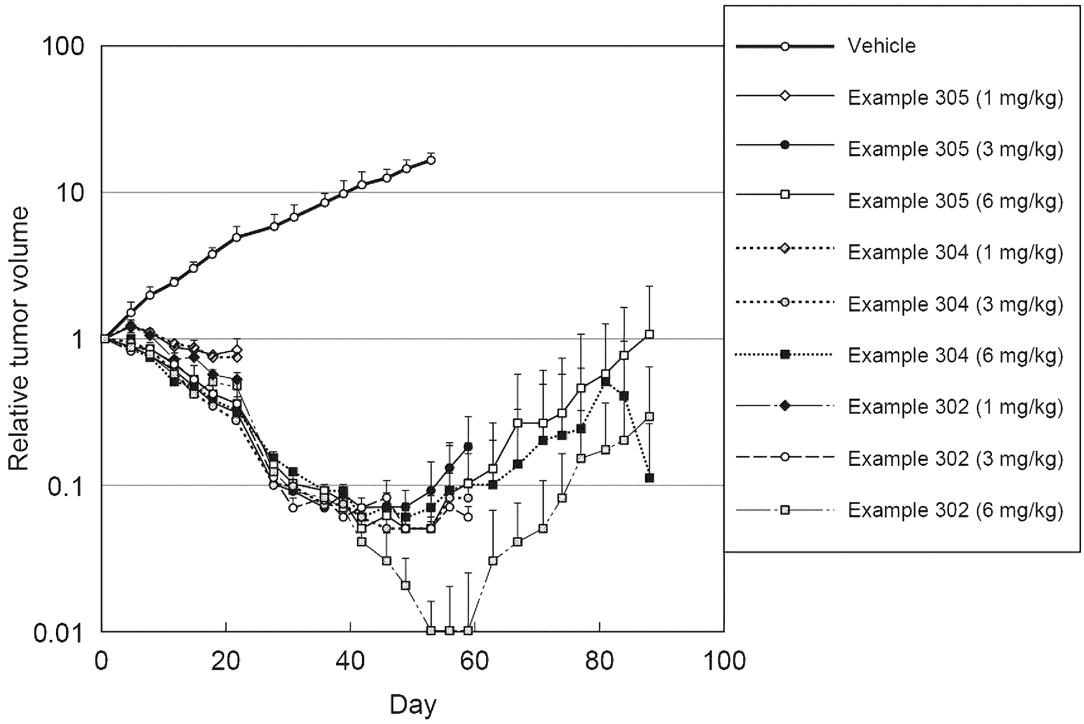

## Description

### Field of Invention

The present invention relates to an antibody-drug conjugate.

### Background Art

Cancer is one of the leading causes of disease and death in the world, with approximately 14,000,000 new cases and 8,200,000 cancer-related deaths reported in 2012. The most common causes of cancer deaths are lung cancer (1,590,000 deaths), liver cancer (745,000 deaths), stomach cancer (723,000 deaths), colorectal cancer (694,000 deaths), breast cancer (521,000 deaths) and esophageal cancer (400,000 deaths). The number of new cancer cases is expected to increase by approximately 70% to about 22,000,000 new cancer cases per year in the next 20 years (NPL 1). Among the different types of cancer, pancreatic cancer has few treatment options with a very low 5-year survival rate of 10% even in medically advanced countries, and it is therefore a very significant unmet medical need. Unlike other cancer types, deaths from pancreatic cancer are expected to continue to rise in the future.

Protein degraders are artificial chimeric molecules that can induce decomposition of specific proteins in cells in a proteasome-dependent manner, and they are of interest as a new modality for molecular targeted drugs. BRD2/4 (Bromodomain-containing protein 2/4) is a protein that recognizes acetylated histone and mobilizes transcription factors to affect gene transcription, with BRD2/4 inhibition being known to produce antitumor activity. Some degraders have previously been reported to decompose BRD2/4 (NPL 2 and PTL 1). However, none have seen clinical application due to their powerful toxicity. Most protein degraders have issues because of their physical properties as drugs, and this has constituted another hurdle against their clinical application.

CEACAM6 (carcinoembryonic antigen-related cell adhesion molecule 6) is a membrane protein reported to have high expression in numerous cancer types including pancreatic cancer. However, CEACAM6 is also expressed in a few important normal cells, and no clinical application has been developed with it as the target of an antibody-drug conjugate using small molecule drugs.

### Citation List

### Patent Literature

[PTL 1] International Patent Publication No. WO2020/086858

### Non-Patent Literature

[NPL 1] World Cancer Report 2014

[NPL 2] Thomas et al. "Antibody Conjugation of a Chimeric BET Degrader Enables in vivo Activity" ChemMedChem 2020, 15, 17-25

### Summary of Invention

### Technical Problem

It is an object of the present invention to provide an antibody-drug conjugate that is effective against cancer.

### Solution to Problem

The present inventors have conducted much research with the goal of achieving this object, and have completed this invention upon finding that an antibody-drug conjugate represented by formula (I) has antitumor activity against different types of cancer including pancreatic cancer.

Specifically, the invention relates to the following [1] to [35].

[1] An antibody-drug conjugate represented by formula (I):

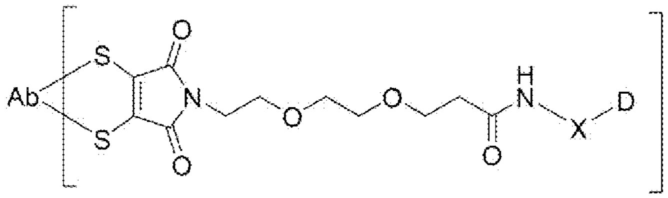

(I)

(where

Ab is an antibody,
X is a group represented by formula (X-1), formula (X-2) or formula (X-3):

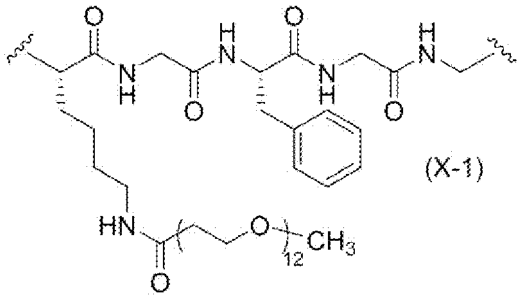

(X-1)

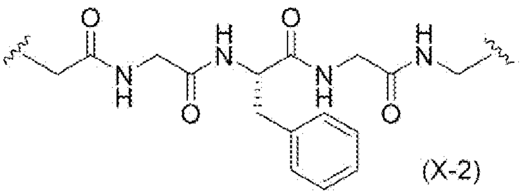

(X-2)

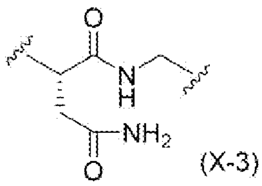

(X-3)

(where

at the left represents the binding site with NH and
at the right represents the binding site with D),
D is a group represented by formula (D-1) or formula (D-2):

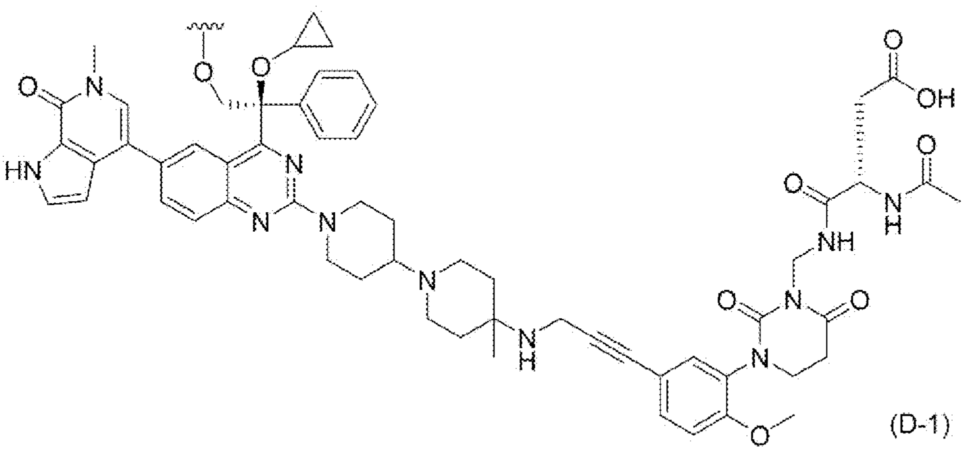

(D-1)

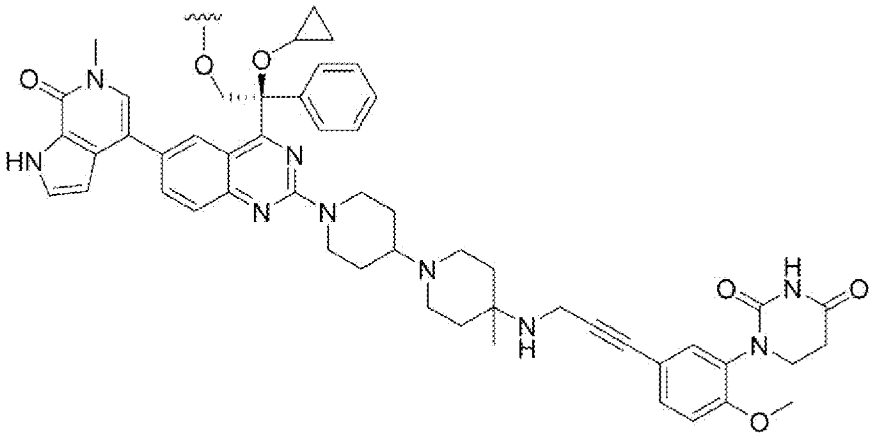

(D-2)

(where 

represents the binding site with X), and
n is in the range of about 1 to about 8).

[2] The antibody-drug conjugate according to [1], wherein the antibody is anti-CEACAM6 antibody, anti-folate receptor α antibody, anti-mesothelin antibody, anti-HER2 antibody, anti-FLT3 antibody, anti-DLL3 antibody, anti-CLDN6 antibody, anti-EGFR antibody, anti-Nectin4 antibody, anti-CA9 antibody, anti-CLDN3/4 antibody or anti-TROP2 antibody.
[3] The antibody-drug conjugate according to [1] or [2], wherein the antibody is anti-CEACAM6 antibody, D is a group represented by formula (D-1), and X is a group represented by formula (X-1) or formula (X-3).
[4] The antibody-drug conjugate according to [2] or [3], wherein the anti-CEACAM6 antibody includes:

a heavy chain including:

(a) the heavy chain CDR1 comprising the amino acid sequence listed as SEQ ID NO: 18;
(b) the heavy chain CDR2 comprising the amino acid sequence listed as SEQ ID NO: 19; and
(c) the heavy chain CDR3 comprising the amino acid sequence listed as SEQ ID NO: 20; and

a light chain including:

(d) the light chain CDR1 comprising the amino acid sequence listed as SEQ ID NO: 21;
(e) the light chain CDR2 comprising the amino acid sequence listed as SEQ ID NO: 21-1; and
(f) the light chain CDR3 comprising the amino acid sequence listed as SEQ ID NO: 22.

[5] A pharmaceutical composition comprising an antibody-drug conjugate according to any one of [1] to [4] above.
[6] The pharmaceutical composition according to [5] above, which is to be used for treatment of cancer.
[7] A method for treating cancer which includes administering an antibody-drug conjugate according to any one of [1] to [4] above to a patient in need of the same.
[8] The use of an antibody-drug conjugate according to any one of [1] to [4] above in the manufacture of an anticancer agent.
[9] The antibody-drug conjugate according to any one of [1] to [4] above, which is for use in treatment of cancer.
[10] A compound represented by formula (D'):

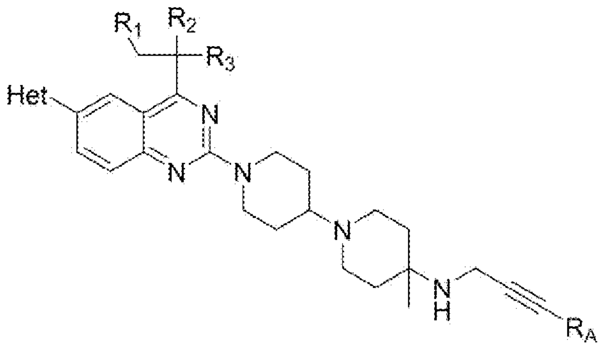

(D')

(where

Het is a group selected from the group consisting of the following formula:

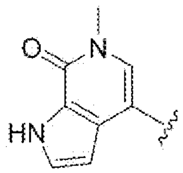 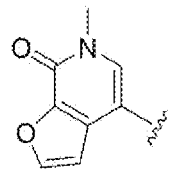 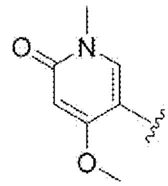 and 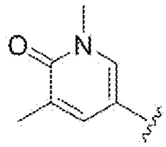

(where 

represents a binding site),

$R_1$ is a hydroxyl, amino or optionally substituted $C_{1-6}$ alkylcarbamoyloxy group,

$R_2$ is a $C_{1-6}$ alkyl, $C_{3-6}$ cycloalkyl, $C_{1-6}$ alkoxy or $C_{3-6}$ cycloalkoxy group,

$R_3$ is a $C_{1-6}$ alkyl or $C_{6-10}$ aryl group, and

$R_A$ is group represented by formula (a) or formula (b):

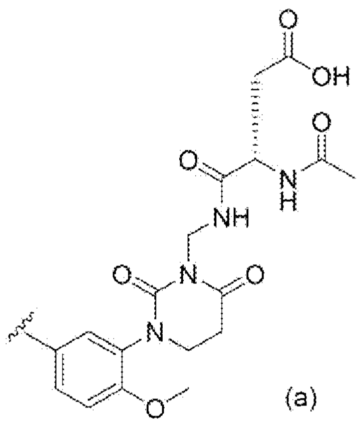

(a)

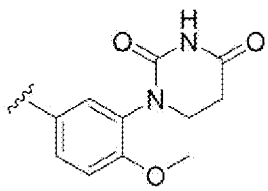

(b)

(where 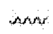

represents a binding site),

or a pharmacologically acceptable salt thereof.

[11] A compound represented by formula (D'-1) or formula (D'-2):

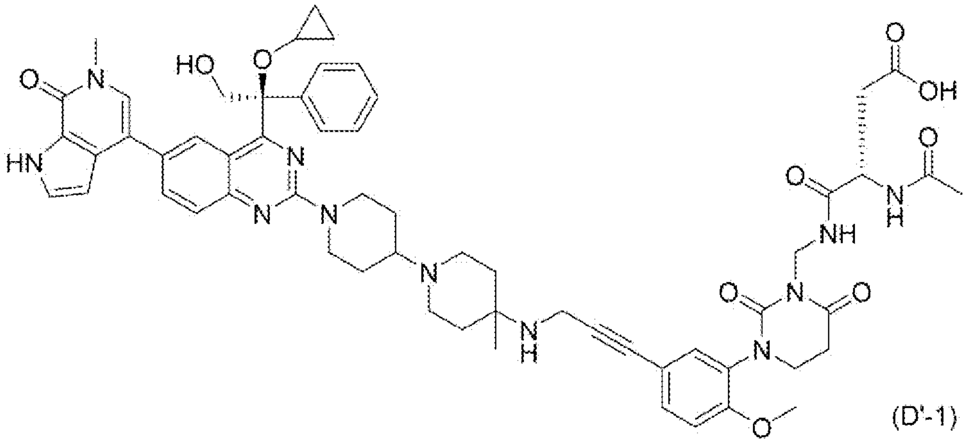

(D'-1)

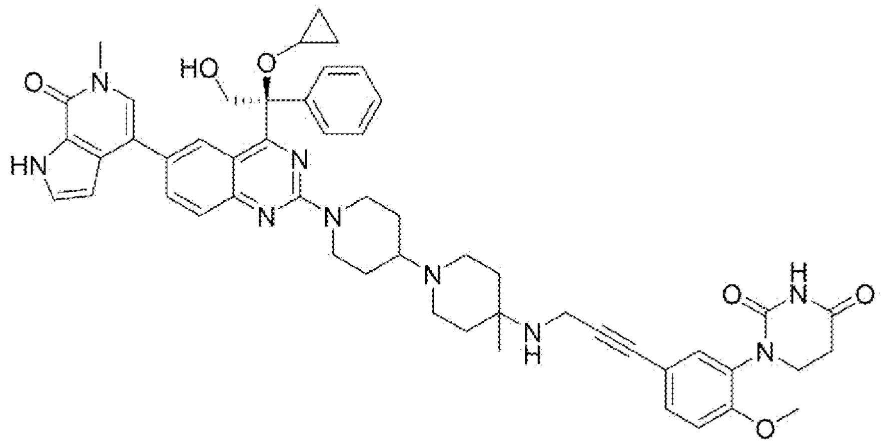

(D'-2)

or a pharmacologically acceptable salt thereof.

[12] A compound represented by formula (M):

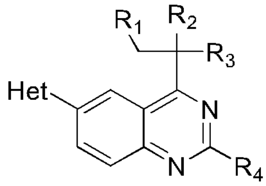

(M)

(where

Het is a group selected from the group consisting of the following formula:

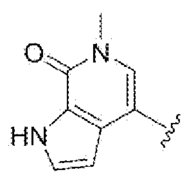 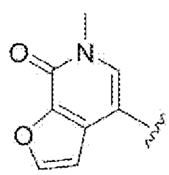 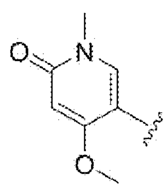 and 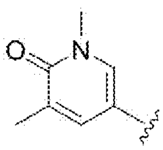

(where

represents a binding site),

$R_1$ is a hydroxyl, amino or optionally substituted $C_{1-6}$ alkylcarbamoyloxy group,

$R_2$ is a $C_{1-6}$ alkyl, $C_{3-6}$ cycloalkyl, $C_{1-6}$ alkoxy or $C_{3-6}$ cycloalkoxy group,

$R_3$ is a $C_{1-6}$ alkyl or $C_{6-10}$ aryl group, and

$R_4$ is an amino group or a group represented by the formula:

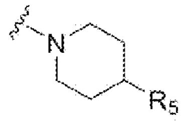

(where

Rs is an ethynyl, piperidyl, piperazinyl, piperidyloxy or piperazinylmethyl group, the piperidyl, piperazinyl, piperidyloxy, and piperazinylmethyl group being optionally substituted with one or more groups selected from the group consisting of methyl, ethynyl, hydroxyl, amino and propynylamino groups),
or a pharmacologically acceptable salt thereof.

[13] An anti-CEACAM6 antibody that includes:

a heavy chain including:

(a) the heavy chain CDR1 comprising the amino acid sequence listed as SEQ ID NO: 18;
(b) the heavy chain CDR2 comprising the amino acid sequence listed as SEQ ID NO: 19; and
(c) the heavy chain CDR3 comprising the amino acid sequence listed as SEQ ID NO: 20; and

a light chain including:

(d) the light chain CDR1 comprising the amino acid sequence listed as SEQ ID NO: 21;
(e) the light chain CDR2 comprising the amino acid sequence listed as SEQ ID NO: 21-1; and
(f) the light chain CDR3 comprising the amino acid sequence listed as SEQ ID NO: 22.

[14] The anti-CEACAM6 antibody according to [13], wherein the heavy chain includes a variable region comprising the amino acid sequence listed as SEQ ID NO: 6, and the light chain includes a variable region comprising the amino acid sequence listed as SEQ ID NO: 7.
[15] The anti-CEACAM6 antibody according to [13] or [14], wherein the constant region of the heavy chain and the constant region of the light chain include a human antibody-derived amino acid sequence.
[16] The anti-CEACAM6 antibody according to [15], wherein the constant region of the heavy chain includes human IgG1 and human IgG2.
[17] The anti-CEACAM6 antibody according to [16], wherein the constant region of the human IgG1 and human IgG2 includes the amino acid sequence listed as SEQ ID NO: 10.
[18] The anti-CEACAM6 antibody according to any one of [15] to [17], wherein the constant region of the light chain includes the constant region of human Igλ.
[19] The anti-CEACAM6 antibody according to [18], wherein the human Igλ includes the amino acid sequence listed as SEQ ID NO: 11.
[20] An anti-CEACAM6 antibody including a heavy chain and a light chain, wherein the heavy chain includes the amino acid sequence listed as SEQ ID NO: 14, and the light chain includes a light chain including the amino acid sequence listed as SEQ ID NO: 15.
[21] An antibody-drug conjugate represented by formula (I):

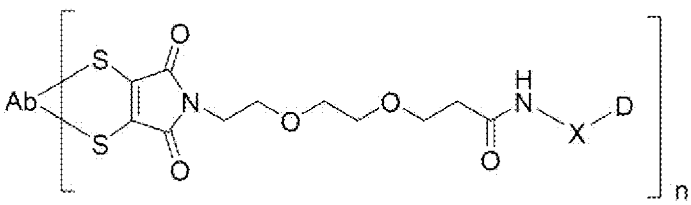

(I)

(where

Ab is an anti-CEACAM6 antibody,
X is a group represented by formula (X-1), formula (X-2) or formula (X-3):

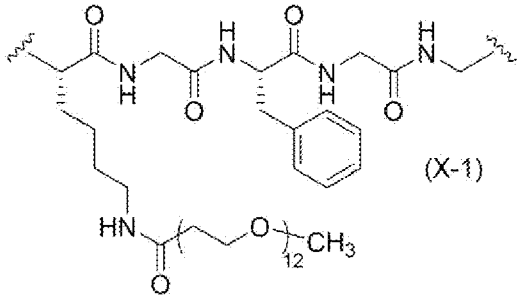
(X-1)

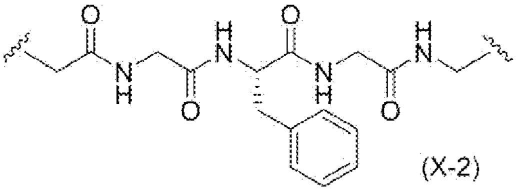
(X-2)

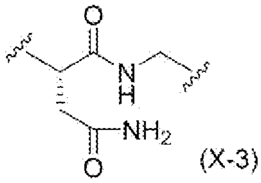
(X-3)

(where

at the left represents the binding site with NH and
at the right represents the binding site with D),
D is a group represented by formula (D-1) or formula (D-2):

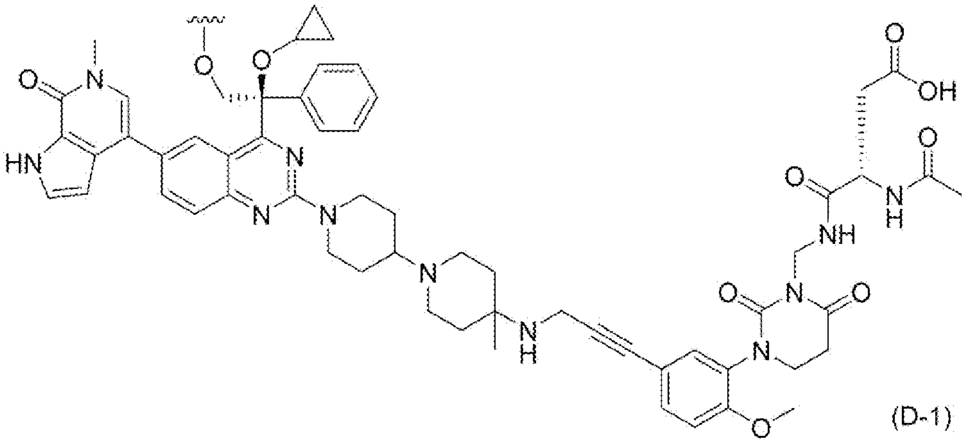
(D-1)

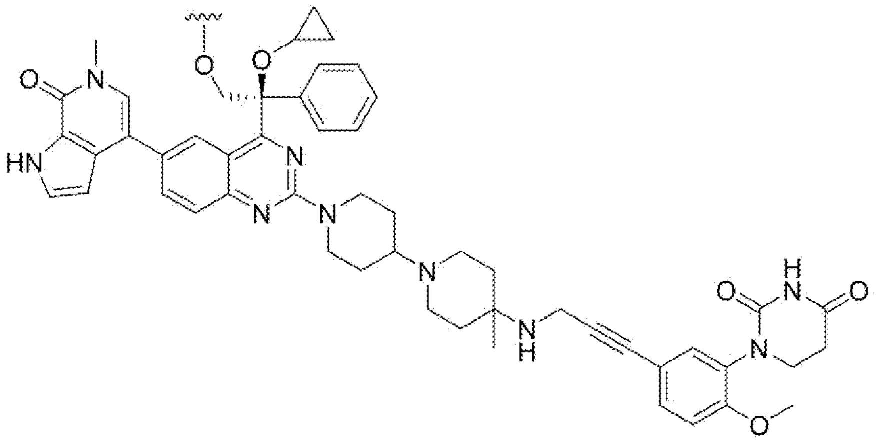
(D-2)

(where

represents the binding site with X), and

n is in the range of about 1 to about 8).

[22] An antibody-drug conjugate represented by formula (I):

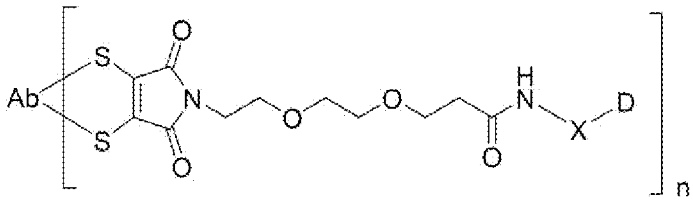

(I)

(where

Ab is an anti-folate receptor α antibody,
X is a group represented by formula (X-1), formula (X-2) or formula (X-3):

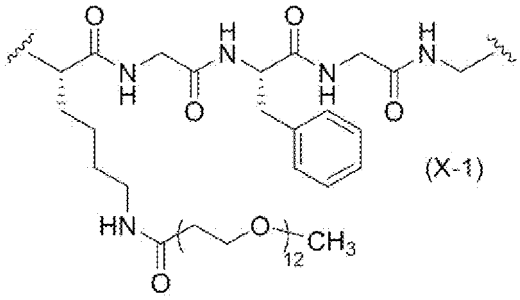

(X-1)

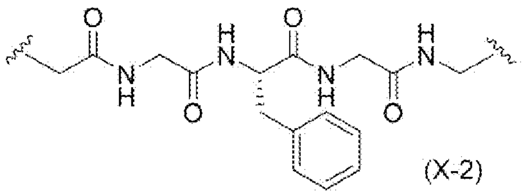

(X-2)

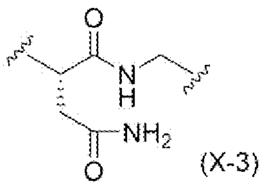

(X-3)

(where

at the left represents the binding site with NH and
at the right represents the binding site with D),
D is a group represented by formula (D-1) or formula (D-2):

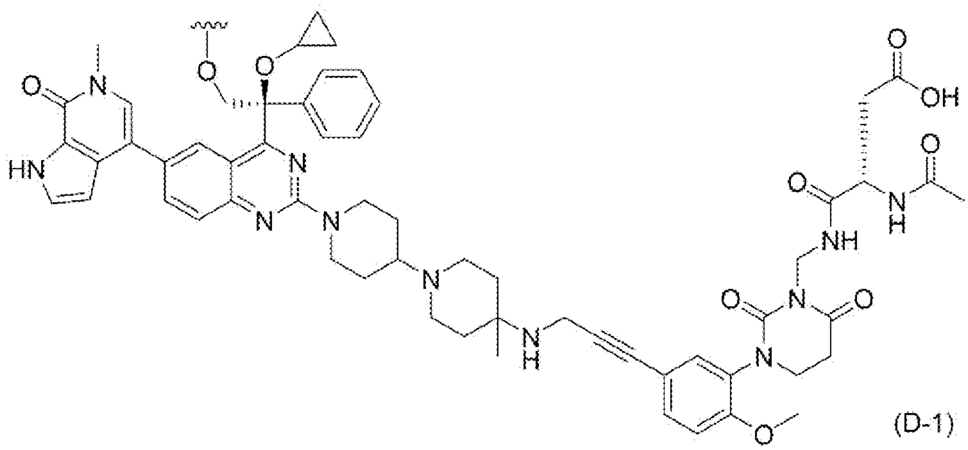

(D-1)

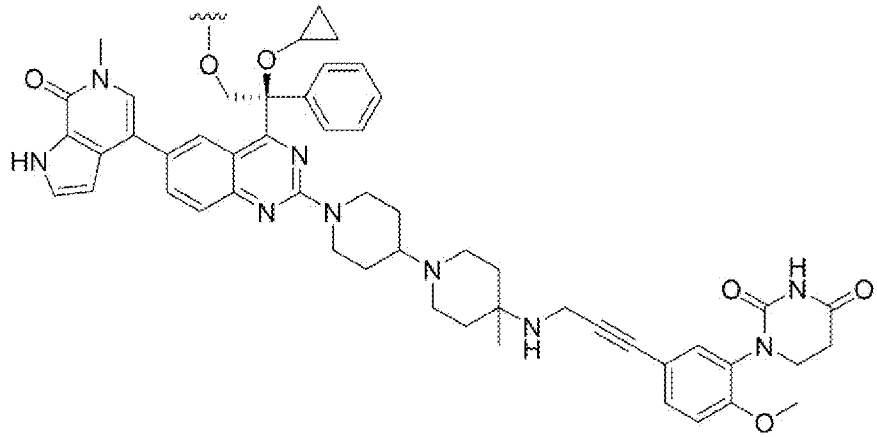

(D-2)

(where

represents the binding site with X), and
n is in the range of about 1 to about 8).

[23] An antibody-drug conjugate represented by formula (I):

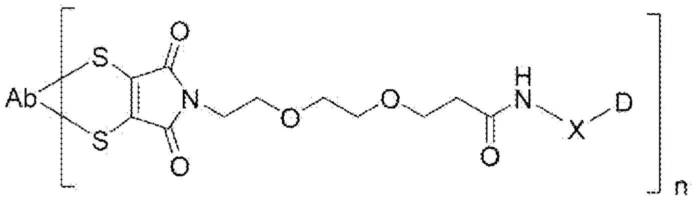

(I)

(where

Ab is an anti-mesothelin antibody,
X is a group represented by formula (X-1), formula (X-2) or formula (X-3):

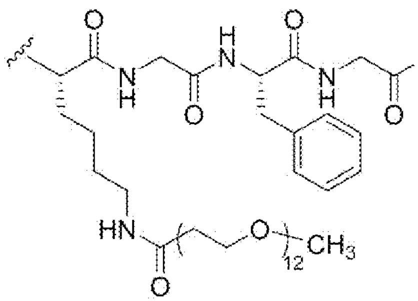

(X-1)

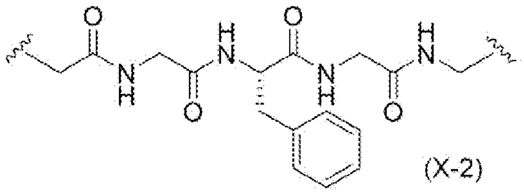

(X-2)

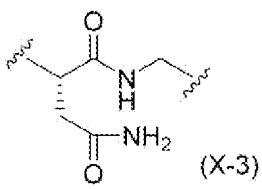

(X-3)

(where

at the left represents the binding site with NH and
at the right represents the binding site with D),
D is a group represented by formula (D-1) or formula (D-2):

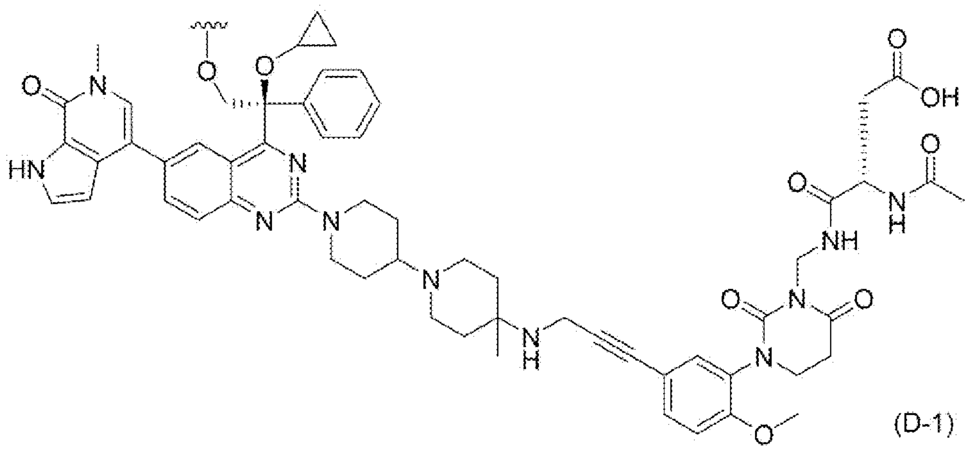

(D-1)

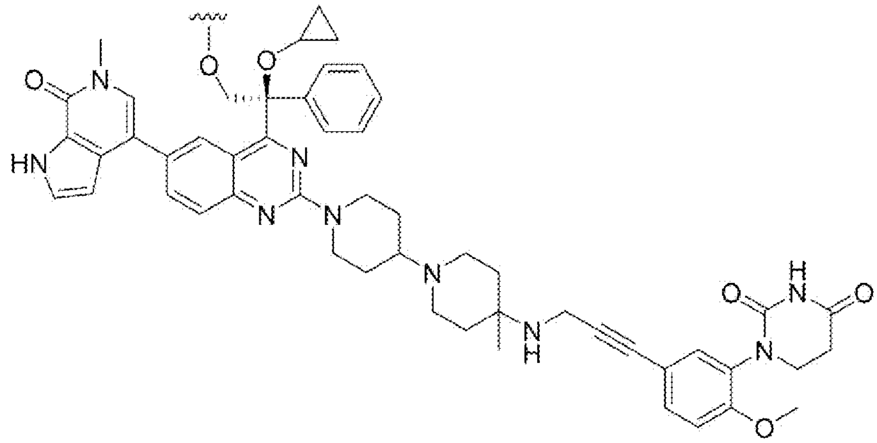

(D-2)

(where

represents the binding site with X), and
n is in the range of about 1 to about 8).

[24] An antibody-drug conjugate represented by formula (I):

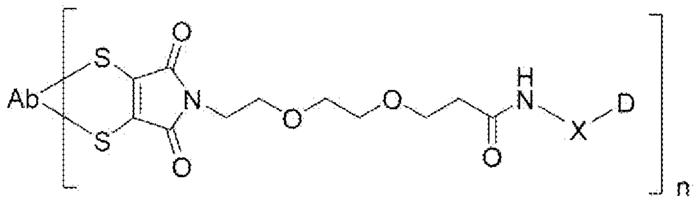

(I)

(where

Ab is an anti-HER2 antibody, anti-FLT3 antibody, anti-DLL3 antibody, anti-CLDN6 antibody, anti-EGFR antibody, anti-Nectin4 antibody, anti-CA9 antibody, anti-CLDN3/4 antibody or anti-TROP2 antibody,
X is a group represented by formula (X-1), formula (X-2) or formula (X-3):

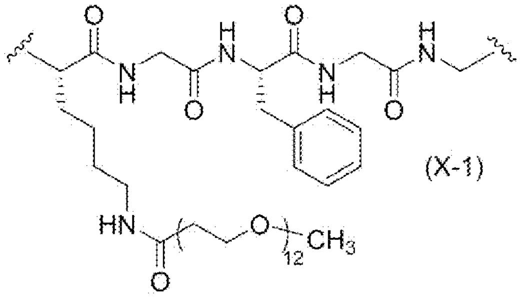
(X-1)

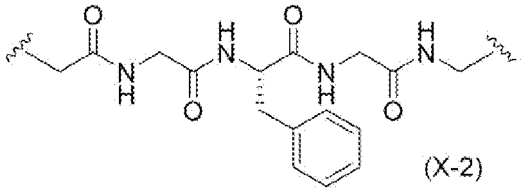
(X-2)

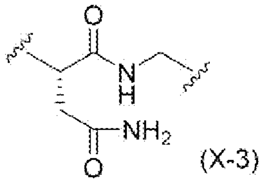
(X-3)

(where

at the left represents the binding site with NH and
at the right represents the binding site with D),
D is a group represented by formula (D-1) or formula (D-2):

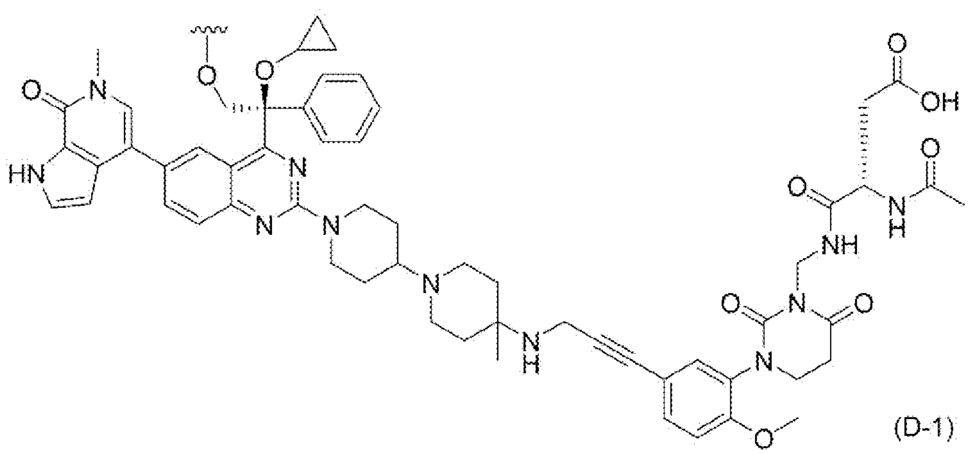
(D-1)

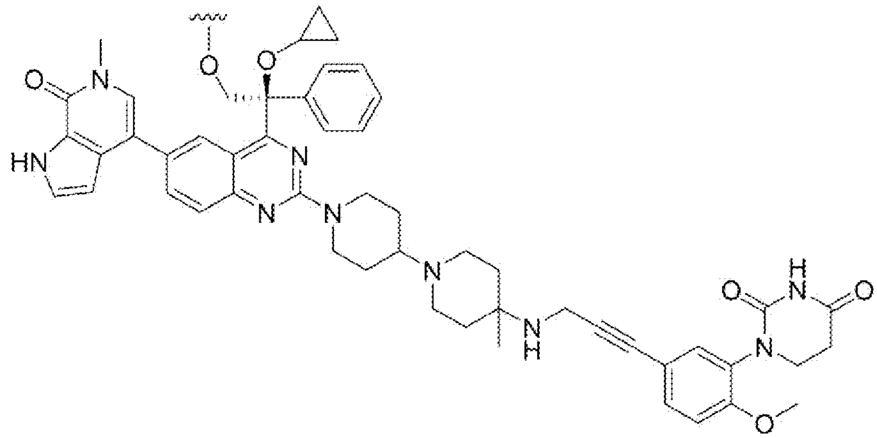
(D-2)

(where

represents the binding site with X), and

n is in the range of about 1 to about 8).

[25] A pharmaceutical composition that is to be administered in combination with a PD-1 antagonist and comprises an antibody-drug conjugate represented by formula (I):

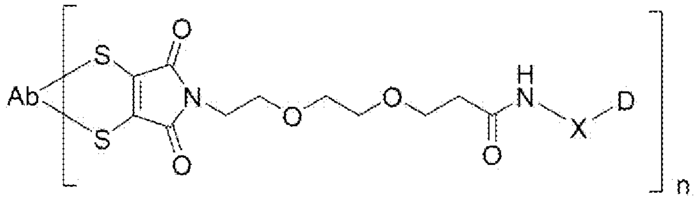

(I)

(where

Ab is an anti-CEACAM6 antibody,
X is a group represented by formula (X-1), formula (X-2) or formula (X-3):

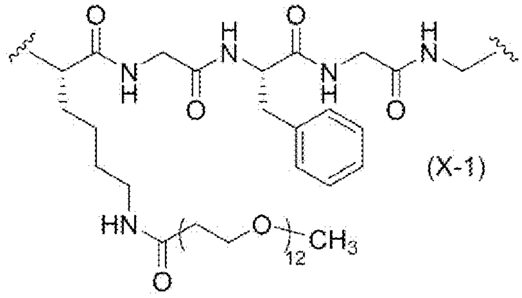

(X-1)

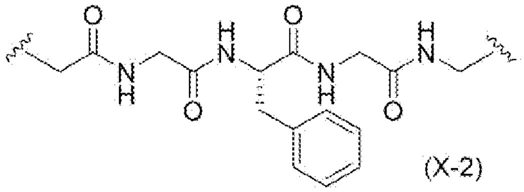

(X-2)

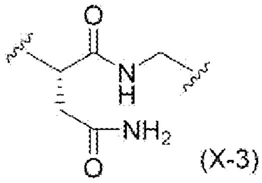

(X-3)

(where

at the left represents the binding site with NH and
at the right represents the binding site with D),
D is a group represented by formula (D-1) or formula (D-2):

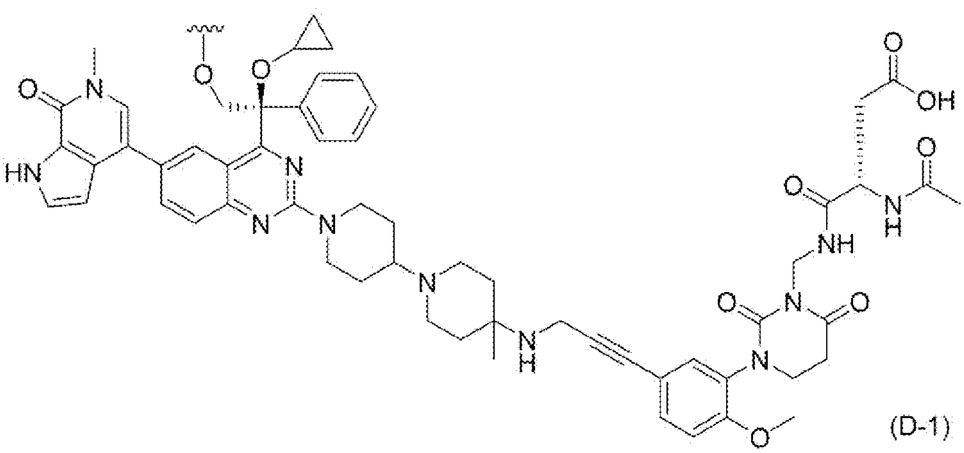

(D-1)

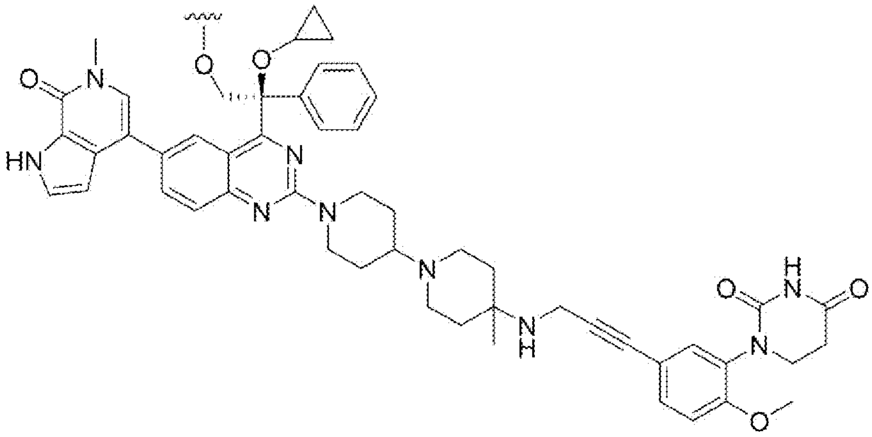
(D-2)

(where

represents the binding site with X), and
n is in the range of about 1 to about 8).

[26] A pharmaceutical composition comprising a PD-1 antagonist, that is to be administered in combination with an antibody-drug conjugate represented by formula (I):

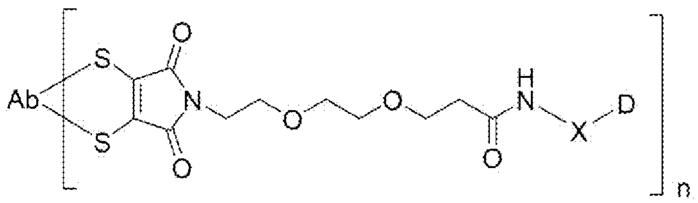
(I)

(where

Ab is an anti-CEACAM6 antibody,
X is a group represented by formula (X-1), formula (X-2) or formula (X-3):

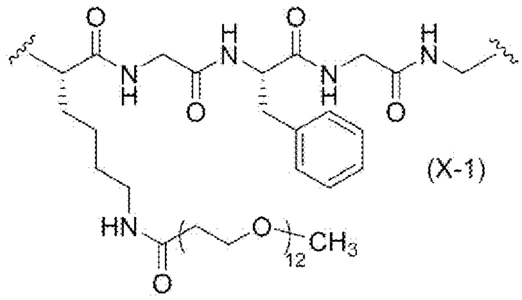
(X-1)

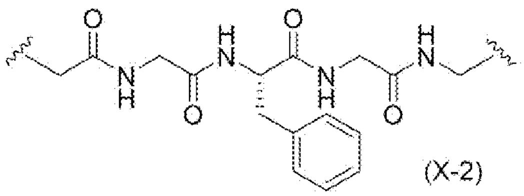
(X-2)

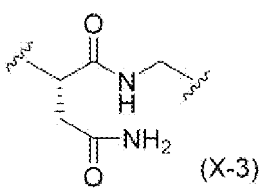
(X-3)

(where

at the left represents the binding site with NH and
at the right represents the binding site with D),

D is a group represented by formula (D-1) or formula (D-2):

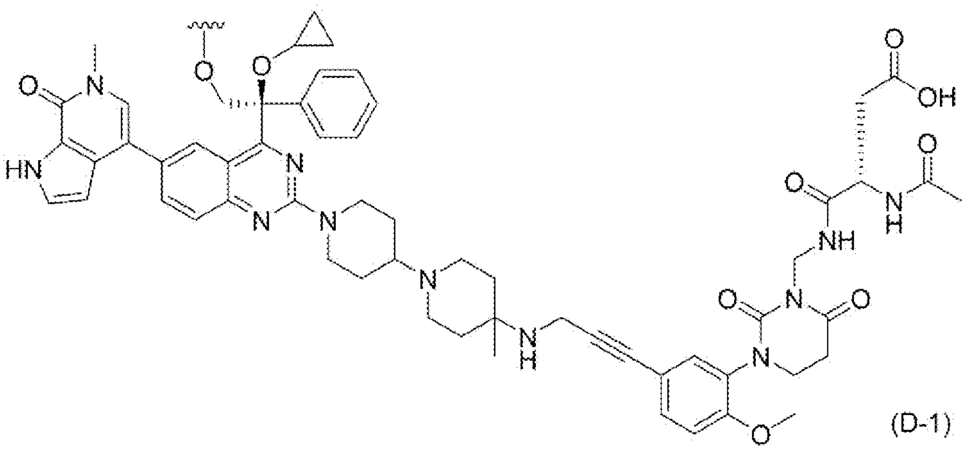

(D-1)

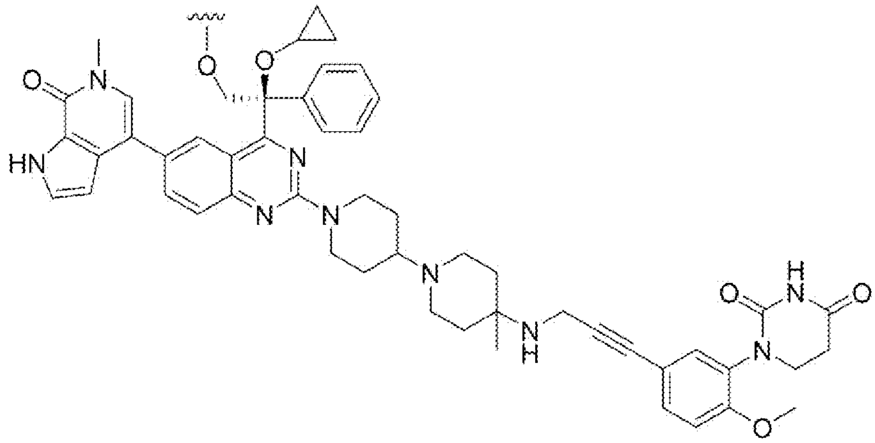

(D-2)

(where

represents the binding site with X), and
n is in the range of about 1 to about 8).

[27] A pharmaceutical composition that is to be administered in combination with a PD-1 antagonist and comprises an antibody-drug conjugate represented by formula (I):

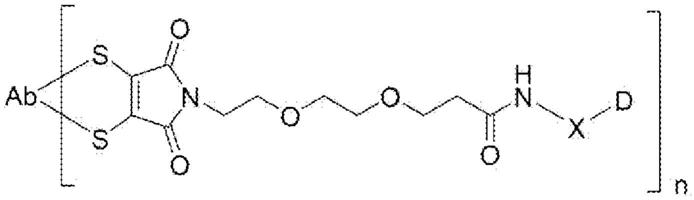

(I)

(where

Ab is an anti-folate receptor α antibody, anti-mesothelin antibody, anti-HER2 antibody, anti-FLT3 antibody, anti-DLL3 antibody, anti-CLDN6 antibody, anti-EGFR antibody, anti-Nectin4 antibody, anti-CA9 antibody, anti-CLDN3/4 antibody or anti-TROP2 antibody,
X is a group represented by formula (X-1), formula (X-2) or formula (X-3):

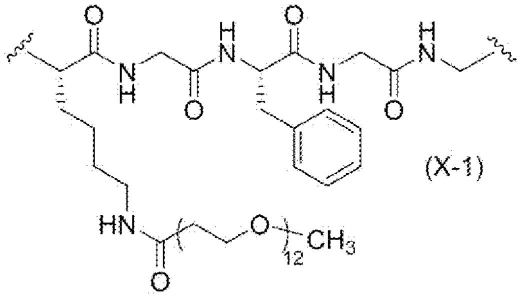

(X-1)

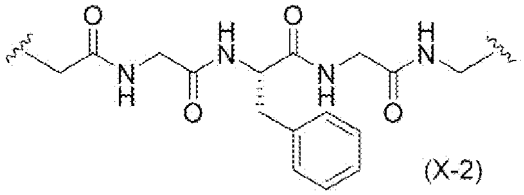

(X-2)

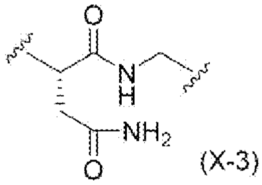

(X-3)

(where

at the left represents the binding site with NH and
at the right represents the binding site with D),
D is a group represented by formula (D-1) or formula (D-2):

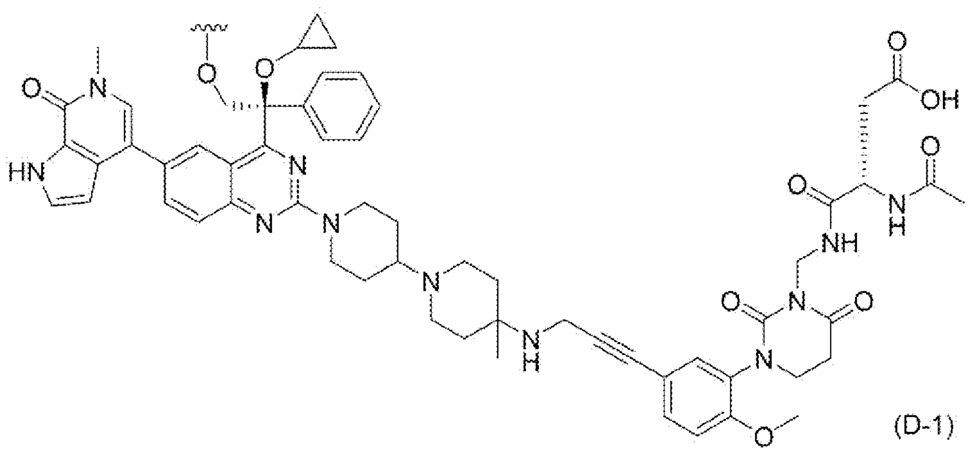

(D-1)

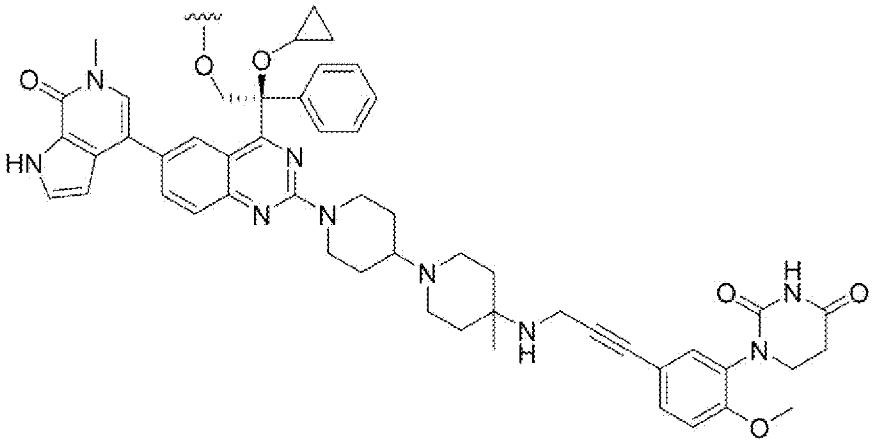

(D-2)

(where

represents the binding site with X), and

n is in the range of about 1 to about 8).

[28] A pharmaceutical composition comprising a PD-1 antagonist, that is to be administered in combination with an antibody-drug conjugate represented by formula (I):

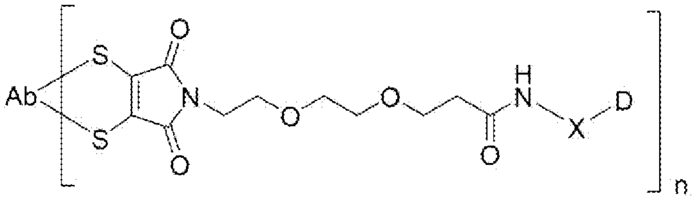

(I)

(where

Ab is an anti-folate receptor α antibody, anti-mesothelin antibody, anti-HER2 antibody, anti-FLT3 antibody, anti-DLL3 antibody, anti-CLDN6 antibody, anti-EGFR antibody, anti-Nectin4 antibody, anti-CA9 antibody, anti-CLDN3/4 antibody or anti-TROP2 antibody,
X is a group represented by formula (X-1), formula (X-2) or formula (X-3):

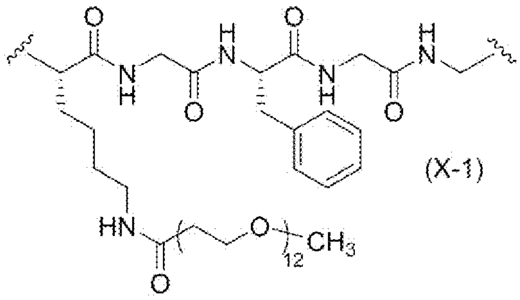

(X-1)

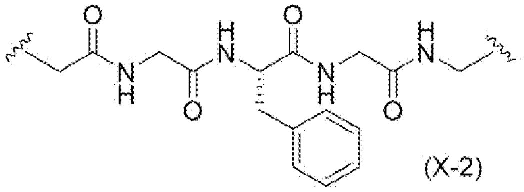

(X-2)

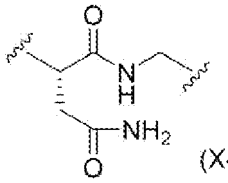

(X-3)

(where

at the left represents the binding site with NH and
at the right represents the binding site with D),
D is a group represented by formula (D-1) or formula (D-2):

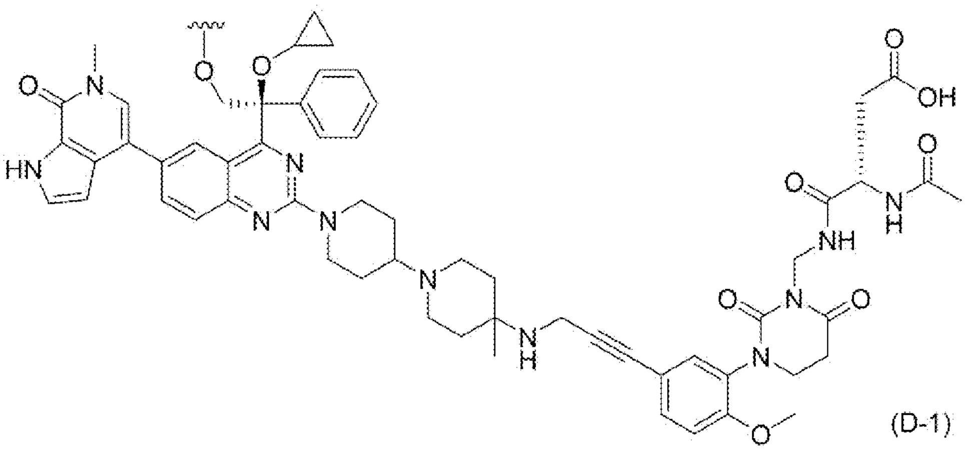

(D-1)

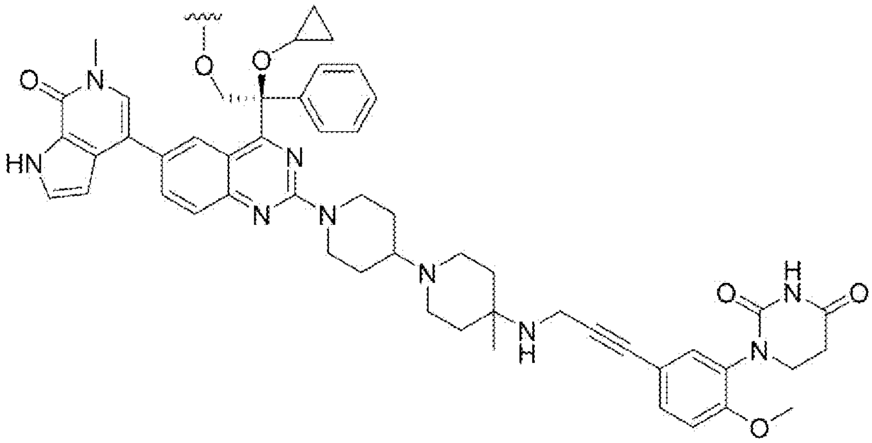

(D-2)

(where

represents the binding site with X), and
n is in the range of about 1 to about 8).

[29] The pharmaceutical composition according to any one of [25] to [28], wherein the PD-1 antagonist is an anti-PD-1 antibody.
[30] The pharmaceutical composition according to [29], wherein the anti-PD-1 antibody is selected from the group consisting of Nivolumab, Pembrolizumab, Cemiplimab, Sintilimab, Toripalimab, Spartalizumab, Tislelizumab, Dostarlimab, Camrelizumab, Genolimzumab, Lodapolimab, Retifanlimab, Balstilimab, Serplulimab, Budigalimab, Prolgolimab, Sasanlimab, Cetrelimab, Zimberelimab, Penpulimab, AMP-514, STI-A1110, ENUM388D4, ENUM244C8, GLS010, CS1003, BAT-1306, AK103, BI754091, LZM009, CMAB819, Sym021, SSI-361, JY034, HX008, ISU106 and CX-188.
[31] The pharmaceutical composition according to [30], wherein the anti-PD-1 antibody is selected from the group consisting of Nivolumab, Pembrolizumab, Cemiplimab, Sintilimab and Toripalimab.
[32] A method for treating a tumor, which includes administering an antibody-drug conjugate represented by formula (I) and a PD-1 antagonist to a patient in need of thereof:

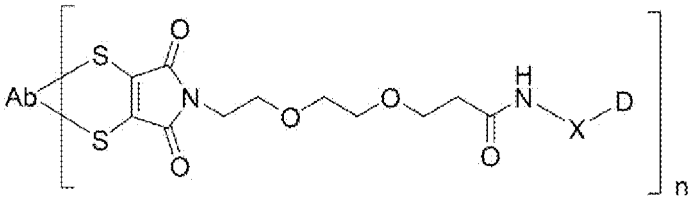

(I)

(where

Ab is an anti-CEACAM6 antibody, anti-folate receptor α antibody, anti-mesothelin antibody, anti-HER2 antibody, anti-FLT3 antibody, anti-DLL3 antibody, anti-CLDN6 antibody, anti-EGFR antibody, anti-Nectin4 antibody, anti-CA9 antibody, anti-CLDN3/4 antibody or anti-TROP2 antibody,
X is a group represented by formula (X-1), formula (X-2) or formula (X-3):

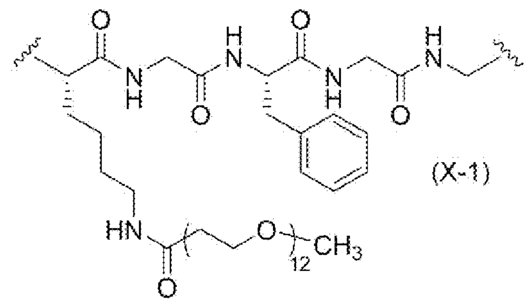

(X-1)

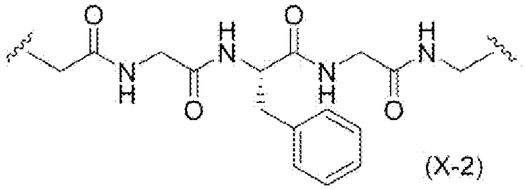
(X-2)
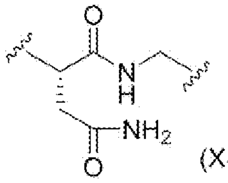
(X-3)

(where

at the left represents the binding site with NH and
at the right represents the binding site with D),
D is a group represented by formula (D-1) or formula (D-2):

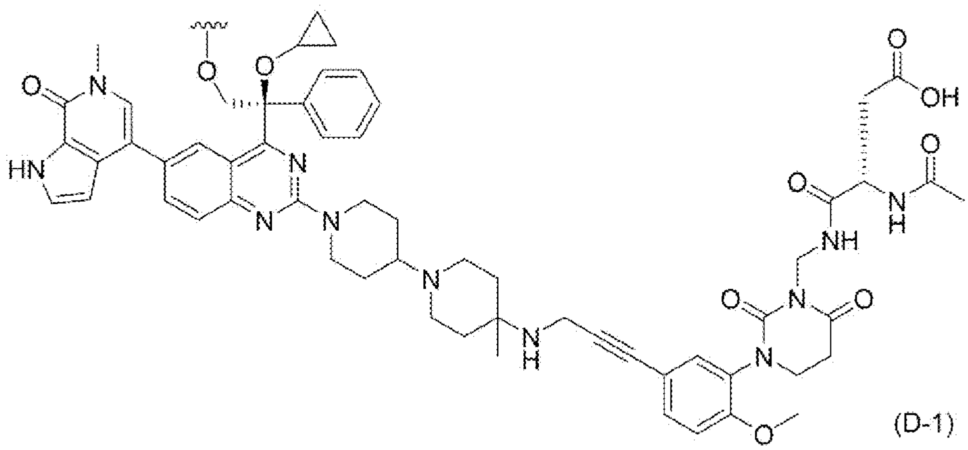
(D-1)

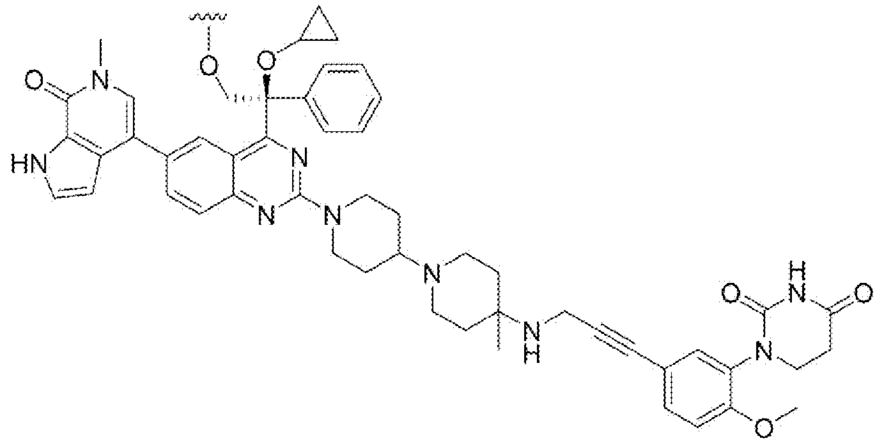
(D-2)

(where

represents the binding site with X), and
n is in the range of about 1 to about 8).

[33] The method according to [32], wherein the PD-1 antagonist is an anti-PD-1 antibody.
[34] The method according to [33], wherein the anti-PD-1 antibody is selected from the group consisting of Nivolumab, Pembrolizumab, Cemiplimab, Sintilimab, Toripalimab, Spartalizumab, Tislelizumab, Dostarlimab, Camrelizumab, Genolimzumab, Lodapolimab, Retifanlimab, Balstilimab, Serplulimab, Budigalimab, Prolgolimab, Sasanlimab, Cetrelimab, Zimberelimab, Penpulimab, AMP-514, STI-A1110, ENUM388D4, ENUM244C8, GLS010, CS1003, BAT-1306, AK103, BI754091, LZM009, CMAB819, Sym021, SSI-361, JY034, HX008, ISU106 and CX-188.
[35] The method according to [34], wherein the anti-PD-1 antibody is selected from the group consisting of Nivolumab, Pembrolizumab, Cemiplimab, Sintilimab and Toripalimab.

## Advantageous Effects of Invention

According to the invention it is possible to provide an antibody-drug conjugate that is effective against cancer. The antibody-drug conjugate of the invention has potential utility as an anticancer agent.

## Brief Description of Drawings

Fig. 1 is a graph showing change in tumor volume after injecting an antibody-drug conjugate into mice that had been subcutaneously transplanted with the human-derived pancreatic cancer cell line AsPC-1 in Example 401.
Fig. 2 is a graph showing change in tumor volume after injecting an antibody-drug conjugate into mice that had been subcutaneously transplanted with the human-derived pancreatic cancer cell line AsPC-1 in Example 402.
Fig. 3 is a graph showing change in tumor volume after injecting an antibody-drug conjugate into mice that had been subcutaneously transplanted with the human-derived uterine cancer cell line HEC-251 in Example 403.
Fig. 4 is a graph showing change in tumor volume after injecting an antibody-drug conjugate into mice that had been subcutaneously transplanted with the human-derived lung cancer cell line NCI-H226 in Example 404.
Fig. 5 is a graph showing change in tumor volume after injecting an antibody-drug conjugate into mice that had been subcutaneously transplanted with the human-derived stomach cancer cell line NCI-N87 in Example 405.
Fig. 6 is a graph showing change in tumor volume after injecting an antibody-drug conjugate into mice that had been subcutaneously transplanted with the human-derived bladder cancer cell line HT-1376 in Example 406.
Fig. 7 is a graph showing change in tumor volume after injecting an antibody-drug conjugate into mice that had been subcutaneously transplanted with the human-derived pancreatic cancer cell line HPAF-II in Example 407.
Fig. 8 is a graph showing change in tumor volume after injecting an antibody-drug conjugate into mice that had been subcutaneously transplanted with the human-derived ovarian cancer cell line COV644 in Example 408.
Fig. 9 is a graph showing change in tumor volume after injecting an antibody-drug conjugate into mice that had been subcutaneously transplanted with the human-derived pancreatic cancer cell line HPAF-II in Example 409.
Fig. 10 is a graph showing change in tumor volume after injecting an antibody-drug conjugate into mice that had been subcutaneously transplanted with the human-derived pancreatic cancer cell line HPAF-II in Example 410.
Fig. 11 is a graph showing change in mean tumor volume of each group after beginning administration of the antibody-drug conjugate and/or the mouse PD-1 antibody in Example 502.

## Description of Embodiments

The present invention will now be explained in detail.

As used herein, "$C_{1-6}$ alkyl group" means a straight-chain or branched saturated aliphatic hydrocarbon group 1 to 6 carbon atoms. Examples of $C_{1-6}$ alkyl groups include methyl, ethyl, 1-propyl, 2-propyl, 2-methyl-1-propyl, 2-methyl-2-propyl, 1-butyl, 2-butyl, 1-pentyl, 2-pentyl, 3-pentyl, 1-hexyl, 2-hexyl and 3-hexyl groups, with methyl, ethyl and 1-propyl groups being preferred.

As used herein, "$C_{2-6}$ alkynyl group" means a straight-chain or branched unsaturated aliphatic hydrocarbon group of 2 to 6 carbon atoms, having one or more carbon-carbon triple bonds. Examples of $C_{2-6}$ alkynyl groups include ethynyl, 1-propynyl, 2-propynyl, 1-butynyl, 1-methyl-2-propynyl, 3-butynyl, 1-pentynyl and 1-hexynyl groups, with ethynyl group being preferred.

As used herein, "$C_{3-6}$ cycloalkyl group" means an aliphatic saturated hydrocarbon ring group of 3 to 6 carbon atoms. Examples of $C_{3-6}$ cycloalkyl groups include cyclopropyl, cyclobutyl, cyclopentyl and cyclohexyl groups, with cyclopropyl group being preferred.

The term "$C_{6-10}$ aryl group" as used herein refers to an aromatic cyclic hydrocarbon group of 6 to 10 carbon atoms. Examples of $C_{6-10}$ aryl groups include phenyl, 1-naphthyl and 2-naphthyl groups, with phenyl being preferred.

The term "$C_{1-6}$ alkoxy group" as used herein means the aforementioned "$C_{1-6}$ alkyl group" having an oxygen atom bonded to the end. Examples of $C_{1-6}$ alkoxy groups include methoxy, ethoxy, 1-propoxy, 2-propoxy, 2-methyl-1-propoxy, 2-methyl-2-propoxy, 1-butoxy, 2-butoxy, 1-pentyloxy, 2-pentyloxy, 3-pentyloxy, 1-hexyloxy, 2-hexyloxy and 3-hexyloxy groups, with methoxy, ethoxy and 1-propoxy groups being preferred.

The term "$C_{3-6}$ cycloalkoxy group" as used herein means the aforementioned "$C_{3-6}$ cycloalkyl group" having an oxygen atom bonded to the end. Exampless of $C_{3-6}$ cycloalkoxy groups include cyclopropoxy, cyclobutoxy, cyclopentyloxy and cyclohexyloxy groups, with cyclopropoxy being preferred.

The term "$C_{1-6}$ alkylcarbamoyloxy group" as used herein means the aforementioned "$C_{1-6}$ alkyl group" having a carbamoyloxy group bonded to the end. Examples of $C_{1-6}$ alkylcarbamoyloxy groups include methylcarbamoyloxy and ethylcarbamoyloxy groups.

As used herein, "pharmacologically acceptable salt" refers to a salt of an inorganic acid, a salt of an organic acid, or a salt of an acidic amino acid, for example.

Preferred examples of salts with inorganic acids include salts with hydrochloric acid, hydrobromic acid, sulfuric acid, nitric acid and phosphoric acid.

Preferred examples of salts with organic acids include salts with acetic acid, succinic acid, fumaric acid, maleic acid, tartaric acid, citric acid, lactic acid, stearic acid, benzoic acid, methanesulfonic acid, ethanesulfonic acid and *p*-toluenesulfonic acid.

Preferred examples of salts with acidic amino acids include salts with aspartic acid and glutamic acid.

[Drug portion of antibody-drug conjugate]

The drug portion of the antibody-drug conjugate of the embodiment is a group represented by formula (D-1) or formula (D-2):

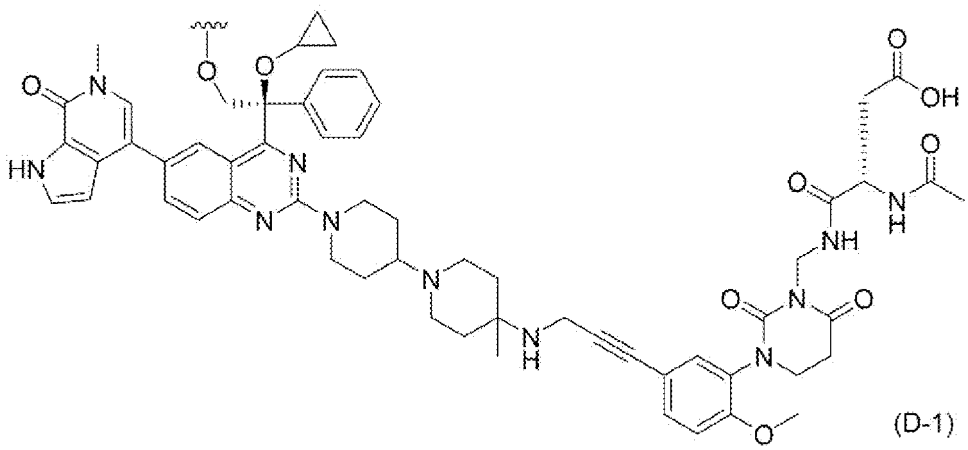

(D-1)

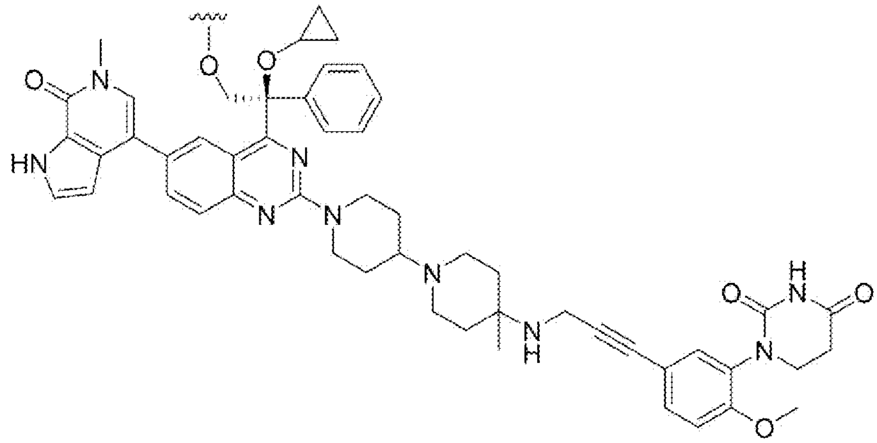

(D-2)

(where
represents the binding site with the linker).

[Drug released from antibody-drug conjugate]

The drug released from antibody-drug conjugate of one embodiment is a compound represented by formula (D'):

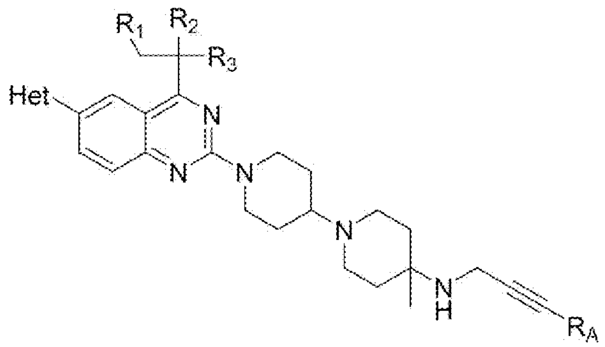

(D')

(where the symbols have the same definitions as in [10] above),
or a pharmaceutically acceptable salt thereof.

The drug released from antibody-drug conjugate of one embodiment is a compound represented by formula (D'-1) or formula (D'-2), or a pharmaceutically acceptable salt thereof.

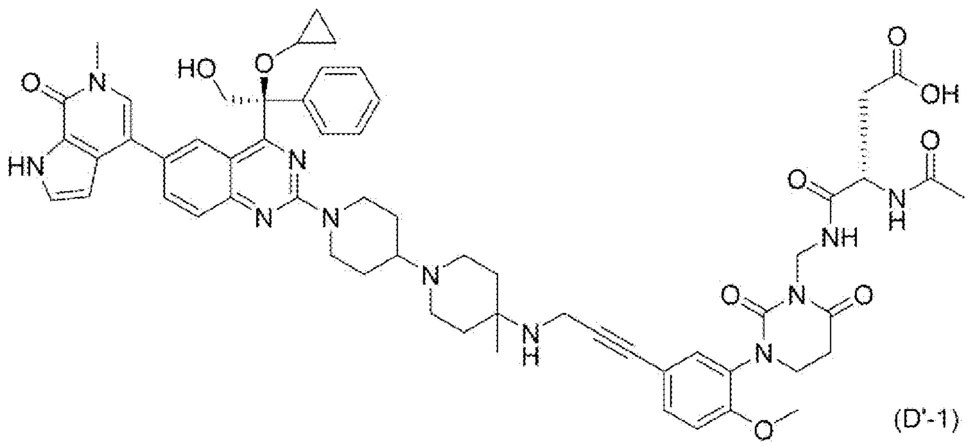

(D'-1)

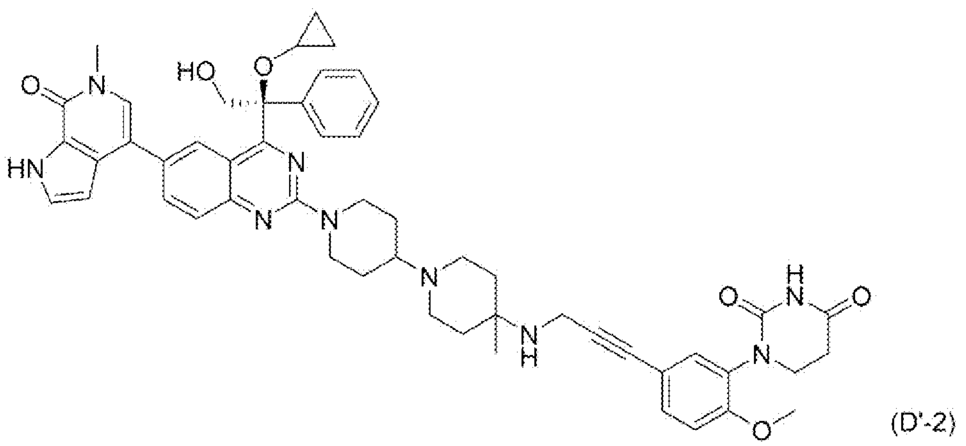

(D'-2)

The partial structure of the drug released from antibody-drug conjugate of one embodiment may be a compound represented by formula (M):

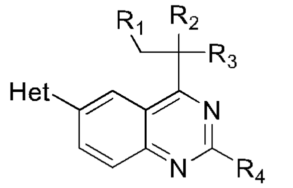

(M)

(where the symbols have the same definitions as in [12] above),
or a pharmacologically acceptable salt thereof.

[Linker of antibody-drug conjugate]

The linker in the antibody-drug conjugate of the embodiment is stable outside of the cell so that the therapeutic effect is adequate. According to one embodiment, since the linker is stable outside of cells, the antibody portion of the antibody-drug conjugate remains bonded to the drug portion through the linker under extracellular conditions (for example, before transport or delivery to cells).

The linker may be "cleavable" or "non-cleavable". A cleavable linker is designed so as to release the drug when exposed to specific environmental factors, such as when internalized in the target cells. Anon-cleavable linker is generally dependent on the antibody portion itself. The linker according to one embodiment is a cleavable linker, and may be any linker that includes a cleavable portion. As used herein, the term "cleavable portion" refers to any chemical bond that can be cleaved. Suitable cleavable chemical bonds are known in the technical field, and without being limitative they include acid-labile bonds, protease/peptidase-labile bonds, photolabile bonds, disulfide bonds and esterase-labile bonds.

The linker according to one embodiment is cleavable by cleaving agents such as enzymes in the intracellular environment (such as in lysosomes). The linker may be, but is not limited to, a peptide linker that is cleaved by intracellular peptidases or proteases (such as in lysosomes). According to one embodiment, the linker is a cleavable peptide linker. As used herein, the term "cleavable peptide linker" refers to any linker containing a cleavable peptide portion. The term "cleavable peptide portion" refers to any chemical bond-crosslinkable amino acid (natural or synthetic amino acid derivative) that can be cleaved by an active substance in the intracellular environment. For example, the linker may be cleavable by a cathepsin (such as cathepsin B), or a cysteine protease such as legumain.

The linker according to one embodiment is bonded to the antibody portion by a chemically active group of one or more amino acid residues of the antibody portion. For example, the linker may be bonded to the antibody portion (for example, to the N-terminus or C-terminus, to ε-amino groups of one or more lysine residues, to free carboxylic acid groups of one or more glutamic acid or aspartic acid residues, or to sulfhydryl groups of one or more cysteine residues), via a free amino, imino, hydroxyl, thiol or carboxyl group. The bonding site for the linker may be a natural residue in the amino acid sequence of the antibody portion, or it may be introduced into the antibody portion by DNA recombinant technology (such as introduction of a cysteine residue into the amino acid sequence), or by using protein biochemistry (such as reduction, pH adjustment or hydrolysis).

The linker according to one embodiment may also include at least one spacer unit that connects the antibody portion to the drug portion. According to one embodiment, the spacer unit connects a cleavage site in the linker (such as a cleavable peptide portion) to the antibody portion, and according to another embodiment the linker includes one or more polyethylene glycol (PEG) portions.

The linker according to one embodiment is a group represented by the following formula (X-1'), formula (X-2') or formula (X-3'):

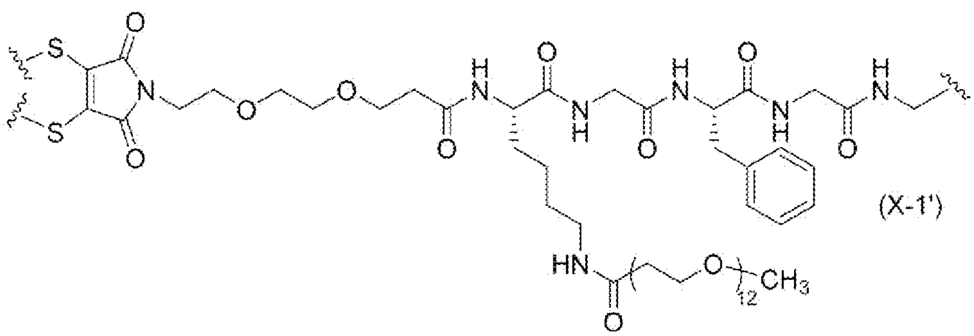

(X-1')

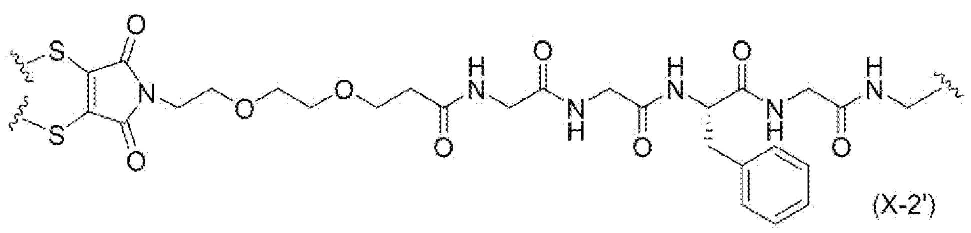

(X-2')

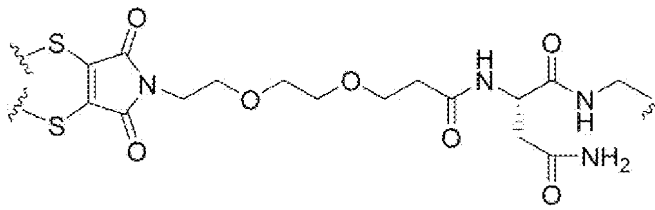

(X-3')

[Antibody portion of antibody-drug conjugate]

The antibody portion of the antibody-drug conjugate for this embodiment may be an anti-CEACAM6 antibody, anti-folate receptor α antibody, anti-mesothelin antibody, anti-HER2 antibody, anti-FLT3 antibody, anti-DLL3 antibody, anti-CLDN6 antibody, anti-EGFR antibody, anti-Nectin4 antibody, anti-CA9 antibody, anti-CLDN3/4 antibody or anti-TROP2 antibody. The antibody may be a monoclonal antibody (mAb) or an antigen binding fragment thereof. A monoclonal antibody may be a human antibody, humanized antibody or chimeric antibody, and it may also include the human constant region. The human constant region may be selected from the group consisting of IgG1, IgG2, IgG3 and IgG4 constant regions. The antigen binding fragment is selected from the group consisting of Fab, Fab'-SH, $F(ab')_2$, scFv and Fv fragments. The antibody portion of the antibody-drug conjugate for this embodiment may be of any class such as IgG, IgA or IgM (or their subclass), with no limitation to any particular class. Immunoglobulins are classified according to class based on the antibody amino acid sequences of the constant region of the heavy chain (H chain). Several of the 5 major immunoglobulin classes: IgA, IgD, IgE, IgG and IgM are further subdivided into the subclasses (isotypes) of IgG1, IgG2, IgG3, IgG4, IgA1 and IgA2. The constant regions of the heavy chains corresponding to the specific immunoglobulin classes are referred to as α, δ, ε, γ and μ. Antibody light chains (also known as L chains) have λ chain and κ chain types.

The antibody portion of the antibody-drug conjugate of the embodiment may be an IgG antibody, such as an IgG1 antibody or IgG2 antibody. The antibody portion of the antibody-drug conjugate of the embodiment may be a monomer, dimer or multimer.

The variable region of the antibody of the disclosure may be the variable region of the light chain of the antibody and/or the variable region of the heavy chain of the antibody, and the constant region of the antibody may be the constant region of the light chain of the antibody and/or the constant region of the heavy chain of the antibody. The variable regions of the heavy chain and light chain are each linked by three CDRs, also known as complementarity determining regions, to form four framework regions (FR). The CDR of each chain is tightly bound with the FR, and together with the CDR of the other chain, contributes to formation of the antigen binding site of the antibody. There is no restriction on the technique used to determine the CDR, and examples include (1) an approach based on the sequence variability between different types (for example, Kabat et al, Sequences of Proteins of Immunological Interest, 5th ed., 1991, National Institutes of Health, Bethesda MD); and (2) an approach based on crystallographic studies of antigen-antibody complexes (Al-lazikani et al., 1997 J. Molec. Biol. 273:927-948). These approaches may also be combined with other approaches.

As used herein, "monoclonal antibody" may also refer to an antibody obtained from a population of an essentially homogeneous antibody. In other words, the individual antibodies in the population are identical, except for natural mutants which may be present in small amounts. A monoclonal antibody is highly specific for a single antigen site. In contrast to a typical polyclonal antibody which has different antigens and different epitopes as targets, a monoclonal antibody targets only one single epitope of an antigen. The modifying term "monoclonal" indicates the specificity of the antibody obtained from an essentially homogeneous antibody population, and should not be interpreted as requiring production of the antibody by a particular method.

The antibody portion of the antibody-drug conjugate of the embodiment may be a mouse antibody, chimeric antibody, humanized antibody or fully human antibody. A chimeric antibody may be an antibody having the variable region of a non-human (such as a mouse or rat) antibody fused with the constant region of a human antibody, such as an antibody having a non-human antibody-derived variable region and a human antibody-derived constant region. A humanized antibody may be an antibody having the complementarity determining region (CDR) (also known as the hypervariable region) of a non-human antibody introduced into a human antibody, such as an antibody with a non-human antibody-derived CDR and human antibody-derived regions for the other antibody regions. However, the boundary between chimeric antibody and humanized antibody does not need to be distinct, and the antibody may be one qualifying as either a chimeric antibody or humanized antibody. In a chimeric antibody or humanized antibody, the entirety of the human antibody-derived antibody region (FR, or constant region) does not need to be composed of human antibody-derived amino acids, and one or more non-human antibody-derived amino acids may also be included so long as the antibody can be properly used in a human subject. One embodiment of a humanized antibody is one wherein the CDR is derived from a rodent antibody and the rest of the antibody regions are human antibody-derived. A more specific embodiment of a humanized antibody is one wherein the CDR is derived from a mouse antibody and the rest of the antibody regions are human antibody-derived. For such embodiments, the CDR may include one or more non-rodent antibody-derived amino

acids, or one or more non-mouse antibody-derived amino acids, and the antibody regions other than the CDR may include one or more non-human antibody-derived amino acids. Without being limitative, the qualifier "or more" may signify 2 to 20, 2 to 15, or 14, 13, 12, 11, 10, 9, 8, 7, 6, 5, 4, 3 or 2, or it may be within 10%, within 9%, within 8%, within 7%, within 6%, within 5%, within 4%, within 3%, within 2% or within 1% of the number of amino acids in the amino acid sequence. Humanization of an antibody can be carried out by CDR grafting (Kontermann and Dubel, Antibody Engineering, Springer Lab Manual (2001) and Tsurushita et al., Methods 36:69-83(2005)), or by a method publicly known in the technical field (such as Jones et al., Nature 321:522-525(1986); Riechmann et al., Nature 332:323-327(1988) or Verhoeyen et al., Science 239:1534-1536(1988)), or by substitution of the CDR sequence with the corresponding sequence of the human antibody.

Also included within the scope of the antibody of the present disclosure are the chimeric antibodies or humanized antibodies described above with appropriate modification (such as antibody modification or partial substitution, addition and/or deletion in the antibody amino acid sequence), while maintaining the function of the antibodies (or with additional or enhanced antibody functions by the modification). More specifically, the scope of the present disclosure also includes antibodies with variations in the amino acid sequence of the constant region for modification of the effector function of the antibody, such as an antibody having valine (Val) at position 234 replaced by alanine (Ala) and the glycine (Gly) at position 237 replaced by alanine (Ala), based on Eu numbering of the human IgG2 antibody, in order to lower the antibody-dependent cell-mediated cytotoxicity (ADCC) activity and/or antibody-dependent cell phagocytosis (ADCP) activity. The scope of the present disclosure also includes bispecific antibodies having an antigen binding site that binds with a different antigen (Kontermann(2012), mAbs 4, 182-97) in addition to the antibody binding site with the CDR sequence of the antibody of the disclosure.

The antibody portion of the antibody-drug conjugate of the embodiment may also be modified if desired. Modification of the antibody may be modification that alters (a) the three-dimensional structure of the amino acid sequence at the modified region, such as its sheet or helical conformation; (b) the charged or hydrophobic state of molecules at the target site; or (c) the maintenance of the volume of side chains; or it may be modification where such alterations are not clearly observed. Modification of the antibody of the disclosure can be carried out by substitution, deletion or addition of constituent amino acid residues, for example.

The term "amino acid" is used herein in its widest sense to include not only natural amino acids such as serine (Ser), asparagine (Asn), valine (Val), leucine (Leu), isoleucine (Ile), alanine (Ala), tyrosine (Tyr), glycine (Gly), lysine (Lys), arginine (Arg), histidine (His), aspartic acid (Asp), glutamic acid (Glu), glutamine (Gln), threonine (Thr), cysteine (Cys), methionine (Met), phenylalanine (Phe), tryptophan (Trp) and proline (Pro), but also non-natural amino acids such as amino acid variants and derivatives. A person skilled in the art will naturally understand that the amino acids referred to herein throughout, in this wide sense, include L-amino acids; D-amino acids; chemically modified amino acids such as amino acid variants and amino acid derivatives; amino acids that are not constituents of biological proteins, such as norleucine, β-alanine and omithine; and chemically synthesized compounds having the properties of amino acids, which are publicly known to those skilled in the art. Examples of non-natural amino acids include α-methylamino acids (such as α-methylalanine), D-amino acids (such as D-aspartic acid and D-glutamic acid), histidine-like amino acids (such as 2-amino-histidine, β-hydroxy-histidine, homohistidine, α-fluoromethyl-histidine and α-methyl-histidine), amino acids having an extra methylene on a side chain ("homo" amino acids), and amino acids having a carboxylic acid functional group on the side chain replaced with a sulfonic acid group (such as cysteic acid).

Naturally occurring amino acid residues can be classified into the following groups based on common side chain properties:

(1) Hydrophobic: Met, Ala, Val, Leu, Ile;
(2) Neutral hydrophilic: Asn, Gln, Cys, Ser, Thr;
(3) Acidic: Asp, Glu;
(4) Basic: His, Lys, Arg;
(5) Residues that affect chain orientation: Gly, Pro; and
(6) Aromatic: Trp, Tyr, Phe.

A non-conservative substitution in a constituent amino acid sequence of an antibody can be made by substituting an amino acid belonging to one of these groups with an amino acid belonging to another group. For more conservative substitution, an amino acid belonging to one of these groups can be substituted with another amino acid belonging to the same group. Deletion or substitution in the amino acid sequence may likewise be carried out as appropriate.

An example of modification of a constituent amino acid of an antibody is post-translational modification such as glycosylation with a sugar, acetylation or phosphorylation. The antibody may be glycosylated at a conserved position in the constant region. Glycosylation of an antibody will usually be N-linked or O-linked. N-linked glycosylation is bonding of the sugar portion at the side chain of an asparagine residue. The tripeptide sequences asparagine-X-serine, asparagine-X-threonine and asparagine-X-cysteine (where X is any amino acid other than proline) are recognition sequences for enzymatic addition of sugar portions onto asparagine side chains. All of these tripeptide sequences when present in an

antibody serve as latent glycosylation sites. O-linked glycosylation may be bonding of N-acetylgalactosamine, galactose or xylose to a hydroxy (such as serine or threonine), or optionally bonding to 5-hydroxyproline or 5-hydroxylysine. The conditions for glycosylation (for example, the types and pH of the host cells and cell culture medium, if the glycosylation is by a biological method), may be appropriately selected for the purpose by a person skilled in the art.

The antibody of the disclosure may also be modified by other methods of modification based on common technical knowledge to those skilled in the art, either alone or in combinations. The antibody of the disclosure can also be produced by a method known to those skilled in the art. For example, nucleic acid coding for the antibody of the disclosure may be incorporated into an expression vector which is introduced into host cells, producing the antibody by culturing the host cells.

The abbreviation "CEACAM6" as used herein refers to "carcinoembryonic antigen-related cell adhesion molecule 6", also known as "CD66c" (cluster of differentiation 66c), nonspecific crossreactive antigen, NCA, or NCA-50/90. As used herein, "CEACAM6" can be understood to refer to human, mouse, rat or monkey CEACAM6. Human, mouse, rat and monkey CEACAM6 are available from public databases with registered sequence information, such as Genbank provided by the National Center for Biotechnology Information, while sequence information for a CEACAM6 gene can also be obtained by cloning from RNA extracted from an animal species of interest, designing primers based on nucleotide sequence information for CEACAM6 of a closely related animal species. CEACAM6 is a glycosylphosphatidylinositol (GPI)-binding cell surface protein involved in cell-to-cell adhesion. CEACAM6 is highly expressed on the surfaces of different types of tumor cells including colon cancer, pancreatic cancer, breast cancer and lung cancer cells.

According to one embodiment, the anti-CEACAM6 antibody of the disclosure includes the following CDRs:

(a) the heavy chain CDR1 comprising the amino acid sequence listed as SEQ ID NO: 18;
(b) the heavy chain CDR2 comprising the amino acid sequence listed as SEQ ID NO: 19; and
(c) the heavy chain CDR3 comprising the amino acid sequence listed as SEQ ID NO: 20; and
(d) the light chain CDR1 comprising the amino acid sequence listed as SEQ ID NO: 21;
(e) the light chain CDR2 comprising the amino acid sequence listed as SEQ ID NO: 21-1; and
(f) the light chain CDR3 comprising the amino acid sequence listed as SEQ ID NO: 22.

According to one embodiment, the anti-CEACAM6 antibody is a humanized antibody, fully human antibody or chimeric antibody, and particularly a chimeric antibody according to a more specific embodiment. According to a more specific embodiment, the anti-CEACAM6 antibody is a chimeric antibody of IgG1 and IgG2m without affinity for FcgR and disulfide isoforms.

According to one embodiment, the anti-CEACAM6 antibody includes a heavy chain and a light chain, the variable region of the heavy chain comprising the amino acid sequence listed as SEQ ID NO: 6, and the variable region of the light chain comprising the amino acid sequence listed as SEQ ID NO: 7. According to this embodiment, the heavy chain variable region and/or light chain variable region may include an amino acid sequence having a substitution, addition and/or deletion of one or more amino acids of the amino acid sequence listed as SEQ ID NO: 6 and/or the amino acid sequence listed as SEQ ID NO: 7. The qualifier "or more" is not restricted so long as binding affinity with CEACAM6 is conserved and cleavage of CEACAM6 is promoted, and it may signify 2 to 15, 2 to 10, or 9, 8, 7, 6, 5, 4, 3 or 2, or it may be within 10%, such as within 9%, within 8%, within 7%, within 6%, within 5%, within 4%, within 3%, within 2% or within 1% of the number of amino acids in the amino acid sequence.

According to one embodiment, the heavy chain of the anti-CEACAM6 antibody includes part of human IgG1 and part of the human IgG2 constant region. According to a specific embodiment, the anti-CEACAM6 antibody includes the amino acid sequence listed as SEQ ID NO: 10, with the CH1 portion and hinge portion as human IgG1 and the CH2 portion and CH3 portion as the constant region of human IgG2 containing V234A and G237A.

According to one embodiment, the light chain of the anti-CEACAM6 antibody includes part of the constant region of human Igλ. According to a specific embodiment, the constant region of human Igλ includes the amino acid sequence listed as SEQ ID NO: 11.

According to one embodiment, the anti-CEACAM6 antibody includes a heavy chain including the amino acid sequence listed as SEQ ID NO: 6 and a light chain including the amino acid sequence listed as SEQ ID NO: 7.

The antibody portion of the antibody-drug conjugate of the embodiment may be an anti-folate receptor α antibody, and according to one embodiment it may be farletuzumab. Farletuzumab is a humanized IgG1 monoclonal antibody for folate receptor α (FRA), a type of folate receptor, and it can be produced by the method described in US Patent No. 4805848, for example.

According to one embodiment, the anti-folate receptor α antibody of the disclosure includes the following CDRs:

(g) the heavy chain CDR1 comprising the amino acid sequence listed as SEQ ID NO: 28;
(h) the heavy chain CDR2 comprising the amino acid sequence listed as SEQ ID NO: 29; and
(i) the heavy chain CDR3 comprising the amino acid sequence listed as SEQ ID NO: 30; and

(j) the light chain CDR1 comprising the amino acid sequence listed as SEQ ID NO: 31;
(k) the light chain CDR2 comprising the amino acid sequence listed as SEQ ID NO: 31-1; and
(l) the light chain CDR3 comprising the amino acid sequence listed as SEQ ID NO: 32.

According to a specific embodiment, the anti-folate receptor α antibody includes a heavy chain and a light chain, the variable region of the heavy chain comprising the amino acid sequence listed as SEQ ID NO: 33, and the variable region of the light chain comprising the amino acid sequence listed as SEQ ID NO: 34. According to this embodiment, the heavy chain variable region and/or light chain variable region may include an amino acid sequence having a substitution, addition and/or deletion of one or more amino acids of the amino acid sequence listed as SEQ ID NO: 33 and/or the amino acid sequence listed as SEQ ID NO: 34. The qualifier "or more" is not restricted so long as binding affinity with folate receptor α is conserved and cleavage of folate receptor α is promoted, and it may signify 2 to 15, 2 to 10, or 9, 8, 7, 6, 5, 4, 3 or 2, or it may be within 10%, such as within 9%, within 8%, within 7%, within 6%, within 5%, within 4%, within 3%, within 2% or within 1% of the number of amino acids in the amino acid sequence.
According to one embodiment, the anti-folate receptor α antibody includes a heavy chain including the amino acid sequence listed as SEQ ID NO: 35 and a light chain including the amino acid sequence listed as SEQ ID NO: 36.
The antibody portion of the antibody-drug conjugate of the embodiment may be anti-mesothelin antibody. The anti-mesothelin antibody can be produced by the method described in International Patent Publication No. 2021/0900062.
According to one embodiment, the anti-mesothelin antibody of the disclosure includes the following CDRs:

(g) the heavy chain CDR1 comprising the amino acid sequence listed as SEQ ID NO: 38;
(h) the heavy chain CDR2 comprising the amino acid sequence listed as SEQ ID NO: 39; and
(i) the heavy chain CDR3 comprising the amino acid sequence listed as SEQ ID NO: 40; and
(j) the light chain CDR1 comprising the amino acid sequence listed as SEQ ID NO: 41;
(k) the light chain CDR2 comprising the amino acid sequence listed as SEQ ID NO: 41-1; and
(l) the light chain CDR3 comprising the amino acid sequence listed as SEQ ID NO: 42.

According to one embodiment, the anti-mesothelin antibody includes a heavy chain and a light chain, the variable region of the heavy chain comprising the amino acid sequence listed as SEQ ID NO: 43, and the variable region of the light chain comprising the amino acid sequence listed as SEQ ID NO: 44. According to this embodiment, the heavy chain variable region and/or light chain variable region may include an amino acid sequence having a substitution, addition and/or deletion of one or more amino acids of the amino acid sequence listed as SEQ ID NO: 43 and/or the amino acid sequence listed as SEQ ID NO: 44. The qualifier "or more" is not restricted so long as binding affinity with mesothelin is conserved and cleavage of mesothelin is promoted, and it may signify 2 to 15, 2 to 10, or 9, 8, 7, 6, 5, 4, 3 or 2, or it may be within 10%, such as within 9%, within 8%, within 7%, within 6%, within 5%, within 4%, within 3%, within 2% or within 1% of the number of amino acids in the amino acid sequence.
According to one embodiment, the anti-mesothelin antibody includes a heavy chain including the amino acid sequence listed as SEQ ID NO: 45 and a light chain including the amino acid sequence listed as SEQ ID NO: 46.
One example of anti-HER2 antibody is trastuzumab.
According to one embodiment, the anti-FLT3 antibody includes a heavy chain including the amino acid sequence listed as SEQ ID NO: 48 and a light chain including the amino acid sequence listed as SEQ ID NO: 49.
According to one embodiment, the anti-DLL3 antibody includes a heavy chain including the amino acid sequence listed as SEQ ID NO: 50 and a light chain including the amino acid sequence listed as SEQ ID NO: 51.
According to one embodiment, the anti-CLDN6 antibody includes a heavy chain including the amino acid sequence listed as SEQ ID NO: 52 and a light chain including the amino acid sequence listed as SEQ ID NO: 53.
According to one embodiment the anti-EGFR antibody may be cetuximab, for example.
According to one embodiment, the anti-Nectin4 antibody includes a heavy chain including the amino acid sequence listed as SEQ ID NO: 54 and a light chain including the amino acid sequence listed as SEQ ID NO: 55.
According to one embodiment, the anti-TROP2 antibody includes a heavy chain including the amino acid sequence listed as SEQ ID NO: 56 and a light chain including the amino acid sequence listed as SEQ ID NO: 57.
According to one embodiment, the anti-CA9 antibody includes a heavy chain including the amino acid sequence listed as SEQ ID NO: 58 and a light chain including the amino acid sequence listed as SEQ ID NO: 59.
According to one embodiment, the anti-CLDN3/4 antibody includes a heavy chain including the amino acid sequence listed as SEQ ID NO: 60 and a light chain including the amino acid sequence listed as SEQ ID NO: 61.

[Antibody-drug conjugate]

The antibody-drug conjugate of this embodiment is represented by formula (I):

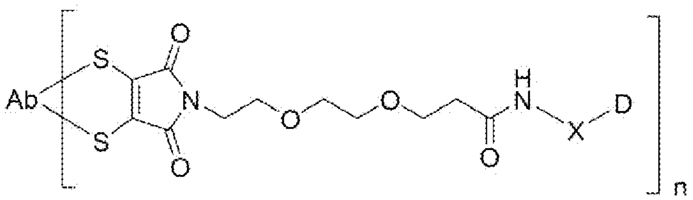

(I)

(where the symbols are as defined in [1] above).

The antibody-drug conjugate of the embodiment may be administered by injection (intravenous injection, intraarterial injection or local injection), or by an intranasal, transdermal or transpulmonary route, or by eye drop, and for example, injection may be intravenous injection, subcutaneous injection, intradermal injection or intraarterial injection, or local injection into the target cells or organ. The dosage form for the antibody-drug conjugate for parenteral administration may be injection, drip, eye drop, ointment, suppository, suspension, poultice, lotion, aerosol or plaster, or it may be injection or drip, according to one embodiment. The antibody-drug conjugate of the embodiment can be formulated by a method described in Japanese Pharmacopoeia, 17th Edition (JP), U.S. Pharmacopeia (USP) or European Pharmacopeia (EP), for example.

The type of cancer to be treated by the pharmaceutical composition of the embodiment is not particularly restricted, and may be pancreatic cancer, breast cancer, stomach cancer, non-small-cell lung cancer, bladder cancer, endometrial cancer, hepatocellular carcinoma, bile duct cancer, melanoma, esophageal cancer, colorectal cancer, renal cell carcinoma, head and neck cancer, pleural mesothelioma or Hodgkin’s lymphoma. According to one embodiment, the cancer to be treated is pancreatic cancer.

[Drug-antibody ratio (DAR) of antibody-drug conjugate]

The drug-antibody ratio (DAR) of the antibody-drug conjugate is represented as "n" and is the average number of drug portions per antibody portion (i.e. the average DAR or average "n"), which can be calculated by a publicly known method in the technical field such as mass spectrometry (including reversed-phase LC-MS) or hydrophobic interaction chromatography (HIC). According to a specific embodiment, the drug-antibody ratio is determined by hydrophobic interaction chromatography (HIC). According to another specific embodiment, the drug-antibody ratio is determined by reversed-phase liquid chromatography-mass spectrometry (LC-MS) and capillary SDS gel electrophoresis (c-SDS).

According to another specific embodiment, the drug-antibody ratio may be in the range of 1 to 8 per antibody portion. According to another specific embodiment, "n" is an integer of 1 to 8. According to another specific embodiment, "n" is in the range of about 1 to 8, about 1 to 7, about 1 to 6, about 1 to 5, about 1 to 4, about 1 to 3 or about 1 to 2. According to another specific embodiment, "n" is in the range of about 2 to 8, about 2 to 7, about 2 to 6, about 2 to 5, about 2 to 4 or about 2 to 3. According to another specific embodiment, "n" is an integer of about 3 to 4. According to another specific embodiment, "n" is about 1, about 2, about 3, about 4, about 5 or about 6, and preferably about 3 or about 4.

The drug-antibody ratio (DAR) for the antibody-drug conjugate can be limited by the number of binding sites of the antibody portion. The term "about" as used herein in regard to the drug-antibody ratio means +/-10%.

One specific embodiment of the disclosure provides a pharmaceutical composition that includes the antibody-drug conjugate of the embodiment and a pharmaceutically acceptable diluent, carrier and/or additive.

Another embodiment includes the use of the antibody-drug conjugate of the embodiment for treatment and diagnosis in managing cancer. Yet another embodiment includes a method for managing cancer which expresses an antigen (such as CEACAM6) targeted by the antibody portion of the antibody-drug conjugate of the embodiment. Yet another embodiment provides a method of causing death of tumor cells or cancer cells, or inhibiting their proliferation, by administering a therapeutically effective dose and/or regimen of any antibody-drug conjugate of the embodiment.

According to one specific embodiment of the disclosure, the antibody-drug conjugate of the embodiment is administered in combination with a PD-1 antagonist.

The PD-1 antagonist of the disclosure may include any compound or biomolecule that blocks binding of PD-L1 expressed by cancer cells to PD-1 expressed on immunocytes (T cells, B cells or Natural Killer T (NKT) cells), or that blocks binding of PD-L2 expressed by cancer cells to PD-1 expressed on immunocytes. The PD-1 antagonist blocks binding of human PD-L1 to human PD-1, and according to one embodiment it blocks binding of both human PD-L1 and PD-L2 to human PD-1. The amino acid sequence of human PD-1 can be confirmed at NCBI Locus No.: NP_005009. The amino acid sequences of human PD-L1 and PD-L2 can be confirmed as NCBI Locus No.: NP_054862 and NP_079515, respectively.

The PD-1 antagonist of the disclosure may also include a monoclonal antibody (mAb) that specifically binds to PD-1 or PD-L1, or specifically binds to human PD-1 or human PD-L1, or its antigen-binding fragment. The mAb may be a human antibody, humanized antibody or chimeric antibody, and it may also include the human constant region. The human constant region is selected from the group consisting of the IgG1, IgG2, IgG3 and IgG4 constant regions, and according to

one embodiment the human constant region is the IgG1 or IgG4 constant region. The antigen-binding fragment may be selected from the group consisting of Fab, Fab'-SH, $F(ab')_2$, scFv and Fv fragments.

An example of a PD-1 antagonist is anti-PD-1 antibody, and according to one embodiment it is anti-human PD-1 antibody, and according to a more specific embodiment it is anti-human PD-1 monoclonal antibody (anti-human PD-1 mAb). Examples of mAb that bind to human PD-1 are described in US Patent No. 7488802, US Patent No. 7521051, US Patent No. 8008449, US Patent No. 8354509, US Patent No. 8168757, International Patent Publication No. WO2004/004771, International Patent Publication No. WO2004/072286, International Patent Publication No. WO2004/056875 and U.S. Patent Application Publication No. 2011/0271358. When the PD-1 antagonist is an anti-human PD-1 monoclonal antibody, "anti-human PD-1 monoclonal antibody" includes Nivolumab, Pembrolizumab, Cemiplimab, Sintilimab and Toripalimab. When the PD-1 antagonist is an anti-PD-1 antibody, "anti-PD-1 antibody" further includes Spartalizumab, Tislelizumab, Dostarlimab, Camrelizumab, Genolimzumab, Lodapolimab, Retifanlimab, Balstilimab, Serplulimab, Budigalimab, Prolgolimab, Sasanlimab, Cetrelimab, Zimberelimab, Penpulimab, AMP-514, STI-A1110, ENUM388D4, ENUM244C8, GLS010, CS1003, BAT-1306, AK103, BI754091, LZM009, CMAB819, Sym021, SSI-361, JY034, HX008, ISU106 and CX-188.

Another example of a PD-1 antagonist is anti-PD-L1 antibody, and according to one embodiment it is anti-human PD-L1 antibody, and according to a more specific embodiment it is anti-human PD-L1 monoclonal antibody (anti-human PD-L1 mAb). When the PD-1 antagonist is an anti-PD-L1 antibody, "anti-PD-L1 antibody" includes Atezolizumab, Avelumab, Durvalumab, Manelimab, Pacmilimab, Envafolimab, Cosibelimab, BMS-936559, STI-1014, KN035, LY33,00054, HLX20, SHR-1316, CS1001, MSB2311, BGB-A333 and KL-A16.

The PD-1 antagonist of the disclosure may be administered by injection (intravenous injection, intraarterial injection or local injection), or by an oral, intranasal, transdermal or transpulmonary route, or by eye drop, and for example, injection may be intravenous injection, subcutaneous injection, intradermal injection or intraarterial injection, or local injection into the target cells or organ. The dosage form of a formulation comprising the PD-1 antagonist for oral administration may be a tablet, powder, granules, syrup, capsule or internal liquid drug, for example. The dosage form of a formulation comprising the PD-1 antagonist for parenteral administration may be injection, drip, eye drop, ointment, suppository, suspension, poultice, lotion, aerosol or plaster, or it may be injection or drip, according to one embodiment. The PD-1 antagonist of the disclosure can be formulated by a method described in Japanese Pharmacopoeia, 17th Edition (JP), U.S. Pharmacopeia (USP) or European Pharmacopeia (EP), for example.

When the PD-1 antagonist is an anti-PD-1 antibody, the anti-PD-1 antibody may be provided as a liquid drug, or it may be prepared as a liquid solution of freeze-dried powder in sterile water for injection before use.

When an anti-human PD-1 mAb, as a PD-1 antagonist, is to be administered as a single agent to a patient, the dose will differ significantly depending on the type of disease being treated, and the age, gender, body weight and severity of symptoms of the patient. The anti-human PD-1 mAb is administered in a dose of 1, 2, 3, 5 or 10 mg/kg (body weight), at intervals of about 14 days ($\pm$2 days), about 21 days ($\pm$2 days) or about 30 days ($\pm$2 days).

The form of administration of the antibody-drug conjugate and PD-1 antagonist of the embodiment is not particularly restricted, so long as the antibody-drug conjugate represented by formula (I) and the PD-1 antagonist are administered in combination. For example, the antibody-drug conjugate represented by formula (I) and the PD-1 antagonist may be administered to a patient simultaneously, separately, continuously, or at a time difference. The term "simultaneously" means that each component is administered within the same time period or exactly at the same time, or via the same route of administration. The term also means that both components are administered without a notable interval so that they can exhibit an additive effect, and preferably a synergistic effect. The term "separately" means that the components are administered at different intervals or at different frequencies, or by different routes of administration. The term "continuously" means that the components are administered during a fixed period by either the same route or different routes of administration, in any order. The phrase "at a time difference" means that the components are administered over different intervals for the respective components, by either the same route or different routes of administration. When the PD-1 antagonist is administered during a period of one cycle of administration of the antibody-drug conjugate represented by formula (I), or during a period of repeated cycles, this is considered to be administration of both in combination.

There is no particular restriction on the manner of combination of the antibody-drug conjugate represented by formula (I) and the PD-1 antagonist, and any method known to those skilled in the art may be used.

## Examples

The compounds of the invention may be produced by the methods described in the following Production Examples and Examples. However, these specific examples are merely illustrative and are not intended to restrict the compounds of the invention in any way.

Unless otherwise specified, the purifying silica gels used in silica gel column chromatography for the Production Examples and Examples were YMC GEL SILICA (YMC Co., Ltd., catalog code: SL06I52W), Silica Gel 60 (Kanto Chemicals), Silica Gel Spheres Fuji Silysia Chemical Ltd., catalog code: PSQ60B), Silica Gel 60 (Merck KGaA, catalog

code: 1.07734), CHROMATOREX BW (Fuji Silysia Chemical Ltd., catalog code: BW-300), Hi-Flash Column (Yamazen Corporation) or Presep Silica Gel (Wako), the purifying silica gels used for NH silica gel column chromatography were NH Silica Gel (Fuji Silysia Chemical Ltd., catalog code: NH-DM2035), Hi-Flash Column Amino (Yamazen Corporation) or Presep NH2 HC (Wako), and the purifying silica gel used for ODS silica gel column chromatography was Hi-Flash Column ODS (Yamazen Corporation). The purifying TLC plate used for silica gel thin-layer chromatography was TLC Silica Gel $60F_{254}$ (Merck KGaA, catalog code: 1.05715 or 1.05744), and the purifying PLC plate used for NH silica gel thin-layer chromatography was a CHROMATOREX NH-PLC05 plate (Fuji Silysia Chemical Ltd., catalog code: NH-PLC05). The solid-phase extraction column used was a Presep (Wako Pure Chemical Industries, Ltd., diatomaceous earth, granular).

Unless otherwise specified, the microwave reactor used in the Production Examples and Examples was Initiator+ Eight (Biotage).

Unless otherwise specified, a fully automatic fractionation LC system (Waters MassLynx MS, Fractionation System) was used for fractionating purification in the Production Examples and Examples. The column used was a Xbridge Prep C18 5 μm OBD (19 mm × 100 mm) by Waters.

Unless otherwise specified, supercritical fluid chromatography (SFC) (Prep100 SFC system by Waters) was used for chiral resolution in the Production Examples and Examples. The columns used were CHIRALPAK (registered trademark) IB (2 cm × 25 cm), CHIRALPAK (registered trademark) IF (3 cm × 25 cm) and CHIRALPAK (registered trademark) IG (2 cm × 25 cm), by Daicel Corp.

A Varian Mercury 400, Varian Mercury Plus 400, JEOL 400 (JMTC-400/54/SS, ECZ400S), JEOL 500 (JMTC-500/54/JJ, ECZ500RS) or Avance Neo 700MHz (Bruker) was used for nuclear magnetic resonance spectrum analysis. The chemical shifts in the proton nuclear magnetic resonance spectra are recorded in δ units (ppm) with respect to tetramethylsilane, and the coupling constants are recorded in Hertz (Hz). The abbreviations for the splitting patterns are the following. s: singlet, d: doublet, dd: double doublet, t: triplet, dt: double triplet, q: quartet, quin: quintet, spt: septet, m: multiplet, brs: broad singlet, brd: broad doublet, brdd: broad double doublet, brt: broad triplet. In this specification, b or br may be used as a broad singlet.

Mass spectral analysis was performed using a Waters UPLC™. The ionization method used was electrospray ionization (ESI).

Commercial products were used as appropriate for the compounds in the Production Examples and Examples.

The abbreviations used below have the following meanings.

*n*-heptane: Normal-heptane
$CDCl_3$: Deuterated chloroform
DABCO: 1,4-Diazabicyclo[2.2.2]octane
DHP: 3,4-Dihydro-2H-pyran
DIBAL-H: Diisobutylaluminum hydride
DMF: N,N-Dimethylformamide
DMSO: Dimethyl sulfoxide
DMSO-$d_6$: Deuterated dimethyl sulfoxide
HATU: N,N,N,N-Tetramethyl-O-(7-azobenzotriazol-1-yl)uronium hexafluorophosphate
LHMDS: Lithium hexamethyl disilazide
NMP: N-Methyl-2-pyrrolidone
TBME: *tert*-Butylmethyl ether
TFA: Trifluoroacetic acid
THF: Tetrahydrofuran

[Example 1]

N-(6-(1,5-Dimethyl-6-oxo-1,6-dihydropyridin-3-yl)-4-(2-hydroxy-1-phenyl-1-(4-(trifluoromethyl)phenoxy)ethyl)quinazolin-2-yl)methanesulfonamide

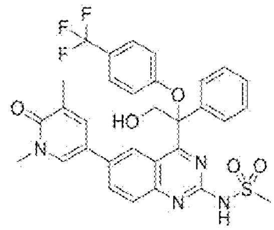

After adding methanesulfonamide (4.39 mg, 0.046 mmol), cesium carbonate (18.8 mg, 0.058 mmol), 4,5-bis(diphenylphosphino)-9,9-dimethylxanthine (4.01 mg, 6.92 μmol) and tris(dibenzylideneacetone)dipalladium(O) (6.34 mg, 6.92 μmol) to a solution of the 5-(2-chloro-4-(1-phenyl-2-((tetrahydro-2H-pyran-2-yl)oxy)-1-(4-(trifluoromethyl)phenoxy)ethyl)quinazolin-6-yl)1,3-dimethylpyridin-2(1H)-one (15.0 mg, 0.023 mmol) of Production Example 1-4 in 1,4-dioxane (1 mL) at room temperature, the mixture was stirred for 3 hours at 100°C. The reaction mixture was restored to room temperature and filtered, after which the filtrate was concentrated under reduced pressure. The obtained residue was purified by silica gel thin-layer chromatography (ethyl acetate:methanol = 19: 1) to obtain a crude product. To a solution of the obtained crude product in methanol (1 mL) there was added p-toluenesulfonic acid monohydrate (8.78 mg, 0.046 mmol) at room temperature, and the mixture was stirred for 2 hours at room temperature. After adding a saturated aqueous sodium hydrogencarbonate solution to the reaction mixture and stirring for 10 minutes, the mixture was concentrated under reduced pressure. Dichloromethane was added to the obtained residue and the mixture was filtered, and the filtrate was concentrated under reduced pressure. The obtained residue was purified by silica gel thin-layer chromatography (ethyl acetate:methanol = 19: 1) to obtain the title compound (2.0 mg).

ESI-MS (m/z): 625.33 $[M+H]^+$.

[Production Example 1-1]

Ethyl 2-phenyl-2-(4-(trifluoromethyl)phenoxy)acetate

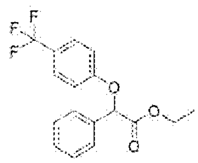

After adding potassium carbonate (639 mg, 4.63 mmol) to a solution of 4-hydroxybenzotrifluoride (500 mg, 3.08 mmol) and ethyl alpha-chlorophenylacetate (613 mg, 3.08 mmol) in DMF (10 mL) at room temperature, the mixture was stirred for 12 hours at room temperature. Water was added to the reaction mixture, and extraction was performed with ethyl acetate. The organic layer was washed with brine. The organic layer was dried over magnesium sulfate and filtered. The filtrate was concentrated under reduced pressure and the residue was purified by silica gel column chromatography (n-heptane to *n*-heptane:ethyl acetate = 7:3) to obtain the title compound (864 mg).

ESI-MS (m/z): 325.08 $[M+H]^+$.

[Production Example 1-2]

Ethyl 2-(2-chloro-6-iodoquinazolin-4-yl)-2-phenyl-2-(4-(trifluoromethyl)phenoxy)acetate

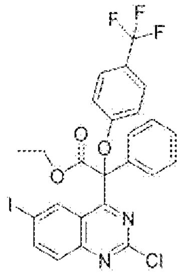

LHMDS (1 M THF solution, 4.00 mL, 4.00 mmol) was added dropwise to a solution of the ethyl 2-phenyl-2-(4-(trifluoromethyl)phenoxy)acetate of 1-1 (864 mg, 2.66 mmol) and 2,4-dichloro-6-iodoquinazoline (866 mg, 2.66 mmol) in THF (20 mL) at -78°C, and the mixture was stirred for 2 hours at room temperature. Saturated aqueous ammonium chloride was added to the reaction mixture, and extraction was performed with ethyl acetate. The organic layer was washed with brine. The organic layer was dried over magnesium sulfate and filtered. The filtrate was concentrated under reduced pressure and the residue was purified by silica gel column chromatography (n-heptane to n-heptane:ethyl acetate = 7:3) to obtain the title compound (1.28 g).
ESI-MS (m/z): 613.19 $[M+H]^+$.

[Production Example 1-3]

2-Chloro-6-iodo-4-(1-phenyl-2-((tetrahydro-2H-pyran-2-yl)oxy)-1-(4-(trifluoromethyl)phenoxy)ethyl)quinazoline

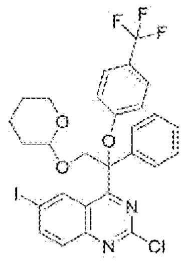

DIBAL-H(1 M toluene solution, 6.27 mL, 6.27 mmol) was added dropwise to a solution of the ethyl 2-(2-chloro-6-iodoquinazolin-4-yl)-2-phenyl-2-(4-(trifluoromethyl)phenoxy)acetate of Production Example 1-2 (1.28 g, 2.09 mmol) in toluene (20 mL) at -78°C under a nitrogen atmosphere, and the mixture was stirred for 2 hours at room temperature. A saturated aqueous Rochelle salt solution and ethyl acetate were added, and stirring was continued. After oil-water distribution of the reaction mixture, the organic layer was washed with brine and dried over magnesium sulfate. The organic layer was filtered, and the filtrate was then concentrated under reduced pressure to obtain a crude product. To a solution of the obtained crude product in dichloromethane (20 mL) there were added DHP (879 mg, 10.4 mmol) andp-toluenesulfonic acid monohydrate (199 mg, 1.04 mmol), and the mixture was stirred at room temperature for 3 days. The reaction mixture was purified by silica gel column chromatography (n-heptane to n-heptane:ethyl acetate = 7:3) to obtain the title compound (677 mg).
ESI-MS (m/z): 655.32 $[M+H]^+$.

[Production Example 1-4]

5-(2-Chloro-4-(1-phenyl-2-((tetrahydro-2H-pyran-2-yl)oxy)-1-(4-(trifluoromethyl)phenoxy)ethyl)quinazolin-6-yl)-1,3-dimethylpyridin-2(1H)-one

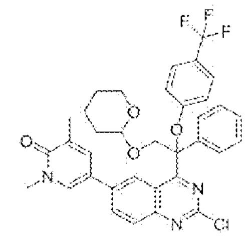

To a solution of the 2-chloro-6-iodo-4-(1-phenyl-2-((tetrahydro-2H-pyran-2-yl)oxy)-1-(4-(trifluoromethyl)phenoxy)ethyl)quinazoline of Production Example 1-3 (300 mg, 0.458 mmol) and 1,3-dimethyl-5-(4,4,5,5-tetramethyl-1,3,2-dioxaborolan-2-yl)pyridin-2(1H)-one (137 mg, 0.550 mmol) in toluene (4 mL) there were added ethanol (1 mL), sodium carbonate (2 M aqueous solution, 2.00 mL, 4.00 mmol) and tetrakis(triphenylphosphine)palladium(0) (26.5 mg, 0.023 mmol) at room temperature, and the mixture was stirred for 12 hours at 70°C. The reaction mixture was returned to room temperature, water was added, and extraction was performed with ethyl acetate. The organic layer was washed with brine. The organic layer was dried over magnesium sulfate and filtered. The filtrate was concentrated under reduced pressure and the residue was purified by silica gel column chromatography (n-heptane:ethyl acetate = 1:1 to ethyl acetate) to obtain the title compound (153 mg).

ESI-MS (m/z): 650.39 $[M+H]^+$.

[Example 2]

5-(2-Amino-4-(1-cyclopropoxy-2-hydroxy-1-phenylethyl)quinazolin-6-yl)-1,3-dimethylpyridin-2(1H)-one

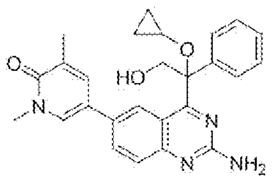

To a solution of the 5-(2-amino-4-(1-cyclopropoxy-1-phenyl-2-((tetrahydro-2H-pyran-2-yl)oxy)ethyl)quinazolin-6-yl)-1,3-dimethylpyridin-2(1H)-one of Production Example 2-5 (10 mg, 0.19 mmol) in methanol (0.384 mL) there was added *p*-toluenesulfonic acid monohydrate (5.42 mg, 0.28 mmol) at room temperature, and the mixture was stirred for 1 hour. Dichloromethane and an aqueous sodium hydrogencarbonate solution were added to the reaction mixture, and extraction was performed with dichloromethane. The organic layer was dried over sodium sulfate and filtered, and the filtrate was concentrated under reduced pressure. The residue was purified by NH silica gel thin-layer chromatography (ethyl acetate) to obtain the title compound (3.3 mg).

$^1$H-NMR Spectrum (400 MHz, $CDCl_3$) δ(ppm): 0.17-0.32 (1H, m), 0.32-0.43 (1H, m), 0.44-0.58 (1H, m), 0.64-0.79 (1H, m), 2.16 (3H, s), 2.93-3.05 (1H, m), 3.27-3.38 (1H, m), 3.54 (3H, s), 4.36-4.50 (1H, m), 4.54-4.62 (1H, m), 5.21 (2H, s), 6.89-6.94 (1H, m), 7.10-7.13 (1H, m), 7.26-7.38 (5H, m), 7.53-7.58 (1H, m), 7.58-7.62 (1H, m), 7.85-7.88 (1H, m).

[Production Example 2-1]

Ethyl 2-cyclopropoxy-2-phenylacetate

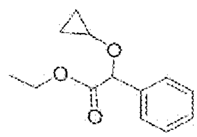

To a solution of cyclopropanol (3.36 g, 57.8 mmol) and rhodium(II) acetate dimer (320 mg, 0.723 mmol) in dichloromethane (20 mL) there was added dropwise a solution of ethyl 2-diazo-2-phenylacetate (5.50 g, 28.9 mmol) in dichloromethane (10 mL), and the mixture was stirred for 4 hours at room temperature. The reaction mixture was

concentrated under reduced pressure and the residue was purified by silica gel column chromatography (n-heptane to *n*-heptane:ethyl acetate = 4:1) to obtain the title compound (5.83 g).

$^1$H-NMR Spectrum (500 MHz, $CDCl_3$) $\delta$(ppm): 0.43-0.56 (2H, m), 0.64-0.78 (2H, m), 1.19-1.24 (3H, m), 3.38-3.45 (1H, m), 4.10-4.26 (2H, m), 4.94 (1H, s), 7.29-7.38 (3H, m), 7.42-7.47 (2H, m).

[Production Example 2-2]

Ethyl 2-(2-chloro-6-iodoquinazolin-4-yl)-2-cyclopropoxy-2-phenylacetate

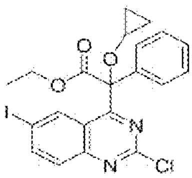

To a solution of the ethyl 2-cyclopropoxy-2-phenylacetate of Production Example 2-1 (661 mg, 3.00 mmol) and 2,4-dichloro-6-iodoquinazoline (1.17 g, 3.60 mmol) in THF (10 mL) there was added dropwise LHMDS (1.09 M THF solution, 3.30 mL, 3.60 mmol) at -78°C under a nitrogen atmosphere, and the mixture was stirred for 10 minutes at -78°C. The reaction mixture was then stirred for 1 hour at room temperature. Saturated aqueous ammonium chloride was added to the reaction mixture, and extraction was performed with ethyl acetate. The organic layer was washed with brine. The organic layer was dried over magnesium sulfate and filtered. The filtrate was concentrated under reduced pressure and the residue was purified by NH silica gel column chromatography (n-heptane to *n*-heptane:ethyl acetate = 7:3) to obtain the title compound (1.35 g).

ESI-MS (m/z): 509.25 $[M+H]^+$.

[Production Example 2-3]

2-Chloro-4-(1-cyclopropoxy-1-phenyl-2-((tetrahydro-2H-pyran-2-yl)oxy)ethyl)-6-iodoquinazoline

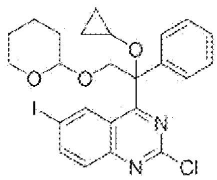

To a solution of the ethyl 2-(2-chloro-6-iodoquinazolin-4-yl)-2-cyclopropoxy-2-phenylacetate of Production Example 2-2 (1.35 g, 2.65 mmol) in toluene (15 mL) there was added dropwise DIBAL-H (1 M toluene solution, 5.84 mL, 5.84 mmol) at -78°C, and the mixture was stirred for 1 hour at 0°C. A saturated aqueous Rochelle salt solution and ethyl acetate were added, and after continuing stirring for 3 hours, the mixture was extracted with ethyl acetate. The organic layer was washed with brine and dried over sodium sulfate. The organic layer was filtered, and the filtrate was then concentrated under reduced pressure to obtain a crude product. To a solution of the obtained crude product in dichloromethane (10 mL) there were added DHP (720 μL, 7.96 mmol) and (1S)-(+)-10-camphorsulfonic acid (123 mg, 0.531 mmol) at room temperature, and the mixture was stirred for 1.5 hours at room temperature. A saturated aqueous sodium hydrogencarbonate solution was added to the reaction mixture to halt the reaction, and extraction was performed with ethyl acetate. After washing the organic layer with brine, it was dried over sodium sulfate and filtered, and the filtrate was concentrated under reduced pressure. The residue was purified by NH silica gel column chromatography (*n*-heptane:ethyl acetate = 95:5 to 7:3) to obtain the title compound (1.67 g).

ESI-MS (m/z): 551.25 $[M+H]^+$.

[Production Example 2-4]

5-(2-Chloro-4-(1-cyclopropoxy-1-phenyl-2-((tetrahydro-2H-pyran-2-yl)oxy)ethyl)quinazolin-6-yl)-1,3-dimethylpyridin-2(1H)-one

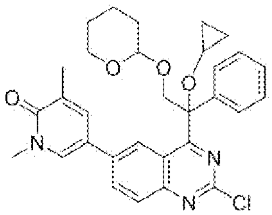

To a solution of the 2-chloro-4-(1-cyclopropoxy-1-phenyl-2-((tetrahydro-2H-pyran-2-yl)oxy)ethyl)-6-iodoquinazoline of Production Example 2-3 (846 mg, 1.54 mmol) and 1,3-dimethyl-5-(4,4,5,5-tetramethyl-1,3,2-dioxaborolan-2-yl)pyridin-2(1H)-one (421 mg, 1.69 mmol) in toluene (5 mL) there were added ethanol (1.25 mL), sodium carbonate (2 M aqueous solution, 2.50 mL, 5.00 mmol) and tetrakis(triphenylphosphine)palladium(0) (177 mg, 0.154 mmol) at room temperature, and the mixture was stirred for 18 hours at 70°C. The reaction mixture was filtered with Celite and washed with ethyl acetate. The filtrate was concentrated under reduced pressure and the residue was purified by NH silica gel column chromatography (*n*-heptane:ethyl acetate = 1:1 to ethyl acetate) to obtain the title compound (659 mg).
ESI-MS (m/z): 546.38 $[M+H]^+$

[Production Example 2-5]

5-(2-Amino-4-(1-cyclopropoxy-1-phenyl-2-((tetrahydro-2H-pyran-2-yl)oxy)ethyl)quinazolin-6-yl)-1,3-dimethylpyridin-2(1H)-one

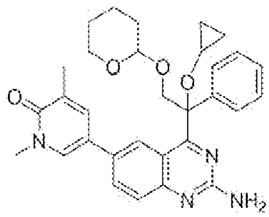

To a solution of the 5-(2-chloro-4-(1-cyclopropoxy-1-phenyl-2-((tetrahydro-2H-pyran-2-yl)oxy)ethyl)quinazolin-6-yl)-1,3-dimethylpyridin-2(1H)-one of Production Example 2-4 (50 mg, 0.092 mmol) in NMP (0.5 mL) there was added a 28% ammonia water solution (0.5 mL) at room temperature, and the mixture was stirred for 21 hours at 90°C in a sealed tube. The reaction mixture was returned to room temperature, water was added, and extraction was performed with ethyl acetate. The organic layer was washed with brine, dried over sodium sulfate and filtered. The filtrate was concentrated under reduced pressure and the residue was purified by NH silica gel column chromatography (n-heptane:ethyl acetate = 1:1 to ethyl acetate) to obtain the title compound (23.0 mg).

ESI-MS (m/z): 527.82 $[M+H]^+$.

[Example 3]

5-(4-(1-Cyclopropyl-3-hydroxy-2-phenylpropan-2-yl)-2-((dimethyl(oxo)-16-sulfanylidene)amino)quinazolin-6-yl)-1,3-dimethylpyridin-2(1H)-one

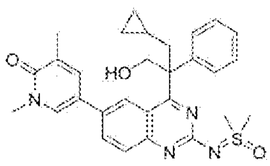

To a solution of the 5-(2-chloro-4-(1-cyclopropyl-2-phenyl-3-((tetrahydro-2H-pyran-2-yl)oxy)propan-2-yl)quinazolin-6-yl)-1,3-dimethylpyridin-2(1H)-one of Production Example 3-4 (20 mg, 0.037 mmol) in 1,4-dioxane (1 mL) there were added dimethylsulfoximine (6.85 mg, 0.074 mmol), cesium carbonate (29.9 mg, 0.092 mmol), 4,5-bis(diphenylphosphino)-9,9-dimethylxanthine (6.38 mg, 0.011 mmol) and tris(dibenzylideneacetone)dipalladium(0) (10.1 mg, 0.011 mmol) at room temperature, and the mixture was stirred for 3 hours at 100°C. The reaction mixture was returned to room temperature, and then water was added and extraction was performed with ethyl acetate. The organic layer was washed with brine and dried over magnesium sulfate. The organic layer was filtered, and the filtrate was then concentrated under reduced pressure. The obtained residue was purified by NH silica gel column chromatography (n-heptane:ethyl acetate = 1:1 to ethyl acetate to ethyl acetate:methanol = 7:3) to obtain a crude product. To a solution of the obtained crude product in methanol (1 mL) there was added *p*-toluenesulfonic acid monohydrate (3.50 mg, 0.018 mmol) at room temperature, and the mixture was stirred for 12 hours at room temperature. After adding a saturated aqueous sodium hydrogencarbonate solution to the reaction mixture and stirring for 15 minutes, the mixture was concentrated under reduced pressure. The obtained residue was purified by NH silica gel column chromatography (ethyl acetate to ethyl acetate:methanol = 7:3) to obtain the title compound (4.38 mg). ESI-MS (m/z): 517.34 $[M+H]^+$.

[Production Example 3-1]

Ethyl 3-cyclopropyl-2-phenylpropanoate

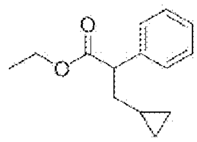

To a solution of (bromomethyl)cyclopropane (1.07 mL, 11.0 mmol) and ethyl 2-phenylacetate (1.64 g, 9.99 mmol) in DMF (39 mL) there was added 50% to 72% sodium hydride oil (440 mg) at 0°C, and the mixture was stirred for 1 hour at room temperature. Saturated aqueous ammonium chloride was added to the reaction mixture, and extraction was performed with ethyl acetate. The organic layer was washed with water and brine. The organic layer was dried over sodium sulfate and filtered, and the filtrate was concentrated under reduced pressure. The residue was purified by silica gel column chromatography (n-heptane:ethyl acetate = 97:3 to 9:1) to obtain the title compound (1.37 g). $^1$H-NMR Spectrum (400 MHz, $CDCl_3$) δ(ppm): -0.04-0.15 (2H, m), 0.28-0.47 (2H, m), 0.53-0.70 (1H, m), 1.16-1.24 (3H, m), 1.66-1.80 (1H, m), 1.83-1.95 (1H, m), 3.53-3.70 (1H, m), 4.01-4.23 (2H, m), 7.22-7.40 (5H, m).

[Production Example 3-2]

Ethyl 2-(2-chloro-6-iodoquinazolin-4-yl)-3-cyclopropyl-2-phenylpropanoate

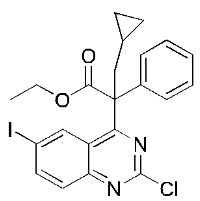

To a solution of 2,4-dichloro-6-iodoquinazoline (744 mg, 2.29 mmol) in THF (18 mL) there was added the ethyl 3-cyclopropyl-2-phenyl propanoate of Production Example 3-1 (500 mg, 2.29 mmol) at -78°C. LHMDS (1 M THF solution, 3.44 mL, 3.44 mmol) was added dropwise to the reaction mixture, after which it was stirred for 10 minutes at -78°C. The

reaction mixture was gradually increased in temperature and then stirred for 2 hours at room temperature. A saturated aqueous ammonium chloride was added to the reaction mixture, and the resulting mixture was stirred for 5 minutes, after which ethyl acetate and water were added. After oil-water distribution, the aqueous layer was extracted with ethyl acetate. The organic layer was dried over magnesium sulfate and filtered, and the filtrate was concentrated under reduced pressure. The residue was purified by silica gel column chromatography (n-heptane: ethyl acetate = 20:1 to 4:1) to obtain the title compound (892 mg).

$^1$H-NMR Spectrum (400 MHz, $CDCl_3$) δ(ppm): -0.24--0.06 (2H, m), 0.22-0.36 (2H, m), 0.83-0.95 (1H, m), 0.99-1.12 (3H, m), 2.41-2.56 (1H, m), 2.56-2.68 (1H, m), 3.99-4.27 (2H, m), 7.13-7.39 (3H, m), 7.42-7.60 (2H, m), 7.60-7.69 (1H, m), 7.89-8.03 (2H, m).

[Production Example 3-3]

2-Chloro-4-(1-cyclopropyl-2-phenyl-3-((tetrahydro-2H-pyran-2-yl)oxy)propan-2-yl)-6-iodoquinazoline

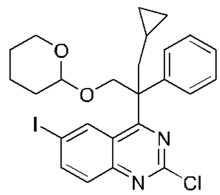

To a solution of the ethyl 2-(2-chloro-6-iodoquinazolin-4-yl)-3-cyclopropyl-2-phenyl propanoate of Production Example 3-2 (892 mg, 1.76 mmol) in toluene (12 mL) there was added DIBAL-H (1 M, THF solution, 5.28 ml, 5.28 mmol) at -78°C. The mixture was increased in temperature to 0°C and stirred for 10 minutes. A saturated aqueous Rochelle salt solution was added, and the mixture was stirred. After adding ethyl acetate and water, and oil-water distribution, the organic layer was dried over sodium sulfate and filtered, and then the filtrate was concentrated under reduced pressure to obtain a reaction intermediate. To a solution of the obtained reaction intermediate in dichloromethane (17.6 mL) there were added DHP (740 mg, 8.80 mmol) and (1S)-(+)-10-camphorsulfonic acid (82.0 mg, 0.352 mmol) at room temperature, and the mixture was stirred for 10 minutes at room temperature. A saturated aqueous sodium hydrogencarbonate solution was added to the reaction mixture, and extraction was performed with dichloromethane. The organic layer was dried over sodium sulfate and filtered, and the filtrate was concentrated under reduced pressure. The residue was purified by silica gel column chromatography (n-heptane:ethyl acetate = 19:1 to 4:1) to obtain the title compound (714 mg).
$^1$H-NMR Spectrum (400 MHz, $CDCl_3$) δ(ppm): -0.51--0.36 (2H, m), -0.04-0.17 (4H, m), 0.22-0.44 (4H, m), 1.00-1.50 (6H, m), 1.70-1.92 (3H, m), 2.13-2.30 (2H, m), 2.55-2.67 (2H, m), 2.71-2.82 (1H, m), 3.12-3.21 (1H, m), 3.32-3.45 (2H, m), 3.47-3.56 (1H, m), 3.83-3.91 (1H, m), 4.08-4.13 (1H, m), 4.25-4.33 (2H, m), 4.47-4.53 (1H, m), 4.66-4.71 (1H, m), 4.81-4.86 (1H, m), 4.91-4.96 (1H, m), 7.13-7.19 (4H, m), 7.26-7.33 (6H, m), 7.56-7.64 (2H, m), 7.79-7.86 (2H, m), 7.87-7.92 (2H, m). (diastereomixture)

[Production Example 3-4]

5-(2-Chloro-4-(1-cyclopropyl-2-phenyl-3-((tetrahydro-2H-pyran-2-yl)oxy)propan-2-yl)quinazolin-6-yl)-1,3-dimethylpyridin-2(1H)-one

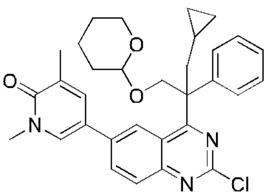

To a solution of the 2-chloro-4-(1-cyclopropyl-2-phenyl-3-((tetrahydro-2H-pyran-2-yl)oxy)propan-2-yl)-6-iodoquinazoline of Production Example 3-3 (400 mg, 0.729 mmol) and 1,3-dimethyl-5-(4,4,5,5-tetramethyl-1,3,2-dioxaborolan-2-yl)pyridin-2(1H)-one (218 mg, 0.875 mmol) in toluene (7.2 mL) and ethanol (1.8 mL) there were added sodium

carbonate (0.5 M aqueous solution, 3.64 mL, 1.82 mmol) and tetrakis(triphenylphosphine)palladium(0) (84.0 mg, 0.073 mmol) at room temperature, and the mixture was stirred for 16 hours at 80°C under a nitrogen atmosphere. The reaction mixture was then stirred for 6 hours at 90°C. Water was added to the reaction mixture, and extraction was performed with ethyl acetate. The organic layer was dried over sodium sulfate and filtered, and the filtrate was concentrated under reduced pressure. The residue was purified by silica gel column chromatography (n-heptane:ethyl acetate = 3:7 to 1:9) to obtain the title compound (200 mg).

$^1$H-NMR Spectrum (400 MHz, $CDCl_3$) $\delta$(ppm): -0.54--0.44 (2H, m), 0.01-0.14 (4H, m), 0.28-0.48 (4H, m), 1.00-1.50(104 m), 2.15 (6H, s), 2.17-2.32 (4H, m), 2.65-2.75 (2H, m), 2.75-2.86 (1H, m), 3.10-3.21 (1H, m), 3.28-3.42 (2H, m), 3.53 (6H, s), 4.14-4.21 (1H, m), 4.30-4.34 (1H, m), 4.34-4.39 (1H, m), 4.49-4.59 (1H, m), 4.65-4.72 (1H, m), 4.80-4.91 (1H, m), 6.77-6.83 (2H, m), 6.90-6.98 (2H, m), 7.28-7.45(12H, m), 7.72-7.78 (2H, m), 7.88-7.95 (2H, m).(diastereomixture)

[Example 4]

N-(6-(5-Methoxy-1-methyl-6-oxo-1,6-dihydroxypyridin-3-yl)-4-(2-(pyridin-2-yl)propan-2-yl)quinazolin-2-yl)methanesulfonamide

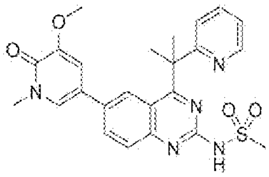

To a solution of the 5-(2-chloro-4-(1-(pyridin-2-yl)ethyl)quinazolin-6-yl)-3-methoxy-1-methylpyridin-2(1H)-one of Production Example 4-6 (27 mg, 0.066 mmol) and iodomethane (28.3 mg, 0.199 mmol) in THF (1 mL) there was added potassium *tert*-butoxide (14.9 mg, 0.133 mmol) at -78°C under a nitrogen atmosphere, and the mixture was stirred for 12 hours at room temperature. Water was added to the reaction mixture, and extraction was performed with ethyl acetate. The organic layer was washed with brine and dried over magnesium sulfate. The organic layer was filtered, and the filtrate was then concentrated under reduced pressure. To a solution of part of the obtained reaction intermediate (13.0 mg, 0.031 mmol) in 1,4-dioxane (1 mL) there were added methanesulfonamide (5.88 mg, 0.062 mmol), cesium carbonate (25.2 mg, 0.077 mmol), 4,5-bis(diphenylphosphino)-9,9-dimethylxanthine (5.36 mg, 9.3 μmol) and tris(dibenzylideneacetone) dipalladium(0) (8.49 mg, 9.3 μmol) at room temperature, and the mixture was stirred for 12 hours at 100°C. The reaction mixture was returned to room temperature, a saturated aqueous citric acid solution was added, and then the mixture was extracted with ethyl acetate. The organic layer was washed with brine and dried over magnesium sulfate. The organic layer was filtered, and the filtrate was then concentrated under reduced pressure. The obtained residue was purified by silica gel column chromatography (n-heptane:ethyl acetate = 1:1 to ethanol acetate to methanol:ethyl acetate = 3:7) to obtain the title compound (2.00 mg).

ESI-MS (m/z): 480.21 $[M+H]^+$.

[Production Example 4-1]

4-Methoxybenzyl 2-(pyridin-2-yl)propanoate

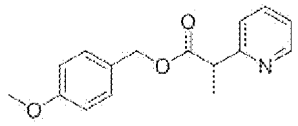

To a solution of 2-(pyridin-2-yl)propanoate (970 mg, 6.42 mmol) and potassium carbonate (2.66 g, 19.3 mmol) in DMF (30 mL) there was added 4-methoxybenzyl chloride (1.51 g, 9.63 mmol), and the mixture was stirred for 2 days at 80°C. Water was added to the reaction mixture, and extraction was performed with ethyl acetate. The organic layer was washed with brine. The organic layer was dried over magnesium sulfate and filtered. The filtrate was concentrated under reduced pressure and the residue was purified by silica gel column chromatography (n-heptane to n-heptane:ethyl acetate = 7:3) to obtain the title compound (1.60 g).

ESI-MS (m/z): 272.04 $[M+H]^+$.

[Production Example 4-2]

4-Methoxybenzyl 2-(2-chloro-6-iodoquinazolin-4-yl)-2-(pyridin-2-yl)propanoate

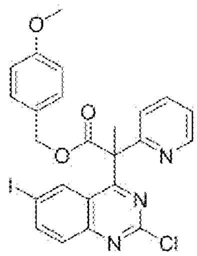

To a solution of the 4-methoxybenzyl 2-(pyridin-2-yl)propanoate of 4-1 (1.60 g, 3.24 mmol) and 2,4-dichloro-6-iodoquinazoline (1.58 g, 4.87 mmol) in THF (30 mL) there was added dropwise LHMDS (1 M THF solution, 4.87 mL, 4.87 mmol) at -78°C under a nitrogen atmosphere, and the mixture was stirred for 3 hours at room temperature. Saturated aqueous ammonium chloride was added to the reaction mixture, and extraction was performed with ethyl acetate. The organic layer was washed with brine. The organic layer was dried over magnesium sulfate and filtered. The filtrate was concentrated under reduced pressure and the residue was purified by silica gel column chromatography (n-heptane to *n*-heptane:ethyl acetate = 4:1) to obtain the title compound (710 mg).

$^1$H-NMR Spectrum (500 MHz, $CDCl_3$) $\delta$(ppm): 2.18 (3H, s), 3.78 (3H, s), 5.01 (1H, d, J=10.0 Hz), 5.21 (1H, d, J=10.0 Hz), 6.73-6.78 (2H, m), 7.01-7.06 (2H, m), 7.17-7.21 (1H, m), 7.49-7.53 (1H, m), 7.59-7.64 (1H, m), 7.64-7.70 (2H, m), 7.88-7.93 (1H, m), 8.38-8.43 (1H, m).

[Production Example 4-3]

2-Chloro-6-iodo-4-(1-(pyridin-2-yl)ethyl)quinazoline

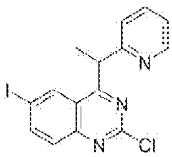

To the 4-methoxybenzyl 2-(2-chloro-6-iodoquinazolin-4-yl)-2-(pyridin-2-yl)propanoate of Production Example 4-2 (710.0 mg, 1.27 mmol) there was added hydrochloric acid (4 M ethyl acetate solution, 10.0 mL, 40.0 mmol) at room temperature, and the mixture was stirred for 12 hours. A saturated aqueous sodium hydrogencarbonate solution was added to the reaction mixture, and extraction was performed with ethyl acetate. The organic layer was washed with brine, dried over magnesium sulfate, and filtered. The filtrate was concentrated under reduced pressure and the residue was purified by silica gel column chromatography (n-heptane to n-heptane:ethyl acetate = 7:3) to obtain the title compound (263 mg).

ESI-MS (m/z): 396.03 $[M+H]^+$.

[Production Example 4-4]

5-Bromo-3-methoxy-1-methylpyridin-2(1H)-one

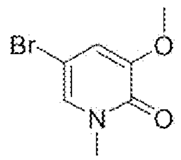

To a solution of 5-bromo-2,3-dimethoxypyridine (207 mg, 949 μmol) in acetonitrile (1 mL) there was added iodomethane (18 μL, 285 μmol) at room temperature, and the mixture was stirred for 24 hours at 150°C. The reaction mixture was concentrated under reduced pressure and the residue was purified by silica gel column chromatography (n-heptane:ethyl acetate = 1:1 to ethyl acetate) to obtain the title compound (155 mg).

$^1$H-NMR Spectrum (500 MHz, $CDCl_3$) δ(ppm): 3.55 (3H, s), 3.82 (3H, s), 6.65 (1H, d, J=2.4 Hz), 7.05 (1H, d, J=2.4 Hz).

[Production Example 4-5]

3-Methoxy-1-methyl-5-(4,4,5,5-tetramethyl-1,3,2-dioxaborolan-2-yl)pyridin-2(1H)-one

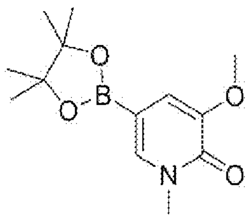

To a solution of the 5-bromo-3-methoxy-1-methylpyridin-2(1H)-one of Production Example 4-4 (155 mg, 0.711 mmol), bis(pinacolato)diboron (361 mg, 1.42 mmol) and potassium acetate (140 mg, 1.42 mmol) in 1,4-dioxane (10 mL) there was added 1,1'-bis(diphenylphosphino)ferrocenepalladium(II) dichloride and dichloromethane (29 mg, 36 μmol), and the mixture was stirred for 24 hours at 100°C. It was then concentrated under reduced pressure. The residue was purified by silica gel column chromatography (n-heptane:ethyl acetate = 1: 1 to ethyl acetate) to obtain the title compound (54 mg). $^1$H-NMR Spectrum (400 MHz, $CDCl_3$) δ(ppm): 1.28 (12H, s), 3.60 (3H, s), 3.85 (3H, s), 6.87 (1H, d, J=1.56 Hz), 7.42 (1H, d, J=1.56 Hz).

[Production Example 4-6]

5-(2-Chloro-4-(1-(pyridin-2-yl)ethyl)quinazolin-6-yl)-3-methoxy-1-methylpyridin-2(1H)-one

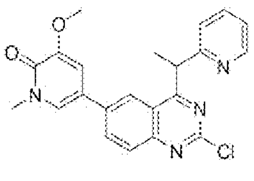

To a solution of the 2-chloro-6-iodo-4-(1-(pyridin-2-yl)ethyl)quinazoline of Production Example 4-3 (50.0 mg, 0.126 mmol) and the 3-methoxy-1-methyl-5-(4,4,5,5-tetramethyl-1,3,2-dioxaborolan-2-yl)pyridin-2(1H)-one of Production Example 4-5 (40.2 mg, 0.152 mmol) in toluene (0.8 mL) there was added ethanol (0.2 mL), sodium carbonate (2 M aqueous solution, 0.40 mL, 0.80 mmol) and tetrakis(triphenylphosphine)palladium(0) (7.30 mg, 6.32 μmol) at room temperature, and the mixture was stirred for 12 hours at 70°C. The reaction mixture was returned to room temperature, water was added, and extraction was performed with ethyl acetate. The organic layer was washed with brine. The organic layer was dried over magnesium sulfate and filtered. The filtrate was concentrated under reduced pressure and the residue was purified by silica gel column chromatography (n-heptane:ethyl acetate = 1: 1 to ethyl acetate) to obtain the title compound (27.0 mg). ESI-MS (m/z): 407.16 $[M+H]^+$.

[Example 5]

N-(4-(1-Cyclopropyl-3-hydroxy-2-(pyridin-2-yl)propan-2-yl)-6-(1,5-dimethyl-6-oxo-1,6-dihydropyridin-3-yl)quinazolin-2-yl)methanesulfonamide

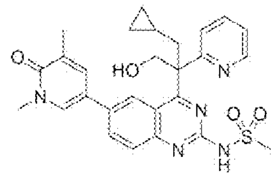

To a solution of the 5-(2-chloro-4-(1-cyclopropyl-2-(pyridin-2-yl)-3-((tetrahydro-2H-pyran-2-yl)oxy)propan-2-yl)quinazolin-6-yl)-1,3-dimethylpyridin-2(1H)-one of Production Example 5-4 (15.0 mg, 0.028 mmol) in 1,4-dioxane (1 mL) there were added methanesulfonamide (5.24 mg, 0.055 mmol), cesium carbonate (22.4 mg, 0.069 mmol), 4,5-bis(diphenylphosphino)-9,9-dimethylxanthine (4.78 mg, 8.26 μmol) and tris(dibenzylideneacetone)dipalladium(0) (7.56 mg, 8.26 μmol) at room temperature, and the mixture was stirred for 12 hours at 100°C. The reaction mixture was returned to room temperature, and then a saturated aqueous citric acid solution was added and the mixture was extracted with ethyl acetate. The organic layer was washed with brine and dried over magnesium sulfate. The organic layer was filtered, and the filtrate was then concentrated under reduced pressure. The obtained residue was purified by silica gel column chromatography (n-heptane:ethyl acetate = 1:1 to ethyl acetate to ethyl acetate:methanol = 7:3) to obtain a reaction intermediate. To a solution of the reaction intermediate in methanol (1 mL) there was added *p*-toluenesulfonic acid monohydrate (2.62 mg, 14 μmol) at room temperature, and the mixture was stirred for 12 hours at room temperature. It was then concentrated under reduced pressure. The obtained residue was purified by silica gel column chromatography (ethyl acetate to ethyl acetate:methanol = 7:3) to obtain the title compound (6.52 mg).

ESI-MS (m/z): 520.32 $[M+H]^+$.

[Production Example 5-1]

Ethyl 3-cyclopropyl-2-(pyridin-2-yl)propanoate

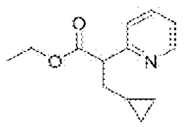

To a solution of ethyl 2-pyridylacetate (2.00 g, 12.1 mmol) in DMF (25 mL) there was added 50% to 72% sodium hydride oil (581 mg) at 0°C under a nitrogen atmosphere, and the mixture was stirred for 15 minutes at room temperature. Cyclopropylmethyl bromide (1.72 g, 12.7 mmol) was added and the mixture was stirred for 30 minutes. Water was added to the reaction mixture, which was then diluted with brine and extracted with ethyl acetate. The organic layer was washed with brine and the organic layer was dried over magnesium sulfate, and then filtered. The filtrate was concentrated under reduced pressure. The obtained residue was purified by NH silica gel column chromatography (n-heptane to *n*-heptane:ethyl acetate = 7:3) to obtain the title compound (2.39 g).

ESI-MS (m/z): 219.93 $[M+H]^+$.

[Production Example 5-2]

Ethyl 2-(2-chloro-6-iodoquinazolin-4-yl)-3-cyclopropyl-2-(pyridin-2-yl)propanoate

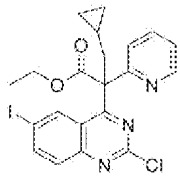

To a solution of the ethyl 3-cyclopropyl-2-(pyridin-2-yl)propanoate of Production Example 5-1 (2.39 g, 10.9 mmol) and 2,4-dichloro-6-iodoquinazoline (3.90 g, 12.0 mmol) in THF (20 mL) there was added dropwise LHMDS (1.09 M THF solution, 12.0 mL, 13.1 mmol) at - 78°C under a nitrogen atmosphere, and after stirring for 10 minutes, the mixture was stirred for 3 hours at room temperature. Saturated aqueous ammonium chloride was added to the reaction mixture, and extraction was performed with ethyl acetate. The organic layer was washed with brine. The organic layer was dried over sodium sulfate and filtered. The filtrate was concentrated under reduced pressure and the residue was purified by NH silica gel column chromatography (n-heptane to n-heptane:ethyl acetate = 7:3) to obtain the title compound (6.5 g).
ESI-MS (m/z): 508.29 $[M+H]^+$.

[Production Example 5-3]

2-(2-Chloro-6-iodoquinazolin-4-yl)-3-cyclopropyl-2-(pyridin-2-yl)propan-1-ol

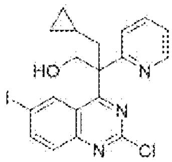

To a solution of the ethyl 2-(2-chloro-6-iodoquinazolin-4-yl)-3-cyclopropyl-2-(pyridin-2-yl)propanoate of Production Example 5-2 (5.59 g, 11.0 mmol) in toluene (20 mL) and THF (2 mL) there was added DIBAL-H (1 M toluene solution, 22.0 ml, 22.0 mmol) at -78°C. After stirring for 10 minutes, the temperature was increased to 0°C and the mixture was stirred for 10 minutes. A saturated aqueous Rochelle salt solution was added, and the mixture was stirred overnight at room temperature. After oil-water distribution, the organic layer was washed twice with brine and then dried over sodium sulfate and filtered. The filtrate was concentrated under reduced pressure, and then the residue was purified by NH silica gel column chromatography (n-heptane:ethyl acetate = 9:1 to ethyl acetate) to obtain the title compound (2.2 g).
ESI-MS (m/z): 466.18 $[M+H]^+$.

[Production Example 5-4]

5-(2-Chloro-4-(1-cyclopropyl-2-(pyridin-2-yl)-3-((tetrahydro-2H-pyran-2-yl)oxy)propan-2-yl)quinazolin-6-yl)-1,3-dimethylpyridin-2(1H)-one

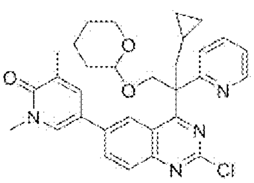

To a solution of the 2-(2-chloro-6-iodoquinazolin-4-yl)-3-cyclopropyl-2-(pyridin-2-yl)propan-1-ol of Production Example 5-3 (2.2 g, 4.72 mmol) and DHP (1.59 g, 18.9 mmol) in dichloromethane (30 mL) there was added (1S)-(+)-10-camphorsulfonic acid (1.21 g, 5.20 mmol), and the mixture was stirred overnight at room temperature. A saturated aqueous sodium hydrogencarbonate solution was added to the reaction mixture, and extraction was performed with ethyl acetate. The organic layer was washed with brine, dried over sodium sulfate and filtered. The filtrate was concentrated under reduced pressure to obtain a crude product. To a solution of the obtained crude product and 1,3-dimethyl-5-(4,4,5,5-tetramethyl-1,3,2-dioxaborolan-2-yl)pyridin-2(1H)-one (1.23 g, 4.94 mmol) in toluene (15 mL) there were added ethanol (3

mL), sodium carbonate (2 M aqueous solution, 7.5 mL, 15 mmol) and tetrakis(triphenylphosphine)palladium(0) (272 mg, 0.235 mmol) at room temperature, and the mixture was stirred for 18 hours at 70°C under a nitrogen atmosphere. The reaction mixture was filtered with ethyl acetate, the filtrate was concentrated under reduced pressure, and the residue was purified by NH silica gel column chromatography (n-heptane:ethyl acetate = 1:1 to ethyl acetate to ethyl acetate:methanol = 9:1) to obtain the title compound (1.25 g).

ESI-MS (m/z): 545.45 $[M+H]^+$.

[Example 6]

5-(2-((Dimethyl(oxo)-16-sulfanylidene)amino)-4-(1-hydroxy-2-phenyl-3-(tetrahydro-2H-pyran-4-yl)propan-2-yl)quinazolin-6-yl)-1,3-dimethylpyridin-2(1H)-one

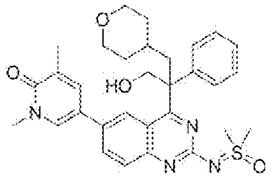

The title compound (7.40 mg) was obtained from the 5-(2-chloro-4-(2-phenyl-1-((tetrahydro-2H-pyran-2-yl)oxy)-3-(tetrahydro-2H-pyran-4-yl)propan-2-yl)quinazolin-6-yl)-1,3-dimethylpyridin-2(1H)-one of Production Example 6-3 (18.0 mg, 0.031 mmol), by the same method as Example 3.

ESI-MS (m/z): 561.35 $[M+H]^+$.

[Production Example 6-1]

Ethyl 2-(2-chloro-6-iodoquinazolin-4-yl)-2-phenyl-3-(tetrahydro-2H-pyran-4-yl)propanoate

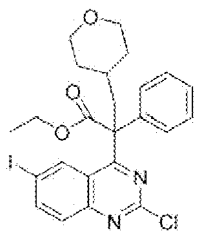

To a solution of 4-(iodomethyl)tetrahydro-2H-pyran (757 mg, 3.35 mmol) and ethyl phenylacetate (500 mg, 3.05 mmol) in DMF (10 mL) there was added 50% to 72% sodium hydride oil (219 mg) at 0°C under a nitrogen atmosphere, and the mixture was stirred for 30 minutes at room temperature. Water was added to the reaction mixture, and extraction was performed with ethyl acetate. The organic layer was washed with brine. The organic layer was dried over magnesium sulfate and filtered. The filtrate was concentrated under reduced pressure to obtain a crude product. To a solution of the obtained crude product and 2,4-dichloro-6-iodoquinazoline (989 mg, 3.05 mmol) in THF (20 mL) there was added dropwise LHMDS (1 M THF solution, 4.57 mL, 4.57 mmol) at -78°C under a nitrogen atmosphere, and the mixture was stirred for 2 hours at room temperature. Saturated aqueous ammonium chloride was added to the reaction mixture, and extraction was performed with ethyl acetate. The organic layer was washed with brine. The organic layer was dried over magnesium sulfate and filtered. The filtrate was concentrated under reduced pressure and the residue was purified by silica gel column chromatography (n-heptane to n-heptane:ethyl acetate = 9:1) to obtain the title compound (506 mg).

ESI-MS (m/z): 551.28 $[M+H]^+$.

[Production Example 6-2]

2-Chloro-6-iodo-4-(2-phenyl-1-((tetrahydro-2H-pyran-2-yl)oxy)-3-(tetrahydro-2H-pyran-4-yl)propan-2-yl)quinazoline

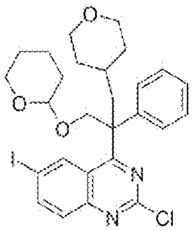

To a solution of the ethyl 2-(2-chloro-6-iodoquinazolin-4-yl)-2-phenyl-3-(tetrahydro-2H-pyran-4-yl)propanoate of Production Example 6-1 (506 mg, 0.919 mmol) in toluene (10 mL) there was added dropwise DIBAL-H (1 M toluene solution, 2.76 mL, 2.76 mmol) at -78°C under a nitrogen atmosphere, and the mixture was stirred for 2 hours at 0°C. A saturated aqueous Rochelle salt solution and ethyl acetate were added to the reaction mixture, and stirring was continued. The reaction mixture was extracted with ethyl acetate and the organic layer was washed with brine and then dried over magnesium sulfate. The organic layer was filtered, and the filtrate was then concentrated under reduced pressure to obtain a crude product. To a solution of the obtained crude product in dichloromethane (10 mL) there were added DHP (386 mg, 4.59 mmol) and *p*-toluenesulfonic acid monohydrate (87.0 mg, 0.459 mmol) at room temperature, and the mixture was stirred for 12 hours at room temperature. A saturated aqueous sodium hydrogencarbonate solution was added to the reaction mixture, and extraction was performed with ethyl acetate. The organic layer was washed with brine. The organic layer was dried over magnesium sulfate and filtered. The filtrate was concentrated under reduced pressure and the residue was purified by silica gel column chromatography (n-heptane to n-heptane:ethyl acetate = 7:3) to obtain the title compound (477 mg).

ESI-MS (m/z): 593.28 $[M+H]^+$.

[Production Example 6-3]

5-(2-Chloro-4-(2-phenyl-1-((tetrahydro-2H-pyran-2-yl)oxy)-3-(tetrahydro-2H-pyran-4-yl)propan-2-yl)quinazolin-6-yl)-1,3-dimethylpyridin-2(1H)-one

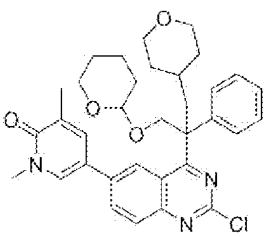

To a solution of the 2-chloro-6-iodo-4-(2-phenyl-1-((tetrahydro-2H-pyran-2-yl)oxy)-3-(tetrahydro-2H-pyran-4-yl)propan-2-yl)quinazoline of Production Example 6-2 (150 mg, 0.253 mmol) and 1,3-dimethyl-5-(4,4,5,5-tetramethyl-1,3,2-dioxaborolan-2-yl)pyridin-2(1H)-one (118 mg, 0.304 mmol) in toluene (2 mL) there were added ethanol (0.5 mL), sodium carbonate (2 M aqueous solution, 1.00 mL, 2.00 mmol) and tetrakis(triphenylphosphine)palladium(0) (14.6 mg, 0.013 mmol), and the mixture was stirred for 12 hours at 70°C. The reaction mixture was returned to room temperature, water was added, and extraction was performed with ethyl acetate. The organic layer was washed with brine. The organic layer was dried over magnesium sulfate and filtered. The filtrate was concentrated under reduced pressure, and the residue was purified by silica gel column chromatography (n-heptane:ethyl acetate = 1:1 to ethyl acetate to ethyl acetate:methanol = 9:1) to obtain the title compound (37.0 mg).

ESI-MS (m/z): 588.41 $[M+H]^+$.

[Example 7]

5-(2-((Dimethyl(oxo)-16-sulfanylidene)amino)-4-(1-hydroxy-4-methyl-2-phenylpentan-2-yl)quinazolin-6-yl)-1,3-dimethylpyridin-2(1H)-one

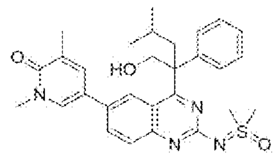

The title compound (4.56 mg) was obtained from the 5-(2-chloro-4-(4-methyl-2-phenyl-1-((tetrahydro-2H-pyran-2-yl)oxy)pentan-2-yl)quinazolin-6-yl)-1,3-dimethylpyridin-2(1H)-one of Production Example 7-3 (15.0 mg, 0.027 mmol), by the same method as Example 3.
ESI-MS (m/z): 519.42 $[M+H]^+$.

[Production Example 7-1]

Ethyl 2-(2-chloro-6-iodoquinazolin-4-yl)-4-methyl-2-phenylpentanoate

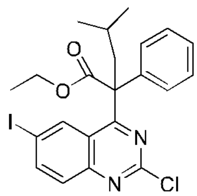

The title compound (740 mg) was obtained from 1-iodo-2-methylpropane (616 mg, 3.35 mmol) and ethyl phenylacetate (500 mg, 3.05 mmol) by the same method as Production Example 6-1.
ESI-MS (m/z): 509.18 $[M+H]^+$.

[Production Example 7-2]

2-Chloro-6-iodo-4-(4-methyl-2-phenyl-1-((tetrahydro-2H-pyran-2-yl)oxy)pentan-2-yl)quinazoline

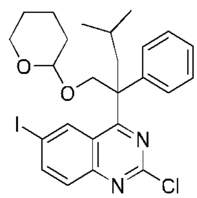

To a solution of the ethyl 2-(2-chloro-6-iodoquinazolin-4-yl)-4-methyl-2-phenylpentanoate of Production Example 7-1 (740 mg, 1.45 mmol) in toluene (10 mL) there was added dropwise DIBAL-H (1 M toluene solution, 4.36 mL, 4.36 mmol) at -78°C under a nitrogen atmosphere, and the mixture was stirred for 2 hours at 0°C. A saturated aqueous Rochelle salt solution and ethyl acetate were added, and stirring was continued. The organic layer was washed with brine and the organic layer was dried over magnesium sulfate. The organic layer was filtered, and the filtrate was then concentrated under reduced pressure to obtain a crude product. To a solution of the obtained crude product in dichloromethane (10 mL) there were added DHP (612 mg, 7.27 mmol) and *p*-toluenesulfonic acid monohydrate (138 mg, 0.727 mmol) at room temperature, and the mixture was stirred for 12 hours at room temperature. A saturated aqueous sodium hydrogencarbonate solution was added to the reaction mixture, and extraction was performed with ethyl acetate. The organic layer was washed with brine. The organic layer was dried over magnesium sulfate and filtered. The filtrate was concentrated under reduced pressure and the residue was purified by silica gel column chromatography (n-heptane to n-heptane:ethyl acetate = 7:3) to obtain the title compound (704 mg).
ESI-MS (m/z): 551.21 $[M+H]^+$.

[Production Example 7-3]

5-(2-Chloro-4-(4-methyl-2-phenyl-1-((tetrahydro-2H-pyran-2-yl)oxy)pentan-2-yl)quinazolin-6-yl)-1,3-dimethylpyridin-2(1H)-one

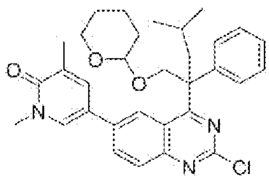

To a solution of the 2-chloro-6-iodo-4-(4-methyl-2-phenyl-1-((tetrahydro-2H-pyran-2-yl)oxy)pentan-2-yl)quinazoline of Production Example 7-2 (150 mg, 0.272 mmol) and 1,3-dimethyl-5-(4,4,5,5-tetramethyl-1,3,2-dioxaborolan-2-yl) pyridin-2(1H)-one (127 mg, 0.327 mmol) in toluene (2 mL) there were added ethanol (0.5 mL), sodium carbonate (2 M aqueous solution, 1.00 mL, 2.00 mmol) and tetrakis(triphenylphosphine)palladium(0) (15.7 mg, 0.014 mmol) at room temperature, and the mixture was stirred for 12 hours at 70°C. The reaction mixture was returned to room temperature, water was added, and extraction was performed with ethyl acetate. The organic layer was washed with brine. The organic layer was dried over magnesium sulfate and filtered. The filtrate was concentrated under reduced pressure, and the residue was purified by silica gel column chromatography (n-heptane:ethyl acetate = 1:1 to ethyl acetate to ethyl acetate:methanol = 9:1) to obtain the title compound (81.0 mg).
ESI-MS (m/z): 546.38 $[M+H]^+$.

[Example 8]

N-(4-(1-Cyclobutyl-3-hydroxy-2-phenylpropan-2-yl)-6-(1,5-dimethyl-6-oxo-1,6-dihydropyridin-3-yl)quinazolin-2-yl) methanesulfonamide

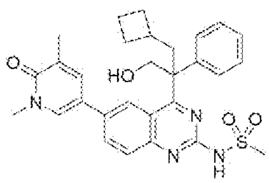

The title compound (12.2 mg) was obtained from the 5-(2-chloro-4-(1-cyclobutyl-2-phenyl-3-((tetrahydro-2H-pyran-2-yl)oxy)propan-2-yl)quinazolin-6-yl)-1,3-dimethoxypyridin-2(1H)-one of Production Example 8-3 (15.0 mg, 0.027 mmol) by the same method as Example 1.
ESI-MS (m/z): 533.35 $[M+H]^+$.

[Production Example 8-1]

Ethyl 2-(2-chloro-6-iodoquinazolin-4-yl)-3-cyclobutyl-2-phenyl propanoate

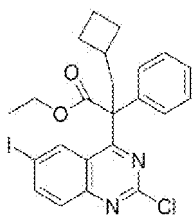

To a solution of (bromomethyl)cyclobutane (454 mg, 3.05 mmol) and ethyl phenylacetate (500 mg, 3.05 mmol) in DMF (20 mL) there was added 50% to 72% sodium hydride oil (219 mg) at 0°C under a nitrogen atmosphere, and the

mixture was stirred for 30 minutes at room temperature. Water was added to the reaction mixture, and extraction was performed with ethyl acetate. The organic layer was washed with brine. The organic layer was dried over magnesium sulfate and filtered. The filtrate was concentrated under reduced pressure to obtain a crude product. To a solution of the obtained crude product and 2,4-dichloro-6-iodoquinazoline (989 mg, 3.05 mmol) in THF (20 mL) there was added dropwise LHMDS (1 M THF solution, 4.57 mL, 4.57 mmol) at -78°C under a nitrogen atmosphere, and the mixture was stirred for 2 hours at room temperature. Saturated aqueous ammonium chloride was added to the reaction mixture, and extraction was performed with ethyl acetate. The organic layer was washed with brine. The organic layer was dried over magnesium sulfate and filtered. The filtrate was concentrated under reduced pressure and the residue was purified by silica gel column chromatography (n-heptane to n-heptane:ethyl acetate = 9:1) to obtain the title compound (1.13 g).
ESI-MS (m/z): 521.17 $[M+H]^+$.

[Production Example 8-2]

2-Chloro-4-(1-cyclobutyl-2-phenyl-3-((tetrahydro-2H-pyran-2-yl)oxy)propan-2-yl)-6-iodoquinazoline

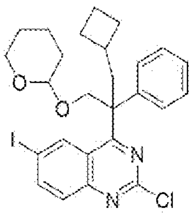

The title compound (565 mg) was obtained from the ethyl 2-(2-chloro-6-iodoquinazolin-4-yl)-3-cyclobutyl-2-phenyl propanoate of Production Example 8-1 (1.13 g, 2.17 mmol), by the same method as Production Example 1-3.
ESI-MS (m/z): 563.208 $[M+H]^+$.

[Production Example 8-3]

5-(2-Chloro-4-(1-cyclobutyl-2-phenyl-3-((tetrahydro-2H-pyran-2-yl)oxy)propan-2-yl)quinazolin-6-yl)-1,3-dimethylpyridin-2(1H)-one

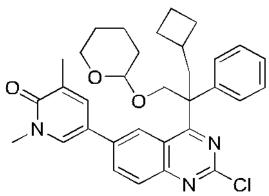

The title compound (125 mg) was obtained from the 2-chloro-4-(1-cyclobutyl-2-phenyl-3-((tetrahydro-2H-pyran-2-yl)oxy)propan-2-yl)-6-iodoquinazoline of Production Example 8-2 (150 mg, 0.266 mmol), by the same method as Production Example 1-4.
ESI-MS (m/z): 558.10 $[M+H]^+$.

[Example 9]

5-(1-(1-Cyclopropoxy-2-hydroxy-1-phenylethyl)-3-((dimethyl(oxo)-16-sulfanylidene)amino)isoquinolin-7-yl)-1,3-dimethylpyridin-2(1H)-one

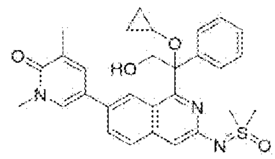

To a solution of the 5-(3-chloro-1-(1-cyclopropoxy-1-phenyl-2-((tetrahydro-2H-pyran-2-yl)oxy)ethyl)isoquinolin-7-yl)-1,3-dimethylpyridin-2(1H)-one of Production Example 9-3 (32.0 mg, 0.059 mmol) in 1,4-dioxane (1 mL) there were added dimethylsulfoximine (10.9 mg, 0.117 mmol), cesium carbonate (47.8 mg, 0.147 mmol), BRETTPHOS (9.45 mg, 0.018 mmol) and tris(dibenzylideneacetone)dipalladium(0) (16.1 mg, 0.018 mmol) at room temperature, and the mixture was stirred for 13 hours at 90°C. The reaction mixture was then returned to room temperature. Water was added to the reaction mixture, and extraction was performed with ethyl acetate. The organic layer was washed with brine. The organic layer was dried over magnesium sulfate and filtered. The filtrate was concentrated under reduced pressure and the residue was purified by NH silica gel column chromatography (n-heptane:ethyl acetate = 1:1 to ethyl acetate) to obtain a crude product. To a solution of the obtained crude product in methanol (1 mL) there was added *p*-toluenesulfonic acid monohydrate (5.58 mg, 0.029 mmol) at room temperature, and the mixture was stirred for 12 hours at room temperature. After adding saturated aqueous sodium hydrogencarbonate solution to the reaction mixture and stirring for 15 minutes, the mixture was concentrated under reduced pressure and the residue was purified by NH silica gel column chromatography (ethyl acetate to ethyl acetate:methanol = 9:1) to obtain the title compound (8.99 mg).
ESI-MS (m/z): 518.45 $[M+H]^+$.

[Production Example 9-1]

Ethyl 2-(7-bromo-3-chloroisoquinolin-1-yl)-2-cyclopropoxy-2-phenylacetate

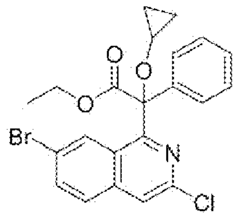

To a solution of the ethyl 2-cyclopropoxy-2-phenylacetate of Production Example 2-1 (477 mg, 2.17 mmol) and 7-bromo-1,3-dichloroisoquinoline (500 mg, 1.81 mmol) in THF (20 mL) there was added bis(tri-tert-butylphosphine) palladium(0) (46.1 mg, 0.090 mmol) under a nitrogen atmosphere. LHMDS (1 M THF solution, 4.15 mL, 4.15 mmol) was added dropwise at -78°C, and the mixture was stirred at room temperature for 3 days. Saturated aqueous ammonium chloride was added to the reaction mixture, and extraction was performed with ethyl acetate. The organic layer was dried over magnesium sulfate and filtered. The filtrate was concentrated under reduced pressure and the residue was purified by silica gel column chromatography (n-heptane to n-heptane:ethyl acetate = 9:1) to obtain the title compound (568 mg).
ESI-MS (m/z): 460.16 $[M+H]^+$.

[Production Example 9-2]

7-Bromo-3-chloro-1-(1-cyclopropoxy-1-phenyl-2-((tetrahydro-2H-pyran-2-yl)oxy)ethyl)isoquinoline

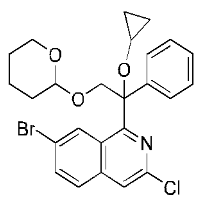

The title compound (352 mg) was obtained from the ethyl 2-(7-bromo-3-chloroisoquinolin-1-yl)-2-cyclopropoxy-2-phenylacetate of Production Example 9-1 (568 mg, 1.23 mmol), by the same method as Production Example 3-3.
ESI-MS (m/z): 502.16 [M+H]+.

[Production Example 9-3]

5-(3-Chloro-1-(1-cyclopropoxy-1-phenyl-2-((tetrahydro-2H-pyran-2-yl)oxy)ethyl)isoquinolin-7-yl)-1,3-dimethylpyridin-2(1H)-one

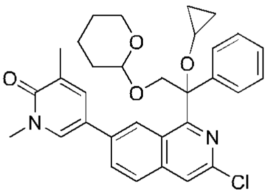

The title compound (32.0 mg) was obtained from the 7-bromo-3-chloro-1-(1-cyclopropoxy-1-phenyl-2-((tetrahydro-2H-pyran-2-yl)oxy)ethyl)isoquinoline of Production Example 9-2 (30.0 mg, 0.060 mmol), by the same method as Production Example 1-4.
ESI-MS (m/z): 545.45 [M+H]+.

[Example 10]

N-(4-(1-Cyclopropoxy-2-hydroxy-1-phenylethyl)-6-(1,5-dimethyl-6-oxo-1,6-dihydropyridin-3-yl)quinazolin-2-yl)-3-hydroxyazetidine-1-carboxamide

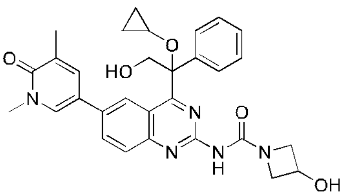

To a solution of the phenyl (4-(1-cyclopropoxy-1-phenyl-2-((tetrahydro-2H-pyran-2-yl)oxy)ethyl)-6-(1,5-dimethyl-6-oxo-1,6-dihydropyridin-3-yl)quinazolin-2-yl)carbamate of Production Example 10-1 (30.0 mg, 0.046 mmol) in DMF (2 mL) there were added 3-hydroxyazetidine hydrochloride (6.10 mg, 0.056 mmol) and triethylamine (14.1 mg, 0.139 mmol) at room temperature, and the mixture was stirred for 12 hours. Water was added to the reaction mixture, and extraction was performed with ethyl acetate. The organic layer was washed with brine. The organic layer was dried over magnesium sulfate and filtered. The filtrate was concentrated under reduced pressure to obtain a crude product. To a solution of the obtained crude product in methanol (2 mL) there was added *p*-toluenesulfonic acid monohydrate (4.41 mg, 0.023 mmol) at room temperature, and the mixture was stirred for 3 hours at room temperature. After adding a saturated aqueous sodium hydrogencarbonate solution to the reaction mixture and stirring for 30 minutes, the mixture was concentrated under reduced pressure. The obtained residue was purified by NH silica gel column chromatography (n-heptane:ethyl acetate = 1:1 to ethyl acetate to ethyl acetate:methanol = 4:1) to obtain the title compound (10.7 mg).
ESI-MS (m/z): 542.41 [M+H]+.

[Production Example 10-1]

Phenyl (4-(1-cyclopropoxy-1-phenyl-2-((tetrahydro-2H-pyran-2-yl)oxy)ethyl)-6-(1,5-dimethyl-6-oxo-1,6-dihydropyridin-3-yl)quinazolin-2-yl)carbamate

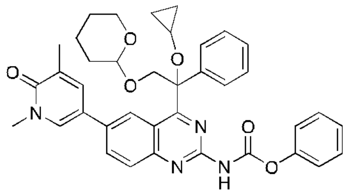

To a solution of the 5-(2-chloro-4-(1-cyclopropoxy-1-phenyl-2-((tetrahydro-2H-pyran-2-yl)oxy)ethyl)quinazolin-6-yl)-1,3-dimethylpyridin-2(1H)-one of Production Example 2-4 (100.0 mg, 0.183 mmol) in NMP (2 mL) there was added ammonia water (28 to 30% aqueous solution, 1.00 mL) at room temperature, and the mixture was stirred for 6 hours at 90°C in a shield tube. The reaction mixture was returned to room temperature, water was added, and extraction was performed with ethyl acetate. The organic layer was washed with water and brine. The organic layer was dried over magnesium sulfate and filtered. The filtrate was concentrated under reduced pressure to obtain a crude product. To a solution of the obtained crude product in THF (5 mL) there were added pyridine (29.0 mg, 0.366 mmol) and phenyl chloroformate (43.0 mg, 0.275 mmol) at room temperature, and the mixture was stirred for 30 minutes at room temperature. Water was added to the reaction mixture, and extraction was performed with ethyl acetate. The organic layer was washed with brine. The organic layer was dried over magnesium sulfate and filtered. The filtrate was concentrated under reduced pressure to obtain the title compound (92.0 mg).
ESI-MS (m/z): 647.59 $[M+H]^+$.

[Example 11]

7-(1,5-Dimethyl-6-oxo-1,6-dihydropyridin-3-yl)-1-(1-hydroxy-2-(pyridin-2-yl)pentan-2-yl)isoquinoline-3(2H)-one

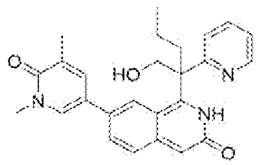

To a solution of the 5-(3-chloro-1-(2-(pyridin-2-yl)-1-((tetrahydro-2H-pyran-2-yl)oxy)pentan-2-yl)isoquinolin-7-yl)-1,3-dimethylpyridin-2(1H)-one of Production Example 11-3 (20.0 mg, 0.038 mmol) in 1,4-dioxane (1 mL) there were added water (0.5 mL), 2-di-*tert*-butylphosphino-2',4',6'-triisopropylbiphenyl (1.60 mg, 3.76 μmol) and tris(dibenzylideneacetone)dipalladium(0) (1.72 mg, 1.88 μmol) at room temperature, and the mixture was stirred for 3 hours at 100°C. The reaction mixture was then returned to room temperature. The reaction mixture was purified by silica gel column chromatography (n-heptane:ethyl acetate = 1:1 to ethyl acetate) to obtain a crude product. To a solution of the obtained crude product in methanol (1 mL) there was added *p*-toluenesulfonic acid monohydrate (3.57 mg, 0.019 mmol) at room temperature, and the mixture was stirred for 3 hours at room temperature. After adding a saturated aqueous sodium hydrogencarbonate solution to the reaction mixture and stirring for 30 minutes, the mixture was concentrated under reduced pressure. The obtained residue was purified by silica gel column chromatography (ethyl acetate to ethyl acetate:methanol = 4:1) to obtain the title compound (4.20 mg).
ESI-MS (m/z): 430.35 $[M+H]^+$.

[Production Example 11-1]

Ethyl 2-(7-bromo-3-chloroisoquinolin-1-yl)-2-(pyridin-2-yl) pentanoate

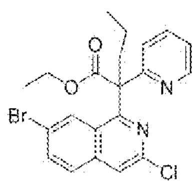

To a solution of ethyl 2-pyridylacetate (750 mg, 4.54 mmol) and 1-iodopropane (772 mg, 4.54 mmol) in DMF (20 mL) there was added 50% to 72% sodium hydride oil (327 mg) at 0°C, and the mixture was stirred for 30 minutes at room temperature. Water was added to the reaction mixture, and extraction was performed with ethyl acetate. The organic layer was washed with brine. The organic layer was dried over magnesium sulfate and filtered. The filtrate was concentrated under reduced pressure to obtain a crude product. To a solution of the obtained crude product and 7-bromo-1,3-dichloroisoquinoline (1.26 g, 4.54 mmol) in toluene (40 mL) there was added bis(tri-*tert*-butylphosphine)palladium(0) (116 mg, 0.227 mmol). LHMDS (1 M THF solution, 6.81 mL, 6.81 mmol) was added dropwise at - 78°C, and the mixture was stirred for 12 hours at room temperature. Water was added to the reaction mixture, and extraction was performed with ethyl acetate. The organic layer was washed with brine. The organic layer was dried over magnesium sulfate and filtered. The filtrate was concentrated under reduced pressure and the residue was purified by silica gel column chromatography (n-heptane to n-heptane:ethyl acetate = 4:1) to obtain the title compound (1.01 g).

ESI-MS (m/z): 449.19 $[M+H]^+$.

[Production Example 11-2]

2-(7-Bromo-3-chloroisoquinolin-1-yl)-2-(pyridin-2-yl)pentan-1-ol

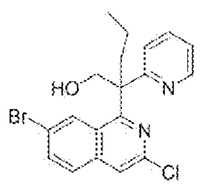

To a solution of the ethyl 2-(7-bromo-3-chloroisoquinolin-1-yl)-2-(pyridin-2-yl) pentanoate of Production Example 11-1 (1.01 g, 2.26 mmol) in toluene (20 mL) there was added dropwise DIBAL-H (1 M toluene solution, 6.77 mL, 6.77 mmol) at -78°C under a nitrogen atmosphere, and the mixture was stirred for 3 hours at 0°C. A saturated aqueous Rochelle salt solution and ethyl acetate were added to the reaction mixture, and stirring was continued for 3 hours. After oil-water distribution of the reaction mixture, the organic layer was washed with brine. The organic layer was then dried over magnesium sulfate and filtered. The filtrate was concentrated under reduced pressure and the residue was purified by silica gel column chromatography (n-heptane:ethyl acetate = 9:1 to 1:1) to obtain the title compound (385 mg).

ESI-MS (m/z): 407.12 $[M+H]^+$.

[Production Example 11-3]

5-(3-Chloro-1-(2-(pyridin-2-yl)-1-((tetrahydro-2H-pyran-2-yl)oxy)pentan-2-yl)isoquinolin-7-yl)-1,3-dimethylpyridin-2(1H)-one

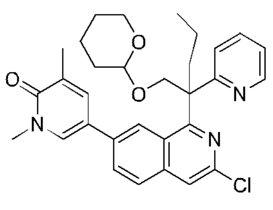

To a solution of the 2-(7-bromo-3-chloroisoquinolin-1-yl)-2-(pyridin-2-yl)pentan-1-ol of Production Example 11-2 (385 mg, 0.949 mmol) in dichloromethane (10 mL) there were added DHP (399 mg, 4.75 mmol) andp-toluenesulfonic acid monohydrate (90.0 mg, 0.474 mmol) at room temperature, and the mixture was stirred for 12 hours at room temperature. A saturated aqueous sodium hydrogencarbonate solution was added to the reaction mixture, and extraction was performed with ethyl acetate. The organic layer was washed with brine. The organic layer was dried over magnesium sulfate and filtered. The filtrate was concentrated under reduced pressure to obtain a crude product. To a solution of the obtained crude product and 1,3-dimethyl-5-(4,4,5,5-tetramethyl-1,3,2-dioxaborolan-2-yl)pyridin-2(1H)-one (284 mg, 1.14 mmol) in toluene (4 mL) there were added ethanol (1 mL), sodium carbonate (2 M aqueous solution, 2 mL, 4.00 mmol) and tetrakis(triphenylphosphine)palladium(0) (54.8 mg, 0.047 mmol) at room temperature, and the mixture was stirred for 3

days at 70°C. The reaction mixture was returned to room temperature, water was added, and extraction was performed with ethyl acetate. The organic layer was washed with brine. The organic layer was dried over magnesium sulfate and filtered. The filtrate was concentrated under reduced pressure and the residue was purified by silica gel column chromatography (n-heptane:ethyl acetate = 1:1 to ethyl acetate) to obtain the title compound (302 mg).

ESI-MS (m/z): 532.48 $[M+H]^+$.

[Example 12]

5-(2-Amino-4-(4-ethoxy-1-hydroxy-2-(pyridin-2-yl)butan-2-yl)quinazolin-6-yl)-1,3-dimethylpyridin-2(1H)-one

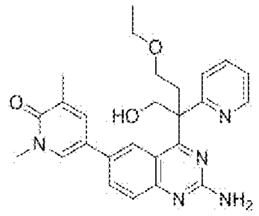

To a solution of the 5-(2-chloro-4-(4-ethoxy-2-(pyridin-2-yl)-1-((tetrahydro-2H-pyran-2-yl)oxy)butan-2-yl)quinazolin-6-yl)-1,3-dimethylpyridin-2(1H)-one of Production Example 12-3 (20.0 mg, 0.036 mmol) in NMP (1 mL) there was added ammonia (28 to 30% aqueous solution, 0.500 mL) at room temperature, and the mixture was stirred for 12 hours at 90°C in a shield tube. The reaction mixture was returned to room temperature, water was added, and extraction was performed with ethyl acetate. The organic layer was washed with brine. The organic layer was dried over magnesium sulfate and filtered. The filtrate was concentrated under reduced pressure to obtain a crude product. To a solution of the obtained crude product in methanol (1 mL) there was added *p*-toluenesulfonic acid monohydrate (3.38 mg, 0.018 mmol) at room temperature, and the mixture was stirred for 3 hours at room temperature. After adding a saturated aqueous sodium hydrogencarbonate solution to the reaction mixture and stirring for 30 minutes, the mixture was concentrated under reduced pressure. The obtained residue was purified by NH silica gel column chromatography (n-heptane: ethyl acetate = 1:1 to ethyl acetate) to obtain the title compound (6.55 mg).

$^1$H-NMR Spectrum (500 MHz, $CDCl_3$) $\delta$(ppm):1.02 (3H, t, J=6.9 Hz), 2.14 (3H, s), 2.84 (2H, t, J=6.9 Hz), 3.25-3.34 (2H, m), 3.35-3.41 (1H, m), 3.53 (3H, s), 3.59-3.58-3.65 (1H, m), 4.19-4.24 (2H, m), 4.43-4.49 (1H, m), 5.23 (2H, brs), 6.88-6.94 (2H, m), 7.02-7.05 (1H, m), 7.12-7.14 (1H, m), 7.22-7.26 (1H, m), 7.52-7.58 (2H, m), 7.59-7.63 (1H, m), 8.63-8.69 (1H, m).

ESI-MS (m/z): 460.34 $[M+H]^+$.

[Production Example 12-1]

Ethyl 2-(2-chloro-6-iodoquinazolin-4-yl)-4-ethoxy-2-(pyridin-2-yl) butanoate

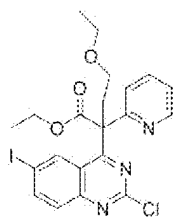

To a solution of ethyl 2-pyridylacetate (500 mg, 3.03 mmol) and 2-bromoethyl ethyl ether (486 mg, 3.18 mmol) in DMF (10 mL) there was added 50% to 72% sodium hydride oil (218 mg) at 0°C under a nitrogen atmosphere, and the mixture was stirred for 30 minutes at room temperature. Water was added to the reaction mixture, and extraction was performed with ethyl acetate. The organic layer was washed with brine. The organic layer was dried over magnesium sulfate and filtered. The filtrate was concentrated under reduced pressure to obtain a crude product. To a solution of the obtained crude product and 2,4-dichloro-6-iodoquinazoline (885 mg, 2.72 mmol) in THF (20 mL) there was added dropwise LHMDS (1 M THF solution, 4.54 mL, 4.54 mmol) at -78°C under a nitrogen atmosphere, and the mixture was

stirred for 2 hours at room temperature. Water was added to the reaction mixture, and extraction was performed with ethyl acetate. The organic layer was washed with brine. The organic layer was dried over magnesium sulfate and filtered. The filtrate was concentrated under reduced pressure and the residue was purified by silica gel column chromatography (n-heptane to n-heptane:ethyl acetate = 4:1) to obtain the title compound (406 mg).

ESI-MS (m/z): 526.19 [M+H]+.

[Production Example 12-2]

2-Chloro-4-(4-ethoxy-2-(pyridin-2-yl)-1-((tetrahydro-2H-pyran-2-yl)oxy)butan-2-yl)-6-iodoquinazoline

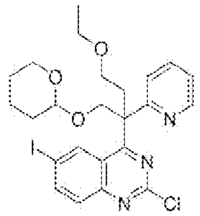

To a solution of the ethyl 2-(2-chloro-6-iodoquinazolin-4-yl)-4-ethoxy-2-(pyridin-2-yl) butanoate of Production Example 12-1 (406 mg, 0.772 mmol) in toluene (10 mL) there was added dropwise DIBAL-H (1 M toluene solution, 2.32 mL, 2.32 mmol) at -78°C under a nitrogen atmosphere, and the mixture was stirred for 30 minutes at 0°C. A saturated aqueous Rochelle salt solution and ethyl acetate were added, and stirring was continued. After oil-water distribution of the reaction mixture, the organic layer was washed with brine. The organic layer was dried over magnesium sulfate and filtered. The filtrate was concentrated under reduced pressure to obtain a crude product. To a solution of the obtained crude product in dichloromethane (10 mL) there were added DHP (325 mg, 3.86 mmol) and *p*-toluenesulfonic acid monohydrate (73.4 mg, 0.386 mmol) at room temperature, and the mixture was stirred for 6 hours at room temperature. A saturated aqueous sodium hydrogencarbonate solution was added to the reaction mixture, and extraction was performed with ethyl acetate. The organic layer was washed with brine. The organic layer was dried over magnesium sulfate and filtered. The filtrate was concentrated under reduced pressure and the residue was purified by silica gel column chromatography (n-heptane to *n*-heptane:ethyl acetate = 4:1) to obtain the title compound (261 mg).

ESI-MS (m/z): 568.29 [M+H]+.

[Production Example 12-3]

5-(2-Chloro-4-(4-ethoxy-2-(pyridin-2-yl)-1-((tetrahydro-2H-pyran-2-yl)oxy)butan-2-yl)quinazolin-6-yl)-1,3-dimethylpyridin-2(1H)-one

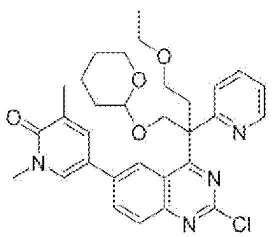

To a solution of the 2-chloro-4-(4-ethoxy-2-(pyridin-2-yl)-1-((tetrahydro-2H-pyran-2-yl)oxy)butan-2-yl)-6-iodoquinazoline of Production Example 12-2 (261 mg, 0.460 mmol) and 1,3-dimethyl-5-(4,4,5,5-tetramethyl-1,3,2-dioxaborolan-2-yl)pyridin-2(1H)-one (137 mg, 0.552 mmol) in toluene (4 mL) there were added ethanol (1 mL), sodium carbonate (2 M aqueous solution, 2 mL, 4.00 mmol) and tetrakis(triphenylphosphine)palladium(0) (26.6 mg, 0.023 mmol) at room temperature, and the mixture was stirred for 6 hours at 70°C.

The reaction mixture was returned to room temperature, water was added, and extraction was performed with ethyl acetate. The organic layer was washed with brine. The organic layer was dried over magnesium sulfate and filtered. The filtrate was concentrated under reduced pressure and the residue was purified by silica gel column chromatography

(n-heptane:ethyl acetate = 1: 1 to ethyl acetate) to obtain the title compound (213 mg). ESI-MS (m/z): 563.39 [M+H]+.

[Example 13]

5-(4-(1-Cyclobutoxy-2-hydroxy-1-phenylethyl)-2-((dimethyl(oxo)-16-sulfanylidene)amino)quinazolin-6-yl)-1,3-dimethylpyridin-2(1H)-one

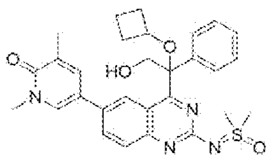

To a solution of the 5-(2-chloro-4-(1-cyclobutoxy-1-phenyl-2-((tetrahydro-2H-pyran-2-yl)oxy)ethyl)quinazolin-6-yl)-1,3-dimethylpyridin-2(1H)-one of Production Example 13-4 (20.0 mg, 0.036 mmol) in 1,4-dioxane (1 mL) there were added dimethylsulfoximine (4.99 mg, 0.054 mmol), cesium carbonate (29.1 mg, 0.089 mmol), BRETTPHOS (5.75 mg, 0.011 mmol) and tris(dibenzylideneacetone)dipalladium(0) (9.81 mg, 0.011 mmol) at room temperature, and the mixture was stirred for 13 hours at 90°C. The reaction mixture was then returned to room temperature. Purification was carried out by NH silica gel column chromatography (n-heptane:ethyl acetate = 1:1 to ethyl acetate) to obtain a crude product. To a solution of the obtained crude product in methanol (1 mL) there was added*p*-toluenesulfonic acid monohydrate (3.40 mg, 0.018 mmol) at room temperature, and the mixture was stirred for 12 hours at room temperature. After adding a saturated aqueous sodium hydrogencarbonate solution to the reaction mixture and stirring for 15 minutes, the mixture was concentrated under reduced pressure. The obtained residue was purified by NH silica gel column chromatography (ethyl acetate to ethyl acetate:methanol = 9:1) to obtain the title compound as a racemic mixture. It was then purified by SFC (IB, carbon dioxide:methanol = 75:25) to obtain the title compound (5.03 mg) as the former isomer. $^1$H-NMR Spectrum (500 MHz, $CDCl_3$) $\delta$(ppm): 1.15-1.27 (1H, m), 1.46-1.56 (2H, m), 1.71-1.87 (1H, m), 2.14-2.24 (4H, m), 2.25-2.34 (1H, m), 3.51 (3H, s), 3.51 (3H, s), 3.54 (3H, s), 3.56-3.62 (1H, m), 3.98-4.04 (1H, m), 4.18-4.22 (1H, m), 4.33-4.36 (1H, m), 6.98-7.02 (1H, m), 7.17-7.18 (1H, m), 7.21-7.25 (1H, m), 7.27-7.30 (2H, m), 7.37-7.41 (2H, m), 7.61-7.64 (1H, m), 7.69-7.73 (1H, m), 7.99-8.02 (1H, m).

ESI-MS (m/z): 533.35 [M+H]+.

[Production Example 13-1]

Ethyl 2-cyclobutoxy-2-phenylacetate

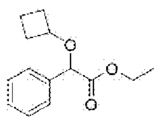

To a solution of cyclobutanol (500 mg, 6.94 mmol) and rhodium(II) acetate dimer (64.0 mg, 0.145 mmol) in dichloromethane (5 mL) there was added dropwise a solution of ethyl 2-diazo-2-phenylacetate (1.10 g, 5.78 mmol) in dichloromethane (5 mL) at room temperature, and the mixture was stirred for 12 hours at room temperature. The reaction mixture was concentrated under reduced pressure and the residue was purified by silica gel column chromatography (n-heptane to n-heptane:ethyl acetate = 7:3) to obtain the title compound (1.19 g).

ESI-MS (m/z): 235.05 [M+H]+.

[Production Example 13-2]

Ethyl 2-(2-chloro-6-iodoquinazolin-4-yl)-2-cyclobutoxy-2-phenylacetate

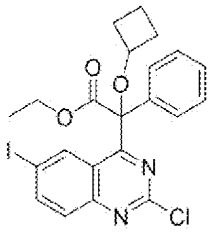

To a solution of the ethyl 2-cyclobutoxy-2-phenylacetate of Production Example 13-1 (397 mg, 1.69 mmol) and 2,4-dichloro-6-iodoquinazoline (500 mg, 1.54 mmol) in THF (10 mL) there was added dropwise LHMDS (1 M THF solution, 2.31 mL, 2.31 mmol) at -78°C under a nitrogen atmosphere, and the mixture was stirred for 2 hours at room temperature. Saturated aqueous ammonium chloride was added to the reaction mixture, and extraction was performed with ethyl acetate. The organic layer was washed with brine. The organic layer was dried over magnesium sulfate and filtered. The filtrate was concentrated under reduced pressure and the residue was purified by silica gel column chromatography (n-heptane to n-heptane:ethyl acetate = 9:1) to obtain the title compound (679 mg).

ESI-MS (m/z): 523.18 $[M+H]^+$.

[Production Example 13-3]

2-Chloro-4-(1-cyclobutoxy-1-phenyl-2-((tetrahydro-2H-pyran-2-yl)oxy)ethyl)-6-iodoquinazoline

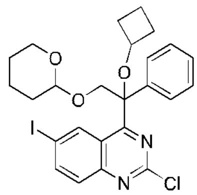

To a solution of the ethyl 2-(2-chloro-6-iodoquinazolin-4-yl)-2-cyclobutoxy-2-phenylacetate of Production Example 13-2 (679 mg, 1.30 mmol) in toluene (15 mL) there was added dropwise DIBAL-H (1 M toluene solution, 3.90 mL, 3.90 mmol) at -78°C under a nitrogen atmosphere, and the mixture was stirred for 30 minutes at 0°C. A saturated aqueous Rochelle salt solution and ethyl acetate were added, and stirring was continued. Oil-water distribution of the reaction mixture was carried out. The organic layer was washed with brine and the organic layer was dried over magnesium sulfate. After filtration, the filtrate was concentrated under reduced pressure to obtain a crude product. To a solution of the obtained crude product in dichloromethane (10 mL) there were added DHP (546 mg, 6.49 mmol) and (1S)-(+)-10-camphorsulfonic acid (151 mg, 0.649 mmol) at room temperature, and the mixture was stirred for 12 hours at room temperature. A saturated aqueous sodium hydrogencarbonate solution was added to the reaction mixture, and extraction was performed with ethyl acetate. The organic layer was washed with brine. The organic layer was dried over magnesium sulfate and filtered. The filtrate was concentrated under reduced pressure and the residue was purified by silica gel column chromatography (n-heptane to *n*-heptane:ethyl acetate = 4:1) to obtain the title compound (430 mg).

ESI-MS (m/z): 565.21 $[M+H]^+$.

[Production Example 13-4]

5-(2-Chloro-4-(1-cyclobutoxy-1-phenyl-2-((tetrahydro-2H-pyran-2-yl)oxy)ethyl)quinazolin-6-yl)-1,3-dimethylpyridin-2(1H)-one

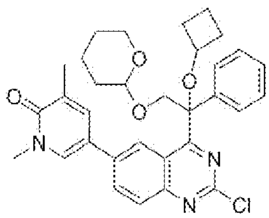

To a solution of the 2-chloro-4-(1-cyclobutoxy-1-phenyl-2-((tetrahydro-2H-pyran-2-yl)oxy)ethyl)-6-iodoquinazoline of Production Example 13-3 (430 mg, 0.761 mmol) and 1,3-dimethyl-5-(4,4,5,5-tetramethyl-1,3,2-dioxaborolan-2-yl) pyridin-2(1H)-one (228 mg, 0.914 mmol) in toluene (4 mL) there were added ethanol (1 mL), sodium carbonate (2 M aqueous solution, 2.00 mL, 4.00 mmol) and tetrakis(triphenylphosphine)palladium(0) (44.0 mg, 0.038 mmol) at room temperature, and the mixture was stirred for 12 hours at 70°C. The reaction mixture was returned to room temperature, water was added, and extraction was performed with ethyl acetate. The organic layer was washed with brine. The organic layer was dried over magnesium sulfate and filtered. The filtrate was concentrated under reduced pressure and the residue was purified by silica gel column chromatography (n-heptane:ethyl acetate = 1: 1 to ethyl acetate) to obtain the title compound (244 mg).
ESI-MS (m/z): 560.42 $[M+H]^+$.

[Example 14]

7-(4-(1-Cyclopropoxy-2-hydroxy-1-phenylethyl)-2-((1-methylpiperidin-4-yl)oxy)quinazolin-6-yl)-5-methyl-3,5-dihydrofuro[3,4-c]pyridine-4(1H)-one

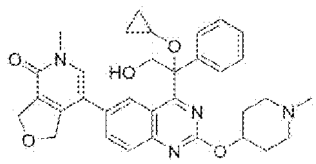

To a solution of the 7-bromo-5-methyl-3,5-dihydrofuro[3,4-c]pyridine-4(1H)-one of Production Example 14-5 (316 mg, 1.37 mmol), bis(pinacolato)diboron (523 mg, 2.06 mmol) and potassium acetate (404 mg, 4.12 mmol) in DMSO (10 mL) there were added 1,1'-bis(diphenylphosphino)ferrocenepalladium(II) dichloride and dichloromethane (50.3 mg, 69 μmol), and the mixture was stirred for 3 hours at 80°C. Water was added to the reaction mixture, which was then extracted 7 times with ethyl acetate. The organic layer was dried over anhydrous sodium sulfate and filtered, and then the filtrate was concentrated under reduced pressure to obtain a crude product (381 mg).
To a solution of a portion (19.8 mg) of the obtained crude product and the 4-(1-cyclopropoxy-1-phenyl-2((tetrahydro-2H-pyran-2-yl)oxy)ethyl)-6-iodo-2-((1-methylpiperidin-4-yl)oxy)quinazoline of Production Example 14-1 (30.0 mg, 0.048 mmol) in toluene (1 mL) there were added ethanol (0.25 mL), sodium carbonate (2 M aqueous solution, 0.500 mL, 1.00 mmol) and tetrakis(triphenylphosphine)palladium(0) (2.75 mg, 0.0024 mmol) at room temperature, and the mixture was stirred for 12 hours at 80°C. The reaction mixture was returned to room temperature, water was added, and extraction was performed with ethyl acetate. The organic layer was washed with brine. The organic layer was dried over magnesium sulfate and filtered. The filtrate was concentrated under reduced pressure and the residue was purified by NH silica gel column chromatography (n-heptane:ethyl acetate = 1:1 to ethyl acetate) to obtain a crude product. To a solution of the obtained crude product in methanol (1 mL) there was added *p*-toluenesulfonic acid monohydrate (4.53 mg, 0.024 mmol) at room temperature, and the mixture was stirred for 2 hours at room temperature. After adding a saturated aqueous sodium hydrogencarbonate solution to the reaction mixture and stirring for 30 minutes, the mixture was concentrated under reduced pressure. The obtained residue was purified by NH silica gel column chromatography (n-heptane:ethyl acetate = 1:1 to ethyl acetate to ethyl acetate:methanol = 4:1) to obtain the title compound as a racemic mixture. It was then purified by SFC (IG, carbon dioxide:methanol = 70:30) to obtain the title compound (9.46 mg) as the former isomer.
ESI-MS (m/z): 569.54 $[M+H]^+$.

[Production Example 14-1]

4-(1-Cyclopropoxy-1-phenyl-2-((tetrahydro-2H-pyran-2-yl)oxy)ethyl)-6-iodo-2-((1-methylpiperidin-4-yl)oxy)quinazoline

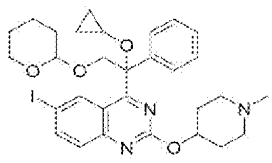

To a solution of the 2-chloro-4-(1-cyclopropoxy-1-phenyl-2-((tetrahydro-2H-pyran-2-yl)oxy)ethyl)-6-iodoquinazoline of Production Example 2-3(30.0 mg, 0.054 mmol) and 4-hydroxy-1-methylpiperidine(9.41 mg, 0.082 mmol) in THF (1 mL) there was added 50% to 72% sodium hydride oil (3.92 mg) at 0°C under a nitrogen atmosphere, and the mixture was stirred for 12 hours at room temperature. Water was added to the reaction mixture, and extraction was performed with ethyl acetate. The organic layer was washed with brine and the organic layer was dried over magnesium sulfate. After filtration, the filtrate was concentrated under reduced pressure. The obtained residue was purified by NH silica gel column chromatography (n-heptane:ethyl acetate = 7:3 to ethyl acetate) to obtain the title compound (30.0 mg).
ESI-MS (m/z): 630.34 $[M+H]^+$.

[Production Example 14-2]

3-Amino-4-chloro-1-methylpyridin-2(1H)-one

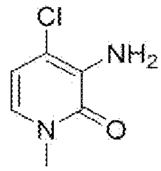

To a solution of 4-chloro-3-nitro-2-pyridone (3.00 g, 17.2 mmol) and iodomethane (3.66 g, 25.8 mmol) in DMF (50 mL) there was added potassium carbonate (4.75 g, 34.4 mmol), and the mixture was stirred for 2 hours at 70°C. Water was added to the reaction mixture, and extraction was performed with ethyl acetate. The organic layer was washed with brine and the organic layer was dried over magnesium sulfate. The organic layer was filtered, and the filtrate was then concentrated under reduced pressure to obtain a crude product. To a solution of the obtained crude product in ethyl acetate (100 mL) there was added tin dichloride dihydrate (8.26 g, 36.3 mmol), and the mixture was stirred for 3 hours at room temperature. The reaction mixture was cooled to 0°C, and then an aqueous potassium carbonate solution was added to adjust the pH to about 10. The reaction mixture was extracted 3 times with ethyl acetate, and the organic layer was dried over sodium sulfate. The organic layer was filtered, and the filtrate was then concentrated under reduced pressure. The obtained residue was purified by silica gel column chromatography (n-heptane:ethyl acetate = 7:3 to ethyl acetate) to obtain the title compound (1.7 g).
$^1$H-NMR Spectrum (500 MHz, $CDCl_3$) $\delta$(ppm): 3.53 (3H, s), 4.48 (2H, brs), 6.16 (1H, d, J=7.3 Hz), 6.63 (1H, d, J=7.3 Hz).

[Production Example 14-3]

3,4-Dichloro-1-methylpyridin-2(1H)-one

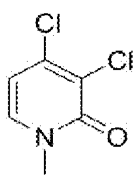

To a mixture of the 3-amino-4-chloro-1-methylpyridin-2(1H)-one of Production Example 14-2 (1.70 g, 10.7 mmol) and concentrated hydrochloric acid (30 mL) there was added dropwise a solution of sodium nitrite (3.70 g, 53.6 mmol) in water (15 mL) at 0°C over a period of at least 2 minutes, and the mixture was stirred for 2 minutes. Copper chloride (10.6 g, 107 mmol) was added in multiple divided portions at 0°C, and then the mixture was stirred for 5 minutes and further stirred for 30 minutes at room temperature. After adding a 5 N sodium hydroxide aqueous solution to the reaction mixture, it was extracted 3 times with ethyl acetate. The organic layer was washed with brine and dried over magnesium sulfate. The organic layer was filtered and the filtrate was concentrated under reduced pressure to obtain the title compound (1.27 g).
ESI-MS (m/z): 177.81 $[M+H]^+$.

[Production Example 14-4]

5-Methyl-3,5-dihydrofuro[3,4-c]pyridine-4(1H)-one

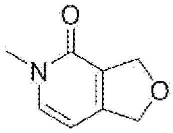

To a solution of the 3,4-dichloro-1-methylpyridin-2(1H)-one of Production Example 14-3 (700 mg, 3.93 mmol) in 1,4-dioxane (20 mL) there were added tris(dibenzylideneacetone)dipalladium(0) (360 mg, 0.393 mmol), 2-dicyclohexylphosphino-2', 4', 6'-triisopropylbiphenyl (412 mg, 0.865 mmol) and (oxybis(methylene))bis(tributylstannan) (2.58 g, 4.13 mmol), and the mixture was stirred for 12 hours at 95°C. The reaction mixture was purified by direct silica gel column chromatography (n-heptane:ethyl acetate = 1:1 to ethyl acetate to ethyl acetate:methanol = 10:1) to obtain the title compound (339 mg).
ESI-MS (m/z): 151.83 $[M+H]^+$.

[Production Example 14-5]

7-Bromo-5-methyl-3,5-dihydrofuro[3,4-c]pyridine-4(1H)-one

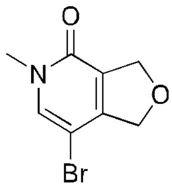

To a solution of the 5-methyl-3,5-dihydrofuro[3,4-c]pyridine-4(1H)-one of Production Example 14-4 (339 mg, 2.24 mmol) in chloroform (15 mL) there was added N-bromosuccinimide (419 mg, 2.36 mmol), and the mixture was stirred for 3 hours at 0°C. Water was added to the reaction mixture, which was then extracted with dichloromethane. The organic layer was dried over sodium sulfate. The organic layer was filtered, and the filtrate was then concentrated under reduced pressure. The obtained residue was purified by silica gel column chromatography (n-heptane:ethyl acetate = 1:1 to 1:4) to obtain the title compound (316 mg).
ESI-MS (m/z): 229.85 $[M+H]^+$.

[Example 15]

5-(2-Amino-4-(1-cyclopropoxy-2-hydroxy-1-(thiophen-2-yl)ethyl)quinazolin-6-yl)-1,3-dimethylpyridin-2(1H)-one

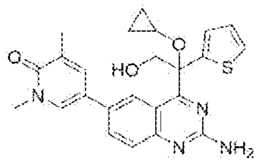

The title compound (7 mg) was obtained from the 5-(2-chloro-4-(1-cyclopropoxy-2-((tetrahydro-2H-pyran-2-yl) oxy)-1-(thiophen-2-yl)ethyl)quinazolin-6-yl)-1,3-dimethylpyridin-2(1H)-one of Production Example 15-4 (10 mg), using the same method as Production Example 2-5 and Example 2.
ESI-MS (m/z): 449.38 $[M+H]^+$.

[Production Example 15-1]

Ethyl 2-cyclopropoxy-2-(thiophenephen-2-yl)acetate

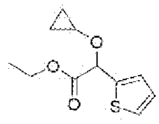

The title compound (320 mg) was obtained from ethyl 2-diazo-2-(thiophenephen-2-yl)acetate (830 mg) using the same method as Production Example 2-1.
$^1$H-NMR Spectrum (400 MHz, $CDCl_3$) δ(ppm): 0.47-0.60 (2H, m), 0.67-0.82 (2H, m), 1.24-1.37 (4H, m), 3.44-3.56 (1H, m), 4.20-4.36 (2H, m), 6.95-7.04 (1H, m), 7.11-7.19 (1H, m), 7.29-7.37 (1H, m).

[Production Example 15-2]

Ethyl 2-(2-chloro-6-iodoquinazolin-4-yl)-2-cyclopropoxy-2-(thiophenephen-2-yl)acetate

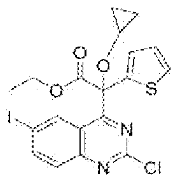

The title compound (740 mg) was obtained from the ethyl 2-cyclopropoxy-2-(thiophenephen-2-yl)acetate of Production Example 15-1 (320 mg) using the same method as Production Example 2-2.
$^1$H-NMR Spectrum (400 MHz, $CDCl_3$) δ(ppm): 0.20-0.42 (2H, m), 0.44-0.61 (2H, m), 1.20 (3H, t), 3.49-3.64 (1H, m), 4.20-4.36 (2H, m), 6.97-7.06 (1H, m), 7.25-7.27 (m, 1H), 7.38-7.48 (1H, m), 7.63-7.76 (1H, m), 8.00-8.16 (1H, m), 8.78-8.87 (1H, m).

[Production Example 15-3]

2-Chloro-4-(1-cyclopropoxy-2-((tetrahydro-2H-pyran-2-yl)oxy)-1-(thiophen-2-yl)ethyl)-6-iodoquinazoline

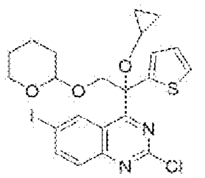

The title compound (791 mg) was obtained from the ethyl 2-(2-chloro-6-iodoquinazolin-4-yl)-2-cyclopropoxy-2-(thiophenephen-2-yl)acetate of Production Example 15-2 (981 mg) using the same method as Production Example 2-3.

ESI-MS (m/z): 557.00 [M+H]+.

[Production Example 15-4]

5-(2-Chloro-4-(1-cyclopropoxy-2-((tetrahydro-2H-pyran-2-yl)oxy)-1-(thiophen-2-yl)ethyl)quinazolin-6-yl)-1,3-dimethylpyridin-2(1H)-one

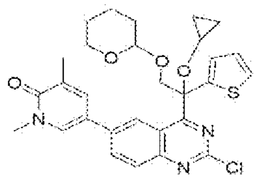

The title compound (290 mg) was obtained from the 2-chloro-4-(1-cyclopropoxy-2-((tetrahydro-2H-pyran-2-yl)oxy)-1-(thiophen-2-yl)ethyl)-6-iodoquinazoline of Production Example 15-3 (370 mg) using the same method as Production Example 2-4.

$^1$H-NMR Spectrum (400 MHz, $CDCl_3$) δ(ppm): 0.20-1.04 (4H, m), 1.25-1.75 (6H, m), 2.23 (3H, s), 3.05-3.95 (6H, m), 4.40-5.10 (3H, m), 6.55-6.69 (1H, m), 6.78-6.94 (1H, m), 7.21-7.32 (2H, m), 7.34-7.44 (1H, m), 7.78-8.01 (2H, m), 8.46-8.61 (1H, m).

[Example 16]

7-(1,5-Dimethyl-6-oxo-1,6-dihydropyridin-3-yl)-1-(1-ethoxy-2-hydroxy-1-phenylethyl)isoquinoline-3-carboxamide

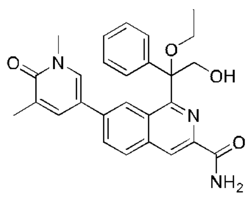

The title compound was obtained as a racemic mixture from the 7-(1,5-dimethyl-6-oxo-1,6-dihydropyridin-3-yl)-1-(1-ethoxy-1-phenyl-2-((tetrahydro-2H-pyran-2-yl)oxy)ethyl)isoquinoline-3-carboxamide of Production Example 16-6 (48 mg, 0.089 mmol), using the same method as Example 2. The obtained racemic mixture was purified by SFC (IG, carbon dioxide:methanol = 8:2) to obtain the title compound (21.2 mg) as the former isomer.

ESI-MS (m/z): 458.30 [M+H]+.

[Production Example 16-1]

Ethyl 2-ethoxy-2-phenylacetate

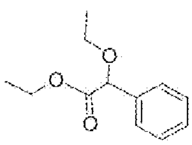

The title compound (2.34 g) was obtained from ethyl 2-diazo-2-phenylacetate (2.30 g, 12.1 mmol) using the same

method as Production Example 2-1.

$^1$H-NMR Spectrum (400 MHz, $CDCl_3$) δ(ppm): 1.14-1.34 (6H, m), 3.45-3.66 (2H, m), 4.06-4.25 (2H, m), 4.85 (1H, s), 7.28-7.56 (5H, m).

[Production Example 16-2]

7-Bromo-1,3-dichloroisoquinoline

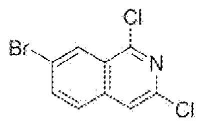

To a solution of 7-bromo-1-chloroisoquinoline (5.00 g, 20.6 mmol) in dichloromethane (100 mL) there was added metachloroperbenzoic acid (9.24 g, 41.2 mmol), and the mixture was stirred for 2 days at room temperature. Dichloromethane and a 1 N sodium hydroxide aqueous solution were added to the reaction mixture, and after stirring for 2 hours and oil-water distribution, the organic layer was washed with brine. The organic layer was dried over magnesium sulfate and filtered. The filtrate was concentrated under reduced pressure to obtain a crude product. Phosphoryl chloride (40.0 mL, 429 mmol) was added to the obtained crude product, and the mixture was stirred for 12 hours at 120°C. The reaction mixture was concentrated under reduced pressure, and then toluene was added to the obtained residue and the mixture was again concentrated under reduced pressure. The residue was dissolved in dichloromethane, and a saturated sodium bicarbonate water solution was added for oil-water distribution. The aqueous layer was extracted with dichloromethane. The organic layer was washed with brine. The organic layer was dried over magnesium sulfate and filtered. The filtrate was concentrated under reduced pressure and the residue was purified by silica gel column chromatography (n-heptane:ethyl acetate = 19:1 to 9:1) to obtain the title compound (2.05 g).

ESI-MS (m/z): 277.84 $[M+H]^+$.

[Production Example 16-3]

Ethyl 2-(7-bromo-3-chloroisoquinolin-1-yl)-2-ethoxy-2-phenylacetate

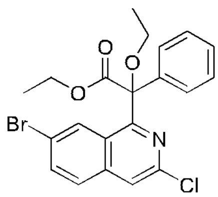

To a solution of the ethyl 2-ethoxy-2-phenylacetate of Production Example 16-1 (564 mg, 2.71 mmol), the 7-bromo-1,3-dichloroisoquinoline of Production Example 16-2 (500 mg, 1.80 mmol) and bis(tri-tert-butylphosphine)palladium(0) (27.7 mg, 0.054 mmol) in toluene (10 mL) there was added LHMDS (1 M THF solution, 5.42 mL, 5.42 mmol) at 0°C under a nitrogen atmosphere, and the mixture was stirred for 14 hours at room temperature. Ethyl acetate and water were added to the reaction mixture for oil-water distribution. The organic layer was washed with brine. The organic layer was dried over magnesium sulfate and filtered. The filtrate was concentrated under reduced pressure and the residue was purified by silica gel column chromatography (n-heptane to n-heptane:ethyl acetate = 4:1) to obtain the title compound (538 mg).

ESI-MS (m/z): 448.13 $[M+H]^+$.

[Production Example 16-4]

7-Bromo-3-chloro-1-(1-ethoxy-1-phenyl-2-((tetrahydro-2H-pyran-2-yl)oxy)ethyl)isoquinoline

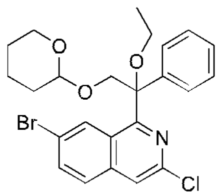

The title compound (588 mg) was obtained from the ethyl 2-(7-bromo-3-chloroisoquinolin-1-yl)-2-ethoxy-2-phenylacetate of Production Example 16-3 (538 mg, 1.20 mmol), using the same method as Production Example 2-3.
ESI-MS (m/z): 490.20 $[M+H]^+$.

[Production Example 16-5]

5-(3-Chloro-1-(1-ethoxy-1-phenyl-2-((tetrahydro-2H-pyran-2-yl)oxy)ethyl)isoquinolin-7-yl)-1,3-dimethylpyridin-2(1H)-one

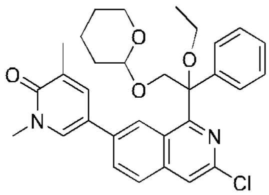

The title compound (382 mg) was obtained from the 7-bromo-3-chloro-1-(1-ethoxy-1-phenyl-2-((tetrahydro-2H-pyran-2-yl)oxy)ethyl)isoquinoline of Production Example 16-4 (588 mg, 1.20 mmol), by the same method as Production Example 2-4.
ESI-MS (m/z): 533.35 $[M+H]^+$.

[Production Example 16-6]

7-(1,5-Dimethyl-6-oxo-1,6-dihydropyridin-3-yl)-1-(1-ethoxy-1-phenyl-2-((tetrahydro-2H-pyran-2-yl)oxy)ethyl)isoquinoline-3-carboxamide

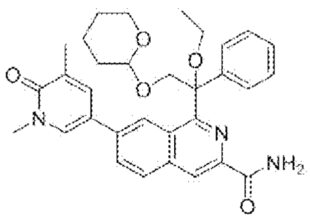

To a solution of the 5-(3-chloro-1-(1-ethoxy-1-phenyl-2-((tetrahydro-2H-pyran-2-yl)oxy)ethyl)isoquinolin-7-yl)-1,3-dimethylpyridin-2(1H)-one of Production Example 16-5 (73.0 mg, 0.137 mmol) and zinc cyanide (32.2 mg, 0.274 mmol) in DMF (1 mL) there was added tetrakis(triphenylphosphine)palladium(0) (158 mg, 0.137 mmol), and the mixture was stirred for 5 hours at 140°C under a nitrogen atmosphere. The reaction mixture was filtered and washed with ethyl acetate. The filtrate was concentrated under reduced pressure and the residue was purified by NH silica gel column chromatography (ethyl acetate to ethyl acetate:methanol = 9:1) to obtain a crude product. To a solution of the obtained crude product in THF (1 mL) there was added lithium hydroxide peroxide prepared by adding a 30% aqueous hydrogen peroxide solution (0.093 mL, 0.822 mmol) to a solution of lithium hydroxide (11.8 mg, 0.493 mmol) in water (0.30 mL), and the mixture was stirred for 1 hour at room temperature. A saturated sodium sulfite aqueous solution was added to the reaction mixture, and extraction was performed with ethyl acetate. The organic layer was washed with brine. The organic layer was dried over sodium sulfate and filtered. The filtrate was concentrated under reduced pressure and the residue was purified by NH silica gel column chromatography (ethyl acetate to ethyl acetate:methanol = 9:1) to obtain the title

compound (48 mg).
ESI-MS (m/z): 542.44 [M+H]+.

[Example 17]

6-(1,5-Dimethyl-6-oxo-1,6-dihydropyridin-3-yl)-1-ethyl-4-(2-hydroxy-1-methoxy-1-phenylethyl)quinazoline-2(1H)-one

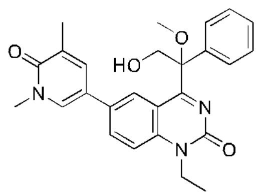

To a solution of the 6-(1,5-dimethyl-6-oxo-1,6-dihydropyridin-3-yl)-1-ethyl-4-(1-methoxy-1-phenyl-2-((tetrahydro-2H-pyran-2-yl)oxy)ethyl)quinazoline-2(1H)-one of Production Example 17-5 (530 mg, 1.00 mmol) in methanol (10 mL) there was addedp-toluenesulfonic acid monohydrate (381 mg, 2.00 mmol) at room temperature, and the mixture was stirred for 1 hour at room temperature. A saturated aqueous sodium hydrogencarbonate solution was added to the reaction mixture, and extraction was performed with ethyl acetate. The organic layer was washed with brine. The organic layer was dried over magnesium sulfate and filtered. The filtrate was concentrated under reduced pressure and the residue was purified by NH silica gel column chromatography (ethyl acetate to ethyl acetate:methanol = 9:1) to obtain a racemic mixture of the title compound. The obtained racemic mixture was purified by SFC (IF, carbon dioxide:methanol = 70:30) to obtain the title compound (124 mg).
$^1$H-NMR Spectrum (500 MHz, $CDCl_3$) δ(ppm): 1.40-1.45 (3H, m), 3.11 (1H, brs), 3.46 (3H, s), 3.52 (3H, s), 4.23-4.35 (3H, m), 4.41-4.47 (1H, m), 5.26 (3H, s), 6.89 (1H, brs), 7.06 (1H, brs), 7.23-7.28 (1H, m), 7.31-7.35 (3H, m), 7.45-7.50 (2H, m), 7.60-7.65 (1H, m), 8.10 (1H, brs).
ESI-MS (m/z): 446.34 [M+H]+.

[Production Example 17-1]

Ethyl 2-methoxy-2-phenylacetate

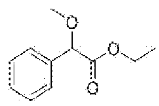

To a solution of DL-alpha-methoxyphenylacetic acid (3.00 g, 18.1 mmol) in ethanol (30 mL) there was added dropwise sulfuric acid (0.193 mL, 3.61 mmol) at room temperature, and the mixture was stirred for 4 hours at 80°C. After then adding sodium carbonate (1.00 g, 11.9 mmol) to the reaction mixture at room temperature, it was stirred for 1 hour. The reaction mixture was then filtered and the filtrate was concentrated under reduced pressure. The residue was diluted with ethyl acetate and filtered using silica gel. The filtrate was concentrated under reduced pressure to obtain the title compound (2.73 g).
$^1$H-NMR Spectrum (400 MHz, $CDCl_3$) δ(ppm): 1.17-1.24 (3H, m), 3.40 (3H, s), 4.10-4.25 (2H, m), 4.74 (1H, s), 7.30-7.38 (3H, m), 7.40-7.46 (2H, m).

[Production Example 17-2]

Ethyl 2-(2-chloro-6-iodoquinazolin-4-yl)-2-methoxy-2-phenylacetate

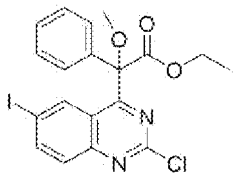

To a solution of the ethyl 2-methoxy-2-phenylacetate of Production Example 17-1 (1.00 g, 5.15 mmol) and 2,4-dichloro-6-iodoquinazoline (2.34 g, 7.21 mmol) in THF (15 mL) there was added dropwise LHMDS (1.09 M THF solution, 7.09 mL, 7.72 mmol) at -78°C under a nitrogen atmosphere. The reaction mixture was stirred for 2 hours at room temperature. Saturated aqueous ammonium chloride was added to the reaction mixture, and extraction was performed with ethyl acetate. The organic layer was washed with brine. The organic layer was dried over sodium sulfate and filtered. The filtrate was concentrated under reduced pressure and the residue was purified by NH silica gel column chromatography (n-heptane to n-heptane:ethyl acetate = 7:3) to obtain the title compound (2.52 g).

ESI-MS (m/z): 483.18 $[M+H]^+$.

[Production Example 17-3]

2-Chloro-6-iodo-4-(1-methoxy-1-phenyl-2-((tetrahydro-2H-pyran-2-yl)oxy)ethyl)quinazoline

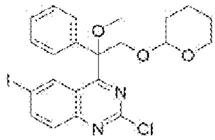

To a solution of the ethyl 2-(2-chloro-6-iodoquinazolin-4-yl)-2-methoxy-2-phenylacetate of Production Example 17-2 (2.52 g, 5.22 mmol) in dichloromethane (20 mL) there was added dropwise DIBAL-H (1 M toluene solution, 12.0 mL, 12.0 mmol) at -78°C, and the mixture was stirred for 1 hour at 0°C. A saturated aqueous Rochelle salt solution and ethyl acetate were added to the reaction mixture and stirring was continued, after which the mixture was extracted with ethyl acetate. The organic layer was washed with brine and the organic layer was dried over sodium sulfate. The organic layer was filtered, and the filtrate was then concentrated under reduced pressure to obtain a crude product. To a solution of the obtained crude product in dichloromethane (20 mL) there were added DHP (1.42 mL, 15.7 mmol) and (1S)-(+)-10-camphorsulfonic acid (243 mg, 1.04 mmol) at room temperature, and the mixture was stirred for 1 hour at room temperature. A saturated aqueous sodium hydrogencarbonate solution was added to the reaction mixture, and extraction was performed with ethyl acetate. The organic layer was washed with brine and the organic layer was dried over sodium sulfate. After filtration, the filtrate was concentrated under reduced pressure and purified by NH silica gel column chromatography (n-heptane to *n*-heptane:ethyl acetate = 1:1) to obtain the title compound (3.00 g).

ESI-MS (m/z): 525.24 $[M+H]^+$.

[Production Example 17-4]

5-(2-Chloro-4-(1-methoxy-1-phenyl-2-((tetrahydro-2H-pyran-2-yl)oxy)ethyl)quinazolin-6-yl)-1,3-dimethylpyridin-2(1H)-one

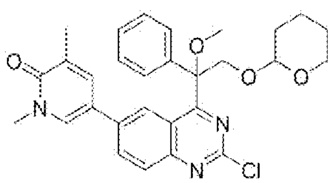

To a solution of the 2-chloro-6-iodo-4-(1-methoxy-1-phenyl-2-((tetrahydro-2H-pyran-2-yl)oxy)ethyl)quinazoline of Production Example 17-3 (3.00 g, 5.72 mmol) and 1,3-dimethyl-5-(4,4,5,5-tetramethyl-1,3,2-dioxaborolan-2-yl)pyridin-2(1H)-one (1.50 g, 6.00 mmol) in toluene (20 mL) there were added ethanol (5 mL), sodium carbonate (2 M aqueous

solution, 10 mL, 20.0 mmol) and tetrakis(triphenylphosphine)palladium(0) (661 mg, 0.572 mmol) at room temperature, and the mixture was stirred for 18 hours at 70°C. The reaction mixture was filtered with Celite and washed with ethyl acetate. The filtrate was concentrated under reduced pressure and the residue was purified by NH silica gel column chromatography (n-heptane:ethyl acetate = 1: 1 to ethyl acetate) to obtain the title compound (3.11 g).

ESI-MS (m/z): 520.42 $[M+H]^+$.

[Production Example 17-5]

6-(1,5-Dimethyl-6-oxo-1,6-dihydropyridin-3-yl)-1-ethyl-4-(1-methoxy-1-phenyl-2-((tetrahydro-2H-pyran-2-yl)oxy)ethyl) quinazoline-2(1H)-one

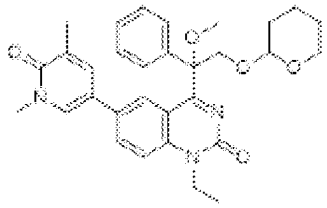

To a solution of the 5-(2-chloro-4-(1-methoxy-1-phenyl-2-((tetrahydro-2H-pyran-2-yl)oxy)ethyl)quinazolin-6-yl)-1,3-dimethylpyridin-2(1H)-one of Production Example 17-4 (1.00 g, 1.92 mmol) in 1,4-dioxane (6 mL) and water (2 mL) there were added potassium carbonate (0.532 g, 3.85 mmol) and DABCO (0.108 g, 0.961 mmol) at room temperature, and the mixture was stirred for 2 hours at 80°C. Saturated aqueous ammonium chloride was added to the reaction mixture, and extraction was performed with ethyl acetate. The organic layer was washed with brine. The organic layer was dried over sodium sulfate and filtered. The filtrate was concentrated under reduced pressure to obtain a crude product. To a solution of the obtained crude product in DMF (7.5 mL) there were added potassium carbonate (0.797 g, 5.77 mmol) and ethyl iodide (461 μL, 5.77 mmol), and the mixture was stirred for 20 hours at room temperature. Brine and ethyl acetate were added to the reaction mixture, and extraction was performed with ethyl acetate. The organic layer was washed with brine. The organic layer was dried over sodium sulfate and filtered. The filtrate was concentrated under reduced pressure, and the residue was purified by NH silica gel column chromatography (heptane:ethyl acetate = 1:1 to ethyl acetate to ethyl acetate:methanol = 9: 1) to obtain the title compound (0.530 g).

ESI-MS (m/z): 530.48 $[M+H]^+$.

[Example 18]

N-((4-(1-Cyclopropoxy-2-hydroxy-1-phenylethyl)-6-(1,5-dimethyl-6-oxo-1,6-dihydropyridin-3-yl)quinazolin-2-yl)methyl) methanesulfonamide

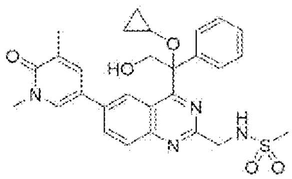

To a solution of the 5-(2-(aminomethyl)-4-(1-cyclopropoxy-1-phenyl-2-((tetrahydro-2H-pyran-2-yl)oxy)ethyl) quinazolin-6-yl)-1,3-dimethylpyridin-2(1H)-one of Production Example 18-2 (19.5 mg, 0.036 mmol) in dichloromethane (1 mL) there were added 4-dimethylaminopyridine (5.29 mg, 0.043 mmol) and methanesulfonyl chloride (3.35 μL, 0.043 mmol) at room temperature, and the mixture was stirred for 1 hour at room temperature. A saturated aqueous sodium hydrogencarbonate solution was added to the reaction mixture, and extraction was performed with ethyl acetate. The organic layer was washed with brine. The organic layer was dried over sodium sulfate and filtered. The filtrate was concentrated under reduced pressure to obtain a crude product. To a solution of the obtained crude product in methanol (1.0 mL) there was added *p*-toluenesulfonic acid monohydrate (13.7 mg, 0.072 mmol) at room temperature, and the mixture was stirred for 1 hour at room temperature. A saturated aqueous sodium hydrogencarbonate solution was added

to the reaction mixture, and extraction was performed with ethyl acetate. The organic layer was washed with brine. The organic layer was dried over sodium sulfate and filtered. The filtrate was concentrated under reduced pressure and the residue was purified by NH silica gel column chromatography (ethyl acetate to ethyl acetate:methanol = 9:1) to obtain the title compound (7.1 mg).

$^1$H-NMR Spectrum (400 MHz, $CDCl_3$) δ(ppm): 0.17-0.30 (2H, m), 0.48-0.55 (1H, m), 0.72-0.79 (1H, m), 2.18 (3H, s), 2.78 (1H, brs), 3.09 (3H, s), 3.23-3.27 (1H, m), 3.56 (3H, s), 4.53-4.60 (1H, m), 4.64-4.70 (1H, m), 4.81-4.84 (2H, m), 5.80-5.86 (1H, m), 7.00-7.02 (1H, m), 7.13-7.16 (1H, m), 7.27-7.34 (5H, m), 7.80-7.85 (1H, m), 7.94-7.98 (1H, m), 8.07-8.10 (1H, m).

ESI-MS (m/z): 535.35 [M+H]$^+$.

[Production Example 18-1]

4-(1-Cyclopropoxy-1-phenyl-2-((tetrahydro-2H-pyran-2-yl)oxy)ethyl)-6-iodoquinazoline-2-carbonitrile

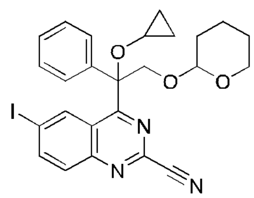

To a solution of the 2-chloro-4-(1-cyclopropoxy-1-phenyl-2-((tetrahydro-2H-pyran-2-yl)oxy)ethyl)-6-iodoquinazoline of Production Example 2-3 (950 mg, 1.73 mmol) in dimethyl sulfoxide (7 mL) there were added sodium cyanide (101 mg, 2.07 mmol) and DABCO (232 mg, 2.07 mmol) at room temperature, and the mixture was stirred for 32 hours at room temperature. Water was added to the reaction mixture, and extraction was performed with ethyl acetate. The organic layer was washed with brine. The organic layer was dried over sodium sulfate and filtered. The filtrate was concentrated under reduced pressure. The residue was purified by NH silica gel column chromatography (heptane to heptane:ethyl acetate = 1: 1) to obtain the title compound (496 mg).

ESI-MS (m/z): 542.26 [M+H]$^+$.

[Production Example 18-2]

5-(2-(Aminomethyl)-4-(1-cyclopropoxy-1-phenyl-2-((tetrahydro-2H-pyran-2-yl)oxy)ethyl)quinazolin-6-yl)-1,3-dimethylpyridin-2(1H)-one

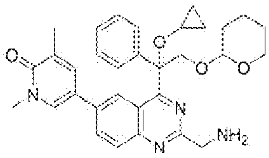

To a solution of the 4-(1-cyclopropoxy-1-phenyl-2-((tetrahydro-2H-pyran-2-yl)oxy)ethyl)-6-iodoquinazoline-2-carbonitrile of Production Example 18-1 (100 mg, 0.185 mmol) in toluene (1 mL) there was added dropwise DIBAL-H (1 M toluene solution, 0.406 mL, 0.406 mmol) at -78°C, and the mixture was stirred for 15 minutes at 0°C. A saturated aqueous Rochelle salt solution and ethyl acetate were added to the reaction mixture and stirring was continued, after which the mixture was extracted with ethyl acetate. The organic layer was washed with brine and the organic layer was dried over sodium sulfate. After filtration, the filtrate was concentrated under reduced pressure to obtain a crude product. To a solution of the obtained crude product and 1,3-dimethyl-5-(4,4,5,5-tetramethyl-1,3,2-dioxaborolan-2-yl)pyridin-2(1H)-one (69.0 mg, 0.277 mmol) in toluene (1 mL) there were added ethanol (0.25 mL), sodium carbonate (2 M aqueous solution, 0.5 mL, 1.00 mmol) and tetrakis(triphenylphosphine)palladium(0) (21.3 mg, 0.018 mmol) at room temperature, and the mixture was stirred for 18 hours at 70°C. The reaction mixture was filtered with Celite and washed with ethyl acetate. The filtrate was concentrated under reduced pressure and the residue was purified by NH silica gel column chromatography (ethyl acetate to ethyl acetate:methanol = 9: 1) to obtain the title compound (59.7 mg).

ESI-MS (m/z): 541.23 [M+H]$^+$.

[Example 19]

5-(2-((Dimethyl(oxo)-16-sulfanylidene)amino)-4-(2-hydroxy-1-isopropoxy-1-phenylethyl)quinazolin-6-yl)-1,3-dimethylpyridin-2(1H)-one

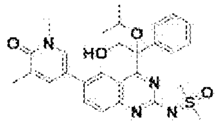

To a solution of the 5-(2-((dimethyl(oxo)-16-sulfanylidene)amino)-4-(1-isopropoxy-1-phenyl-2-((tetrahydro-2H-pyran-2-yl)oxy)ethyl)quinazolin-6-yl)-1,3-dimethylpyridin-2(1H)-one of Production Example 19-5 (32 mg) in methanol (2 mL) there was added*p*-toluenesulfonic acid (20 mg) at 0°C, and the mixture was stirred for 1 hour at room temperature. A saturated aqueous sodium hydrogencarbonate solution was added to the reaction mixture, and extraction was performed with dichloromethane. The organic layer was dried over sodium sulfate and filtered. The filtrate was concentrated under reduced pressure and the residue was purified by NH silica gel column chromatography to obtain a racemic mixture of the title compound. The obtained racemic mixture was purified by SFC (IG, carbon dioxide:methanol = 80:20) to obtain the title compound (6.63 mg) as the former isomer.

$^1$H-NMR Spectrum (400 MHz, $CDCl_3$) δ(ppm): 0.58-0.69 (3H, m), 1.30-1.37 (3H, m), 2.16 (3H, s), 3.52 (6H, s), 3.53 (3H, s), 3.65-3.78 (1H, m), 3.99-4.15 (1H, m), 4.24-4.35 (1H, m), 4.36-4.51 (1H, m), 6.85-6.99 (1H, m), 7.06-7.15 (1H, m), 7.06-7.15 (1H, m), 7.23-7.32 (3H, m), 7.36-7.38 (2H, m), 7.56-7.67 (1H, m), 7.67-7.76 (1H, m), 7.84-7.96 (1H, m)

[Production Example 19-1]

Ethyl 2-isopropoxy-2-phenylacetate

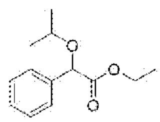

To a solution of propan-2-ol (1.58 g, 26.3 mmol) and rhodium(II) acetate dimer (58 mg, 0.131 mmol) in dichloromethane (10 mL) there was added dropwise a solution of ethyl 2-diazo-2-phenylacetate (1.00 g, 5.26 mmol) in dichloromethane (10 mL) at room temperature, and the mixture was stirred for 2 hours at room temperature. The reaction mixture was concentrated under reduced pressure and the residue was purified by silica gel column chromatography (*n*-heptane:ethyl acetate = 99:1 to *n*-heptane:ethyl acetate = 4:1) to obtain the title compound (1.00 g).

$^1$H-NMR Spectrum (400 MHz, $CDCl_3$) δ(ppm): 0.86-0.88 (3H, m), 1.18-1.25 (6H, m), 3.62-3.73 (1H, m), 4.10-4.24 (2H, m), 4.91-4.99 (1H, m), 7.30-7.36 (3H, m), 7.44-7.46 (2H, m).

[Production Example 19-2]

Ethyl 2-(2-chloro-6-iodoquinazolin-4-yl)-2-isopropoxy-2-phenylacetate

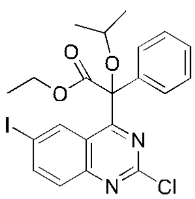

To a solution of the ethyl 2-isopropoxy-2-phenylacetate of Production Example 19-1 (1.23 g, 5.54 mmol) and 2,4-dichloro-6-iodoquinazoline (1.2 g, 3.69 mmol) in THF (20 mL) there was added dropwise LHMDS (1 M THF solution, 3.69

mL, 3.69 mmol) at -78°C, and the mixture was stirred for 1.5 hours at room temperature. Saturated aqueous ammonium chloride was added to the reaction mixture, and extraction was performed with ethyl acetate. The organic layer was washed with brine. The organic layer was dried over sodium sulfate and filtered. The filtrate was concentrated under reduced pressure and the residue was purified by silica gel column chromatography (n-heptane:ethyl acetate = 99:1 to *n*-heptane:ethyl acetate = 4:1) to obtain the title compound (1.15 g).
ESI-MS (m/z): 511.24 $[M+H]^+$.

[Production Example 19-3]

2-Chloro-6-iodo-4-(1-isopropoxy-1-phenyl-2-((tetrahydro-2H-pyran-2-yl)oxy)ethyl)quinazoline

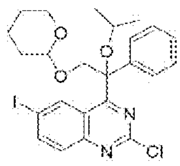

To a solution of the ethyl 2-(2-chloro-6-iodoquinazolin-4-yl)-2-isopropoxy-2-phenylacetate of Production Example 19-2 (1.15 g, 2.25 mmol) in toluene (20 mL) there was added dropwise DIBAL-H (1 M toluene solution, 7.88 mL, 7.88 mmol) at -78°C, and the mixture was stirred for 30 minutes at 0°C. A saturated aqueous Rochelle salt solution and ethyl acetate were added, and stirring was continued. After oil-water distribution of the reaction mixture, the organic layer was dried over sodium sulfate. The organic layer was filtered, and the filtrate was then concentrated under reduced pressure to obtain a crude product. To a solution of the obtained crude product in dichloromethane (20 mL) there were added DHP (758 mg, 9.01 mmol) and p-toluenesulfonic acid monohydrate (86 mg, 0.45 mmol) at room temperature, and the mixture was stirred for 4 hours at room temperature. A saturated aqueous sodium hydrogencarbonate solution was added to the reaction mixture, and extraction was performed with dichloromethane. The organic layer was dried over sodium sulfate and filtered, and the filtrate was concentrated under reduced pressure. The residue was purified by silica gel column chromatography (n-heptane:ethyl acetate = 99:1 to *n*-heptane:ethyl acetate = 7:3) to obtain the title compound (980 mg).
ESI-MS (m/z): 553.26 $[M+H]^+$.

[Production Example 19-4]

5-(2-Chloro-4-(1-isopropoxy-1-phenyl-2-((tetrahydro-2H-pyran-2-yl)oxy)ethyl)quinazolin-6-yl)-1,3-dimethylpyridin-2(1H)-one

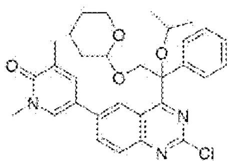

To a solution of the 2-chloro-6-iodo-4-(1-isopropoxy-1-phenyl-2-((tetrahydro-2H-pyran-2-yl)oxy)ethyl)quinazoline of Production Example 19-3 (980 mg, 1.77 mmol) and 1,3-dimethyl-5-(4,4,5,5-tetramethyl-1,3,2-dioxaborolan-2-yl)pyridin-2(1H)-one (486 mg, 1.95 mmol) in toluene (12 mL) there were added ethanol (3 mL), sodium carbonate (2 M aqueous solution, 1.77 mL, 3.54 mmol) and tetrakis(triphenylphosphine)palladium(0) (205 mg, 0.177 mmol) at room temperature, and the mixture was stirred for 12 hours at 70°C. The reaction mixture was returned to room temperature, water was added, and extraction was performed with ethyl acetate. The organic layer was washed with brine. The organic layer was dried over sodium sulfate and filtered. The filtrate was concentrated under reduced pressure and the residue was purified by NH silica gel column chromatography (n-heptane:ethyl acetate = 9:1 to n-heptane:ethyl acetate = 35:65) to obtain the title compound (706 mg).
ESI-MS (m/z): 548.40 $[M+H]^+$.

[Production Example 19-5]

5-(2-((Dimethyl(oxo)-16-sulfanylidene)amino)4-(1-isopropoxy-1-phenyl-2-((tetrahydro-2H-pyran-2-yl)oxy)ethyl)quinazolin-6-yl)-1,3-dimethylpyridin-2(1H)-one

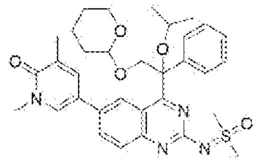

To a solution of the 5-(2-chloro-4-(1-isopropoxy-1-phenyl-2-((tetrahydro-2H-pyran-2-yl)oxy)ethyl)quinazolin-6-yl)-1,3-dimethylpyridin-2(1H)-one of Production Example 19-4 (80.0 mg, 0.146 mmol) in 1,4-dioxane (2 mL) there were added dimethylsulfoximine (20.4 mg, 0.219 mmol), cesium carbonate (95.0 mg, 0.292 mmol), BRETTPHOS (15.7 mg, 0.029 mmol) and tris(dibenzylideneacetone)palladium (13.4 mg, 0.015 mmol) at room temperature, and the mixture was stirred for 2 hours at 80°C. The reaction mixture was returned to room temperature and the insolubles were filtered out, after which the filtrate was concentrated under reduced pressure. The residue was purified by NH silica gel column chromatography (n-heptane:ethyl acetate = 1:9 to ethyl acetate to ethyl acetate:methanol = 95:5) to obtain the title compound (32 mg).
ESI-MS (m/z): 605.45 $[M+H]^+$.

[Example 20]

5-(2-Amino-4-(1-cyclopropyl-3-hydroxy-2-(pyridin-3-yl)propan-2-yl)quinazolin-6-yl)-1,3-dimethylpyridin-2(1H)-one

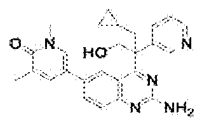

To a solution of the 5-(2-amino-4-(1-cyclopropyl-2-(pyridin-3-yl)-3-((tetrahydro-2H-pyran-2-yl)oxy)propan-2-yl) quinazolin-6-yl)-1,3-dimethylpyridin-2(1H)-one of Production Example 20-3 (25 mg, 47 μmol) in ethanol (2 mL) there was added 2 M hydrochloric acid (ethanol solution, 1 mL) at 0°C, and the mixture was stirred for 1 hour at room temperature. A saturated aqueous sodium hydrogencarbonate solution was added to the reaction mixture, and extraction was performed with dichloromethane. The organic layer was dried over sodium sulfate and filtered. The filtrate was concentrated under reduced pressure and the residue was purified by NH silica gel column chromatography (ethyl acetate to ethyl acetate:methanol = 85:15) to obtain the title compound (16 mg).
$^1$H-NMR Spectrum (400 MHz, $CDCl_3$) δ(ppm): -0.49-0.47 (1H, m), -0.01-0.08 (2H, m), 0.34-0.40 (1H, m), 0.46-0.55 (1H, m), 2.13 (3H, s), 2.33 (1H, dd, J=6.8, 13.6 Hz), 2.47 (1H, dd, J=5.8, 13.6 Hz), 3.51 (3H, s), 3.91 (1H, d, J=11.5 Hz), 4.67 (1H, d, J=11.5 Hz), 5.28 (2H, s), 6.77 (1H, d, J=2.9 Hz), 6.89-6.90 (1H, m), 7.05 (1H, brs), 7.30 (1H, dd, J=4.9, 8.0 Hz), 7.54 (1H, dt, J=1.8,1.8, 8.0 Hz), 7.59 (2H, d, J=1.0 Hz), 8.53 (1H, d, J=2.0 Hz), 8.59 (1H, dd, J=1.5, 4.9 Hz)
ESI-MS (m/z): 442.36 $[M+H]^+$.

[Production Example 20-1]

Ethyl 2-(2-chloro-6-iodoquinazolin-4-yl)-3-cyclopropyl-2-(pyridin-3-yl)propanoate

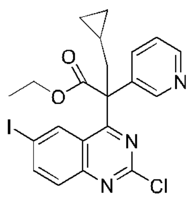

To a solution of ethyl 2-(pyridin-3-yl)acetate (1.00 g, 6.05 mmol) and (bromomethyl)cyclopropane (899 mg, 6.66 mmol) in DMF (10 mL) there was added dropwise a solution of potassium *tert*-butoxide (1.02 g, 9.08 mmol) in DMF (10 mL) at 0°C, and the mixture was stirred for 30 minutes at 0°C. Water was added to the reaction mixture, extraction was performed with ethyl acetate, and the organic layer was dried over sodium sulfate and filtered. After concentration under reduced pressure, the residue was purified by silica gel column chromatography (n-heptane:ethyl acetate = 9:1 to 2:1) to obtain a crude product (760 mg). To a solution of the obtained crude product (506 mg) and 2,4-dichloro-6-iodoquinazoline (750 mg, 2.31 mmol) in THF (15 mL) there was added dropwise LHMDS (1 M THF solution, 3.00 mL, 3.00 mmol) at -78°C, and the mixture was stirred for 1 hour at room temperature. Saturated aqueous ammonium chloride was added to the reaction mixture, and extraction was performed with ethyl acetate. The organic layer was washed with brine. The organic layer was dried over sodium sulfate and filtered. The filtrate was concentrated under reduced pressure and the residue was purified by silica gel column chromatography (*n*-heptane:ethyl acetate = 95:5 to 4:1) to obtain the title compound (1.08 g).

$^1$H-NMR Spectrum (400 MHz, $CDCl_3$) $\delta$(ppm): -0.20--0.13 (2H, m), -0.27-0.38 (2H, m) 0.20-0.40 (3H, s), 0.45-0.50 (1H, m) 1.14-1.18 (3H, m), 1.97 (1H, dd, J=6.8, 14.2 Hz), 2.10 (1H, dd, J=5.8, 14.2 Hz), 4.05 -4.16 (2H, m), 7.18-7.25 (1H, m), 7.63-7.65 (1H, m), 7.94-8.01 (2H, m), 8.42-8.55 (3H, m)

[Production Example 20-2]

2-Chloro-4-(1-cyclopropyl-2-(pyridin-3-yl)-3-((tetrahydro-2H-pyran-2-yl)oxy)propan-2-yl)-6-iodoquinazoline

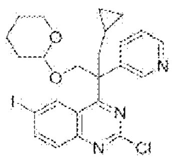

To a solution of the ethyl 2-(2-chloro-6-iodoquinazolin-4-yl)-3-cyclopropyl-2-(pyridin-3-yl)propanoate of Production Example 20-1 (720 mg, 1.42 mmol) in toluene (10 mL) there was added dropwise DIBAL-H (1 M toluene solution, 4.96 mL, 4.96 mmol) at -78°C, and the mixture was stirred for 30 minutes at 0°C. A saturated aqueous Rochelle salt solution and ethyl acetate were added to the reaction mixture, and stirring was continued. After oil-water distribution of the reaction mixture, the organic layer was dried over sodium sulfate. After filtration, the filtrate was concentrated under reduced pressure. The residue was purified by silica gel column chromatography (n-heptane:ethyl acetate = 3:1 to 1:4) to obtain a crude product. To a solution of the obtained crude product in dichloromethane (6 mL) there were added DHP (716 mg, 8.51 mmol) andp-toluenesulfonic acid monohydrate (135 mg, 0.709 mmol) at room temperature, and the mixture was stirred for 4 hours at room temperature. A saturated aqueous sodium hydrogencarbonate solution and dichloromethane were added, and the mixture was stirred. After oil-water distribution of the reaction mixture, the organic layer was dried over sodium sulfate. The organic layer was filtered, and the filtrate was then concentrated under reduced pressure. The residue was purified by silica gel column chromatography (n-heptane:ethyl acetate = 4:1 to 1:2) to obtain the title compound (600 mg).

ESI-MS (m/z): 550.25 $[M+H]^+$.

[Production Example 20-3]

5-(2-Chloro-4-(1-cyclopropyl-2-(pyridin-3-yl)-3-((tetrahydro-2H-pyran-2-yl)oxy)propan-2-yl)quinazolin-6-yl)-1,3-dimethylpyridin-2(1H)-one

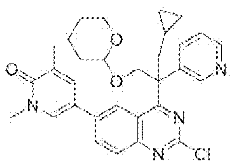

To a solution of the 2-chloro-4-(1-cyclopropyl-2-(pyridin-3-yl)-3-((tetrahydro-2H-pyran-2-yl)oxy)propan-2-yl)-6-iodoquinazoline of Production Example 20-2 (70.0 mg, 0.127 mmol) and 1,3-dimethyl-5-(4,4,5,5-tetramethyl-1,3,2-dioxaborolan-2-yl)pyridin-2(1H)-one (34.9 mg, 0.140 mmol) in toluene (2.0 mL) there were added ethanol (0.5 mL),

sodium carbonate (2 M aqueous solution, 0.127 mL, 0.225 mmol) and tetrakis(triphenylphosphine)palladium(0) (14.7 mg, 0.013 mmol) at room temperature, and the mixture was stirred for 3 hours at 75°C under a nitrogen atmosphere. The reaction mixture was returned to room temperature, water was added, and extraction was performed with ethyl acetate. The organic layer was dried over sodium sulfate and filtered. The filtrate was concentrated under reduced pressure and the residue was purified by NH silica gel column chromatography (n-heptane:ethyl acetate = 3:2 to ethyl acetate) to obtain the title compound (40.5 mg).

ESI-MS (m/z): 545.38 $[M+H]^+$.

[Production Example 20-4]

5-(2-Amino-4-(1-cyclopropyl-2-(pyridin-3-yl)-3-((tetrahydro-2H-pyran-2-yl)oxy)propan-2-yl)quinazolin-6-yl)-1,3-dimethylpyridin-2(1H)-one

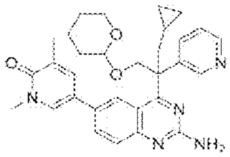

To a solution of the 5-(2-chloro-4-(1-cyclopropyl-2-(pyridin-3-yl)-3-((tetrahydro-2H-pyran-2-yl)oxy)propan-2-yl) quinazolin-6-yl)-1,3-dimethylpyridin-2(1H)-one of Production Example 20-3 (40.5 mg, 0.074 mmol) in NMP (0.7 mL) there was added a 28% ammonia water solution (0.5 mL, 7.48 mmol) at room temperature, and the mixture was stirred for 8 hours in a sealed tube at 90°C. The reaction mixture was returned to room temperature, water was added, and extraction was performed with ethyl acetate. The organic layer was dried over sodium sulfate and filtered. The filtrate was concentrated under reduced pressure, and the residue was purified by NH silica gel column chromatography (n-heptane:ethyl acetate = 2:3 to ethyl acetate to methanol:ethyl acetate = 5:95) to obtain the title compound (24.5 mg).

ESI-MS (m/z): 526.44 $[M+H]^+$.

[Example 21]

5-(2-(((Dimethyl(oxo)-16-sulfanylidene)amino)-4-(1-(3-fluoro-4-methoxyphenyl)-3-hydroxy-2-(pyridin-2-yl)propan-2-yl) quinazolin-6-yl)-1,3-dimethylpyridin-2(1H)-one

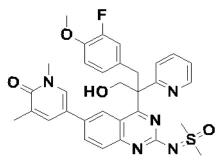

To a solution of the 5-(2-((dimethyl(oxo)-16-sulfanylidene)amino)-4-(1-(3-fluoro-4-methoxyphenyl)-2-(pyridin-2-yl)-3-((tetrahydro-2H-pyran-2-yl)oxy)propan-2-yl)quinazolin-6-yl)-1,3-dimethylpyridin-2(1H)-one of Production Example 21-3 (30 mg, 44 μmol) in ethanol (2 mL) there was added 2 M hydrochloric acid (ethanol solution, 1 mL) at 0°C, and the mixture was stirred for 2 hours at room temperature. A saturated aqueous sodium hydrogencarbonate solution was added to the reaction mixture, and extraction was performed with dichloromethane. The organic layer was dried over sodium sulfate and filtered. The filtrate was concentrated under reduced pressure and the residue was purified by NH silica gel column chromatography (ethyl acetate to ethyl acetate:methanol = 9:1) to obtain the title compound (15 mg).

$^1$H-NMR Spectrum (400 MHz, $CDCl_3$) δ(ppm): 2.17 (3H, s), 3.02 (3H, s), 3.28 (3H, s), 3.55 (3H, s), 3.72 (3H, s), 3.80-3.92 (4H, m), 4.67 (1H, dd, J=2.0, 8.8 Hz), 6.21-6.23 (1H, m), 6.41 (1H, dd, J=2.2, 12.5 Hz), 6.54 (1H, t, J=8.8 Hz), 6.92-6.98 (3H, m), 7.10 (1H, d, J=1.9 Hz), 7.31 (1H, ddd, J=1.0,4.9, 7.3 Hz), 7.55 (1H, dt, J=1.9,7.8, 7.8 Hz), 7.66 (1H, dd, J=1.9, 8.8 Hz), 7.77-7.86 (1H, m), 8.79-8.80 (1H, m)
ESI-MS (m/z): 602.42 $[M+H]^+$.

[Production Example 21-1]

Ethyl 3-(3-fluoro-4-methoxyphenyl)-2-(pyridin-2-yl)propanoate

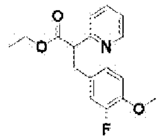

To a solution of ethyl 2-(pyridin-2-yl)acetate (500 mg, 3.03 mmol) in DMF (10 mL) there was added 50% to 72% sodium hydride oil (127 mg) at 0°C, and the mixture was stirred for 10 minutes at 0°C. A solution of 4-(chloromethyl)-2-fluoro-1-methoxybenzene (528 mg, 3.03 mmol) in DMF (2 mL) was added dropwise to the reaction mixture, which was then stirred for 4 hours at room temperature. Water was added to the reaction mixture at 0°C, extraction was performed with ethyl acetate, and the organic layer was dried over sodium sulfate and filtered. The filtrate was concentrated under reduced pressure and the residue was purified by silica gel column chromatography (n-heptane:ethyl acetate = 7:3 to 35:65) to obtain the title compound (649 mg).

ESI-MS (m/z): 304.13 $[M+H]^+$.

[Production Example 21-2]

2-Chloro-4-(1-(3-fluoro-4-methoxyphenyl)-2-(pyridin-2-yl)-3-((tetrahydro-2H-pyran-2-yl)oxy)propan-2-yl)-6-iodoquinazoline

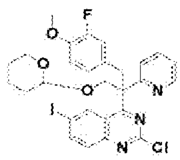

To a solution of the ethyl 3-(3-fluoro-4-methoxyphenyl)-2-(pyridin-2-yl)propanoate of Production Example 21-1 (652 mg, 2.15 mmol) and 2,4-dichloro-6-iodoquinazoline (698 mg, 2.15 mmol) in THF (20 mL) there was added dropwise LHMDS (1 M THF solution, 3.22 mL, 3.22 mmol) at -78°C under a nitrogen atmosphere, and the mixture was stirred for 3 hours at room temperature. Saturated aqueous ammonium chloride was added to the reaction mixture, and extraction was performed with ethyl acetate. The organic layer was washed with brine. The organic layer was dried over magnesium sulfate and filtered. The filtrate was concentrated under reduced pressure and the residue was purified by silica gel column chromatography (n-heptane to *n*-heptane:ethyl acetate = 4:1) to obtain a crude product. To a solution of the obtained crude product in toluene (20 mL) there was added dropwise DIBAL-H (1 M toluene solution, 4.04 mL, 4.04 mmol) at -78°C, and the mixture was stirred for 6 hours at 0°C. A saturated aqueous Rochelle salt solution and ethyl acetate were added, and stirring was continued. After oil-water distribution of the reaction mixture, the organic layer was dried over magnesium sulfate. After filtration, the filtrate was concentrated under reduced pressure. The residue was purified by silica gel column chromatography (*n*-heptane to *n*-heptane:ethyl acetate = 1:1) to obtain a crude product. To a solution of the obtained crude product in dichloromethane (10 mL) there were added DHP (318 mg, 3.78 mmol) and *p*-toluenesulfonic acid monohydrate (72 mg, 0.378 mmol) at room temperature, and the mixture was stirred for 12 hours at room temperature. A saturated aqueous sodium hydrogencarbonate solution and dichloromethane were added to the reaction mixture, which was then stirred. After oil-water distribution of the reaction mixture, the organic layer was dried over sodium sulfate. After filtration, the filtrate was concentrated under reduced pressure. The residue was purified by silica gel column chromatography (*n*-heptane to *n*-heptane:ethyl acetate = 7:3) to obtain the title compound (480 mg).
ESI-MS (m/z): 634.27 $[M+H]^+$

[Production Example 21-3]

5-(2-(((Dimethyl(oxo)-16-sulfanylidene)amino)-4-(1-(3-fluoro-4-methoxyphenyl)-2-(pyridin-2-yl)-3-((tetrahydro-2H-pyran-2-yl)oxy)propan-2-yl)quinazolin-6-yl)-1,3-dimethylpyridin-2(1H)-one

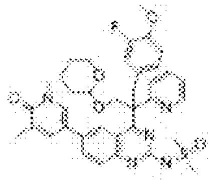

To a solution of the 2-chloro-4-(1-(3-fluoro-4-methoxyphenyl)-2-(pyridin-2-yl)-3-((tetrahydro-2H-pyran-2-yl)oxy)propan-2-yl)-6-iodoquinazoline of Production Example 21-2 (180 mg, 0.284 mmol) and 1,3-dimethyl-5-(4,4,5,5-tetramethyl-1,3,2-dioxaborolan-2-yl)pyridin-2(1H)-one (85 mg, 0.341 mmol) in toluene (4.0 mL) there were added ethanol (1.0 mL), sodium carbonate (2 M aqueous solution, 2.0 mL, 4.0 mmol) and tetrakis(triphenylphosphine)palladium(0) (16.4 mg, 0.014 mmol) at room temperature, and the mixture was stirred for 12 hours at 70°C. The reaction mixture was returned to room temperature, water was added, and extraction was performed with ethyl acetate. The organic layer was dried over magnesium sulfate and filtered. The filtrate was concentrated under reduced pressure and the residue was purified by NH silica gel column chromatography (ethyl acetate to methanol:ethyl acetate = 1:4) to obtain a crude product (141 mg). To a solution of a portion (44.3 mg) of the obtained crude product in 1,4-dioxane (2 mL) there were added dimethylsulfoximine (8.53 mg, 0.092 mmol), sodium *tert*-butoxide (13.5 mg, 0.141 mmol), BRETTPHOS (7.56 mg, 0.014 mmol) and tris(dibenzylideneacetone)dipalladium(0) (6.45 mg, 0.0070 mmol) at room temperature, and the mixture was stirred for 1 hour at 80°C. The reaction mixture was returned to room temperature and diluted with ethyl acetate, and then filtered with Celite. The filtrate was concentrated under reduced pressure, and the residue was purified by NH silica gel column chromatography (*n*-heptane:ethyl acetate = 3:1 to ethyl acetate to n-methanol: ethyl acetate = 5:95) to obtain the title compound (30 mg).

ESI-MS (m/z): 686.54 $[M+H]^+$.

[Example 22]

5-(2-((Dimethyl(oxo)-16-sulfanylidene)amino)-4-(1-hydroxy-4-methoxy-2-phenylbutan-2-yl)quinazolin-6-yl)-1,3-dimethylpyridin-2(1H)-one

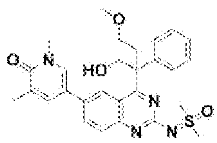

To a solution of the 5-(2-(((dimethyl(oxo)-16-sulfanylidene)amino)-4-(4-methoxy-2-phenyl-1-((tetrahydro-2H-pyran-2-yl)oxy)butan-2-yl)quinazolin-6-yl)-1,3-dimethylpyridin-2(1H)-one of Production Example 22-4 (27 mg, 0.045 mmol) in ethanol (1 mL) there was added 2 M hydrochloric acid (ethanol solution, 0.5 mL) at 0°C, and the mixture was stirred for 1.5 hours at room temperature. A saturated aqueous sodium hydrogencarbonate solution was added to the reaction mixture, and extraction was performed with dichloromethane. The organic layer was dried over sodium sulfate and filtered. The filtrate was concentrated under reduced pressure and the residue was purified by NH silica gel column chromatography (ethyl acetate to ethyl acetate:methanol = 9:1) to obtain the title compound (13 mg).

$^1$H-NMR Spectrum (400 MHz, $CDCl_3$) δ(ppm): 2.13 (3H, s), 2.72-2.81 (2H, m), 3.02-3.08 (1H, m), 3.10 (3H, s), 3.46-3.57(11H, m), 4.16 (1H, d, J=11.6 Hz), 4.26 (1H, d, J= 11.6 Hz), 6.77 (1H, d, J=2.4 Hz), 6.91-6.92 (1H, m), 7.21 (1H, d, J=2.0 Hz), 7.26-7.38 (5H, m), 7.60 (1H, dd, J=2.0, 8.8 Hz), 7.74 (1H, d, J=8.8 Hz)
ESI-MS (m/z): 521.32 $[M+H]^+$.

[Production Example 22-1]

Ethyl 2-(2-chloro-6-iodoquinazolin-4-yl)-4-methoxy-2-phenyl butanoate

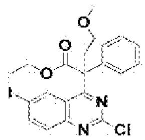

To a solution of ethyl phenylacetate (4.00 g, 24.4 mmol) in DMF (100 mL) there was added 50% to 72% sodium hydride oil (1.29 g) at 0°C, and the mixture was stirred for 30 minutes at 0°C. After adding 2-bromoethylmethyl ether (2.55 mL, 26.8 mmol) dropwise to the reaction mixture, it was stirred for 2 hours at 0°C. An ammonium chloride aqueous solution was added to the reaction mixture at 0°C, and then the mixture was extracted with ethyl acetate. The organic layer was dried over sodium sulfate and filtered to obtain a crude product (5.41 g). To a solution of the obtained crude product (1.58 g) and 2,4-dichloro-6-iodoquinazoline (2.1 g, 6.46 mmol) in THF (50 mL) there was added dropwise LHMDS (1 M THF solution, 9.69 mL, 9.69 mmol) at -78°C, and the mixture was stirred for 30 minutes at room temperature. Saturated aqueous ammonium chloride was added to the reaction mixture, and extraction was performed with ethyl acetate. The organic layer was washed with brine. The organic layer was dried over magnesium sulfate and filtered. The filtrate was concentrated under reduced pressure and the residue was purified by silica gel column chromatography (*n*-heptane to *n*-heptane:ethyl acetate = 4:1) to obtain the title compound (3.3 g).
$^1$H-NMR Spectrum (500 MHz, $CDCl_3$) $\delta$(ppm): 1.11 (3H, t, J=7.1 Hz), 2.79-2.87 (1H, m), 2.93-3.01 (1H, m), 3.15 (3H, s), 3.27-3.35 (1H, m), 3.45-3.52 (1H, m), 4.15 (2H, q, J= 7.1 Hz), 7.25-7.35 (3H, m), 7.39-7.44 (2H, m), 7.65 (1H, d, 8.8 Hz), 7.92-7.98 (2H, m)

[Production Example 22-2]

2-Chloro-6-iodo-4-(4-methoxy-2-phenyl-1-((tetrahydro-2H-pyran-2-yl)oxy)butan-2-yl)quinazoline

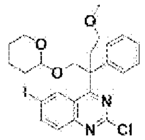

To a solution of the ethyl 2-(2-chloro-6-iodoquinazolin-4-yl)-4-methoxy-2-phenyl butanoate of Production Example 22-1 (1.80 g, 3.52 mmol) in toluene (20 mL) there was added dropwise DIBAL-H (1 M toluene solution, 10.6 mL, 10.6 mmol) at -78°C under a nitrogen atmosphere, and the mixture was stirred for 1 hour at room temperature. A saturated aqueous Rochelle salt solution was added to the reaction mixture, which was then stirred overnight. After oil-water distribution of the reaction mixture, the organic layer was dried over magnesium sulfate. The organic layer was filtered, and the filtrate was then concentrated under reduced pressure. The obtained residue was purified by silica gel column chromatography (*n*-heptane:ethyl acetate = 97:3 to 3:1) to obtain a crude product (1.30 g). To a solution of a portion (1.09 g) of the obtained crude product in THF (20 mL) there were added DHP (633 μL, 7.00 mmol) and p-toluenesulfonic acid monohydrate (665 mg, 3.50 mmol) at room temperature, and the mixture was stirred for 3 hours at 50°C. After further adding DHP (633 μL, 7.00 mmol) and *p*-toluenesulfonic acid monohydrate (665 mg, 3.50 mmol), the mixture was stirred for 6.5 hours at 50°C. DHP (2.00 mL, 22.1 mmol) was further added, and the mixture was stirred for 1 hour at 50°C. The reaction mixture was returned to room temperature, a saturated aqueous sodium hydrogencarbonate solution was added, and extraction was performed with ethyl acetate. The organic layer was dried over magnesium sulfate and filtered. The filtrate was concentrated under reduced pressure, and the residue was purified by silica gel column chromatography (n-heptane:ethyl acetate = 95:5 to 1:1) and then by silica gel column chromatography (n-heptane:ethyl acetate = 97:3 to 7:3), to obtain the title compound (646 mg).
ESI-MS (m/z): 553.14 [M+H]$^+$.

[Production Example 22-3]

5-(2-Chloro-4-(4-methoxy-2-phenyl-1-((tetrahydro-2H-pyran-2-yl)oxy)butan-2-yl)quinazolin-6-yl)-1,3-dimethyldipyridin-2(1H)-one

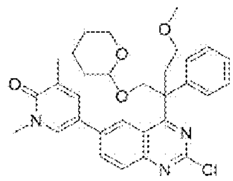

To a solution of the 2-chloro-6-iodo-4-(4-methoxy-2-phenyl-1-((tetrahydro-2H-pyran-2-yl)oxy)butan-2-yl)quinazoline of Production Example 22-2 (500 mg, 0.904 mmol) and 1,3-dimethyl-5-(4,4,5,5-tetramethyl-1,3,2-dioxaborolan-2-yl)pyridin-2(1H)-one (293 mg, 1.18 mmol) in toluene (8 mL) there were added ethanol (2 mL), sodium carbonate (2 M aqueous solution, 0.904 mL, 1.81 mmol) and tetrakis(triphenylphosphine)palladium(0) (105 mg, 0.09 mmol) at room temperature, and the mixture was stirred for 5 hours at 80°C. The reaction mixture was returned to room temperature, water was added, and extraction was performed with ethyl acetate. The organic layer was washed with brine. The organic layer was dried over magnesium sulfate and filtered. The filtrate was concentrated under reduced pressure and the residue was purified by NH silica gel column chromatography (n-heptane:ethyl acetate = 3:2 to ethyl acetate) to obtain the title compound (299 mg).

ESI-MS (m/z): 548.35 $[M+H]^+$.

[Production Example 22-4]

5-(2-((Dimethyl(oxo)-16-sulfanylidene)amino)-4-(4-methoxy-2-phenyl-1-((tetrahydro-2H-pyran-2-yl)oxy)butan-2-yl)quinazolin-6-yl)-1,3-dimethylpyridin-2(1H)-one

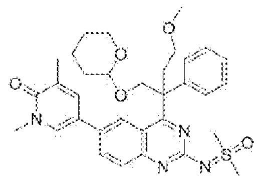

To a solution of the 5-(2-chloro-4-(4-methoxy-2-phenyl-1-((tetrahydro-2H-pyran-2-yl)oxy)butan-2-yl)quinazolin-6-yl)-1,3-dimethyldipyridin-2(1H)-one of Production Example 22-3 (50.0 mg, 0.091 mmol) in 1,4-dioxane (2 mL) there were added dimethylsulfoximine (12.8 mg, 0.137 mmol), sodium *tert*-butoxide (17.5 mg, 0.182 mmol), BRETTPHOS (9.8 mg, 0.018 mmol) and tris(dibenzylideneacetone)palladium (8.35 mg, 9.12 μmol) at room temperature, and the mixture was stirred for 3 hours at 90°C. The reaction mixture was then filtered and the filtrate was concentrated under reduced pressure. The residue was purified by NH silica gel column chromatography (n-heptane:ethyl acetate = 1:1 to ethyl acetate to methanol: ethyl acetate = 5:95) to obtain the title compound (27.3 mg).

ESI-MS (m/z): 605.42 $[M+H]^+$.

[Example 23]

5-(2-((Dimethyl(oxo)-16-sulfanylidene)amino)-4-(1-hydroxy-2,3-diphenylpropan-2-yl)quinazolin-6-yl)-1,3-dimethylpyridin-2(1H)-one

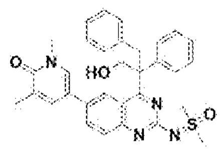

To a solution of the 5-(2-((dimethyl(oxo)-16-sulfanylidene)amino)-4-(1,2-diphenyl-3-((tetrahydro-2H-pyran-2-yl)oxy)propan-2-yl)quinazolin-6-yl)-1,3-dimethylpyridin-2(1H)-one of Production Example 23-5 (18 mg, 29 μmol) in ethanol (1 mL) there was added 2 M hydrochloric acid (ethanol solution, 1 mL) at 0°C, and the mixture was stirred for 2 hours at room temperature. A saturated aqueous sodium hydrogencarbonate solution was added to the reaction mixture, and extraction was performed with dichloromethane. The organic layer was dried over sodium sulfate and filtered. The filtrate

was concentrated under reduced pressure and the residue was purified by NH silica gel thin-layer chromatography (dichloromethane:methanol = 95:5) to obtain the title compound (4.6 mg).

$^1$H-NMR Spectrum (400 MHz, $CDCl_3$) δ(ppm): 2.15 (3H, s), 2.73 (3H, s), 3.16 (3H, s), 3.27-3.31 (1H, m), 3.52 (3H, d, J=2.0 Hz), 3.69-3.83 (3H, m), 4.63-4.68 (1H, m), 6.51-6.53 (2H, m), 6.76-6.77 (1H, m), 6.96-7.05 (4H, m), 7.33-7.46 (6H, m), 7.66 (1H, dt, J=1.5,1.5 8.8 Hz), 7.79 (1H, dd, J=2.0, 8.8 Hz)

ESI-MS (m/z): 553.36 $[M+H]^+$.

[Production Example 23-1]

Ethyl 2,3-diphenyl propanoate

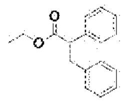

After adding sulfuric acid (800 μL, 15.0 mmol) to a solution of 2,3-diphenylpropionic acid (8.00 g, 35.4 mmol) in ethanol (80 mL), the mixture was stirred for 36 hours at 80°C. An aqueous sodium hydrogencarbonate solution was added to the reaction mixture at 0°C, and the mixture was filtered. The filtrate was concentrated under reduced pressure and the residue was purified by silica gel column chromatography (ethyl acetate) to obtain the title compound (9.5 g).
ESI-MS (m/z): 255.06 $[M+H]^+$

[Production Example 23-2]

Ethyl 2-(2-chloro-6-iodoquinazolin-4-yl)-2,3-diphenyl propanoate

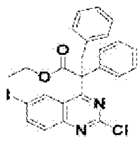

To a solution of the ethyl 2,3-diphenyl propanoate of Production Example 23-1 (9.0 g, 35.4 mmol) and 2,4-dichloro-6-iodoquinazoline (11.5 g, 35.4 mmol) in THF (300 mL) there was added dropwise LHMDS (1 M THF solution, 53.1 mL, 53.1 mmol) at -78°C, and the mixture was stirred for 2 hours at room temperature. Saturated aqueous ammonium chloride was added to the reaction mixture, and extraction was performed with ethyl acetate. The organic layer was washed with brine. The organic layer was dried over sodium sulfate and filtered. The filtrate was concentrated under reduced pressure and the resulting solid was filtered. The obtained residue was purified by silica gel column chromatography (n-heptane:ethyl acetate = 97:3 to 4:1) and the obtained solids were combined to obtain the title compound (16.3 g).
ESI-MS (m/z): 543.27 $[M+H]^+$

[Production Example 23-3]

2-Chloro-4-(1,2-diphenyl-3-((tetrahydro-2H-pyran-2-yl)oxy)propan-2-yl)-6-iodoquinazoline

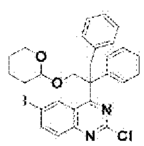

To a solution of the ethyl 2-(2-chloro-6-iodoquinazolin-4-yl)-2,3-diphenyl propanoate of Production Example 23-2 (16.3 g, 30.0 mmol) in toluene (400 mL) there was added dropwise DIBAL-H (1 M toluene solution, 90 mL, 90 mmol) at

-78°C, and the mixture was stirred for 30 minutes at 0°C. A saturated aqueous Rochelle salt solution and ethyl acetate were added, and stirring was continued. After oil-water distribution of the reaction mixture, the organic layer was dried over sodium sulfate. The organic layer was filtered, and the filtrate was then concentrated under reduced pressure to obtain a crude product. To a solution of the obtained crude product in dichloromethane (400 mL) there were added DHP (5.05 g, 60.1 mmol) andp-toluenesulfonic acid monohydrate (1.14 g, 60.1 mmol) at room temperature, and the mixture was stirred for 3 hours at room temperature. A saturated aqueous sodium hydrogencarbonate solution was added to the reaction mixture for oil-water distribution. After drying the organic layer over sodium sulfate and filtering, the filtrate was concentrated under reduced pressure and the obtained residue was purified by silica gel column chromatography (n-heptane:ethyl acetate = 20:1 to 4:1) to obtain the title compound (14.8 g).

ESI-MS (m/z): 585.30 $[M+H]^+$.

[Production Example 23-4]

5-(2-Chloro-4-(1,2-diphenyl-3-((tetrahydro-2H-pyran-2-yl)oxy)propan-2-yl)quinazolin-6-yl)-1,3-dimethyldipyridin-2(1H)-one

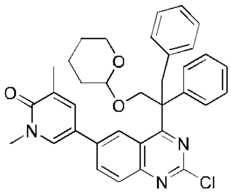

To a solution of the 2-chloro-4-(1,2-diphenyl-3-((tetrahydro-2H-pyran-2-yl)oxy)propan-2-yl)-6-iodoquinazoline of Production Example 23-3 (11.0 g, 18.8 mmol) and 1,3-dimethyl-5-(4,4,5,5-tetramethyl-1,3,2-dioxaborolan-2-yl)pyridin-2(1H)-one (5.62 g, 22.6 mmol) in toluene (160 mL) there were added ethanol (80 mL), sodium carbonate (3.99 g, 37.6 mmol), water (40 mL) and tetrakis(triphenylphosphine)palladium(0) (1.09 g, 0.94 mmol) at room temperature, and the mixture was stirred for 8 hours at 80°C. The reaction mixture was returned to room temperature, water was added, and extraction was performed with ethyl acetate. The organic layer was washed with brine. The organic layer was dried over sodium sulfate and filtered. The filtrate was concentrated under reduced pressure and the residue was purified by silica gel column chromatography (n-heptane:ethyl acetate = 7:3 to ethyl acetate) to obtain the title compound (9.9 g).

ESI-MS (m/z): 580.47 $[M+H]^+$.

[Production Example 23-5]

5-(2-((Dimethyl(oxo)-16-sulfanylidene)amino)-4-(1,2-diphenyl-3-((tetrahydro-2H-pyran-2-yl)oxy)propan-2-yl)quinazolin-6-yl)-1,3-dimethylpyridin-2(1H)-one

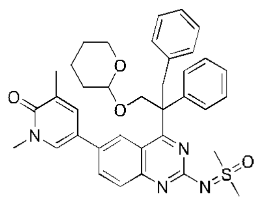

To a solution of the 5-(2-chloro-4-(1,2-diphenyl-3-((tetrahydro-2H-pyran-2-yl)oxy)propan-2-yl)quinazolin-6-yl)-1,3-dimethyldipyridin-2(1H)-one of Production Example 23-4 (40.0 mg, 0.069 mmol) in 1,4-dioxane (2 mL) there were added dimethylsulfoximine (8.35 mg, 0.09 mmol), sodium *tert*-butoxide (13.3 mg, 0.138 mmol), BRETTPHOS (7.4 mg, 0.014 mmol) and tris(dibenzylideneacetone)palladium (6.3 mg, 0.0069 mmol) at room temperature, and the mixture was stirred for 3 hours at 80°C. The reaction mixture was then filtered and the filtrate was concentrated under reduced pressure. The residue was purified by silica gel column chromatography (n-heptane:ethyl acetate = 3:1 to methanol:ethyl acetate = 1:9) to obtain the title compound (18.2 mg).

ESI-MS (m/z): 637.46 $[M+H]^+$.

[Example 24]

5-(2-Amino-4-(1-hydroxy-3-phenyl-2-(pyrimidin-2-yl)propan-2-yl)quinazolin-6-yl)-1,3-dimethylpyridin-2(1H)-one

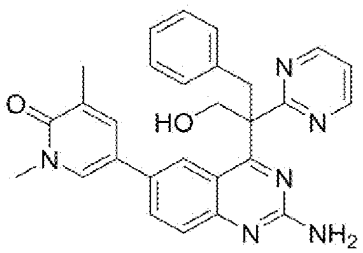

To a solution of the 5-(2-chloro-4-(1-phenyl-2-(pyrimidin-2-yl)-3-((tetrahydro-2H-pyran-2-yl)oxy)propan-2-yl) quinazolin-6-yl)-1,3-dimethylpyridin-2(1H)-one of Production Example 24-5 (10 mg, 0.017 mmol) in NMP (1000 μL) there was added a 28% ammonia water solution (322 μL), and the mixture was heated at 110°C in a sealed tube for 20 hours. After adding water at room temperature, extraction was performed with ethyl acetate. The organic layer was dried over anhydrous sodium sulfate. The mixture was filtered and the filtrate was concentrated under reduced pressure. The residue was dissolved in methanol (1000 μL), and then *p*-toluenesulfonic acid monohydrate (10 mg, 0.053 mmol) was added and the mixture was stirred for 30 minutes at room temperature. After adding a saturated sodium bicarbonate water solution to the reaction mixture, it was extracted 3 times with ethyl acetate. The organic layer was concentrated under reduced pressure. The residue was purified by NH silica gel column chromatography (n-heptane:ethyl acetate = 3:2 to ethyl acetate) to obtain the title compound (1.1 mg).
$^1$H-NMR Spectrum (500 MHz, $CDCl_3$) δ(ppm): 2.15 (3H, s), 3.55 (3H, m), 3.97 (2H, s), 4.22-4.31 (1H, m), 4.46-4.54 (1H, m), 5.03-5.13 (2H, m), 6.65-6.73 (2H, m), 6.80-6.85 (1H, m), 6.93-7.08 (6H, m), 7.54-7.61 (2H, m), 8.71-8.79 (2H, m).
ESI-MS (m/z): 479.38 $[M+H]^+$.

[Production Example 24-1]

Ethyl 3-phenyl-2-(pyrimidin-2-yl)propanoate

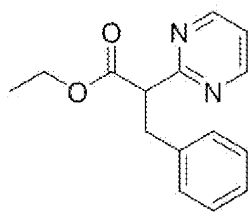

To a solution of 2-pyrimidineacetic acid ethyl ester (1.07 g, 6.43 mmol) in DMF (15 mL) there was added 50% to 72% sodium hydride oil (0.281 g) while cooling on ice. After 10 minutes, benzyl bromide (1.00 g, 5.85 mmol) was added dropwise and the mixture was stirred for 1 hour at room temperature. Saturated aqueous ammonium chloride was added to the reaction mixture and extraction was performed with ethyl acetate. The organic layer was washed with brine and dried over anhydrous sodium sulfate. The mixture was filtered and the filtrate was concentrated under reduced pressure. The residue was purified by silica gel column chromatography (n-heptane:ethyl acetate = 9:1 to 7:3) to obtain the title compound (900 mg).
ESI-MS (m/z): 257.24 $[M+H]^+$.

[Production Example 24-2]

Ethyl 2-(2-chloro-6-iodoquinazolin-4-yl)-3phenyl-2-(pyrimidin-2-yl)propanoate

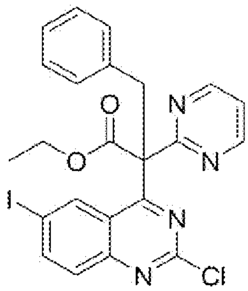

To a solution of 2,4-dichloro-6-iodoquinazoline (1000 mg, 3.08 mmol) and the ethyl 3-phenyl-2-(pyrimidin-2-yl) propanoate of Production Example 24-1 (868 mg, 3.39 mmol) in THF (30 mL) there was added dropwise lithium bis(trimethylsilyl)amide (1.17 M, 5.78 mL, 6.76 mmol) at -78°C. After stirring for 10 minutes at -78°C, the mixture was stirred for 3 hours at room temperature. Saturated ammonium chloride was added to the reaction mixture, and extraction was performed with ethyl acetate. The organic layer was washed with brine and dried over anhydrous sodium sulfate. The mixture was filtered and the filtrate was concentrated under reduced pressure to obtain a crude product of the title compound (1.6 g).

ESI-MS (m/z): 545.22 $[M+H]^+$.

[Production Example 24-3]

2-(2-Chloro-6-iodoquinazolin-4-yl)-3-phenyl-2-(pyrimidin-2-yl)propan-1-ol

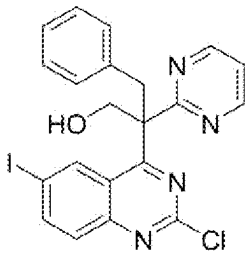

To a solution of a crude product of the ethyl 2-(2-chloro-6-iodoquinazolin-4-yl)-3-phenyl-2-(pyrimidin-2-yl) propanoate of Production Example 24-2 (1.6 g) in toluene (50 ml) there was added dropwise DIBAL-H (1 M toluene solution, 14.69 mL, 14.69 mmol) at -78°C, and then the temperature was increased to 0°C and the mixture was stirred for 10 minutes. A saturated aqueous Rochelle salt solution was added to the reaction mixture, and extraction was performed with ethyl acetate. The organic layer was washed with brine and dried over anhydrous sodium sulfate. The mixture was filtered, the filtrate was concentrated under reduced pressure and the residue was purified by silica gel column chromatography (n-heptane:ethyl acetate = 1:1) to obtain the title compound (140 mg).

$^1$H-NMR Spectrum (400 MHz, $CDCl_3$) $\delta$(ppm): 4.08-4.23 (4H, m), 4.32-4.37 (1H, m), 6.93-7.07 (6H, m), 7.11-7.17 (2H, m), 7.17-7.22 (1H, m), 7.59 (1H, d, J=8.56 Hz), 7.68 (1H, d, J=1.83 Hz), 7.89 (1H, m), 8.66 (2H, m).

ESI-MS (m/z): 503.23 $[M+H]^+$.

[Production Example 24-4]

2-Chloro-6-iodo-4-(1-phenyl-2-(pyrimidin-2-yl)-3-((tetrahydro-2H-pyran-2-yl)oxy)propan-2-yl)quinazoline

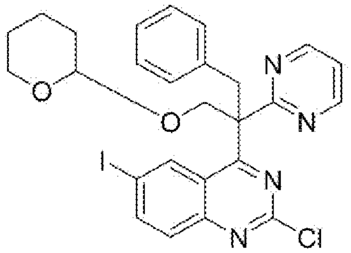

To a solution of the 2-(2-chloro-6-iodoquinazolin-4-yl)-3-phenyl-2-(pyrimidin-2-yl)propan-1-ol of Production Example 24-3 (140 mg, 0.278 mmol) in dichloromethane (3.00 mL) there were added DHP (50.9 μL, 0.557 mmol) andp-toluenesulfonic acid monohydrate (5.3 mg, 0.028 mmol), and the mixture was stirred. After adding saturated sodium bicarbonate water to the reaction mixture, it was concentrated under reduced pressure. The residue was purified by silica gel column chromatography (*n*-(heptane:ethyl acetate = 3:1) to obtain the title compound (80 mg).

ESI-MS (m/z): 587.26 $[M+H]^+$.

[Production Example 24-5]

5-(2-Chloro-4-(1-phenyl-2-(pyrimidin-2-yl)-3-((tetrahydro-2H-pyran-2-yl)oxy)propan-2-yl)quinazolin-6-yl)-1,3-dimethylpyridin-2(1H)-one

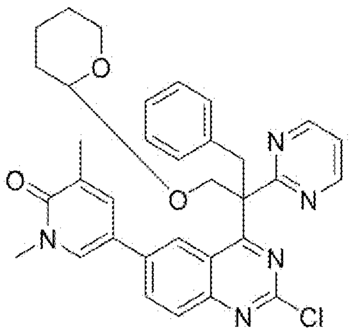

The 2-chloro-6-iodo-4-(1-phenyl-2-(pyrimidin-2-yl)-3-((tetrahydro-2H-pyran-2-yl)oxy)propan-2-yl)quinazoline of Production Example 24-4 (40 mg, 0.068 mmol) and 1,3-dimethyl-5-(4,4,5,5-tetramethyl-1,3,2-dioxaborolan-2-yl)pyridin-2(1H)-one (20.38 mg, 0.082 mmol) were dissolved in toluene (3000 μL), ethanol (500 μL) and water (100 μL), and then sodium carbonate (14.45 mg, 0.136 mmol) and tetrakistriphenylphosphinepalladium(0) (3.94 mg, 3.41 μmol) were added. The reaction mixture was stirred for 18 hours at 80°C under a nitrogen atmosphere, and then water was added at room temperature. The reaction mixture was extracted with ethyl acetate. The organic layer was washed with brine and dried over anhydrous sodium sulfate. The mixture was filtered with Celite, and the filtrate was concentrated under reduced pressure. The residue was purified by silica gel column chromatography (n-heptane:ethyl acetate = 7:3 to 0:1) to obtain the title compound (35 mg). ESI-MS (m/z): 582.45 $[M+H]^+$.

[Example 25]

5-(2-Amino-4-(1-cyclopropyl-3-hydroxy-2-(pyridin-3-yl)propan-2-yl)quinazolin-6-yl)-4-methoxy-1-methylpyridin-2(1H)-one

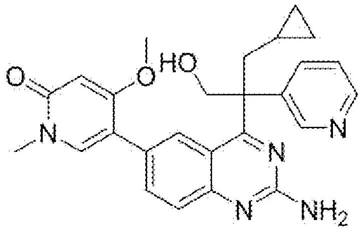

To a mixed solution of the formate of 5-(2-amino-4-(1-cyclopropyl-2-(pyridin-3-yl)-3-((tetrahydro-2H-pyran-2-yl)oxy)propan-2-yl)quinazolin-6-yl)-4-methoxy-1-methylpyridin-2(1H)-one of Production Example 25-2 (25.0 mg), dichloromethane (0.3 mL) and methanol (0.3 mL) there was added trifluoroacetic acid (0.3 mL), and the mixture was stirred for 1 hour at room temperature. Triethylamine (0.55 mL) was added to the reaction mixture at 0°C, and the reaction mixture was then subjected to fractionating purification (0.3% ammonia-containing distilled water:0.3% ammonia-containing acetonitrile = 4:1 to 3:2) to obtain the title compound (6.1 mg).

ESI-MS (m/z): 458.34 $[M+H]^+$.

[Production Example 25-1]

5-(2-Chloro-4-(1-cyclopropyl-2-(pyridin-3-yl)-3-((tetrahydro-2H-pyran-2-yl)oxy)propan-2-yl)quinazolin-6-yl)-4-methoxy-1-methylpyridin-2(1H)-one

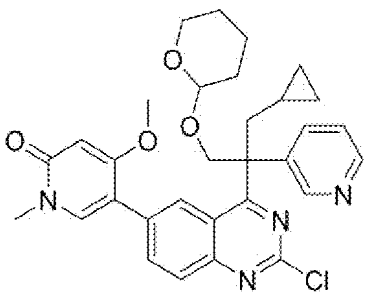

To a solution of the 2-chloro-4-(1-cyclopropyl-2-(pyridin-3-yl)-3-((tetrahydro-2H-pyran-2-yl)oxy)propan-2-yl)-6-iodoquinazoline of Production Example 20-2 (1.95 g, 3.55 mmol) and 4-methoxy-1-methyl-5-(4,4,5,5-tetramethyl-1,3,2-dioxaborolan-2-yl)pyridin-2(1H)-one (1.41 g, 5.32 mmol) in toluene (16 mL) there were added ethanol (8 mL), sodium carbonate (2 M aqueous solution, 4 mL, 8 mmol) and tetrakis(triphenylphosphine)palladium(0) (820 mg, 0.709 mmol) at room temperature, and the mixture was stirred for 4 hours at 75°C.

The reaction mixture was returned to room temperature, water was added, and extraction was performed with ethyl acetate. The organic layer was dried over sodium sulfate and filtered. The filtrate was concentrated under reduced pressure and the residue was purified by silica gel column chromatography (n-heptane to n-heptane:ethyl acetate = 1:1 to ethyl acetate) to obtain the title compound (1.33 g).

ESI-MS (m/z): 561.70 $[M+H]^+$.

[Production Example 25-2] 5-(2-Amino-4-(1-cyclopropyl-2-(pyridin-3-yl)-3-((tetrahydro-2H-pyran-2-yl)oxy)propan-2-yl)quinazolin-6-yl)-4-methoxy-1-methylpyridin-2(1H)-one

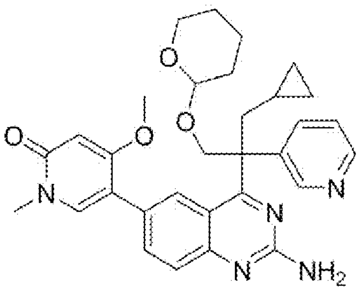

To a solution of the 5-(2-chloro-4-(1-cyclopropyl-2-(pyridin-3-yl)-3-((tetrahydro-2H-pyran-2-yl)oxy)propan-2-yl) quinazolin-6-yl)-4-methoxy-1-methylpyridin-2(1H)-one of Production Example 25-1 (50.0 mg, 0.089 mmol) in NMP (0.5 mL) there was added a 28% ammonia water solution (500 μL, 6.47 mmol) at room temperature, and the mixture was stirred for 3 hours at 100°C in a sealed tube using a microwave reactor. The reaction mixture was purified by ODS silica gel column chromatography (0.3% formic acid-containing distilled water:0.3% formic acid-containing acetonitrile = 9:1 to 0:10) to obtain a formate of the title compound (25.0 mg).
ESI-MS (m/z): 543.36 $[M+H]^+$.

[Example 26]

4-(4-(1-Cyclopropoxy-2-hydroxy-1-phenylethyl)-2-(4-ethynylpiperidin-1-yl)quinazolin-6-yl)-6-methyl-1,6-dihydro-7H-pyrrolo[2,3-c]pyridin-7-one

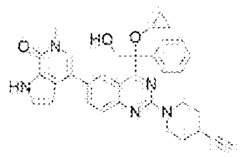

To a solution of the 4-(4-(1-cyclopropoxy-1-phenyl-2-((tetrahydro-2H-pyran-2-yl)oxy)ethyl)-2-(4-ethynylpiperidin-1-yl)quinazolin-6-yl)-6-methyl-1,6-dihydro-7H-pyrrolo[2,3-c]pyridin-7-one of Production Example 26-3 (30 mg, 0.047 mmol) in dichloromethane (1 mL) there was added trifluoroacetic acid (0.5 mL) at room temperature, and the mixture was stirred for 1 hour at room temperature. A saturated aqueous sodium hydrogencarbonate solution was added to the reaction mixture, and extraction was performed with dichloromethane. The organic layer was dried over anhydrous sodium sulfate and filtered, and the filtrate was concentrated under reduced pressure. The residue was purified by NH silica gel column chromatography (*n*-heptane:ethyl acetate = 7:3 to ethyl acetate to methanol:ethyl acetate = 15:85) to obtain the title compound (21 mg).
ESI-MS (m/z): 560.39 $[M+H]^+$.

[Production Example 26-1]

4-(2-Chloro-4-(1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one

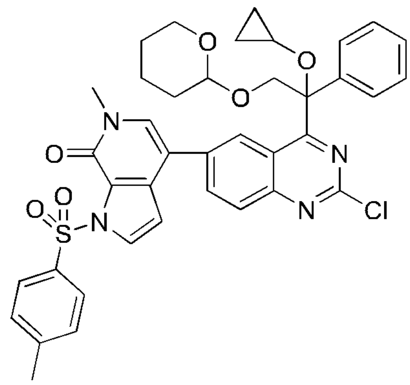

To a solution of the 2-chloro-4-(1-cyclopropoxy-1-phenyl-2-((tetrahydro-2H-pyran-2-yl)oxy)ethyl)-6-iodoquinazoline of Production Example 2-3 (2.90 g, 5.27 mmol) in 1,4-dioxane (24 mL) and water (6 mL) there was added sodium carbonate (1.12 g, 10.5 mmol), and then 6-methyl-4-(4,4,5,5-tetramethyl-1,3,2-dioxaborolan-2-yl)-1-tosyl-1,6-dihydro-7H-pyrrolo[2,3-c]pyridin-7-one (2.71 g, 6.32 mmol) and tetrakis(triphenylphosphine)palladium(0) (608 mg, 0.526 mmol) were added at room temperature and the mixture was stirred for 22 hours at 80°C under a nitrogen atmosphere. The reaction mixture was returned to room temperature, and extraction was performed with ethyl acetate. The organic layer was washed with brine, dried over anhydrous magnesium sulfate and filtered, and the filtrate was concentrated under reduced pressure. The residue was purified by silica gel column chromatography (n-heptane:ethyl acetate = 1:1 to *n*-heptane:ethyl acetate = 1:3) to obtain the title compound (3.08 g).
ESI-MS (m/z): 726.33 $[M+H]^+$.

[Production Example 26-2]

4-(4-(1-Cyclopropoxy-1-phenyl-2-((tetrahydro-2H-pyran-2-yl)oxy)ethyl)-2-(4-ethynylpiperidin-1-yl)quinazolin-6-yl)-6-methyl-1-tosyl-1,6-dihydro-7H-pyrrolo[2,3-c]pyridin-7-one

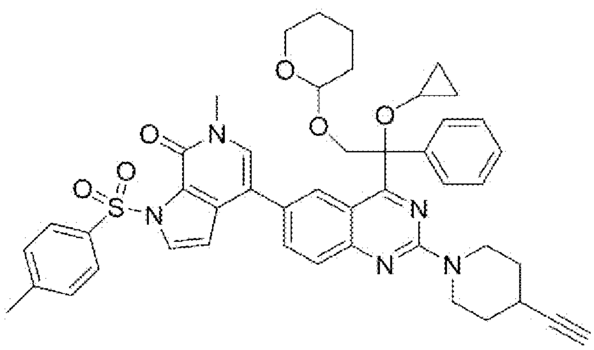

To a solution of the 4-(2-chloro-4-(1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 26-1 (500 mg, 0.689 mmol) in DMF (5 mL) there were added 4-ethynylpiperidine hydrochloride (201 mg, 1.38 mmol) and N,N-diisopropylethylamine (482 μL, 2.76 mmol) at room temperature, and the mixture was stirred for 3 hours at 80°C. The reaction mixture was returned to room temperature, water was added, and extraction was performed with ethyl acetate. The organic layer was washed with water and brine, dried over anhydrous sodium sulfate and filtered, and the filtrate was concentrated under reduced pressure. The residue was purified by silica gel column chromatography (n-heptane:ethyl acetate = 1:1) to obtain the title compound (550 mg).
ESI-MS (m/z): 798.22 $[M+H]^+$.

[Production Example 26-3]

4-(4-(1-Cyclopropoxy-1-phenyl-2-((tetrahydro-2H-pyran-2-yl)oxy)ethyl)-2-(4-ethynylpiperidin-1-yl)quinazolin-6-yl)-6-methyl-1,6-dihydro-7H-pyrrolo[2,3-c]pyridin-7-one

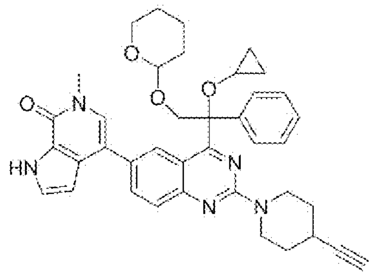

To a solution of the 4-(4-(1-cyclopropoxy-1-phenyl-2-((tetrahydro-2H-pyran-2-yl)oxy)ethyl)-2-(4-ethynylpiperidin-1-yl)quinazolin-6-yl)-6-methyl-1-tosyl-1,6-dihydro-7H-pyrrolo[2,3-c]pyridin-7-one of Production Example 26-2 (300 mg, 0.376 mmol) in ethanol (15 mL) there was added a 1 M sodium hydroxide aqueous solution (1.88 mL, 1.88 mmol) at room temperature, and the mixture was stirred for 1 hour and 30 minutes at 50°C. The reaction mixture was returned to room temperature and concentrated under reduced pressure. Water was added to the residue and extraction was performed with ethyl acetate. The organic layer was dried over anhydrous sodium sulfate and filtered, and then the filtrate was concentrated under reduced pressure to obtain the title compound (240 mg).

ESI-MS (m/z): 644.31 $[M+H]^+$.

[Example 27]

4-(2-(4-Amino-4-methyl-[1,4'-bipiperidin]-l'-yl)-4-(1-cyclopropoxy-2-hydroxy-1-phenylethyl)quinazolin-6-yl)-6-methyl-1,6-dihydro-7H-pyrrolo[2,3-c]pyridin-7-one

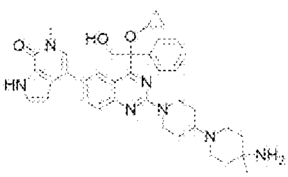

To a solution of the *tert-butyl* (1'-(4-(1-cyclopropoxy-1-phenyl-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)carbamate of Production Example 27-5 (106 mg, 0.127 mmol) in dichloromethane (3 mL) there was added TFA (3 mL) at room temperature, and the mixture was stirred for 40 minutes at room temperature. It was then concentrated under reduced pressure. A saturated aqueous sodium hydrogencarbonate solution was added to the residue, and extraction was performed with dichloromethane. The organic layer was dried over anhydrous magnesium sulfate and filtered, and the filtrate was concentrated under reduced pressure. The residue was purified by ODS column chromatography (0.1% formic acid-containing water:0.1% formic acid-containing acetonitrile = 95:5 to 1:1) to obtain a formate of the title compound (55 mg).

ESI-MS (m/z): 648.39 $[M+H]^+$.

[Production Example 27-1]

Benzyl 4-((*tert*-butoxycarbonyl)amino)-4-methyl-[1,4'-bipiperidine]-1'-carboxylate

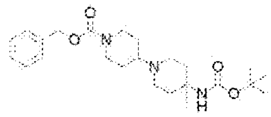

To a solution of *tert-butyl* (4-methylpiperidin-4-yl)carbamate (1286 mg, 6.00 mmol) in dichloromethane (10 mL) and acetic acid (172 μL, 3.00 mmol) there was added 1-(benzyloxycarbonyl)-4-piperidinone (700 mg, 3.00 mmol) at room temperature, and the mixture was stirred for 5 minutes at room temperature. Sodium triacetoxyborohydride (>80 wt%, 827 mg) was then added and the mixture was stirred for 19 hours and 25 minutes at room temperature. Water was added to the reaction mixture, and extraction was performed with ethyl acetate. The organic layer was washed with brine, dried over anhydrous magnesium sulfate and filtered, and the filtrate was concentrated under reduced pressure. The residue was purified by NH silica gel column chromatography (n-heptane:ethyl acetate = 6:4 to ethyl acetate) to obtain the title compound (941 mg).

ESI-MS (m/z): 432.41 $[M+H]^+$.

[Production Example 27-2]

*tert*-Butyl (4-methyl-[1,4'-bipiperidin]-4-yl)carbamate

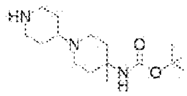

To a solution of the benzyl 4-((*tert*-butoxycarbonyl)amino)-4-methyl-[1,4'-bipiperidine]-1'-carboxylate of Production Example 27-1 (279 mg, 0.646 mmol) in methanol (10 mL) there was added 10% palladium-carbon (50% water, 138 mg) at room temperature, and the mixture was stirred for 2 hours and 25 minutes at room temperature and ordinary pressure, under a hydrogen atmosphere. After switching the reaction mixture to a nitrogen atmosphere, it was filtered with Celite and washed with ethyl acetate. The filtrate was concentrated under reduced pressure to obtain the title compound (192 mg).

ESI-MS (m/z): 298.30 $[M+H]^+$.

[Production Example 27-3]

*tert-Butyl* (1'-(4-(1-cyclopropoxy-1-phenyl-2-((tetrahydro-2H-pyran-2-yl)oxy)ethyl)-6-iodoquinazolin-2-yl)-4-methyl-[1,4'-bipiperidin]-4-yl)carbamate

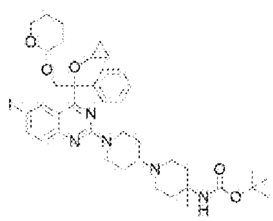

To a solution of the 2-chloro-4-(1-cyclopropoxy-1-phenyl-2-((tetrahydro-2H-pyran-2-yl)oxy)ethyl)-6-iodoquinazoline of Production Example 2-3 (100 mg, 0.182 mmol) in DMF (2 mL) there were added the *tert-butyl* (4-methyl-[1,4'-bipiperidin]-4-yl)carbamate (64.8 mg, 0.218 mmol) of Production Example 27-2 and N,N-diisopropylethylamine (63 μL, 0.36 mmol) at room temperature, and the mixture was stirred for 1 hour and 30 minutes at 80°C. The reaction mixture was returned to room temperature, and then water was added to halt the reaction and extraction was performed with ethyl acetate. The organic layer was washed with brine, dried over anhydrous magnesium sulfate and filtered, and the filtrate was concentrated under reduced pressure. The residue was purified by NH silica gel column chromatography (n-heptane:ethyl acetate = 8:2 to ethyl acetate) to obtain the title compound (132 mg).

ESI-MS (m/z): 812.34 $[M+H]^+$.

[Production Example 27-4]

*tert-Butyl* (1'-(4-(1-cyclopropoxy-1-phenyl-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)-4-methyl-[1,4'-bipiperidin]-4-yl)carbamate

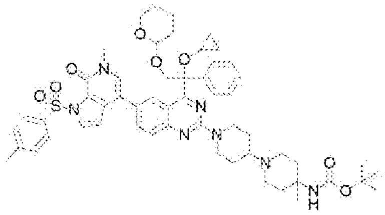

To a solution of the *tert-butyl* (1'-(4-(1-cyclopropoxy-1-phenyl-2-((tetrahydro-2H-pyran-2-yl)oxy)ethyl)-6-iodoquinazolin-2-yl)-4-methyl-[1,4'-bipiperidin]-4-yl)carbamate of Production Example 27-3 (131 mg, 0.161 mmol) in 1,4-dioxane (4 mL) and water (1 mL) there were added 6-methyl-4-(4,4,5,5-tetramethyl-1,3,2-dioxaborolan-2-yl)-1-tosyl-1,6-dihydro-7H-pyrrolo[2,3-c]pyridin-7-one (83 mg, 0.19 mmol), tetrakis(triphenylphosphine)palladium(0) (18.7 mg, 16 μmol) and cesium carbonate (79 mg, 0.24 mmol) at room temperature, and the mixture was stirred for 1 hour and 10 minutes at 80°C under a nitrogen atmosphere. Water was added to the reaction mixture, and extraction was performed with ethyl acetate. The organic layer was washed with brine, dried over anhydrous magnesium sulfate and filtered, and the filtrate was concentrated under reduced pressure. The residue was purified by NH silica gel column chromatography (n-heptane:ethyl acetate = 8:2 to ethyl acetate to ethyl acetate:methanol = 9: 1) to obtain the title compound (150 mg).
ESI-MS (m/z): 986.51 $[M+H]^+$.

[Production Example 27-5]

*tert-Butyl* (1'-(4-(1-cyclopropoxy-1-phenyl-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)carbamate

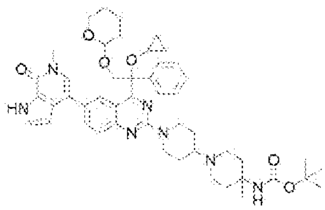

To a solution of the *tert-butyl* (1'-(4-(1-cyclopropoxy-1-phenyl-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)-4-methyl-[1,4'-bipiperidin]-4-yl)carbamate of Production Example 27-4 (149 mg, 0.151 mmol) in THF (4 mL) and methanol (2 mL) there was added a 1 M sodium hydroxide aqueous solution (1.51 mL, 1.51 mmol) at room temperature, and the mixture was stirred for 1 hour at 80°C. After returning the reaction mixture to room temperature, brine was added and extraction was performed with ethyl acetate. The organic layer was dried over anhydrous magnesium sulfate and filtered, and the filtrate was concentrated under reduced pressure. The residue was purified by NH silica gel column chromatography (n-heptane:ethyl acetate = 1:1 to ethyl acetate to ethyl acetate:methanol = 8:2) to obtain the title compound (107 mg).
ESI-MS (m/z): 832.46 $[M+H]^+$.

[Example 28]

4-(4-(2-Amino-1-cyclopropoxy-1-phenylethyl)-2-(4-(((2S,5R)-2,5-dimethylpiperazin-1-yl)methyl)piperidin-1-yl)quinazolin-6-yl)-6-methyl-1,6-dihydro-7H-pyrrolo[2,3-c]pyridin-7-one

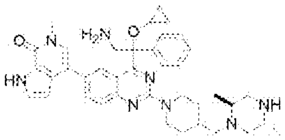

To a solution of a formate of the *tert-butyl* (2R,5 S)-4-((1-(4-(2-amino-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)methyl)-2,5-dimethylpiperazine-1-carboxylate of Production Example 28-6 (30 mg) in dichloromethane (0.5 mL) there was added TFA (0.5 mL) at room temperature, and the mixture was stirred for 90 minutes at room temperature. It was then concentrated under reduced pressure. A saturated aqueous sodium hydrogencarbonate solution was added to the residue, and extraction was performed with chloroform/isopropanol (4:1). The organic layer was dried over anhydrous magnesium sulfate and filtered, and the filtrate was concentrated under reduced pressure. The residue was purified by NH silica gel thin-layer chromatography (chloroform:methanol = 30:1) to obtain the title compound (21 mg). ESI-MS (m/z): 661.43 $[M+H]^+$.

[Production Example 28-1]

*tert*-Butyl (2R,5 S)-4-((1-((benzyloxy)carbonyl)piperidin-4-yl)methyl)-2,5-dimethylpiperazine-1-carboxylate

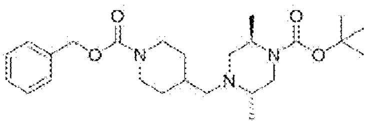

To a solution of *tert-butyl* (2R,5S)-2,5-dimethylpiperazine-1-carboxylate (3.90 g, 18.2 mmol) in dichloromethane (90 mL) and acetic acid (69 μL, 1.21 mmol) there was added 4-formyl-N-CBZ-piperidine (3.00 g, 12.1 mmol) at room temperature, and the mixture was stirred for 30 minutes at room temperature. Sodium triacetoxyborohydride (>80 wt%, 7.71 g) was then added and the mixture was stirred for 12 hours and 20 minutes at room temperature. A saturated aqueous sodium hydrogencarbonate solution was added to the reaction mixture, and extraction was performed with chloroform. The organic layer was dried over anhydrous magnesium sulfate and filtered, and the filtrate was concentrated under reduced pressure. The residue was purified by NH silica gel column chromatography (n-heptane:ethyl acetate = 4:1 to 1:1) to obtain the title compound (5.12 g).

ESI-MS (m/z): 446.37 $[M+H]^+$.

[Production Example 28-2]

*tert*-Butyl (2R,5S)-2,5-dimethyl-4-(piperidin-4-ylmethyl)piperazine-1-carboxylate

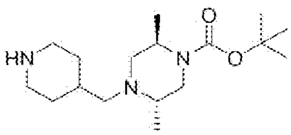

To a solution of the *tert-butyl* (2R,5S)-4-((1-((benzyloxy)carbonyl)piperidin-4-yl)methyl-2,5-dimethylpiperazine-1-carboxylate of Production Example 28-1 (5.12 g, 11.5 mmol) in THF (100 mL) there was added 10% palladium-carbon (50% water, 9.78 g) at room temperature, and the mixture was stirred for 18 hours at room temperature and ordinary pressure under a hydrogen atmosphere. After switching the reaction mixture to a nitrogen atmosphere, it was filtered with Celite and washed with methanol. The filtrate was concentrated under reduced pressure to obtain a crude product of the title compound (3.02 g).

[Production Example 28-3]

*tert*-Butyl (2R,5S)-4-((1-(4-(1-cyclopropoxy-1-phenyl-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)piperidin-4-yl)methyl)-2,5-dimethylpiperazine-1-carboxylate

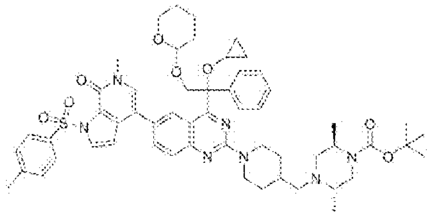

To a solution of the 4-(2-chloro-4-(1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 26-1 (585 mg, 0.807 mmol) and the *tert-butyl* (2R,SS)-2,5-dimethyl-4-(piperidin-4-ylmethyl)piperazine-1-carboxylate of Production Example 28-2 (352 mg) in DMF (5 mL) there was added N,N-diisopropylethylamine (352 μL, 2.02 mmol), and the mixture was stirred overnight at 80°C. Water was added to the reaction mixture, and extraction was performed with ethyl acetate. The organic layer was washed with water and brine. The organic layer was dried over anhydrous magnesium sulfate and filtered, and the filtrate was concentrated under reduced pressure. The residue was purified by NH silica gel column chromatography (n-heptane:ethyl acetate = 1:3 to ethyl acetate) to obtain the title compound (565 mg).
ESI-MS (m/z): 1000.73 $[M+H]^+$

[Production Example 28-4]

*tert*-Butyl (2R,5S)-4-((1-(4-(1-cyclopropoxy-2-hydroxy-1-phenylethyl)-6-(6-methyl-7-oxo-1-tosyl-6,7-dihydro-1H-pyrrolo[2,3-c]pyridin-4-yl)quinazolin-2-yl)piperidin-4-yl)methyl)-2,5-dimethylpiperazine-1-carboxylate

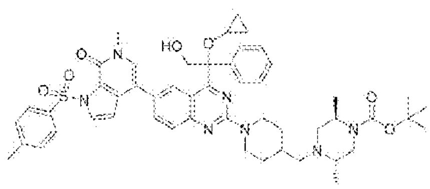

To a solution of the *tert-butyl* (2R,5S)-4-((1-(4-(1-cyclopropoxy-1-phenyl-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)piperidin-4-yl)methyl)-2,5-dimethylpiperazine-1-carboxylate of Production Example 28-3 (785 mg, 0.785 mmol) in methanol (9 mL) there was added*p*-toluenesulfonic acid monohydrate (373 mg, 1.96 mmol) at room temperature, and the mixture was stirred for 2 hours at room temperature. A saturated aqueous sodium hydrogencarbonate solution was added to the reaction mixture, and extraction was performed with ethyl acetate. The organic layer was dried over anhydrous magnesium sulfate and filtered, and the filtrate was concentrated under reduced pressure. The residue was purified by NH silica gel column chromatography (n-heptane:ethyl acetate = 1:1 to ethyl acetate) to obtain the title compound (678 mg).
ESI-MS (m/z): 917.83 $[M+H]^+$

[Production Example 28-5]

*tert*-Butyl (2R,5S)-4-((1-(4-(2-amino-1-cyclopropoxy-1-phenylethyl)-6-(6-methyl-7-oxo-1-tosyl-6,7-dihydro-1H-pyrrolo[2,3-c]pyridin-4-yl)quinazolin-2-yl)piperidin-4-yl)methyl)-2,5-dimethylpiperazine-1-carboxylate

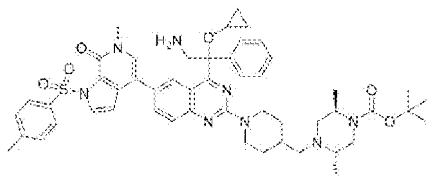

To a solution of the *tert-butyl* (2R,5S)-4-((1-(4-(1-cyclopropoxy-2-hydroxy-1-phenylethyl)-6-(6-methyl-7-oxo-1-tosyl-6,7-dihydro-1H-pyrrolo[2,3-c]pyridin-4-yl)quinazolin-2-yl)piperidin-4-yl)methyl)-2,5-dimethylpiperazine-1-carboxylate of Production Example 28-4 (150 mg, 0.164 mmol) in dichloromethane (1.5 mL) there were added triethylamine (68 μL, 0.491 mmol) and methanesulfonyl chloride (25 μL, 0.327 mmol) at room temperature, and the mixture was stirred for 10 minutes while cooling on ice and for 20 minutes at room temperature. Water was added to the reaction mixture, which was then extracted with dichloromethane. The organic layer was dried over anhydrous sodium sulfate and filtered, and the filtrate was concentrated under reduced pressure. To a solution of the residue in NMP (2 mL) there was added 28% ammonia water (1 mL) at room temperature, and a microwave reactor was used for reaction at 130°C for 45 minutes. The reaction mixture was purified by ODS silica gel column chromatography (acetonitrile (containing 0.1% formic acid):water (containing 0.1% formic acid) = 1:9 to 1:0) to obtain a formate of the title compound (124 mg).

ESI-MS (m/z): 915.67 $[M+H]^+$.

[Production Example 28-6]

*tert*-Butyl (2R,5S)-4-((1-(4-(2-amino-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)methyl)-2,5-dimethylpiperazine-1-carboxylate

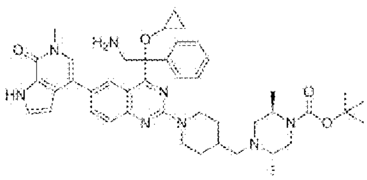

To a solution of the *tert-butyl* (2R,5S)-4-((1-(4-(2-amino-1-cyclopropoxy-1-phenylethyl)-6-(6-methyl-7-oxo-1-tosyl-6,7-dihydro-1H-pyrrolo[2,3-c]pyridin-4-yl)quinazolin-2-yl)piperidin-4-yl)methyl)-2,5-dimethylpiperazine-1-carboxylate of Production Example 28-5 (104 mg, 0.114 mmol) in THF (0.5 mL)/methanol (1 mL) there was added a 2 M sodium hydroxide aqueous solution (0.5 mL, 1.00 mmol), and the mixture was stirred for 2 hours at 60°C. The reaction mixture was returned to room temperature and diluted with water, and the resulting solid was filtered. The solid was washed with TBME and dried, to obtain a partially purified product of the title compound (74 mg).

ESI-MS (m/z): 761.58 $[M+H]^+$.

[Example 29]

4-(4-(1-Cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one

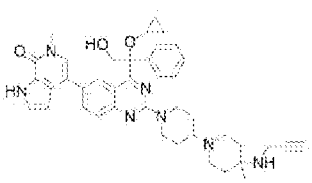

To a solution of the 4-(4-(1-cyclopropoxy-2-hydroxy-1-phenylethyl)-2-(4-methyl-4-(prop-2-yn-1-ylamino)-[1,4'-bipiperdin]-1'-yl)quinazolin-6-yl)-6-methyl-1-tosyl-1,6-dihydro-7H-pyrrolo[2,3-c]pyridin-7-one of Production Example 29-5 (569 mg, 0.677 mmol) in ethanol (13.8 mL) there was added a 1 M sodium hydroxide aqueous solution (2.71 mL, 2.71 mmol), and the mixture was stirred for 20 hours at room temperature. After diluting the reaction mixture with 5 mL

of water, it was extracted twice with a 10% methanol/chloroform solution (50 mL). The combined organic layers were washed with brine (10 mL), and then dried over sodium sulfate. The organic layer was filtered, and the filtrate was concentrated under reduced pressure. Addition of ethyl acetate (5 mL) to the residue produced a solid, which was filtered. The obtained solid was further washed with ethyl acetate (10 mL) to obtain the title compound (360 mg). A 5 mg portion was purified by NH silica gel thin-layer chromatography (methanol:ethyl acetate = 9:1) to obtain the title compound (4.3 mg). ESI-MS (m/z): 686.41 $[M+H]^+$.

[Production Example 29-1]

2-(2-Chloro-6-iodoquinazolin-4-yl)-2-cyclopropoxy-2-phenylethan-1-ol

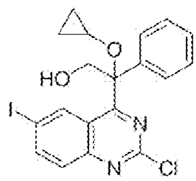

To a solution of the 2-chloro-4-(1-cyclopropoxy-1-phenyl-2-((tetrahydro-2H-pyran-2-yl)oxy)ethyl)-6-iodoquinazoline of Production Example 2-3 (1 g, 2 mmol) in dichloromethane (7 mL) there was added TFA (3 mL), and the mixture was stirred for 30 minutes at room temperature. The reaction mixture was concentrated under reduced pressure and purified by NH silica gel column chromatography (n-heptane:ethyl acetate = 9:1 to 7:3) to obtain the title compound (630 mg).
ESI-MS (m/z): 467.05 $[M+H]^+$.

[Production Example 29-2]

4-(2-Chloro-4-(1-cyclopropoxy-2-hydroxy-1-phenylethyl)quinazolin-6-yl)-6-methyl-1-tosyl-1,6-dihydro-7H-pyrrolo[2,3-c]pyridin-7-one

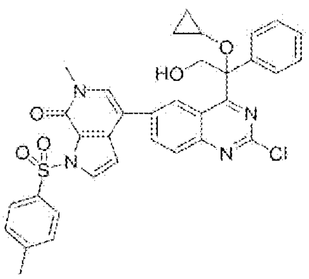

To a solution of the 2-(2-chloro-6-iodoquinazolin-4-yl)-2-cyclopropoxy-2-phenylethan-1-ol of Production Example 29-1 (600 mg, 1.29 mmol) and 6-methyl-4-(4,4,5,5,-tetramethyl-1,3,2-dioxaborolan-2-yl)-1-tosyl-1,6-dihydro-7H-pyrrolo[2,3-c]pyridin-7-one (578 mg, 1.35 mmol) in toluene (7.12 mL) there were added ethanol (1.80 mL), sodium carbonate (2 M aqueous solution, 3.54 mL, 7.07 mmol) and tetrakis(triphenylphosphine)palladium(0) (149 mg, 0.129 mmol) at room temperature, and the mixture was stirred for 40 hours at 70°C. Water and ethyl acetate were added to the reaction mixture, and extraction was performed with ethyl acetate. The organic layer was dried over sodium sulfate and filtered, and then concentrated under reduced pressure. The residue was purified by silica gel column chromatography (n-heptane:ethyl acetate = 3:1 to ethyl acetate to ethyl acetate:methanol = 9:1) to obtain the title compound (700 mg).
ESI-MS (m/z): 641.23 $[M+H]^+$.

[Production Example 29-3]

4-Methyl-N-(prop-2-yn-1-yl)piperidine-4-amine

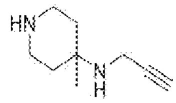

To a solution of *tert*-butyl 4-methyl-4-(prop-2-yn-1-ylamino)piperidine-1-carboxylate (10.0 g, 39.6 mmol) in dichloromethane (25 mL) there was added trifluoroacetic acid (25 mL), and the mixture was stirred for 1 hour at room temperature. The reaction mixture was concentrated under reduced pressure and the residue was purified by ODS silica gel column chromatography (0.1% formic acid aqueous solution) to obtain a formate of the title compound (9.80 g).
ESI-MS (m/z): 153.05 $[M+H]^+$.

[Production Example 29-4]

*tert*-Butyl 4-methyl-4-(prop-2-yn-1-ylamino)-[1,4'-bipiperidine]-1'-carboxylate

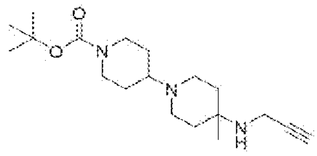

To a solution of the formate of 4-methyl-N-(prop-2-yn-1-yl)piperidine-4-amine obtained in Production Example 29-3 (4.2 g, 17 mmol) in tetrahydrofuran (30 mL) and dichloromethane (30 mL) there was added N,N-diisopropylethylamine (15 mL), and the mixture was stirred for 5 minutes at room temperature. After then adding BOC-4-piperidone (5.14 g, 25.8 mmol) to the reaction mixture, it was stirred for 1 hour at room temperature. The reaction mixture was cooled to 0°C, sodium triacetoxyborohydride(>80 wt%, 9.11 g) was added, and the mixture was stirred overnight at room temperature. The reaction mixture was quenched with water, and then ethyl acetate and 1 N hydrochloric acid were added. The aqueous layer was separated off and washed with ethyl acetate to remove the unreacted ketone and its reduced form. The aqueous layer was neutralized with a saturated aqueous sodium hydrogencarbonate solution and extracted 3 times with a chloroform:isopropyl alcohol = 4:1 solution. The organic layer was washed with brine and dried over magnesium sulfate. After filtering the organic layer, it was concentrated under reduced pressure and the residue was purified by NH silica gel column chromatography (n-heptane:ethyl acetate = 75:25 to 0:100) to obtain the title compound (4.23 g).
ESI-MS (m/z): 336.32 $[M+H]^+$.

[Production Example 29-5]

4-(4-(1-Cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1-tosyl-1,6-dihydro-7H-pyrrolo[2,3-c]pyridin-7-one

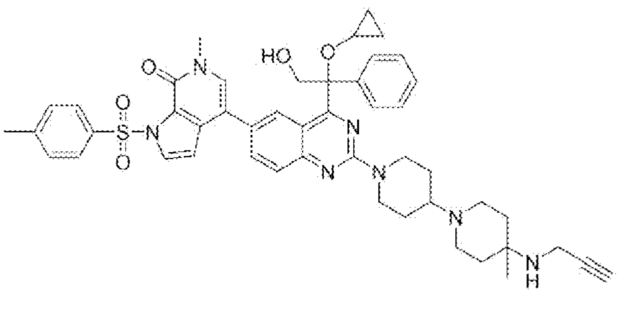

To a solution of the *tert*-butyl 4-methyl-4-(prop-2-yn-1-ylamino)-[1,4'-bipiperidine]-1'-carboxylate of Production

Example 29-4 (610 mg, 90% purity, 1.64 mmol) in dichloromethane (3.44 mL) there was added TFA (3.37 mL), and the mixture was stirred for 30 minutes at room temperature. The reaction mixture was concentrated under reduced pressure (azeotropic distillation with addition of toluene), to obtain a crude product. To the obtained crude product there were added DMF (9.47 mL), N,N-diisopropylethylamine (2.86 mL) and the 4-(2-chloro-4-(1-cyclopropoxy-2-hydroxy-1-phenylethyl) quinazolin-6-yl)-6-methyl-1-tosyl-1,6-dihydro-7H-pyrrolo[2,3-c]pyridin-7-one of Production Example 29-2 (700 mg, 1.09 mmol, azeotropic distillation with toluene before use). After stirring the mixture for 1 hour at 85°C, N,N-diisopropylethylamine (0.95 mL) was added and the mixture was stirred for 3 hours. After cooling to room temperature, the mixture was concentrated under reduced pressure to 1/4 volume, and then ethyl acetate and an aqueous sodium hydrogencarbonate solution were added. The organic layer was separated off and the aqueous layer was extracted with ethyl acetate. The combined organic layers were dried over sodium sulfate and filtered, and then concentrated under reduced pressure. The residue was purified by NH silica gel column chromatography (n-heptane:ethyl acetate = 1:1 to ethyl acetate to ethyl acetate:methanol = 7:3). It was further purified by silica gel column chromatography (ethyl acetate to ethyl acetate:methanol = 1:1) to obtain the title compound (569 mg).

ESI-MS (m/z): 840.42 $[M+H]^+$.

[Example 30]

4-[4-(1-Cyclopropoxy-2-hydroxy-1-phenylethyl-2-{4-ethynyl-4-hydroxy-[1,4-bipiperidin]-1'-yl}quinazolin-6-yl]-6-methyl-1H,6H,7H-pyrrolo[2,3-c]pyridin-7-one

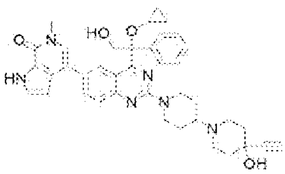

To a solution of the 4- {4-[1-cyclopropoxy-2-(oxan-2-yloxy)-1-phenylethyl]-2-{4-ethynyl-4-hydroxy-[1,4'-bipiperidin]-1'-yl}quinazolin-6-yl}-6-methyl-1H,6H,7H-pyrrolo[2,3-c]pyridin-7-one of Production Example 30-2 (5.2 mg, 7.0 μmol) in dichloromethane (225 μL) there was added TFA (268 μL), and the mixture was stirred for 30 minutes at room temperature. The reaction mixture was then concentrated under reduced pressure. The obtained residue was purified by ODS silica gel column chromatography (acetonitrile (containing 0.1% formic acid):water (containing 0.1% formic acid) = 2:98 to 70:30). The compound obtained by concentration under reduced pressure was dissolved in dichloromethane and washed with a saturated aqueous sodium hydrogencarbonate solution and brine. The organic layer was dried over anhydrous sodium sulfate and filtered, and then the filtrate was concentrated under reduced pressure to obtain the title compound (3.02 mg).

ESI-MS (m/z): 659.39 $[M+H]^+$.

[Production Example 30-1]

*tert*-Butyl 4-ethynyl-4-hydroxy-[1,4'-bipiperidine]-1'-carboxylate

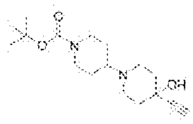

To a solution of N-BOC-4-ethynylpiperidin-4-ol (600 mg, 2.66 mmol) in dichloromethane (10.3 mL) there was added TFA (10.2 mL), and the mixture was stirred for 1 hour at room temperature and then concentrated under reduced pressure. Tetrahydrofuran (21.8 mL), N-BOC-4-piperidone (796 mg, 4.0 mmol) and sodium triacetoxyborohydride (>80 wt%, 2.82 g) were added to the obtained residue, and the mixture was stirred for 14 hours at room temperature. A saturated aqueous sodium hydrogencarbonate solution was added and the mixture was stirred for 30 minutes at room temperature and neutralized, and then extracted with ethyl acetate. The organic layer was washed with brine and dried over anhydrous sodium sulfate. The organic layer was filtered, and the filtrate was concentrated under reduced pressure. The residue was

purified by ODS silica gel column chromatography (acetonitrile (containing 0.1% formic acid):water (containing 0.1% formic acid) = 2:98 to 100:0) to obtain a formate of the title compound (344 mg).

ESI-MS (m/z): 309.67 $[M+H]^+$.

[Production Example 30-2]

4-{4-[1-Cyclopropoxy-2-(oxan-2-yloxy)-1-phenylethyl]-2-{4-ethynyl-4-hydroxy-[1,4'-bipiperidin]-1'-yl}quinazolin-6-yl}-6-methyl-1H,6H,7H-pyrrolo[2,3-c]pyridin-7-one

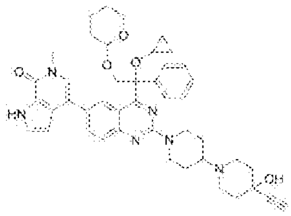

To a solution of the formate of *tert*-butyl 4-ethynyl-4-hydroxy-[1,4'-bipiperidine]-1'-carboxylate of Production Example 30-1 (221 mg) in dichloromethane (14.2 mL) there was added TFA (4.25 mL), and the mixture was stirred for 30 minutes at room temperature and then concentrated under reduced pressure. A procedure of dissolving the residue in toluene (3 mL) and concentrating under reduced pressure was repeated 3 times. The residue was then dissolved in dimethylformamide (14.9 ml). Diisopropylethylamine (1.93 mL, 11.0 mmol) and the 4-(2-chloro-4-(1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 26-1 (400 mg, 0.552 mmol) were added, and the mixture was stirred for 4 hours at 50°C. The reaction mixture was concentrated under reduced pressure. To a solution of the residue in tetrahydrofuran (9.04 mL) and methanol (8.93 mL) there was added a 2 M sodium hydroxide aqueous solution (2.21 mL, 4.41 mmol), and the mixture was stirred for 50 minutes at 70°C. Ethyl acetate and brine were added to the reaction mixture for oil-water distribution. The organic layer was dried over anhydrous sodium sulfate and filtered, and then the filtrate was concentrated under reduced pressure. The residue was purified by ODS silica gel column chromatography (acetonitrile (containing 0.1% formic acid):water (containing 0.1% formic acid) = 0:100 to 70:30) to obtain a formate of the title compound (170 mg).

ESI-MS (m/z): 743.57 $[M+H]^+$.

[Example 31]

4-(4-(2-Amino-1-cyclopropoxy-1-phenylethyl)-2-(4-((2S,5R)-2,5-dimethylpiperazin-1-yl)piperidin-1-yl)quinazolin-6-yl)-6-methyl-1,6-dihydro-7H-pyrrolo[2,3-c]pyridin-7-one

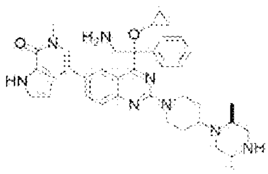

To a solution of the *tert-butyl* (2R,5S)-4-(1-(4-(2-amino-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate of Production Example 31-5 (115 mg, 0.154 mmol) in dichloromethane (1 mL) there was added TFA (1 mL), and the mixture was stirred for 1 hour at room temperature. After concentrating the reaction mixture under reduced pressure, the residue was purified by ODS silica gel column chromatography (acetonitrile (containing 0.1% formic acid):water (containing 0.1% formic acid) = 5:95 to 3:7), to obtain a formate of the title compound. The formate of the title compound was neutralized by passing it through NH silica gel column chromatography, and the filtrate was concentrated to obtain the title compound (41 mg).

ESI-MS (m/z): 647.38 $[M+H]^+$.

[Example 31-1]

*tert*-Butyl (2R,5S)-4-(1-((benzyloxy)carbonyl)piperidin-4-yl)-2,5-dimethylpiperazine-1-carboxylate

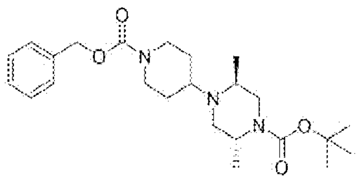

To a solution of *tert-butyl* (2R,5S)-2,5-dimethylpiperazine-1-carboxylate (4.82 g, 22.5 mmol) in dichloromethane (100 mL) and acetic acid (86 μL, 1.50 mmol) there was added 1-(benzyloxycarbonyl)-4-piperidinone (3.50 g, 15.0 mmol) at room temperature, and the mixture was stirred for 10 minutes at room temperature. Sodium triacetoxyborohydride (>80 wt%, 9.54 g) was then added and the mixture was stirred for 99 hours at room temperature. A saturated aqueous sodium hydrogencarbonate solution was added to the reaction mixture, and extraction was performed with chloroform. The organic layer was dried over anhydrous magnesium sulfate and filtered, and the filtrate was concentrated under reduced pressure. The residue was purified by NH silica gel column chromatography (n-heptane:ethyl acetate = 4:1 to 1:1) to obtain the title compound (4.29 g).
ESI-MS (m/z): 432.79 $[M+H]^+$.

[Production Example 31-2]

*tert*-Butyl (2R,5S)-2,5-dimethyl-4-(piperidin-4-yl)piperazine-1-carboxylate

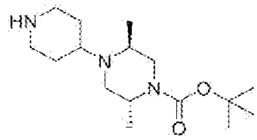

To a solution of the *tert-butyl* (2R,5S)-4-(1-((benzyloxy)carbonyl)piperidin-4-yl)-2,5-dimethylpiperazine-1-carboxylate of Production Example 31-1 (4.29 g, 9.94 mmol) in THF (25 mL) and ethyl acetate (25 mL) there was added 10% palladium-carbon (50% water, 8.46 g) at room temperature, and the mixture was stirred for 22 hours and 35 minutes at room temperature, ordinary pressure under a hydrogen atmosphere. After switching the reaction mixture to a nitrogen atmosphere, it was filtered with Celite and washed with methanol. The filtrate was concentrated under reduced pressure to obtain the title compound (2.81 g). ESI-MS (m/z): 298.22 $[M+H]^+$.

[Production Example 31-3]

*tert*-Butyl (2R,5S)-4-(1-(4-(1-cyclopropoxy-2-hydroxy-1-phenylethyl)-6-iodoquinazolin-2-yl)piperidin-4-yl)-2,5-dimethylpiperazine-1-catboxylate

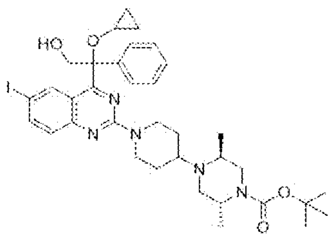

To a solution of the 2-chloro-4-(1-cyclopropoxy-1-phenyl-2-((tetrahydro-2H-pyran-2-yl)oxy)ethyl)-6-iodoquinazoline of Production Example 2-3 (4.6 g, 8.4 mmol) and the *tert*-butyl (2R,5S)-2,5-dimethyl-4-(piperidin-4-yl)piperazine-1-catboxylate of Production Example 31-2 (2.86 g, 9.60 mmol) in DMF (30 mL) there was added N,N-diisopropylethylamine

(3.65 mL, 20.9 mmol), and the mixture was stirred overnight at 80°C. Water was added to the reaction mixture, and extraction was performed with ethyl acetate. The organic layer was washed with water and brine. The organic layer was dried over anhydrous magnesium sulfate and then filtered, and the filtrate was concentrated under reduced pressure to obtain a crude product. To a solution of the obtained crude product in methanol (30 mL) and dichloromethane (5 mL) there was added p-toluenesulfonic acid monohydrate (3.97 g, 20.9 mmol), and the mixture was stirred for 4 hours at room temperature. After adding N,N-diisopropylethylamine (7.29 mL, 41.8 mmol) to the reaction mixture, the mixture was concentrated under reduced pressure. The residue was purified by NH silica gel column chromatography (n-heptane:ethyl acetate = 50:50 to 25:75) to obtain the title compound (6.1 g).
ESI-MS (m/z): 728.91 $[M+H]^+$

[Production Example 31-4]

*tert*-Butyl (2R,5 S)-4-(1-(4-(1-cyclopropoxy-2-hydroxy-1-phenylethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-carboxylate

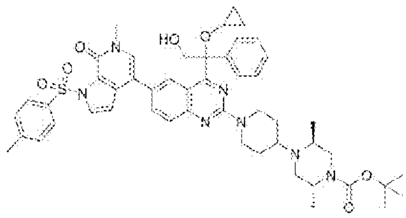

To a solution of the *tert-butyl* (2R,5S)-4-(1-(4-(1-cyclopropoxy-2-hydroxy-1-phenylethyl)-6-iodoquinazolin-2-yl) piperidin-4-yl)-2,5-dimethylpiperazine-1-carboxylate of Production Example 31-3 (1.00 g, 1.37 mmol) in 1,4-dioxane (11 mL) and water (2.75 mL) there were added sodium carbonate (291 mg, 2.75 mmol), 6-methyl-4-(4,4,5,5,-tetramethyl-1,3,2-dioxaborolan-2-yl)-1-tosyl-1,6-dihydro-7H-pyrrolo[2,3-c]pyridin-7-one (706 mg, 1.65 mmol) and tetrakis(triphenylphosphine)palladium(0) (159 mg, 0.137 mmol), and the mixture was stirred for 20 hours and 30 minutes at 80°C under a nitrogen atmosphere. The reaction mixture was returned to room temperature and diluted with ethyl acetate, and then filtered with Celite. The filtrate was concentrated under reduced pressure and the residue was purified by NH silica gel column chromatography (n-heptane:ethyl acetate = 1:1 to 1:4) to obtain the title compound (983 mg).
ESI-MS (m/z): 902.97 $[M+H]^+$.

[Production Example 31-5]

*tert*-Butyl (2R,5S)-4-(1-(4-(2-amino-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate

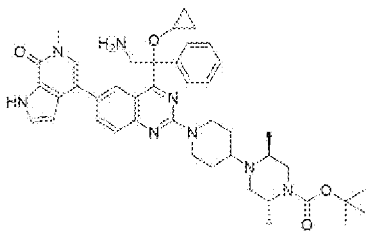

To a solution of the *tert-butyl* (2R,5S)-4-(1-(4-(1-cyclopropoxy-2-hydroxy-1-phenylethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-carboxylate of Production Example 31-4 (200 mg, 0.222 mmol) in dichloromethane (1.5 mL) there were added triethylamine (93 μL, 0.665 mmol) and methanesulfonyl chloride (34 μL, 0.443 mmol) at room temperature, and the mixture was stirred for 10 minutes while cooling on ice and for 10 minutes at room temperature. Saturated aqueous ammonium chloride was added to the reaction mixture, and extraction was performed with dichloromethane. The organic layer was dried over anhydrous sodium sulfate and filtered, and the filtrate was concentrated under reduced pressure. To a solution of the residue in NMP

(3 mL) there was added 28% ammonia water (1.5 mL) at room temperature, and a microwave reactor was used for reaction at 130°C for 40 minutes. The reaction mixture was extracted with dichloromethane and the organic layer was washed with water, and then dried over anhydrous sodium sulfate and filtered, and the filtrate was concentrated under reduced pressure. To a solution of the residue in THF (0.5 mL) and methanol (1 mL) there was added a 2 M sodium hydroxide aqueous solution (500 μL, 1.00 mmol), and the mixture was stirred for 3 hours at 50°C. The reaction mixture was returned to room temperature, water was added, and extraction was performed with dichloromethane. The organic layer was dried over anhydrous sodium sulfate and filtered, and the filtrate was concentrated under reduced pressure. The residue was purified by NH silica gel column chromatography (chloroform:methanol = 100:0 to 97:3) to obtain the title compound (115 mg).

ESI-MS (m/z): 747.30 $[M+H]^+$.

[Example 32]

5-(4-(4-(Hydroxymethyl)heptan-4-yl)-2-(4-(piperazin-1-ylmethyl)piperidin-1-yl)quinazolin-6-yl)-4-methoxy-1-methylpyridin-2(1H)-one

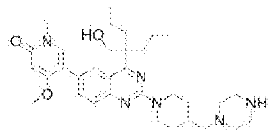

To a solution of the *tert-butyl* 4-((1-(6-(4-methoxy-1-methyl-6-oxo-1,6-dihydropyridin-3-yl)-4-(4-(((tetrahydro-2H-pyran-2-yl)oxy)methyl)heptan-4-yl)quinazolin-2-yl)piperidin-4-yl)methyl)piperazine-1-carboxylate of Production Example 32-4 (84 mg, 0.110 mmol) in dichloromethane (1 mL) there was added TFA (0.5 mL) at room temperature, and the mixture was stirred for 1 hour at room temperature. The reaction mixture was purified by ODS silica gel column chromatography (0.1% formic acid aqueous solution: 0.1% acetonitrile formate solution = 95:5 to 30:70), to obtain a formate of the title compound (41.2 mg).

ESI-MS (m/z): 577.52 $[M+H]^+$.

[Production Example 32-1]

Ethyl 2-(2-chloro-6-iodoquinazolin-4-yl)-2-propyl pentanoate

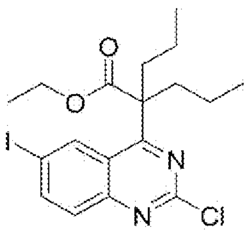

To a solution of ethyl 2-propylpentanoate (2.76 g, 16.0 mmol) in THF (20 mL) there was added dropwise lithium diisopropyl amide (1.09 M THF solution, 14.68 mL, 16.00 mmol) at -78°C, and the mixture was stirred for 10 minutes at -78°C. A solution of 2,4-dichloro-6-iodoquinazoline (4.00 g, 12.3 mmol) in THF (20 mL) was then added at -78°C, the temperature was slowly increased, and the mixture was stirred for 2 hours at room temperature. Saturated aqueous ammonium chloride was added to the reaction mixture, and extraction was performed with ethyl acetate. The organic layer was dried over sodium sulfate and filtered. The filtrate was concentrated under reduced pressure. The obtained solid was washed with diethyl ether:n-heptane = 1:1 to obtain a portion of the title compound. The filtrate was concentrated under reduced pressure and washed with diethyl ether:n-heptane = 1:1 to obtain a portion of the title compound. The filtrate was purified with silica gel column chromatography (n-heptane to n-heptane:ethyl acetate = 99:1 to 9:1) to obtain the total title compound (3.40 g).

ESI-MS (m/z): 461.18 $[M+H]^+$.

[Production Example 32-2]

2-Chloro-6-iodo-4-(4-(((tetrahydro-2H-pyran-2-yl)oxy)methyl)heptan-4-yl)quinazoline

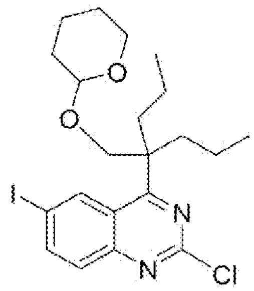

To a solution of the ethyl 2-(2-chloro-6-iodoquinazolin-4-yl)-2-propylpentanoate of Production Example 32-1 (1.60 g, 3.47 mmol) in toluene (150 mL) there was added dropwise DIBAL-H (1 M toluene solution, 8.68 mL, 8.68 mmol) at -78°C, and the mixture was stirred for 2 hours at 0°C. A saturated aqueous Rochelle salt solution and ethyl acetate were added to the reaction mixture, and stirring was continued. After oil-water distribution of the reaction mixture, the organic layer was washed with brine and dried over sodium sulfate. The organic layer was filtered, and the filtrate was then concentrated under reduced pressure. The residue was purified by silica gel column chromatography (n-heptane:ethyl acetate = 99:1 to 7:3) to obtain a crude product. To a solution of the obtained crude product in dichloromethane (5 mL) at room temperature there were added DHP (380 mg, 4.51 mmol) and *p*-toluenesulfonic acid monohydrate (132 mg, 0.695 mmol) at 0°C, and the mixture was stirred for 4 hours at room temperature. A saturated aqueous sodium hydrogencarbonate solution was added to the reaction mixture, and extraction was performed with dichloromethane. The organic layer was dried over anhydrous sodium sulfate and filtered, and the filtrate was concentrated under reduced pressure. The residue was purified by silica gel column chromatography (n-heptane:ethyl acetate = 99:1 to 7:3) to obtain the title compound (430 mg).
ESI-MS (m/z): 503.22 $[M+H]^+$

[Production Example 32-3]

*tert*-Butyl 4-((6-iodo-4-(4-(((tetrahydro-2H-pyran-2-yl)oxy)methyl)heptan-4-yl)quinazolin-2-yl)piperidin-4-yl)methyl-)piperazine-1-carboxylate

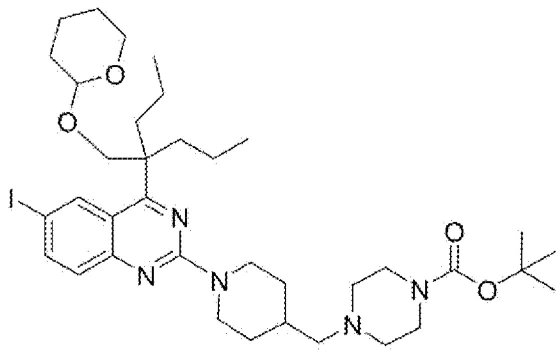

To a solution of the 2-chloro-6-iodo-4-(4-(((tetrahydro-2H-pyran-2-yl)oxy)methyl)heptan-4-yl)quinazoline of Production Example 32-2 (80 mg, 0.16 mmol) in DMF (2 mL) there were added *tert*-butyl 4-(piperidin4-ylmethyl) piperazine-1-carboxylate (54.1 mg, 0.191 mmol) and N,N-diisopropylethylamine (100 μL, 0.573 mmol) at room temperature, and the mixture was stirred for 3 hours at 80°C. Water was added to the reaction mixture, which was then extracted with dichloromethane. The organic layer was dried over anhydrous sodium sulfate and filtered, and then the filtrate was concentrated under reduced pressure to obtain the title compound (112 mg).
ESI-MS (m/z): 750.50 $[M+H]^+$.

[Production Example 32-4]

*tert*-Butyl 4-((1-(6-(4-methoxy-1-methyl-6-oxo-1,6-dihydropyridin-3-yl)-4-(4-(((tetrahydro-2H-pyran-2-yl)oxy)methyl) heptan-4-yl)quinazolin-2-yl)piperidin-4-yl)methyl)piperazine-1-carboxylate

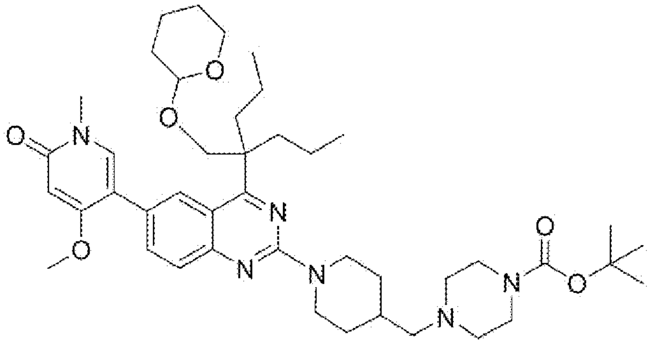

To a solution of the *tert*-butyl 4-((1-6-iodo-4-(4-(((tetrahydro-2H-pyran-2-yl)oxy)methyl)heptan-4-yl)quinazolin-2-yl)piperidine-4-methyl)piperazine-1-carboxylate of Production Example 32-3 (112 mg, 0.149 mmol) in toluene (2 mL), ethanol (0.5 mL) and aqueous 2 M sodium carbonate (0.5 mL) there were added 4-methoxy-1-methyl-5-(4,4,5,5-tetramethyl-1,3,2-dioxaborolan-2-yl)pyridin-2(1H)-one (55.4 mg, 0.209 mmol) and tetrakis(triphenylphosphine)palladium(0) (17.26 mg, 0.015 mmol) at room temperature, and the mixture was stirred for 3 hours at 90°C under a nitrogen atmosphere. The reaction mixture was returned to room temperature, and extraction was performed with dichloromethane. The organic layer was washed with brine, dried over anhydrous sodium sulfate and filtered, and the filtrate was concentrated under reduced pressure. The residue was purified by NH silica gel column chromatography (n-heptane:ethyl acetate = 7:3 to ethyl acetate) to obtain the title compound (84 mg).
ESI-MS (m/z): 761.68 $[M+H]^+$.

[Example 33]

5-(2-(4-Amino-4-methyl-[1,4'-bipiperidin]-1'-yl)-4-(1-cyclopropoxy-2-hydroxy-1-(naphthalen-1-yl)ethyl)quinazolin-6-yl)-4-methoxy-1-methylpyridin-2(1H)-one

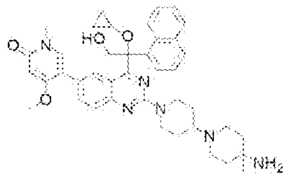

To a solution of the *tert-butyl* (1'-(4-(1-cyclopropoxy-2-hydroxy-1-(naphthalen-1-yl)ethyl)-6-(4-methoxy-1-methyl-6-oxo-1,6-dihydropyridin-3-yl)quinazolin-2-yl)-4-methyl-[1,4'-bipiperidin]-4-yl)carbamate of Production Example 33-5 (31.4 mg, 0.0400 mmol) in dichloromethane (1.5 mL) there was added TFA (0.5 mL), and the mixture was stirred for 1 hour at room temperature. The reaction mixture was then concentrated under reduced pressure. The residue was purified by ODS silica gel column chromatography (acetonitrile (containing 0.1% formic acid):water (containing 0.1% formic acid) = 5:95 to 1:0 to methanol = 100%) to obtain a formate of the title compound (22.9 mg).
ESI-MS (m/z): 689.36 $[M+H]^+$.

[Production Example 33-1]

Methyl 2-cyclopropoxy-2-(naphthalen-1-yl)acetate

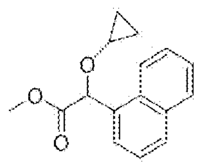

To a solution of 1-naphthylacetic acid (1.50 g, 8.06 mmol) in methanol (20 mL) there was added (diazomethyl) trimethylsilane (8.06 mL, 16.1 mmol) at 0°C, and the mixture was stirred for 1 hour at room temperature. Acetic acid (3 mL) was added to the reaction mixture, which was then concentrated under reduced pressure. The residue was purified by silica gel column chromatography (n-heptane:ethyl acetate = 1:0 to 1:1) to obtain a crude product. To a solution of the obtained crude product and 4-acetamidobenzenesulfonyl azide (2.32 g, 9.67 mmol) in acetonitrile (15 mL) there was added DBU (1.80 mL, 12.1 mmol) at 0°C, and the mixture was stirred for 3 hours at room temperature. Saturated aqueous ammonium chloride was added to the reaction mixture, and extraction was performed with ethyl acetate. The organic layer was washed with brine, and then the organic layer was dried over anhydrous magnesium sulfate and filtered, and the filtrate was concentrated under reduced pressure. The residue was purified by silica gel column chromatography (n-heptane:ethyl acetate = 1:0 to 1:1) to obtain a crude product. To a solution of the obtained crude product and cyclopropanol (0.936 ml, 16.1 mmol) in dichloromethane (15 mL) there was added diacetoxyrhodium (0.0890 g, 0.201 mmol) at room temperature, and the mixture was stirred overnight at room temperature. The reaction mixture was purified by silica gel column chromatography (n-heptane:ethyl acetate = 1:0 to 7:3) to obtain the title compound (1.37 g).
1H-NMR Spectrum (400 MHz, CDCl3) δ(ppm): 0.42-0.53 (2H, m), 0.67-0.79 (2H, m), 3.39-3.51 (1H, m), 3.67 (3H, s), 5.57-5.60 (1H, m), 7.43-7.57 (3H, m), 7.63 (1H, dd, J=7.3, 0.98 Hz), 7.85 (2H, t, J=7.8 Hz), 8.27 (1H, t, J=7.8 Hz).

[Production Example 33-2]

Methyl 2-(2-chloro-6-iodoquinazolin-4-yl)-2-cyclopropoxy-2-(naphthalen-1-yl)acetate

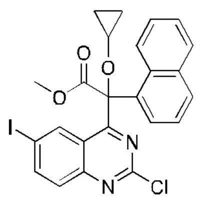

To a solution of the methyl 2-cyclopropoxy-2-(naphthalen-1-yl)acetate of Production Example 33-1 (1.40 g, 5.46 mmol) in THF (15 mL) there was added dropwise lithium diisopropyl amide (1.43 M THF solution, 7.64 mL, 10.9 mmol) at -78°C, and then a solution of 2,4-dichloro-6-iodoquinazoline (1.95 g, 6.01 mmol) in THF (15 mL) was added at -78°C, the temperature was slowly increased, and the mixture was stirred for 40 minutes at room temperature. Saturated aqueous ammonium chloride was added to the reaction mixture, and extraction was performed with ethyl acetate. The organic layer was washed with water and brine, and then the organic layer was dried over anhydrous magnesium sulfate and filtered, and the filtrate was concentrated under reduced pressure. The residue was purified by silica gel column chromatography (n-heptane:ethyl acetate = 1:0 to 1:1) to obtain the title compound (1.43 g).
ESI-MS (m/z): 545.15 $[M+H]^+$.

[Production Example 33-3]

2-(2-Chloro-6-iodoquinazolin-4-yl)-2-cyclopropoxy-2-(naphthalen-1-yl)ethan-1-ol

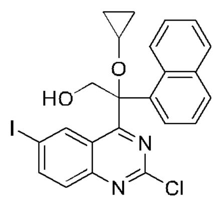

To a solution of the methyl 2-(2-chloro-6-iodoquinazolin-4-yl)-2-cyclopropoxy-2-(naphthalen-1-yl)acetate of Production Example 33-2 (250 mg, 0.459 mmol) in toluene (5 mL) there was added dropwise DIBAL-H (1 M toluene solution, 1.38 ml, 1.38 mmol) at - 78°C, and the mixture was stirred for 10 minutes. The reaction mixture was increased in temperature to 0°C and stirred for 30 minutes. A saturated aqueous Rochelle salt solution was added to the reaction mixture, and extraction was performed with ethyl acetate. The organic layer was dried over anhydrous magnesium sulfate and filtered, and the filtrate was concentrated under reduced pressure. The residue was purified by silica gel column chromatography (n-heptane:ethyl acetate = 1:0 to 1:1) to obtain the title compound (69.8 mg).
ESI-MS (m/z): 516.68 $[M+H]^+$.

[Production Example 33-4]

5-(2-Chloro-4-(1-cyclopropoxy-2-hydroxy-1-(naphthalen-1-yl)ethyl)quinazolin-6-yl)-4-methoxy-1-methylpyridin-2(1H)-one

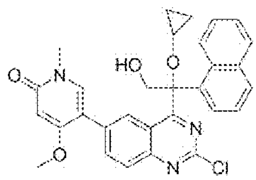

To a solution of the 2-(2-chloro-6-iodoquinazolin-4-yl)-2-cyclopropoxy-2-(naphthalen-1-yl)ethan-1-ol of Production Example 33-3 (69.8 mg, 0.135 mmol) in 1,4-dioxane (5 mL) and water (0.5 mL) there were added 4-methoxy-1-methyl-5-(4,4,5,5-tetramethyl-1,3,2-dioxaborolan-2-yl)pyridin-2(1H)-one (53.7 mg, 0.203 mmol), cesium carbonate (66.0 mg, 0.203 mmol) and tetrakis(triphenylphosphine)palladium(0) (31.2 mg, 0.0270 mmol) at room temperature, and the mixture was stirred for 3 hours at 80°C. Water was added to the reaction mixture, and extraction was performed with ethyl acetate. The organic layer was washed with water and brine, and then the organic layer was dried over anhydrous magnesium sulfate and filtered, and the filtrate was concentrated under reduced pressure. The residue was purified by silica gel column chromatography (ethyl acetate:methanol = 1:0 to 1:1) to obtain the title compound (49.6 mg).
ESI-MS (m/z): 528.23 $[M+H]^+$.

[Production Example 33-5]

*tert*-Butyl (1'-(4-(1-cyclopropoxy-2-hydroxy-1-(naphthalen-1-yl)ethyl)-6-(4-methoxy-1-methyl-6-oxo-1,6-dihydropyridin-3-yl)quinazolin-2-yl)-4-methyl-[1,4'-bipiperidin]-4-yl)carbamate

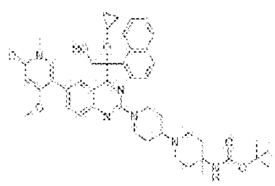

To a solution of the 5-(2-chloro-4-(1-cyclopropoxy-2-hydroxy-1-(naphthalen-1-yl)ethyl)quinazolin-6-yl)-4-methoxy-1-methylpyridin-2(1H)-one of Production Example 33-4 (49.6 mg, 0.0940 mmol) in DMF (3 mL) there were added the *tert-butyl* (4-methyl-[1,4'-bipiperidin]-4-yl)carbamate of Production Example 27-2 (30.7 mg, 0.103 mmol) and N,N-diisopropylethylamine (49 μL, 0.28 mmol) at room temperature, and the mixture was stirred for 2 hours and 30 minutes at 80°C. Water was added to the reaction mixture, and extraction was performed with ethyl acetate. The organic layer was washed with brine, and then the organic layer was dried over anhydrous magnesium sulfate and filtered, and the filtrate was concentrated under reduced pressure. The residue was purified by NH silica gel column chromatography (ethyl acetate:methanol = 1:0 to 1:1) to obtain the title compound (31.2 mg).
ESI-MS (m/z): 789.43 $[M+H]^+$.

[Example 34]

2-Cyclopropoxy-2-(2-(4-(((2S,5R)-2,5-dimethylpiperazin-1-yl)methyl)piperidin-1-yl)-6-(4-methoxy-1-methyl-6-oxo-1,6-dihydropyridin-3-yl)quinazolin-4-yl)-2-phenylethyl methyl(2-(methylamino)ethyl carbamate

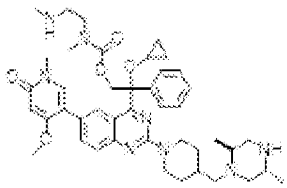

To a solution of the *tert*-butyl (2R,5S)-4-((1-(4-(1-cyclopropoxy-2-((methyl(2-(methylamino)ethyl)carbamoyl)oxy)-1-phenylethyl)-6-(4-methoxy-1-methyl-6-oxo-1,6-dihydropyridin-3-yl)quinazolin-2-yl)piperidin-4-yl)methyl)-2,5-dimethylpiperazine-1-carboxylate of Production Example 34-3 (120 mg, 0.138 mmol) in dichloromethane (2 mL) there was added TFA (2 mL), and the mixture was stirred for 1 hour at room temperature. The reaction mixture was concentrated under reduced pressure, a saturated aqueous sodium hydrogencarbonate solution was added to the residue, and extraction was performed with dichloromethane. The organic layer was dried over anhydrous magnesium sulfate and filtered, and the filtrate was concentrated under reduced pressure. The residue was purified by NH silica gel column chromatography (ethyl acetate:methanol = 9:1 to chloroform:methanol = 99:1 to 9:1) to obtain the title compound (91 mg).
ESI-MS (m/z): 767.31 $[M+H]^+$.

[Production Example 34-1]

5-(2-Chloro-4-(1-cyclopropoxy-1-phenyl-2-((tetrahydro-2H-pyran-2-yl)oxy)ethyl)quinazolin-6-yl)-4-methoxy-1-methylpyridin-2(1H)-one

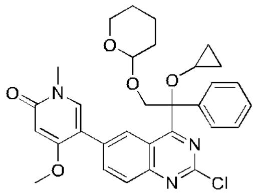

To a solution of the 2-chloro-4-(1-cyclopropoxy-1-phenyl-2-((tetrahydro-2H-pyran-2-yl)oxy)ethyl)-6-iodoquinazoline of Production Example 2-3 (1.00 g, 1.82 mmol) in 1,4-dioxane (7.3 mL) and water (1.82 mL) there were added 4-methoxy-1-methyl-5-(4,4,5,5-tetramethyl-1,3,2-dioxaborolan-2-yl)pyridin-2(1H)-one (825 mg, 2.18 mmol), sodium carbonate (385 mg, 3.63 mmol) and tetrakis(triphenylphosphine)palladium(0) (210 mg, 0.182 mmol) at room temperature, and the mixture was stirred for 24 hours at 80°C under a nitrogen atmosphere. The reaction mixture was returned to room temperature and diluted with ethyl acetate. The diluted reaction mixture was filtered with Celite, and the filtrate was concentrated under reduced pressure. The residue was purified by silica gel column chromatography (n-heptane:ethyl acetate = 1:3 to ethyl acetate to ethyl acetate:methanol = 9:1) to obtain the title compound (292 mg).
ESI-MS (m/z): 562.31 $[M+H]^+$

[Production Example 34-2]

*tert*-Butyl (2R,5S)-4-((1-(4-(1-cyclopropoxy-2-hydroxy-1-phenylethyl)-6-(4-methoxy-1-methyl-6-oxo-1,6-dihydropyridin-3-yl)quinazolin-2-yl)piperidin-4-yl)methyl)-2,5-dimethylpiperazine-1-carboxylate

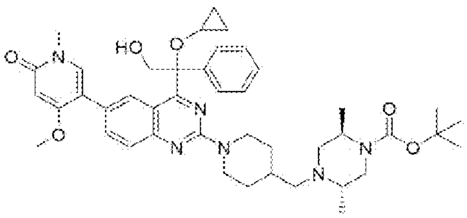

To a solution of the 5-(2-chloro-4-(1-cyclopropoxy-1-phenyl-2-((tetrahydro-2H-pyran-2-yl)oxy)ethyl)quinazolin-6-yl)-4-methoxy-1-methylpyridin-2(1H)-one of Production Example 34-1 (290 mg, 0.516 mmol) and the *tert-butyl* (2R,5S)-2,5-dimethyl-4-(piperidin-4-ylmethyl)piperazine-1-carboxylate of Production Example 28-2 (225 mg, 0.722 mmol) in DMF (2 mL), there was added N,N-diisopropylethylamine (270 μL, 1.55 mmol), and the mixture was stirred for 8 hours at 80°C. Water was added to the reaction mixture, and extraction was performed with ethyl acetate. The organic layer was washed with water and brine. The organic layer was dried over anhydrous magnesium sulfate and filtered, and the filtrate was concentrated under reduced pressure. To a solution of the residue in methanol (3 mL) there was added p-toluenesulfonic acid monohydrate (294 mg, 1.55 mmol) at room temperature, and the mixture was stirred for 2 hours at room temperature. After adding triethylamine (431 μL, 3.10 mmol) to the reaction mixture, the mixture was concentrated under reduced pressure. The residue was purified by NH silica gel column chromatography (n-heptane:ethyl acetate = 1:4 to ethyl acetate to ethyl acetate:methanol = 9:1) to obtain the title compound (326 mg).
ESI-MS (m/z): 754.07 $[M+H]^+$

[Production Example 34-3]

*tert-Butyl* (2R,5S)-4-((1-(4-(1-cyclopropoxy-2-((methyl(2-(methylamino)ethyl)carbamoyl)oxy)-1-phenylethyl)-6-(4-methoxy-1-methyl-6-oxo-1,6-dihydropyridin-3-yl)quinazolin-2-yl)piperidin-4-yl)methyl)-2,5-dimethylpiperazine-1-carboxylate

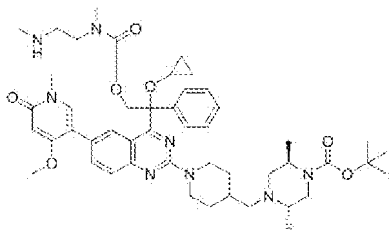

To a solution of the *tert-butyl* (2R,5S)-4-((1-(4-(1-cyclopropoxy-2-hydroxy-1-phenylethyl)-6-(4-methoxy-1-methyl-6-oxo-1,6-dihydropyridin-3-yl)quinazolin-2-yl)piperidin-4-yl)methyl)-2,5-dimethylpiperazine-1-carboxylate of Production Example 34-2 (150 mg, 0.199 mmol) and 4-dimethylaminopyridine (73 mg, 0.60 mmol) in dichloromethane (3 mL) there was added triphosgene (35.5 mg, 0.12 mmol) while cooling on ice, and after stirring for 5 minutes, the mixture was further stirred for 10 minutes at room temperature. After again ice-cooling the reaction mixture, N,N'-dimethylethylenediamine (214 μL, 1.99 mmol) was added, stirring was continued for 10 minutes, and stirring was further continued for 30 minutes at room temperature. The reaction mixture was concentrated under reduced pressure, and the residue was purified by NH silica gel column chromatography (ethyl acetate:methanol = 99:1 to 9:1) to obtain the title compound (143 mg).
ESI-MS (m/z): 867.19 $[M+H]^+$

[Example 35]

4-(4-(2-Hydroxy-1,1-diphenylethyl)-2-(4-(piperidin-4-yloxy)piperidin-1-yl)quinazolin-6-yl)-6-methylfuro[2,3-c]pyridine-7(6H)-one

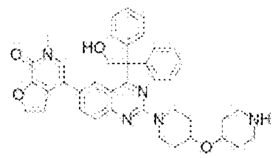

To a solution of the 4-(2-chloro-4-(1,1-diphenyl-2-((tetrahydro-2H-pyran-2-yl)oxy)ethyl)quinazolin-6-yl)-6-methylfuro[2,3-c]pyridine-7(6H)-one of Production Example 35-3 (30 mg, 0.051 mmol) in DMF (1 mL) there were added *tert*-butyl 4-(piperidin-4-yloxy)piperidine-1-carboxylate (18.73 mg, 0.066 mmol) and N,N-diisopropylethylamine (100 μL, 0.573 mmol) at room temperature, and the mixture was stirred for 2 hours at 80°C. Water was added to the reaction mixture, and the resulting solid was washed with water to obtain a crude product. To a solution of the crude product in dichloromethane (1 mL) there was added TFA (0.5 mL) at room temperature, and the mixture was stirred for 1 hour at room temperature. The reaction mixture was purified by ODS silica gel column chromatography (0.1% formic acid aqueous solution: 0.1% acetonitrile formate solution = 95:5 to 30:70), to obtain a formate of the title compound (13.0 mg).

ESI-MS (m/z): 656.45 $[M+H]^+$.

[Production Example 35-1]

Methyl 2-(2-chloro-6-iodoquinazolin-4-yl)-2-diphenyl acetate

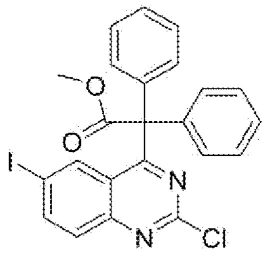

To a solution of 2,4-dichloro-6-iodoquinazoline (2.90 g, 8.93 mmol) and methyl diphenylacetate (2.12 g, 9.37 mmol) in THF (50 mL) there was added dropwise LHMDS (1.43 M THF solution, 8.11 mL, 11.6 mmol) at -78°C, and the mixture was stirred for 2 hours at 0°C. Saturated aqueous ammonium chloride was added to the reaction mixture, and extraction was performed with ethyl acetate. The organic layer was washed with brine, dried over magnesium sulfate, and filtered. The filtrate was concentrated under reduced pressure. The obtained solid was washed with TBME to obtain the title compound (3.68 g). ESI-MS (m/z): 515.04 $[M+H]^+$.

[Production Example 35-2]

2-Chloro-4-(1,1-diphenyl-2-((tetrahydro-2H-pyran-2-yl)oxy)ethyl)-6-iodoquinazoline

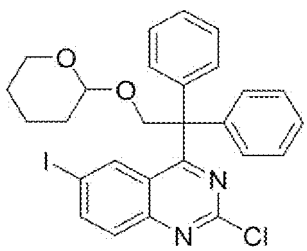

To a solution of the methyl 2-(2-chloro-6-iodoquinazolin-4-yl)-2,2-diphenyl acetate of Production Example 35-1 (15 g, 29 mmol) in toluene (300 mL) there was added dropwise DIBAL-H (1 M toluene solution, 72.9 mL, 72.9 mmol) at -78°C, and after stirring for 30 minutes, the mixture was stirred for 4 hours at 0°C. A saturated aqueous Rochelle salt solution and ethyl acetate were added to the reaction mixture, and stirring was continued for 4 hours. After oil-water distribution of the reaction mixture, the organic layer was washed with brine and dried over magnesium sulfate. The

organic layer was filtered, and the filtrate was then concentrated under reduced pressure to obtain a crude product. To a solution of the obtained crude product in dichloromethane (300 mL) at room temperature there were added DHP (10.7 mL, 117 mmol) and *p*-toluenesulfonic acid monohydrate (1.11 g, 5.83 mmol) at 0°C, and the mixture was stirred for 2 hours at room temperature. A saturated aqueous sodium hydrogencarbonate solution was added to the reaction mixture, and extraction was performed with dichloromethane. The organic layer was dried over anhydrous magnesium sulfate and filtered, and the filtrate was concentrated under reduced pressure. The obtained solid was washed with heptane:TBME = 1:1 to obtain a portion of the title compound. The filtrate was purified by silica gel column chromatography (n-heptane:ethyl acetate = 99:1 to 7:3) to obtain the total amount of title compound (7.76 g). ESI-MS (m/z): 571.14 $[M+H]^+$

[Production Example 35-3]

4-(2-Chloro-4-(1,1-diphenyl-2-((tetrahydro-2H-pyran-2-yl)oxy)ethyl)quinazolin-6-yl)-6-methylfuro[2,3-c]pyridine-7(6H)-one

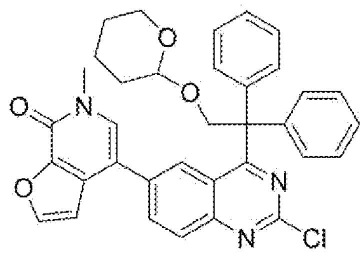

To a solution of the 2-chloro-4-(1,1-diphenyl-2-((tetrahydro-2H-pyran-2-yl)oxy)ethyl)-6-iodoquinazoline of Production Example 35-2 (400 mg, 0.701 mmol) in toluene (1 mL) and ethanol (4 mL) there were added a 2 M sodium carbonate aqueous solution (1 mL), 6-methyl-4-(4,4,5,5-tetramethyl-1,3,2-dioxaborolan-2-yl)furo[2,3-c]pyridine-7(6H)-one (212 mg, 0.771 mmol) and tetrakis(triphenylphosphine)palladium(0) (81 mg, 0.070 mmol) at room temperature, and the mixture was stirred for 4 hours at 80°C under a nitrogen atmosphere. The reaction mixture was returned to room temperature, water was added, and extraction was performed with ethyl acetate. The organic layer was dried over anhydrous sodium sulfate and filtered, and the filtrate was concentrated under reduced pressure. The residue was purified by NH silica gel column chromatography (n-heptane:ethyl acetate = 7:3 to ethyl acetate) to obtain the title compound (177 mg).

ESI-MS (m/z): 592.24 $[M+H]^+$.

[Example 36]

(S)-5-(2-Amino-4-(1-cyclopropoxy-2-hydroxy-1-phenylethyl)quinazolin-6-yl)-1,3-dimethylpyridin-2(1H)-one

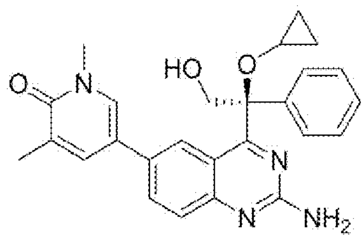

A solution of the 5-(2-amino-4-(1-cyclopropoxy-2-hydroxy-1-phenylethyl)quinazolin-6-yl)-1,3-dimethylpyridin-2(1H)-one of Example 2 (120 mg, 0.271 mmol) in methanol (ca. 30 mg/mL) was resolved by SFC (IG, carbon dioxide:methanol = 75:25), to obtain (S)-5-(2-amino-4-(1-cyclopropoxy-2-hydroxy-1-phenylethyl)quinazolin-6-yl)-1,3-dimethylpyridin-2(1H)-one (48.3 mg) as the former isomer.

$^1H$-NMR Spectrum (400 MHz, $CDCl_3$) $\delta$(ppm): 0.20-0.28 (1H, m), 0.32-0.39 (1H, m), 0.47-0.54 (1H, m), 0.69-0.76 (1H, m), 2.16 (3H, s), 2.99-3.06 (1H, m), 3.29-3.34 (1H, m), 3.53 (3H, s), 4.42-4.45 (1H, m), 4.59-4.63 (1H, m), 5.29 (2H, s), 6.91-6.92 (1H, m), 7.11-7.12 (1H, m), 7.26-7.36 (5H, m), 7.54-7.58 (1H, m), 7.59-7.61 (1H, m), 7.86-7.86 (1H, m).

[Pharmacological Test Examples]

1. BRD4 binding inhibition assay

This assay measures binding inhibition activity of the test substance on the bromodomain of BRD4 protein.

BRD4 binding inhibition activity assay was carried out using a BRD4(BD 1+BD2)TR-FRET Assay Kit (BPS Bioscience Inc. Catalog#32612), according to the attached instructions. In a flat-bottom 384-well white plate (Greiner 781080) there was added 8 μL of Tb-labeled donor/dye-labeled acceptor solution diluted 160-fold with 1 × BRD TR-FRET Assay Buffer1, with 5 μL of BET Bromodomain Ligand diluted 40-fold with 1 × BRD TR-FRET Assay Buffer 1 in the test substance measuring wells and the Positive Control wells, and 5 μL of Non-acetylated Ligand1 diluted 40-fold with 1 × BRD TR-FRET Assay Buffer1 in the Negative Control wells. After adding 2 μL of test substance diluted with 1 × BRD TR-FRET Assay Buffer 1 to the test substance measuring wells, and 2 μL of Dimethyl Sulfoxide solution diluted 100-fold with 1 × BRD TR-FRET Assay Buffer1 to the Positive Control wells and Negative Control wells, 5 μL of BRD4(BD1+BD2) protein solution prepared to 3.6 ng/μL with 1 × BRD TR-FRET Assay Buffer 1 was added and reaction was conducted for 2 hours at room temperature. The fluorescence intensity was measured at 620 nm and 665 nm with irradiation of 340 nm excitation light into each well, using an Envision™ (Perkin-Elmer). The ratio of fluorescence intensity at 655 nm with respect to fluorescence intensity at 620 nm for each well was recorded as the TR-FRET ratio, and the TR-FRET ratio in the presence of the test substance was calculated with the Positive Control well value as 100%, subtracting the Negative Control well value. The concentration of test substance necessary for 50% inhibition of BRD4 binding activity ($IC_{50}$) was calculated from the TR-FRET ratio, and shown in Table 1.

[Table 1]

| | $IC_{50}$ (microM) |
|---|---|
| Example 2 | 0.026 |
| Example 25 | 0.13 |
| Example 26 | 0.047 |
| Example 27 | 0.080 |
| Example 28 | 0.082 |
| Example 29 | 0.057 |
| Example 30 | 0.027 |
| Example 31 | 0.061 |
| Example 32 | 0.081 |
| Example 33 | 0.44 |
| Example 34 | 0.091 |
| Example 35 | 0.087 |
| Example 36 | 0.025 |

[Example 101]

(S)-1-(5-(3-((1'-(4-(1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)dihydropyrimidine-2,4(1H,3H)-dione

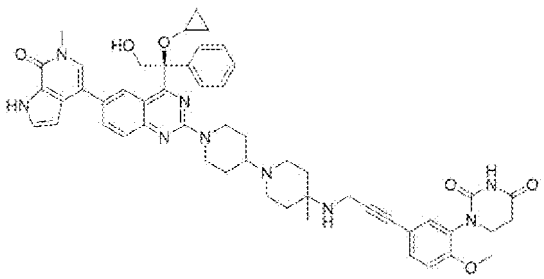

To a solution of the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 101-9 (250 mg, 364 μmol), the 1-(5-iodo-2-methoxyphenyl)dihydropyrimidine-2,4(1H,3H)-dione of Production Example 101-10 (252 mg, 729 μmol) and CuI (34.7 mg, 182 μmol) in DMF (5.65 mL) there were added N,N-diisopropylethylamine (637 μL, 3.65 mmol) and tetrakis(triphenylphosphine)palladium(0) (211 mg, 182 μmol), and the mixture was stirred for 1 hour at 50°C under a nitrogen atmosphere. After cooling to room temperature, the reaction mixture was purified by ODS silica gel chromatography (0.1% formic acid aqueous solution:0.1% acetonitrile formate solution = 9: 1 to 7:3), to obtain a crude product. The crude product was purified by NH silica gel column chromatography (n-heptane:ethyl acetate = 1:1 to ethyl acetate to ethyl acetate:methanol = 7:3) to obtain the title compound (193 mg).

ESI-MS (m/z): 904.69 $[M+H]^+$.

[Production Example 101-1]

Ethyl 2-cyclopropoxy-2-phenylacetate

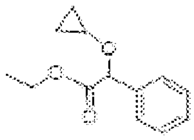

To a solution of cyclopropanol (3.36 g, 57.8 mmol) and rhodium(II) acetate dimer (320 mg, 723 μmol) in dichloromethane (20 mL) there was added dropwise a solution of ethyl 2-diazo-2-phenylacetate (5.50 g, 28.9 mmol) in dichloromethane (10 mL), and the mixture was stirred for 4 hours at room temperature. The reaction mixture was concentrated under reduced pressure and the residue was purified by silica gel column chromatography (n-heptane to n-heptane:ethyl acetate = 4:1) to obtain the title compound (5.83 g).

$^1$H-NMR Spectrum (500 MHz, $CDCl_3$) δ(ppm): 0.43-0.56(2H, m), 0.64-0.78 (2H, m), 1.19-1.24 (3H, m), 3.38-3.45 (1H, m), 4.10-4.26 (2H, m), 4.94 (1H, s), 7.29-7.38 (3H, m), 7.42-7.47 (2H, m).

[Production Example 101-2]

Ethyl 2-(2-chloro-6-iodoquinazolin-4-yl)-2-cyclopropoxy-2-phenylacetate

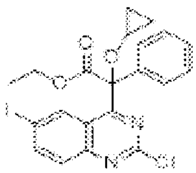

To a solution of the ethyl 2-cyclopropoxy-2-phenylacetate of Production Example 101-1 (661 mg, 3.00 mmol) and 2,4-dichloro-6-iodoquinazoline (1.17 g, 3.60 mmol) in THF (10 mL) there was added dropwise LHMDS (1.09 M THF solution, 3.30 mL, 3.60 mmol) at - 78°C under a nitrogen atmosphere, and the mixture was stirred for 10 minutes at -78°C. The reaction mixture was then stirred for 1 hour at room temperature. Saturated aqueous ammonium chloride was added to the reaction mixture, and extraction was performed with ethyl acetate. The organic layer was washed with brine. The

organic layer was dried over sodium sulfate and filtered. The filtrate was concentrated under reduced pressure and the residue was purified by NH silica gel column chromatography (n-heptane to n-heptane:ethyl acetate = 7:3) to obtain the title compound (1.35 g).

ESI-MS (m/z): 509.25 $[M+H]^+$.

[Production Example 101-3]

Ethyl (R)-2-(2-chloro-6-iodoquinazolin-4-yl)-2-cyclopropoxy-2-phenylacetate ethyl (S)-2-(2-chloro-6-iodoquinazolin-4-yl)-2-cyclopropoxy-2-phenylacetate

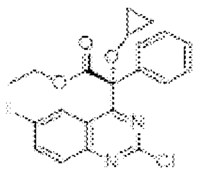

(R)-isomer

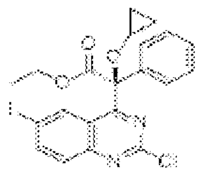

(S)-isomer

The ethyl 2-(2-chloro-6-iodoquinazolin-4-yl)-2-cyclopropoxy-2-phenylacetate of Production Example 101-2 (100 g, 197 mmol) was resolved by supercritical fluid chromatography (SFC) (Waters SFC 350, Whelk-O1(250*50 mm), carbon dioxide:isopropyl alcohol = 70:30), to obtain the R-form of the title compound (48.0 g, 94 mmol, 100% ee) and the S-form of the title compound (48.0 g, 94 mmol, 99% ee). The absolute configuration was determined by X-ray crystallography of the compound of Production Example 101-9.

(R)-isomer, ESI-MS (m/z): 509.14 $[M+H]^+$.

(S)-isomer, ESI-MS (m/z): 509.14 $[M+H]^+$.

X-ray crystallography of the compound of Production Example 101-9 is described below as Reference Example 1.

[Reference Example 1]

Methanol (2 mL) was added to the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 101-9 (1.30 mg). The mixture was filtered, and then a 400 μL portion thereof was placed in a vial, the vial was placed in a vessel containing 2 mL of 1-propanol and the vessel was capped and allowed to stand at room temperature (vapor diffusion method using 1-propanol as the poor solvent). After 3 days, a single crystal was obtained in the vial. The single crystal was analyzed by X-ray diffraction. The crystallographic data and structural analysis data are shown in Table 2, and the atomic coordinate data are shown in Table 3. The absolute configuration of the compound was determined in this manner.

[Table 2]

| | |
|---|---|
| Temperature | 103K |
| Wavelength | 1.54184 Å |
| Crystal System, Space Group | Triclinic, P1 (#1) |
| Lattice Parameters | a = 9.04430(15) Å<br>b = 10.05135(14) Å<br>c = 19.7658(3) Å<br>α = 101.1364(13)<br>β = 97.1938(14)<br>γ = 91.9841(12)<br>V = 1745.95(5) $Å^3$ |
| Z value, calculated density | 2, 1.305 |
| Crystal size | 0.2x0.15x0.01 mm |

(continued)

| | |
|---|---|
| Total number of reflections/number of unique reflections | 38827 / 13007 [R(intensity)0.0391] |
| Completeness | 93.5 % |
| Phase determination | Direct method (SHELXT) |
| Refinement method | Least square method with respect to F2 |
| Data/parameter | 13007 / 919 |
| Goodness of fit indicator | 1.053 |
| R value (whole data) | 0.0563 |
| R value ($I > 2\sigma(I)$) | 0.0487 |
| Flack parameter (Parsons' quotients = 4924) | 0.03(10) |
| Maximum and minimum peak difference | 0.63 e/Å$^3$ and -0.47 e/Å$^3$ |

[Table 3]

| atom | x | y | z | $B_{iso}$ |
|---|---|---|---|---|
| O1 | 0.5727(3) | 0.8391(2) | 0.18250(12) | 2.04(4) |
| O2 | 0.2317(3) | -0.0753(3) | 0.79789(14) | 2.72(5) |
| O3 | 0.7417(3) | 1.0739(2) | 0.18449(14) | 2.54(5) |
| O4 | 0.4905(3) | 0.3124(3) | 0.43852(14) | 2.77(5) |
| O5 | 0.5042(3) | 0.7096(3) | 0.55180(14) | 2.76(5) |
| O26 | 0.3216(3) | 0.1713(3) | 0.76455(14) | 2.77(5) |
| N7 | 0.2839(4) | 0.5156(3) | 0.99112(16) | 2.16(5) |
| N8 | -0.0179(3) | 0.2733(3) | 1.22391(16) | 2.18(5) |
| N9 | 0.2253(4) | 0.2809(3) | 0.93773(16) | 2.21(5) |
| N10 | 0.5025(3) | 0.2439(3) | 0.32196(15) | 2.06(5) |
| N11 | 0.4906(4) | 0.7717(3) | 0.66845(16) | 2.28(5) |
| N12 | 0.1487(4) | 0.3728(3) | 1.04351(17) | 2.29(5) |
| N13 | 0.7815(4) | 0.7155(3) | 0.04800(16) | 2.13(5) |
| N14 | 0.8546(4) | 0.6186(3) | -0.05781(16) | 2.26(5) |
| N15 | 0.7135(3) | 0.4824(3) | -0.00512(16) | 2.15(5) |
| N16 | 0.4553(4) | 0.5917(3) | 0.41132(17) | 2.58(6) |
| N17 | -0.0614(4) | 0.1527(4) | 1.41108(18) | 2.86(6) |
| N18 | 1.0086(3) | 0.7135(3) | -0.24148(16) | 2.21(5) |
| N19 | 0.5501(4) | 0.4309(3) | 0.57569(17) | 2.49(6) |
| N86 | 1.0491(4) | 0.8623(3) | -0.41947(18) | 2.80(6) |
| C20 | 0.8297(4) | 0.8166(3) | 0.22297(19) | 2.17(6) |
| C21 | 0.2201(4) | 0.3942(4) | 0.98920(19) | 2.05(6) |
| C22 | 0.5752(4) | 0.3444(4) | 0.0548(2) | 2.30(6) |
| C23 | 0.6091(4) | 0.5496(4) | 0.17275(19) | 2.10(6) |
| C24 | 0.4679(4) | 0.6839(4) | 0.8731(2) | 2.32(6) |
| C25 | 0.3469(4) | 0.5311(4) | 0.93364(19) | 2.11(6) |
| C27 | 0.4762(4) | 0.4751(4) | 0.3650(2) | 2.17(6) |
| C28 | 0.4795(4) | 0.7388(4) | 0.73263(19) | 2.17(6) |
| C29 | 0.8993(4) | 0.8316(4) | -0.3313(2) | 2.52(7) |
| C30 | 0.8670(4) | 0.7532(4) | -0.2755(2) | 2.56(7) |
| C31 | 0.4904(4) | 0.3418(4) | 0.37972(19) | 2.28(6) |
| C32 | 0.7224(4) | 0.7021(4) | 0.10345(19) | 2.05(6) |
| C33 | 0.6605(4) | 0.5740(4) | 0.11164(19) | 2.03(6) |
| C34 | 0.5053(4) | 0.4002(4) | 0.24275(19) | 1.98(6) |

(continued)

| atom | x | y | z | $B_{iso}$ |
|---|---|---|---|---|
| C35 | 0.9956(4) | 0.6603(4) | -0.17694(19) | 2.21(6) |
| C36 | 0.1248(4) | 0.4368(4) | 1.1677(2) | 2.25(6) |
| C37 | 0.5240(4) | 0.5095(4) | 0.6883(2) | 2.20(6) |
| C38 | 0.4529(4) | 0.8240(4) | 0.12879(19) | 2.37(6) |
| C39 | 0.5218(4) | 0.5438(4) | 0.62313(19) | 2.21(6) |
| C40 | 0.5418(4) | 0.4264(4) | 0.1753(2) | 2.12(6) |
| C41 | 0.4580(4) | 0.5811(4) | 0.8122(2) | 2.14(6) |
| C42 | 0.9765(4) | 0.7889(4) | 0.2124(2) | 2.58(6) |
| C43 | 0.9875(5) | 0.7552(4) | -0.3865(2) | 2.59(7) |
| C44 | 1.0751(4) | 0.6184(4) | -0.2934(2) | 2.45(6) |
| C45 | 0.7189(4) | 0.8294(4) | 0.16012(19) | 2.14(6) |
| C46 | 0.7814(4) | 0.6015(4) | -0.00342(19) | 2.15(6) |
| C47 | 0.4780(4) | 0.5057(4) | 0.2990(2) | 2.12(6) |
| C48 | 0.4931(4) | 0.6122(4) | 0.7454(2) | 2.21(6) |
| C49 | 0.4548(4) | 0.6482(4) | 0.3070(2) | 2.36(6) |
| C50 | 0.3470(4) | 0.4242(4) | 0.87506(19) | 2.03(6) |
| C51 | 0.5046(4) | 0.6773(4) | 0.6096(2) | 2.33(6) |
| C52 | 0.0030(4) | 0.3240(4) | 1.1598(2) | 2.25(6) |
| C53 | 0.8999(5) | 0.8154(4) | 0.3443(2) | 2.86(7) |
| C54 | 0.7621(4) | 0.9580(4) | 0.1341(2) | 2.37(6) |
| C55 | 0.6504(4) | 0.4689(4) | 0.05213(19) | 2.01(6) |
| C56 | -0.0814(4) | 0.3754(4) | 1.2750(2) | 2.38(6) |
| C57 | 0.9716(5) | 0.7276(4) | -0.0493(2) | 2.44(7) |
| C58 | 0.4424(4) | 0.6942(4) | 0.3752(2) | 2.57(7) |
| C59 | 1.0437(5) | 0.7861(4) | 0.3323(2) | 3.09(7) |
| C60 | 0.1308(5) | 0.4862(4) | 1.1000(2) | 2.48(7) |
| C61 | 0.5155(4) | 0.2726(4) | 0.25732(19) | 2.08(6) |
| C62 | 0.5605(5) | 0.3702(4) | 0.6792(2) | 2.63(7) |
| C63 | 0.8676(5) | 0.5032(4) | -0.11403(19) | 2.44(7) |
| C64 | 0.5722(5) | 0.3273(4) | 0.6102(2) | 2.90(7) |
| C65 | 0.5206(4) | 0.3249(4) | 0.1133(2) | 2.24(6) |
| C66 | 1.0820(4) | 0.7752(4) | 0.2667(2) | 2.92(7) |
| C67 | 0.1901(5) | 0.1483(4) | 0.7172(2) | 3.19(8) |
| C68 | 0.9773(5) | 0.7770(4) | -0.1175(2) | 2.54(7) |
| C69 | 1.1184(5) | 0.6918(4) | -0.3496(2) | 2.57(7) |
| C70 | 0.0301(5) | 0.2088(4) | 1.1003(2) | 2.73(7) |
| C71 | 0.1928(5) | 0.0486(4) | 0.8372(2) | 2.75(7) |
| C72 | 0.8704(4) | 0.5506(4) | -0.1824(2) | 2.29(6) |
| C73 | 0.0357(5) | 0.2604(4) | 1.0337(2) | 2.62(7) |
| C74 | 0.5209(5) | 0.1040(4) | 0.3321(2) | 2.41(6) |
| C75 | 0.4135(4) | 0.6597(4) | 0.93158(19) | 2.32(6) |
| C76 | 0.0010(4) | 0.2481(4) | 1.3725(2) | 2.47(7) |
| C77 | 0.4016(4) | 0.4538(4) | 0.8152(2) | 2.25(6) |
| C78 | -0.1278(4) | 0.3114(4) | 1.3333(2) | 2.48(7) |
| C79 | -0.1318(5) | 0.2067(5) | 1.4720(2) | 3.17(8) |
| C80 | 0.4751(5) | 0.9138(4) | 0.6614(2) | 2.76(7) |
| C81 | 0.2882(4) | 0.2943(4) | 0.88281(19) | 2.12(6) |
| C82 | 0.3112(4) | 0.1574(4) | 0.8341(2) | 2.32(6) |
| C83 | 1.2127(5) | 0.9338(5) | -0.4950(2) | 3.01(7) |
| C84 | 0.1071(5) | 0.3597(5) | 1.4197(2) | 3.13(8) |

(continued)

| atom | x | y | z | $B_{iso}$ |
|---|---|---|---|---|
| C85 | 0.1190(4) | 0.2297(4) | 1.2598(2) | 2.40(6) |
| C87 | 0.7930(5) | 0.8308(4) | 0.2900(2) | 2.52(6) |
| C88 | 0.4042(5) | 0.9453(4) | 0.1006(2) | 3.13(7) |
| C89 | 0.3155(5) | 0.8890(5) | 0.1492(2) | 3.32(8) |
| C90 | 0.8863(5) | 0.6477(4) | -0.4382(2) | 3.00(7) |
| C91 | 0.0809(5) | 0.1600(4) | 1.3178(2) | 2.62(7) |
| C92 | 1.1363(5) | 0.8171(5) | -0.4758(2) | 3.06(7) |
| C93 | 0.4675(5) | 0.1183(4) | 0.8628(2) | 2.81(7) |
| C94 | -0.2125(5) | 0.0949(5) | 1.4937(2) | 3.19(8) |
| C95 | 1.2785(5) | 1.0270(5) | -0.5096(2) | 3.31(8) |
| C96 | 0.4849(5) | 0.0465(4) | 0.9161(2) | 3.52(8) |
| C97 | 0.7490(7) | 0.0571(5) | 0.9191(3) | 5.21(13) |
| C98 | 0.1684(6) | 0.2357(5) | 0.6640(2) | 3.65(8) |
| C99 | 0.6254(7) | 0.0142(5) | 0.9431(3) | 4.56(11) |
| C100 | 0.7346(6) | 0.1289(5) | 0.8654(4) | 5.25(12) |
| C101 | 0.5935(5) | 0.1602(5) | 0.8393(3) | 4.01(9) |
| C102 | 0.0716(5) | 0.2476(5) | 0.7211(3) | 3.85(8) |
| C103 | -0.2808(5) | 0.0046(5) | 1.5092(2) | 3.46(8) |
| H2 | 0.16937 | -0.13594 | 0.79879 | 3.261 |
| H3 | 0.76465 | 1.14262 | 0.17066 | 3.051 |
| H10A | 0.08796 | 0.32793 | 0.75796 | 4.623 |
| H10B | -0.03142 | 0.21231 | 0.70881 | 4.623 |
| H17 | -0.05633 | 0.06646 | 1.39776 | 3.437 |
| H22 | 0.56285 | 0.27471 | 0.01565 | 2.765 |
| H23 | 0.62091 | 0.61842 | 0.21224 | 2.514 |
| H24 | 0.51185 | 0.76904 | 0.87335 | 2.781 |
| H28 | 0.46165 | 0.80763 | 0.76893 | 2.610 |
| H29A | 0.95486 | 0.91617 | -0.30893 | 3.022 |
| H29B | 0.80516 | 0.85420 | -0.35443 | 3.022 |
| H30A | 0.81595 | 0.80952 | -0.24126 | 3.071 |
| H30B | 0.80275 | 0.67278 | -0.29646 | 3.071 |
| H35 | 1.09015 | 0.62056 | -0.16444 | 2.649 |
| H36A | 0.10609 | 0.51227 | 1.20364 | 2.700 |
| H36B | 0.22063 | 0.40335 | 1.18212 | 2.700 |
| H38 | 0.43873 | 0.73620 | 0.09626 | 2.840 |
| H42 | 1.00314 | 0.77955 | 0.16787 | 3.095 |
| H44A | 1.00406 | 0.54264 | -0.31427 | 2.942 |
| H44B | 1.16287 | 0.58302 | -0.27126 | 2.942 |
| H49 | 0.44930 | 0.69904 | 0.27228 | 2.827 |
| H52 | -0.09125 | 0.36157 | 1.14469 | 2.698 |
| H53 | 0.87460 | 0.82490 | 0.38913 | 3.435 |
| H54A | 0.86565 | 0.95715 | 0.12587 | 2.838 |
| H54B | 0.70049 | 0.96041 | 0.09052 | 2.838 |
| H56A | -0.16751 | 0.41166 | 1.25200 | 2.851 |
| H56B | -0.00775 | 0.44990 | 1.29421 | 2.851 |
| H57A | 1.06745 | 0.69445 | -0.03468 | 2.933 |
| H57B | 0.95200 | 0.80279 | -0.01339 | 2.933 |
| H58 | 0.42717 | 0.78373 | 0.39466 | 3.078 |
| H59 | 1.11422 | 0.77370 | 0.36873 | 3.707 |
| H60A | 0.03956 | 0.52955 | 1.08824 | 2.973 |

(continued)

| atom | x | y | z | $B_{iso}$ |
|---|---|---|---|---|
| H60B | 0.21384 | 0.55296 | 1.10579 | 2.973 |
| H61 | 0.53205 | 0.20163 | 0.22178 | 2.501 |
| H62 | 0.57365 | 0.31875 | 0.71359 | 3.157 |
| H63A | 0.78371 | 0.43791 | -0.11839 | 2.930 |
| H63B | 0.95851 | 0.45866 | -0.10316 | 2.930 |
| H64 | 0.59232 | 0.23961 | 0.58984 | 3.478 |
| H65 | 0.46828 | 0.24340 | 0.11286 | 2.693 |
| H66 | 1.17958 | 0.75864 | 0.25881 | 3.510 |
| H67 | 0.15453 | 0.05291 | 0.70090 | 3.833 |
| H68A | 0.88604 | 0.82052 | -0.12894 | 3.053 |
| H68B | 1.06022 | 0.84390 | -0.11155 | 3.053 |
| H69A | 1.16119 | 0.62776 | -0.38397 | 3.084 |
| H69B | 1.19485 | 0.76284 | -0.32844 | 3.084 |
| H70A | 0.12361 | 0.16946 | 1.11276 | 3.277 |
| H70B | -0.04941 | 0.13818 | 1.09316 | 3.277 |
| H71A | 0.18886 | 0.04037 | 0.88501 | 3.299 |
| H71B | 0.09548 | 0.07212 | 0.81786 | 3.299 |
| H72A | 0.77521 | 0.58648 | -0.19557 | 2.749 |
| H72B | 0.88464 | 0.47374 | -0.21859 | 2.749 |
| H73A | 0.05859 | 0.18696 | 0.99765 | 3.150 |
| H73B | -0.06137 | 0.29084 | 1.01868 | 3.150 |
| H74A | 0.52795 | 0.04567 | 0.28814 | 2.887 |
| H74B | 0.61030 | 0.10142 | 0.36349 | 2.887 |
| H74C | 0.43645 | 0.07341 | 0.35123 | 2.887 |
| H75 | 0.42053 | 0.72911 | 0.97070 | 2.784 |
| H77 | 0.39915 | 0.38436 | 0.77638 | 2.699 |
| H78A | -0.20646 | 0.24142 | 1.31397 | 2.980 |
| H78B | -0.16841 | 0.38020 | 1.36627 | 2.980 |
| H79A | -0.20116 | 0.27349 | 1.46116 | 3.805 |
| H79B | -0.05653 | 0.25109 | 1.50964 | 3.805 |
| H80A | 0.46639 | 0.96883 | 0.70604 | 3.307 |
| H80B | 0.56144 | 0.94581 | 0.64414 | 3.307 |
| H80C | 0.38744 | 0.91955 | 0.62954 | 3.307 |
| H84A | 0.14463 | 0.41891 | 1.39224 | 3.755 |
| H84B | 0.05431 | 0.41096 | 1.45421 | 3.755 |
| H84C | 0.18897 | 0.31907 | 1.44227 | 3.755 |
| H85A | 0.18760 | 0.30793 | 1.27917 | 2.881 |
| H85B | 0.16745 | 0.16740 | 1.22685 | 2.881 |
| H86 | 1.03389 | 0.94668 | -0.40593 | 3.362 |
| H87 | 0.69662 | 0.85071 | 0.29859 | 3.025 |
| H88A | 0.36232 | 0.93122 | 0.05199 | 3.762 |
| H88B | 0.46207 | 1.03066 | 0.11854 | 3.762 |
| H89A | 0.31973 | 0.94061 | 0.19628 | 3.987 |
| H89B | 0.22001 | 0.84119 | 0.12974 | 3.987 |
| H90A | 0.84965 | 0.58130 | -0.41441 | 3.606 |
| H90B | 0.80364 | 0.68992 | -0.45911 | 3.606 |
| H90C | 0.94178 | 0.60409 | -0.47375 | 3.606 |
| H91A | 0.17241 | 0.13222 | 1.34080 | 3.147 |
| H91B | 0.01796 | 0.07848 | 1.29727 | 3.147 |
| H92A | 1.07104 | 0.76699 | -0.51599 | 3.677 |

(continued)

| atom | x | y | z | $B_{iso}$ |
|---|---|---|---|---|
| H92B | 1.20983 | 0.75653 | -0.46139 | 3.677 |
| H95 | 1.32973 | 1.09960 | -0.52102 | 3.968 |
| H96 | 0.40123 | 0.01939 | 0.93418 | 4.219 |
| H97 | 0.84318 | 0.03838 | 0.93846 | 6.258 |
| H98A | 0.12374 | 0.19311 | 0.61725 | 4.380 |
| H98B | 0.24315 | 0.30875 | 0.66642 | 4.380 |
| H99 | 0.63471 | -0.03686 | 0.97781 | 5.469 |
| H100 | 0.81841 | 0.15554 | 0.84729 | 6.300 |
| H101 | 0.58426 | 0.21142 | 0.80468 | 4.807 |
| H103 | -0.33431 | -0.06597 | 1.52126 | 4.155 |

[Production Example 101-4]

(S)-2-(2-Chloro-6-iodoquinazolin-4-yl)-2-cyclopropoxy-2-phenylethan-1-ol

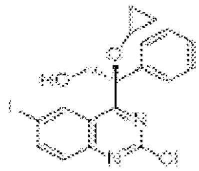

To a solution of the ethyl (S)2-(2-chloro-6-iodoquinazolin-4-yl)-2-cyclopropoxy-2-phenylacetate of Production Example 101-3 (10 g, 19.7 mmol) in toluene (147 mL) there was added dropwise DIBAL-H (1.0 M toluene solution, 47.2 mL) at -78°C over a period of 20 minutes. The reaction mixture was increased in temperature to 0°C and stirred for 10 minutes, after which a saturated aqueous Rochelle salt solution (100 mL) and ethyl acetate (100 mL) were added and the mixture was stirred for 2 hours at room temperature. The organic layer was separated off, and the aqueous layer was extracted with ethyl acetate (100 mL). The organic layer was then washed with brine (100 mL), and dried over sodium sulfate. The organic layer was filtered, and the filtrate was then concentrated under reduced pressure. The residue was purified by silica gel column chromatography (*n*-heptane:ethyl acetate = 9:1 to 7:3) to obtain a crude product of the title compound (8.8 g).

ESI-MS (m/z): 467.09 $[M+H]^+$.

[Production Example 101-5]

(S)-4-(2-Chloro-4-(1-cyclopropoxy-2-hydroxy-1-phenylethyl)quinazolin-6-yl)-6-methyl-1-tosyl-1,6-dihydro-7H-pyrrolo [2,3-c]pyridin-7-one

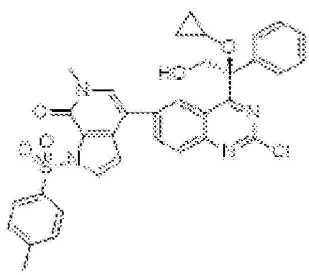

To a solution of the crude product of the (S)-2-(2-chloro-6-iodoquinazolin-4-yl)-2-cyclopropoxy-2-phenylethan-1-ol of Production Example 101-4 (6.30 g) and 6-methyl-4-(4,4,5,5,-tetramethyl-1,3,2-dioxaborolan-2-yl)-1-tosyl-1,6-dihydro-7H-pyrrolo[2,3-c]pyridin-7-one (5.77 g, 13.5 mmol) in toluene (71 mL) there were added ethanol (18.0 mL), sodium carbonate (2 M aqueous solution, 35.3 mL, 70.5 mmol) and tetrakis(triphenylphosphine)palladium(0) (1.48 g, 1.28 mmol) at room temperature, and the mixture was stirred for 24 hours at 70°C. Water and ethyl acetate were added to the reaction mixture, and extraction was performed with ethyl acetate. The organic layer was dried over sodium sulfate and filtered, and the filtrate was concentrated under reduced pressure. The residue was purified by silica gel column chromatography (n-heptane:ethyl acetate = 3:1 to ethyl acetate to ethyl acetate:methanol = 9:1) to obtain a crude product of the title compound (6.76 g).

ESI-MS (m/z): 641.23 $[M+H]^+$.

[Production Example 101-6]

4-Methyl-N-(prop-2-yn-1-yl)piperidine-4-amine

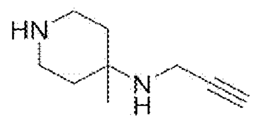

To a solution of *tert*-butyl 4-methyl-4-(prop-2-yn-1-ylamino)piperidine-1-carboxylate (10.0 g, 39.6 mmol) in dichloromethane (25 mL) there was added TFA (25 mL), and the mixture was stirred for 1 hour at room temperature. The reaction mixture was concentrated under reduced pressure and the residue was purified by ODS silica gel column chromatography (0.1% formic acid aqueous solution:0.1% acetonitrile formate solution = 1:0), to obtain a formate of the title compound (9.80 g).

ESI-MS (m/z): 153.05 $[M+H]^+$.

[Production Example 101-7]

*tert*-Butyl 4-methyl-4-(prop-2-yn-1-ylamino)-[1,4'-bipiperidine]-1'-carboxylate

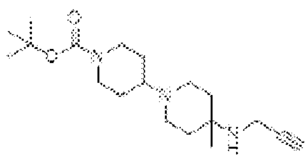

To a solution of the formate of 4-methyl-N-(prop-2-yn-1-yl)piperidine-4-amine obtained in Production Example 101-6 (4.2 g) in THF (30 mL) and dichloromethane (30 mL) there was added N,N-diisopropylethylamine (15 mL), and the mixture was stirred for 5 minutes at room temperature. After then adding BOC-4-piperidone (5.14 g, 25.8 mmol), the mixture was stirred for 1 hour at room temperature. The reaction mixture was cooled to 0°C, sodium triacetoxyborohydride (>80 wt%, 9.11 g) was added, and the mixture was stirred overnight at room temperature. Water, ethyl acetate and 1 N hydrochloric acid were then added to the reaction mixture. The aqueous layer was separated off and washed with ethyl acetate to remove the unreacted ketone and its reduced form. The aqueous layer was neutralized with a saturated aqueous sodium hydrogencarbonate solution and extracted 3 times with a chloroform:isopropyl alcohol = 4:1 solution. The organic layer was washed with brine and dried over magnesium sulfate. The organic layer was filtered and concentrated under reduced pressure. The residue was purified by NH silica gel column chromatography (n-heptane:ethyl acetate = 75:25 to 0:100) to obtain the title compound (4.2 g).

ESI-MS (m/z): 336.32 $[M+H]^+$.

[Production Example 101-8]

(S)-4-(4-(1-Cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1-tosyl-1,6-dihydro-7H-pyrrolo[2,3-c]pyridin-7-one

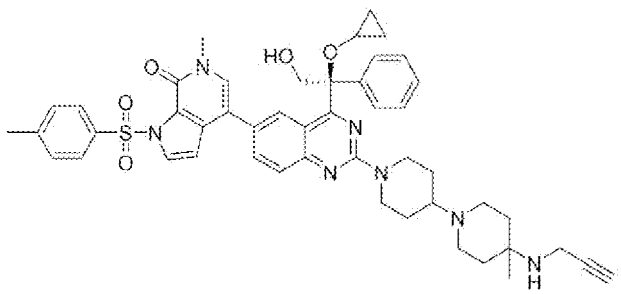

To a solution of the *tert*-butyl 4-methyl-4-(prop-2-yn-1-ylamino)-[1,4'-bipiperidine]-1'-carboxylate of Production Example 101-7 (1.56 g, 4.65 mmol) in dichloromethane (9.78 mL) there was added TFA (9.56 mL), and the mixture was stirred for 30 minutes at room temperature. The reaction mixture was concentrated under reduced pressure and processed by azeotropic distillation with toluene, to obtain an intermediate. To a solution of the obtained intermediate in DMF (26.9 mL) there were added N,N-diisopropylethylamine (8.10 mL) and the crude product of the (S)-4-(2-chloro-4-(1-cyclopropoxy-2-hydroxy-1-phenylethyl)quinazolin-6-yl)-6-methyl-1-tosyl-1,6-dihydro-7H-pyrrolo[2,3-c]pyridin-7-one of Production Example 101-5 (2.21 g, azeotropic distillation with toluene before use). After stirring the reaction mixture for 1 hour at 85°C, N,N-diisopropylethylamine (1.62 mL) was added and the mixture was stirred for 2 hours at 85°C. After further adding N,N-diisopropylethylamine (1.08 mL), the mixture was stirred for 1 hour at 85°C. The reaction mixture was cooled to room temperature and then concentrated under reduced pressure to 1/4 volume, and ethyl acetate (50 mL) and an aqueous sodium hydrogencarbonate solution (30 mL) were added. The organic layer was separated off, and the aqueous layer was extracted twice with ethyl acetate (50 mL). The combined organic layers were dried over sodium sulfate and filtered, and the filtrate was concentrated under reduced pressure. The residue was purified by NH silica gel column chromatography (n-heptane:ethyl acetate = 1:1 to ethyl acetate to ethyl acetate:methanol = 7:3) to obtain the title compound (1.91 g).

ESI-MS (m/z): 840.42 $[M+H]^+$.

[Production Example 101-9]

(S)-4-(4-(1-Cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one

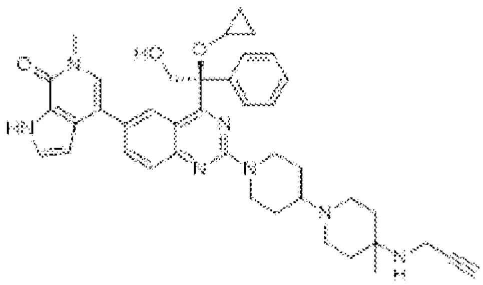

To a solution of the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1-tosyl-1,6-dihydro-7H-pyrrolo[2,3-c]pyridin-7-one of Production Example 101-8 (1.85 g, 2.20 mmol) in ethanol (44.9 mL) there was added a sodium hydroxide aqueous solution (1 M, 8.81 mL, 8.81 mmol), and the mixture was stirred for 18 hours at room temperature. After diluting with 10 mL of water, extraction was performed with chloroform. After washing the organic layer with brine and drying over sodium sulfate, it was filtered and the filtrate was concentrated under reduced pressure. Ethyl acetate (10 mL) was added to the residue and the precipitated solid was filtered. The obtained solid was further washed with ethyl acetate (10 mL) to obtain the title compound (1.25 g).

ESI-MS (m/z): 686.58 $[M+H]^+$.

[Production Example 101-10]

1-(5-Iodo-2-methoxyphenyl)dihydropyrimidine-2,4(1H,3H)-dione

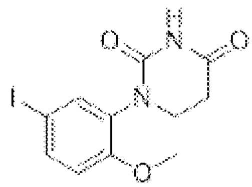

After adding acrylic acid (10.3 mL, 151 mmol) and acetic acid (20 mL) to 5-iodo-2-methoxyaniline (15 g, 60 mmol), the mixture was stirred for 3 hours at 110°C under a nitrogen atmosphere. The reaction mixture was cooled to room temperature, and then urea (14.5 g, 241 mmol) and acetic acid (20 mL) were added and the mixture was stirred for 21.5 hours at 110°C under a nitrogen atmosphere. The reaction mixture was poured onto crushed ice and the mixture was stirred for 1 hour at room temperature. Ultrasonic waves were applied to the reaction mixture for 30 minutes, and the precipitated solid was filtered out and washed with water. A mixed solvent of diethyl ether and ethyl acetate (6:1,400 mL) was added to the obtained solid, and ultrasonic waves were applied for 20 minutes. The precipitated solid was filtered out and washed with diethyl ether to obtain the title compound (12 g).

ESI-MS (m/z): 347.05 $[M+H]^+$.

[Example 102]

(S)-1-(5-((1'-4-(1-Cyclopropoxy-2-hydroxy-1-phenylethyl)-6-(6-methyl-7-oxo-6,7-dihydro-1H-pyrrolo[2,3-c]pyridin-4-yl) quinazolin-2-yl)-4-hydroxy-[1,4'-bipiperidin]-4-yl)ethynyl)-2-methoxyphenyl)dihydropyrimidine-2,4-(1H,3H)-dione

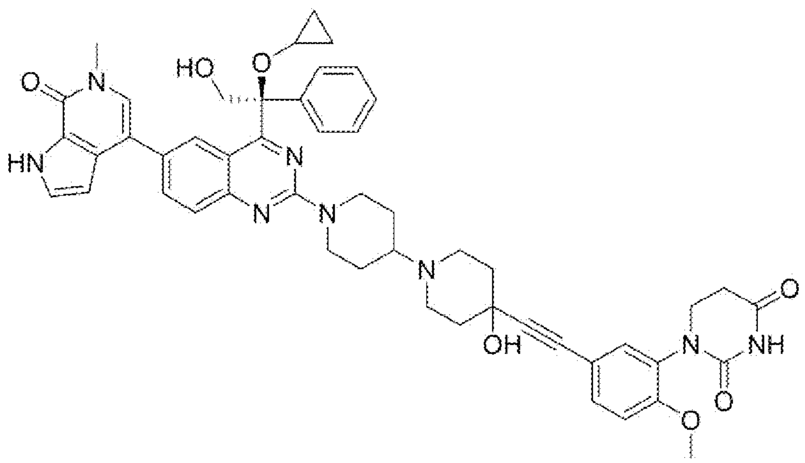

To a solution of the 1-(5-iodo-2-methoxyphenyl)dihydropyrimidine-2,4(1H,3H)-dione of Production Example 101-10 (24 mg, 0.69 mmol) and CuI (3.3 mg, 17 μmol) in DMF (0.69 mL) there were added N,N-diisopropylethylamine (0.17 mL, 34 μmol), tetrakis(triphenylphosphine)palladium(0) (20 mg, 17 μmol) and the (S)-4-(4-(1-cyclopropoxy-2-hydroxy-1-phenylethyl)-2-(4-ethynyl-4-hydroxy-[1,4'-bipiperidin]-1'-yl)quinazolin-6-yl)-6-methyl-1,6-dihydro-7H-pyrrolo [2,3-c]pyridin-7-one of Production Example 102-7 (23 mg, 34 μmol) under a nitrogen atmosphere, and the mixture was stirred for 1 hour at 50°C. Tetrakis(triphenylphosphine)palladium(0) (20 mg, 17 μmol) and CuI (3.3 mg, 17 μmol) were added to the reaction mixture, which was then stirred for 1 hour at 50°C. After cooling the reaction mixture to room temperature, it was purified by ODS silica gel chromatography (0.1% formic acid aqueous solution:0.1% acetonitrile formate solution = 19:1 to 0:1), to obtain the title compound (22.4 mg).

ESI-MS (m/z): 877.30 $[M+H]^+$.

[Production Example 102-1]

2-Chloro-4-((1S)-cyclopropoxy-1-phenyl-2-((tetrahydro-2H-pyran-2-yl)oxy)ethyl)-6-iodoquinazoline

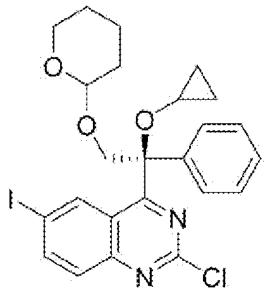

The title compound (150 mg) was obtained from the ethyl (S)-2-(2-chloro-6-iodoquinazolin-4-yl)-2-cyclopropoxy-2-phenylacetate of Production Example 101-3 (155 mg, 305 μmol) by the same method as Production Example 104-3.

ESI-MS (m/z): 551.17 $[M+H]^+$.

[Production Example 102-2]

4-(2-Chloro-4-((1S)-1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one

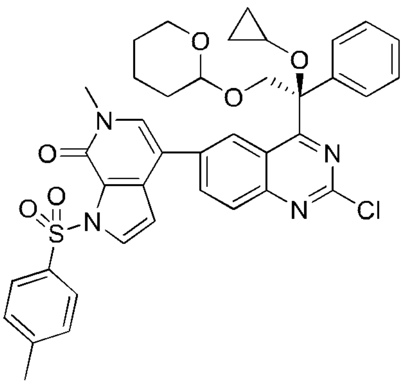

The title compound (35 mg) was obtained from the 2-chloro-4-((1S)-cyclopropoxy-1-phenyl-2-((tetrahydro-2H-pyran-2-yl)oxy)ethyl)-6-iodoquinazoline of Production Example 102-1 (30 mg, 54 μmol), by the same method as Production Example 101-5.

ESI-MS (m/z): 725.38 $[M+H]^+$.

[Production Example 102-3]

*tert*-Butyl 4-hydroxy-4-((triethylsilyl)ethynyl)piperidine-1-carboxylate

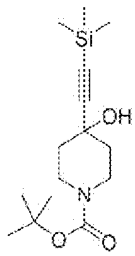

To a solution of trimethylsilylacetylene (2.71 g, 27.6 mmol) in THF (50 mL) there was added n-butyllithium (2.6 M, 10.6 mL, 27.6 mmol) at -78°C, and the mixture was stirred for 50 minutes. After then adding Boc-4-piperidone (5.00 g, 25.1 mmol) to the reaction mixture, it was stirred for 3 hours and 10 minutes at room temperature. Saturated aqueous ammonium chloride was added to the reaction mixture, and extraction was performed 3 times with ethyl acetate. The organic layer was dried over anhydrous sodium sulfate and filtered, and the filtrate was concentrated under reduced pressure. The residue was purified by silica gel column chromatography (n-heptane:ethyl acetate = 1:4) to obtain the title compound (6.17 g).

$^1$H-NMR Spectrum (400 MHz, $CDCl_3$) δ(ppm): 0.16 (9H, s), 1.45 (9H, s), 1.60-1.73 (2H, m), 1.82-1.93 (2H, m), 2.01 (1H, s), 3.17-3.27 (2H, m), 3.70-3.90 (2H, m).

[Production Example 102-4]

*tert*-Butyl 4-ethynyl-4-hydroxypiperidine-1-carboxylate

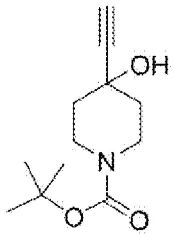

To a solution of the *tert*-butyl 4-hydroxy-4-((triethylsilyl)ethynyl)piperidine-1-carboxylate of Production Example 102-3 (6.17 g, 20.7 mmol) in THF (100 mL) there was added tetrabutylammonium fluoride (1 M, 22.8 mL, 22.8 mmol) at 0°C, and the mixture was stirred for 3 hours at room temperature. Water was added to the reaction mixture, which was then extracted 3 times with ethyl acetate. The organic layer was dried over anhydrous sodium sulfate and filtered, and the filtrate was concentrated under reduced pressure. The residue was purified by silica gel column chromatography (n-heptane:ethyl acetate = 1:4) to obtain the title compound (4.43 g).

$^1$H-NMR Spectrum (400 MHz, $CDCl_3$) δ(ppm): 1.44 (9H, s), 1.65-1.75 (2H, m), 1.84-1.95 (2H, m), 2.04 (1H, s), 2.52 (1H, s), 3.22-3.29 (2H, m), 3.65-3.89 (2H, m).

[Production Example 102-5]

*tert*-Butyl 4-ethynyl-4-hydroxy-[1,4'-bipiperidine]-1'-carboxylate

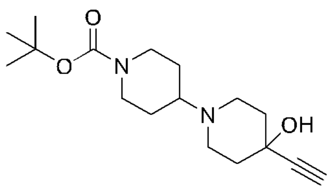

To a solution of the *tert*-butyl 4-ethynyl-4-hydroxypiperidine-1-carboxylate of Production Example 102-4 (300 mg, 1.33 mmol) in dichloromethane (5.1 mL) there was added TFA (0.2 mL), and the mixture was stirred for 1 hour at room

temperature. The reaction mixture was concentrated under reduced pressure and processed by azeotropic distillation with toluene. To a solution of the residue in THF (10.9 mL) there were added N,N-diisopropylethylamine (233 μL, 1.33 mmol), BOC-4-piperidone (398 mg, 2.00 mmol) and sodium triacetoxyborohydride (>80 wt%, 847 mg), and the mixture was stirred for 1 day at room temperature. A saturated aqueous sodium hydrogencarbonate solution was added to the reaction mixture, and extraction was performed 3 times with dichloromethane. The organic layer was washed with water and brine, dried over anhydrous magnesium sulfate and filtered, and the filtrate was concentrated under reduced pressure. The residue was purified by NH silica gel column chromatography (ethyl acetate:methanol = 1:0 to 0:1) to obtain the title compound in crude form (530 mg).

ESI-MS (m/z): 309.26 [M+H]+.

[Production Example 102-6]

(S)-4-(4-(1-Cyclopropoxy-2-hydroxy-1-phenylethyl)-2-(4-ethynyl-4-hydroxy-[1,4'-bipiperidin]-1'-yl)quinazolin-6-yl)-6-methyl-1-tosyl-1,6-dihydro-7H-pyrrolo[2,3-c]pyridin-7-one

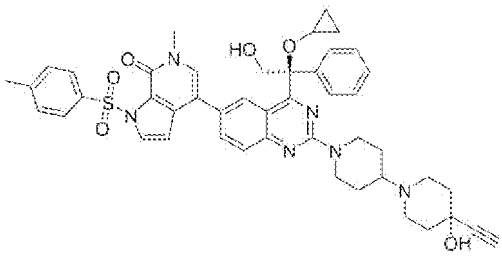

To a solution of the *tert*-butyl 4-ethynyl-4-hydroxy-[1,4'-bipiperidine]-1'-carboxylate of Production Example 102-5 (80 mg, 0.26 mmol) in dichloromethane (1 mL) there was added TFA (0.2 mL), and the mixture was stirred for 45 minutes at room temperature. The reaction mixture was concentrated under reduced pressure and processed by azeotropic distillation with toluene, to obtain a crude product. Separately, TFA (0.2 mL) was added to a solution of the 4-(2-chloro-4-((1S)-1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 102-2 (170 mg, 234 μmol) in dichloromethane (1 mL), and the mixture was stirred for 20 minutes at room temperature. A saturated aqueous sodium hydrogencarbonate solution was added to the reaction mixture, and extraction was performed 3 times with dichloromethane. The organic layer was washed with brine and then dried over anhydrous magnesium sulfate. The organic layer was filtered, and the filtrate was concentrated under reduced pressure to obtain a crude product. To a solution of the two crude products in DMF (2 mL) there was added N,N-diisopropylethylamine (409 μL, 2.34 mmol), and the mixture was stirred for 4 hours at 70°C. The reaction mixture was purified by NH silica gel column chromatography (ethyl acetate to ethyl acetate:methanol = 1:0 to 1:1) and then by ODS silica gel chromatography (0.1% formic acid aqueous solution:0.1% acetonitrile formate solution = 4:1 to 0:1) to obtain the title compound (90.3 mg).

ESI-MS (m/z): 813.72 [M+H]+.

[Production Example 102-7]

(S)-4-(4-(1-Cyclopropoxy-2-hydroxy-1-phenylethyl)-2-(4-ethynyl-4-hydroxy-[1,4'-bipiperidin]-1'-yl)quinazolin-6-yl)-6-methyl-1,6-dihydro-7H-pyrrolo[2,3-c]pyridin-7-one

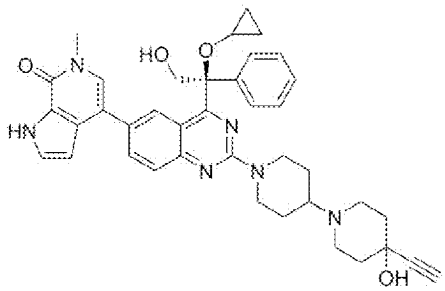

To a solution of the (S)-4-(4-(1-cyclopropoxy-2-hydroxy-1-phenylethyl)-2-(4-ethynyl-4-hydroxy-[1,4'-bipiperidin]-1'-yl)quinazolin-6-yl)-6-methyl-1-tosyl-1,6-dihydro-7H-pyrrolo[2,3-c]pyridin-7-one of Production Example 102-6 (90.4 mg, 111 µmol) in methanol (1.1 mL) and THF (1.1 mL) there was added a sodium hydroxide aqueous solution (2 M, 445 µL, 0.89 mmol), and the mixture was stirred for 4 hours at room temperature. Water was added to the reaction mixture, which was then extracted with dichloromethane. The organic layer was washed with brine, dried over anhydrous magnesium sulfate and filtered, and the filtrate was concentrated under reduced pressure. The residue was purified by NH silica gel column chromatography (ethyl acetate:methanol = 1:0 to 1:1) to obtain the title compound (67.7 mg).
ESI-MS (m/z): 659.35 [M+H]

[Example 103]

(S)-1-(3-(4-((1-(4-(1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)azetidin-3-yl)oxy)piperidine-1-carbonyl)phenyl)dihydropyrimidine-2,4(1H,3H)-dione

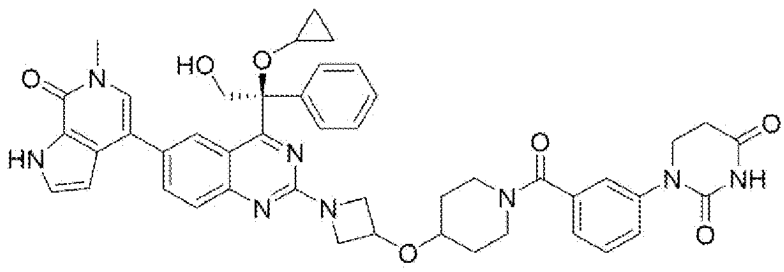

To a solution of the (S)-4-(4-(1-cyclopropoxy-2-hydroxy-1-phenylethyl)-2-(3-(piperidin-4-yloxy)azetidin-1-yl) quinazolin-6-yl)6-methyl-1,6-dihydro-7H-pyrrolo[2,3-c]pyridin-7-one of Production Example 103-5 (17.5 mg, 29.0 µmol) and the 3-(2,4-dioxotetrahydropyrimidine-1(2H)-yl)benzoic acid of Production Example 103-6 (8.78 mg, 37.0 µmol) in DMF (1000 µL) there were added N,N-diisopropylethylamine (10.1 µL, 58.0 µmol) and HATU (21.9 mg, 58.0 µmol), and the mixture was stirred for 3 hours and 30 minutes at room temperature. The reaction mixture was purified by ODS silica gel chromatography (0.1% formic acid aqueous solution:0.1% acetonitrile formate solution = 19:1 to 9:11), to obtain a crude product. The obtained crude product was purified by NH silica gel thin-layer chromatography (ethyl acetate:methanol = 9:1) to obtain the title compound (10.5 mg). ESI-MS (m/z): 823.31 $[M+H]^+$.

[Production Example 103-1]

*tert*-Butyl 3-(pyridin-4-yloxy)azetidine-1-carboxylate

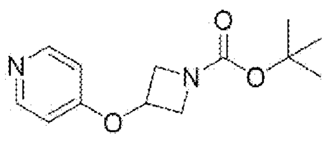

To a solution of N-Boc-3-hydroxyazetidine (1138 mg, 6.57 mmol, 4-hydroxypyridine (500 mg, 5.26 mmol) and triphenylphosphine (1793 mg, 6.84 mmol) in THF (15 mL) there was added diisopropyl azodicarboxylate (2.2 M, 3.11 mL,

6.84 mmol), and the mixture was stirred for 9 hours at 55°C. The reaction mixture was returned to room temperature, and then water was added and extraction was performed with ethyl acetate. The organic layer was washed with brine, dried over anhydrous sodium sulfate and filtered, and the filtrate was concentrated under reduced pressure. The residue was purified by silica gel column chromatography (n-heptane:ethyl acetate = 1:1 to ethyl acetate) to obtain the title compound (525 mg).

$^1$H NMR (400 MHz, $CDCl_3$): δ= 1.44 (9H, s), 3.93-4.08 (2H, m), 4.27-4.42 (2H, m), 4.89-4.97 (1H, m), 6.64-6.68 (2H, m), 8.44-8.48 (2H, m).

[Production Example 103-2]

Benzyl 4-(azetidin-3-yloxy)piperidine-1-catboxylate

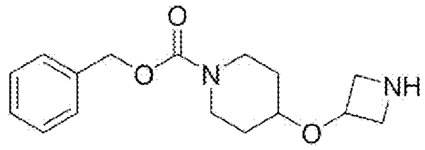

To a solution of the *tert-butyl* 3-(pyridin-4-yloxy)azetidine-1-carboxylate of Production Example 103-1 (678 mg, 2.71 mmol) in acetic acid (15 mL) there was added platinum(IV) oxide (338 mg, 1.49 mmol), and the mixture was stirred for 15 hours at 70°C under a hydrogen atmosphere. The reaction mixture was returned to room temperature and filtered with Celite under a nitrogen atmosphere. The filtrate was concentrated under reduced pressure and processed 3 times by azeotropic distillation with toluene, to obtain a crude product of *tert*-butyl 3-(piperidin-4-yloxy)azetidine-1-carboxylate. To a solution of the obtained crude product in THF (30 mL) there were added triethylamine (7.55 mL, 54.1 mmol) and benzyl chloroformate (464 μL, 3.25 mmol), and the mixture was stirred for 5 hours. Water was added to the reaction mixture, and extraction was performed with ethyl acetate. The organic layer was washed with brine, dried over anhydrous sodium sulfate and filtered, and the filtrate was concentrated under reduced pressure. The residue was purified by silica gel column chromatography (*n*-heptane:ethyl acetate = 0:1 to 1:0) to obtain a reaction intermediate. To a solution of the obtained reaction intermediate in dichloromethane (15 mL) there was added TFA (4 mL), and the mixture was stirred for 2 hours. The reaction mixture was concentrated under reduced pressure and processed twice by azeotropic distillation with toluene. A saturated aqueous sodium hydrogencarbonate solution was added to the obtained residue, and extraction was performed 3 times with dichloromethane. The organic layer was washed with brine, dried over anhydrous sodium sulfate, and filtered. The filtrate was concentrated under reduced pressure to obtain the title compound (153 mg).

$^1$H NMR (400 MHz, $CDCl_3$): δ= 1.40-1.85 (4H, m), 3.10-3.24 (2H, m), 3.41-3.52 (1H, m), 3.56-3.72 (4H, m), 3.76-3.92 (2H, m), 4.35-4.45 (1H, m), 5.11 (2H, s), 7.28-7.40 (5H, m).

[Production Example 103-3]

Benzyl 4-((1-(4-((1S)-1-cyclopropoxy-1-phenyl-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)azetidin-3-yl)oxy)piperidine-1-carboxylate

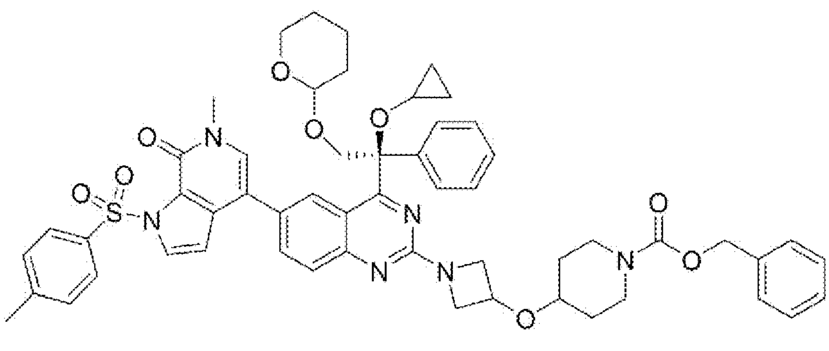

To a solution of the 4-(2-chloro-4-((1S)-1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 102-2 (200 mg, 276 μmol) and the benzyl 4-(azetidin-3-yloxy)piperidine-1-carboxylate of Production Example 103-2 (96.0 mg, 331 μmol) in DMF (2000 μL) there was added N,N-diisopropylethylamine (96 μL, 0.55 mol), and the mixture was stirred for 5 hours and 30 minutes at 80°C. The reaction mixture was returned to room temperature and concentrated under reduced pressure, and the residue was purified by NH silica gel column chromatography (*n*-heptane:ethyl acetate = 1:1 to ethyl acetate) to obtain the title compound (232 mg).

ESI-MS (m/z): 979.26 $[M+H]^+$.

[Production Example 103-4]

4-(4-((1S)-1-Cyclopropoxy-1-phenyl-2-((tetrahydro-2H-pyran-2-yl)oxy)ethyl)-2-(3-(piperidin-4-yloxy)azetidin-1-yl)quinazolin-6-yl)-6-methyl-1-tosyl-1,6-dihydro-7H-pyrrolo[2,3-c]pyridin-7-one

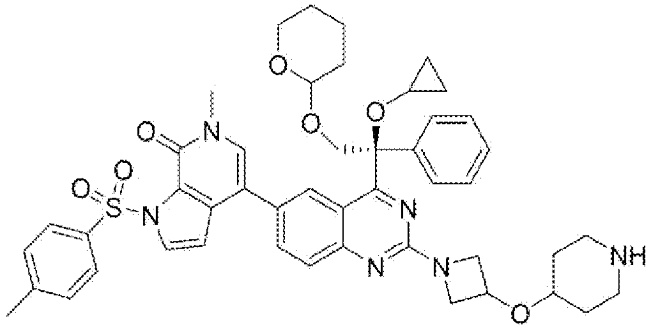

To a solution of the benzyl 4-((1-(4-((1S)-1-cyclopropoxy-1-phenyl-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)azetidin-3-yl)oxy)piperidine-1-carboxylate of Production Example 103-3 (139 mg, 142 μmol) in ethanol (3000 μL) and THF (1000 μL) there was added 10% palladium-carbon (50% water, 90 mg), and the mixture was stirred for 4 hours at room temperature, ordinary pressure under a hydrogen atmosphere. The reaction mixture was placed under a nitrogen atmosphere and filtered with Celite, and the filtrate was concentrated under reduced pressure to obtain the title compound (112 mg).

ESI-MS (m/z): 845.50 $[M+H]^+$.

[Production Example 103-5]

(S)-4-(4-(1-Cyclopropoxy-2-hydroxy-1-phenylethyl)-2-(3-(piperidin-4-yloxy)azetidin-1-yl)quinazolin-6-yl)-6-methyl-1,6-dihydro-7H-pyrrolo[2,3-c]pyridin-7-one

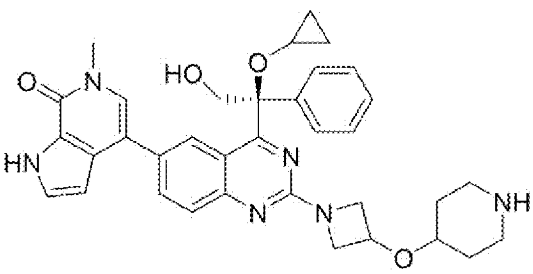

To a solution of the 4-(4-((1S)-1-cyclopropoxy-1-phenyl-2-((tetrahydro-2H-pyran-2-yl)oxy)ethyl)-2-(3-(piperidin-4-yloxy)azetidin-1-yl)quinazolin-6-yl)-6-methyl-1-tosyl-1,6-dihydro-7H-pyrrolo[2,3-c]pyridin-7-one of Production Example 103-4 (112 mg, 133 μmol) in ethanol (4000 μL) there was added a 1 N sodium hydroxide aqueous solution (665 μL, 665 μmol), and the mixture was stirred for 1 hour at 50°C. The reaction mixture was then concentrated under reduced pressure. The residue was added to water and the mixture was extracted with dichloromethane. The organic layer was washed with brine, dried over anhydrous sodium sulfate and filtered, and the filtrate was concentrated under reduced pressure. To a solution of the residue in dichloromethane (5000 μL) there was added TFA (1025 μL), and the mixture was

stirred for 2 hours and 40 minutes. A 1 N sodium hydroxide aqueous solution was added to the reaction mixture, which was then extracted 3 times with dichloromethane. The organic layer was dried over anhydrous sodium sulfate and filtered, and the filtrate was concentrated under reduced pressure. The residue was purified by NH silica gel column chromatography (ethyl acetate:methanol = 1:0 to 9:1) to obtain the title compound (34.5 mg).

ESI-MS (m/z): 607.25 $[M+H]^+$.

[Production Example 103-6]

3-(2,4-Dioxotetrahydropyrimidine-1(2H)-yl)benzoic acid

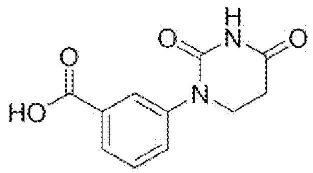

The title compound (1.95 g) was obtained from 3-aminobenzoic acid (3.00 g, 21.9 mmol) by the same method as Example 101-10.

ESI-MS (m/z): 235.093 $[M+H]^+$.

[Example 104]

N-(1'-(4-(1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)-3-(2,4-dioxotetrahydropyrimidine-1(2H)-yl)-4-methoxybenzamide

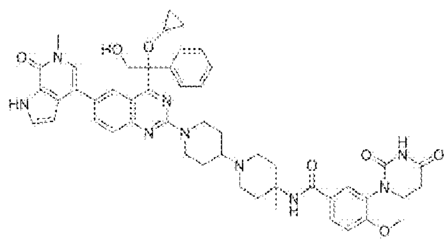

To a solution of the *tert-butyl* (1'-(4-(1-cyclopropoxy-1-phenyl-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)carbamate of Production Example 104-6 (36 mg, 43 μmol) in dichloromethane (1 mL) there was added TFA (1 mL) at room temperature, and the mixture was stirred for 1.5 hours at room temperature. The reaction mixture was then concentrated under reduced pressure. To a solution of the residue in DMF (1 mL) there were added N,N-diisopropylethylamine (151 μL, 865 μmol), the 3-(2,4-dioxotetrahydropyrimidine-1 (2H)-yl)-4-methoxybenzoic acid of Production Example 104-7 (13 mg, 48 μmol) and HATU (20 mg, 52 μmol) at room temperature, and the mixture was stirred for 1 hour at room temperature. Water was added to the reaction mixture, and extraction was performed with ethyl acetate. The organic layer was washed with water and brine, dried over anhydrous magnesium sulfate and filtered, and the filtrate was concentrated under reduced pressure. The residue was purified by NH silica gel thin-layer chromatography (ethyl acetate:methanol = 10:1, followed by development 4 times with ethyl acetate:methanol = 10:3), to obtain the title compound (22 mg).

ESI-MS (m/z): 894.63 $[M+H]^+$.

[Production Example 104-1]

Benzyl 4-((*tert*-butoxycarbonyl)amino)-4-methyl-[1,4'-bipiperidine]-1'-carboxylate

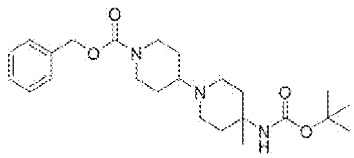

To a solution of *tert-butyl* (4-methylpiperidin-4-yl)carbamate (1286 mg, 6.00 mmol) in dichloromethane (10 mL) and acetic acid (172 μL, 3.00 mmol) there was added 1-(benzyloxycarbonyl)-4-piperidinone (700 mg, 3.00 mmol) at room temperature, and the mixture was stirred for 5 minutes at room temperature. Sodium triacetoxyborohydride (>80 wt%, 827 mg) was then added to the reaction mixture at room temperature, and the mixture was stirred for 19 hours and 25 minutes at room temperature. Water was added to the reaction mixture at room temperature, and extraction was performed with ethyl acetate. The organic layer was washed with brine, dried over anhydrous magnesium sulfate and filtered, and the filtrate was concentrated under reduced pressure. The residue was purified by NH silica gel column chromatography (n-heptane:ethyl acetate = 6:4 to ethyl acetate) to obtain the title compound (941 mg).

ESI-MS (m/z): 432.41 $[M+H]^+$.

[Production Example 104-2]

*tert*-Butyl (4-methyl-[1,4'-bipiperidin]-4-yl)carbamate

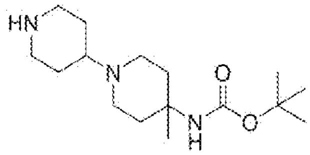

To a solution of the benzyl 4-((*tert*-butoxycarbonyl)amino)-4-methyl-[1,4'-bipiperidine]-1'-carboxylate of Production Example 104-1 (279 mg, 646 μmol) in methanol (10 mL) there was added 10% palladium-carbon (50% water, 138 mg) at room temperature, and the mixture was stirred for 2 hours and 25 minutes at room temperature, ordinary pressure under a hydrogen atmosphere. After switching the reaction mixture to a nitrogen atmosphere, it was filtered with Celite and washed with ethyl acetate. The filtrate was concentrated under reduced pressure to obtain the title compound (192 mg).

ESI-MS (m/z): 298.30 $[M+H]^+$.

[Production Example 104-3]

2-Chloro-4-(1-cyclopropoxy-1-phenyl-2-((tetrahydro-2H-pyran-2-yl)oxy)ethyl-6-iodoquinazoline

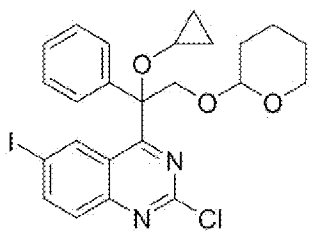

To a solution of the ethyl 2-(2-chloro-6-iodoquinazolin-4-yl)-2-cyclopropoxy-2-phenylacetate of Production Example 101-2 (1.08 g, 2.12 mmol) in dichloromethane (10 mL) there was added DIBAL-H (1.0 M toluene solution, 4.67 mL, 4.67 mmol) at -78°C, and the mixture was stirred for 10 minutes. The reaction mixture was increased in temperature to 0°C and stirred for 10 minutes. A saturated aqueous Rochelle salt solution was added to the reaction mixture, which was then stirred overnight at room temperature. The reaction mixture was extracted with ethyl acetate. The organic layer was washed with brine, dried over anhydrous sodium sulfate and filtered, and the filtrate was concentrated under reduced pressure. To a solution of the residue in dichloromethane (10 mL) there were added DHP (576 mL, 6.37 mmol) and CSA (99 mg, 0.43 mmol) at room temperature, and the mixture was stirred for 1 hour at room temperature. A

saturated aqueous sodium hydrogencarbonate solution was added to the reaction mixture, and extraction was performed with ethyl acetate. The organic layer was washed with brine, dried over anhydrous sodium sulfate and filtered, and the filtrate was concentrated under reduced pressure. The residue was purified by NH silica gel column chromatography (n-heptane:ethyl acetate = 95:5 to 8:2) to obtain the title compound (733 mg).

ESI-MS (m/z): 551.25 $[M+H]^+$.

[Production Example 104-4]

4-(2-Chloro-4-(1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one

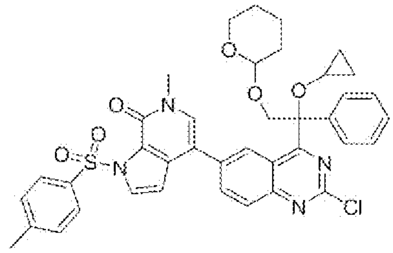

To a solution of the 2-chloro-4-(1-cyclopropoxy-1-phenyl-2-((tetrahydro-2H-pyran-2-yl)oxy)ethyl-6-iodoquinazoline of Production Example 104-3 (4.00 g, 7.26 mmol) and 6-methyl-4-(4,4,5,5-tetramethyl-1,3,2-dioxaboran-2-yl)-1-tosyl-1,6-dihydro-7H-pyrrolo[2,3-c]pyridin-7-one (3.73 g, 8.71 mmol) in toluene (70 mL), ethanol (17.5 mL) and aqueous 2 M sodium carbonate (17.5 mL) there was added tetrakis(triphenylphosphine)palladium(0) (839 mg, 726 μmol) under a nitrogen atmosphere, and the mixture was stirred for 18 hours at 70°C. The reaction mixture was returned to room temperature, and extraction was performed with ethyl acetate. The organic layer was washed with brine, dried over anhydrous magnesium sulfate and filtered, and the filtrate was concentrated under reduced pressure. The residue was purified by silica gel column chromatography (n-heptane:ethyl acetate = 1:1 to 3:7) to obtain the title compound (4.78 g).

ESI-MS (m/z): 725.38 $[M+H]^+$.

[Production Example 104-5]

*tert*-Butyl (1'-(4-(1-cyclopropoxy-1-phenyl-2-((tetrahydro-2H-pyran-2-yl)oxyethyl)-6-(6-methyl-7-oxy-1-tosyl-6,7-dihydro-1H-pyrrolo[2,3-c]pyridin-4-yl)quinazolin-2-yl)-4-methyl-[1,4'-bipiperidin]-4-yl)carbamate

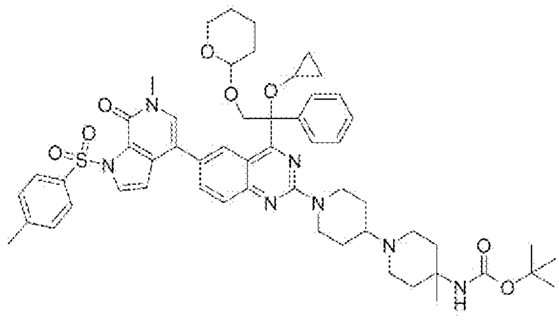

To a solution of the 4-(2-chloro-4-(1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 104-4 (300 mg, 414 μmol) in DMF (2 mL) there were added the *tert*-butyl (4-methyl-[1,4'-bipiperidin]-4-yl)carbamate of Production Example 104-2 (148 mg, 496 μmol) and N,N-diisopropylethylamine (144 μL, 827 μmol) at room temperature, and the mixture was stirred for 3 hours and 15 minutes at 80°C. The reaction mixture was returned to room temperature, and then water was added and extraction was performed with ethyl acetate. The organic layer was washed with water and brine, dried over anhydrous magnesium sulfate and filtered, and the filtrate was concentrated under reduced pressure. The residue was purified by NH silica gel column chromatography (n-heptane:ethyl acetate = 7:3 to ethyl acetate) to obtain the title compound (388 mg).

ESI-MS (m/z): 986.86 $[M+H]^+$.

[Production Example 104-6]

*tert-Butyl* (1'-(4-(1-cyclopropoxy-1-phenyl-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)-4-methyl-[1,4'-bipiperidine]4-yl)carbamate

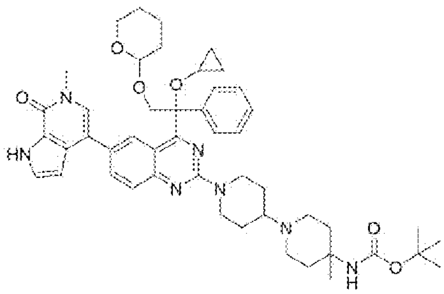

To a solution of the *tert-butyl* (1'-(4-(1-cyclopropoxy-1-phenyl-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)-4-methyl-[1,4'-bipiperidin]-4-yl)carbamate of Production Example 104-5 (149 mg, 151 μmol) in THF (4 mL) and methanol (2 mL) there was added a 1 M sodium hydroxide aqueous solution (1.51 mL, 1.51 mmol) at room temperature, and the mixture was stirred for 1 hour at 80°C. After returning the reaction mixture to room temperature, brine was added and extraction was performed with ethyl acetate. The organic layer was dried over anhydrous magnesium sulfate and filtered, and the filtrate was concentrated under reduced pressure. The residue was purified by NH silica gel column chromatography (n-heptane:ethyl acetate = 1:1 to ethyl acetate to ethyl acetate:methanol = 8:2) to obtain the title compound (107 mg).
ESI-MS (m/z): 832.46 $[M+H]^+$.

[Production Example 104-7]

3-(2,4-Dioxotetrahydropyrimidine-1(2H)-yl)-4-methoxybenzoic acid

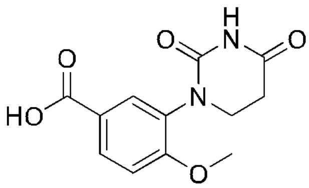

A solution of 3-amino-4-methoxybenzoic acid (2.5 g, 15 mmol) in acrylic acid (4 mL) was stirred for 3 hours at 100°C. The reaction mixture was cooled to room temperature, and then acetic acid (16 mL) was added and the mixture was stirred for 20 minutes at 100°C. Urea (5.5 g, 92 mmol) was then added and the mixture was stirred overnight at 110°C. The reaction mixture was cooled on ice, concentrated hydrochloric acid was added, and the mixture was stirred for 20 minutes while cooling on ice. The reaction mixture was allowed to stand for 2 hours at 4°C, and the obtained solid was filtered and washed with water, and then dried. After adding an aqueous 1 M hydrochloric acid solution to the obtained solid and crushing and filtering the solid, it was washed with TBME and dried to obtain the title compound (2.36 g).
ESI-MS (m/z): 265.12 $[M+H]^+$.

[Example 105]

N-(1'-(4-(1-Cyclopropoxy-1-(4-fluorophenyl)-2-hydroxyethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)-3-(2,4-dioxotetrahydropyrimidine-1(2H)-yl)-4-methyl benzamide

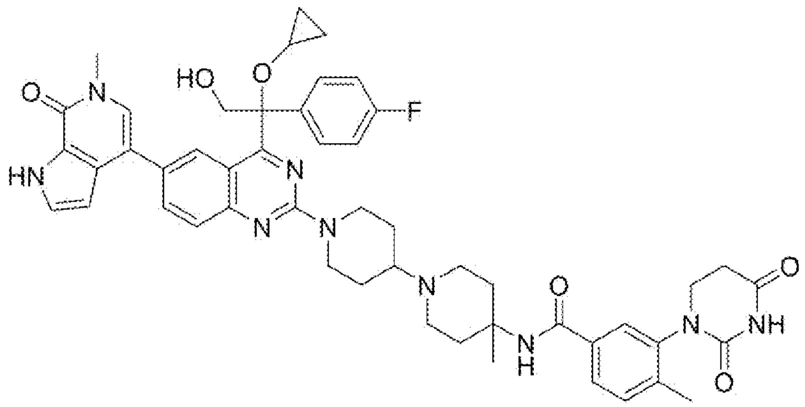

To a solution of the *tert*-butyl (1'-(4-(1-cyclopropoxy-1-(4-fluorophenyl)-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)carbamate of Production Example 105-5 (25 mg, 29 μmol) in dichloromethane (750 μL) there was added TFA (250 μL), and the mixture was stirred for 1 hour at room temperature. The reaction mixture was concentrated under reduced pressure to obtain a crude product. To a solution of the crude product and the 3-(2,4-dioxotetrahydropyrimidine-1(2H)-yl)-4-methylbenzoic acid of Production Example 105-6 (11.0 mg, 64 μmol) in DMF (500 μL) there were added N,N-diisopropylethylamine (51.4 μL, 294 μmol) and HATU (16.8 mg, 44 μmol), and the mixture was stirred for 1 hour at room temperature. The reaction mixture was purified by ODS silica gel chromatography (0.1% formic acid aqueous solution:0.1% acetonitrile formate solution = 9:1 to 55:45), to obtain a crude product. The obtained crude product was purified by NH silica gel thin-layer chromatography (ethyl acetate:methanol = 85:15) to obtain the title compound (11 mg). ESI-MS (m/z): 896.56 $[M+H]^+$.

[Production Example 105-1]

Methyl 2-cyclopropoxy-2-(4-fluorophenyl)acetate

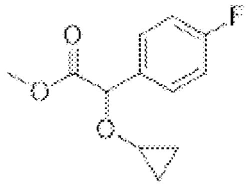

The title compound (364 mg) was obtained from methyl 2-diazo-2-(4-fluorophenyl)acetate (436 mg, 2.25 mmol) by the same method as Production Example 101-1.
$^1$H-NMR Spectrum (400 MHz, $CDCl_3$) δ(ppm): 0.42-0.58 (2H, m), 0.62-0.80 (2H, m), 3.23-3.47 (1H, m), 3.72 (3H, s), 4.85-5.04 (1H, m), 6.98-7.11 (2H, m), 7.35-7.45 (2H, m).

[Production Example 105-2]

2-Chloro-4-(1-cyclopropoxy-1-(4-fluorophenyl)-2-((tetrahydro-2H-pyran-2-yl)oxy)ethyl)6-iodoquinazoline

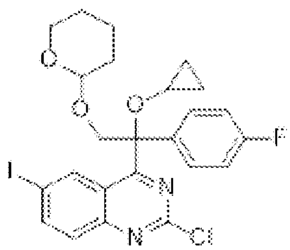

The title compound (400 mg) was obtained from the methyl 2-cyclopropoxy-2-(4-fluorophenyl)acetate of Production Example 105-1 (360 mg, 1.61 mmol) by the same method as Production Example 101-2 and Production Example 104-3.

ESI-MS (m/z): 569.19 [M+H]$^+$.

[Production Example 105-3]

*tert*-Butyl (1'-(4-(1-cyclopropoxy-1-(4-fluorophenyl)-2-((tetrahydro-2H-pyran-2-yl)oxy)ethyl)-6-iodoquinazolin-2-yl)-4-methyl-[1,4'-bipiperidin]-4-yl)carbamate

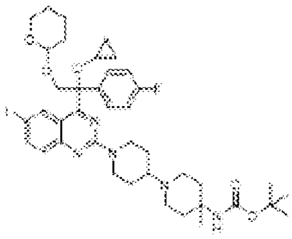

The title compound (88 mg) was obtained from the 2-chloro-4-(1-cyclopropoxy-1-(4-fluorophenyl)-2-((tetrahydro-2H-pyran-2-yl)oxy)ethyl)-6-iodoquinazoline of Production Example 105-2 (60 mg, 105 μmol) by the same method as Production Example 104-5. ESI-MS (m/z): 830.95 [M+H]$^+$.

[Production Example 105-4]

*tert*-Butyl (1'-(4-(1-cyclopropoxy-1-(4-fluorophenyl)-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)-4-methyl-[1,4'-bipiperidin]-4-yl)carbamate

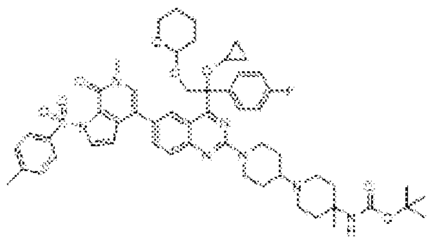

The title compound (45 mg) was obtained from the *tert*-butyl (1'-(4-(1-cyclopropoxy-1-(4-fluorophenyl)-2-((tetrahydro-2H-pyran-2-yl)oxy)ethyl)-6-iodoquinazolin-2-yl)-4-methyl-[1,4'-bipiperidin]-4-yl)carbamate of Production Example 105-3 (44 mg, 53 μmol) by the same method as Production Example 104-4.

ESI-MS (m/z): 1004.71 [M+H]$^+$.

[Production Example 105-5]

*tert*-Butyl (1'-(4-(1-cyclopropoxy-1-(4-fluorophenyl)-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)carbamate

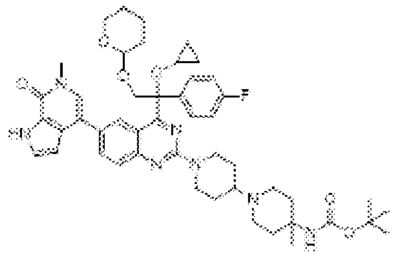

The title compound (38 mg) was obtained from the *tert*-butyl (1'-(4-(1-cyclopropoxy-1-(4-fluorophenyl)-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)-4-methyl-[1,4'-bipiperidin]-4-yl)carbamate of Production Example 105-4 (45 mg, 45 μmol) by the same method as Production Example 104-6.
ESI-MS (m/z): 850.62 $[M+H]^+$.

[Production Example 105-6]

3-(2,4-Dioxotetrahydropyrimidine-1(2H)-yl)-4-methylbenzoic acid

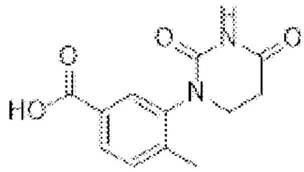

A solution of 3-amino-4-methylbenzoic acid (4.0 g, 26 mmol) in acrylic acid (7 mL) and acetic acid (30 mL) was stirred for 3 hours at 100°C. The reaction mixture was cooled to room temperature, and then urea (9.53 g, 159 mmol) was added and the mixture was stirred overnight at 110°C. After adding the reaction mixture to crushed ice, concentrated hydrochloric acid (12 mL) was added and the mixture was stirred for 4 hours. The precipitated solid was filtered and washed with water (100 mL × 3) and acetone (50 mL) and then dried, to obtain the title compound (5.04 g).
ESI-MS (m/z): 249.06 $[M+H]^+$.

[Example 106]

N-(1'-(4-(1-Cyclopropoxy-1-(3,5-difluorophenyl)-2-hydroxyethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)-3-(2,4-dioxotetrahydropyrimidine-1(2H)-yl)-4-methyl benzamide

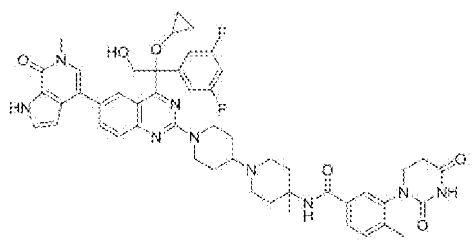

To a solution of the *tert*-butyl (1'-(4-(1-cyclopropoxy-1-(3,5-difluorophenyl)-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)carbamate of Production Example 106-6 (37 mg, 43 μmol) in dichloromethane (1 mL) there was added TFA (1 mL) at room temperature, and the mixture was stirred for 30 minutes at room temperature. The reaction mixture was concentrated under reduced pressure to obtain a crude product. To a solution of the 3-(2,4-dioxotetrahydropyrimidine-1(2H)-yl)-4-methylbenzoic acid of Production Example 105-6 (15.9 mg, 64 μmol) in DMF (2 mL) there were added N,N-diisopro-

pylethylamine (149 μL, 852 μmol) and HATU (24 mg, 64 μmol) at room temperature, and the mixture was stirred for 15 minutes at room temperature. This reaction mixture was added to the crude product at room temperature, and the mixture was stirred for 14 hours and 15 minutes. Water was added to the reaction mixture, and extraction was performed with ethyl acetate. The organic layer was washed with water and brine, dried over anhydrous magnesium sulfate and filtered, and the filtrate was concentrated under reduced pressure. The residue was purified by NH silica gel thin-layer chromatography (ethyl acetate:methanol = 10:1) to obtain the title compound (17 mg).

ESI-MS (m/z): 914.57 $[M+H]^+$.

[Production Example 106-1]

Methyl 2-diazo-2-(3,5-difluorophenyl)acetate

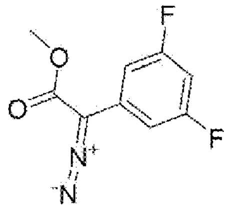

To a solution of methyl 2-(3,5-difluorophenyl)acetate (5.12 g, 27.5 mmol) and 4-acetamidobenzenesulfonyl azide (7.59 g, 31.6 mmol) in acetonitrile (150 mL) there was added DBU (4.76 mL, 31.6 mmol) while cooling on ice, and the mixture was stirred for 15 hours at room temperature. Diethyl ether and water were added to the reaction mixture for oil-water distribution. The organic layer was dried over anhydrous magnesium sulfate and filtered, and the filtrate was concentrated under reduced pressure. The residue was purified by silica gel column chromatography (heptane:ethyl acetate, gradient) to obtain the title compound (5.12 g).

$^1$H-NMR Spectrum (400 MHz, $CDCl_3$) δ(ppm): 3.88 (3H, s), 6.60 (1H, d, J=1.2 Hz), 7.06 (2H, dt, J=7.9 Hz, 1.0 Hz).

[Production Example 106-2]

Methyl 2-cyclopropoxy-2-(3,5-difluorophenyl)acetate

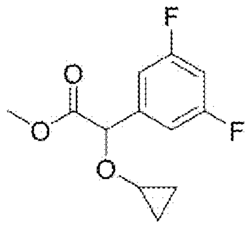

The title compound (1507 mg) was obtained from the methyl 2-diazo-2-(3,5-difluorophenyl)acetate of Production Example 106-1 (1888 mg, 8.90 mmol) by the same method as Production Example 101-1.

$^1$H-NMR Spectrum (500 MHz, $CDCl_3$) δ(ppm): 0.45-0.58 (2H, m), 0.62-0.81 (2H, m), 3.44 (1H, tt, J=6.1, 2.9 Hz), 3.74 (3H, s), 4.93 (1H, s), 6.76 (1H, tt, J=8.8, 2.5 Hz), 6.97-7.00 (2H, m).

[Production Example 106-3]

Methyl 2-(2-chloro-6-iodoquinazolin-4-yl)-2-cyclopropoxy-2-(3,5-difluorophenyl)acetate

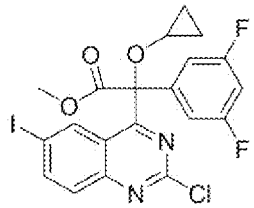

The title compound (2944 mg) was obtained from the methyl 2-cyclopropoxy-2-(3,5-difluorophenyl)acetate of Production Example 106-2 (1507 mg, 6.22 mmol) by the same method as Production Example 101-2.
ESI-MS (m/z): 531.08 [M+H]+.

[Production Example 106-4]

*tert*-Butyl (1'-(4-(1-cyclopropoxy-1-(3,5-difluorophenyl)-2-((tetrahydro-2H-pyran-2-yl)oxy)ethyl-6-iodoquinazolin-2-yl)-4-methyl[1,4'-bipiperidin]-4-yl)carbamate

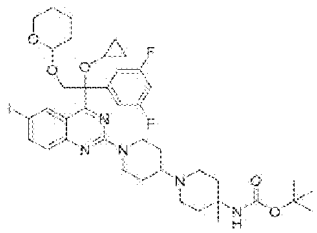

To a solution of the methyl 2-(2-chloro-6-iodoquinazolin-4-yl)-2-cyclopropoxy-2-(3,5-difluorophenyl)acetate of Production Example 106-3 (2865 mg, 5.40 mmol) in toluene (40 mL) there was added DIBAL-H (1 M toluene solution, 16.2 mL, 16.2 mmol) at -78°C under a nitrogen atmosphere, and the mixture was stirred for 10 minutes at -78°C. The reaction mixture was increased in temperature to 0°C and stirred for 1 hour. A saturated Rochelle salt solution was added to the reaction mixture at 0°C, ethyl acetate was added, and the mixture was stirred for 1 hour at 0°C. The reaction mixture was extracted with ethyl acetate and the organic layer was washed with brine. The organic layer was dried over anhydrous magnesium sulfate and filtered, and the filtrate was concentrated under reduced pressure.
To a solution of the residue in dichloromethane (30 mL) there were added DHP (2.47 mL, 27.0 mmol) and CSA (1254 mg, 5.40 mmol) at 0°C, and the mixture was stirred for 1 hour at room temperature. A saturated aqueous sodium hydrogencarbonate solution was added to the reaction mixture, and extraction was performed with dichloromethane. The organic layer was washed with brine, dried over anhydrous magnesium sulfate and filtered, and the filtrate was concentrated under reduced pressure. The residue was purified by silica gel column chromatography (n-heptane:ethyl acetate = 95:5 to 7:3) to obtain a mixture of 2-chloro-4-(1-cyclopropoxy-1-(3,5-difluorophenyl)-2-((tetrahydro-2H-pyran-2-yl)oxy)ethyl)-6-iodoquinazoline and DHP (3995 mg). A solution of the obtained mixture (200 mg), the *tert-butyl* (4-methyl-[1,4'-bipiperidin]-4-yl)carbamate of Production Example 104-2 (152 mg, 511 μmol) and N,N-diisopropylethylamine (0.119 mL, 682 μmol) in DMF (2.0 mL) was stirred for 2 hours at 80°C. The reaction mixture was returned to room temperature, and then water was added and extraction was performed with ethyl acetate. The organic layer was washed with water and brine, dried over anhydrous magnesium sulfate and filtered, and the filtrate was concentrated under reduced pressure. The residue was purified by NH silica gel column chromatography (n-heptane:ethyl acetate = 1:1 to ethyl acetate to ethyl acetate:methanol = 9:1) to obtain the title compound (126 mg).
ESI-MS (m/z): 848.42 [M+H]+.

[Production Example 106-5]

*tert*-Butyl (1'-(4-(1-cyclopropoxy-1-(3,5-difluorophenyl)-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)-4-methyl-[1,4'-bipiperidin]-4-yl)carbamate

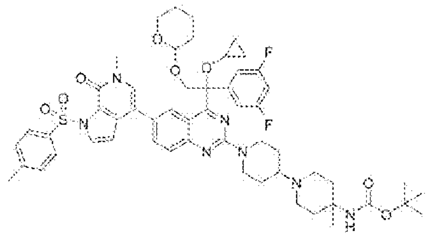

A solution of the *tert-butyl* (1'-(4-(1-cyclopropoxy-1-(3,5-difluorophenyl)-2-((tetrahydro-2H-pyran-2-yl)oxy) ethyl)-6-iodoquinazolin-2-yl)-4-methyl-[1,4'-bipiperidin]-4-yl)carbamate of Production Example 106-4 (63 mg, 74 μmol), 6-methyl-4-(4,4,5,5-tetramethyl-1,3,2-dioxaborolan-2-yl)-tosyl-1,6-dihydro-7H-pyrrolo[2,3-c]pyridin-7-one (38 mg, 89 μmol), cesium carbonate (36.3 mg, 111 μmol) and tetrakis(triphenylphosphine)palladium(0) (8.59 mg, 7.43 μmol) in 1,4-dioxane (4 mL) and water (2 mL) was stirred for 1 hour and 30 minutes at 80°C under a nitrogen atmosphere. The reaction mixture was returned to room temperature, and then water was added and extraction was performed with ethyl acetate. The organic layer was washed with brine, dried over anhydrous magnesium sulfate and filtered, and the filtrate was concentrated under reduced pressure. The residue was purified by NH silica gel column chromatography (n-heptane:ethyl acetate = 1:1 to ethyl acetate) to obtain the title compound (69 mg).
ESI-MS (m/z): 1022.68 $[M+H]^+$.

[Production Example 106-6]

*tert*-Butyl (1'-(4-(1-cyclopropoxy-1-(3,5-difluorophenyl)-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)carbamate

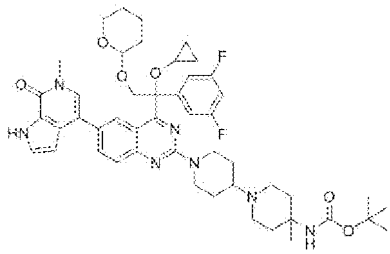

The title compound (46 mg) was obtained from the *tert-butyl* (1'-(4-(1-cyclopropoxy-1-(3,5-difluorophenyl)-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)-4-methyl-[1,4'-bipiperidin]-4-yl)carbamate of Production Example 106-5 (69 mg, 67 μmol), by the same method as Production Example 104-6.
ESI-MS (m/z): 868.59 $[M+H]^+$.

[Example 107]

N-(1'-(4-(1-Cyclopropoxy-1-(3,5-difluorophenyl)-2-hydroxyethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)-3-(2,4-dioxotetrahydropyrimidine-1(2H)-yl)-4-methoxybenzamide

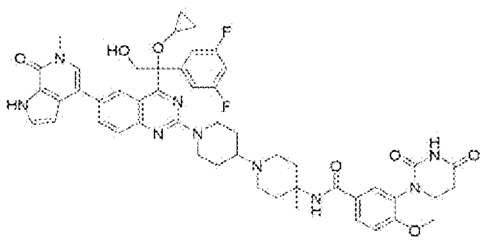

The title compound (11 mg) was obtained from the *tert-butyl* (1'-(4-(1-cyclopropoxy-1-(3,5-difluorophenyl)-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)carbamate of Production Example 106-6 (23 mg, 26 μmol) and the 3-(2,4-dioxotetrahydropyrimidine-1(2H)-yl-4-methoxybenzoic acid of Production Example 104-7 (8.4 mg, 32 μmol), by the same method as Example 106.
ESI-MS (m/z): 930.53 $[M+H]^+$.

[Example 108]

N-(1-((1-(4-(1-Cyclopropoxy-2-hydroxy-1-phenylethyl)-6-(6-methyl-7-oxo-6,7-dihydrofuro[2,3-c]pyridin-4-yl)quinazolin-2-yl)piperidin-4-yl)methyl)-4-methylpiperidin-4-yl)-3-(2,4-dioxotetrahydropyrimidine-1(2H)-yl)-4-methoxybenzamide

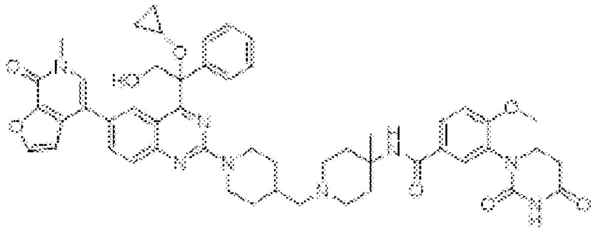

The title compound (1.68 mg) was obtained from the *tert-butyl* (1-((1-(4-(1-cyclopropoxy-1-phenyl-2-((tetrahydro-2H-pyran-2-yl)oxy)ethyl)-6-(6-methyl-7-oxo-6,7-dihydrofuro[2,3-c]pyridin-4-yl)quinazolin-2-yl)piperidin-4-yl)methyl)-4-methylpiperidin-4-yl)carbamate of Production Example 108-2 (13.0 mg, 15.0 μmol) and the 3-(2,4-dioxotetrahydropyrimidine-1(2H)-yl-4-methoxybenzoic acid of Production Example 104-7 (5.27 mg, 20 μmol), by the same method as Example 104.
ESI-MS (m/z): 909.68 $[M+H]^+$.

[Production Example 108-1]

*tert-Butyl* (1-((1-(4-(1-cyclopropoxy-1-phenyl-2-((tetrahydro-2H-pyran-2-yl)oxy)ethyl)-6-iodoquinazolin-2-yl)piperidin-4-yl)methyl)-4-methylpiperidin-4-yl)carbamate

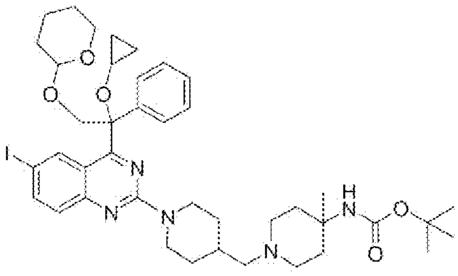

To a solution of the 2-chloro-4-(1-cyclopropoxy-1-phenyl-2-((tetrahydro-2H-pyran-2-yl)oxy)ethyl)6-iodoquinazoline of Production Example 104-3 (160 mg, 290 μmol) in DMF (2 mL) there were added the *tert-butyl* (4-methyl-1-(piperidin-4-ylmethyl)piperidin-4-yl)carbamate of Production Example 109-2 (109 mg, 349 μmol) and N,N-diisopropylethylamine (100 μL, 19.9 mmol) at room temperature, and the mixture was stirred for 3 hours at 80°C. Water was added to the reaction mixture and the precipitated solid was filtered to obtain the title compound (228 mg).
ESI-MS (m/z): 827.01 $[M+H]^+$.

[Production Example 108-2]

*tert-Butyl* (1-((1-(4-(1-cyclopropoxy-1-phenyl-2-((tetrahydro-2H-pyran-2-yl)oxy)ethyl)-6-(6-methyl-7-oxo-6,7-dihydrofuro[2,3-c]pyridin-4-yl)quinazolin-2-yl)piperidin-4-yl)methyl)-4-methylpiperidin-4-yl)carbamate

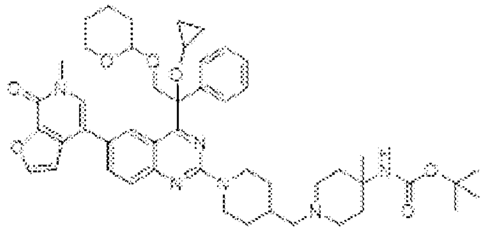

The title compound (13 mg) was obtained from the *tert-butyl* (1-((1-(4-(1-cyclopropoxy-1-phenyl-2-((tetrahydro-2H-pyran-2-yl)oxy)ethyl)-6-iodoquinazolin-2-yl)piperidin-4-yl)methyl)-4-methylpiperidin-4-yl)carbamate of Production Example 108-1 (30 mg, 36 μmol) and 6-methyl-4-(4,4,5,5-tetramethyl-1,3,2-dioxaborolan-2-yl)furo[2,3-c]pyridine-7(6H)-one known from published literature (14 mg, 51 μmol), by the same method as Production Example 104-4.
ESI-MS (m/z): 847.70 $[M+H]^+$.

[Example 109]

N-(1-((1-(4-(1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)piperidin-4-yl)methyl)-4-methylpiperidin-4-yl)-3-(2,4-dioxotetrahydropyrimidine-1(2H)-yl)-4-methoxybenzamide

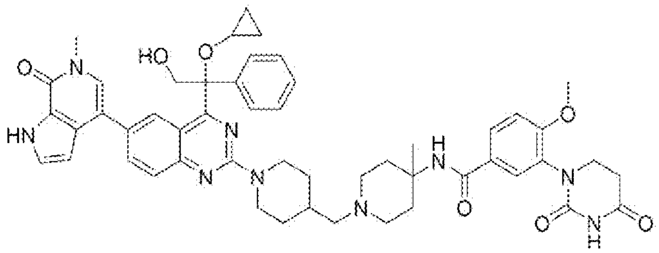

To a solution of the *tert-butyl* (1-((1-(4-(1-cyclopropoxy-1-phenyl-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)piperidin-4-yl)methyl)-4-methylpiperidin-4-yl) carbamate of Production Example 109-4 (18.6 mg, 22.0 μmol) in dichloromethane (3 mL) there was added TFA (1 mL) at room temperature, and the mixture was stirred for 1 hour at room temperature. Toluene was added to the reaction mixture, which was then concentrated under reduced pressure. To a solution of the residue in DMF (3 mL) there were added N,N-diisopropylethylamine (115 μL, 660 μmol), the 3-(2,4-dioxotetrahydropyrimidine-1(2H)-yl)-4-methoxybenzoic acid of Production Example 104-7 (6.97 mg, 26.0 μmol) and HATU (12.5 mg, 32.9 μmol) at room temperature, and the mixture was stirred for 1 hour and 30 minutes at room temperature. Water was added to the reaction mixture to halt the reaction, and extraction was performed with ethyl acetate. The organic layer was dried over anhydrous sodium sulfate and filtered, and the filtrate was concentrated under reduced pressure. The residue was purified by NH silica gel thin-layer chromatography (dichloromethane:methanol = 24:1) to obtain the title compound (9.16 mg).
ESI-MS (m/z): 908.58 $[M+H]^+$.

[Production Example 109-1]

Benzyl 4-((4-((*tert*-butoxycarbonyl)amino)-4-methylpiperidin-1-yl)methyl)piperidine-1-carboxylate

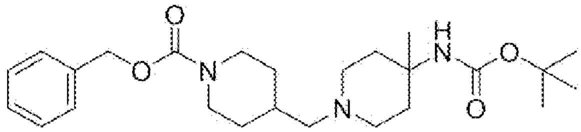

To a solution of 4-formyl-N-cbz-piperidine (1.00 g, 4.04 mmol) in dichloromethane (10 mL) there were added *tert-butyl* (4-methylpiperidin-4-yl)carbamate (953 mg, 4.45 mmol), acetic acid (100 μL, 1.75 mmol) and sodium triacetoxyborohydride (>80 wt%, 1.11 g) at room temperature, and the mixture was stirred for 2 hours at room temperature. A saturated aqueous sodium hydrogencarbonate solution was added to the reaction mixture at room temperature, and extraction was performed with dichloromethane. The organic layer was dried over anhydrous sodium sulfate and filtered, and the filtrate was concentrated under reduced pressure. The residue was purified by NH silica gel column chromatography (n-heptane to n-heptane:ethyl acetate = 7:3) to obtain the title compound (1.21 g).
ESI-MS (m/z): 446.77 $[M+H]^+$.

[Production Example 109-2]

*tert*-Butyl (4-methyl-1-(piperidin-4-ylmethyl)piperidin-4-yl)carbamate

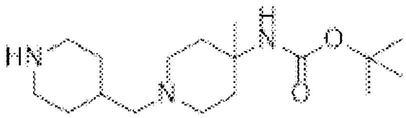

To a solution of the benzyl 4-((4-((tert-butoxycarbonyl)amino)-4-methylpiperidin-1-yl)methyl)piperidine-1-carboxylate of Production Example 109-1 (1.20 g, 2.69 mmol) in methanol (10 mL) there was added 10% palladium-carbon (50% water, 143 mg) at room temperature, and the mixture was stirred for 1 hour and 30 minutes at room temperature, ordinary pressure under a hydrogen atmosphere. The reaction mixture was placed under a nitrogen atmosphere and filtered with Celite, and the filtrate was concentrated under reduced pressure to obtain the title compound (710 mg).
ESI-MS (m/z): 312.33 $[M+H]^+$.

[Production Example 109-3]

*tert*-Butyl (1-((1-(4-(1-cyclopropoxy-1-phenyl-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)piperidin-4-yl)methyl)-4-methylpiperidin-4-yl)carbamate

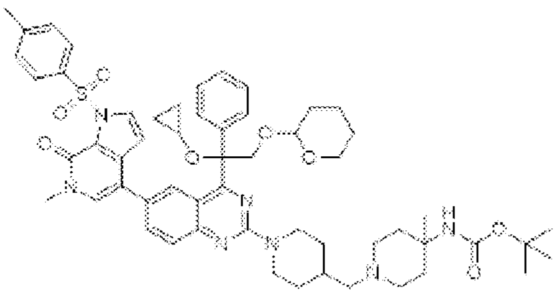

To a solution of the 4-(2-chloro-4-(1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 104-4 (200 mg, 276 μmol) in DMF (5 mL) there were added the *tert*-butyl (4-methyl-1-(piperidin-4-ylmethyl)piperidin-4-yl)carbamate of Production Example 9-2 (94 mg, 0.30 mmol) and N,N-diisopropylethylamine (96 μL, 0.55 mmol) at room temperature, and the mixture was stirred for 1 hour and 30 minutes at 75°C. The reaction mixture was returned to room temperature, and then water was added to halt the reaction and extraction was performed with ethyl acetate. The organic layer was dried over anhydrous sodium sulfate and filtered, and the filtrate was concentrated under reduced pressure. The residue was purified by NH silica gel column chromatography (*n*-heptane:ethyl acetate = 7:3 to ethyl acetate) to obtain the title compound (300 mg).

ESI-MS (m/z): 1000.81 [M+H]+.

[Production Example 109-4]

*tert-Butyl* (1-((1-(4-(1-cyclopropoxy-1-phenyl-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)piperidin-4-yl)methyl)-4-methylpiperidin-4-yl)carbamate

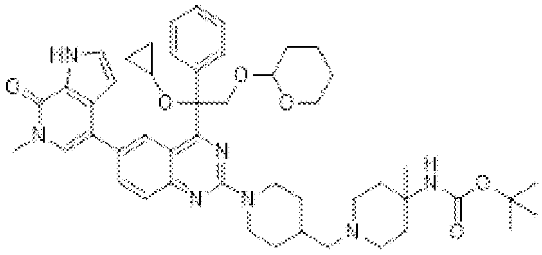

To a solution of the *tert-butyl* (1-((1-(4-(1-cyclopropoxy-1-phenyl-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)piperidin-4-yl)methyl)-4-methylpiperidin-4-yl)carbamate of Production Example 109-3 (300 mg, 300 μmol) in methanol (2.7 mL) and THF (2.7 mL) there was added a 1 M sodium hydroxide aqueous solution (3.0 mL), and the mixture was stirred for 1 hour at 75°C. The reaction mixture was returned to room temperature, water was added, and extraction was performed with ethyl acetate. The organic layer was dried over anhydrous sodium sulfate and filtered, and then the filtrate was concentrated under reduced pressure to obtain the title compound (226 mg).
ESI-MS (m/z): 846.68 [M+H]+

[Example 110]

1-(5-((1-(4-(1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl-hydroxypiperidin-4-yl)ethynyl)-2-methoxyphenyl)dihydropyrimidine-2,4(1H,3H)-dione

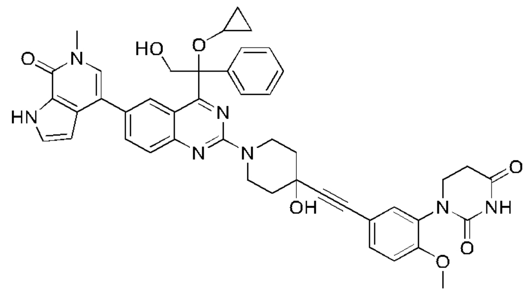

A mixture of the 4-(4-(1-cyclopropoxy-1-phenyl-2-((tetrahydro-2H-pyran-2-yl)oxy)ethyl)-2-(4-ethynyl-4-hydroxypiperidin-1-yl)quinazolin-6-yl)-6-methyl-1,6-dihydro-7H-pyrrolo[2,3-c]pyridin-7-one of Production Example 110-2 (27 mg, 41 μmol), the 1-(5-iodo-2-methoxyphenyl)dihydropyrimidine-2,4(1H,3H)-dione of Production Example 101-10 (18 mg, 53 μmol), tetrakis(triphenylphosphine)palladium(0) (14 mg, 12 μmol), CuI (2.3 mg, 12 μmol), N,N-diisopropylethylamine (71.5 μL, 409 μmol) and DMF (1000 μL) was stirred for 3 hours and 30 minutes at 50°C. The reaction mixture was returned to room temperature and concentrated under reduced pressure. DCM (1 mL) and TFA (1 mL) were added to the residue, and the mixture was stirred for 1 hour. The reaction mixture was concentrated under reduced pressure and purified by ODS silica gel column chromatography (0.1% formic acid aqueous solution:0.1% acetonitrile formate solution = 99:1 to 4:6), to obtain a crude product. The obtained crude product was purified by NH silica gel thin-layer chromatography (ethyl acetate:methanol = 10:1) to obtain a crude product. The obtained crude product was purified by silica gel thin-layer chromatography (ethyl acetate:methanol = 10:1) to obtain the title compound (3.2 mg).
ESI-MS (m/z): 794.28 [M+H]+.

[Production Example 110-1]

4-(4-(1-Cyclopropoxy-1-phenyl-2-((tetrahydro-2H-pyran-2-yl)oxy)ethyl)-2-(4-ethynyl-4-hydroxypiperidin-1-yl)quinazolin-6-yl)-6-methyl-1-tosyl-1,6-dihydro-7H-pyrrolo[2,3-c]pyridin-7-one

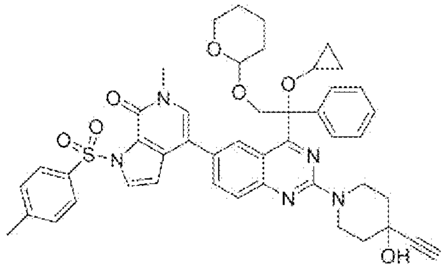

To a solution of the *tert*-butyl 4-ethynyl-4-hydroxypiperidine-1-carboxylate of Production Example 102-4 (186 mg, 827 μmol) in DCM (2000 μL) there was added TFA (1593 μL), and the mixture was stirred for 1 hour. The reaction mixture was concentrated under reduced pressure and processed twice by azeotropic distillation with toluene. To a solution of the residue in DMF (1000 μL) there was added N,N-diisopropylethylamine (361 μL, 2.07 mmol) and the 4-(2-chloro-4-(1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 104-4 (300 mg, 414 μmol), and the mixture was stirred for 4 hours at 80°C. The reaction mixture was returned to room temperature, water was added, and extraction was performed with ethyl acetate. The organic layer was washed with water and brine, dried over anhydrous sodium sulfate and filtered, and the filtrate was concentrated under reduced pressure. The residue was purified by silica gel column chromatography (n-heptane:ethyl acetate = 1: 1 to 1:4) to obtain the title compound (250 mg).
ESI-MS (m/z): 814.29 $[M+H]^+$

[Production Example 110-2]

4-(4-(1-Cyclopropoxy-1-phenyl-2-((tetrahydro-2H-pyran-2-yl)oxy)ethyl)-2-(4-ethynyl-4-hydroxypiperidin-1-yl)quinazolin-6-yl)-6-methyl-1,6-dihydro-7H-pyrrolo[2,3-c]pyridin-7-one

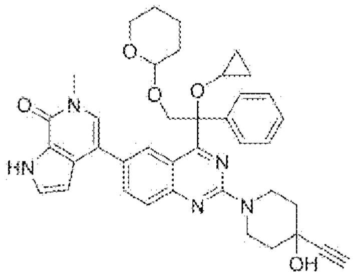

To a solution of the 4-(4-(1-cyclopropoxy-1-phenyl-2-((tetrahydro-2H-pyran-2-yl)oxy)ethyl)-2-(4-ethynyl-4-hydroxypiperidin-1-yl)quinazolin-6-yl)-6-methyl-1-tosyl-1,6-dihydro-7H-pyrrolo[2,3-c]pyridin-7-one of Production Example 110-1 (100 mg, 123 μmol) in ethanol (5 mL) there was added a 1 N sodium hydroxide aqueous solution (1.229 mL), and the mixture was stirred for 1 hour at 60°C. The reaction mixture was returned to room temperature and concentrated under reduced pressure. Water was added to the residue and extraction was performed twice with ethyl acetate. The organic layer was washed with brine, dried over anhydrous sodium sulfate and filtered, and then the filtrate was concentrated under reduced pressure to obtain the title compound (86.7 mg) in crude form.
ESI-MS (m/z): 660.94 $[M+H]^+$

[Example 111]

1-(5-((2R,5S)-4-((1-(4-(1-Cyclopropoxy-2-hydroxy-1-(pyridin-3-yl)ethyl)-6-(6-methyl-7-oxo-6,7-dihydro-1H-pyrrolo[2,3-c]pyridin-4-yl)quinazolin-2-yl)piperidin-4-yl)methyl)-2,5-dimethylpiperazine-1-carbonyl)-2-methylphenyl)dihydropyrimidine-2,4(1H,3H)-dione

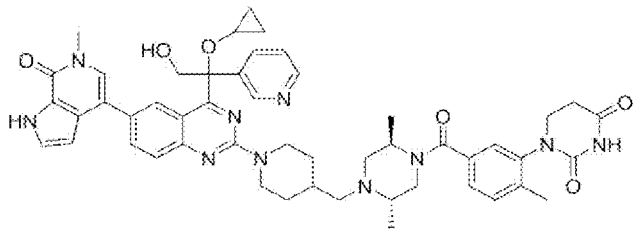

To a solution of the *tert*-butyl (2R,5S)-4-((1-(4-(1-cyclopropoxy-1-(pyridin-3-yl)-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)piperidin-4-yl)methyl)-2,5dimethylpiperazine-1-carboxylate of Production Example 111-7 (25 mg, 30 μmol) in dichloromethane (750 μL) there was added TFA (250 μL), and the mixture was stirred for 1 hour at room temperature. The reaction mixture was concentrated under reduced pressure to obtain a crude product. To a solution of the obtained crude product and the 3-(2,4-dioxotetrahydropyrimidine-1(2H)-yl)-4-methylbenzoic acid of Production Example 105-6 (11 mg, 44 μmol) in DMF (500 μL) there was added N,N-diisopropylethylamine (51.5 μL, 295 μmol), and after stirring for 5 minutes at room temperature, HATU (16.8 mg, 44 μmol) was further added and the mixture was stirred for 30 minutes at room temperature. The reaction mixture was purified by ODS silica gel column chromatography (0.1% formic acid aqueous solution:0.1% acetonitrile formate solution = 9:1 to 6:4), to obtain a crude product. The obtained crude product was purified by NH silica gel thin-layer chromatography (ethyl acetate:methanol = 85:15) to obtain the title compound (11 mg).

ESI-MS (m/z): 893.53 $[M+H]^+$.

[Production Example 111-1]

Ethyl 2-cyclopropoxy-2-(pyridin-3-yl)acetate

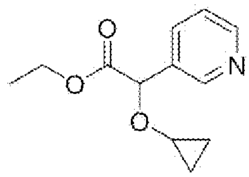

To a solution of ethyl 2-diazo-2-(pyridin-3-yl)acetate (800 mg, 4.18 mmol) in toluene (16 mL) there were added dichloropropanol (2.43 g, 41.8 mmol) and rhodium(II) acetate dimer (185 mg, 0.418 mmol), and the mixture was stirred for 45 minutes at 90°C. After cooling to room temperature, the mixture was purified by silica gel column chromatography (n-heptane:ethyl acetate = 3:1 to 1:3) to obtain the title compound (360 mg).

$^1$H-NMR Spectrum (500 MHz, $CDCl_3$) δ(ppm): 0.45-0.80 (4H, m), 1.23 (3H, m), 3.42-3.49 (1H, m), 4.13-4.27 (2H, m), 4.97 (1H, s), 7.26-7.32 (1H, m), 7.76-7.82 (1H, m), 8.55-8.60 (1H, m), 8.65-8.68 (1H, m).

[Production Example 111-2]

Ethyl 2-(2-chloro-6-iodoquinazolin-4-yl)-2-cyclopropoxy-2-(pyridin-3-yl)acetate

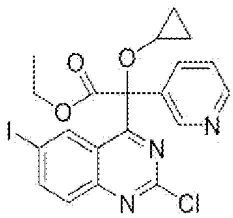

The title compound (756 mg) was obtained from the ethyl 2-cyclopropoxy-2-(pyridin-3-yl)acetate of Production Example 111-1 (360 mg, 1.63 mmol), by the same method as Production Example 101-2.

ESI-MS (m/z): 510.57 $[M+H]^+$.

[Production Example 111-3]

2-Chloro-4-(1-cyclopropoxy-1-(pyridin-3-yl)-2-((tetrahydro-2H-pyran-2-yl)oxy)ethyl)6-iodoquinazoline

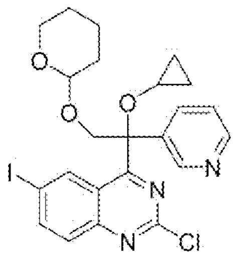

The title compound (86 mg) was obtained from the ethyl 2-(2-chloro-6-iodoquinazolin-4-yl)-2-cyclopropoxy-2-(pyridin-3-yl)acetate of Production Example 111-2 (300 mg, 589 μmol), by the same method as Production Example 104-3.

ESI-MS (m/z): 552.61 $[M+H]^+$.

[Production Example 111-4]

4-(2-Chloro-4-(1-cyclopropoxy-1-(pyridin-3-yl)-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one

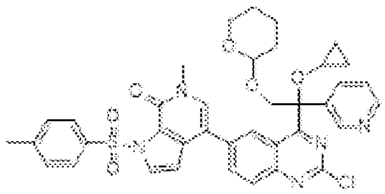

The title compound (246 mg) was obtained from the 2-chloro-4-(1-cyclopropoxy-1-(pyridin-3-yl)-2-((tetrahydro-2H-pyran-2-yl)oxy)ethyl)6-iodoquinazoline of Production Example 111-3 (200 mg, 362 μmol), by the same method as Production Example 104-4.

ESI-MS (m/z): 726.37 $[M+H]^+$.

[Production Example 111-5]

*tert*-Butyl (2R,5 S)-4-((1-((benzyloxy)carbonyl)piperidin-4-yl)methyl)-2,5-dimethylpiperazine-1-carboxylate

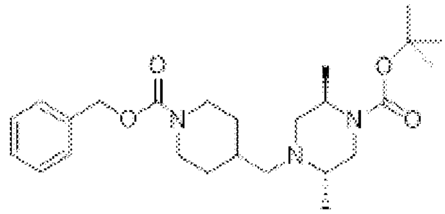

The title compound (83 mg) was obtained from *tert-butyl* (2R,5S)-2,5-dimethylpiperazine-1-carboxylate (182 mg, 849 μmol) and 4-formyl-N-Cbz-piperazine (200 mg, 809 μmol), by the same method as Production Example 104-1.
ESI-MS (m/z): 446.37 [M+H]+.

[Production Example 111-6]

*tert*-Butyl (2R,5S)-2,5-dimethyl-4-(piperidin-4-ylmethyl)piperazine-1-carboxylate

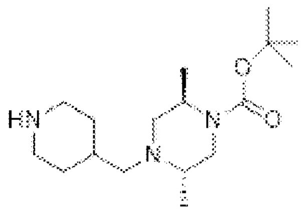

The title compound (62 mg) was obtained from the *tert-butyl* (2R,5S)-4-((1-((benzyloxy)carbonyl)piperidin-4-yl) methyl)-2,5-dimethylpiperazine-1-carboxylate of Production Example 111-5 (83 mg, 0.19 mmol), by the same method as Production Example 104-2.
ESI-MS (m/z): 312.37 [M+H]+.

[Production Example 111-7]

*tert*-Butyl (2R,5S)-4-((1-(4-(1-cyclopropoxy-1-(pyridin-3-yl)-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)piperidin-4-yl)methyl)-2,5dimethylpiperazine-1-carboxylate

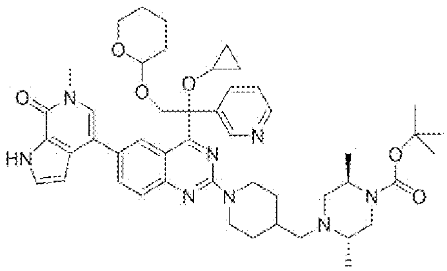

The title compound (25 mg) was obtained from the 4-(2-chloro-4-(1-cyclopropoxy-1-(pyridin-3-yl)-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 111-4 (25 mg, 34 μmol) and the *tert*-butyl (2R,5S)-2,5-dimethyl-4-(piperidin-4-ylmethyl)piperazine-1-carboxylate of Production Example 111-6 (19 mg, 62 μmol), by the same method as Production Example 104-5 and Production Example 104-6.
ESI-MS (m/z): 847.63 [M+H]+.

[Example 112]

1-(3-(4-((1-(4-(1-Cyclopropoxy-2-hydroxy-1-phenylethyl)-6-(6-methyl-7-oxy-6,7-dihydrofuro[2,3-c]pyridin-4-yl)quinazolin-2-yl)azetidin-3-yl)oxy)piperidine-1-carbonyl)phenyl)dihydropyrimidine-2,4(1H,3H)-dione

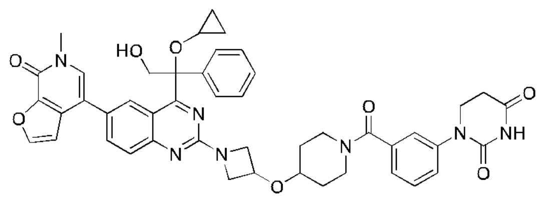

The compound *tert*-butyl 3-((1-(3-(2,4-dioxotetrahydropyrimidine-1(2H)-yl)benzoyl)piperidin-4-yl)oxy)azetidine-1-carboxylate (18.59 mg) was obtained from the *tert*-butyl 3-(piperidin-4-yloxy)azetidine-1-carboxylate of Production Example 103-2 (107 mg, 417 μmol) and the 3-(2,4-dioxotetrahydropyrimidine-1(2H)-yl)benzoic acid of Production Example 103-6 (127 mg, 543 μmol), by the same method as Example 103. To a solution of the obtained *tert*-butyl 3-((1-(3-(2,4-dioxotetrahydropyrimidine-1(2H)-yl)benzoyl)piperidin-4-yl)oxy)azetidine-1-carboxylate (18.59 mg) in DCM (1000 μL) there was added TFA (200 μL), and the mixture was stirred for 30 minutes. The reaction mixture was concentrated under reduced pressure and processed by azeotropic distillation with toluene. To a solution of the obtained residue in DMF (1000 μL) there was added the 4-(2-chloro-4-(1-cyclopropoxy-1-phenyl-2-((tetrahydro-2H-pyran-2-yl)oxy)ethyl)quinazolin-6-yl)-6-methylfuro[2,3-c]pyridine-7(6H)-one of Production Example 112-1 (15 mg, 26 μmol) and N,N-diisopropylethylamine (92 μL, 0.52 mmol), and the mixture was stirred for 1 hour and 50 minutes at 80°C. The reaction mixture was returned to room temperature, and then water was added and extraction was performed with ethyl acetate. The organic layer was washed with water and brine, dried over anhydrous sodium sulfate and filtered, and the filtrate was concentrated under reduced pressure. DCM (1 mL) and TFA (200 μL) were added to the residue, and the mixture was stirred for 2 hours and 10 minutes. A saturated aqueous sodium hydrogencarbonate solution was added to the reaction mixture, and extraction was performed with DCM. The organic layer was dried over anhydrous sodium sulfate and filtered, and the filtrate was concentrated under reduced pressure. The residue was purified by NH silica gel thin-layer chromatography (ethyl acetate:methanol = 10:1), and then purified by silica gel column chromatography (ethyl acetate:methanol = 10:1), to obtain the title compound (11 mg).

ESI-MS (m/z): 824.33 $[M+H]^+$.

[Production Example 112-1] 4-(2-Chloro-4-(1-cyclopropoxy-1-phenyl-2-((tetrahydro-2H-pyran-2-yl)oxy)ethyl)quinazolin-6-yl)-6-methylfuro[2,3-c]pyridine-7(6H)-one

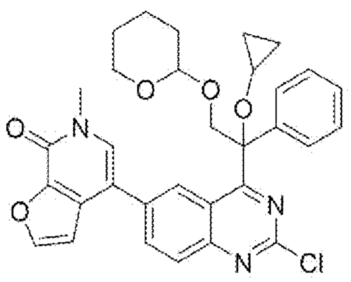

The title compound (119 mg) was obtained from the 2-chloro-4-(1-cyclopropoxy-1-phenyl-2-((tetrahydro-2H-pyran-2-yl)oxy)ethyl)-6-iodoquinazoline of Production Example 104-3 (220 mg, 399 μmol) and the known compound 6-methyl-4-(4,4,5,5-tetramethyl-1,3,2-dioxaborolan-2-yl)furo[2,3-c]pyridine-7(6H)-one (154 mg, 559 μmol), by the same method as Production Example 104-4.

ESI-MS (m/z): 572.32 $[M+H]^+$.

[Example 113]

1-(5-((2R,5 S)-4-(1-(4-(2-Hydroxy-1,1-diphenylethyl-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carbonyl)-2-methoxyphenyl)dihydropyrimidine-2,4(1H,3H)-dione

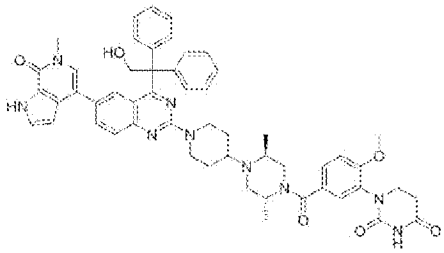

The title compound (23 mg) was obtained from the *tert*-butyl (2R,5S)-4-(1-(4-(1,1-diphenyl-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate of Production Example 113-5 (79 mg, 93 μmol) and a crude product of the 3-(2,4-dioxotetrahydropyrimidine-1 (2H)-yl)-4-methoxybenzoic acid of Production Example 104-7 (32 mg), by the same method as Example 104.

ESI-MS (m/z): 914.61 $[M+H]^+$.

[Production Example 113-1]

Methyl 2-(2-chloro-6-iodoquinazolin-4-yl)-2,2-diphenyl acetate

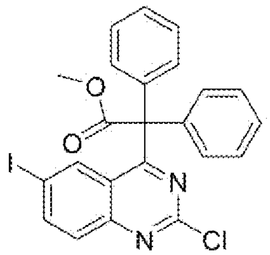

To a solution of 2,4-dichloro-6-iodoquinazoline (2.90 g, 8.93 mmol) and methyl diphenylacetate (2.12 g, 9.37 mmol) in THF (50 mL) there was added dropwise LHMDS (1.43 M THF solution, 8.11 mL, 11.6 mmol) at -78°C, and the mixture was stirred for 1.5 hours at 0°C. Saturated aqueous ammonium chloride was added to the reaction mixture, and extraction was performed with ethyl acetate. The organic layer was washed with brine, dried over magnesium sulfate, and filtered. The filtrate was concentrated under reduced pressure. The obtained solid was washed with TBME to obtain the title compound (3.68 g). ESI-MS (m/z): 515.04 $[M+H]^+$.

[Production Example 113-2]

2-Chloro-4-(1,1-diphenyl-2-((tetrahydro-2H-pyran-2-yl)oxy)ethyl)-6-iodoquinazoline

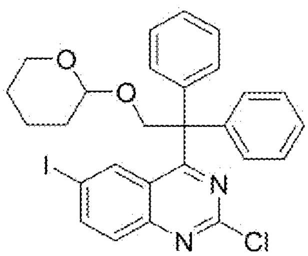

To a solution of the methyl 2-(2-chloro-6-iodoquinazolin-4-yl)-2,2-diphenyl acetate of Production Example 113-1 (15.0 g, 29.1 mmol) in toluene (300 mL) there was added dropwise DIBAL-H (1 M toluene solution, 72.9 mL, 72.9 mmol) at -78°C, and after stirring for 30 minutes, the mixture was stirred for 4 hours at 0°C. A saturated aqueous Rochelle salt solution and ethyl acetate were added, and the mixture was stirred for 4 hours. After oil-water distribution of the reaction mixture, the organic layer was washed with brine and dried over sodium sulfate. The organic layer was filtered, and the filtrate was then concentrated under reduced pressure to obtain a crude product. To a solution of the obtained crude product in dichloromethane (300 mL) at room temperature there were added DHP (10.7 mL, 117 mmol) and *p*-toluenesulfonic acid monohydrate (1.11 g, 5.83 mmol) at 0°C, and the mixture was stirred for 2 hours at room temperature. A saturated aqueous sodium hydrogencarbonate solution was added to the reaction mixture, and extraction was performed with dichloromethane. The organic layer was dried over anhydrous magnesium sulfate and filtered, and the filtrate was concentrated under reduced pressure. After adding a heptane: TBME = 1:1 mixed solvent to the residue, the precipitated solid was filtered out to obtain a portion of the title compound. The filtrate was purified by silica gel column chromatography (*n*-heptane: ethyl acetate = 99:1 to 7:3) to obtain the total amount of title compound (7.76 g).
ESI-MS (m/z): 571.14 $[M+H]^+$.

[Production Example 113-3]

4-(2-Chloro-4-(1,1-diphenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one

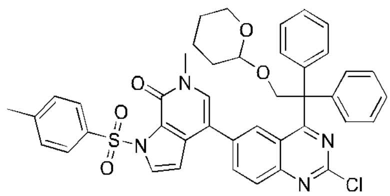

The title compound (550 mg) was obtained from the 2-chloro-4-(1,1-diphenyl-2-((tetrahydro-2H-pyran-2-yl)oxy)ethyl)-6-iodoquinazoline of Production Example 113-2 (800 mg, 1.40 mmol), by the same method as Production Example 104-4.
ESI-MS (m/z): 745.32 $[M+H]^+$.

[Production Example 113-4]

*tert*-Butyl (2R,5S)-4-(1-(4-(1,1-diphenyl-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-carboxylate

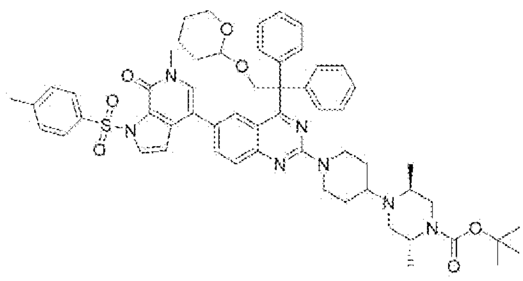

The title compound (93 mg) was obtained from the 4-(2-chloro-4-(1,1-diphenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 113-3 (80 mg, 0.11 mmol) and the *tert*-butyl (2R,5S)-2,5-dimethyl-4-(piperidin-4-yl)piperazine-1-carboxylate of Production Example 114-2 (42 mg, 0.14 mmol), by the same method as Production Example 108-1. ESI-MS (m/z): 1006.72 $[M+H]^+$.

[Production Example 113-5]

*tert-Butyl* (2R,5S)-4-(1-(4-(1,1-diphenyl-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate

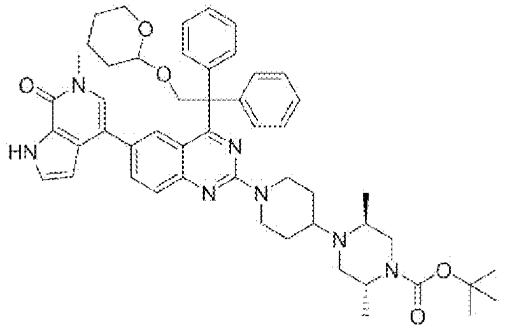

A crude product of the title compound (79 mg) was obtained from the *tert-butyl* (2R,5 S)-4-(1-(4-(1,1-diphenyl-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-carboxylate of Production Example 113-4 (93 mg, 92 μmol), by the same method as Production Example 104-6.
ESI-MS (m/z): 853.08 $[M+H]^+$

[Example 114]

1-(5-((2R,5S)-4-(1-(4-((R)-2-Amino-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carbonyl)-2-methoxyphenyl)dihydropyrimidine-2,4(1H,3H)-dione

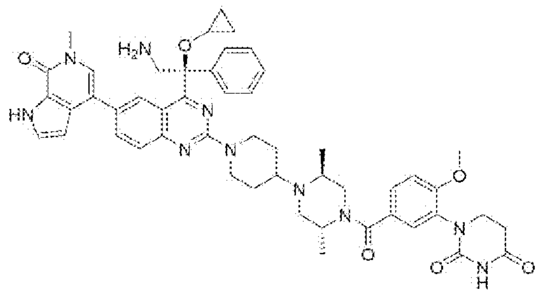

To a solution of the *tert*-butyl (2R,5S)-4-(1-(4-((R)-2-((((9H-fluoren-9-yl)methoxy)carbonyl)amino)-1-cyclopropoxy-1-phenylethyl)-6-(6-methyl-7-oxo-6,7-dihydro-1H-pyrrolo[2,3-c]pyridin-4-yl)quinazolin-2-yl)-piperidin-4-yl)-2,5-dimethylpiperazine-1-carboxylate of Production Example 114-5 (15 mg, 15 μmol) in dichloromethane (750 μL) there was added TFA (250 μL), and the mixture was stirred for 45 minutes at room temperature. The reaction mixture was concentrated under reduced pressure to obtain a crude product. To a solution of the obtained crude product and the 3-(2,4-dioxotetrahydropyrimidine-1(2H)-yl)-4-methoxybenzoic acid of Production Example 104-7 (6.1 mg, 23 μmol) in DMF (500 μL) there were added N,N-diisopropylethylamine (27.0 μL, 155 μmol) and HATU (8.8 mg, 23 μmol), and the mixture was stirred for 30 minutes at room temperature. After adding diethylamine (48.5 μL, 464 μmol) to the reaction mixture, the mixture was stirred for 45 minutes. The reaction mixture was purified by ODS silica gel chromatography (0.1% formic acid aqueous solution:0.1% acetonitrile formate solution = 9:1 to 6:4) to obtain a formate of the title compound (9.1 mg).
ESI-MS (m/z): 893.57 $[M+H]^+$.

[Production Example 114-1]

*tert*-Butyl (2R,5 S)-4-(1-((benzyloxy)carbonyl)piperidin-4-yl)-2,5-dimethylpiperazine-1-carboxylate

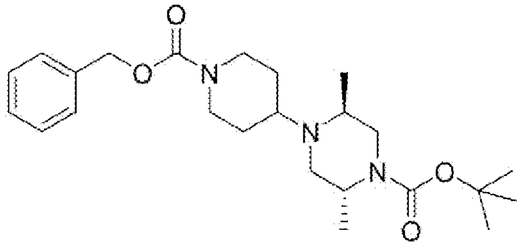

The title compound (1.16 g) was obtained from *tert-butyl* (2R,5S)-2,5-dimethylpiperazine-1-carboxylate (1.10 g, 5.14 mmol) and 1-(benzyloxycarbonyl)-4-piperidinone (1.00 g, 4.29 mmol), by the same method as Production Example 104-1.

ESI-MS (m/z): 432.34 $[M+H]^+$.

[Production Example 114-2]

*tert*-Butyl (2R,5S)-2,5-dimethyl-4-(piperidin-4-yl)piperazine-1-carboxylate

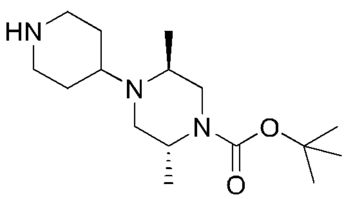

The title compound (2.81 g) was obtained from the *tert*-butyl (2R,5S)-4-(1-((benzyloxy)carbonyl)piperidin-4-yl)-2,5-dimethylpiperazine- 1-carboxylate of Production Example 114-1 (4.29 g, 9.94 mmol), by the same method as Production Example 104-2. ESI-MS (m/z): 298.30 $[M+H]^+$.

[Production Example 114-3]

*tert*-Butyl (2R,5S)-4-(1-(4-((1S)-1-cyclopropoxy-1-phenyl-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)-piperidin-4-yl)-2,5-dimethylpiperazine-1-carboxylate

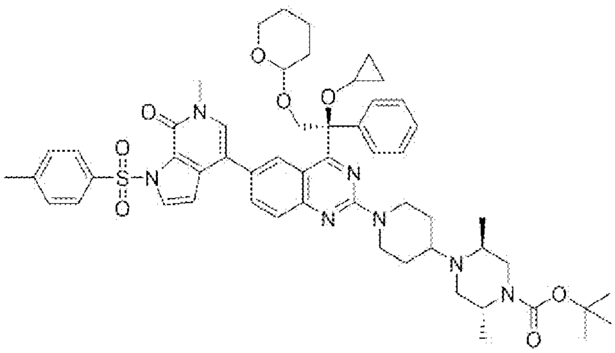

The title compound (44 mg) was obtained from the 2-chloro-4-((1S)-cyclopropoxy-1-phenyl-2-((tetrahydro-2H-pyran-2-yl)oxy)ethyl)-6-iodoquinazoline of Production Example 102-1 (44 mg, 54 μmol) and the *tert-butyl* (2R,5S)-2,5-dimethyl-4-(piperidin-4-yl)piperazine-1-carboxylate of Production Example 114-2 (29.2 mg, 98 μmol), by the same

method as Production Example 104-5 and Production Example 104-4.
ESI-MS (m/z): 986.74 [M+H]+.

[Production Example 114-4]

*tert*-Butyl (2R,5S)-4-(1-(4-((R)-2-amino-1-cyclopropoxy-1-phenylethyl)-6-(6-methyl-7-oxo-1-tosyl-6,7-dihydro-1H-pyrrolo[2,3-c]pyridin-4-yl)quinazolin-2-yl)-piperidin-4-yl)-2,5-dimethylpiperazine-1-carboxylate

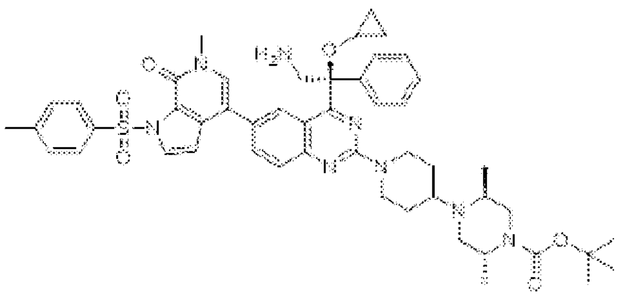

The title compound (22 mg) was obtained from the *tert*-butyl (2R,5S)-4-(1-(4-((1S)-1-cyclopropoxy-1-phenyl-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)-piperidin-4-yl)-2,5-dimethylpiperazine-1-carboxylate of Production Example 114-3 (44 mg, 45 μmol), by the same method as Production Example 117-7 and Production Example 117-8.
ESI-MS (m/z): 901.57 [M+H]+.

[Production Example 114-5]

*tert*-Butyl (2R,5S)-4-(1-(4-((R)-2-((((9H-fluoren-9-yl)methoxy)carbonyl)amino)-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate

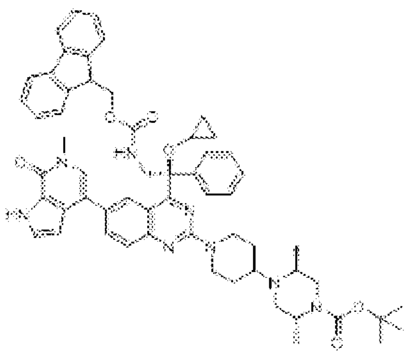

The title compound (15 mg) was obtained from the *tert*-butyl (2R,5S)-4-(1-(4-((R)-2-amino-1-cyclopropoxy-1-phenylethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-carboxylate of Production Example 114-4 (22 mg, 24 μmol), by the same method as Production Example 117-9.
ESI-MS (m/z): 969.65 [M+H]+.

[Example 115]

1-(5-((2R,5S)-4-((1-(4-((R)-2-Amino-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)methyl)-2,5-dimethylpiperazine-1-carbonyl)-2-methoxyphenyl)dihydropyrimidine-2,4-(1H,3H)-dione

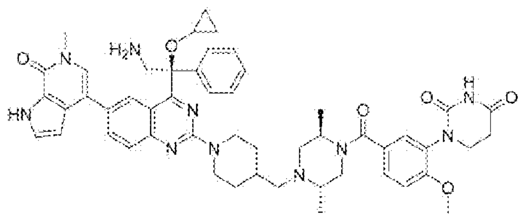

The title compound (14 mg) was obtained from the *tert*-butyl (2R,5S)-4-((1-(4-((R)-2-((((OH-fluoren-9-yl)methoxy)carbonyl)amino)-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)methyl)-2,5-dimethylpiperazine-1-carboxylate of Production Example 115-2 (44 mg, 45 μmol) and the 3-(2,4-dioxotetrahydropyrimidine-1(2H)-yl-4-methoxybenzoic acid of Production Example 104-7 (18 mg, 67 μmol), by the same method as Example 114.
ESI-MS (m/z): 908.54 $[M+H]^+$.

[Production Example 115-1]

*tert-Butyl* (2R,5 S)-4-((1-(4-((S)-1-cyclopropoxy-2-hydroxy-1-phenylethyl)-6-(6-methyl-7-oxo-1-tosyl-6,7-dihydro-1H-pyrrolo[2,3-c]pyridin-4-yl)quinazolin-2-yl)piperidin-4-yl)methyl)-2,5-dimethylpiperazine-1-carboxylate

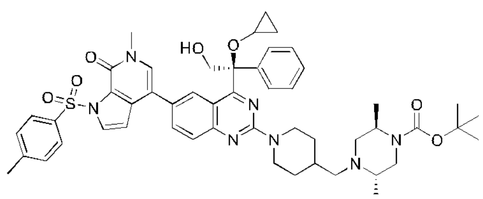

The title compound (130 mg) was obtained from the 4-(2-chloro-4-((1S)-1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 102-2 (100 mg, 138 μmol) and the *tert*-butyl (2R,5S)-2,5-dimethyl-4-(piperidin-4-ylmethyl)piperazine-1-carboxylate of Production Example 111-6 (55.8 mg, 179 μmol), by the same method as Production Example 104-5 and Production Example 117-7.
ESI-MS (m/z): 917.26 $[M+H]^+$.

[Production Example 115-2]

*tert*-Butyl (2R,5S)-4-((1-(4-((R)-2-((((9H-fluoren-9-yl)methoxy)carbonyl)amino)-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)methyl)-2,5-dimethylpiperazine-1-carboxylate

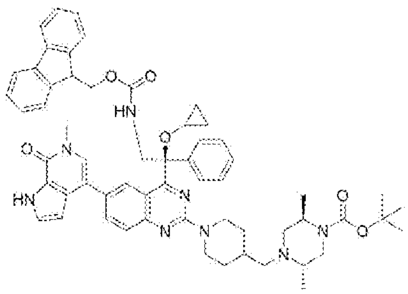

The title compound (46 mg) was obtained from the *tert-butyl* (2R,5S)-4-((1-(4-((S)-1-cyclopropoxy-2-hydroxy-1-phenylethyl)-6-(6-methyl-7-oxo-1-tosyl-6,7-dihydro-1H-pyrrolo[2,3-c]pyridin-4-yl)quinazolin-2-yl)piperidin-4-yl) methyl)-2,5-dimethylpiperazine-1-carboxylate of Production Example 115-1 (65 mg, 71 μmol), by the same method as Production Example 117-8 and Production Example 117-9.
ESI-MS (m/z): 983.67 $[M+H]^+$.

[Example 116]

1-(5-((2R,5S)-4-(1-(4-(2-Amino-1-cyclopropoxy-1-(4-fluorophenyl)ethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carbonyl)-2-methoxyphenyl)dihydropyrimidine-2,4(1H,3H)-dione

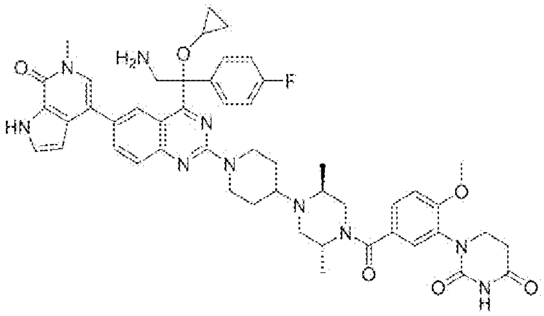

To a solution of the *tert*-butyl (2R,5S)-4-(1-(4-(2-((((9H-fluoren-9-yl)methoxy)carbonyl)amino-1-cyclopropoxy-1-(4-fluorophenyl)ethyl)-6-(6-methyl-7-oxo-6,7-dihydro-1H-pyrrolo[2,3-c]pyridin-4-yl)quinazolin-2-yl)-piperidin-4-yl)-2,5-dimethylpiperazine-1-carboxylate of Production Example 116-4 (15.6 mg, 16 μmol) in dichloromethane (750 μL) there was added TFA (250 μL), and the mixture was stirred for 45 minutes at room temperature. The reaction mixture was concentrated under reduced pressure to obtain a crude product. To a solution of the obtained crude product and the 3-(2,4-dioxotetrahydropyrimidine-1(2H)-yl-4-methoxybenzoic acid of Production Example 104-7 (6.3 mg, 24 μmol) in DMF (500 μL) there were added N,N-diisopropylethylamine (27.6 μL, 158 μmol) and HATU (9.0 mg, 24 μmol), and the mixture was stirred for 30 minutes at room temperature, after which diethylamine (49.5 μL, 474 μmol) was added and the mixture was further stirred for 45 minutes. The reaction mixture was purified by ODS silica gel chromatography (0.1% formic acid aqueous solution:0.1% acetonitrile formate solution = 9:1 to 6:4) to obtain a formate of the title compound (8.2 mg).
ESI-MS (m/z): 911.37 $[M+H]^+$.

[Production Example 116-1]

4-(2-Chloro-4-(1-cyclopropoxy-1-(4-fluorophenyl)-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one

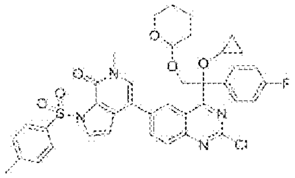

The title compound (103 mg) was obtained from the 2-chloro-4-(1-cyclopropoxy-1-(4-fluorophenyl)-2-((tetrahydro-2H-pyran-2-yl)oxy)ethyl)6-iodoquinazoline of Production Example 105-2 (87.5 mg, 154 μmol), by the same method as Production Example 104-4. ESI-MS (m/z): 743.31 [M+H]+.

[Production Example 116-2]

*tert*-Butyl (2R,5S)-4-(1-(4-(1-cyclopropoxy-1-(4-fluorophenyl)-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-catboxylate

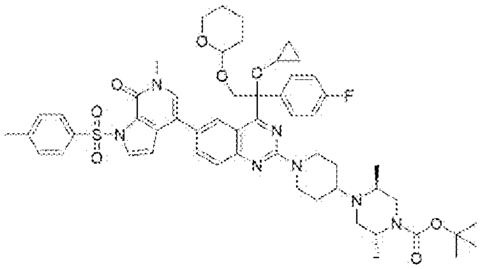

The title compound (54 mg) was obtained from the 4-(2-chloro-4-(1-cyclopropoxy-1-(4-fluorophenyl)-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 116-1 (40 mg, 54 μmol) and the *tert*-butyl (2R,5S)-2,5-dimethyl-4-(piperidin-4-yl)piperazine-1-carboxylate of Production Example 114-2 (29 mg, 97 μmol), by the same method as Production Example 104-5.
ESI-MS (m/z): 1005.43 [M+H]+.

[Production Example 116-3]

*tert-Butyl* (2R,5S)-4-(1-(4-(2-amino-1-cyclopropoxy-1-(4-fluorophenyl)ethyl)-6-(6-methyl-7-oxo-1-tosyl-6,7-dihydro-1H-pyrrolo[2,3-c]pyridin-4-yl)quinazolin-2-yl)-piperidin-4-yl)-2,5-dimethylpiperazine-1-carboxylate

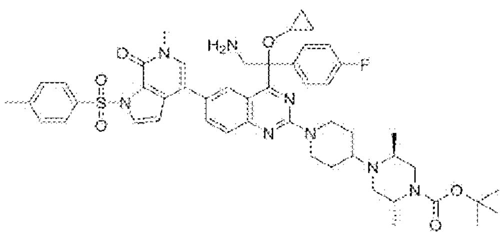

A formate of the title compound (20 mg) was obtained from the *tert*-butyl (2R,5S)-4-(1-(4-(1-cyclopropoxy-1-(4-fluorophenyl)-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)-piperidin-4-yl)-2,5-dimethylpiperazine-1-carboxylate of Production Example 116-2 (36 mg, 36 μmol), by the same method as Production Example 117-7 and Production Example 117-8.
ESI-MS (m/z): 919.40 [M+H]+.

[Production Example 116-4]

*tert*-Butyl (2R,5S)-4-(1-(4-(2-((((9H-fluoren-9-yl)methoxy)carbonyl)amino-1-cyclopropoxy-1-(4-fluorophenyl) ethyl)-6-(6-methyl-7-oxo-6,7-dihydro-1H-pyrrolo[2,3-c]pyridin4-yl)quinazolin-2-yl)-piperidin-4-yl)-2,5-dimethylpiperazine-1-carboxylate

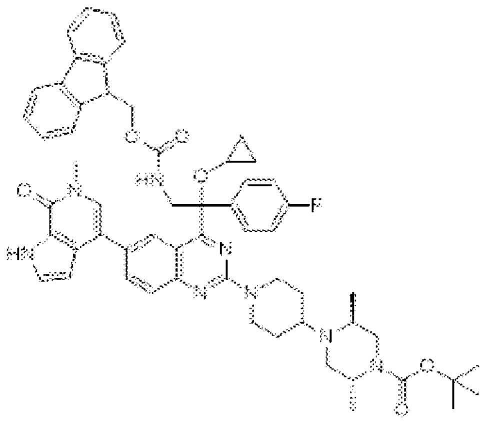

The title compound (16 mg) was obtained from a formate of the *tert*-butyl (2R,5 S)-4-(1-(4-(-2-amino-1-cyclopropoxy-1-(4-fluorophenyl)ethyl)-6-(6-methyl-7-oxo-1-tosyl-6,7-dihydro-1H-pyrrolo[2,3-c]pyridin-4-yl)quinazolin-2-yl)-piperidin-4-yl)-2,5-dimethylpiperazine-1-carboxylate of Production Example 116-3 (20 mg), by the same method as Production Example 117-9.

ESI-MS (m/z): 987.65 $[M+H]^+$.

[Example 117]

1-(5-((2R,5S)-4-(1-(4-(2-Amino-1-cyclopropoxy-1-(3-fluorophenyl)ethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carbonyl)-2-methoxyphenyl)dihydropyrimidine-2,4(1H,3H)-dione

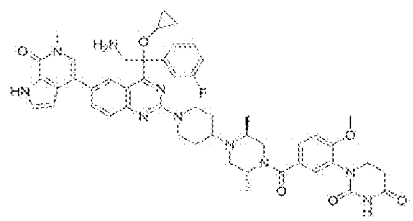

To a solution of the *tert*-butyl (2R,5S)-4-(1-(4-(2-((((9H-fluoren-9-yl)methoxy)carbonyl)amino)-1-cyclopropoxy-1-(3-fluorophenyl)ethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate of Production Example 117-9 (25 mg, 25 μmol) in dichloromethane (1 mL) there was added TFA (1 mL) at room temperature, and the mixture was stirred for 30 minutes at room temperature. The reaction mixture was concentrated under reduced pressure to obtain a crude product. Separately, to a solution of the 3-(2,4-dioxotetrahydropyrimidine-1 (2H)-yl)-4-methoxybenzoic acid of Production Example 104-7 (13 mg, 51 μmol) in DMF (2 mL) there were added N,N-diisopropylethylamine (88 μL, 0.51 mmol) and HATU (16 mg, 43 μmol) at room temperature, and the mixture was stirred for 30 minutes at room temperature. This reaction mixture was added to the obtained crude product at room temperature, and the mixture was stirred for 1 hour at room temperature. Diethylamine (500 μL) was then added to the reaction mixture at room temperature, and the mixture was stirred for 30 minutes at room temperature. After concentrating the reaction mixture under reduced pressure, the residue was purified by ODS silica gel column chromatography (0.1% formic acid-containing water:0.1% formic acid-containing acetonitrile = 95:5 to 1:1), to obtain a formate of the title compound (26 mg).

ESI-MS (m/z): 911.61 $[M+H]^+$.

[Production Example 117-1]

Methyl 2-diazo-2-(3-fluorophenyl)acetate

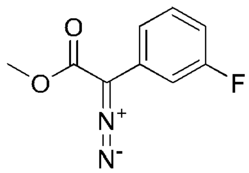

The title compound (2.52 g) was obtained from methyl 2-(3-fluorophenyl)acetate (2.50 g, 14.9 mmol), by the same method as Production Example 106-1.

$^1$H-NMR Spectrum (400 MHz, $CDCl_3$) δ(ppm): 3.87 (3H, s), 6.82-6.88 (2H, m), 7.13-7.18 (1H, m), 7.31-7.35 (2H, m).

[Production Example 117-2]

Methyl 2-cyclopropoxy-2-(3-fluorophenyl)acetate

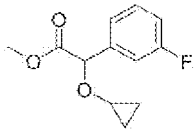

The title compound (2191 mg) was obtained from the methyl 2-diazo-2-(3-fluorophenyl)acetate of Production Example 117-1 (2516 mg, 13.0 mmol), by the same method as Production Example 101-1.

$^1$H-NMR Spectrum (400 MHz, $CDCl_3$) δ(ppm): 0.43-0.57 (2H, m), 0.63-0.78 (2H, m), 3.42 (1H, tt, J=6.1, 2.9 Hz), 3.73 (3H, s), 4.95 (1H, s), 6.97-7.05 (1H, m), 7.15-7.19 (1H, m), 7.20-7.23 (1H, m), 7.29-7.33 (1H, m).

[Production Example 117-3]

Methyl 2-(2-chloro-6-iodoquinazolin-4-yl)-2-cyclopropoxy-2-(3-fluorophenyl)acetate

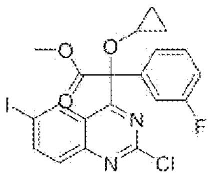

The title compound (1242 mg) was obtained from the methyl 2-cyclopropoxy-2-(3-fluorophenyl)acetate of Production Example 117-2 (1166 mg, 5.20 mmol), by the same method as Production Example 101-2.

ESI-MS (m/z): 513.11 [M+H]$^+$.

[Production Example 117-4]

2-Chloro-4-(1-cyclopropoxy-1-(3-fluorophenyl)-2-((tetrahydro-2H-pyran-2-yl)oxy)ethyl)-6-iodoquinazoline

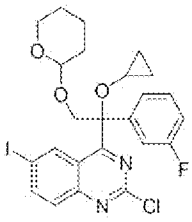

The title compound (442 mg) was obtained from the methyl 2-(2-chloro-6-iodoquinazolin-4-yl)-2-cyclopropoxy-2-(3-fluorophenyl)acetate of Production Example 117-3 (1242 mg, 2.42 mmol), by the same method as Production Example 104-3.
ESI-MS (m/z): 569.13 $[M+H]^+$.

[Production Example 117-5]

*tert*-Butyl (2R,5S)-4-(1-(4-(1-cyclopropoxy-1-(3-fluorophenyl)-2-((tetrahydro-2H-pyran-2-yl)oxy)ethyl)-6-iodoquinazolin-2-yl)piperidin-4-yl)-2,5-dimethylpiperazine-1-carboxylate

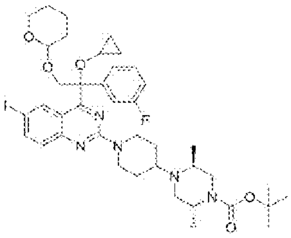

The title compound (314 mg) was obtained from the 2-chloro-4-(1-cyclopropoxy-1-(3-fluorophenyl)-2-((tetrahydro-2H-pyran-2-yl)oxy)ethyl)-6-iodoquinazoline of Production Example 117-4 (240 mg, 422 μmol) and the *tert-butyl* (2R,5S)-2,5-dimethyl-4-(piperidin-4-yl)piperazine-1-carboxylate of Production Example 114-2 (163 mg, 549 μmol), by the same method as Production Example 104-5.
ESI-MS (m/z): 830.45 $[M+H]^+$.

[Production Example 117-6]

*tert*-Butyl (2R,5S)-4-(1-(4-(1-cyclopropoxy-1-(3-fluorophenyl)-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-carboxylate

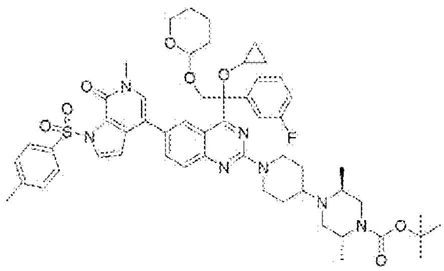

To a solution of the *tert*-butyl (2R,5S)-4-(1-(4-(1-cyclopropoxy-1-(3-fluorophenyl)-2-((tetrahydro-2H-pyran-2-yl)oxy)ethyl)-6-iodoquinazolin-2-yl)piperidin-4-yl)-2,5-dimethylpiperazine-1-carboxylate of Production Example 117-5 (314 mg, 378 μmol) in 1,4-dioxane (4 mL) and water (2 mL) there were added 6-methyl-4-(4,4,5,5-tetramethyl-1,3,2-dioxaborolan-2-yl)-1-tosyl-1,6-dihydro-7H-pyrrolo[2,3-c]pyridin-7-one (194 mg, 454 μmol), tetrakis(triphenylphosphine)palladium(0) (44 mg, 38 μmol) and cesium carbonate (185 mg, 568 μmol) at room temperature, and the mixture

was stirred for 1 hour at 80°C under a nitrogen atmosphere. The reaction mixture was returned to room temperature, and then water was added and extraction was performed with ethyl acetate. The organic layer was washed with brine, dried over anhydrous magnesium sulfate and filtered, and the filtrate was concentrated under reduced pressure. The residue was purified by NH silica gel column chromatography (*n*-heptane: ethyl acetate = 7:3 to ethyl acetate) to obtain the title compound (379 mg).

ESI-MS (m/z): 1004.67 [M+H]+.

[Production Example 117-7]

*tert*-Butyl (2R,5S)-4-(1-4-(1-cyclopropoxy-1-(3-fluorophenyl)-2-hydroxyethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-carboxylate

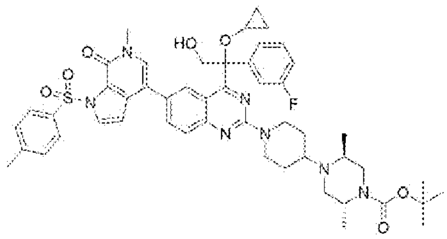

A solution of the *tert*-butyl (2R,5S)-4-(1-(4-(1-cyclopropoxy-1-(3-fluorophenyl)-2-((tetrahydro-2H-pyran-2-yl) oxy)ethyl)-6-(6-methyl-7-oxo-1-tosyl-6,7-dihydro-1H-pyrrolo[2,3-c]pyridin-4-yl)-quinazolin-2-yl)piperidin-4-yl)-2,5-dimethylpiperazine-1-carboxylate of Production Example 117-6 (379 mg, 377 μmol) and p-toluenesulfonic acid monohydrate (215 mg, 1.13 mmol) in methanol (5 mL) was stirred for 1 hour at room temperature. A saturated aqueous sodium hydrogencarbonate solution was added to the reaction mixture, and extraction was performed with dichloromethane. The organic layer was dried over anhydrous magnesium sulfate and filtered, and the filtrate was concentrated under reduced pressure. The residue was purified by NH silica gel column chromatography (*n*-heptane:ethyl acetate = 7:3 to ethyl acetate) to obtain the title compound (286 mg). ESI-MS (m/z): 920.60 [M+H]+.

[Production Example 117-8]

*tert*-Butyl (2R,5S)-4-(1-(4-(2-amino-1-cyclopropoxy-1-(3-fluorophenyl)ethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-carboxylate

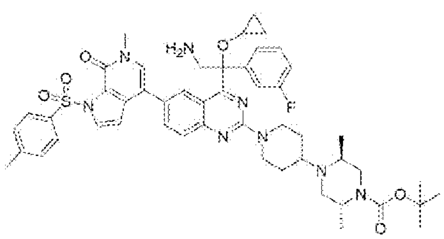

To a solution of the *tert*-butyl (2R,5S)-4-(1-(4-(1-cyclopropoxy-1-(3-fluorophenyl)-2-hydroxyethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-carboxylate of Production Example 117-7 (241 mg, 262 μmol) in dichloromethane (4 mL) there were added N,N-diisopropylethylamine (137 μL, 786 μmol) and methanesulfonyl chloride (41 μL, 0.52 mmol) at room temperature, and the mixture was stirred for 30 minutes at room temperature. Water was added to the reaction mixture, which was then extracted with dichloromethane. The organic layer was dried over anhydrous magnesium sulfate and filtered, and the filtrate was concentrated under reduced pressure. To a solution of the residue in NMP (4 mL) there was added 28% ammonia water (2 mL) at room temperature, and a microwave reactor was used for reaction at 130°C for 50 minutes. The reaction mixture was purified by ODS silica gel column chromatography (0.1% formic acid-containing water:0.1% formic acid-containing acetonitrile = 95:5 to 1:1) to obtain a formate of the title compound (141 mg). ESI-MS (m/z): 919.65 [M+H]+.

[Production Example 117-9]

*tert*-Butyl (2R,5S)-4-(1-(4-(2-((((9H-fluoren-9-yl)methoxy)carbonyl)amino)-1-cyclopropoxy-1-(3-fluorophenyl)ethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate

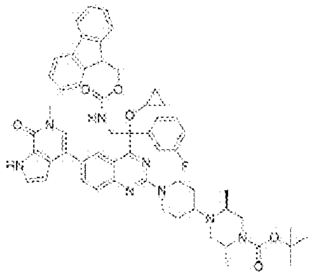

To a solution of the formate of *tert*-butyl (2R,5S)-4-(1-(4-(2-amino-1-cyclopropoxy-1-(3-fluorophenyl)ethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-carboxylate of Production Example 117-8 (141 mg) in THF (4 mL) and methanol (2 mL) there was added a 1 M sodium hydroxide aqueous solution (1.53 mL, 1.53 mmol) at room temperature, and the mixture was stirred for 30 minutes at 60°C. After returning the reaction mixture to room temperature, brine was added and extraction was performed with ethyl acetate. The organic layer was dried over anhydrous magnesium sulfate and filtered, and the filtrate was concentrated under reduced pressure. To a solution of the residue in dichloromethane (3 mL) there were added triethylamine (64 μL, 0.46 mmol) and 9-fluorenylmethyl chloroformate (47.6 mg, 184 μmol) at room temperature, and the mixture was stirred for 15 minutes at room temperature. The reaction mixture was concentrated under reduced pressure and purified by silica gel column chromatography (n-heptane:ethyl acetate = 1:1 to ethyl acetate to ethyl acetate:methanol = 9:1) to obtain the title compound (84 mg).

ESI-MS (m/z): 987.61 $[M+H]^+$.

[Example 118]

1-(5-((2R,5S)-4-(1-(4-(2-Amino-1-cyclopropoxy-1-(2-fluorophenyl)ethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carbonyl)-2-methoxyphenyl)dihydropyrimidine-2,4(1H,3H)-dione

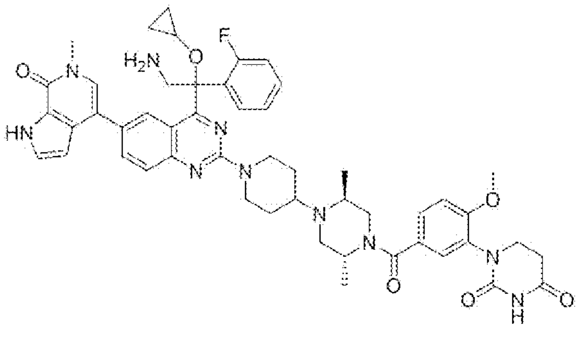

The title compound (4.6 mg) was obtained from the *tert-butyl* (2R,5S)-4-(1-(4-(2-((((9H-fluoren-9-yl)methoxy)carbonyl)amino)-1-cyclopropoxy-1-(2-fluorophenyl)ethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate of Production Example 118-7 (40.5 mg, 41 μmol), by the same method as Example 117.

ESI-MS (m/z): 911.61 $[M+H]^+$.

[Production Example 118-1]

Methyl 2-diazo-2-(2-fluorophenyl)acetate

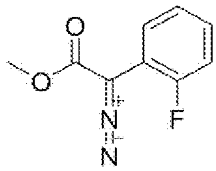

The title compound (2.3 g) was obtained from methyl (2-fluorophenyl)acetate (2.0 g, 12 mmol), by the same method as Production Example 106-1.

$^1$H-NMR Spectrum (400 MHz, $CDCl_3$) δ(ppm): 3.84 (3H, s), 7.00-7.12 (1H, m), 7.15-7.27 (2H, m), 7.57-7.74 (1H, m).

[Production Example 118-2]

Methyl 2-cyclopropoxy-2-(2-fluorophenyl)acetate

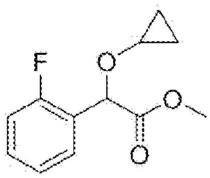

The title compound (1.81 g) was obtained from the methyl 2-diazo-2-(2-fluorophenyl)acetate of Production Example 118-1 (2.30 g, 11.8 mmol), by the same method as Production Example 101-1.

$^1$H-NMR Spectrum (400 MHz, $CDCl_3$) δ(ppm): 0.42-0.52 (2H, m), 0.59-0.74 (2H, m), 3.39-3.53 (1H, m), 3.73 (3H, s), 5.31 (1H, s), 6.97-7.10 (1H, m), 7.12-7.19 (1H, m), 7.27-7.37 (1H, m), 7.39-7.50 (1H, m).

[Production Example 118-3]

Methyl 2-(2-chloro-6-iodoquinazolin-4-yl)-2-cyclopropoxy-2-(2-fluorophenyl)acetate

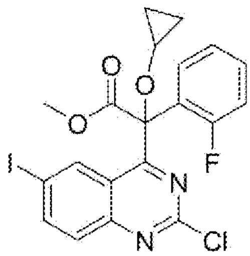

The title compound (3.22 g) was obtained from the methyl 2-cyclopropoxy-2-(2-fluorophenyl)acetate of Production Example 118-2 (1.80 g, 8.03 mmol), by the same method as Production Example 101-2.

ESI-MS (m/z): 513.03 [M+H]$^+$.

[Production Example 118-4]

2-Chloro-4-(1-cyclopropoxy-1-(2-fluorophenyl)-2-((tetrahydro-2H-pyran-2-yl)oxy)ethyl)-6-iodoquinazoline

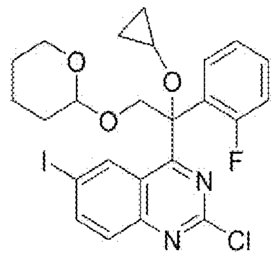

The title compound (2.73 g) was obtained from the methyl 2-(2-chloro-6-iodoquinazolin-4-yl)-2-cyclopropoxy-2-(2-fluorophenyl)acetate of Production Example 118-3 (3.22 g, 6.28 mmol), by the same method as Production Example 104-3.

ESI-MS (m/z): 569.08 $[M+H]^+$.

[Production Example 118-5]

*tert*-Butyl (2R,5S)-4-(1-(4-(1-cyclopropoxy-1-(2-fluorophenyl)-2-hydroxyethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-carboxylate

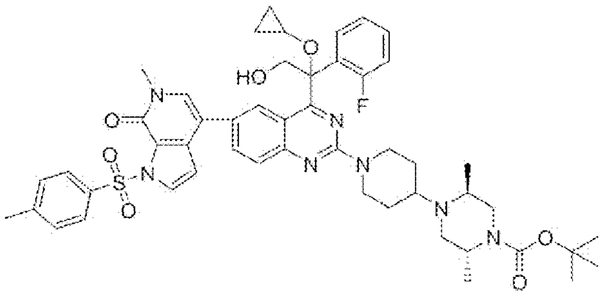

The title compound (152 mg) was obtained from the 2-chloro-4-(1-cyclopropoxy-1-(2-fluorophenyl)-2-((tetrahydro-2H-pyran-2-yl)oxy)ethyl)-6-iodoquinazoline of Production Example 118-4 (106 mg, 186 μmol) and the *tert-butyl* (2R,5S)-2,5-dimethyl-4-(piperidin-4-yl)piperazine-1-carboxylate of Production Example 114-2 (66.5 mg, 224 μmol), by the same method as Production Example 117-5, Production Example 117-6 and Production Example 117-7.

ESI-MS (m/z): 921.16 $[M+H]^+$.

[Production Example 118-6]

*tert*-Butyl (2R,5S)-4-(1-(4-(2-amino-1-cyclopropoxy-1-(2-fluorophenyl)ethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-carboxylate

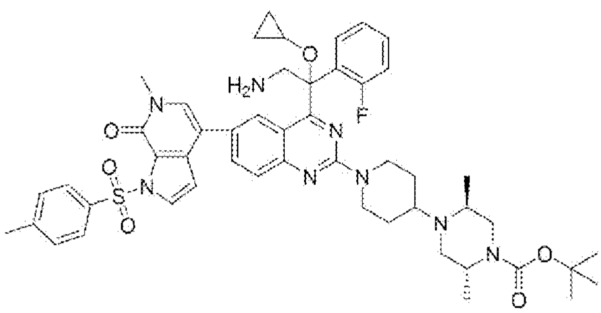

The title compound (64 mg) was obtained from the *tert*-butyl (2R,5S)-4-(1-(4-(1-cyclopropoxy-1-(2-fluorophenyl)-2-hydroxyethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-carboxylate of Production Example 118-5 (152 mg, 165 μmol), by the same method as Production Example 117-8.

ESI-MS (m/z): 919.69 [M+H]+.

[Production Example 118-7]

*tert*-Butyl (2R,5S)-4-(1-(4-(2-((((9H-fluoren-9-yl)methoxy)carbonyl)amino)-1-cyclopropoxy-1-(2-fluorophenyl)ethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate

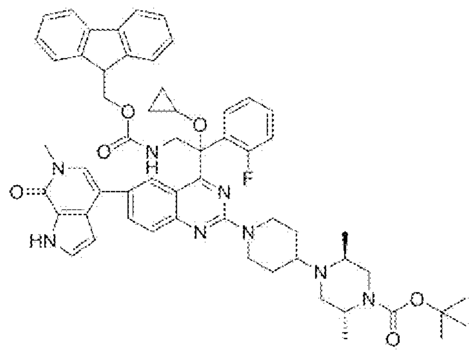

The title compound (41 mg) was obtained from the *tert-butyl* (2R,5S)-4-(1-(4-(2-amino-1-cyclopropoxy-1-(2-fluorophenyl)ethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-carboxylate of Production Example 118-6 (64 mg, 70 μmol), by the same method as Production Example 117-9.

ESI-MS (m/z): 987.73 [M+H]+.

[Example 119]

2-Cyclopropoxy-2-(2-(4-(((2S,5R)-4-(3-(2,4-dioxotetrahydropyrimidine-1(2H)-yl)-4-methoxybenzoyl)-2,5-dimethylpiperazin-1-yl)methyl)piperidin-1-yl)-6-(6-methyl-7-oxo-6,7-dihydro-1H-pyrrolo[2,3-c]pyridin-4-yl)quinazolin-4-yl)-2-phenylethyl methyl(2-(methylamino)ethyl)carbamate

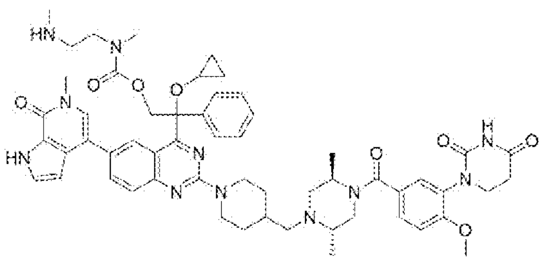

To a solution of the benzyl (2-cyclopropoxy-2-(2-(4-(((2S,5R)-4-(3-(2,4-dioxotetrahydropyrimidine-1(2H)-yl)-4-methoxybenzoyl)-2,5-dimethylpiperazin-1-yl)methyl)piperidin-1-yl)-6-(6-methyl-7-oxo-6,7-dihydro-1H-pyrrolo[2,3-c]pyridin-4-yl)quinazolin-4-yl)-2-phenylethyl ethane-1,2-diylbis(methylcarbamate) of Production Example 119-5 (57.5 mg, 50.0 μmol) in THF (2 mL) and ethyl acetate (2 mL) there was added 10% palladium-carbon (50% water, 42.3 mg) at room temperature, and the mixture was stirred for 1.5 hours at room temperature, ordinary pressure under a hydrogen atmosphere. After further adding 10% palladium-carbon (50% water, 106 mg), the mixture was stirred for 16 hours and 20 minutes at room temperature, ordinary pressure under a hydrogen atmosphere. After switching the reaction mixture to a nitrogen atmosphere, it was filtered with Celite and washed with methanol. The filtrate was concentrated under reduced pressure and the residue was purified by NH silica gel thin-layer chromatography (chloroform:methanol = 20:1), to obtain the title compound (18.5 mg).

ESI-MS (m/z): 1022.72 [M+H]+.

[Production Example 119-1]

*tert*-Butyl (2R,5S)-4((1-(4-(1-cyclopropoxy-1-phenyl-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)piperidin-4-yl)methyl)-2,5-dimethylpiperazine-1-carboxylate

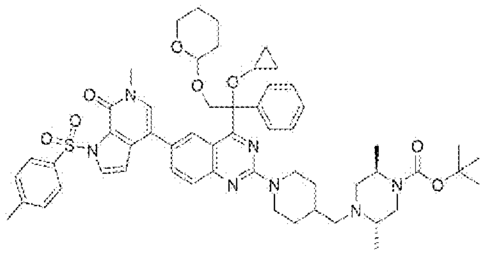

To a solution of the 4-(2-chloro-4-(1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 104-4 (400 mg, 552 µmol) in DMF (2 mL) there were added the *tert*-butyl (2R,5S)-2,5-dimethyl-4-(piperidin-4-ylmethyl)piperazine-1-carboxylate of Production Example 111-6 (240 mg, 772 µmol) and N,N-diisopropylethylamine (295 µL, 1.66 mmol) at room temperature, and the mixture was stirred for 7 hours at 80°C. The reaction mixture was returned to room temperature, and then water was added and extraction was performed with ethyl acetate. The organic layer was washed with water and brine, dried over anhydrous magnesium sulfate and filtered, and the filtrate was concentrated under reduced pressure. The residue was purified by NH silica gel column chromatography (n-heptane:ethyl acetate = 1:3 to ethyl acetate) to obtain the title compound (389 mg).

ESI-MS (m/z): 1000.84 [M+H]+.

[Production Example 119-2]

*tert*-Butyl (2R,5 S)-4-((1-(4-(1-cyclopropoxy-2-hydroxy-1-phenylethyl)-6-(6-methyl-7-oxo-1-tosyl-6,7-dihydro-1H-pyrrolo[2,3-c]pyridin-4-yl)quinazolin-2-yl)piperidin-4-yl)methyl)-2,5-dimethylpiperazine-1-carboxylate

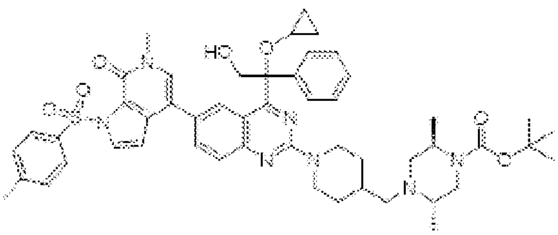

The title compound (232 mg) was obtained from the *tert*-butyl (2R,5S)-4-((1-(4-(1-cyclopropoxy-1-phenyl-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)piperidin-4-yl)methyl-2,5-dimethylpiperazine-1-carboxylate of Production Example 119-1 (250 mg, 250 µmol), by the same method as Production Example 117-7.

ESI-MS (m/z): 916.77 [M+H]+.

[Production Example 119-3]

*tert*-Butyl (2R,5S)-4-((1-(4-(11-cyclopropoxy-4,7-dimethyl-3,8-dioxo-1,11-diphenyl-2,9-dioxa-4,7-diazaundecan-11-yl)-66-methyl-7-oxo-1-tosyl-6,7-dihydro-1H-pyrrolo[2,3-c]pyridin-4-yl)quinazolin-2-yl)piperidin-4-yl)methyl)-2,5-dimethylpiperazine-1-carboxylate

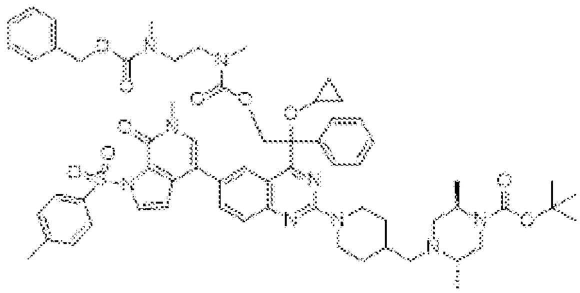

To a solution of the *tert*-butyl (2R,5S)-4-((1-(4-(1-cyclopropoxy-2-hydroxy-1-phenylethyl)-6-(6-methyl-7-oxo-1-tosyl-6,7-dihydro-1H-pyrrolo[2,3-c]pyridin-4-yl)quinazolin-2-yl)piperidin-4-yl)methyl)-2,5-dimethylpiperazine-1-carboxylate of Production Example 119-2 (120 mg, 131 μmol) and 4-dimethylaminopyridine (48 mg, 0.39 mmol) in dichloromethane (3 mL) there was added triphosgene (23.3 mg, 79 μmol) while cooling on ice, and after stirring for 10 minutes, the mixture was further stirred for 30 minutes at room temperature. The reaction mixture was again cooled on ice, and then benzyl methyl(2-methylamino)ethyl)carbamate (131 mg, 589 μmol) was added and the mixture was stirred for 5 minutes, after which it was further stirred for 30 minutes at room temperature. The reaction mixture was purified by NH silica gel column chromatography (*n*-heptane:ethyl acetate = 44:56 to ethyl acetate) to obtain a crude product of the title compound (220 mg).

[Production Example 119-4]

*tert*-Butyl (2R,5S)-4-((1-(4-(11-cyclopropoxy-4,7-dimethyl-3,8-dioxo-1,11-diphenyl-2,9-dioxa-4,7-diazaundecan-11-yl)-6-(6-methyl-7-oxo-6,7-dihydro-1H-pyrrolo[2,3-c]pyridin-4-yl)quinazolin-2-yl)piperidin-4-yl)methyl)-2,5-dimethylpiperazine-1-carboxylate

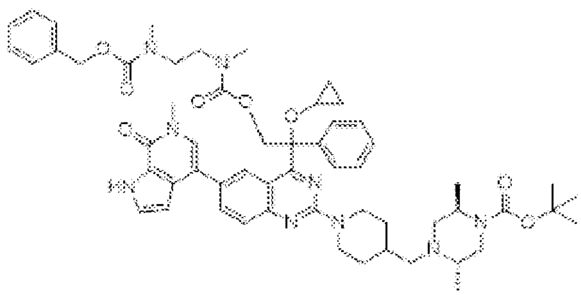

To a solution of a crude product of the *tert-butyl* (2R,5S)-4-((1-(4-(11-cyclopropoxy-4,7-dimethyl-3,8-dioxo-1,11-diphenyl-2,9-dioxa-4,7-diazaundecan-11-yl)-6-(6-methyl-7-oxo-1-tosyl-6,7-dihydro-1H-pyrrolo[2,3-c]pyridin-4-yl)quinazolin-2-yl)piperidin-4-yl)methyl)-2,5-dimethylpiperazine-1-carboxylate of Production Example 119-3 (220 mg) in THF (400 μL) and methanol (800 μL) there was added a 2 M sodium hydroxide aqueous solution (400 μL, 800 μmol) at room temperature, and the mixture was stirred for 3 hours at 60°C. The reaction mixture was returned to room temperature, and extraction was performed with dichloromethane. The organic layer was dried over anhydrous magnesium sulfate and filtered, and the filtrate was concentrated under reduced pressure. The residue was purified by NH silica gel column chromatography (n-heptane:ethyl acetate = 15:85 to ethyl acetate to ethyl acetate:methanol = 95:5 to 9:1) to obtain a crude product of the title compound (106 mg).

ESI-MS (m/z): 1010.74 $[M+H]^+$.

[Production Example 119-5]

Benzyl (2-cyclopropoxy-2-(2-(4-(((2S,5R)-4-(3-(2,4-dioxotetrahydropyrimidine-1(2H)-yl)-4-methoxybenzoyl)-2,5-dimethylpiperazin-1-yl)methyl)piperidin-1-yl)-6-(6-methyl-7-oxo-6,7-dihydro-1H-pyrrolo[2,3-c]pyridin-4-yl)quinazolin-4-yl)-2-phenylethyl)ethane-1,2-diylbis(methylcarbamate)

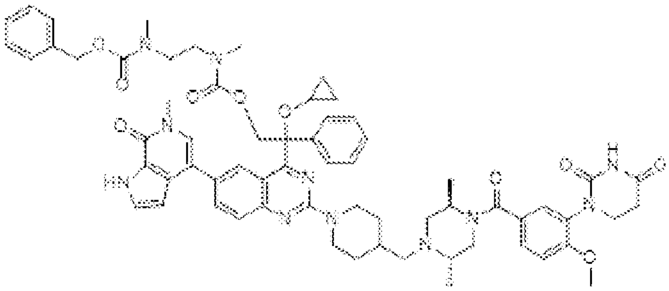

The title compound (57.5 mg) was obtained from a crude product of the *tert-butyl* (2R,5S)-4((1-(4-(11-cyclopropox-4,7-dimethyl-3,8-dioxo-1,11-diphenyl-2,9-dioxa-4,7-diazaundecan-11-yl)-6-(6-methyl-7-oxo-6,7-dihydro-1H-pyrrolo[2,3-c]pyridin-4-yl)quinazolin-2-yl)piperidin-4-yl)methyl)-2,5-dimethylpiperazine-1-carboxylate of Production Example 119-4 (106 mg) and the 3-(2,4-dioxotetrahydropyrimidine-1(2H)-yl-4-methoxybenzoic acid of Production Example 104-7 (41.6 mg, 157 μmol), by the same method as Example 106.
ESI-MS (m/z): 1156.79 [M+H]$^+$.

[Example 120]

2-Cyclopropoxy-2-(2-(4-((3-(2,4-dioxotetrahydropyrimidine-1(2H)-yl)-methoxyphenyl)ethynyl)piperidin-1-yl)-6-(6-methyl-7-oxo-6,7-dihydro-1H-pyrrolo[2,3-c]pyridin-4-yl)quinazolin-4-yl)-2-phenylethyl methyl(2-(methylamino)ethyl) carbamate

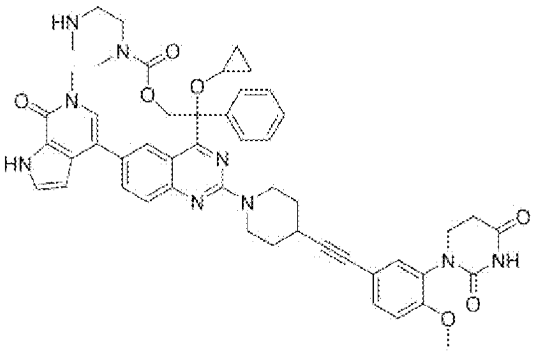

To a solution of the *tert-butyl* (2-cyclopropoxy-2-(2-(4-((3-(2,4-dioxotetrahydropyrimidine-1(2H)-yl)-4-methoxyphenyl)ethynyl)piperidin-1-yl)-6-(6-methyl-7-oxo-6,7-dihydro-1H-pyrrolo[2,3-c]pyridin-4-yl)quinazolin-4-yl)-2-phenylethyl)ethane-1,2-diylbis(methylcarbamate) of Production Example 120-4 (15 mg, 15 μmol) in DCM (1000 μL) there was added TFA (100 μL), and the mixture was stirred for 4 hours and 10 minutes. A saturated sodium hydrogencarbonate solution was added to the reaction mixture, and extraction was performed with DCM. The organic layer was dried over anhydrous sodium sulfate and filtered, and the filtrate was concentrated under reduced pressure. The residue was purified by NH silica gel thin-layer chromatography (ethyl acetate:methanol = 9: 1) to obtain the title compound (8.2 mg).
ESI-MS (m/z): 892.31 [M+H]$^+$.

[Production Example 120-1]

4-(4-(1-Cyclopropoxy-2-hydroxy-1-phenylethyl)-2-(4-ethynylpiperidin-1-yl)quinazolin-6-yl)-6-methyl-1-tosyl-1,6-dihydro-7H-pyrrolo[2,3-c]pyridin-7-one

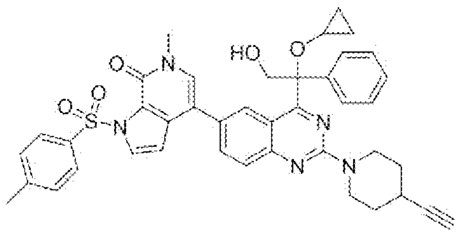

To a solution of the 4-(2-chloro-4-(1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 104-4 (300 mg, 414 μmol) and the known compound 4-ethynylpiperidine hydrochloride (120 mg, 827 μmol) in DMF (5 mL) there was added N,N-diisopropylethylamine (361 μL, 2.07 mmol), and the mixture was stirred for 3 hours and 20 minutes at 80°C. The reaction mixture was returned to room temperature, water was added, and extraction was performed with ethyl acetate. The obtained organic layer was washed with water and brine, dried over anhydrous sodium sulfate and filtered, and the filtrate was concentrated under reduced pressure. To a solution of the residue in DCM (5 mL) there was added TFA (1 mL), and the mixture was stirred for 1 hour. TFA (1 mL) was added to the reaction mixture, and stirring was continued for 30 minutes. The reaction mixture was concentrated under reduced pressure, DCM and a saturated aqueous sodium hydrogencarbonate solution were added, and extraction was performed with DCM. The organic layer was dried over anhydrous sodium sulfate and filtered, the filtrate was concentrated under reduced pressure, and the residue was purified by silica gel column chromatography (*n*-heptane:ethyl acetate = 1: 1) to obtain the title compound (235 mg).
ESI-MS (m/z): 714.22 $[M+H]^+$.

[Production Example 120-2]

*tert*-Butyl (2-cyclopropoxy-2-(2-(4-ethynylpiperidin-1-yl)-6-6-methyl-7-oxy-1-tosyl-6,7-dihydro-1H-pyrrolo[2,3-c]pyridin-4-yl)quinazolin-4-yl)-2-phenylethyl)ethane-1,2-diylbis(methylcarbamate)

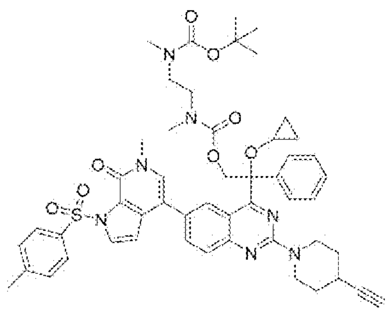

The title compound (105 mg) was obtained from the 4-(4-(1-cyclopropoxy-2-hydroxy-1-phenylethyl)-2-(4-ethynylpiperidin-1-yl)quinazolin-6-yl)-6-methyl-1-tosyl-1,6-dihydro-7H-pyrrolo[2,3-c]pyridin-7-one of Production Example 120-1 (89 mg, 0.13 mmol) and the known compound *tert-butyl* methyl(2-(methylamino)ethyl)carbamate (70.4 mg, 374 μmol), by the same method as Production Example 119-3.
ESI-MS (m/z): 928.26 $[M+H]^+$.

[Production Example 120-3]

*tert*-Butyl (2-cyclopropoxy-2-(2-(4-ethynylpiperidin-1-yl)-6-(6-methyl-7-oxo-6,7-dihydro-1H-pyrrolo[2,3-c]pyridin-4-yl) quinazolin-4-yl)-2-phenylethyl)ethane-1,2-diylbis(methylcarbamate)

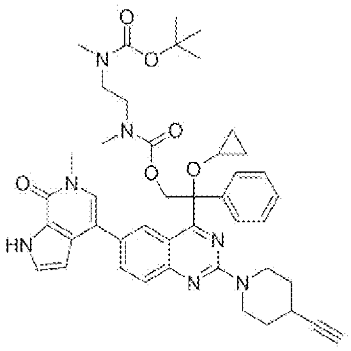

A crude product of the title compound (93.8 mg) was obtained from the *tert*-butyl (2-cyclopropoxy-2-(2-(4-ethynylpiperidin-1-yl)-6-(6-methyl-7-oxy-1-tosyl-6,7-dihydro-1H-pyrrolo[2,3-c]pyridin-4-yl)quinazolin-4-yl-2-phenylethyl)ethane-1,2-diylbis(methylcarbamate) of Production Example 120-2 (105 mg, 113 μmol), by the same method as Production Example 110-2.

ESI-MS (m/z): 774.35 $[M+H]^+$.

[Production Example 120-4]

*tert-Butyl* (2-cyclopropoxy-2-(2-(4-((3-(2,4-dioxotetrahydropyrimidine-1(2H)-yl)-4-methoxyphenyl)ethynyl)piperidin-1-yl)-6-(6-methyl-7-oxo-6,7-dihydro-1H-pyrrolo[2,3-c]pyridin-4-yl)quinazolin-4-yl)-2-phenylethyl)ethane-1,2-diylbis(methylcarbamate)

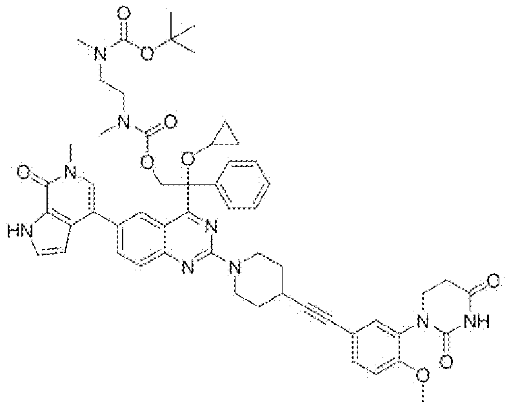

The title compound (89.8 mg) was obtained from a crude product of the *tert*-butyl (2-cyclopropoxy-2-(2-(4-ethynylpiperidin-1-yl)-6-(6-methyl-7-oxo-6,7-dihydro-1H-pyrrolo[2,3-c]pyridin-4-yl)quinazolin-4-yl)-2-phenylethyl) ethane-1,2-diylbis(methylcarbamate) of Production Example 120-3 (93.8 mg) and the 1-(5-iodo-2-methoxyphenyl) dihydropyrimidine-2,4(1H,3H)-dione of Production Example 101-10 (54.5 mg, 158 μmol), by the same method as Example 101.

ESI-MS (m/z): 992.66 $[M+H]^+$.

[Example 121]

2-Cyclopropoxy-2-(2-(4-((3-(2,4-dioxotetrahydropyrimidine-1(2H)-yl)-4-methoxyphenyl)ethynyl)piperidin-1-yl)-6-(6-methyl-7-oxo-6,7-dihydro-1H-pyrrolo[2,3-c]pyridin-4-yl)quinazolin-4-yl)-2-phenylethyl (2-aminoethyl) (methyl)carbamate

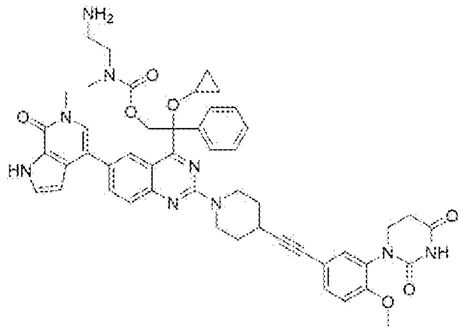

The title compound (7.2 mg) was obtained from the 2-cyclopropoxy-2-(2-(4-((3-(2,4-dioxotetrahydropyrimidine-1(2H)-yl)-4-methoxyphenyl)ethynyl)piperidin-1-yl)-6-(6-methyl-7-oxo-6,7-dihydro-1H-pyrrolo[2,3-c]pyridin-4-yl)quinazolin-4-yl)-2-phenylethyl) (2-((*tert*-butoxycarbonyl)amino)ethyl) (methyl)carbamate of Production Example 121-3 (26.4 mg, 27 μmol), by the same method as Example 120.
ESI-MS (m/z): 878.56 [M+H]+.

[Production Example 121-1]

2-Cyclopropoxy-2-(2-(4-ethynylpiperidin-1-yl)-6-(6-methyl-7-oxo-1-tosyl-6,7-dihydro-1H-pyrrolo[2,3-c]pyridin-4-yl)quinazolin-4-yl)-2-phenylethyl (2-((*tert*-butoxycarbonyl)amino)ethyl) (methyl)carbamate

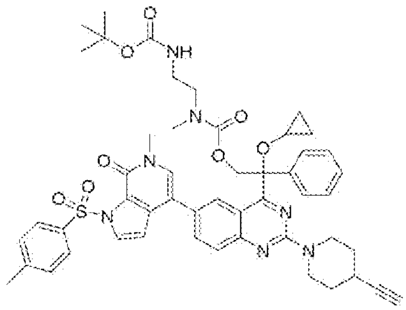

The title compound (34 mg) was obtained from the 4-(4-(1-cyclopropoxy-2-hydroxy-1-phenylethyl)-2-(4-ethynylpiperidin-1-yl)quinazolin-6-yl)-6-methyl-1-tosyl-1,6-dihydro-7H-pyrrolo[2,3-c]pyridin-7-one of Production Example 120-1 (23 mg, 32 μmol) and the known compound *tert-butyl* N-(2-(methylamino)ethyl)carbamate hydrochloride (33.9 mg, 161 μmol), by the same method as Production Example 119-3.
ESI-MS (m/z): 914.49 [M+H]+.

[Production Example 121-2]

2-Cyclopropoxy-2-(2-(4-ethynylpiperidin-1-yl)-6-(6-methyl-7-oxo-6,7-dihydro-1H-pyrrolo[2,3-c]pyridin-4-yl)quinazolin-4-yl)-2-phenylethyl)(2-((*tert*-butoxycarbonyl)amino)ethyl) (methyl)carbamate

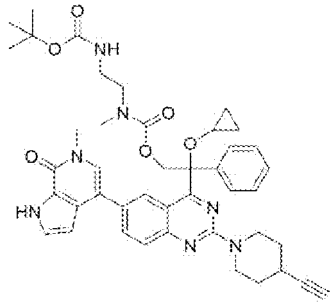

The title compound (24 mg) was obtained from the 2-cyclopropoxy-2-(2-(4-ethynylpiperidin-1-yl)-6-(6-methyl-7-oxo-1-tosyl-6,7-dihydro-1H-pyrrolo[2,3-c]pyridin-4-yl)quinazolin-4-yl)-2-phenylethyl (2-((*tert*-butoxycarbonyl)amino) ethyl) (methyl)carbamate of Production Example 121-1 (34.2 mg), by the same method as Production Example 110-2. ESI-MS (m/z): 758.59 $[M-H]^-$.

[Production Example 121-3]

2-Cyclopropoxy-2-(2-(4-((3-(2,4-dioxotetrahydropyrimidine-1(2H)-yl)-4-methoxyphenyl)ethynyl)piperidin-1-yl)-6-(6-methyl-7-oxo-6,7-dihydro-1H-pyrrolo[2,3-c]pyridin-4-yl)quinazolin-4-yl)-2-phenylethyl) (2-((*tert*-butoxycarbonyl)amino) ethyl) (methyl)carbamate

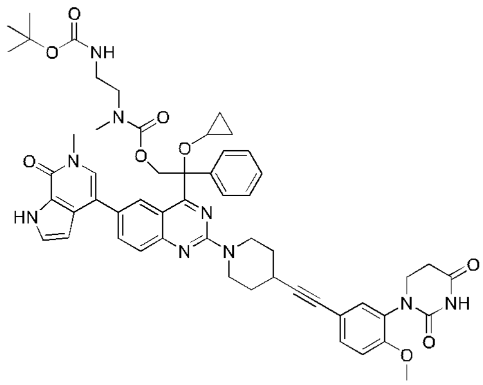

The title compound (26 mg) was obtained from the 2-cyclopropoxy-2-(2-(4-ethynylpiperidin-1-yl)-6-(6-methyl-7-oxo-6,7-dihydro-1H-pyrrolo[2,3-c]pyridin-4-yl)quinazolin-4-yl)-2-phenylethyl) (2-((*tert*-butoxycarbonyl)amino)ethyl) (methyl)carbamate of Production Example 121-2 (22 mg, 29 μmol) and the 1-(5-iodo-2-methoxyphenyl)dihydropyrimidine-2,4(1H,3H)-dione of Production Example 101-10 (13 mg, 38 μmol), by the same method as Example 101.
ESI-MS (m/z): 978.59 $[M+H]^+$.

[Example 122]

4-(2-(4-((1-(4-(1-Cyclopropoxy-2-hydroxy-1-phenylethyl)-6-(1,5-dimethyl-6-oxo-1,6-dihydropyridin-3-yl)quinazolin-2-yl) piperidin-4-yl)methyl)piperazin-1-yl)-2-oxoethoxy)-2-(2,6-dioxopiperidin-3-yl)isoindoline-1,3-dione

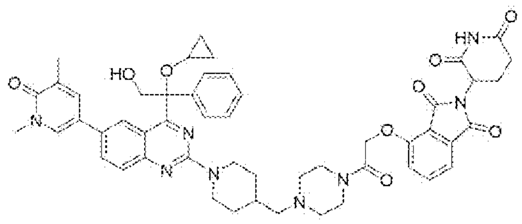

The title compound (9.1 mg) was obtained from the *tert-butyl* 4-((1-(4-(1-cyclopropoxy-1-phenyl-2-((tetrahydro-2H-pyran-2-yl)oxy)ethyl)-6-(1,5-dimethyl-6-oxo-1,6-dihydropyridin-3-yl)quinazolin-2-yl)piperidin-4-yl)methyl)piperazine-1-carboxylate of Production Example 122-2 (12 mg, 15 μmol) and 2-((2-(2,6-dioxopiperidin-3-yl)-1,3-dioxoisoindolin-4-yl)oxy)acetic acid (7.5 mg, 23 μmol), by the same method as Example 123.
ESI-MS (m/z): 923.51 $[M+H]^+$

[Production Example 122-1]

5-(2-Chloro-4-(1-cyclopropoxy-1-phenyl-2-((tetrahydro-2H-pyran-2-yl)oxy)ethyl)quinazolin-6-yl)-1,3-dimethylpyridin-2(1H)-one

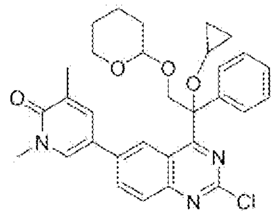

To a solution of the 2-chloro-4-(1-cyclopropoxy-1-phenyl-2-((tetrahydro-2H-pyran-2-yl)oxy)ethyl)-6-iodoquinazoline of Production Example 104-3 (846 mg, 1.54 mmol) and 1,3-dimethyl-5-(4,4,5,5-tetramethyl-1,3,2-dioxaborolan-2-yl)pyridin-2(1H)-one (421 mg, 1.69 mmol) in toluene (5 mL) there were added ethanol (1.25 mL), sodium carbonate (2 M aqueous solution, 2.50 mL, 5.00 mmol) and tetrakis(triphenylphosphine)palladium(0) (177 mg, 154 μmol) at room temperature, and the mixture was stirred for 18 hours at 70°C under a nitrogen atmosphere. The reaction mixture was filtered with Celite and washed with ethyl acetate. The filtrate was concentrated under reduced pressure and the residue was purified by NH silica gel column chromatography (*n*-heptane:ethyl acetate = 1:1 to ethyl acetate) to obtain the title compound (659 mg).
ESI-MS (m/z): 546.38 $[M+H]^+$

[Production Example 122-2]

*tert*-Butyl 4-((1-(4-(1-cyclopropoxy-1-phenyl-2-((tetrahydro-2H-pyran-2-yl)oxy)ethyl)-6-(1,5-dimethyl-6-oxo-1,6-dihydropyridin-3-yl)quinazolin-2-yl)piperidin-4-yl)methyl)piperazine-1-carboxylate

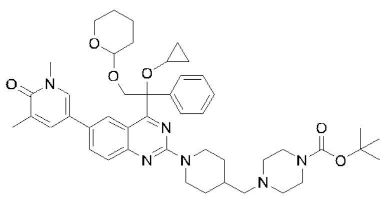

The title compound (299 mg) was obtained from the 5-(2-chloro-4-(1-cyclopropoxy-1-phenyl-2-((tetrahydro-2H-pyran-2-yl)oxy)ethyl)quinazolin-6-yl)-1,3-dimethylpyridin-2(1H)-one of Production Example 122-1 (200 mg, 366 μmol), by the same method as Production Example 123-2.

ESI-MS (m/z): 794.60 $[M+H]^+$.

[Example 123]

3-(4-((2-(4-((1-(4-(1-Cyclopropoxy-2-hydro-1-phenylethyl)-6-(4-methoxy-1-methyl-6-oxo-1,6-dihydropyridin-3-yl)quinazolin-2-yl)piperidin-4-yl)methyl)piperazin-1-yl)-2-oxoethyl)amino)-1-oxoisoindolin-2-yl)piperidine-2,6-dione

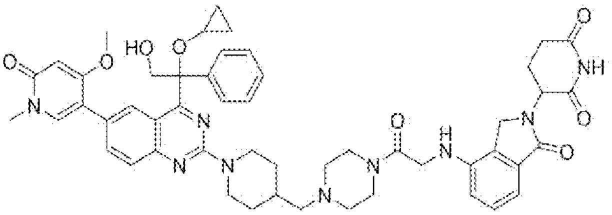

To a solution of the *tert*-butyl 4-((1-(4-(1-cyclopropoxy-1-phenyl-2-((tetrahydro-2H-pyran-2-yl)oxy)ethyl)-6-(4-methoxy-1-methyl-6-oxo-1,6-dihydropyridin-3-yl)quinazolin-2-yl)piperidin-4-yl)methyl)piperazine-1-carboxylate of Production Example 123-2 (28.1 mg, 35 μmol) in DCM (1 mL) there was added TFA (0.5 mL), and the mixture was stirred for 1 hour. The reaction mixture was concentrated under reduced pressure and processed twice by azeotropic distillation with toluene. To a solution of the obtained residue in DMF (1 mL) there were added diisopropylethyl (61 μL, 0.35 mmol), the known compound (2-(2,6-dioxopiperidin-3-yl)-1-oxoisoindolin-4-yl)glycine (16.5 mg, 52 μmol) and HATU (26.4 mg, 69 μmol), and the mixture was stirred for 1 hour and 30 minutes. Water was added to the reaction mixture, and extraction was performed with ethyl acetate. The organic layer was washed with brine, dried over anhydrous sodium sulfate and filtered, and the filtrate was concentrated under reduced pressure. The residue was purified by NH silica gel thin-layer chromatography ((ethyl acetate:methanol = 10: 1), and the obtained solid was washed with a mixed solvent of ethyl acetate and diethyl ether to obtain the title compound (3.9 mg). ESI-MS (m/z): 924.51 $[M+H]^+$.

[Production Example 123-1]

5-(2-Chloro-4-(1-cyclopropoxy-1-phenyl-2-((tetrahydro-2H-pyran-2-yl)oxy)ethyl)quinazolin-6-yl)-4-methoxy-1-methylpyridin-2(1H)-one

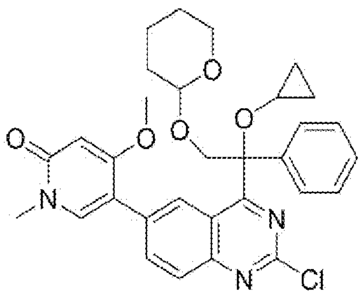

The title compound (99 mg) was obtained from the 2-chloro-4-(1-cyclopropoxy-1-phenyl-2-((tetrahydro-2H-pyran-2-yl)oxy)ethyl-6-iodoquinazoline of Production Example 104-3 (150 mg, 272 μmol) and the known compound 4-methoxy-1-methyl-5-(4,4,5,5-tetramethyl-1,3,2-dioxaborolan-2-yl)pyridin-2(1H)-one (94 mg, 0.35 mmol), by the same method as

Production Example 104-4.

ESI-MS (m/z): 562.26 $[M+H]^+$.

[Production Example 123-2]

*tert*-Butyl 4-((1-(4-(1-cyclopropoxy-1-phenyl-2-((tetrahydro-2H-pyran-2-yl)oxy)ethyl)-6-(4-methoxy-1-methyl-6-oxo-1,6-dihydropyridin-3-yl)quinazolin-2-yl)piperidin-4-yl)methyl)piperazine-1-carboxylate

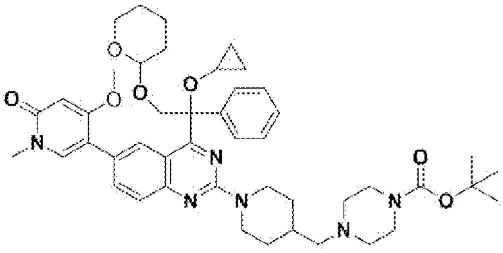

The title compound (28 mg) was obtained from the 5-(2-chloro-4-(1-cyclopropoxy-1-phenyl-2-((tetrahydro-2H-pyran-2-yl)oxy)ethyl)quinazolin-6-yl)-4-methoxy-1-methylpyridin-2(1H)-one of Production Example 123-1 (20 mg, 36 μmol) and *tert*-butyl 4-(piperidin-4-ylmethyl)piperazine-1-carboxylate (12 mg, 43 μmol), by the same method as Production Example 103-3.
ESI-MS (m/z): 809.52 $[M+H]^+$.

[Example 124]

3-(4-(2-(4-((1-(4-(1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)piperidin-4-yl)methyl)piperazin-1-yl)-2-oxoethoxy)-1-oxoisoindolin-2-yl)piperidine-2,6-dione

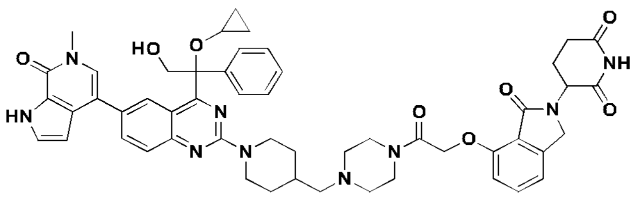

The title compound (18 mg) was obtained from the *tert-butyl* 4-((1-(4-(1-cyclopropoxy-1-phenyl-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)piperidin-4-yl)methyl)piperazine-1-carboxylate of Production Example 124-2 (31 mg, 38 μmol) and the known compound 2-((2-(2,6-dioxopiperidin-3-yl)-1-oxoisoindolin-4-yl)oxy acetic acid (9.1 mg, 28 μmol), by the same method as Example 123.
ESI-MS (m/z): 934.58 $[M+H]^+$.

[Production Example 124-1]

*tert*-Butyl 4-((1-(4-(1-cyclopropoxy-1-phenyl-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)piperidin-4-yl)methyl)piperazine-1-carboxylate

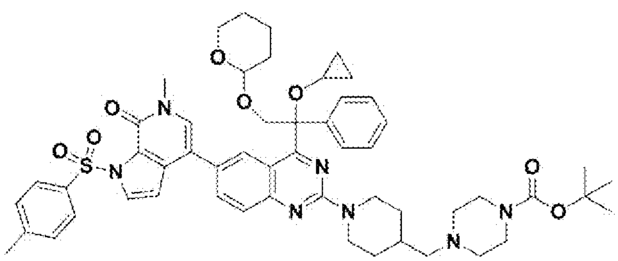

To a solution of the 4-(2-chloro-4-(1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 104-4 (72 mg, 99 μmol) and *tert-butyl* 4-(piperidin-4-ylmethyl)piperazine-1-carboxylate (28 mg, 99 μmol) in NMP (500 μL) there was added N,N-diisopropylethylamine (52.0 μL, 298 μmol), and the mixture was stirred for 3 hours at 80°C. The reaction mixture was returned to room temperature and then purified by NH silica gel column chromatography (*n*-heptane to *n*-heptane:ethyl acetate = 1:1 to ethyl acetate) to obtain the title compound (82 mg).

ESI-MS (m/z): 972.68 [M+H]+.

[Production Example 124-2]

*tert*-Butyl 4-((1-(4-(1-cyclopropoxy-1-phenyl-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)piperidin-4-yl)methyl)piperazine-1-carboxylate

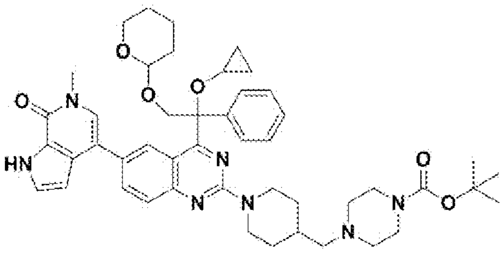

The title compound (62 mg) was obtained from the *tert-butyl* 4-((1-(4-(1-cyclopropoxy-1-phenyl-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)piperidin-4-yl)methyl)piperazine-1-carboxylate of Production Example 124-1 (82 mg, 84 μmol), by the same method as Production Example 104-6.

ESI-MS (m/z): 818.63 [M+H]+.

[Example 125]

3-(2,4-Dioxotetrahydropyrimidine-1(2H)-yl)-N-(1'-(4-(1-hydroxy-4-methyl-2-(thiophen-2-yl)pentan-2-yl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)-4-methoxybenzamide

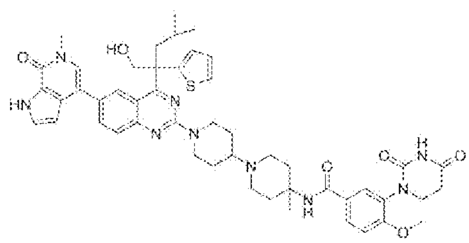

A solution of the *tert-butyl* (4-methyl-1'-(4-(4-methyl-1-((tetrahydro-2H-pyran-2-yl)oxy)-2-(thiophen-2-yl)pentan-2-yl)-6-(6-methyl-7-oxo-6,7-dihydro-1H-pyrrolo[2,3-c]pyridin-4-yl)quinazolin-2-yl)-[1,4'-bipiperidin]-4-yl)carbamate of Production Example 125-5 (64 mg, 76 μmol) in dichloromethane (1 mL) and TFA (1 mL) was stirred for 2 hours at room temperature. The reaction mixture was concentrated under reduced pressure to obtain a crude product (50 mg). To a solution of a portion of the obtained crude product (24 mg) in DMF (1 mL) there were added the 3-(2,4-dioxotetrahydropyrimidine-1(2H)-yl)-4-methoxybenzoic acid of Production Example 104-7 (11 mg, 40 μmol), HATU (17 mg, 44 μmol) and N,N-diisopropylethylamine (128 μL, 734 μmol) at room temperature, and the mixture was stirred for 50 minutes at room temperature. Water was added to the reaction mixture at room temperature, and extraction was performed with ethyl acetate. The organic layer was washed with water and brine, dried over anhydrous magnesium sulfate and filtered, and the filtrate was concentrated under reduced pressure. The residue was purified by NH silica gel thin-layer chromatography (ethyl acetate:methanol = 10:1) to obtain the title compound (9 mg).

ESI-MS (m/z): 900.62 [M+H]+.

[Production Example 125-1]

Ethyl 2-(2-chloro-6-iodoquinazolin-4-yl)-4-methyl-2-(thiophen-2-yl) pentanoate

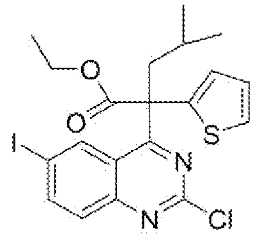

To a solution of ethyl 2-thiopheneacetate (1.50 g, 8.81 mmol) and 1-bromo-2-methylpropane (1.05 mL, 9.69 mmol) in DMF (20 mL) there was added 50% to 72% sodium hydride oil (465 mg) at 0°C under a nitrogen atmosphere, and the mixture was stirred for 1 hour and 50 minutes at room temperature. Water was added to the reaction mixture, and extraction was performed with ethyl acetate. The organic layer was washed with water and brine and filtered, and the filtrate was then concentrated under reduced pressure. The residue was purified by NH silica gel column chromatography (n-heptane:ethyl acetate = 95:5 to 7:3) to obtain a crude product (1.28 g). The title compound (1.64 g) was then obtained from the obtained crude product (1.28 g) by the same method as Production Example 101-2. ESI-MS (m/z): 515.16 $[M+H]^+$.

[Production Example 125-2]

2-Chloro-6-iodo-4-(4-methyl-1-((tetrahydro-2H-pyran-2-yl)oxy)-2-(thiophen-2-yl)pentan-2-yl)quinazoline

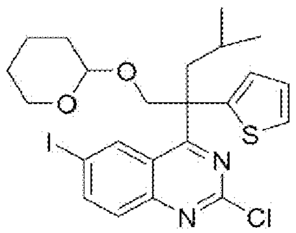

The title compound (1.91 g) was obtained from the ethyl 2-(2-chloro-6-iodoquinazolin-4-yl)-4-methyl-2-(thiophen-2-yl) pentanoate of Production Example 125-1 (1.64 g, 3.19 mmol), by the same method as Production Example 104-3.

ESI-MS (m/z): 557.23 $[M+H]^+$.

[Production Example 125-3]

4-(2-Chloro-4-(4-methyl-1-((tetrahydro-2H-pyran-2-yl)oxy)-2-(thiophen-2-yl)pentan-2-yl)quinazolin-6-yl)-6-methyl-1-tosyl-1,6-dihydro-7H-pyrrolo[2,3-c]pyridin-7-one

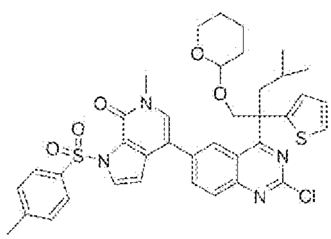

The title compound (178 mg) was obtained from the 2-chloro-6-iodo-4-(4-methyl-1-((tetrahydro-2H-pyran-2-yl) oxy)-2-(thiophen-2-yl)pentan-2-yl)quinazoline of Production Example 125-2 (343 mg, 616 μmol), by the same method as Production Example 104-4. ESI-MS (m/z): 731.37 $[M+H]^+$.

[Production Example 125-4]

*tert-Butyl* (4-methyl-1'-(4-(4-methyl-1-((tetrahydro-2H-pyran-2-yl)oxy)-2-(thiophen-2-yl)pentan-2-yl)-6-(6-methyl-7-oxo-1-tosyl-6,7-dihydro-1H-pyrrolo[2,3-c]pyridin-4-yl)quinazolin-2-yl)-[1,4'-bipiperidin]-4-yl)carbamate

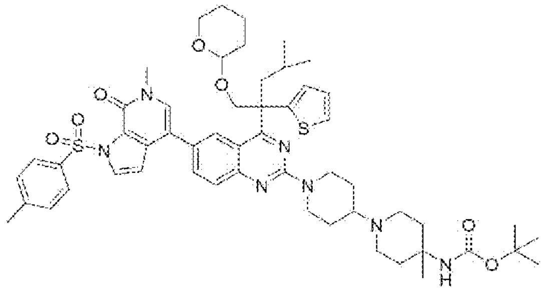

The title compound (84 mg) was obtained from the 4-(2-chloro-4-(4-methyl-1-((tetrahydro-2H-pyran-2-yl) oxy)-2-(thiophen-2-yl)pentan-2-yl)quinazolin-6-yl)-6-methyl-1-tosyl-1,6-dihydro-7H-pyrrolo[2,3-c]pyridin-7-one of Production Example 125-3 (80 mg, 0.11 mmol) and the *tert-butyl* (4-methyl-[1,4'-bipiperidin]-4-yl)carbamate of Production Example 104-2 (36 mg, 0.12 mmol), by the same method as Production Example 104-5.
ESI-MS (m/z): 992.77 $[M+H]^+$.

[Production Example 125-5]

*tert-Butyl* (4-methyl-1'-(4-(4-methyl-1-((tetrahydro-2H-pyran-2-yl)oxy)-2-(thiophen-2-yl)pentan-2-yl)-6-(6-methyl-7-oxo-6,7-dihydro-1H-pyrrolo[2,3-c]pyridin-4-yl)quinazolin-2-yl)-[1,4'-bipiperidin]-4-yl)carbamate

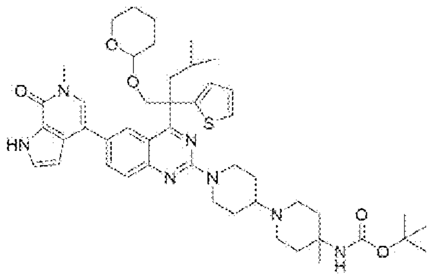

The title compound (64 mg) was obtained from the *tert-butyl* (4-methyl-1'-(4-(4-methyl-1-((tetrahydro-2H-pyran-2-yl)oxy)-2-(thiophen-2-yl)pentan-2-yl)-6-(6-methyl-7-oxo-1-tosyl-6,7-dihydro-1H-pyrrolo[2,3-c]pyridin-4-yl)quinazolin-2-yl)-[1,4'-bipiperidin]-4-yl)carbamate of Production Example 125-4 (84 mg, 85 μmol), by the same method as Production Example 104-6.
ESI-MS (m/z): 838.64 $[M+H]^+$.

[Example 126]

1-(5-(4-((1-(4-(4-(Hydroxymethyl)heptan-4-yl)-6-(4-methoxy-1-methyl-6-oxo-1,6-dihydropyridin-3-yl)quinazolin-2-yl)piperidin-4-yl)oxy)piperidine-1-carbonyl)-2-methoxyphenyl)dihydropyrimidine-2,4(1H,3H)-dione

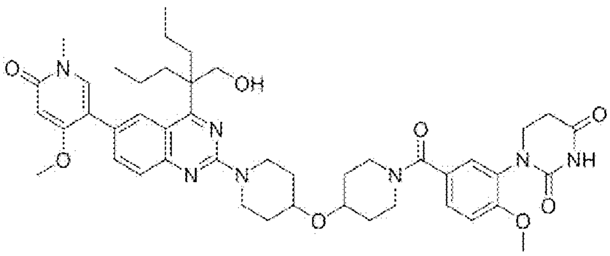

The title compound (5.2 mg) was obtained from the *tert*-butyl 4-((1-(6-(4-methoxy-1-methyl-6-oxo-1,6-dihydropyridin-3-yl)-4-(4-(((tetrahydro-2H-pyran-2-yl)oxy)methyl)heptan-4-yl)quinazolin-2-yl)piperidin-4-yl)oxy)piperidine-1-carboxylate of Production Example 126-4 (20.5 mg, 27 μmol) and the 3-(2,4-dioxotetrahydropyrimidine-1(2H)-yl)-4-methoxybenzoic acid of Production Example 104-7 (9.24 mg, 35 μmol), by the same method as Example 123.
ESI-MS (m/z): 824.37 $[M+H]^+$

[Production Example 126-1]

Ethyl 2-(2-chloro-6-iodoquinazolin-4-yl)-2-propyl pentanoate

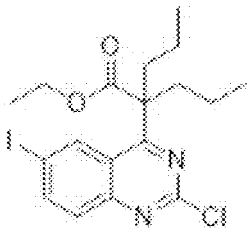

To a solution of ethyl 2-propylpentanoate (2.76 g, 16.0 mmol) in THF (20 mL) there was added dropwise LDA (1.09 M THF solution, 14.68 mL, 16.00 mmol) at -78°C, and the mixture was stirred for 10 minutes at -78°C. A solution of 2,4-dichloro-6-iodoquinazoline (4.00 g, 12.31 mmol) in THF (20 mL) was added to the reaction mixture at -78°C, the temperature was slowly increased, and the mixture was stirred for 2 hours at room temperature. Saturated aqueous ammonium chloride was added to the reaction mixture, and extraction was performed with ethyl acetate. The organic layer was dried over sodium sulfate and filtered. The filtrate was concentrated under reduced pressure. After adding a diethyl ether:n-heptane = 1:1 mixed solvent to the residue, the precipitated solid was filtered out to obtain a portion of the title compound. The filtrate was concentrated under reduced pressure, a diethyl ether:n-heptane = 1:1 mixed solvent was again added and the precipitated solid was filtered out to obtain a portion of the title compound. The filtrate was concentrated under reduced pressure and the residue was purified with silica gel column chromatography (*n*-heptane to *n*-heptane:ethyl acetate = 99:1 to 9:1) to obtain the total title compound (3.4 g).
ESI-MS (m/z): 461.18 $[M+H]^+$.

[Production Example 126-2]

2-Chloro-6-iodo-4-(4-(((tetrahydro-2H-pyran-2-yl)oxy)methyl)heptan-4-yl)quinazoline

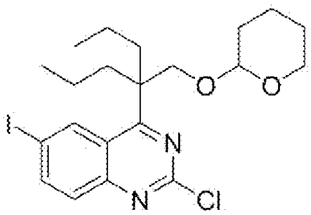

To a solution of the ethyl 2-(2-chloro-6-iodoquinazolin-4-yl)-2-propyl pentanoate of Production Example 126-1

(1.60 g, 3.47 mmol) in toluene (150 mL) there was added dropwise DIBAL-H (1 M toluene solution, 8.68 mL, 8.68 mmol) at -78°C, and the mixture was stirred for 2 hours at 0°C. A saturated aqueous Rochelle salt solution and ethyl acetate were added, and stirring was continued. After oil-water distribution, the organic layer was dried over sodium sulfate and filtered. The filtrate was concentrated under reduced pressure. The residue was purified by silica gel column chromatography (*n*-heptane:ethyl acetate = 99:1 to 7:3) to obtain a crude product. To a solution of the obtained crude product in dichloromethane (5 mL) there were added DHP (380 mg, 4.51 mmol) and*p*-toluenesulfonic acid monohydrate (132 mg, 695 μmol) at 0°C, and the mixture was stirred for 4 hours at room temperature. A saturated aqueous sodium hydrogencarbonate solution was added to the reaction mixture, and extraction was performed with dichloromethane. The organic layer was dried over anhydrous sodium sulfate and filtered, and the filtrate was concentrated under reduced pressure. The residue was purified by silica gel column chromatography (n-heptane:ethyl acetate = 99:1 to 7:3) to obtain the title compound (430 mg).
ESI-MS (m/z): 503.22 $[M+H]^+$

[Production Example 126-3]

5-(2-Chloro-4-(4-(((tetrahydro-2H-pyran-2-yl)oxy)methyl)heptan-4-yl)quinazolin-6-yl)-4-methoxy-1-methylpyridin-2(1H)-one

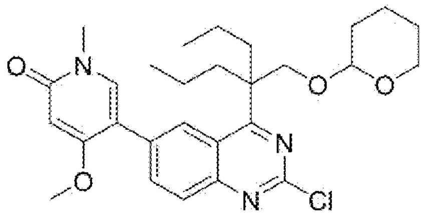

The title compound (82 mg) was obtained from the 2-chloro-6-iodo-4-(4-(((tetrahydro-2H-pyran-2-yl)oxy)methyl) heptan-4-yl)quinazoline of Production Example 126-2 (90 mg, 0.18 mmol) and 4-methoxy-1-methyl-5-(4,4,5,5-tetramethyl-1,3,2-dioxaborolan-2-yl)pyridin-2(1H)-one (64.1 mg, 242 μmol), by the same method as Production Example 104-4. ESI-MS (m/z): 514.44 $[M+H]^+$.

[Production Example 126-4]

*tert*-Butyl 4-((1-(6-(4-methoxy-1-methyl-6-oxo-1,6-dihydropyridin-3-yl)-4-(4-(((tetrahydro-2H-pyran-2-yl)oxy)methyl) heptan-4-yl)quinazolin-2-yl)piperidin-4-yl)oxy)piperidine-1-carboxylate

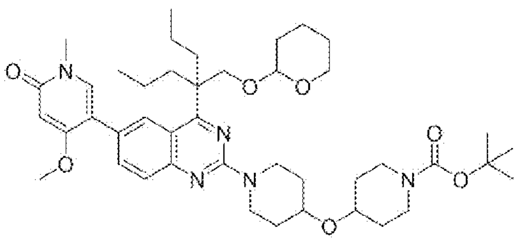

A crude product of the title compound (21 mg) was obtained from the 5-(2-chloro-4-(4-(((tetrahydro-2H-pyran-2-yl)oxy)methyl)heptan-4-yl)quinazolin-6-yl)-4-methoxy-1-methylpyridin-2(1H)-one of Production Example 126-3 (25 mg, 49 μmol) and *tert*-butyl 4-(piperidin-4-yloxy)piperidine-1-carboxylate (17 mg, 58 μmol), by the same method as Production Example 103-3.
ESI-MS (m/z): 762.38 $[M+H]^+$.

[Example 127]

1-(5-((2R,5S)-4-(1-(4-(1-Cyclopropoxy-2-hydroxy-1-(3-hydroxyphenyl)ethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carbonyl)-2-methylphenyl)dihydropyrimidine-2,4-(1H,3H)-dione

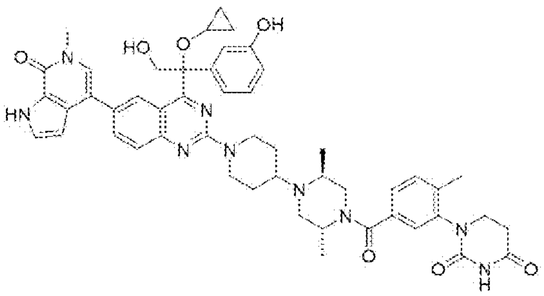

The title compound (13 mg) was obtained from *tert*-butyl (2R,5S)-4-(1-(4-(1-cyclopropoxy-1-(3-hydroxyphenyl)-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate (25 mg, 29 μmol) and the 3-(2,4-dioxotetrahydropyrimidine-1(2H)-yl)-4-methylbenzoic acid of Production Example 105-6 (9.5 mg, 38 μmol), by the same method as Example 104.
ESI-MS (m/z): 892.51 $[M-H]^+$.

[Production Example 127-1]

Methyl 2-(3-benzyloxy)phenyl)-2-diazoacetate

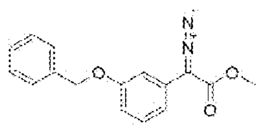

The title compound (4.58 g) was obtained from methyl 2-(3-benzyloxy)phenyl)acetate (5.00 g, 19.5 mmol), by the same method as Production Example 106-1.
$^1$H NMR (500 MHz, $CDCl_3$): δ= 3.87 (3H, s), 5.09 (2H, s), 6.77-6.86 (1H, m), 7.02 (1H, dd, J=8.0, 1.8 Hz), 7.25 (1H, t, J=2.1 Hz), 7.30 (1H, t, J=8.3 Hz), 7.33-7.37 (1H, m), 7.38-7.43 (2H, m), 7.44-7.49 (2H, m).

[Production Example 127-2]

Methyl 2-(3-benzyloxy)phenyl)-2-cyclopropoxyacetate

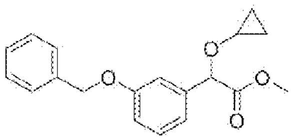

The title compound (3.75 g) was obtained from methyl 2-(3-benzyloxy)phenyl)-2-diazoacetate of Production Example 127-1 (4.51 g, 16.0 mmol), by the same method as Production Example 101-1.
$^1$H NMR (400 MHz, $CDCl_3$): δ= 0.43-0.57 (2H, m), 0.61-0.79 (2H, m), 3.42 (1H, tt, J=6.1, 3.0 Hz), 3.65-3.80 (3H, m), 4.95 (1H, s), 5.03-5.17 (2H, m), 6.96 (1H, dt, J=8.2, 1.4 Hz), 7.05 (1H, d, J=7.8 Hz), 7.08-7.13 (1H, m), 7.26-7.29 (1H, m), 7.29-7.36 (1H, m), 7.36-7.42 (2H, m), 7.42-7.51 (2H, m).

[Production Example 127-3]

Methyl 2-(3-(benzyloxy)phenyl)-2-(2-chloro-6-iodoquinazolin-4-yl)-2-cyclopropoxyacetate

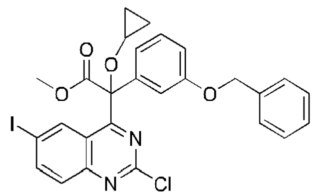

The title compound (7.3 g) was obtained from the methyl 2-(3-benzyloxy)phenyl)-2-cyclopropoxyacetate of Production Example 127-2 (3.75 g, 12.0 mmol), by the same method as Production Example 101-2.

[Production Example 127-4]

4-(1-(3-(Benzyloxy)phenyl)-1-cyclopropoxy-2-((tetrahydro-2H-pyran-2-yl)oxy)ethyl)-2-chloro-6-iodoquinazoline

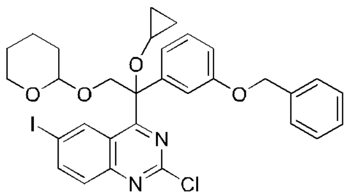

The title compound (5.98 g) was obtained from the methyl 2-(3-(benzyloxy)phenyl)-2-(2-chloro-6-iodoquinazolin-4-yl)-2-cyclopropoxyacetate of Production Example 127-3 (7.21 g, 12.0 mmol), by the same method as Production Example 104-3.

ESI-MS (m/z): 657.23 $[M+H]^+$.

[Production Example 127-5]

4-(4-(1-(3-(Benzyloxy)phenyl)-1-cyclopropoxy-2-((tetrahydro-2H-pyran-2-yl)oxy)ethyl)-2-chloroquinazolin-6-yl)-6-methyl-1-tosyl-1,6-dihydro-7H-pyrrolo[2,3-c]pyridin-7-one

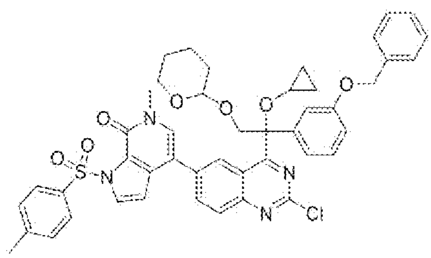

The title compound (2.05 g) was obtained from the 4-(1-(3-(benzyloxy)phenyl)-1-cyclopropoxy-2-((tetrahydro-2H-pyran-2-yl)oxy)ethyl)-2-chloro-6-iodoquinazoline of Production Example 127-4 (2.00 g, 3.04 mmol), by the same method as Production Example 101-5.

ESI-MS (m/z): 831.36 $[M+H]^+$.

[Production Example 127-6]

*tert*-Butyl (2R,5S)-4-(1-(4-((1R)-1-(3-(benzyloxy)phenyl)-1-cyclopropoxy-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-carboxylate

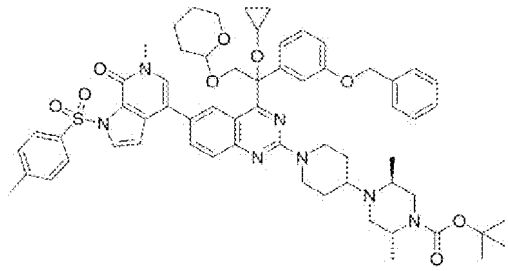

The title compound (1.49 g) was obtained from the 4-(4-(1-(3-(benzyloxy)phenyl)-1-cyclopropoxy-2-((tetrahydro-2H-pyran-2-yl)oxy)ethyl)-2-chloroquinazolin-6-yl)-6-methyl-1-tosyl-1,6-dihydro-7H-pyrrolo[2,3-c]pyridin-7-one of Production Example 127-5 (1.30 g, 1.56 mmol) and the *tert-butyl* (2R,SS)-2,5-dimethyl-4-(piperidin-4-yl)piperazine-1-carboxylate of Production Example 114-2 (651 mg, 2.19 mmol), by the same method as Production Example 103-3.
ESI-MS (m/z): 1093.45 $[M+H]^+$.

[Production Example 127-7]

*tert*-Butyl (2R,5S)-4-(1-(4-((1R)-1-(3-(benzyloxy)phenyl)-1-cyclopropoxy-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate

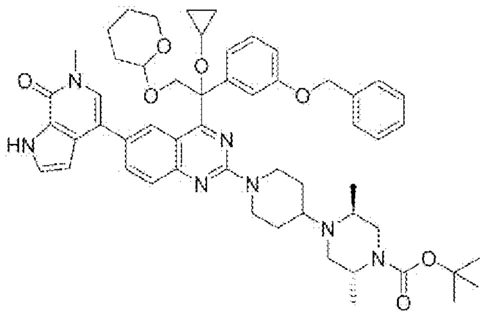

A crude product of the title compound (1.06 g) was obtained from the *tert*-butyl (2R,5S)-4-(1-(4-((1R)-1-(3-(benzyloxy)phenyl)-1-cyclopropoxy-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-carboxylate of Production Example 127-6 (1.19 g, 1.09 mmol), by the same method as Production Example 101-9. ESI-MS (m/z): 939.66 $[M+H]^+$.

[Production Example 127-8]

*tert*-Butyl (2R,5S)-4-(1(4-(1-cyclopropoxy-1-(3-hydroxyphenyl)-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate

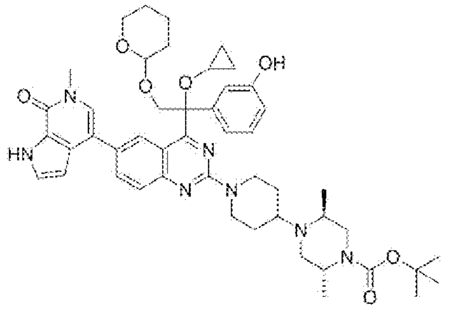

To a solution of the *tert-butyl* (2R,5S)-4-(1-(4-((1R)-1-(3-(benzyloxy)phenyl)-1-cyclopropoxy-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate of Production Example 127-7 (300 mg, 0.32 mmol) in THF (2 mL) and ethyl acetate (2 mL) there was added 10% palladium-carbon (50% water, 272 mg) at room temperature, and the mixture was stirred for 13 hours at room temperature, ordinary pressure under a hydrogen atmosphere. After switching the reaction mixture to a nitrogen atmosphere, it was filtered with Celite and washed with a mixed solvent of ethyl acetate and methanol (6: 1). The filtrate was concentrated under reduced pressure to obtain the title compound (235 mg).

ESI-MS (m/z): 848.61 $[M+H]^+$.

[Example 128]

N-(1'-(4-(1-Cyclopropoxy-2-hydroxy-1-(naphthalen-1-yl)ethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)-3-(2,4-dioxotetrahydropyrimidine-1(2H)-yl)-4-methoxybenzamide

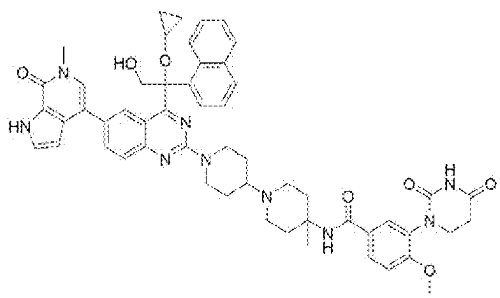

The title compound (7.6 mg) was obtained from the *tert-butyl* (1'-(4-(1-cyclopropoxy-1-(naphthalen-1-yl)-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)carbamate of Production Example 128-6 (8.6 mg, 9.7 μmol), by the same method as Example 104.

ESI-MS (m/z): 944.29 $[M+H]^+$.

[Production Example 128-1]

Methyl 2-cyclopropoxy-2-(naphthalen-1-yl)acetate

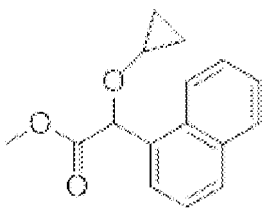

To a solution of 1-naphthylacetic acid (1.50 g, 8.06 mmol) in methanol (20 mL) there was added (diazomethyl) trimethylsilane (8.06 mL, 16.1 mmol) at 0°C, and the mixture was stirred for 1 hour at room temperature. Acetic acid (3 mL) was added to the reaction mixture, which was then concentrated under reduced pressure. The residue was purified by silica gel column chromatography (n-heptane:ethyl acetate = 1:0 to 1:1) to obtain a reaction intermediate. To a solution of the obtained reaction intermediate and 4-acetamidobenzenesulfonyl azide (2.32 g, 9.67 mmol) in acetonitrile (15 mL) there was added DBU (1.80 mL, 12.1 mmol) at 0°C, and the mixture was stirred for 3 hours at room temperature. Saturated aqueous ammonium chloride was added to the reaction mixture, and extraction was performed with ethyl acetate. The organic layer was washed with brine, and then the organic layer was dried over anhydrous magnesium sulfate and filtered, and the filtrate was concentrated under reduced pressure. The residue was purified by silica gel column chromatography (n-heptane:ethyl acetate = 1:0 to 1:1) to obtain a reaction intermediate. To a solution of the obtained reaction intermediate and cyclopropanol (936 μL, 16.1 mmol) in dichloromethane (15 mL) there was added diacetoxyrhodium (89.0 mg, 0.201 mmol) at room temperature, and the mixture was stirred overnight at room temperature. The reaction mixture was purified by silica gel column chromatography (n-heptane:ethyl acetate = 1:0 to 7:3) to obtain the title compound (1.37 g).

1H-NMR Spectrum (400 MHz, CDCl3) δ(ppm): 0.42-0.53 (2H, m), 0.67-0.79 (2H, m), 3.39-3.51 (1H, m), 3.67 (3H, s), 5.57-5.60 (1H, m), 7.43-7.57 (3H, m), 7.63 (1H, dd, J=7.3, 0.98 Hz), 7.85 (2H, t, J=7.8 Hz), 8.27 (1H, d, J=7.8 Hz).

[Production Example 128-2]

Methyl 2-(2-chloro-6-iodoquinazolin-4-yl)-2-cyclopropoxy-2-(naphthalen-1-yl)acetate

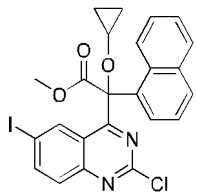

The title compound (1.43 g) was obtained from the methyl 2-cyclopropoxy-2-(naphthalen-1-yl)acetate of Production Example 128-1 (1.40 g, 5.46 mmol), by the same method as Production Example 126-1.

ESI-MS (m/z): 545.15 [M+H]$^+$.

[Production Example 128-3]

2-Chloro-4-(1-cyclopropoxy-1-(naphthalen-1-yl)-2-((tetrahydro-2H-pyran-2-yl)oxy)ethyl)-6-iodoquinazoline

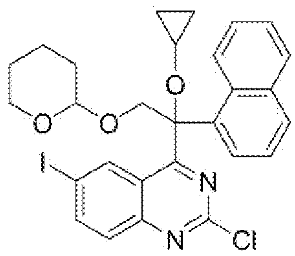

The title compound (6.3 mg) was obtained from the methyl 2-(2-chloro-6-iodoquinazolin-4-yl)-2-cyclopropoxy-2-(naphthalen-1-yl)acetate of Production Example 128-2 (50 mg, 0.092 mmol), by the same method as Production Example 126-2.

ESI-MS (m/z): 601.22 [M+H]$^+$.

[Production Example 128-4]

4-(2-Chloro-4-(-1-cyclopropoxy-1-(naphthalen-1-yl)-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one

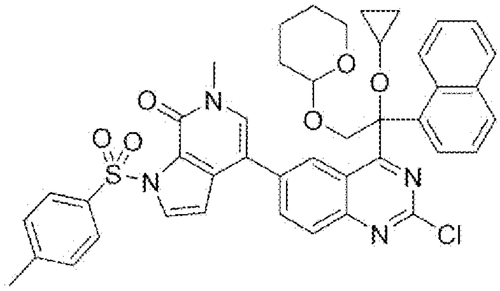

The title compound (73.5 mg) was obtained from the 2-chloro-4-(-1-cyclopropoxy-1-(naphthalen-1-yl)-2-((tetrahydro-2H-pyran-2-yl)oxy)ethyl)-6-iodoquinazoline of Production Example 128-3 (86.0 mg, 143 μmol), by the same method as Production Example 101-5.
ESI-MS (m/z): 775.15 $[M+H]^+$.

[Production Example 128-5]

*tert*-Butyl (1'-(4-(1-cyclopropoxy-1-(naphthalen-1-yl)-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)-4-methyl-[1,4'-bipiperidin]-4-yl)carbamate

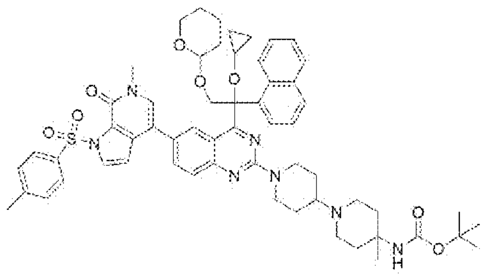

The title compound (17.8 mg) was obtained from the 4-(2-chloro-4-(1-cyclopropoxy-1-(naphthalen-1-yl)-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 128-4 (27 mg, 35 μmol), by the same method as Production Example 104-5.
ESI-MS (m/z): 1037.42 $[M+H]^+$.

[Production Example 128-6]

*tert*-Butyl (1'-(4-(1-cyclopropoxy-1-(naphthalen-1-yl)-2-((tetrahydro-2H-pyran-2-yl)oxy)ethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)carbamate

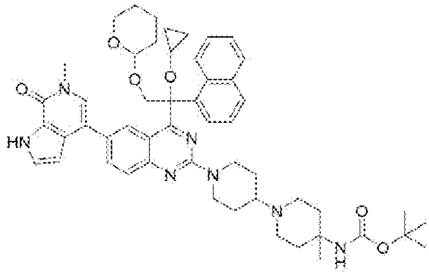

The title compound (8.6 mg) was obtained from the *tert-butyl* (1'-(4-(-1-cyclopropoxy-1-(naphthalen-1-yl)-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)-4-methyl-[1,4'-bipiperidin]-4-yl)carbamate of Production Example 128-5 (18 mg, 17 μmol), by the same method as Production Example 104-6.

ESI-MS (m/z): 882.34 [M+H]$^+$.

[Example 129]

1-(5-(4-(2-((4-(1-Cyclopropoxy-2-hydroxy-1-phenylethyl)-6-(1,5-dimethyl-6-oxo-1,6-dihydropyridin-3-yl)quinazolin-2-yl)oxy)ethyl)piperidine-1-carbonyl)-2-methoxyphenyl)dihydropyrimidine-2,4(1H,3H)-dione

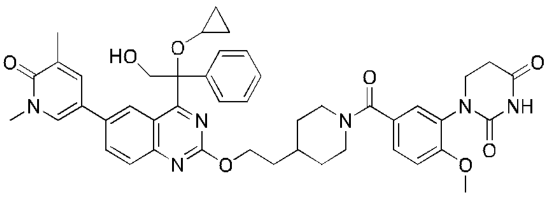

The title compound (3.3 mg) was obtained from the *tert*-butyl 4-(2-((4-(1-cyclopropoxy-1-phenyl-2-((tetrahydro-2H-pyran-2-yl)oxy)ethyl)-6-(1,5-dimethyl-6-oxo-1,6-dihydropyridin-3-yl)quinazolin-2-yl)oxy)ethyl)piperidine- 1-carboxylate of Production Example 129-2 (20 mg, 27 μmol) and the 3-(2,4-dioxotetrahydropyrimidine-1(2H)-yl-4-methoxybenzoic acid of Production Example 104-7 (9.3 mg, 35 μmol), by the same method as Example 123.
ESI-MS (m/z): 801.44 [M+H]$^+$

[Production Example 129-1]

5-(2-Chloro-4-(1-cyclopropoxy-1-phenyl-2-((tetrahydro-2H-pyran-2-yl)oxy)ethyl)quinazolin-6-yl)-1,3-dimethylpyridin-2(1H)-one

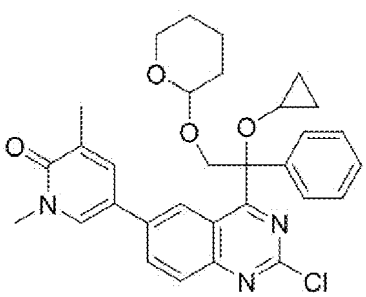

The title compound (659 mg) was obtained from the 2-chloro-4-(1-cyclopropoxy-1-phenyl-2-((tetrahydro-2H-pyran-2-yl)oxy)ethyl)-6-iodoquinazoline of Production Example 104-3 (846 mg, 1.54 mmol) and 1,3-dimethyl-5-(4,4,5,5-tetramethyl-1,3,2-dioxaborolan-2-yl)pyridin-2(1H)-one (421 mg, 1.69 mmol), by the same method as Production Example 104-4.

ESI-MS (m/z): 546.38 [M+H]+

[Production Example 129-2]

*tert*-Butyl 4-(2-((4-(1-cyclopropoxy-1-phenyl-2-((tetrahydro-2H-pyran-2-yl)oxy)ethyl)-6-(1,5-dimethyl-6-oxo-1,6-dihydropyridin-3-yl)quinazolin-2-yl)oxy)ethyl)piperidine-1-carboxylate

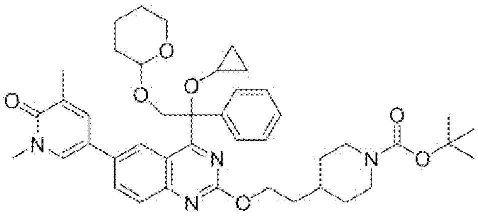

To a solution of N-Boc4-piperidineethanol (44.1 mg, 192 μmol) in DMF (1 mL) there was added 50% to 72% sodium hydride oil (9.23 mg), and the mixture was stirred for 10 minutes. To the reaction mixture there was added the 5-(2-chloro-4-(1-cyclopropoxy-1-phenyl-2-((tetrahydro-2H-pyran-2-yl)oxy)ethyl)quinazolin-6-yl)-1,3-dimethylpyridin-2(1H)-one of Production Example 129-1 (35 mg, 64 μmol), and the mixture was stirred for 1 hour and 30 minutes. Water was added to the reaction mixture, and extraction was performed with ethyl acetate. The organic layer was washed with brine, dried over anhydrous sodium sulfate and filtered, and the filtrate was concentrated under reduced pressure. The residue was purified by NH silica gel column chromatography (n-heptane:ethyl acetate = 1:1 to 3:7) to obtain a crude product of the title compound (59 mg).
ESI-MS (m/z): 739.42 [M+H]+.

[Example 130]

1-(5-(4-((4-((1-(4-(1-Cyclopropoxy-2-hydroxy-1-phenylethyl)-6-(1,5-dimethyl-6-oxo-1,6-dihydropyridin-3-yl)quinazolin-2-yl)piperidin-4-yl)methyl)piperazin-1-yl)methyl)-1H-1,2,3-triazol-1-yl)-2-methoxyphenyl)-dihydropyrimidine-2,4(1H,3H)-dione

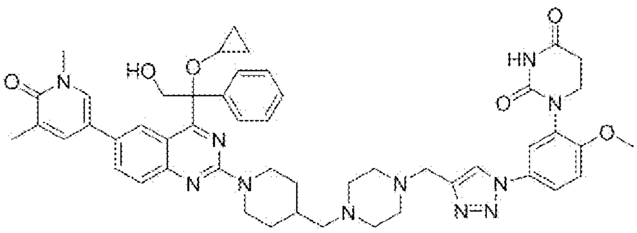

To a solution of the 5-(4-(1-cyclopropoxy-2-hydroxy-1-phenylethyl)-2-(4-((4-(prop-2-yn-1-yl)piperazin-1-yl)methyl)piperidin-1-yl)quinazolin-6-yl)-1,3-dimethylpyridin-2(1H)-one of Production Example 130-3 (10 mg, 15 μmol) and the 1-(5-azide-2-methoxyphenyl)dihydropyrimidine-2,4(1H,3H)-dione of Production Example 130-1 (4.0 mg, 15 μmol) in THF (1.01 mL) there were added N,N-diisopropylethylenediamine (4.0 μL, 23 μmol) and copper(I) iodide (294 μg, 1.55 μmol), and the mixture was stirred for 15 hours at room temperature. The reaction mixture was concentrated under reduced pressure and purified by silica gel thin-layer chromatography (methanol dichloromethane = 1: 10), to obtain the title compound (3.8 mg).
ESI-MS (m/z): 908.22 [M+H]+.

[Production Example 130-1]

1-(5-Azide-2-methoxyphenyl)dihydropyrimidine-2,4(1H,3H)-dione

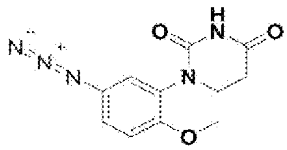

To a solution of the 1-(5-iodo-2-methoxyphenyl)dihydropyrimidine-2,4(1H,3H)-dione of Production Example 101-10 (300 mg, 867 μmol), sodium azide (113 mg, 1.73 mmol) and N,N'-dimethylethylenediamine (14.0 μL, 0.13 mmol) in DMF (3.36 mL) there were added water (1.09 mL), copper(I) iodide (33.0 mg, 173 μmol) and sodium ascorbate (34.3 mg, 173 μmol), and the mixture was stirred overnight at 70°C. The reaction mixture was concentrated under reduced pressure and the residue was purified by ODS silica gel column chromatography (water (containing 0.1% formic acid):acetonitrile (containing 0.1% formic acid) = 97:3 to 50:50), to obtain the title compound (200 mg).

ESI-MS (m/z): 262.13 $[M+H]^+$.

[Production Example 130-2]

*tert*-Butyl 4-((1-(4-(1-cyclopropoxy-1-phenyl-2-((tetrahydro-2H-pyran-2-yl)oxy)ethyl)-6-(1,5-dimethyl-6-oxo-1,6-dihydropyrimidin-3-yl)quinazolin-2-yl)piperidin-4-yl)methyl)piperazine-1-carboxylate

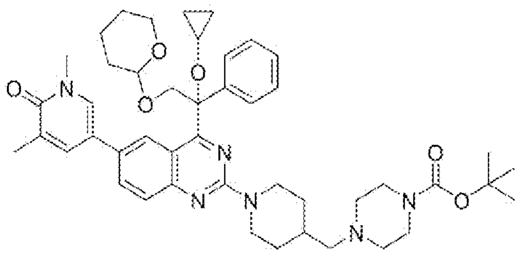

The title compound (36 mg) was obtained from the 5-(2-chloro-4-(1-cyclopropoxy-1-phenyl-2-((tetrahydro-2H-pyran-2-yl)oxy)ethyl)quinazolin-6-yl)-1,3-dimethylpyridin-2(1H)-one of Production Example 129-1 (25 mg, 46 μmol and *tert*-butyl 4-(piperidin-4-ylmethyl)piperazine-1-carboxylate (26 mg, 92 μmol), by the same method as Production Example 103-3.

ESI-MS (m/z): 793.44 $[M+H]^+$.

[Production Example 130-3]

5-(4-(1-Cyclopropoxy-2-hydroxy-1-phenylethyl)-2-(4-((4-(prop-2-yn-1-yl)piperazin-1-yl)methyl)piperidin-1-yl)quinazolin-6-yl)-1,3-dimethyl-pyrimidin-2(1H)-one

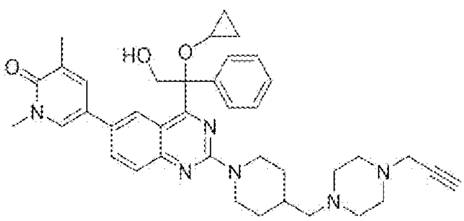

To a solution of the *tert*-butyl 4-((1-(4-(1-cyclopropoxy-1-phenyl-2-((tetrahydro-2H-pyran-2-yl)oxy)ethyl)-6-(1,5-dimethyl-6-oxo-1,6-dihydropyrimidin-3-yl)quinazolin-2-yl)piperidin-4-yl)methyl)piperazine-1-carboxylate of Production Example 130-2 (144 mg, 182 μmol) in dichloromethane (1 mL) there was added TFA (1 mL), and the mixture was stirred for 30 minutes at room temperature. The reaction mixture was concentrated under reduced pressure. To a solution of the residue in acetonitrile (2 mL) there were added N,N-diisopropylethylamine (317 μL, 1.82 mmol) and propargyl bromide (9.2 M toluene solution, 25.7 μL, 236 μmol), and the mixture was stirred for 65 hours at room temperature. Water was added to the reaction mixture, and extraction was performed with ethyl acetate. The organic layer was washed with brine

and dried over anhydrous magnesium sulfate, and then filtered. The filtrate was concentrated under reduced pressure and the residue was purified by NH silica gel column chromatography (n-heptane:ethyl acetate = 1:1 to ethyl acetate to ethyl acetate:methanol = 9:1), to obtain the title compound (71 mg).

ESI-MS (m/z): 647.49 $[M+H]^+$.

[Example 131]

(S)-2-Acetamide-N1-(((S)-2-cyclopropoxy-2-(2-(4-((3-(3-(2,4-dioxotetrahydropyrimidine-1(2H)-yl)-4-methoxyphenyl) prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl) succinamide

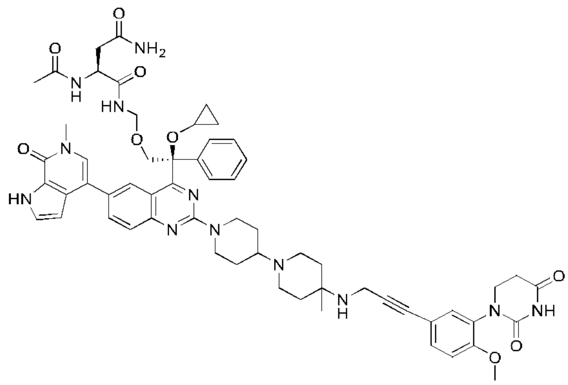

A formate of the title compound (1.5 mg) was obtained from the (S)-2-acetamide-N1-(((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c] pyridin-4-yl)quinazolin-4-yl)-2-phenylethoxy)methyl) succinamide of Production Example 131-5 (1.5 mg, 1.7 μmol) and the 1-(5-iodo-2-methoxyphenyl)dihydropyrimidine-2,4(1H,3H)-dione of Production Example 101-10 (1.2 mg, 3.4 μmol), by the same method as Example 101.

ESI-MS (m/z): 1089.58 $[M+H]^+$.

[Production Example 131-1]

*tert*-Butyl N2-(((9H-fluoren-9-yl)methoxy)carbonyl)-N4-trityl-L-asparaginyl glycinate

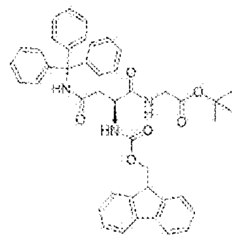

To a solution of FMOC-L-asparagine (TRT)-OH (20 g, 34 mmol) in DMF (50.0 mL) there were added H-glycine-OTBU hydrochloride (5.62 g, 33.5 mmol), HATU (14.0 g, 36.9 mmol) and N,N-diisopropylethylamine (12.9 mL, 73.7 mmol), and after stirring for 3 hours at room temperature, the mixture was stirred overnight at 4°C. Water and dichloromethane were added to the reaction mixture, which was then concentrated under reduced pressure. Water was added to the residue, and the precipitated solid was filtered to obtain the title compound (25 g).

ESI-MS (m/z): 710.33 $[M+H]^+$.

[Production Example 131-2]

(((9H-Fluoren-9-yl)methoxy)carbonyl)-L-asparaginylglycine

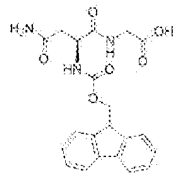

To a solution of the *tert-butyl* N2-(((9H-fluoren-9-yl)methoxy)carbonyl)-N4-trityl-L-asparaginyl glycinate of Production Example 131-1 (25 g, 35 mmol) in dichloromethane (15 mL) there was added TFA (30 mL), and the mixture was stirred for 18 hours at room temperature. The reaction mixture was concentrated under reduced pressure and processed by azeotropic distillation 3 times with ethyl acetate. Ethyl acetate was added to the residue, and the precipitated solid was filtered out and washed with ethyl acetate to obtain the title compound (11.5 g).
ESI-MS (m/z): 412.21 $[M+H]^+$.

[Production Example 131-3]

(S)-(2-((((9H-Fluoren-9-yl)methoxy)carbonyl)amino)-4-amino-4-oxobutaneamide)methyl acetate

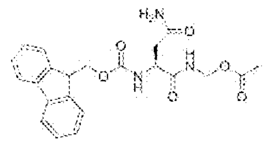

To a solution of the (((9H-fluoren-9-yl)methoxy)carbonyl)-L-asparaginylglycine of Production Example 131-2 (6.95 g, 16.9 mmol) in THF (550 mL), toluene (100 mL) and pyridine (1.64 mL) there was added lead tetraacetate (8.99 g, 20.3 mmol), and the mixture was stirred for 2 hours at 110°C. The reaction mixture was cooled to room temperature, filtered with Celite and washed with an ethyl acetate/THF mixed solution (1:1), and the filtrate was concentrated under reduced pressure. Ethyl acetate (220 mL) was added to the residue, and the precipitated solid was filtered out and washed with ethyl acetate to obtain the title compound (3.29 g).
ESI-MS (m/z): 731.33$[2M-2OAc+H]^+$.

[Production Example 131-4]

(9H-Fluoren-9-yl)methyl((S)-4-amino-1-((((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-1,4-dioxobutan-2-yl)carbamate

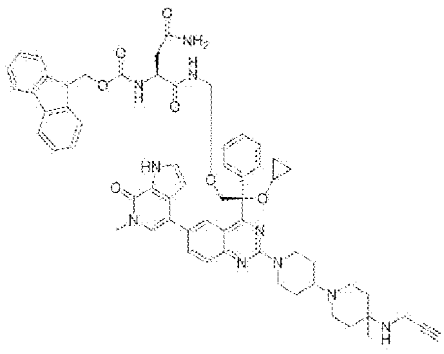

To a solution of the (S)-(2-((((9H-fluoren-9-yl)methoxy)carbonyl)amino)4-amino4-oxobutaneamide)methyl acetate of Production Example 131-3 (112 mg, 262 μmol) in dichloromethane (1.2 mL) there was added chlorotrimethylsilane (41.9 μL, 328 μmol), and the mixture was stirred for 20 minutes at room temperature (flask A). The (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 101-9 (30 mg, 44 μmol) and CSA (61.0 mg, 262 μmol) were processed by azeotropic distillation with a dichloromethane/toluene mixed solvent, and the water was removed (flask B). Dichloromethane (1.2 mL) was added to flask B, the contents were added to flask A, and the mixture was stirred for 50 minutes at room temperature. Sodium bicarbonate water was added to the reaction mixture which was then diluted with dichloromethane and water, and the insolubles were removed by filtration. The filtrate was extracted with dichloromethane, the organic layer was dried over sodium sulfate and filtered, and the filtrate was concentrated under reduced pressure. The residue was purified by ODS silica gel chromatography (0.1% formic acid aqueous solution:0.1% acetonitrile formate solution = 9:1 to 6:4) to obtain a formate of the title compound (17.8 mg).

ESI-MS (m/z): 1051.71 $[M+H]^+$.

[Production Example 131-5]

(S)-2-Acetamide-N1-(((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl) succinamide

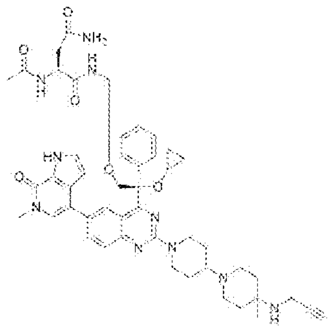

To a solution of the formate of (9H-fluoren-9-yl)methyl ((S)-4-amino-1-((((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-1,4-dioxobutan-2-yl)carbamate of Production Example 131-4 (14.2 mg) in DMF (260 μL) there was added diethylamine (26.0 μL, 248 μmol), and the mixture was stirred for 30 minutes at room temperature. The reaction mixture was concentrated under reduced pressure, the residue was dissolved in THF (300 μL) and dichloromethane (300 μL), and then N,N-diisopropylethylamine (4.3 μL, 25 μmol) and acetic anhydride (1.4 μL, 15 μmol) were added and the mixture was stirred for 30 minutes at room temperature. The reaction mixture was concentrated under reduced pressure, and the residue was purified by NH silica gel column chromatography (ethyl acetate:methanol = 1:0 to 7:3) to obtain the title compound (7.5 mg).

ESI-MS (m/z): 871.55 $[M+H]^+$.

[Example 132]

(S)-2-Acetamide-N1-(2-((((S)-2-cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-dipiperidin]-1'-yl)-6-(6-methyl-7-oxo-6,7-dihydro-1H-pyrrolo[2,3-c]pyridin-4-yl) quinazolin-4-yl)-2-phenylethoxy)methyl)amino)-2-oxoethyl) succinamide

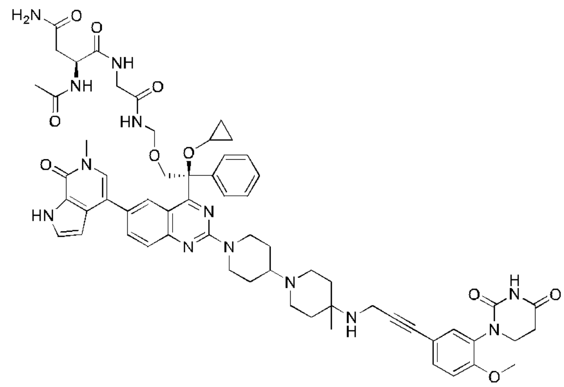

A formate of the title compound (1.5 mg) was obtained from a formate of the (S)-2-acetamide-N1-(2-((((S)-2-cyclopropoxy-2-(2-(4-methyl-4-(prop-2-yn-1-ylamino)-[1,4'-dipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl) succinamide of Production Example 132-3 (6.0 mg), by the same method as Example 101.

ESI-MS (m/z): 1146.81 $[M+H]^+$.

[Production Example 132-1]

(2-((((9H-Fluoren-9-yl)methoxy)carbonyl)amino)acetamide)methyl acetate

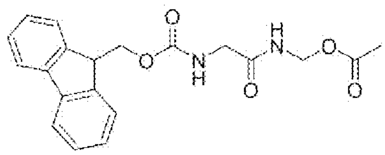

To a solution of (((9H-fluoren-9-yl)methoxy)carbonyl)glycylglycine (9.00 g, 25.4 mmol) in THF (250 mL), toluene (81 mL) and pyridine (2.47 mL) there was added lead tetraacetate (14.6 g, 33.0 mmol), and the mixture was stirred for 1 hour at 100°C. The reaction mixture was cooled to room temperature and filtered with Celite, and the filtrate was concentrated under reduced pressure. Water was added to the residue and extraction was performed with ethyl acetate, after which the organic layer was dried over anhydrous magnesium sulfate and then filtered, and the filtrate was concentrated under reduced pressure. The residue was purified by silica gel column chromatography (n-heptane:ethyl acetate = 9:1 to 0:1) to obtain the title compound (6.68 g).

ESI-MS (m/z): 617.37$[2M-2OAc+H]^+$.

[Production Example 132-2]

(9H-Fluoren-9-yl)methyl(S)-(2-(((2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamate

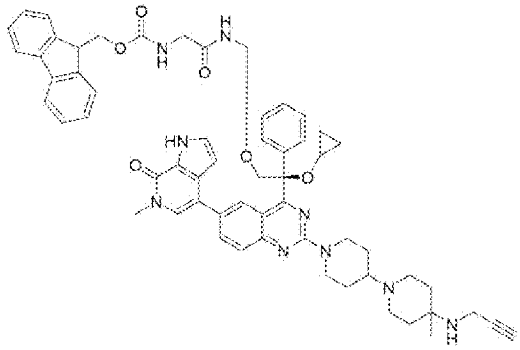

The (2-((((9H-fluoren-9-yl)methoxy)carbonyl)amino)acetamide)methyl acetate of Production Example 132-1 (645 mg, 1.75 mmol) and the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 101-9 (200 mg, 292 μmol) were processed by azeotropic distillation with a mixed solvent of THF (2 mL) and toluene (2 mL). To a mixed solution of the residue in dichloromethane (3.75 mL) and THF (4.78 mL) there was added CSA(339 mg, 1.46 mmol), and the mixture was stirred for 1 hour at room temperature. To the reaction mixture there were added (2-((((9H-fluoren-9-yl)methoxy)carbonyl)amino)acetamide)methyl acetate (645 mg, 1.75 mmol) and CSA(339 mg, 1.46 mmol), and the mixture was further stirred for 1 hour. Triethylamine (406 μL, 2.92 mmol) was added to the reaction mixture to halt the reaction, and the mixture was concentrated under reduced pressure. The residue was purified by ODS silica gel chromatography (0.1% formic acid aqueous solution:0.1% acetonitrile formate solution = 95:5 to 4:6) to obtain a formate of the title compound. The formate of the title compound was dissolved in a chloroform:methanol = 20:1 solution, and neutralized with a saturated aqueous sodium hydrogencarbonate solution. The aqueous layer was extracted with a chloroform:methanol = 20:1 solution, and the collected organic layer was dried over magnesium sulfate and filtered. The filtrate was concentrated under reduced pressure to obtain the title compound (105 mg).

ESI-MS (m/z): 995.01 $[M+H]^+$.

[Production Example 132-3]

(S)-2-Acetamide-N1-(2-((((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)succinamide

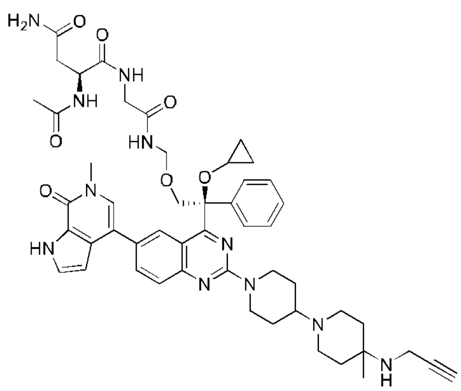

To a solution of the (9H-fluoren-9-yl)methyl (S)-(2-(((2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamate of Production Example 132-2 (40 mg, 40 μmol) in DMF (2 mL) there was added diethylamine (286 μL, 2.74 mmol) at room temperature, and the mixture was stirred for 20 minutes at room temperature. The reaction mixture was concentrated under reduced pressure to obtain crude product 1. Separately, TFA (0.5 mL) was added to a solution of *tert-butyl* acetyl-L-asparaginate in dichloromethane (1 mL), and the mixture was stirred for 1 hour at room temperature. The reaction mixture was concentrated under reduced pressure to obtain crude product 2. To a solution of crude product 1 and crude product 2 in DMF (2 mL) there were added HATU (19.9 mg, 52 μmol) and N,N-

diisopropylethylamine (143 μL, 818 μmol), and the mixture was stirred for 1 hour at room temperature. The residue was purified by ODS silica gel chromatography (0.1% formic acid aqueous solution:0.1% acetonitrile formate solution = 95:5 to 0:100) to obtain a formate of the title compound (22 mg).

ESI-MS (m/z): 928.74 $[M+H]^+$.

[Example 133]

(S)-N1-(2-((((S)-2-Cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)-2-(2,5,8,11,14,17,20,23-octaoxahexacosan-26-amide) succinamide

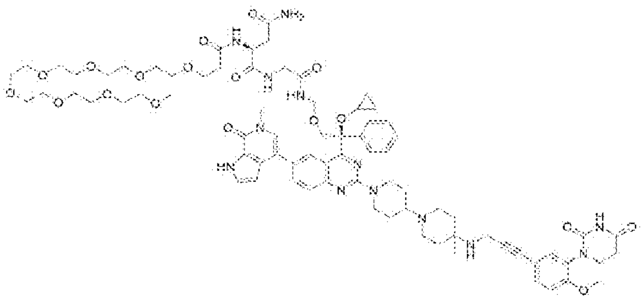

A formate of the title compound (3.0 mg) was obtained from the (S)-N1-(2-((((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)-2-(2,5,8,11,14,17,20,23-octaoxahexacosan-26-amide) succinamide of Production Example 133-2 (6.0 mg, 4.7 μmol), by the same method as Example 101.

ESI-MS (m/z): 1500.28 $[M+H]^+$.

[Production Example 133-1]

*tert*-Butyl (2,5,8,11,14,17,20,23-octaoxahexacosan-26-oyl)-L-asparaginate

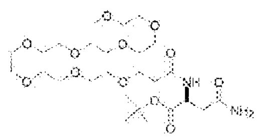

To a solution of methyl-PEG8-NHS ester (135 mg, 266 mol) and *tert-butyl* (2S)-2-amino-3-carbamoyl propanoate (50 mg, 0.27 mmol) in DMF (2 mL) there was added N,N-diisopropylethylamine (186 μL, 1.06 mmol), and the mixture was stirred for 3 hours at 50°C. The reaction mixture was concentrated under reduced pressure to obtain a crude product of the title compound (170 mg).

ESI-MS (m/z): 583.48 $[M+H]^+$.

[Production Example 133-2]

(S)-N1-(2-((((S)-2-Cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)-2-(2,5,8,11,14,17,20,23-octaoxahexacosan-26-amide)succinamide

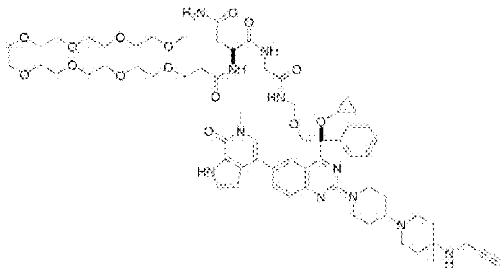

The title compound (25 mg) was obtained from the (9H-fluoren-9-yl)methyl (S)-(2-(((2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamate of Production Example 132-2 (40 mg, 40 μmol) and the *tert-butyl* (2,5,8,11,14,17,20,23-octaoxahexacosan-26-oyl)-L-asparaginate of Production Example 133-1 (46.9 mg, 80 μmol), by the same method as Production Example 132-3.
ESI-MS (m/z): 1281.11 [M+H]+.

[Example 134]

(S)-40-((2-((((S)-2-Cyclopropoxy-2-(2-(4-((3-(3-(2,4-dioxotetrahydropyrimidine-1(2H)-yl)-4-methoxyphenyl)prop-2-yn-1-ylamino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamoyl-38-oxo-2,5,8,11,14,17,20,23,26,29,32,35-dodecaoxa-39-azadotetracontan-42-oic acid

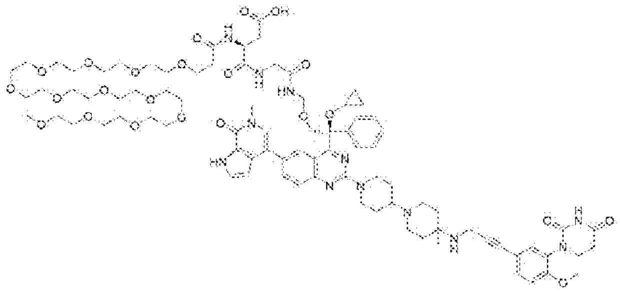

A formate of the title compound (2.96 mg) was obtained from the formate of (S)-40-((2-((((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamoyl)-38-oxo-2,5,8,11,14,17,20,23,26,29,32,35-dodecaoxa-39-azadotetracontan-42-oic acid of Production Example 134-2 (8.00 mg), by the same method as Example 101.
ESI-MS (m/z): 1677.41 [M+H]+.

[Production Example 134-1]

(S)-40-(2-(*tert*-Butoxy)-2-oxoethyl)-38-oxo-2,5,8,11,14,17,20,23,26,29,32,35-dodecaoxa-39-azahentetracontan-41-oic acid

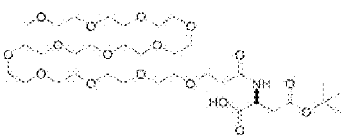

A crude product of the title compound (220 mg) was obtained from 1-[(3 8-oxo-2,5,8,11,14,17,20,23,26,29,32,35-dodecaoxacontatriacontan-38-yl)oxy]-2,5-pyrrolidinedione (200 mg, 292 μmol) and Fmoc-Asp (OTBU)-OH (120 mg, 292 μmol), by the same method as Production Example 133-1.

ESI-MS (m/z): 760.62 [M+H]+.

[Production Example 134-2]

(S)-40-((2-((((S)-2-Cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamoyl)-38-oxo-2,5,8,11,14,17,20,23,26,29,32,35-dodecaoxa-39-azadotetracontan-42-oic acid

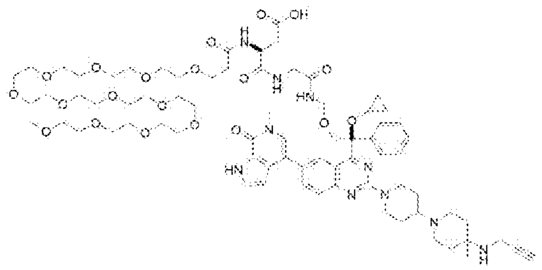

A reaction intermediate was obtained from the (9H-fluoren-9-yl)methyl (S)-(2-(((2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamate of Production Example 132-2 (40 mg, 40 μmol) and the (*S*)-40-(2-(*tert*-butoxy)-2-oxoethyl)-38-oxo-2,5,8,11,14,17,20,23,26,29,32,35-dodecaoxa-39-azahentetracontan-41-oic acid of Production Example 134-1 (31 mg, 40 μmol), by the same method as Production Example 132-3. To a solution of the obtained reaction intermediate in DMF (2 mL) there was added potassium *tert*-butoxide (9.03 mg, 80 μmol) at 0°C, and the mixture was stirred for 1 hour at 0°C. The reaction mixture was purified by ODS silica gel chromatography (0.1% formic acid aqueous solution:0.1% acetonitrile formate solution = 95:5 to 0:100) to obtain a formate of the title compound (20 mg).

ESI-MS (m/z): 1459.11 [M+H]+.

[Example 135]

(S)-2-Acetamide-N1-((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl) succinamide

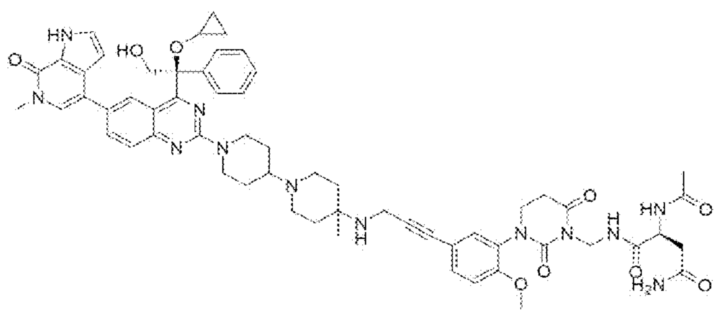

A formate of the title compound (7.5 mg) was obtained from the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 101-9 (8.0 mg, 12 μmol) and the (S)-2-acetamide-N1-((3-(5-iodo-2-methoxyphenyl)-2,6-dioxotetrahydropyrimidine-1(2H)-yl)methyl) succinamide of Production Example 135-2 (9.0 mg, 17 μmol), by the same method as Example 101. ESI-MS (m/z): 1089.82 [M+H]+.

[Production Example 135-1]

(9H-Fluoren-9-yl)methyl (S)-(4-amino-1-(((3-(5-iodo-2-methoxyphenyl)-2,6-dioxotetrahydropyrimidine-1(2H)-yl)methyl) amino)-1,4-dioxobutan-2-yl)carbamate

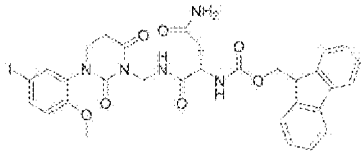

To a solution of the 1-(5-iodo-2-methoxyphenyl)dihydropyrimidine-2,4(1H,3H)-dione of Production Example 101-10 (200 mg, 578 μmol) in THF (2.0 mL) there was added potassium *tert*-butoxide (78 mg, 0.69 mmol) at 0°C, and the mixture was stirred for 10 minutes at room temperature. To the reaction mixture there was added a solution of the (S)-(2-((((9H-fluoren-9-yl)methoxy)carbonyl)amino)-4-amino-4-oxobutanamide)methyl acetate of Production Example 131-3 (258 mg, 607 μmol) in THF (4.0 mL), and the mixture was stirred for 45 minutes at room temperature. The reaction mixture was cooled to 0°C, an ammonium chloride aqueous solution was added, and the mixture was extracted with dichloromethane. The organic layer was dried over sodium sulfate and filtered, and the filtrate was concentrated under reduced pressure. The residue was purified by ODS silica gel chromatography (0.1% formic acid aqueous solution:0.1% acetonitrile formate solution = 85:15 to 25:75) to obtain the title compound (100 mg).
ESI-MS (m/z): 712.19 $[M+H]^+$.

[Production Example 135-2]

(S)-2-Acetamide-N1-((3-(5-iodo-2-methoxyphenyl)-2,6-dioxotetrahydropyrimidine-1(2H)-yl)methyl)succinimide

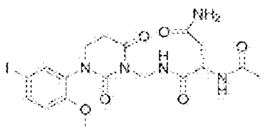

To a solution of the (9H-fluoren-9-yl)methyl (S)-(4-amino-1-(((3-(5-iodo-2-methoxyphenyl)-2,6-dioxotetrahydropyrimidine-1(2H)-yl)methyl)amino)-1,4-dioxobutan-2-yl)carbamate of Production Example 135-1 (100 mg, 141 μmol) in DMF (1.0 mL) there was added diethylamine (218 μL, 2.11 mmol), and the mixture was stirred for 30 minutes at room temperature. The reaction mixture was then concentrated under reduced pressure. To a solution of the residue in THF (1.2 mL) and dichloromethane (1.2 mL) there were added N,N-diisopropylethylamine (36.8 μL, 211 μmol) and acetic anhydride (15.9 μL, 169 μmol), and the mixture was stirred for 10 minutes at room temperature. The reaction mixture was diluted with DMF (400 μL) and stirred for 10 minutes, after which it was concentrated under reduced pressure to 1/6 volume. The residue was purified by ODS silica gel chromatography (0.1% formic acid aqueous solution:0.1% acetonitrile formate solution = 9:1 to 6:4) to obtain the title compound (60 mg).
ESI-MS (m/z): 532.14 $[M+H]^+$.

[Example 136]

(S)-N1-((3-(5-(3-((1'-(4-((S)-1-Cyclopropoxy-2-hydroxy-1-phenylethyl)-6-(6-methyl-7-oxo-6,7-dihydro-1H-pyrrolo[2,3-c] pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)-2-(2,5,8,11,14,17,20,23,26,29,32,35,38,41,44,47,50,53,56,59,62,65,68,71-tetracosaoxatetraheptacontan-74-amide) succinamide

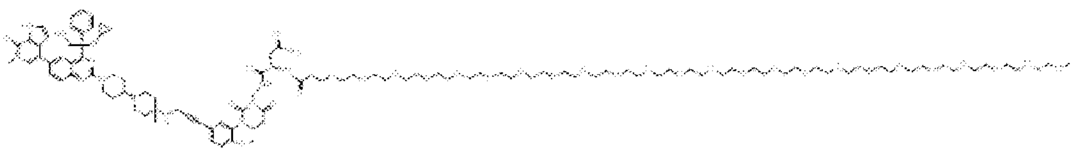

A formate of the title compound (6.0 mg) was obtained from the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 101-9 (5.0 mg, 7.3 μmol) and the (S)-N1-((3-(5-iodo-2-methoxyphenyl)-2,6-dioxotetrahydropyrimidine-1(2H)-yl) methyl)-2-(2,5,8,11,14,17,20,23,26,29,32,35,38,41,44,47,50,53,56,59,62,65,68,71-tetracosaoxatetraheptacontan-74-amide) succinamide of Production Example 136-1 (15 mg, 9.5 μmol), by the same method as Example 101.

ESI-MS (m/z): 1074.16[(M+2H)/2]$^+$.

[Production Example 136-1]

(S)-N1-((3-(5-1odo-2-methoxyphenyl)-2,6-dioxotetrahydropyrimidine-1(2H)-yl) methyl)-2-(2,5,8,11,14,17,20,23,26,29,32,35,38,41,44,47,50,53,56,59,62,65,68,71-tetracosaoxatetraheptacontan-74-amide) succinamide

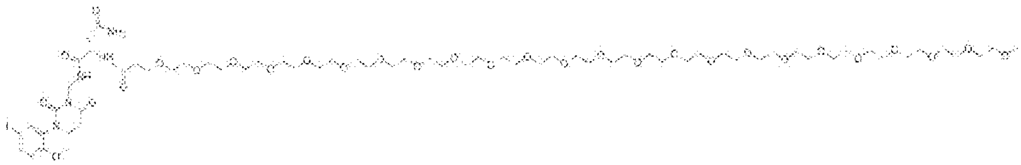

To a solution of the (9H-fluoren-9-yl)methyl (S)-(4-amino-1-(((3-(5-iodo-2-methoxyphenyl)-2,6-dioxotetrahydropyrimidine-1(2H)-yl)methyl)amino)-1,4-dioxobutan-2-yl)carbamate of Production Example 135-1 (40 mg, 56 μmol) in DMF (600 μL) there was added diethylamine (58.7 μL, 562 μmol), and the mixture was stirred for 30 minutes at room temperature. The reaction mixture was then concentrated under reduced pressure. Half of the residue was dissolved in DMF (300 μL), and then N,N-diisopropylethylamine (23.6 μL, 135 μmol) and 2,5-dioxopyrrolidin-1-yl 2,5,8,11,14,17,20,23,26,29,32,35,38,41,44,47,50,53,56,59,62,65,68,71-tetracosaoxatetraheptacontan-74-oate (41 mg, 34 μmol) were added and the mixture was stirred for 1 hour at room temperature. The reaction mixture was purified by ODS silica gel chromatography (0.1% formic acid aqueous solution:0.1% acetonitrile formate solution = 9:1 to 1:1) to obtain the title compound (26 mg).

ESI-MS (m/z): 795.31[(M+2H)/2]$^+$.

[Example 137]

(S)-2-Acetamide-N1-(2-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-2-oxoethyl) succinamide

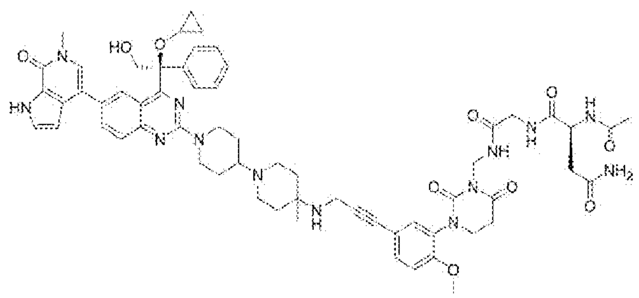

The title compound (4.6 mg) was obtained from the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 101-9 (8.0 mg, 12 μmol) and the (S)-2-acetamide-N1-(2-(((3-(5-iodo-2-methoxyphenyl)-2,6-dioxotetrahydropyrimidine-1(2H)-yl)methyl)amino)-2-oxoethyl) succinamide of Production Example 137-3 (6.9 mg, 12 μmol), by the same method as Example 101.

ESI-MS (m/z): 1146.56 [M+H]$^+$.

[Production Example 137-1]

(9H-Fluoren-9-yl)methyl(2-(((3-(5-iodo-2-methoxyphenyl)-2,6-dioxotetrahydropyrimidine-1(2H)-yl)methyl)amino)-2-oxoethyl)carbamate

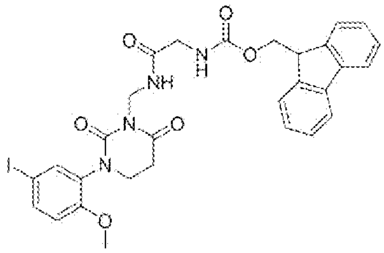

A crude product of the title compound (283 mg) was obtained from the 1-(5-iodo-2-methoxyphenyl)dihydropyrimidine-2,4(1H,3H)-dione of Production Example 101-10 (250 mg, 722 μmol) and (2-((((9H-fluoren-9-yl)methoxy)carbonyl)amino)acetamide)methyl acetate (293 mg, 795 μmol), known from published literature, by the same method as Production Example 135-1.
ESI-MS (m/z): 655.14 $[M+H]^+$.

[Production Example 137-2]

(9H-Fluorolen-9-yl)methyl (S)-(4-amino-1-((2-(((3-(5-iodo-2-methoxyphenyl)-2,6-dioxotetrahydropyrimidine-1(2H)-yl) methyl)amino)-2-oxoethyl)amino)-1,4-dioxobutan-2-yl)carbamate

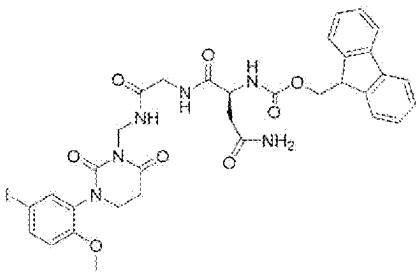

To a solution of the (9H-fluorolen-9-yl)methyl (2-(((3-(5-iodo-2-methoxyphenyl)-2,6-dioxotetrahydropyrimidine-1(2H)-yl)methyl)amino)-2-oxoethyl)carbamate of Production Example 137-1 (100 mg, 153 μmol) in DMF (1 mL) there was added diethylamine (1.00 mL, 9.67 mmol), and the mixture was stirred for 2 hours at room temperature. The reaction mixture was then concentrated under reduced pressure. To a solution of the residue in DMF (1 mL) there were added FMOC-ASN-OH (81 mg, 0.23 mmol), N,N-diisopropylethylamine (80 μL, 0.46 mmol) and HATU (87 mg, 0.23 mol), and the mixture was stirred for 30 minutes at room temperature. The reaction mixture was purified by ODS silica gel chromatography (0.1% formic acid aqueous solution:0.1% acetonitrile formate solution = 15:85 to 45:55) to obtain the title compound (95 mg).
ESI-MS (m/z): 769.24 $[M+H]^+$.

[Production Example 137-3]

(S)-2-Acetamide-N1-(2-(((3-(5-iodo-2-methoxyphenyl)-2,6-dioxotetrahydropyrimidine-1(2H)-yl)methyl)amino)-2-oxoethyl) succinamide

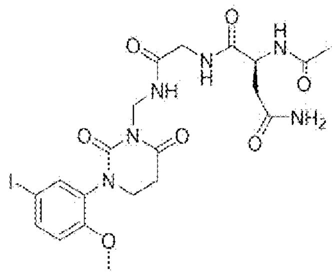

To a solution of the (9H-fluorolen-9-yl)methyl(S)-(4-amino-1-((2-(((3-(5-iodo-2-methoxyphenyl)-2,6-dioxotetrahydropyrimidine-1(2H)-yl)methyl)amino)-2-oxoethyl)amino)-1,4-dioxobutan-2-yl)carbamate of Production Example 137-2 (60 mg, 78 μmol) in DMF (500 μL) there was added diethylamine (81 μL, 0.78 mmol), and the mixture was stirred for 40 minutes at room temperature. The reaction mixture was then concentrated under reduced pressure. To a solution of the residue in DMF (500 μL) there were added triethylamine (32.6 μL, 234 μmol) and acetic anhydride (11.1 μL, 117 μmol), and the mixture was stirred for 1 hour at room temperature. The reaction mixture was purified by ODS silica gel chromatography (0.1% formic acid aqueous solution:0.1% acetonitrile formate solution = 10:90 to 35:65) to obtain the title compound (38 mg). ESI-MS (m/z): 589.11 $[M+H]^+$.

[Example 138]

(S)-3-Acetamide-4-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid

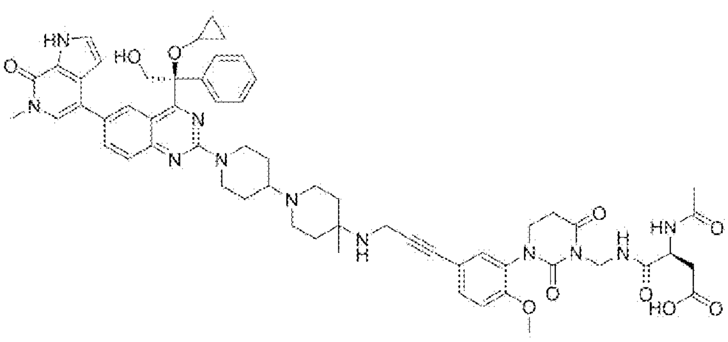

To a solution of the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 101-9 (180 mg, 262 μmol), the (S)-3-acetamide-4-(((3-(5-iodo-2-methoxyphenyl)-2,6-dioxotetrahydropyrimidine-1(2H)-yl)methyl)amino)-4-oxobutanoic acid of Production Example 138-3 (168 mg, 315 μmol) and CuI (40 mg, 0.21 mmol) in DMF (4.06 mL) there were added N,N-diisopropylethylamine (458 μL, 2.62 mmol) and tetrakis(triphenylphosphine)palladium(0) (243 mg, 0.21 mmol), and the mixture was stirred for 1 hour at 50°C under a nitrogen atmosphere. After cooling to room temperature, the reaction mixture was purified by ODS silica gel chromatography (0.1% formic acid aqueous solution:0.1% acetonitrile formate solution = 3:7 to 1:0), followed by (0.1% formic acid aqueous solution:0.1% acetonitrile formate solution = 1:1), and then by (0.1% ammonia water solution:0.1% ammonia-acetonitrile solution = 9:1 to 7:3), to obtain the title compound (161 mg).

ESI-MS (m/z): 1090.78 $[M+H]^+$.

[Production Example 138-1]

*tert*-Butyl (S)-3-((((9H-fluoren-9-yl)methoxy)carbonyl)amino-4-((acetoxymethyl)amino)-4-oxobutanoate

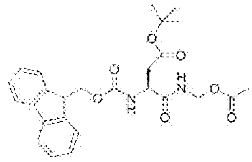

To a solution of (S)-(2-((((9H-fluoren-9-yl)methoxy)carbonyl)amino)-4-(tert-butoxy)-4-oxobutanoyl)glycine (3.20 g, 6.83 mmol) in THF (67.2 mL), toluene (22.6 mL) and pyridine (663 μL) there was added lead tetraacetate (3.79 g, 8.54 mmol), and the mixture was stirred for 1.5 hours at 110°C. The reaction mixture was cooled to room temperature, filtered with Celite and washed with ethyl acetate. The filtrate was concentrated under reduced pressure and the residue was purified by silica gel chromatography (n-heptane:ethyl acetate = 9:1 to 1:1) to obtain the title compound (2.10 g).
ESI-MS (m/z): 845.58[2M-2OAc+H]$^+$.

[Production Example 138-2]

*tert*-Butyl (S)-3((((9H-fluoren-9-yl)methoxy)carbonyl)amino)-4-(((3-(5-iodo-2-methoxyphenyl)-2,6-dioxotetrahydropyrimidine-1(2H)-yl)methyl)amino)-4-oxobutanoate

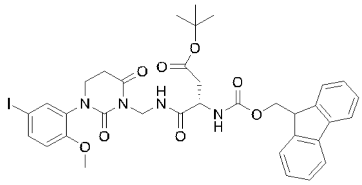

To a solution of the 1-(5-iodo-2-methoxyphenyl)dihydropyrimidine-2,4(1H,3H)-dione of Production Example 101-10 (900 mg, 2.60 mmol) in THF (25 mL) there was added potassium *tert*-butoxide (350 mg, 3.12 mmol) at 0°C, and the mixture was stirred for 10 minutes. To the reaction mixture there was added a solution of the *tert-butyl* (S)-3-((((9H-fluoren-9-yl)methoxy)carbonyl)amino-4-((acetoxymethyl)amino)-4-oxobutanoate of Production Example 138-1 (1380 mg, 2.86 mmol) in THF (25 mL) at 0°C, and the mixture was warmed to room temperature and then stirred for 40 minutes. The reaction mixture was cooled to 0°C, and then an ammonium chloride aqueous solution was added and the mixture was extracted with dichloromethane. The organic layer was dried over magnesium sulfate and filtered, and the filtrate was concentrated under reduced pressure. The residue was purified by silica gel chromatography (n-heptane:ethyl acetate = 90:10 to 0:1) to obtain the title compound (1.75 g).
ESI-MS (m/z): 769.27 [M+H]$^+$.

[Production Example 138-3]

(S)-3-Acetamide-4-(((3-(5-iodo-2-methoxyphenyl)-2,6-dioxotetrahydropyrimidine-1(2H)-yl)methyl)amino)-4-oxobutanoic acid

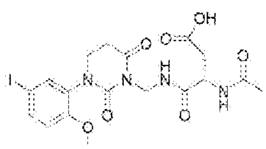

To a solution of the *tert-butyl* (S)-3((((9H-fluoren-9-yl)methoxy)carbonyl)amino)-4-(((3-(5-iodo-2-methoxyphenyl)-2,6-dioxotetrahydropyrimidine-1(2H)-yl)methyl)amino)-4-oxobutanoate of Production Example 138-2 (1.75 g, 2.28 mmol) in DMF (9.6 mL) there was added diethylamine (2.4 mL, 23 mmol), and the mixture was stirred for 30 minutes at room temperature. The reaction mixture was then concentrated under reduced pressure. To a solution of the residue in THF (9 mL) and dichloromethane (18 mL) there were added N,N-diisopropylethylamine 1.19 mL, 6.83 mmol) and acetic anhydride (323 μL, 3.42 mmol) and the mixture was stirred for 30 minutes at room temperature. After further adding acetic anhydride (32 μL, 0.34 mmol), the mixture was stirred for 10 minutes. The reaction mixture was concentrated under

reduced pressure and the residue was purified by ODS silica gel chromatography (0.1% formic acid aqueous solution:0.1% acetonitrile formate solution = 9:1 to 1:1), to obtain an intermediate. The obtained intermediate was processed by azeotropic distillation with toluene to remove the water, and then dichloromethane (18 mL) and TFA (9 mL) were added and the mixture was stirred for 1 hour at room temperature. The reaction mixture was concentrated under reduced pressure and processed by azeotropic distillation with toluene, after which the residue was purified by ODS silica gel chromatography (0.1% formic acid aqueous solution:0.1% acetonitrile formate solution = 9:1 to 6:4) to obtain the title compound (905 mg).

ESI-MS (m/z): 533.20 $[M+H]^+$.

[Example 139]

(S)-40-(((3-(5-(3-((1'-(4-((S)-1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbonyl)-38-oxo-2,5,8,11,14,17,20,23,26,29,32,35-dodecaoxa-39-azadotetracontan-42-oic acid

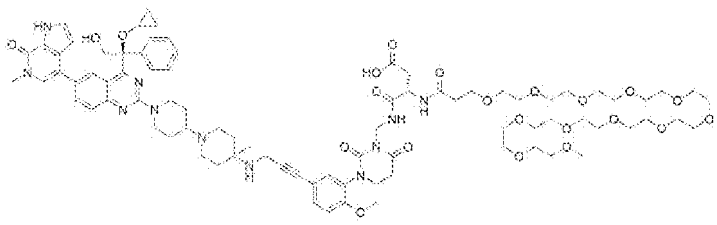

To a solution of the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 101-9 (5.00 mg, 7.29 μmol), the (S)-40-(((3-(5-iodo-2-methoxyphenyl)-2,6-dioxotetrahydropyrimidine-1(2H)-yl)methyl) carbamoyl)-38-oxo-2,5,8,11,14,17,20,23,26,29,32,35-dodecaoxo-39-azadotetracontan-42-oic acid of Production Example 139-2 (11.6 mg, 10.9 μmol), tetrakistriphenylphosphinepalladium(0) (3.37 mg, 2.92 μmol) and N,N-diisopropylethylamine (130 μL, 729 μmol) in DMF (440 μL) there was added copper(I) iodide (555 μg, 2.92 μmol), and the mixture was stirred for 2 hours at 40°C under a nitrogen atmosphere. The reaction mixture was then concentrated under reduced pressure. The residue was purified by ODS silica gel chromatography (water (containing 0.1% formic acid):acetonitrile (containing 0.1% formic acid) = 97:3 to 30:70) to obtain a formate of the title compound (4.2 mg).

ESI-MS (m/z): 1618.70 $[M+H]^+$.

[Production Example 139-1]

*tert*-Butyl (S)-3-((((9H-fluoren-9-yl)methoxy)carbonyl)amino)-4-(((3-(5-iodo-2-methoxyphenyl)-2,6-dioxotetrahydropyrimidine-1(2H)-yl)methyl)amino)-4-oxobutanoate

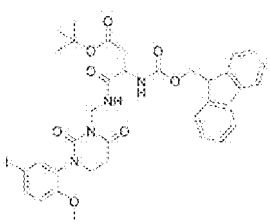

To a solution of the 1-(5-iodo-2-methoxyphenyl)dihydropyrimidine-2,4(1H,3H)-dione of Production Example 101-10 (95 mg, 0.27 mmol) in THF (3 mL) there was added potassium *tert*-butoxide (37.0 mg, 329 μmol) at 0°C, and the mixture was stirred for 5 minutes at 0°C. To the reaction mixture there was added a solution of the *tert-butyl* (S)-3-((((9H-fluoren-9-yl)methoxy)carbonyl)amino-4-((acetoxymethyl)amino)-4-oxobutanoate of Production Example 138-1 (146 mg, 302 μmol) in THF (2 mL), and after increasing the temperature to room temperature, the mixture was stirred for 4 hours. The reaction mixture was cooled to 0°C, an ammonium chloride aqueous solution was added, and the mixture was

extracted with dichloromethane. The organic layer was washed with brine, dried over anhydrous sodium sulfate, and filtered. The filtrate was concentrated under reduced pressure. The residue was purified by silica gel chromatography (n-heptane:ethyl acetate = 9:1 to 0:1) to obtain the title compound (180 mg).

ESI-MS (m/z): 769.34 [M+H]+.

[Production Example 139-2]

(S)-4-(((3-(5-Iodo-2-methoxyphenyl)-2,6-dioxotetrahydropyrimidine-1(2H)-yl)methyl)carbamoyl)-38-oxo-2,5,8,11,14,17,20,23,26,29,32,35-dodecaoxa-39-azadotetracontan-42-oic acid

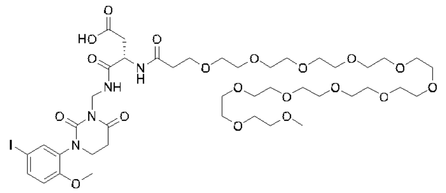

To a solution of the *tert-butyl* (S)-3-((((9H-fluoren-9-yl)methoxy)carbonyl)amino)-4-(((3-(5-iodo-2-methoxyphenyl)-2,6-dioxotetrahydropyrimidine-1(2H)-yl)methyl)amino)-4-oxobutanoate of Production Example 139-1 (100 mg, 130 μmol) in DMF (1.0 mL) there was added diethylamine (336 μL, 3.35 mmol), and the mixture was stirred for 30 minutes at room temperature. The reaction mixture was concentrated under reduced pressure and processed 3 times by azeotropic distillation with toluene (1 mL). The obtained residue was dissolved in DMF (1.0 mL), and then N,N-diisopropylethylamine (227 μL, 1.30 mmol) and 1-[(38-oxo-2,5,8,11,14,17,20,23,26,29,32,35-dodecaoxaoctatriacontan-38-yl)oxy]2,5-pyrrolidinedione (116 mg, 169 μmol) were added and the mixture was stirred for 1 hour at room temperature. Diethylamine (336 μL, 3.25 mmol) was added to the reaction mixture, which was then processed 3 times by azeotropic distillation with toluene (1 mL). The obtained residue was dissolved in dichloromethane (1.26 mL), and then TFA(697 μL) was added and the mixture was stirred for 1 hour at room temperature. The reaction mixture was then concentrated under reduced pressure. The residue was purified by ODS silica gel chromatography (water (containing 0.1% formic acid):acetonitrile (containing 0.1% formic acid) = 2:98 to 0:100), to obtain the title compound (100 mg).

ESI-MS (m/z): 1061.38 [M+H]+.

[Example 140]

(S)-2-Acetamide-N1-((R)-2-cyclopropoxy-2-(2-(4-((2R,5S)-4-(3-(2,4-dioxotetrahydropyrimidine-1(2H)-yl)-4-methoxybenzoyl)-2,5-dimethylpiperazin-1-yl)piperidin-1-yl)-6-(6-methyl-7-oxo-6,7-dihydro-1H-pyrrolo[2,3-c]pyridin-4-yl)quinazolin-4-yl)-2-phenylethyl) succinamide

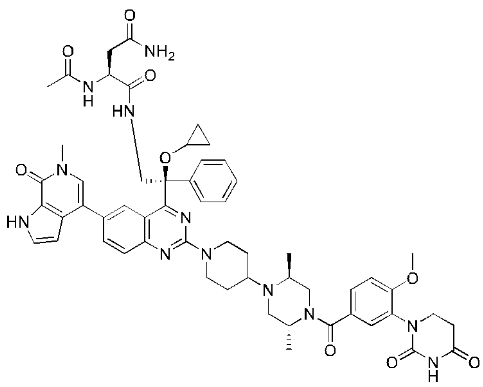

The title compound (1.53 mg) was obtained from the *tert-butyl* (2R,5S)-4-(1-(4-(R)-2((S)-2-acetamide-4-amino-4-oxobutaneamide)-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate of Production Example 140-6 (5.00 mg, 5.54 μmol) and

the 3-(2,4-dioxotetrahydropyrimidine-1(2H)-yl)-4-methoxybenzoic acid of Production Example 104-7 (2.20 mg, 8.31 μmol), by the same method as Example 104.

ESI-MS (m/z): 1049.78 [M+H]+.

[Production Example 140-1]

*tert*-Butyl (2R,5S)-4-(1-(4-(1-cyclopropoxy-2-hydroxy-1-phenylethyl)-6-(iodoquinazolin-2-yl)piperidin-4-yl)-2,5-dimethylpiperazine-1-carboxylate

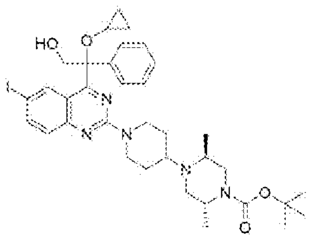

The title compound (6.1 g) was obtained from the 2-chloro-4-(1-cyclopropoxy-1-phenyl-2-((tetrahydro-2H-pyran-2-yl)oxy)ethyl)-6-iodoquinazoline of Production Example 104-3 (4.6 g, 8.4 mmol) and the *tert-butyl* (2R,5S)-2,5-dimethyl-4-(piperidin-4-yl)piperazine-1-carboxylate of Production Example 114-2 (2.86 g, 9.60 mmol), by the same method as Production Example 104-5 and Production Example 117-7.

ESI-MS (m/z): 728.91 [M+H]+.

[Production Example 140-2]

*tert*-Butyl (2R,5S)-4-(1-(4-((S)-1-cyclopropoxy-2-hydroxy-1-phenylethyl)-6-(iodoquinazolin-2-yl)piperidin-4-yl)-2,5-dimethylpiperazine-1-carboxylate

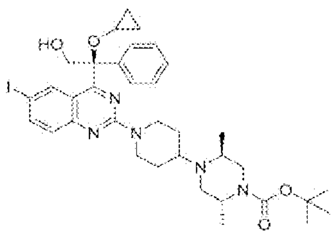

The *tert-butyl* (2R,5S)-4-(1-(4-(1-cyclopropoxy-2-hydroxy-1-phenylethyl)-6-(iodoquinazolin-2-yl)piperidin-4-yl)-2,5-dimethylpiperazine-1-carboxylate of Production Example 140-1 (3.2 g, 4.4 mmol) was separated by SFC (CHIRALPAK (registered trademark) IC (3 cm×25 cm), CO2:methanol = 60:40), to obtain the title compound (1.32 g). ESI-MS (m/z): 728.87 [M+H]+.

[Production Example 140-3]

*tert-Butyl* (2R,5 S)-4-(1-(4-((S)-1-cyclopropoxy-2-hydroxy-1-phenylethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-carboxylate

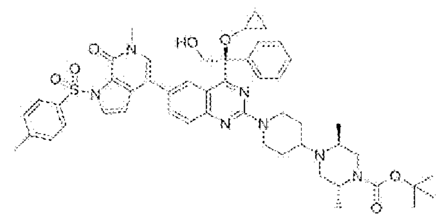

The title compound (1.31 g) was obtained from the *tert-butyl* (2R,5S)-4-(1-(4-((S)-1-cyclopropoxy-2-hydroxy-1-phenylethyl)-6-(iodoquinazolin-2-yl)piperidin-4-yl)-2,5-dimethylpiperazine-1-carboxylate of Production Example 140-2 (1.35 g, 1.86 mmol), by the same method as Production Example 101-5.
ESI-MS (m/z): 903.27 $[M+H]^+$.

[Production Example 140-4]

*tert*-Butyl (2R,5S)-4-(1-(4-((R)-2-amino-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate

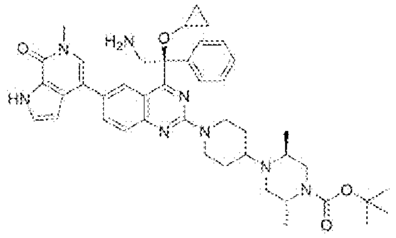

The title compound (1.05 g) was obtained from the *tert-butyl* (2R,5S)-4-(1-(4-((S)-1-cyclopropoxy-2-hydroxy-1-phenylethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-carboxylate of Production Example 140-3 (1.21 g, 1.34 mmol), by the same method as Production Example 117-8 and Production Example 101-9.
ESI-MS (m/z): 747.52 $[M+H]^+$.

[Production Example 140-5]

*tert*-Butyl (2R,5S)-4-(1-(4-((R)-2((S)-2((((9H-fluoren-9-yl)methoxy)carbonyl)amino)-4-amino-4-oxobutaneamide)-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate

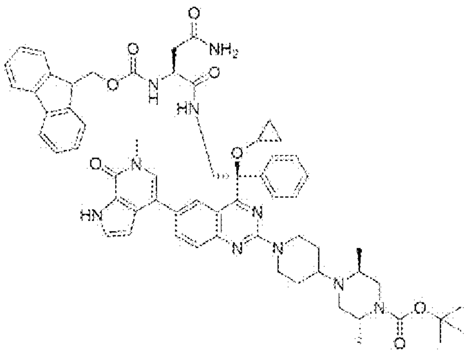

The title compound (310 mg) was obtained from the *tert*-butyl (2R,5S)-4-(1-(4-((R)-2-amino-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate of Production Example 140-4 (200 mg, 268 μmol) and FMOC-ASN-OH (142 mg, 402 μmol), by the

same method as Example 103.
ESI-MS (m/z): 1083.67 [M+H]$^+$.

[Production Example 140-6]

*tert*-Butyl (2R,5S)-4-(1-(4-((R)-2((S)-2-acetamide-4-amino-4-oxobutaneamide)-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate

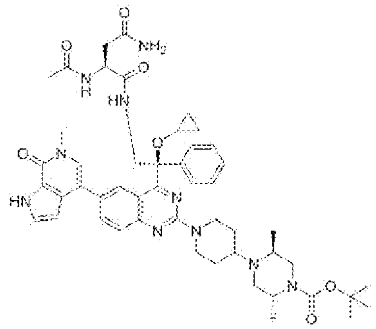

The title compound (21 mg) was obtained from the *tert-butyl* (2R,5S)-4-(1-(4-((R)-2((S)-2((((9H-fluoren-9-yl)methoxy)carbonyl)amino)-4-amino-4-oxobutaneamide)-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate of Production Example 140-5 (50 mg, 46 μmol), by the same method as Production Example 137-3. ESI-MS (m/z): 903.74 [M+H]$^+$.

[Example 141]

(S)-2-Acetamide-N1-((3-(5-((2R,5S)-4-(1-(4-((R)-2-amino-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carbonyl)-2-methoxyphenyl)-2,6-dioxotetrahydropyrimidine-1(2H)-yl)methyl) succinamide

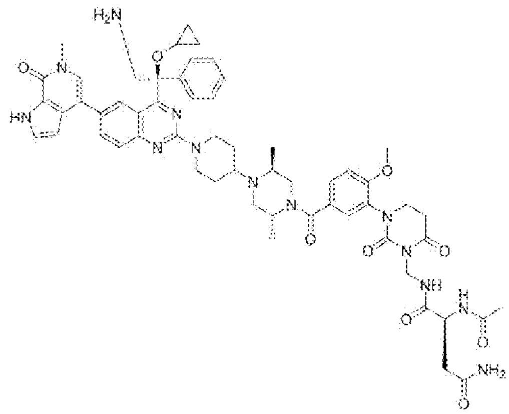

A formate of the title compound (9.1 mg) was obtained from the *tert-butyl* (2R,5S)-4-(1-(4-((R)-2-((((9H-fluoren-9-yl)methoxy)carbonyl)amino)-1-cyclopropoxy-1-phenylethyl)-6-(6-methyl-7-oxo-6,7-dihydro-1H-pyrrolo[2,3-c]pyridin-4-yl)quinazolin-2-yl)-piperidin-4-yl)-2,5-dimethylpiperazine-1-carboxylate of Production Example 114-5 (10 mg, 10 μmol) and the (S)-3-(3-((2-acetamide-4-amino-4-oxobutaneamide)methyl)-2,4-dioxotetrahydropyrimidine-1(2H)-yl)-4-methoxybenzoic acid of Production Example 141-4 (6.96 mg, 15 μmol), by the same method as Example 114.
ESI-MS (m/z): 1078.78 [M+H]$^+$.

[Production Example 141-1]

Benzyl 3-(2,4-dioxotetrahydropyrimidine-1(2H)-yl)-4-methoxybenzoate

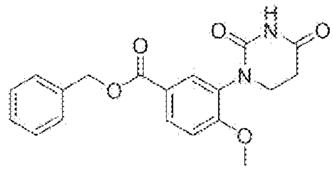

To a solution of the 3-(2,4-dioxotetrahydropyrimidine-1(2H)-yl)-4-methoxybenzoic acid of Production Example 104-7 (1.00 g, 3.78 mmol) in DMF (10 mL) there were added potassium carbonate (1.05 g, 7.57 mmol) and benzyl bromide (494 μL, 4.16 mmol) at room temperature, and the mixture was stirred for 15 hours at room temperature. The reaction mixture was diluted with ethyl acetate and water, and then the aqueous layer was extracted with ethyl acetate. The organic layer was washed with water and brine, dried over magnesium sulfate and filtered, and the filtrate was concentrated under reduced pressure. The residue was purified by silica gel column chromatography (n-heptane:ethyl acetate = 1:1 to ethyl acetate) to obtain the title compound (917 mg).

ESI-MS (m/z): 355.29 $[M+H]^+$.

[Production Example 141-2]

Benzyl (S)-3-(3-((2-((((9H-fluoren-9-yl)methoxy)carbonyl)amino)-4-amino-4-oxobutaneamide)methyl)-2,4-dioxotetrahydropyrimidine-1(2H)-yl)-4-methoxybenzoate

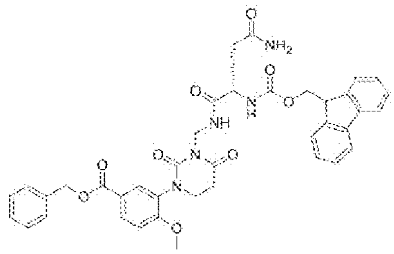

A crude product of the title compound (140 mg) was obtained from the benzyl 3-(2,4-dioxotetrahydropyrimidine-1(2H)-yl)-4-methoxybenzoate of Production Example 141-1 (150 mg, 423 μmol), by the same method as Production Example 135-1.

ESI-MS (m/z): 720.48 $[M+H]^+$.

[Production Example 141-3]

Benzyl (S)-3-(3-((2-acetamide-4-amino-4-oxobutaneamide)methyl)-2,4-dioxotetrahydropyrimidine-1(2H)-yl)-4-methoxybenzoate

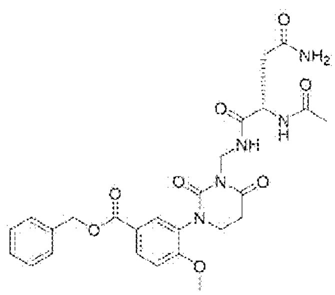

The title compound (43 mg) was obtained from a crude product of the benzyl (S)-3-(3-((2-((((9H-fluoren-9-yl)methoxy)carbonyl)amino)-4-amino-4-oxobutaneamide)methyl)-2,4-dioxotetrahydropyrimidine-1(2H)-yl)-4-methoxybenzoate of Production Example 141-2 (90 mg), by the same method as Production Example 137-3.
ESI-MS (m/z): 540.40 [M+H]+.

[Production Example 141-4]

(S)-3-(3-((2-Acetamide-4-amino-4-oxobutaneamide)methyl)-2,4-dioxotetrahydropyrimidine-1(2H)-yl)-4-methoxybenzoic acid

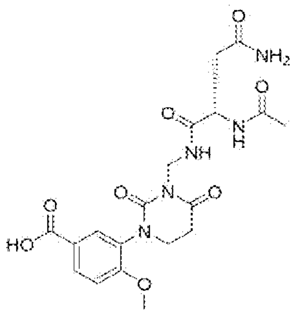

The title compound (39 mg) was obtained from the benzyl (S)-3-(3-((2-acetamide-4-amino-4-oxobutaneamide)methyl)-2,4-dioxotetrahydropyrimidine-1(2H)-yl)-4-methoxybenzoate of Production Example 141-3 (43 mg, 80 μmol), by the same method as Production Example 103-4.
ESI-MS (m/z): 450.33 [M+H]+.

[Example 142]

(S)-3-Acetamide-4-(((3-(5-((2R,5S)-4-(1-(4-((R)-2-amino-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carbonyl)-2-methoxyphenyl)-2,6-dioxotetrahydropyrimidine-1(2H)-yl)methyl)amino)-4-oxobutanoic acid

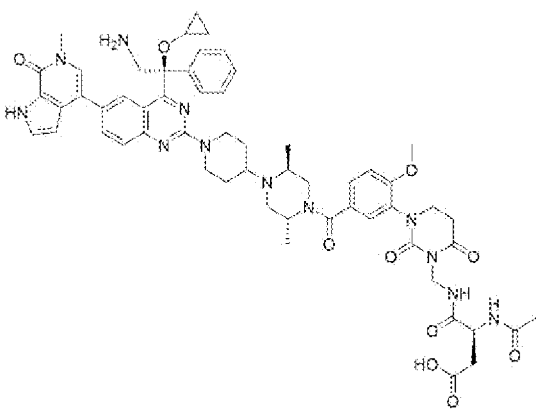

A formate of the title compound (8.5 mg) was obtained from the *tert-butyl* (2R,5S)-4-(1-(4-((R)-2-((((9H-fluoren-9-yl)methoxy)carbonyl)amino)-1-cyclopropoxy-1-phenylethyl)-6-(6-methyl-7-oxo-6,7-dihydro-1H-pyrrolo[2,3-c]pyridin-4-yl)quinazolin-2-yl)-piperidin-4-yl)-2,5-dimethylpiperazine-1-carboxylate of Production Example 114-5 (12 mg, 12 μmol) and the (S)-3-(3-((2-acetamide-4-(*tert*-butoxy)-4-oxobutaneamide)methyl)-2,4-dioxotetrahydropyrimidine-1 (2H)-yl)-4-methoxybenzoic acid of Production Example 142-2 (7.5 mg, 15 μmol), by the same method as Example 114 and Example 120.
ESI-MS (m/z): 1079.50 [M+H]+.

[Production Example 142-1]

Benzyl (S)-3-(3-((2-((((9H-fluoren-9-yl)methoxy)carbonyl)amino)-4-(*tert*-butoxy)-4-oxobutaneamide)methyl)-2,4-dioxotetrahydropyrimidine-1(2H)-yl)-4-methoxybenzoate

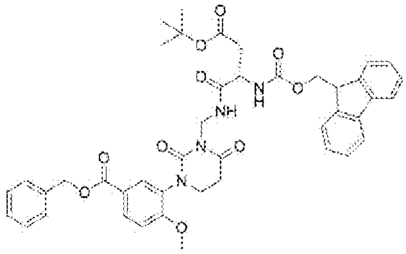

The title compound (185 mg) was obtained from the benzyl 3-(2,4-dioxotetrahydropyrimidine-1(2H)-yl)-4-methoxybenzoate of Production Example 141-1 (150 mg, 423 μmol) and the *tert-butyl* (S)-3-((((9H-fluoren-9-yl)methoxy)carbonyl)amino-4-((acetoxymethyl)amino)-4-oxobutanoate of Production Example 138-1 (225 mg, 466 μmol), by the same method as Production Example 135-1.
ESI-MS (m/z): 777.35 $[M+H]^+$.

[Production Example 142-2]

(S)-3-(3-((2-Acetamide-4-(*tert*-butoxy)-4-oxobutaneamide)methyl)-2,4-dioxotetrahydropyrimidine-1(2H)-yl)-4-methoxybenzoic acid

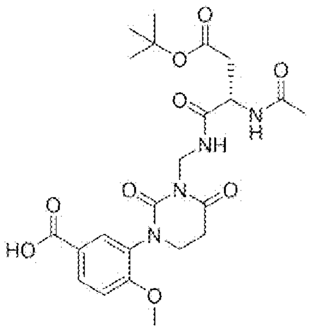

The title compound (72 mg) was obtained from the benzyl (S)-3-(3-((2-((((9H-fluoren-9-yl)methoxy)carbonyl) amino)-4-(*tert*-butoxy)-4-oxobutaneamide)methyl)-2,4-dioxotetrahydropyrimidine-1 (2H)-yl)-4-methoxybenzoate of Production Example 142-1 (130 mg, 167 μmol), by the same method as Production Example 137-3 and Production Example 103-4.
ESI-MS (m/z): 505.19$[M-H]^+$.

[Example 143]

2-Acetamide-N-((3-(5-((2R,5S)-4-(1-(4-((R)-2-amino-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carbonyl)-2-methoxyphenyl)-2,6-dioxotetrahydropyrimidine-1(2H)-yl)methyl)acetamide

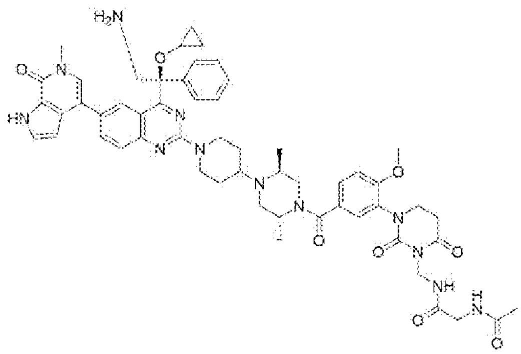

A formate of the title compound (7.4 mg) was obtained from the *tert-butyl* (2R,5S)-4-(1-(4-((R)-2-((((9H-fluoren-9-yl)methoxy)carbonyl)amino)-1-cyclopropoxy-1-phenylethyl)-6-(6-methyl-7-oxo-6,7-dihydro-1H-pyrrolo[2,3-c]pyridin-4-yl)quinazolin-2-yl)-piperidin-4-yl)-2,5-dimethylpiperazine-1-carboxylate of Production Example 114-5 (10 mg, 10 μmol) and the 3-(3-((2-acetamideacetamide)methyl)-2,4-dioxotetrahydropyrimidine-1(2H)-yl)-4-methoxybenzoic acid of Production Example 143-3 (6.1 mg, 15 μmol), by the same method as Example 114.

ESI-MS (m/z): 1022.83 [M+H]+.

[Production Example 143-1]

Benzyl 3-(3-((2-((((9H-fluoren-9-yl)methoxy)carbonyl)amino)acetamide)methyl)-2,4-dioxotetrahydropyrimidine-1(2H)-yl)-4-methoxybenzoate

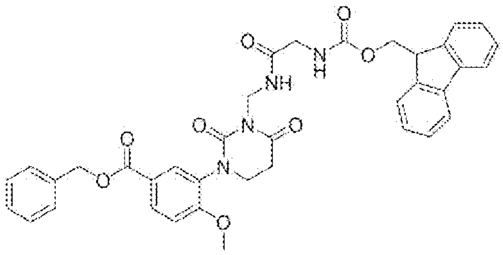

The title compound (125 mg) was obtained from the benzyl 3-(2,4-dioxotetrahydropyrimidine-1(2H)-yl)-4-methoxybenzoate of Production Example 141-1 (150 mg, 423 μmol) and (2-((((9H-fluoren-9-yl)methoxy)carbonyl)amino)acetamide)methyl acetate (172 mg, 466 μmol) known from published literature, by the same method as Production Example 135-1.

ESI-MS (m/z): 663.42 [M+H]+.

[Production Example 143-2]

Benzyl 3-(3-((2-acetamideacetamide)methyl)-2,4-dioxotetrahydropyrimidine-1(2H)-yl)-4-methoxybenzoate

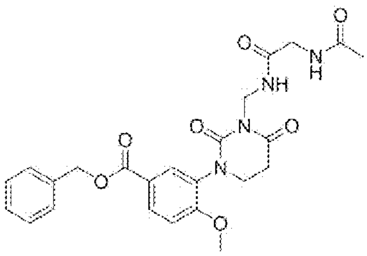

The title compound (33 mg) was obtained from the benzyl 3-(3-((2-((((9H-fluoren-9-yl)methoxy)carbonyl)amino)acetamide)methyl)-2,4-dioxotetrahydropyrimidine-1(2H)-yl)-4-methoxybenzoate of Production Example 143-1 (60 mg, 91 μmol), by the same method as Production Example 137-3.

ESI-MS (m/z): 483.38 [M+H]+.

[Production Example 143-3]

3-(3-((2-Acetamideacetamide)methyl)-2,4-dioxotetrahydropyrimidine-1(2H)-yl)-4-methoxybenzoic acid

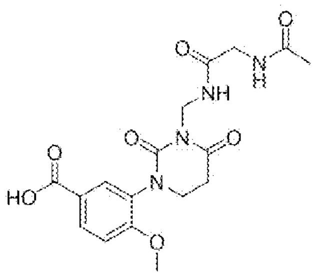

The title compound (26 mg) was obtained from the benzyl 3-(3-((2-acetamideacetamide)methyl)-2,4-dioxotetrahydropyrimidine-1(2H)-yl)-4-methoxybenzoate of Production Example 143-2 (33 mg, 68 μmol), by the same method as Production Example 103-4.

ESI-MS (m/z): 393.27 [M+H]+.

[Example 144]

1-(5-(3-((1'-(4-(1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)dihydropyrimidine-2,4(1H,3H)-dione

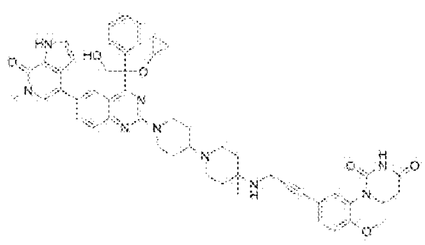

To a solution of the 4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 144-3 (35 mg, 51 μmol), the 1-(5-iodo-2-methoxyphenyl)dihydropyrimidine-2,4(1H,3H)-dione of Production Example 101-10 (35.3 mg, 102 μmol) and CuI (3.9 mg, 20 μmol) in DMF (385 μL) there were added N,N-diisopropylethylamine (454 μL, 2.55 mmol) and tetrakis(triphenylphosphine)palladium(0) (23.6 mg, 20 μmol), and the mixture was stirred for 2 hours at 40°C under a nitrogen atmosphere. The reaction mixture was concentrated under reduced pressure and the residue was purified by ODS silica gel chromatography (0.1% formic acid aqueous solution:0.1% acetonitrile formate solution = 97:3 to 0:100), to obtain a crude product. The crude product was purified with a fully automatic fractionation LC system (0.1% formic acid aqueous solution:0.1% acetonitrile formate solution = 7:3 to 1:1) to obtain a formate of the title compound (11 mg).

$^1$H-NMR Spectrum (500 MHz, $CD_3OD$) δ(ppm): 0.14-0.23 (1H, m), 0.24-0.37 (1H, m), 0.37-0.45 (1H, m), 0.71-0.81 (1H, m), 1.71-1.88 (4H, m), 1.93-2.05 (2H, m), 2.18-2.28 (2H, m), 2.72-2.76 (2H, m), 3.03-3.22 (5H, m), 3.26-3.28 (6H, m), 3.37-3.48 (3H, m), 3.60-3.66 (5H, m), 3.69 (2H, s), 3.81 (3H, s), 4.44 (1H, d, J=12.2 Hz), 5.16 (2H, d, J=12.9 Hz), 5.92 (1H, d, J=3.1 Hz), 7.00 (1H, s), 7.02 (1H, d, J=8.6 Hz), 7.20-7.28 (4H, m), 7.31-7.38 (4H, m), 7.58 (1H, d, J=9.2 Hz), 7.73-7.76 (1H, m), 8.20 (1H, d, J=1.9 Hz), 8.29-8.45 (2H, m) ESI-MS (m/z): 904.64 [M+H]+.

[Production Example 144-1]

4-(2-Chloro-4-(1-cyclopropoxy-2-hydroxy-1-phenylethyl)quinazolin-6-yl)-6-methyl-1-tosyl-1,6-dihydro-7H-pyrrolo[2,3-c]pyridin-7-one

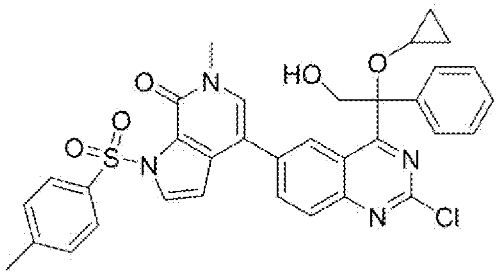

To a solution of the 4-(2-chloro-4-(1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 104-4 (100 mg, 138 μmol) in dichloromethane (5 mL) there was added TFA(1.06 mL), and the mixture was stirred for 1 hour. An aqueous sodium hydrogencarbonate solution was added to the reaction mixture, which was then extracted twice with dichloromethane. The organic layer was dried over sodium sulfate and filtered. The filtrate was concentrated to obtain the title compound (109 mg).

ESI-MS (m/z): 641.16 $[M+H]^+$.

[Production Example 144-2]

4-(4-(1-Cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1-tosyl-1,6-dihydro-7H-pyrrolo[2,3-c]pyridin-7-one

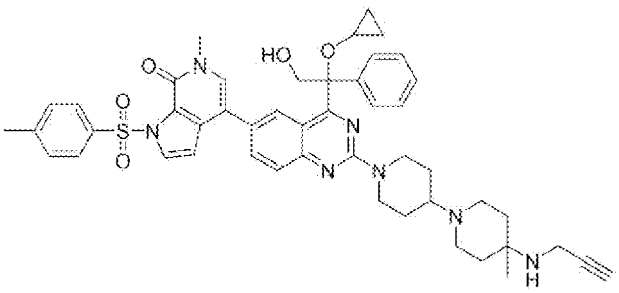

To a solution of the *tert*-butyl 4-methyl-4-(prop-2-yn-1-ylamino)-[1,4'-bipiperidine]-1'-carboxylate of Production Example 101-7 (610 mg, 90% purity, 1.64 mmol) in dichloromethane (3.44 mL) there was added TFA (3.37 mL), and the mixture was stirred for 30 minutes at room temperature. The reaction mixture was concentrated under reduced pressure and processed by azeotropic distillation with toluene, to obtain a crude product. To a solution of the obtained crude product in DMF (9.47 mL) there were added N,N-diisopropylethylamine (2.86 mL) and the 4-(2-chloro-4-(1-cyclopropoxy-2-hydroxy-1-phenylethyl)quinazolin-6-yl)-6-methyl-1-tosyl-1,6-dihydro-7H-pyrrolo[2,3-c]pyridin-7-one of Production Example 144-1 (700 mg, 1.09 mmol, azeotropic distillation with toluene before use), and the mixture was stirred for 1 hour at 85°C. To the reaction mixture there was added N,N-diisopropylethylamine (0.95 mL), and the mixture was stirred for 3 hours at 85°C. The reaction mixture was cooled to room temperature and concentrated under reduced pressure to 1/4 volume, and then ethyl acetate and an aqueous sodium hydrogencarbonate solution were added. After oil-water distribution, the aqueous layer was extracted with ethyl acetate. The organic layer was dried over sodium sulfate and filtered, and the filtrate was concentrated under reduced pressure. The residue was purified by NH silica gel column chromatography (n-heptane:ethyl acetate = 1:1 to ethyl acetate to ethyl acetate:methanol = 7:3). It was further purified by silica gel column chromatography (ethyl acetate to ethyl acetate:methanol = 1:1) to obtain the title compound (569 mg).

ESI-MS (m/z): 840.42 $[M+H]^+$.

[Production Example 144-3]

4-(4-(1-Cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one

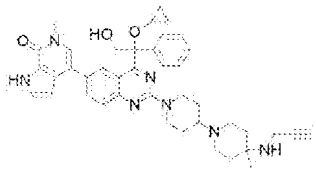

To a solution of the 4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1-tosyl-1,6-dihydro-7H-pyrrolo[2,3-c]pyridin-7-one of Production Example 144-2 (569 mg, 677 μmol) in ethanol (13.8 mL) there was added a sodium hydroxide aqueous solution (1 M, 2.71 mL, 2.71 mmol), and the mixture was stirred for 20 hours at room temperature. After diluting the reaction mixture with 5 mL of water, it was extracted twice with a 10% methanol/chloroform solution. After washing the organic layer with brine (10 mL), it was dried over sodium sulfate and filtered, and the filtrate was concentrated under reduced pressure. Ethyl acetate (5 mL) was added to the residue and the precipitated solid was filtered. The obtained solid was further washed with ethyl acetate (10 mL) to obtain the title compound (360 mg). A5 mg portion was purified by NH silica gel thin-layer chromatography (ethyl acetate:methanol = 9:1) to obtain the title compound (4.3 mg).

ESI-MS (m/z): 686.41 $[M+H]^+$.

[Example 145]

(R)-1-(5-(3-((1'-(4-(1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)dihydropyrimidine-2,4(1H,3H)-dione

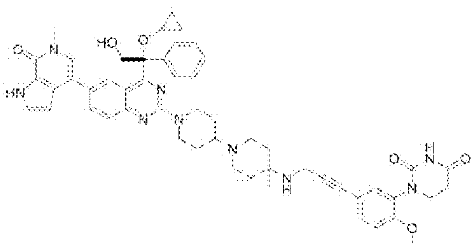

The title compound (9.1 mg) was obtained from the (R)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 145-4 (54 mg, 79 μmol), by the same method as Example 101.

ESI-MS (m/z): 904.49 $[M+H]^+$.

[Production Example 145-1]

2-Chloro-4-((1R)-1-cyclopropoxy-1-phenyl-2-((tetrahydro-2H-pyran-2-yl)oxy)ethyl)-6-iodoquinazoline

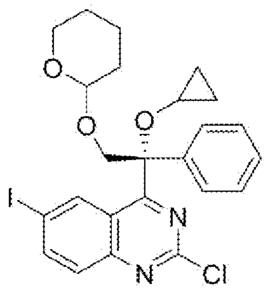

The title compound (1.0 g, 90% purity) was obtained from the ethyl (R)-2-(2-chloro-6-iodoquinazolin-4-yl)-2-cyclopropoxy-2-phenylacetate of Production Example 101-3 (900 mg, 1.77 mmol), by the same method as Production Example 104-3.

ESI-MS (m/z): 551.17 $[M+H]^+$.

[Production Example 145-2]

4-(2-Chloro-4-((1R)-1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one

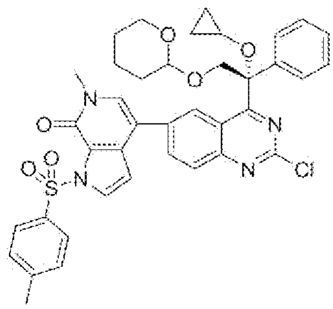

The title compound (960 mg) was obtained from the 2-chloro-4-((1R)-1-cyclopropoxy-1-phenyl-2-((tetrahydro-2H-pyran-2-yl)oxy)ethyl)-6-iodoquinazoline of Production Example 145-1 (950 mg, 90% purity, 1.55 mmol), by the same method as Production Example 101-5. ESI-MS (m/z): 725.38 $[M+H]^+$.

[Production Example 145-3]

4-(4-((1R)-1-Cyclopropoxy-1-phenyl-2-((tetrahydro-2H-pyran-2-yl)oxy)ethyl)-2-(4-methyl-4-(prop-2-yn-1-ylamino)-[1,4'-bipiperidin]-1'-yl)quinazolin-6-yl)-6-methyl-1-tosyl-1,6-dihydro-7H-pyrrolo[2,3-c]pyridin-7-one

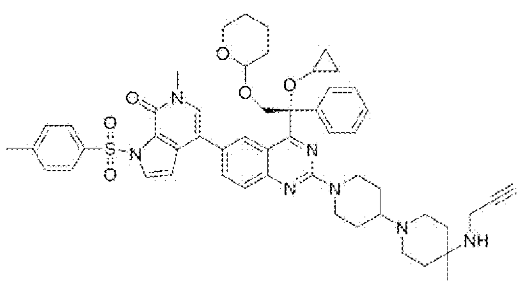

The title compound (91 mg) was obtained from the 4-(2-chloro-4-((1R)-1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 145-2 (100 mg, 138 μmol), by the same method as Production Example 101-8.

ESI-MS (m/z): 924.71 $[M+H]^+$.

[Production Example 145-4]

(R)-4-(4-(1-Cyclopropoxy-2-hydroxy-1-phenylethylethyl)-2-(4-methyl-4-(prop-2-yn-1-ylamino)-[1,4'-bipiperidin]-1'-yl) quinazolin-6-yl)-6-methyl-1,6-dihydro-7H-pyrrolo[2,3-c]pyridin-7-one

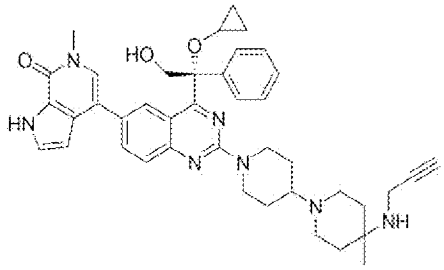

The title compound (54 mg) was obtained from the 4-(4-((1R)-1-cyclopropoxy-1-phenyl-2-((tetrahydro-2H-pyran-2-yl)oxy)ethyl)-2-(4-methyl-4-(prop-2-yn-1-ylamino)-[1,4'-bipiperidin]-1'-yl)quinazolin-6-yl)-6-methyl-1-tosyl-1,6-dihydro-7H-pyrrolo[2,3-c]pyridin-7-one of Production Example 145-3 (81 mg, 88 μmol), by the same method as Production Example 103-5.

ESI-MS (m/z): 686.58 $[M+H]^+$.

[Test Example 1: AsPC-1/Cas9 growth inhibition assay]

The human pancreatic cancer cell line AsPC-1 (ATCC Number CRL1682) was infected with Edit-R Lentiviral hCMV-Blast-Cas9 Nuclease Particle (Dharmacon, VCAS10124) in the presence of 8 μg/ml of Hexadimethrine bromide (Sigma, H9268), and after 24 hours it was exposed to 10 μg/ml of Blastcidin S (Thermo, A11139) to remove the uninfected cells, thereby establishing an AsPC-1/Cas9 cell line. The cells were maintenance cultured in a 5% $CO_2$ incubator (37°C) using RPMI-1640 (FujiFilm-Wako Pure Chemical Industries) containing 10% fetal bovine serum (FBS: SIGMA 173012) and penicillin/streptomycin (FujiFilm-Wako Pure Chemical Industries) (hereunder referred to simply as "culture medium"). After adding 45 μL of AsPC-1/Cas9 cell suspension prepared to $2.0 \times 10^4$ cells/mL using culture medium into each well of a 384-well plate (Greiner 781091), the cells were cultured overnight in a 5% $CO_2$ incubator (37°C). On the following day, 5 μL of the test substance diluted with culture medium was added to each well, and culturing was continued for 5 days in a 5% $CO_2$ incubator (37°C). Next, 25 μL of Cell Titer Glo™ 2.0 (Promega) was added to each well, and the plate contents were mixed for 5 minutes using a plate mixer. The plate was stationed for 10 minutes at room temperature while shielded from light, and then the luminous intensity was measured with an ENVISION™ (Perkin-Elmer). The luminescence value of the wells with addition of test substance was determined as a relative value, with 100% as the luminescence value of the wells with cells but without addition of the test substance, and 0% as the luminescence value of the wells without cells. A plot of test substance concentration against luminescence value % approximated a 4-parameter logistic curve, and the test substance concentration necessary for 50% inhibition of cell growth (Absolute $IC_{50}$) was calculated, and is shown in Table 4.

[Test Example 2: BET BD1 domain degradation assay]

HEK293/LgBiT/hPGK/HiBiT-BRD4-BD1 cells were established by introducing LgBiT and HiBiT-BRD4-BD1 into the human fetus renal cell line HEK293 (ATCC Number CRL-1573). LgBiT was introduced using lentivirus vector LU [Exp]-Bsd-EFS>{LgBiT}, and HiBiT-BRD4-BD1 was introduced with lentivirus vector LV[Exp]-Puro-hPGK> {HiBit-linker-BRD4_BD1(1-209)}. The vector plasmids LV[Exp]-Bsd-EFS>{LgBiT} and LU[Exp]-Puro-hPGK>{HiBit-linker-BRD4_BD1(1-209)} were constructed by VectorBuilder Co. The lentivirus was produced using a Mission Lentivirus Packaging Mix (Sigma) according to the manufacturer's protocol. Viral infection and drug selection of infected cells were carried out by the following published method (Akagi T. et.al, PNAS 2003; 100:13567-72). The cells were maintenance cultured in a 5% $CO_2$ incubator (37°C), using culture medium. A 45 μL portion of cell suspension of HEK293/LgBiT/hPGK/HiBiT-BRD4-BD1 cells prepared to $6.7 \times 10^4$ cells/mL using culture medium was added to each well of a collagen I-coated 384-well plate (Falcon 356667), and the cells were cultured overnight in a 5% $CO_2$ incubator (37°C). On the following day, 5 μL of test substance diluted with RPMI-1640 (FujiFilm-Wako Pure Chemical Industries) containing 10% fetal bovine serum (FBS: SIGMA 173012) and penicillin/streptomycin (FujiFilm-Wako Pure Chemical Industries) was added to each well, and culturing was continued for 3 hours in a 5% $CO_2$ incubator (37°C). The luminous intensity was

measured using a Nono-Glo HiBiT Lytic Detection System (Promega), and the intracellular HiBiT tagged BRD4-BD1 protein level was calculated. After culturing, 25 μL of measuring reagent was added to each well and the plate contents were mixed for 5 minutes using a plate mixer. The plate was stationed for 10 minutes at room temperature while shielded from light, and then the luminous intensity was measured with an ENVISION™ (Perkin-Elmer). The luminescence value of the wells with addition of test substance was determined as a relative value, with 100% as the luminescence value of the wells with cells but without addition of the test substance, and 0% as the luminescence value of the wells without cells. A plot of test substance concentration against luminescence value % approximated a 4-parameter logistic curve, and the test substance concentration necessary for 50% reduction in protein level (Absolute $DC_{50}$) was calculated and is shown in Table 4.

[Table 4]

| | Absolute $IC_{50}$_Day5 (nM) AsPC-1 Cas9 | Absolute $DC_{50}$_3h (nM) HEK293 HiBiT-BRD4-BD1 |
|---|---|---|
| Example 101 | 0.087 | 0.083 |
| Example 103 | 0.073 | 0.045 |
| Example 104 | 0.073 | 0.150 |
| Example 108 | 0.230 | 0.210 |
| Example 110 | 0.170 | 0.160 |
| Example 114 | 0.110 | 0.330 |
| Example 115 | 0.110 | 0.150 |
| Example 119 | 0.230 | 1.600 |
| Example 120 | 0.580 | 2.900 |
| Example 122 | 4.900 | 0.150 |
| Example 125 | 0.200 | 0.190 |
| Example 126 | 0.084 | 0.050 |
| Example 127 | 0.170 | 0.600 |
| Example 128 | 0.320 | 0.250 |
| Example 130 | 0.980 | 0.190 |
| Example 131 | 4.500 | 11.000 |
| Example 135 | 2.400 | >100 |
| Example 138 | 41.000 | >100 |
| Example 144 | 0.130 | 0.110 |
| Example 145 | 0.180 | 0.045 |

[Linker and drug bonding compounds in antibody-drug conjugate]

Unless otherwise specified, the microwave reactor used in the Production Examples and Examples was Biotage Initiator+, by Biotage.

Unless otherwise specified, the purifying silica gels used in silica gel column chromatography for the Production Examples and Examples were YMC GEL SILICA (YMC Co., Ltd., catalog code: SL06I52W), Silica Gel 60 (Kanto Chemicals), Silica Gel Spheres Fuji Silysia Chemical Ltd., catalog code: PSQ60B), Silica Gel 60 (Merck KGaA, catalog code: 1.07734), CHROMATOREX BW (Fuji Silysia Chemical Ltd., catalog code: BW-300), Hi-Flash Column (Yamazen Corporation) or Presep Silica Gel (Wako), and the purifying silica gels used for NH silica gel column chromatography were NH Silica Gel (Fuji Silysia Chemical Ltd., catalog code: NH-DM2035), Hi-Flash Column Amino (Yamazen Corporation) or Presep NH2 HC (Wako), and the purifying silica gel used for reversed-phase silica gel column chromatography or ODS silica gel column chromatography was Hi-Flash Column ODS (Yamazen Corporation). The purifying TLC plate used for silica gel thin-layer chromatography was TLC Silica Gel $60F_{254}$ (Merck KGaA, catalog code: 1.05715 or 1.05744), and the purifying PLC plate used for NH silica gel thin-layer chromatography was a CHROMATOREX NH-PLC05 plate (Fuji Silysia Chemical Ltd., catalog code: NH-PLC05). The solid-phase extraction column used was a Presep (Wako Pure Chemical Industries, Ltd., diatomaceous earth, granular).

Unless otherwise specified, a UFPLC (Shimadzu Ultra Fast Preparative LC system) or a fully automatic fractionation LC system (Waters MassLynx MS, Fractionation System) was used for fractionating purification in the Production Examples and Examples. The column used was an Xbridge Prep C18 5 μm OBD (19 mm × 100 mm) by Waters.

Unless otherwise specified, supercritical fluid chromatography (SFC) (Waters SFC 350) was used for chiral resolution in the Production Examples and Examples.

A Varian Mercury 400, Varian Mercury Plus 400, JEOL 400, JEOL 500 or Avance Neo 700 (Bruker) was used for nuclear magnetic resonance spectrum analysis. The chemical shifts in the proton nuclear magnetic resonance spectra are recorded in δ units (ppm) with respect to tetramethylsilane, and the coupling constants are recorded in Hertz (Hz). The abbreviations for the splitting patterns are the following. s: singlet, d: doublet, t: triplet, q: quartet, quin: quintet, spt: septet, m: multiplet, brs: broad singlet.

Mass spectral analysis was performed using a Waters UPLC™. The ionization method used was electrospray ionization (ESI).

Commercial products were used as appropriate for the compounds in the Production Examples and Examples. The abbreviations used below have the following meanings.

*n*-heptane: normal-Heptane
$CDCl_3$: Deuterated chloroform
CuI: Copper(I) iodide
CSA: (1S)(+)-10-Camphorsulfonic acid
DABCO: 1,4-Diazabicyclo[2.2.2]octane
DBU: 1,8-Diazobicyclo[5.4.0]undec-7-ene
DCM: Dichloromethane
DHP: 3,4-Dihydro-2H-pyran
DIBAL-H: Diisobutyl hydridealuminum
DMSO-$d_6$: Deuterated dimethyl sulfoxide
DMSO: Dimethyl sulfoxide
DMF: N,N-Dimethylformamide
HATU: N,N,N',N'-Tetramethyl-O-(7-azobenzotriazol-1-yl)uronium hexafluorophosphate
LHMDS: Lithiumhexamethyl disilazide
LDA: Lithium diisopropylamide
NMP: 1-Methyl 2-pyrrolidinone
TBME: *tert-Butyl* methyl ether
TFA: Trifluoroacetic acid
THF: Tetrahydrofuran
TSTU: N,N,N',N'-Tetramethyl-O-(N-succinimidyl)uronium tetrafluoroborate

[Example 201]

(S)-3-Acetamide-4-(((3-(5-(3-((1'-(4-((44S,50S,59S)-50-benzyl-59-cyclopropoxy-44-(3-(2-(2-(2,5-dioxo-3,4-bis(phenylthio)-2,5-dihydro-1H-pyrrol-1-yl)ethoxy)ethoxy)propanamido)-38,45,48,51,54-pentaoxo-59-phenyl-2,5,8,11,14,17,20,23,26,29,32,35,57-tridecaoxa-39,46,49,52,55-pentaazanonapentacontan- 59-yl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid

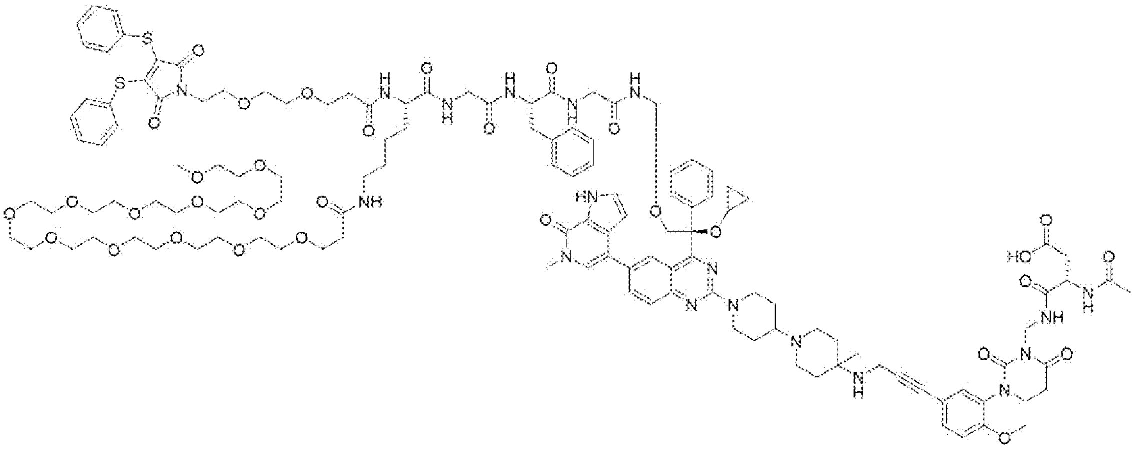

To a solution of the (S)-3-acetamide-4-(((3-(5-(3-((1'-(4-((44S,50S,59S)-44-amino-50-benzyl-59-cyclopropoxy-38,45,48,51,54-pentaoxo-59-phenyl-2,5,8,11,14,17,20,23,26,29,32,35,57-tridecaoxa-39,46,49,52,55-pentaazanonapentacontan-59-yl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid of Production Example 201-20 (175 mg, 0.084 mmol) in DMF (1.6 mL) there was added a solution of N,N-diisopropylethylamine (73.5 μL, 0.421 mmol) and the 2,5-dioxopyrrolidin-1-yl 3-(2-(2-(2,5-dioxo-3,4-bis(phenylthio)-2,5-dihydro-1H-pyrrol-1-yl)ethoxyethoxy)propanocate of Production Example 201-23 (86 mg, 0.15 mmol) in DMF (1.0 mL), and the mixture was stirred for 1 hour at room temperature. To this reaction mixture there was added the 2,5-dioxopyrrolidin-1-yl 3-(2-(2-(2,5-dioxo-3,4-bis(phenylthio)-2,5-dihydro-1H-pyrrol-1-yl)ethoxy)ethoxy)propanoate of Production Example 201-23 (10 mg, 0.018 mmol), and the mixture was stirred for 1 hour at room temperature. The reaction mixture was purified a total of 4 times by direct reversed-phase silica gel chromatography (0.1% formic acid aqueous solution:0.1% acetonitrile formate solution = 90:10 to 60:40), and then dissolved in a 10% methanol dichloromethane solution, after which heptane was added, and the mixture was concentrated to solidification to obtain a formate of the title compound (81 mg). $^1$H-NMR Spectrum (700 MHz, DMSO-$d_6$) δ(ppm): 0.21-0.29 (2H, m), 0.39-0.46 (1H, m), 0.67-0.73 (1H, m), 1.08 (3H, s), 1.16-1.37 (4H, m), 1.41-1.67 (8H, m), 1.81 (3H, s), 1.91-2.00 (2H, m), 2.27 (2H, t, J=6.6 Hz), 2.28-2.35 (1H, m), 2.35- 2.43 (2H, m), 2.52-2.60 (3H, m), 2.69-2.81 (6H, m), 2.94-3.08 (6H, m), 3.22 (3H, s), 3.35-3.60 (64H, m), 3.65-3.75 (4H, m), 3.80 (3H, s), 4.12-4.17 (1H, m), 4.28 (1H, brd, J=11.0 Hz), 4.37-4.42 (1H, m), 4.46-4.53 (3H, m), 4.77 (1H, brd, J=11.0 Hz), 4.86-4.95 (2H, m), 4.97-5.03 (1H, m), 5.07-5.13 (1H, br dd, J=12.3, 5.5 Hz), 5.89 (1H, m), 7.09 (1H, d, J=8.5 Hz), 7.15 (1H, m), 7.17-7.31 (21H, m), 7.32-7.37 (2H, m), 7.53 (1H, d, J=8.7 Hz), 7.73 (1H, t, J= 5.5 Hz), 7.78 (1H, dd, J=8.9, 1.8 Hz), 7.98-8.10 (4H, m), 8.12 (1H, d, J=7.9 Hz), 8.14-8.19 (1H, m), 8.25 (1H, m), 8.42 (1H, m), 12.07 (1H, brs). ESI-MS (m/z): 1268.67 [(M+2H)/2]$^+$.

[Production Example 201-1]

Ethyl 2-cyclopropoxy-2-phenylacetate

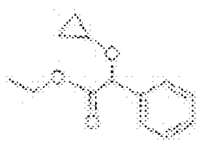

To a solution of cyclopropanol (3.36 g, 57.8 mmol) and rhodium(II) acetate dimer (320 mg, 0.723 mmol) in dichloromethane (20 mL) there was added dropwise a solution of ethyl 2-diazo-2-phenylacetate (5.50 g, 28.9 mmol) in dichloromethane (10 mL), and the mixture was stirred for 4 hours at room temperature. The reaction mixture was concentrated under reduced pressure and the residue was purified by silica gel column chromatography (n-heptane to n-heptane:ethyl acetate = 4:1) to obtain the title compound (5.83 g).

$^1$H-NMR Spectrum (500 MHz, $CDCl_3$) δ(ppm): 0.43-0.56 (2H, m), 0.64-0.78 (2H, m), 1.19-1.24 (3H, m), 3.38-3.45 (1H, m), 4.10-4.26 (2H, m), 4.94 (1H, s), 7.29-7.38 (3H, m), 7.42-7.47 (2H, m).

[Production Example 201-2]

Ethyl 2-(2-chloro-6-iodoquinazolin-4-yl)-2-cyclopropoxy-2-phenylacetate

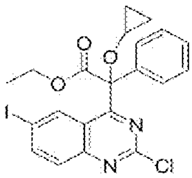

To a solution of the ethyl 2-cyclopropoxy-2-phenylacetate of Production Example 201-1 (2.5 g, 11 mmol) and 2,4-dichloro-6-iodoquinazoline (4.06 g, 12.5 mmol) in THF (90 mL) there was added LHMDS (1.26 M THF solution, 14.4 mL, 18.2 mmol) at -78°C, and the mixture was stirred for 10 minutes. The reaction mixture was then stirred for 3 hours at room temperature. A saturated aqueous ammonium chloride solution was added to the reaction mixture, and then the mixture was diluted with ethyl acetate and water and extracted with ethyl acetate. The organic layer was washed with brine. The organic layer was dried over magnesium sulfate and filtered. The filtrate was concentrated under reduced pressure and the residue was purified by silica gel column chromatography (n-heptane:ethyl acetate = 95:5 to 85:15) to obtain the title compound (4.8 g).
$^1$H-NMR Spectrum (500 MHz, $CDCl_3$) $\delta$(ppm): 0.04-0.10 (1H, m), 0.11-0.18 (m, 1H), 0.45-0.57 (1H, m), 0.69-0.78 (1H, m), 1.27 (3H, t, J=7.0 Hz), 3.69-3.82 (1H, m), 4.21-4.31 (1H, m), 4.31-4.41 (1H, m), 7.29-7.39 (3H, m), 7.48-7.57 (2H, m), 7.62-7.72 (1H, m), 7.97-8.06 (1H, m), 8.34-8.44 (1H, m).
ESI-MS (m/z): 509.14 $[M+H]^+$.

[Production Example 201-3]

Ethyl (R)-2-(2-chloro-6-iodoquinazolin-4-yl)-2-cyclopropoxy-2-phenylacetate ethyl (S)-2-(2-chloro-6-iodoquinazolin-4-yl)-2-cyclopropoxy-2-phenylacetate

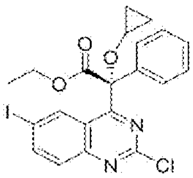

(R)-isomer

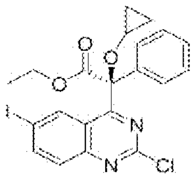

(S)-isomer

The ethyl 2-(2-chloro-6-iodoquinazolin-4-yl)-2-cyclopropoxy-2-phenylacetate of Production Example 201-2 (100 g, 197 mmol) was fractionated by SFC (Waters SFC 350, Whelk-O1(250*50 mm), carbon dioxide:isopropyl alcohol = 70:30), to obtain the R-form of the title compound (48.0 g, 94.3 mmol, 100% ee) and the S-form of the title compound (48.0 g, 94.3 mmol, 99% ee).
(R)-isomer, $^1$H-NMR Spectrum (500 MHz, $CDCl_3$) $\delta$(ppm): 0.03-0.10 (1H, m), 0.11-0.18 (m, 1H), 0.44-0.53 (1H, m), 0.69-0.77 (1H, m), 1.27 (3H, t, J=7.2 Hz), 3.69-3.77 (1H, m), 4.20-4.30 (1H, m), 4.30-4.40 (1H, m), 7.29-7.38 (3H, m), 7.46-7.55 (2H, m), 7.62-7.70 (1H, m), 7.97-8.05 (1H, m), 8.34-8.42 (1H, m).
(S)-isomer, $^1$H-NMR Spectrum (500 MHz, $CDCl_3$) $\delta$(ppm): 0.02-0.10 (1H, m), 0.11-0.20 (1H, m), 0.43-0.59 (1H, m), 0.66-0.79 (1H, m), 1.27 (3H, t, J=6.9 Hz), 3.69-3.78 (1H, m), 4.21-4.30 (1H, m), 4.30-4.39 (1H, m), 7.29-7.37 (3H, m), 7.48-7.55 (2H, m), 7.64-7.68 (1H, m), 7.99-8.04 (1H, m), 8.36-8.41 (1H, m).
(R)-isomer, ESI-MS (m/z): 509.14 $[M+H]^+$.
(S)-isomer, ESI-MS (m/z): 509.14 $[M+H]^+$.

[Production Example 201-4]

(S)-2-(2-Chloro-6-iodoquinazolin-4-yl)-2-cyclopropoxy-2-phenylethan-1-ol

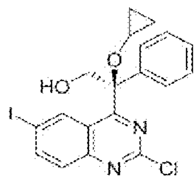

To a solution of the ethyl (S)-2-(2-chloro-6-iodoquinazolin-4-yl)-2-cyclopropoxy-2-phenylacetate of Production Example 201-3 (10 g, 20 mmol) in toluene (147 mL) there was added dropwise DIBAL-H (1.0 M toluene solution, 47.2 mL, 47.2 mmol) at -78°C over a period of 20 minutes. The reaction mixture was increased to 0°C and stirred for 10 minutes, after which it was quenched with a saturated aqueous Rochelle salt solution (100 mL), ethyl acetate (100 mL) was added, and the mixture was stirred for 2 hours at room temperature. The organic layer was separated off, and the aqueous layer was extracted with ethyl acetate (100 mL). The organic layers were combined and washed with brine (100 mL), and then dried over sodium sulfate. After filtration and concentration, the residue was purified by silica gel column chromatography (n-heptane:ethyl acetate = 9:1 to 7:3) to obtain a crude product of the title compound (8.8 g).
$^1$H-NMR Spectrum (500 MHz, $CDCl_3$) δ(ppm): 0.25-0.41 (2H, m), 0.51-0.59 (1H, m), 0.76-0.83 (1H, m), 2.50-2.59 (1H, m), 3.21-3.29 (1H, m), 4.50-4.66 (2H, m), 7.25-7.33 (5H, m), 7.61-7.69 (1H, m), 7.95-8.02 (1H, m), 8.61-8.69 (1H, m).
ESI-MS (m/z): 467.09 $[M+H]^+$.

[Production Example 201-5]

(S)-4-(2-Chloro-4-(1-cyclopropoxy-2-hydroxy-1-phenylethyl)quinazolin-6-yl)-6-methyl-1-tosyl-1,6-dihydro-7H-pyrrolo [2,3-c]pyridin-7-one

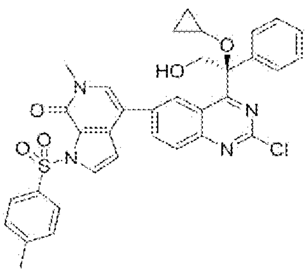

To a solution of a crude product of the (S)-2-(2-chloro-6-iodoquinazolin-4-yl)-2-cyclopropoxy-2-phenylethan-1-ol of Production Example 201-4 (6.30 g) and 6-methyl-4-(4,4,5,5-tetramethyl-1,3,2-dioxaborolan-2-yl)-1-tosyl-1,6-dihydro-7H-pyrrolo[2,3-c]pyridin-7-one (5.77 g, 13.5 mmol) in toluene (71 mL) there were added ethanol (18.0 mL) sodium carbonate (2 M aqueous solution, 35.3 mL, 70.5 mmol) and tetrakis(triphenylphosphine)palladium(0) (1.48 g, 1.28 mmol), and the mixture was stirred for 24 hours at 70°C under a nitrogen atmosphere. Water and ethyl acetate were added to the reaction mixture, and extraction was performed with ethyl acetate. The organic layer was dried over sodium sulfate, filtered and concentrated, and the residue was purified by silica gel column chromatography (n-heptane:ethyl acetate = 3:1 to ethyl acetate to ethyl acetate:methanol = 9:1) to obtain a crude product of the title compound (6.76 g).
$^1$H-NMR Spectrum (500 MHz, $CDCl_3$) δ(ppm): 0.18-0.38 (2H, m), 0.49-0.59 (1H, m), 0.71-0.83 (1H, m), 2.41 (3H, s), 2.70-2.81 (1H, m), 3.18-3.29 (1H, m), 3.52 (3H, s), 4.40-4.52 (1H, m), 4.64-4.76 (1H, m), 5.70-5.78 (1H, m), 6.84-6.90 (1H, m), 7.26-7.29 (2H, m), 7.29-7.35 (5H, m), 7.76-7.82 (1H, m), 7.82-7.87 (1H, m), 7.96-8.07 (3H, m), 8.14-8.26 (1H, m). ESI-MS (m/z): 641.23 $[M+H]^+$.

[Production Example 201-6]

4-Methyl-N-(prop-2-yn-1-yl)piperidine-4-amine

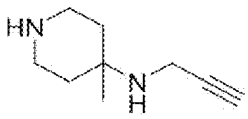

To a solution of tert-butyl 4-methyl-4-(prop-2-yn-1-ylamino)piperidine-1-carboxylate (10.3 g, 40.8 mmol) in ethyl acetate (50 mL) there was added a 4 M hydrochloric acid-ethyl acetate solution (102 mL, 408 mmol) at 0°C, and after stirring for 30 minutes, the mixture was further stirred overnight at room temperature. The reaction mixture was concentrated under reduced pressure and then purified by ODS silica gel column chromatography (0.1% formic acid aqueous solution:0.1% acetonitrile formate solution = 1:0), to obtain a formate of the title compound (7.16 g).
A 1 g portion was further purified by ODS silica gel column chromatography (0.1% ammonia water solution:0.1% ammonia-acetonitrile solution = 1:0 to 0:1) to obtain the title compound (230 mg) as an NMR sample.
$^1$H-NMR Spectrum (500 MHz, DMSO-$d_6$) δ(ppm): 1.00 (3H, s), 1.24-1.35 (2H, m), 1.38-1.49 (2H, m), 2.51-2.63 (2H, m), 2.70-2.86 (2H, m), 2.95 (1H, t, J=2.4 Hz), 3.19-3.31 (2H, m).
ESI-MS (m/z): 153.05 $[M+H]^+$.

[Production Example 201-7]

*tert*-Butyl 4-methyl-4-(prop-2-yn-1-ylamino)-[1,4'-bipiperidine]-1'-carboxylate

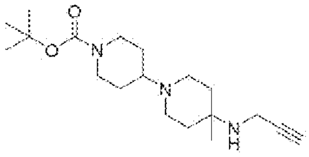

To a solution of the formate of 4-methyl-N-(prop-2-yn-1-yl)piperidine-4-amine of Production Example 201-6 (4.2 g) in tetrahydrofuran (30 mL) and dichloromethane (30 mL) there was added N,N-diisopropylethylamine (15 mL), and the mixture was stirred for 5 minutes at room temperature. After then adding BOC-4-piperidone (5.14 g, 25.8 mmol), the mixture was stirred for 1 hour at room temperature. The reaction mixture was cooled to 0°C, sodium triacetoxyborohydride (>80 wt%, 9.11 g) was added, and the mixture was stirred overnight at room temperature. The reaction mixture was quenched with water, and then ethyl acetate and 1 N hydrochloric acid were added. The aqueous layer was separated off and washed with ethyl acetate to remove the unreacted ketone and its reduced form. The aqueous layer was neutralized with a saturated aqueous sodium hydrogencarbonate solution and extracted 3 times with a chloroform:isopropyl alcohol = 4:1 solution. The organic layer was washed with brine and dried over magnesium sulfate. After filtering the organic layer, the filtrate was concentrated and the residue was purified by NH silica gel column chromatography (*n*-heptane:ethyl acetate = 75:25 to 0:100) to obtain the title compound (4.2 g).
$^1$H-NMR Spectrum (500 MHz, $CDCl_3$) δ(ppm): 1.10 (3H, s), 1.36-1.45 (2H, m), 1.46 (9H, s), 1.51-1.71 (5H, m), 1.74-1.87 (2H, m), 2.19 (1H, t, J=2.6 Hz), 2.34-2.44 (1H, m), 2.46-2.54 (2H, m), 2.55-2.62 (2H, m), 2.64-2.84 (2H, m), 3.34-3.41 (2H, m), 4.02-4.29 (2H, m). ESI-MS (m/z): 336.32 $[M+H]^+$.

[Production Example 201-8]

(S)-4-(4-(1-Cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1-tosyl-1,6-dihydro-7H-pyrrolo[2,3-c]pyridin-7-one

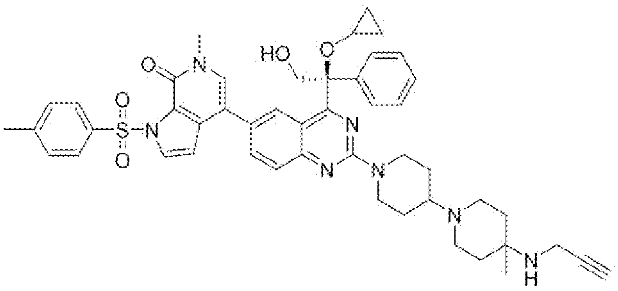

The tert-butyl 4-methyl-4-(prop-2-yn-1-ylamino)-[1,4'-bipiperidine]-1'-carboxylate of Production Example 201-7 (1.56 g, 4.65 mmol) was dissolved in dichloromethane (9.78 mL) and trifluoroacetic acid (9.56 mL) and the mixture was stirred for 30 minutes at room temperature, after which it was concentrated under reduced pressure (azeotropic distillation with addition of toluene) to obtain an intermediate. To the intermediate there were added DMF (26.9 mL), N,N-diisopropylethylamine (8.10 mL) and the crude product of the (S)-4-(2-chloro-4-(1-cyclopropoxy-2-hydroxy-1-phenylethyl)quinazolin-6-yl)-6-methyl-1-tosyl-1,6-dihydro-7H-pyrrolo[2,3-c]pyridin-7-one of Production Example 201-5 (2.21 g, azeotropic distillation with toluene before use). The mixture was stirred for 1 hour at 85°C, and then N,N-diisopropylethylamine (1.62 mL) was added. After further stirring for 2 hours, N,N-diisopropylethylamine (1.08 mL) was added and the mixture was stirred for 1 hour. After cooling to room temperature, the mixture was concentrated under reduced pressure to 1/4 volume, and ethyl acetate (50 mL) and an aqueous sodium hydrogencarbonate solution (30 mL) were added. The organic layer was separated off, and the aqueous layer was extracted twice with ethyl acetate (50 mL). The combined organic layers were dried over sodium sulfate and filtered, the filtrate was concentrated, and the residue was purified by NH silica gel column chromatography (n-heptane:ethyl acetate = 1:1 to ethyl acetate to ethyl acetate:methanol = 7:3) to obtain the title compound (1.91 g). $^1$H-NMR Spectrum (500 MHz, $CDCl_3$) δ(ppm): 0.21-0.29 (1H, m), 0.37-0.53 (2H, m), 0.68-0.77 (1H, m), 1.11 (3H, s), 1.59-1.67 (7H, m), 2.00-2.06 (2H, m), 2.17-2.20 (1H, m), 2.40 (3H, s), 2.53-2.72 (6H, m), 3.00-3.09 (2H, m), 3.18-3.24 (1H, m), 3.35-3.38 (2H, m), 3.50 (3H, s), 4.41-4.49 (1H, m), 4.56-4.64 (1H, m), 4.95-5.04 (2H, m), 5.72-5.76 (1H, m), 6.79 (1H, s), 7.27-7.35 (7H, m), 7.51-7.55 (1H, m), 7.55-7.61 (1H, m), 7.73-7.77 (1H, m), 7.89-7.95 (1H, m), 7.97-8.04 (2H, m).

ESI-MS (m/z): 840.42 $[M+H]^+$.

[Production Example 201-9]

(S)-4-(4-(1-Cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one

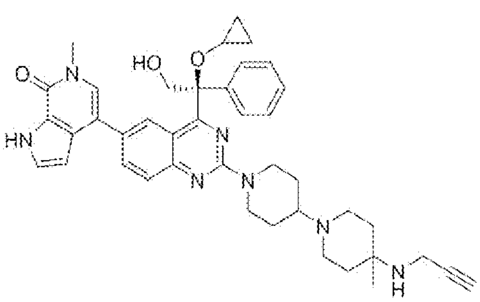

To a solution of the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1-tosyl-1,6-dihydro-7H-pyrrolo[2,3-c]pyridin-7-one of Production Example 201-8 (1.85 g, 2.20 mmol) in ethanol (44.9 mL) there was added a sodium hydroxide aqueous solution (1 M, 8.81 mL, 8.81 mmol), and the mixture was stirred for 18 hours at room temperature. After diluting the reaction mixture with 10 mL of water, it was extracted with chloroform (150 mL + 50 mL). After washing the combined organic layers with brine (30 mL), the mixture was dried over sodium sulfate and filtered, and the filtrate was concentrated under reduced pressure. Addition of ethyl acetate (10 mL) to the residue produced a solid, which was filtered. The obtained solid was further washed with ethyl acetate (10 mL) to obtain the title compound (1.25 g).

$^1$H-NMR Spectrum (500 MHz, $CDCl_3$) δ(ppm): 0.21-0.32 (1H, m), 0.38-0.55 (2H, m), 0.69-0.82 (1H, m), 1.11 (3H, s), 1.60-1.69 (7H, m), 1.98-2.09 (2H, m), 2.16-2.23 (1H, m), 2.54-2.77 (6H, m), 2.97-3.12 (2H, m), 3.19-3.28 (1H, m),

3.33-3.43 (2H, m), 3.62 (3H, s), 4.43-4.52 (1H, m), 4.56-4.66 (1H, m), 4.94-5.05 (2H, m), 5.87-5.92 (1H, m), 6.66-6.75 (1H, m), 7.10-7.18 (1H, m), 7.28-7.35 (5H, m), 7.55-7.64 (1H, m), 7.68-7.74 (1H, m), 8.00-8.08 (1H, m), 9.47-9.54 (1H, m).

ESI-MS (m/z): 686.58 $[M+H]^+$.

[Production Example 201-10]

(2-((((9H-Fluoren-9-yl)methoxy)carbonyl)amino)acetamide)methyl acetate

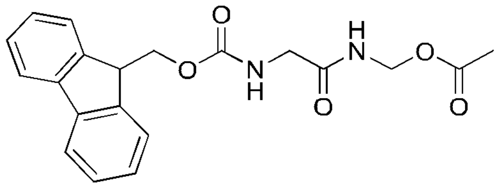

To a solution of (((9H-fluoren-9-yl)methoxy)carbonyl)glycylglycine (20 g, 56 mmol) in THF (555 mL), toluene (180 mL) and pyridine (5.5 mL) there was added lead tetraacetate (32.5 g, 73.4 mmol), and the mixture was stirred for 1 hour at 90°C. The reaction mixture was cooled to room temperature, filtered with Celite and washed with ethyl acetate (500 mL), and then water (300 mL) was added to the filtrate. The organic layer was separated off, and the aqueous layer was extracted with ethyl acetate (200 mL). The combined organic layers were washed with brine and then dried over anhydrous magnesium sulfate. After filtering the organic layer, the filtrate was concentrated under reduced pressure, and the residue was purified by silica gel column chromatography (*n*-heptane:ethyl acetate = 9:1 to 0:1) and then by silica gel column chromatography (*n*-heptane:ethyl acetate = 3:1 to 0:1), to obtain a crude product. The crude product was dissolved in ethyl acetate and diluted with an ethyl acetate:*n*-heptane = 4:1 solution, and then ultrasonic waves were applied to precipitate a solid. The obtained solid was washed with a mixed solvent of ethyl acetate and *n*-heptane and dried to obtain the title compound (15 g).

$^1$H-NMR Spectrum (500 MHz, $CDCl_3$) $\delta$(ppm): 2.02-2.15 (3H, m), 3.83-3.96 (2H, m), 4.24 (1H, t, J=6.9 Hz), 4.40-4.55 (2H, m), 5.20-5.32 (2H, m), 5.34-5.44 (1H, m), 6.95-7.16 (1H, m), 7.30-7.37 (2H, m), 7.38-7.45 (2H, m), 7.56-7.66 (2H, m), 7.74-7.83 (2H, m).

ESI-MS (m/z): 617.27$[2M-2OAc+H]^+$.

[Production Example 201-11]

(9H-Fluoren-9-yl)methyl (S)-(2-(((2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamate

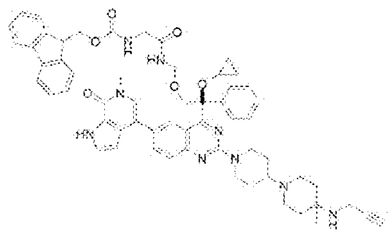

The (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 201-9 (500 mg, 0.729 mmol) and the (2-((((9H-fluoren-9-yl)methoxy)carbonyl)amino)acetamide)methyl acetate of Production Example 201-10 (1.07 g, 2.92 mmol) were suspended in chloroform (5 mL) and toluene (5 mL), and azeotropic distillation was performed twice. To a solution of the reaction mixture in chloroform (10 mL) and THF (10 mL) there was added CSA (677 mg, 2.92 mmol), and the mixture was stirred for 3 hours at room temperature. A saturated aqueous sodium hydrogencarbonate solution was added to the reaction mixture, and extraction was performed with dichloromethane. The organic layer was dried over sodium sulfate and filtered. The filtrate was concentrated under reduced pressure and the

residue was purified by reversed-phase silica gel chromatography (0.1% formic acid aqueous solution:0.1% acetonitrile formate solution = 95:5 to 0:100) to obtain a formate of the title compound (153 mg). The same procedure was carried out twice using (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one (1.0 g, 1.46 mmol) and (1.02 g, 1.49 mmol), to obtain formates of the title compound (499 mg) and (542 mg). The combined formates of the title compound were dissolved in chloroform (100 mL) and methanol (5 mL), and neutralized with a saturated aqueous sodium hydrogencarbonate solution. The aqueous layer was extracted with a chloroform:methanol = 20:1 solution, and the collected organic layer was dried over sodium sulfate and filtered. The filtrate was concentrated under reduced pressure to obtain the title compound (1.0 g).

$^1$H-NMR Spectrum (500 MHz, DMSO-$d_6$) δ(ppm): 0.20-0.25 (2H, m), 0.32-0.43 (1H, m), 0.61-0.72 (1H, m), 0.91 (3H, s), 1.24-1.30 (2H, m), 1.36-1.45 (4H, m), 1.84 (2H, d, J=15.0 Hz), 2.23-2.36 (2H, m), 2.40-2.62 (5H, m), 2.90-2.99 (3H, m), 3.00-3.06 (1H, m), 3.16-3.19 (2H, m), 3.49 (3H, s), 3.57 (1H, d, J=5.0 Hz), 4.18-4.21 (1H, m), 4.23-4.26 (2H, m), 4.34-4.37 (1H, m), 4.49-4.53 (1H, m), 4.74 (1H, d, J=15.0 Hz), 4.84 (2H, d, J=15.0 Hz), 5.84-5.85 (1H, m), 7.15-7.31 (8H, m), 7.36-7.39 (2H, m), 7.48-7.53 (2H, m), 7.68 (2H, d, J=5.0 Hz), 7.72-7.74 (1H, m), 7.80-7.86 (3H, m), 8.11-8.12 (1H, m), 8.56-8.59 (1H, m), 12.06 (1H, brs). ESI-MS (m/z): 994.81 $[M+H]^+$.

[Production Example 201-12]

(S)-44-((((9H-Fluoren-9-yl)methoxy)carbonyl)amino)-38-oxo-2,5,8,11,14,17,20,23,26,29,32,35-dodecaoxa-39-aza-pentatetracontan-45-oic acid

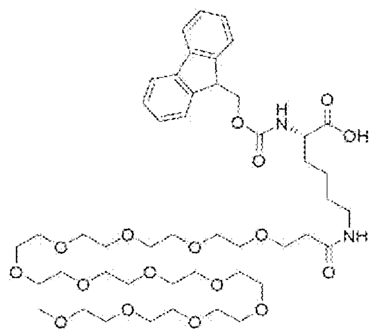

To a solution of FMOC-Lys-OH hydrochloride (1.04 g, 2.56 mmol) and 1-[(38-oxo-2,5,8,11,14,17,20,23,26,29,32,35-dodecaoxaoctatriacontan-38-yl)oxy]-2,5-pyrrolidinedione (1.81 g, 2.64 mmol) in DMF (6 mL) there was added N,N-diisopropylethylamine (1.34 mL, 7.68 mmol), and the mixture was stirred for 3 hours at room temperature. Half of the reaction mixture was purified by direct reversed-phase silica gel chromatography (0.1% formic acid aqueous solution:0.1% acetonitrile formate solution = 85:15 to 50:50). The remaining half was purified by the same procedure, to obtain the total for the title compound (2.18 g).

$^1$H-NMR Spectrum (500 MHz, $CDCl_3$) δ(ppm): 1.13-1.74 (4H, m), 1.77-1.99 (2H, m), 2.46 (2H, t, J=5.5 Hz), 3.21-3.30 (2H, m), 3.38 (3H, s), 3.53-3.58 (2H, m), 3.58-3.68 (m, 42H), 3.68-3.74 (2H, m), 4.19-4.26 (1H, m), 4.30-4.37 (1H, m), 4.38-4.46 (2H, m), 5.74 (1H, d, J=7.3 Hz), 6.78 (1H, t, J=5.5 Hz), 7.29-7.36 (2H, m), 7.37-7.44 (2H, m), 7.59-7.66 (2H, m), 7.74-7.80 (2H, m).

ESI-MS (m/z): 940.39 $[M+H]^+$.

[Production Example 201-13]

*tert*-Butyl ((S)-44-((((9H-fluoren-9-yl)methoxy)carbonyl)amino)-38-oxo-2,5,8,11,14,17,20,23,26,29,32,35-dodecaoxa-39-azapentatetracontan-45-oyl)glycyl-L-phenyl alaninate

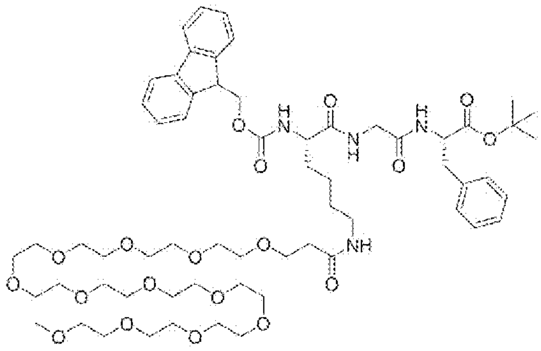

To a solution of the (S)-44-((((9H-fluoren-9-yl)methoxy)carbonyl)amino)-38-oxo-2,5,8,11,14,17,20,23,26,29,32,35-dodecaoxa-39-azapentatetracontan-45-oic acid of Production Example 201-12 (973 mg, 1.04 mmol), the known compound *tert*-butyl glycyl-L-phenyl alaninate (346 mg, 1.24 mmol) and N,N-diisopropylethylamine (271 μL, 1.55 mmol) in DMF (2.5 mL) there was added HATU (473 mg, 1.24 mmol) while cooling on ice, and the mixture was stirred for 1 hour. The reaction mixture was purified by direct reversed-phase silica gel chromatography (0.1% formic acid aqueous solution:0.1% acetonitrile formate solution = 4:1 to 3:7) to obtain the title compound (1.18 g).
$^1$H-NMR Spectrum (500 MHz, $CDCl_3$) δ(ppm): 1.26-1.42 (11H, m), 1.42-1.60 (2H, m), 1.67-1.81 (1H, m), 1.82-1.93 (1H, m), 2.44 (2H, t, J=5.5 Hz), 3.01-3.12 (2H, m), 3.18-3.30 (2H, m), 3.38 (3H, s), 3.52-3.67 (44H, m), 3.67-3.73 (2H, m), 3.83-3.93 (1H, m), 3.94-4.04 (1H, m), 4.07-4.16 (1H, m), 4.17-4.25 (1H, m), 4.35-4.43 (1H, m), 4.43-4.52 (1H, m), 4.66-4.76 (1H, m), 5.89 (1H, d, J=6.1 Hz), 6.63-6.73 (1H, m), 6.73-6.83 (1H, m), 6.94-7.04 (1H, m), 7.15 (2H, d, J=7.3 Hz), 7.17-7.23 (1H, m), 7.23-7.28 (2H, m), 7.28-7.34 (2H, m), 7.40 (2H, t, J=7.6 Hz), 7.61 (2H, t, J=5.8 Hz), 7.76 (2H, d, J=7.3 Hz) .
ESI-MS (m/z): 1200.47 $[M+H]^+$.

[Production Example 201-14]

((S)-44-((((9H-Fluoren-9-yl)methoxy)carbonyl)amino)-38-oxo-2,5,8,11,14,17,20,23,26,29,32,35-dodecaoxa-39-azapentatetracontan-45-oyl)glycyl-L-phenylalanine

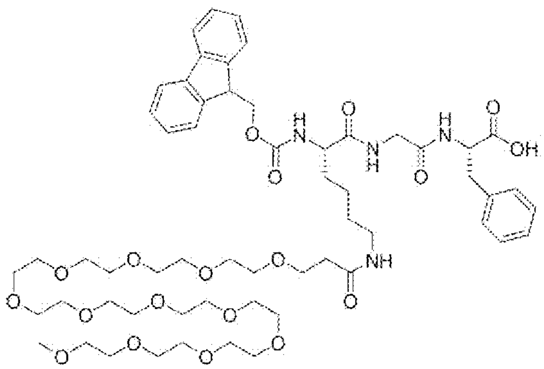

To a solution of the *tert-butyl* ((S)-44-((((9H-fluoren-9-yl)methoxy)carbonyl)amino)-38-oxo-2,5,8,11,14,17,20,23,26,29,32,35-dodecaoxa-39-azapentatetracontan-45-oyl)glycyl-L-phenyl alaninate of Production Example 201-13 (1.14 g, 0.950 mmol) in dichloromethane (3 mL) there was added TFA (3 mL), and the mixture was stirred for 2 hours at room temperature. The reaction mixture was concentrated under reduced pressure and the residue was purified by direct reversed-phase silica gel chromatography (0.1% formic acid aqueous solution:0.1% acetonitrile formate solution = 15:85 to 50:50) to obtain the title compound (971 mg). $^1$H-NMR Spectrum (500 MHz, $CDCl_3$) δ(ppm): 1.15-1.37 (2H, m), 1.37-1.55 (2H, m), 1.60-1.85 (2H, m), 2.43 (2H, t, J=5.8 Hz), 3.09-3.27 (4H, m), 3.36 (3H, s), 3.52-3.67 (44H, m), 3.67-3.73 (2H, m), 3.74-3.88 (1H, m), 4.00 (1H, dd, J=16.2, 6.4 Hz), 4.09-4.17 (1H, m), 4.19 (1H, t, J=6.7 Hz), 4.36-4.55 (2H, m), 4.71-4.83 (1H, m), 5.94 (1H, d, J=7.3 Hz), 6.92-7.03 (2H, m), 7.13-7.22 (3H, m), 7.23-7.33 (5H, m), 7.39 (2H, t, J=7.3 Hz), 7.59 (2H, d, J=7.3 Hz), 7.75 (2H, d, J=7.3 Hz).
ESI-MS (m/z): 1144.47 $[M+H]^+$.

[Production Example 201-15]

(9H-Fluoren-9-yl)methyl ((44S,50S,59S)-50-benzyl-59-cyclopropoxy-59-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-38,45,48,51,54-pentaoxo-59-phenyl-2,5,8,11,14,17,20,23,26,29,32,35,57-tridecaoxa-39,46,49,52,55-pentaazanonapentacontan-44-yl) carbamate

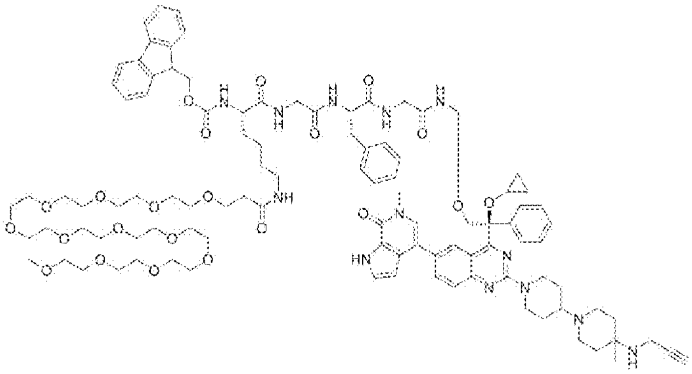

To a solution of the (9H-fluoren-9-yl)methyl (S)-(2-(((2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamate of Production Example 201-11 (150 mg, 0.151 mmol) in DMF (1 mL) there was added diethylamine (156 μL, 1.51 mmol), and the mixture was stirred for 20 minutes at room temperature. The reaction mixture was concentrated under reduced pressure and the residue was again dissolved in DMF (1 mL), after which the ((S)-44-((((9H-fluoren-9-yl)methoxy)carbonyl)amino)-38-oxo-2,5,8,11,14,17,20,23,26,29,32,35-dodecaoxa-39-azapentatetracontan-45-oyl)glycyl-L-phenylalanine of Production Example 201-14 (207 mg, 0.181 mmol) and N,N-diisopropylethylamine (79 μL, 0.45 mmol) were added. The reaction mixture was ice-cooled, and then HATU (68.8 mg, 0.181 mmol) was added and the mixture was stirred for 1 hour. The reaction mixture was purified by direct reversed-phase silica gel chromatography (0.1% formic acid aqueous solution:0.1% acetonitrile formate solution = 90:10 to 55:45) to obtain a formate of the title compound. The formate of the title compound was dissolved in a chloroform:2-propanol (10:1) solution, and then neutralized with a saturated aqueous sodium hydrogencarbonate solution. The mixture was extracted with chloroform:2-propanol (10:1), the combined organic layers were dried over magnesium sulfate and filtered, and the filtrate was concentrated under reduced pressure to obtain the title compound (149 mg).

$^1$H-NMR Spectrum (500 MHz, $CDCl_3$) δ(ppm): 0.23-0.31 (1H, m), 0.33-0.42 (1H, m), 0.43-0.55 (1H, m), 0.78-0.87 (1H, m), 1.12 (3H, s), 1.27-1.38 (2H, m), 1.39-1.53 (2H, m), 1.55-1.86 (8H, m), 2.00-2.10 (2H, m), 2.20 (2H, t, J=2.5 Hz), 2.36-2.45 (2H, m), 2.53-2.78 (5H, m), 2.92 (1H, dd, J=14.7, 9.8 Hz), 2.98-3.11 (2H, m), 3.13-3.30 (3H, m), 3.37 (3H, s), 3.37-3.42 (3H, m), 3.45-3.73 (49H, m), 3.75-3.83 (1H, m), 3.85-3.98 (3H, m), 4.11-4.17 (1H, m), 4.27 (1H, dd, J=10.7, 6.4 Hz), 4.39-4.60 (3H, m), 4.63-4.76 (2H, m), 4.94 (1H, d, J=11.0 Hz), 5.03-5.16 (2H, m), 6.08 (1H, t, J=2.5 Hz), 6.56-6.66 (1H, m), 6.71 (1H, s), 6.86-6.96 (1H, m), 7.08-7.34 (15H, m), 7.35-7.44 (4H, m), 7.47 (1H, d, J=7.3 Hz), 7.57 (1H, d, J=8.6 Hz), 7.61-7.70 (2H, m), 7.72-7.82 (2H, m), 8.12-8.18 (1H, m), 9.82-9.92 (1H, m).

ESI-MS (m/z): 949.37[(M+2H)/2]$^+$.

[Production Example 201-16]

1-(5-Iodo-2-methoxyphenyl)dihydropyrimidine-2,4(1H,3H)-dione

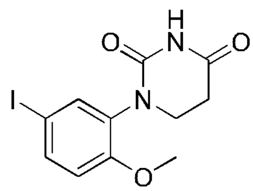

After adding acrylic acid (10.3 mL, 151 mmol) and acetic acid (20 mL) to 5-iodo-2-methoxyaniline (15 g, 60 mmol), the mixture was stirred for 3 hours at 110°C under a nitrogen atmosphere. After cooling to room temperature, urea (14.5 g, 241 mmol) and acetic acid (20 mL) were added and the mixture was stirred for 21.5 hours at 110°C under a nitrogen atmosphere. The reaction mixture was poured onto crushed ice and the mixture was stirred for 1 hour at room temperature. After applying ultrasonic waves to the mixture for 30 minutes, the obtained solid was collected and washed with water. A 6:1 mixed solvent of diethyl ether and ethyl acetate (400 mL) was added, and ultrasonic waves were applied for 20 minutes. The obtained solid was filtered out and washed with diethyl ether to obtain the title compound (12 g).

$^1$H-NMR Spectrum (500 MHz, DMSO-$d_6$) δ(ppm): 2.61-2.74 (2H, m), 3.55 (2H, t, J=6.5 Hz), 3.78 (3H, s), 6.95 (1H, d, J=9.0 Hz), 7.52-7.72 (2H, m), 10.33 (1H, s).

ESI-MS (m/z): 347.05 $[M+H]^+$.

[Production Example 201-17]

*tert*-Butyl (S)-3-((((9H-fluoren-9-yl)methoxy)carbonyl)amino)-4-((acetoxymethyl)amino)-4-oxobutanoate

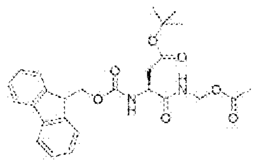

To a solution of (S)-(2-((((9H-fluoren-9-yl)methoxy)carbonyl)amino)-4-(*tert*-butoxy)-4-oxobutanoyl)glycine (3.20 g, 6.83 mmol) in THF (67.2 mL), toluene (22.6 mL) and pyridine (663 μL) there was added lead tetraacetate (3.79 g, 8.54 mmol), and the mixture was stirred for 1.5 hours at 110°C under a nitrogen atmosphere. The reaction mixture was cooled to room temperature, filtered with Celite and washed with ethyl acetate. The filtrate was concentrated under reduced pressure. The residue was purified by silica gel chromatography (heptane:ethyl acetate = 9:1 to 1:1) to obtain the title compound (2.10 g). $^1$H-NMR Spectrum (500 MHz, $CDCl_3$) δ(ppm): 1.44 (9H, s), 2.03 (3H, s), 2.53-2.66 (1H, m), 2.88-2.99 (1H, m), 4.22 (1H, t, J=6.7 Hz), 4.37-4.54 (3H, m), 5.18-5.27 (2H, m), 5.84-5.92 (1H, m), 7.29-7.33 (2H, m), 7.40 (2H, t, J=7.3 Hz), 7.44-7.49 (1H, m), 7.57 (2H, d, J=7.3 Hz), 7.76 (2H, d, J=7.3 Hz).

ESI-MS (m/z): 845.58$[2M-2OAc+H]^+$.

[Production Example 201-18]

*tert*-Butyl (S)-3((((9H-fluoren-9-yl)methoxy)carbonyl)amino)-4-(((3-(5-iodo-2-methoxyphenyl)-2,6-dioxotetrahydropyrimidine-1(2H)-yl)methyl)amino)-4-oxobutanoate

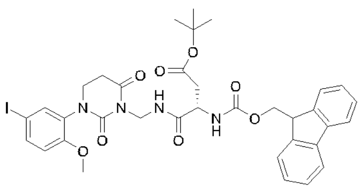

To a solution of the 1-(5-iodo-2-methoxyphenyl)dihydropyrimidine-2,4(1H,3H)-dione of Production Example 201-16 (900 mg, 2.60 mmol) in THF (25 mL) there was added potassium *tert*-butoxide (350 mg, 3.12 mmol) at 0°C, and the mixture was stirred for 10 minutes. To the reaction mixture there was added a solution of the *tert-butyl* (S)-3-((((9H-fluoren-9-yl)methoxy)carbonyl)amino)-4-((acetoxymethyl)amino)-4-oxobutanoate of Production Example 201-17 (1.38 g, 2.86 mmol) in THF (25 mL) at 0°C, and after warming to room temperature, the mixture was stirred for 40 minutes. The reaction mixture was cooled to 0°C and then quenched with an ammonium chloride aqueous solution. The reaction mixture was extracted with dichloromethane and the organic layer was dried over magnesium sulfate, after which it was filtered and the filtrate was concentrated under reduced pressure. The residue was purified by silica gel chromatography (heptane:ethyl acetate = 90:10 to 0:1) to obtain the title compound (1.75 g).

$^1$H-NMR Spectrum (500 MHz, $CDCl_3$) δ(ppm): 1.44 (9H, s), 2.52-2.67 (1H, m), 2.78-2.87 (2H, m), 2.88-3.00 (1H, m), 3.50-3.64 (2H, m), 3.78 (3H, s), 4.16-4.24 (1H, m), 4.32-4.44 (2H, m), 4.45-4.53 (1H, m), 5.31-5.37 (2H, m), 5.85-5.93

(1H, m), 6.70 (1H, d, J=9.0 Hz), 7.27-7.32 (2H, m), 7.33-7.42 (3H, m), 7.48-7.61 (4H, m), 7.72-7.79 (2H, m).
ESI-MS (m/z): 769.27 $[M+H]^+$.

[Production Example 201-19]

(S)-3-Acetamide-4-(((3-(5-iodo-2-methoxyphenyl)-2,6-dioxotetrahydropyrimidine-1(2H)-yl)methyl)amino)-4-oxobutanoic acid

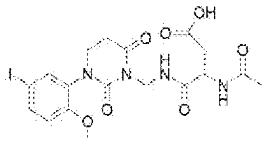

To a solution of the *tert-butyl* (S)-3((((9H-fluoren-9-yl)methoxy)carbonyl)amino)-4-(((3-(5-iodo-2-methoxyphenyl)-2,6-dioxotetrahydropyrimidine-1(2H)-yl)methyl)amino)-4-oxobutanoate of Production Example 201-18 (1.75 g, 2.28 mmol) in DMF (9.6 mL) there was added diethylamine (2.4 mL, 23 mmol), and the mixture was stirred for 30 minutes at room temperature. The reaction mixture was concentrated under reduced pressure, and then the residue was dissolved in THF (9 mL) and dichloromethane (18 mL), N,N-diisopropylethylamine (1.19 mL, 6.83 mmol) and acetic anhydride (323 μL, 3.42 mmol) were added and the mixture was stirred for 20 minutes at room temperature. After further adding acetic anhydride (32 μL, 0.34 mmol), the mixture was stirred for 10 minutes. The reaction mixture was concentrated under reduced pressure and the residue was purified by reversed-phase silica gel chromatography (0.1% formic acid aqueous solution:0.1% acetonitrile formate solution = 9:1 to 1:1), to obtain an intermediate. The intermediate was processed by azeotropic distillation with toluene to remove the water, and then dichloromethane (18 mL) and TFA (9 mL) were added and the mixture was stirred for 1 hour at room temperature. The reaction mixture was concentrated under reduced pressure and processed by azeotropic distillation with toluene. The residue was purified by reversed-phase silica gel chromatography (0.1% formic acid aqueous solution:0.1% acetonitrile formate solution = 9:1 to 6:4) to obtain the title compound (905 mg).
$^1$H-NMR Spectrum (500 MHz, $CDCl_3$) δ(ppm): 2.00 (3H, s), 2.62-2.71 (1H, m), 2.79-2.88 (2H, m), 2.91-3.00 (1H, m), 3.53-3.65 (2H, m), 3.82 (3H, s), 4.70-4.80 (1H, m), 5.17-5.26 (1H, m), 5.31-5.40 (1H, m), 6.70-6.77 (1H, m), 6.82-6.92 (1H, m), 7.44-7.54 (1H, m), 7.54-7.61 (2H, m).
ESI-MS (m/z): 533.20 $[M+H]^+$.

[Production Example 201-20]

(S)-3-Acetamide-4-(((3-(5-(3-((1'-(4-((44S,50S,59S)-44-amino-50-benzyl-59-cyclopropoxy-38,45,48,51,54-pentaoxo-59-phenyl-2,5,8,11,14,17,20,23,26,29,32,35,57-tridecaoxa-39,46,49,52,55-pentaazanonapentacontan-59-yl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid

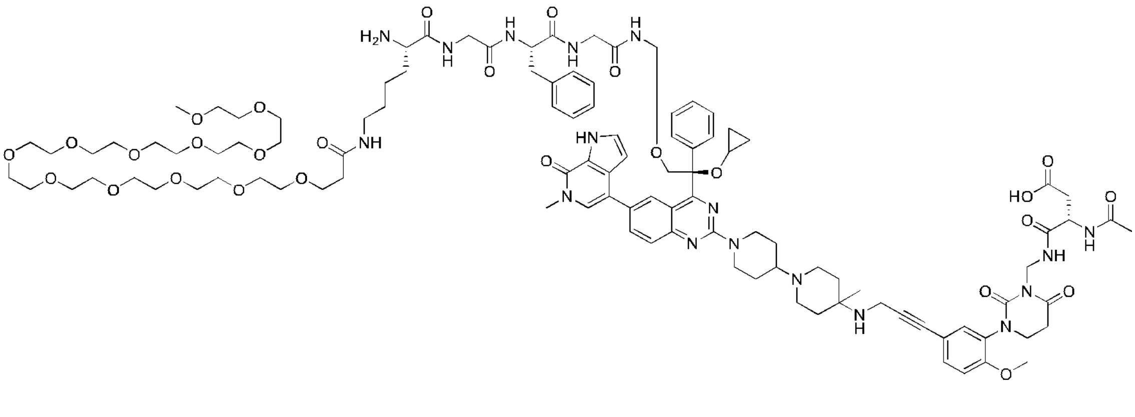

To a solution of the (9H-fluoren-9-yl)methyl ((44S,50S,59S)-50-benzyl-59-cyclopropoxy-59-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-38,45,48,51,54-pentaoxo-59-phenyl-2,5,8,11,14,17,20,23,26,29,32,35,57-tridecaoxa-39,46,49,52,55-pentaazanonapentacontan-44-yl)carbamate of Production Example 201-15 (112 mg, 0.059 mmol), the (S)-3-acetamide-4-(((3-(5-iodo-2-methoxyphenyl)-2,6-dioxotetrahydropyrimidine-1(2H)-yl)methyl)amino)-4-oxobutanoic acid of Production Example 201-19 (50 mg, 0.094 mmol) and CuI (7.9 mg, 0.041 mmol) in DMF (0.91 mL) there were added N,N-diisopropylethylamine (103 μL, 0.59 mmol) and tetrakis(triphenylphosphine)palladium(0) (48 mg, 0.042 mmol), and the mixture was stirred for 1 hour at 50°C under a nitrogen atmosphere. The (S)-2-acetamide-4-(((3-(5-iodo-2-methoxyphenyl)-2,6-dioxotetrahydropyrimidine-1(2H)-yl)methyl)amino)-4-oxobutanoic acid of Production Example 201-19 (5.0 mg, 0.0094 mmol was further added, and the mixture was stirred for 30 minutes at 50°C under a nitrogen atmosphere. The reaction mixture was cooled to room temperature, and then diethylamine (124 μL, 1.19 mmol) was added and the mixture was stirred for 30 minutes at room temperature and subsequently cooled to 4°C (flask A). To a solution of the (9H-fluoren-9-yl)methyl ((44S,50S,59S)-50-benzyl-59-cyclopropoxy-59-(2-(4-methyl-4-(prop-2-yn-1-ylamino)-[1,4'-bipiperidine]-1'-yl)-6-(6-methyl-7-oxo-6,7-dihydro-1H-pyrrolo[2,3-c]pyridin-4-yl)quinazolin-4-yl)-38,45,48,51,54-pentaoxo-59-phenyl-2,5,8,11,14,17,20,23,26,29,32,35,57-tridecaoxa-39,46,49,52,55-pentaazanonapentacontan-44-yl)carbamate of Production Example 201-15 (224 mg, 0.118 mmol), the (S)-3-acetamide-4-(((3-(5-iodo-2-methoxyphenyl)-2,6-dioxotetrahydropyrimidine-1(2H)-yl)methyl)amino)-4-oxobutanoic acid of Production Example 201-19 (110 mg, 0.207 mmol) and CuI (15.8 mg, 0.083 mmol) in DMF (1.82 mL) there were added N,N-diisopropylethylamine (206 μL, 1.18 mmol) and tetrakis(triphenylphosphine)palladium(0) (96 mg, 0.083 mmol), and the mixture was stirred for 1 hour at 50°C under a nitrogen atmosphere. The (S)-2-acetamide-4-(((3-(5-iodo-2-methoxyphenyl)-2,6-dioxotetrahydropyrimidine-1(2H)-yl)methyl)amino)-4-oxobutanoic acid of Production Example 201-19 (10 mg, 0.019 mmol was further added, and the mixture was stirred for 30 minutes at 50°C under a nitrogen atmosphere. The reaction mixture was cooled to room temperature, and then diethylamine (248 μL, 2.38 mmol) was added and the mixture was stirred for 30 minutes at room temperature (flask B). The solutions of flask A and flask B were combined and concentrated under reduced pressure, and then purified by reversed-phase silica gel chromatography (0.1% ammonia water solution:0.1% ammonia-acetonitrile solution = 9:1 to 65:35). The impurity-containing fraction was purified by reversed-phase silica gel chromatography (0.1% formic acid aqueous solution:0.1% acetonitrile formate solution = 9:1 to 6:4) and (0.1% ammonia water solution:0.1% ammonia-acetonitrile solution = 9:1 to 6:4), to obtain the title compound (0.23 g). $^1$H-NMR Spectrum (500 MHz, DMSO-d6) δ(ppm):0.15-0.31 (2H, m), 0.37-0.49 (1H, m), 0.65-0.74 (1H, m), 1.05 (3H, s), 1.18-3.74 (96H, m), 3.80 (3H, s), 4.23-4.31 (1H, m), 4.34-4.42 (1H, m), 4.43-4.56 (3H, m), 4.74-4.81 (1H, m), 4.84-4.95 (2H, m), 4.98-5.11 (2H, m), 5.85-5.92 (1H, m), 7.06-7.12 (1H, m), 7.13-7.39 (14H, m), 7.50-7.54 (1H, m), 7.73-7.81 (3H, m), 8.06-8.17 (3H, m), 8.28-8.37 (1H, m), 8.38-8.46 (1H, m), 8.47-8.55 (1H, m), 12.03-12.16 (1H, m).

ESI-MS (m/z): 1040.71 [(M+2H)/2]$^+$.

[Production Example 201-21]

*tert*-Butyl 3-(2-(2-(3,4-dibromo-2,5-dioxo-2,5-dihydro-1H-pyrrol-1-yl)ethoxy)ethoxy)propanoate

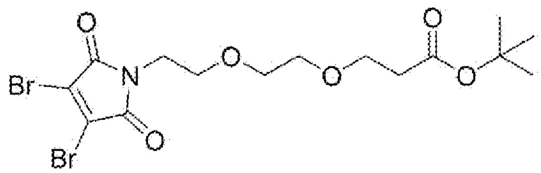

To a solution of 2,3-dibromomaleimide (2.00 g, 7.85 mmol) in THF (80 mL) there were added N-methylmorpholine (0.867 mL, 7.85 mmol) and methyl chloroformate (0.605 mL, 7.85 mmol) at room temperature, and the mixture was stirred for 1.5 hours at room temperature. Water was added to the reaction mixture and extraction was performed with ethyl acetate. The organic layer was washed with water and brine, dried over magnesium sulfate and filtered, and the filtrate was concentrated under reduced pressure to obtain an intermediate. To a solution of the intermediate in dichloromethane (40 mL) there was added *tert*-butyl 3-(2-(2-aminoethoxy)ethoxy)propanoate (1.83 g, 7.85 mmol) at room temperature, and the mixture was stirred for 1 hour at room temperature. The reaction mixture was concentrated under reduced pressure and the residue was purified by silica gel chromatography (heptane:ethyl acetate = 95:5 to 7:3) to obtain the title compound (2.49 g). $^1$H-NMR Spectrum (500 MHz, $CDCl_3$) δ(ppm): 1.43 (9H, s), 2.47 (2H, t, J=6.5 Hz), 3.52-3.60 (4H, m), 3.62-3.69 (4H, m), 3.78-3.81 (2H, m).

[Production Example 201-22]

*tert*-Butyl 3-(2-(2-(2,5-dioxo-3,4-bis(phenylthio)-2,5-dihydro-1H-pyrrol-1-yl)ethoxy)ethoxy)propanoate

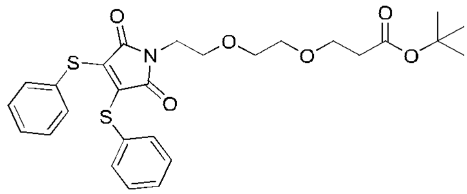

To a solution of the *tert*-butyl 3-(2-(2-(3,4-dibromo-2,5-dioxo-2,5-dihydro-1H-pyrrol-1-yl)ethoxy)ethoxy)propanoate of Production Example 201-21 (2.49 g, 5.29 mmol) in dichloromethane (40 mL) there were added N,N-diisopropylethylamine (4.62 mL, 26.4 mmol) and thiophenol (1.08 mL, 10.6 mmol) at room temperature, and the mixture was stirred for 1 hour at room temperature. The reaction mixture was concentrated under reduced pressure and the residue was purified by silica gel chromatography (heptane:ethyl acetate = 95:5 to 1:1) to obtain the title compound (2.63 g).

$^1$H-NMR Spectrum (500 MHz, $CDCl_3$) $\delta$(ppm): 1.43 (9H, s), 2.48 (2H, t, J=6.5 Hz), 3.52-3.53 (2H, m), 3.55-3.56 (2H, m), 3.59-3.62 (2H, m), 3.65-3.69 (4H, m), 7.15-7.32(10H, m).

[Production Example 201-23]

2,5-Dioxopyrrolidin-1-yl 3-(2-(2-(2,5-dioxo-3,4-bis(phenylthio)-2,5-dihydro-1H-pyrrol-1-yl)ethoxy)ethoxy)propanoate

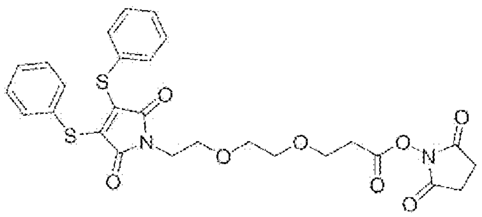

To a solution of the *tert*-butyl 3-(2-(2-(2,5-dioxo-3,4-bis(phenylthio)-2,5-dihydro-1H-pyrrol-1-yl)ethoxy)ethoxy) propanoate of Production Example 201-22 (1.00 g, 1.89 mmol) in dichloromethane (5 mL) there was added TFA (5 mL, 64.9 mmol), and the mixture was stirred for 1 hour at room temperature. The reaction mixture was concentrated under reduced pressure and redissolved in dichloromethane (10 mL), and then again concentrated under reduced pressure. The same procedure was repeated twice again and the residue was dissolved in dichloromethane (10 mL). To the reaction mixture there were added N,N-diisopropylethylamine (3.30 mL, 18.9 mmol) and TSTU (853 mg, 2.83 mmol), and the mixture was stirred for 2 hours at room temperature. The reaction mixture was diluted with ethyl acetate and water and separated by oil-water distribution, and the organic layer was washed with 1 N hydrochloric acid, aqueous saturated sodium carbonate, water and brine, in that order. The organic layer was dried over magnesium sulfate and filtered. The filtrate was concentrated under reduced pressure to obtain a crude product of the title compound (1.04 g).

$^1$H-NMR Spectrum (500 MHz, $CDCl_3$) $\delta$(ppm): 2.70-2.97 (6H, m), 3.52-3.67 (6H, m), 3.68-3.76 (2H, m), 3.80-3.89 (2H, m), 7.14-7.23 (3H, m), 7.23-7.35 (7H, m).

ESI-MS (m/z): 571.13 $[M+H]^+$.

[Example 202]

N-((1S,10S,16S)-10-Benzyl-1-cyclopropoxy-16-(3-(2-(2-(2,5-dioxo-3,4-bis(phenylthio)-2,5-dihydro-1H-pyrrol-1-yl) ethoxy)ethoxy)propanamido)-1-(2-(4-((3-(3-(2,4-dioxotetrahydropyrimidine-1(2H)-yl)-4-methoxyphenyl)prop-2-yn-1-yl) amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-6,9,12,15-tetraoxo-1-phenyl-3-oxa-5,8,11,14-tetraazaeicosan-20-yl)-2,5,8,11,14,17,20,23,26,29,32,35-dodecaoxaoctatriacontan-38-amide

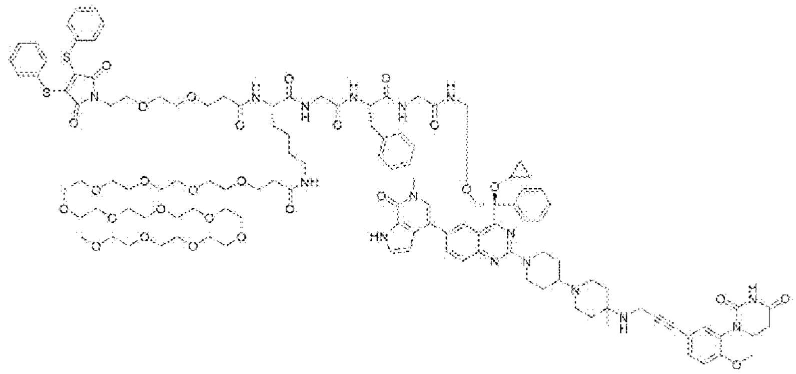

To a solution of the N-((1S,10S,16S)-16-amino-10-benzyl-1-cyclopropoxy-1-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-6,9,12,15-tetraoxo-1-phenyl-3-oxa-5,8,11,14-tetraazaeicosan-20-yl)-2,5,8,11,14,17,20,23,26,29,32,3 5-dodecaoxaoctatriacontan-3 8-amide of Production Example 202-1 (110 mg, 581 μmol) in DMF (1 mL) there were added the 2,5-dioxopyrrolidin-1-yl 3-(2-(2-(2,5-dioxo-3,4-bis(phenylthio)-2,5-dihydro-1H-pyrrol-1-yl)ethoxy)ethoxy)propanoate of Production Example 201-23 (66.3 mg, 116 μmol) and N,N-diisopropylethylamine (30 μL, 0.17 mmol), and the mixture was stirred for 2 hours at room temperature. The reaction mixture was purified by direct reversed-phase silica gel chromatography (0.1% formic acid aqueous solution:0.1% acetonitrile formate solution = 85:15 to 55:45) to obtain a formate of the title compound (58.8 mg).

ESI-MS (m/z): 1174.78 [(M+2H)/2]$^+$.

$^1$H-NMR Spectrum (700 MHz, DMSO-$d_6$) δ(ppm): 0.21-0.29 (2H, m), 0.39-0.46 (1H, m), 0.67-0.73 (1H, m), 1.05 (3H, s), 1.15-1.29 (2H, m), 1.29-1.37 (2H, m), 1.41-1.63 (8H, m), 1.88-1.96 (2H, m), 2.27 (2H, t, J=6.5 Hz), 2.28-2.35 (1 H, m), 2.35-2.41 (1H, m), 2.44-2.50 (2H, m), 2.59-2.69 (5H, m), 2.79 (1H, dd, J=13.6 Hz, 9.6 Hz), 2.93-3.09 (6H, m), 3.22 (3H, s), 3.35-3.60 (64H, m), 3.65-3.75 (3H, m), 3.79 (3H, s), 4.12-4.17 (1H, m), 4.29 (1H, brd, J=11.0 Hz), 4.36-4.43 (1H, m), 4.46-4.53 (2H, m), 4.77 (1H, brd, J=11.0 Hz), 4.86-4.94 (2H, m), 5.89 (1H, m), 7.07 (1H, d, J=8.7 Hz), 7.13-7.17 (1H, m), 7.17-7.31 (22H, m), 7.34 (1H, dd, J=8.6 Hz, 2.0 Hz), 7.53 (1H, d, J=8.7 Hz), 7.73 (1H, t, J= 5.4 Hz), 7.77 (1H, dd, J=8.9, 1.6 Hz), 7.97-8.07 (3H, m), 8.14-8.17 (1H, m), 8.23 (1H, t, J=5.7 Hz), 8.42 (1H, t, J=6.5 Hz), 10.30 (1H, brs), 12.07 (1H, brs)

[Production Example 202-1]

N-((1S,10S,16S)-16-Amino-10-benzyl-1-cyclopropoxy-1-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-6,9,12,15-tetraoxo-1-phenyl-3-oxa-5,8,11,14-tetraazaeicosan-20-yl)-2,5,8,11,14,17,20,23,26,29,32,35-dodecaoxaoctatriacontan-38-amide

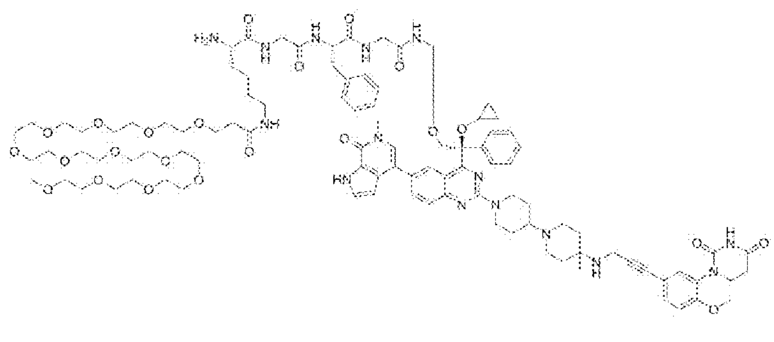

The (9H-fluoren-9-yl)methyl ((44S,50S,59S)-50-benzyl-59-cyclopropoxy-59-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-38,45,48,51,54-pentaoxo-59-phenyl-2,5,8,11,14,17,20,23,26,29,32,35,57-tridecaoxa-39,46,49,52,55-pentaazanonapentacontan-44-yl)carbamate of Production Example 201-15 (145 mg, 76.4 μmol), the 1-(5-iodo-2-methoxyphenyl) dihydropyrimidine-2,4(1H,3H)-dione of Production Example 201-16 (39.7 mg, 115 μmol), copper(I) iodide (5.82 mg, 30.6

μmol) and tetrakis(triphenylphosphine)palladium(0) (35.3 mg, 30.5 μmol) were dissolved in DMF (2.89 mL) and N,N-diisopropylethylamine (680 μL, 3.82 mmol), and after deaeration (pressure reduction/nitrogen substitution repeated 3 times), the mixture was stirred for 1 hour and 30 minutes at 55°C under a nitrogen atmosphere. The reaction mixture was cooled to room temperature, and then diethylamine (79 μL, 0.76 mmol) was added and the mixture was stirred for 30 minutes at room temperature. The reaction mixture was concentrated to 1/3 volume under reduced pressure, and then purified by direct reversed-phase silica gel chromatography (0.1% formic acid aqueous solution:0.1% acetonitrile formate solution = 9:1 to 7:3). The obtained formate of the title compound was neutralized with a saturated aqueous sodium hydrogencarbonate solution and extracted with chloroform/2-propanol(10:1), and then the organic layer was dried over magnesium sulfate. The solution was filtered and the filtrate was concentrated to obtain the title compound (115 mg).

ESI-MS (m/z): 947.25 [(M+2H)/2]$^+$.

$^1$H-NMR Spectrum (500 MHz, $CDCl_3$) δ(ppm): 0.23-0.31 (1H, m), 0.33-0.41 (1H, m), 0.42-0.54 (1H, m), 0.75-0.90 (1H, m), 1.15 (3H, s), 1.29-1.40 (3H, m), 1.41-1.57 (4H, m), 1.58-1.96 (9H, m), 1.99-2.19 (2H, m), 2.43 (2H, t, J=5.8 Hz), 2.55-2.76 (5H, m), 2.77-2.85 (3H, m), 2.96-3.08 (3H, m), 3.10-3.26 (4H, m), 3.26-3.32 (1H, m), 3.38 (3H, s), 3.50-3.57 (4H, m), 3.58-3.73 (44H, m), 3.73-3.93 (9H, m), 4.38 (1H, d, J=11.6 Hz), 4.52-4.69 (3H, m), 4.94 (1H, d, J=11.6 Hz), 4.96-5.07 (2H, m), 6.08 (1H, s), 6.68-6.79 (2H, m), 6.91 (1H, d, J=9.2 Hz), 6.97-7.06 (1H, m), 7.08-7.25 (10H, m), 7.28-7.45 (5H, m), 7.52-7.64 (2H, m), 7.67 (1H, dd, J=8.6, 1.8 Hz), 7.89-8.08 (1H, m), 8.10-8.19 (1H, m), 10.22-10.35 (1H, m).

[Example 203]

(S)-3-Acetamide-4-(((3-(5-(3-((1'-(4-((1S,10S)-10-benzyl-1-cyclopropoxy-26-(2,5-dioxo-3,4-bis(phenylthio)-2,5-dihydro-1H-pyrrol-1-yl)-6,9,12,15,18-pentaoxo-1-phenyl-3,21,24-trioxa-5,8,11,14,17-pentaazahexacosyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid

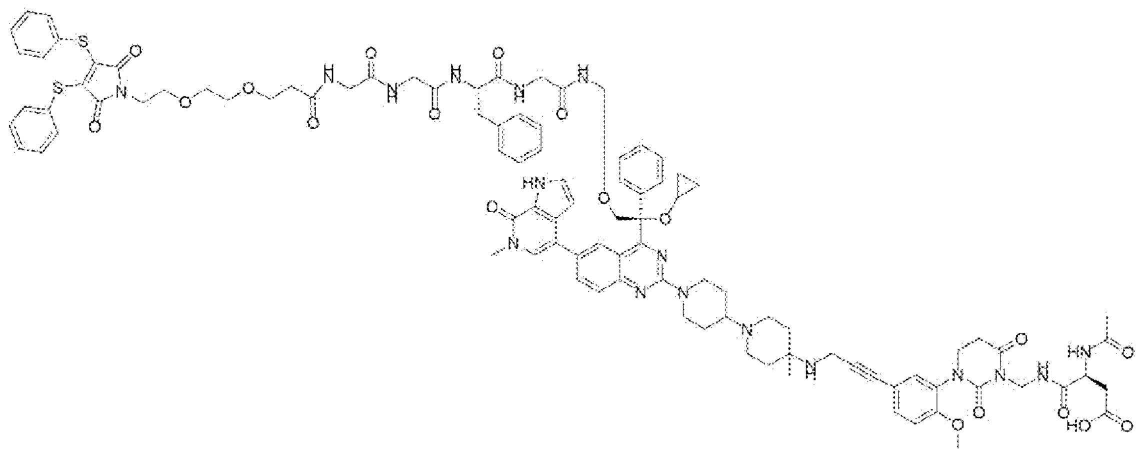

To a solution of the (S)-3-acetamide-4-(((3-(5-(3-((1'-(4-((1S,10S)-16-amino-10-benzyl-1-cyclopropoxy-6,9,12,15-tetraoxo-1-phenyl-3-oxa-5,8,11,14-tetraazahexadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid of Production Example 203-2 (123 mg, 0.086 mmol) and the 2,5-dioxopyrrolidin-1-yl 3-(2-(2-(2,5-dioxo-3,4-bis(phenylthio)-2,5-dihydro-1H-pyrrol-1-yl)ethoxy)ethoxy)propanoate of Production Example 201-23 (95 mg, 0.17 mmol) in DMF (4.0 mL) there was added N,N-diisopropylethylamine (200 μL, 1.12 mmol), and the mixture was stirred for 1 hour at room temperature. The reaction mixture was concentrated to 1/3 volume and purified by reversed-phase silica gel chromatography (0.1% formic acid aqueous solution:0.1% acetonitrile formate solution = 9:1 to 3:7) to obtain a formate of the title compound (82.6 mg).

ESI-MS (m/z): 1893.97 [M+H]$^+$.
$^1$H-NMR Spectrum (700 MHz, DMSO-$d_6$) δ (ppm): 0.22-0.29 (2H, m), 0.39-0.47 (1H, m), 0.67-0.74 (1H, m), 1.08 (3 H, s), 1.45-1.58 (4H, m), 1.58-1.68 (2H, m), 1.81 (3H, s), 1.97 (2H, m), 2.36 (2H, t, J=6.6 Hz), 2.41 (1H, dd, J=16.3 Hz, 7.8 Hz), 2.52-2.61 (3H, m), 2.70-2.82 (6H, m), 2.96-3.09 (4H, m), 3.38-3.42 (2H, m), 3.42-3.45 (2H, m), 3.48 (2H, t, J=6.1 Hz), 3.50-3.60 (12H, m), 3.65-3.70 (4H, m), 3.72 (1H, dd, J=16.7 Hz, 5.9 Hz), 3.80 (3H, s), 4.29 (1H, brd, J=11.0 Hz), 4.40 (1H, dd, J=10.0 Hz, 6.8 Hz), 4.46-4.53 (3H, m), 4.77 (1H, brd, J=11.0 Hz), 4.86-4.95 (2H, m), 4.96-5.03 (1H, m),

5.06-5.13 (1H, m), 5.89 (1H, m), 7.10 (1H, d, J=8.6 Hz), 7.13-7.32 (22H, m), 7.32-7.38 (2H, m), 7.53 (1H, d, J=8.7 Hz), 7.78 (1H, dd, J=8.9, 1.9 Hz), 7.99 (1H, br t, J=5.6 Hz), 8.04-8.10 (2H, m), 8.10-8.14 (2H, m), 8.14-8.19 (1H, m), 8.24 (1H, br t, J=5.8 Hz), 8.44 (1H, br t, J=6.5 Hz), 12.07 (1H, brs)

[Production Example 203-1]

(9H-Fluoren-9-yl)methyl ((1S,10S)-10-benzyl-1-cyclopropoxy-1-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-6,9,12,15-tetraoxo-1-phenyl-3-oxa-5,8,11,14-tetraazahexadecan-16-yl)carbamate

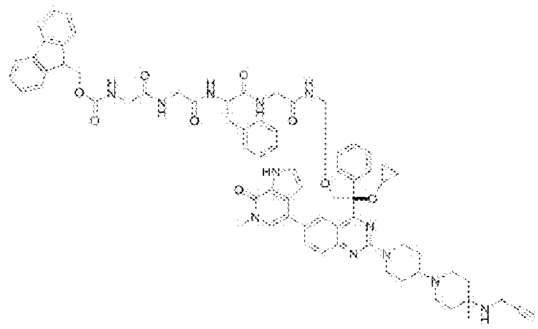

To a solution of the (9H-fluoren-9-yl)methyl (S)-(2-(((2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamate of Production Example 201-11 (150 mg, 0.151 mmol) in DMF (3 mL) there was added diethylamine (200 μL, 1.93 mmol), and the mixture was stirred for 30 minutes at room temperature. The reaction mixture was concentrated under reduced pressure and the residue was again dissolved in DMF (3 mL), after which (((9H-fluoren-9-yl)methoxy)carbonyl)glycylglycyl-L-phenylalanine (91 mg, 0.181 mmol) and N,N-diisopropylamine (100 μL, 0.574 mmol) were added. The reaction mixture was ice-cooled, and then HATU (68.8 mg, 181 μmol) was added and the mixture was stirred for 1 hour at 0°C. The reaction mixture was purified by direct reversed-phase silica gel chromatography (0.1% ammonia water solution:0.1% ammonia-acetonitrile solution = 9:1 to 1:4) to obtain the title compound (110 mg).

$^1$H-NMR Spectrum (500 MHz, DMSO-$d_6$) δ(ppm): 0.19-0.27 (2H, m), 0.36-0.44 (1H, m), 0.63-0.71 (1H, m), 0.94 (3H, s), 1.28-1.35 (2H, m), 1.38-1.48 (4H, m), 1.70-1.74 (1H, m), 1.80-1.89, (2H, m), 2.29-2.36 (2H, m), 2.48-2.60 (3H, m), 2.71-2.79 (1H, m), 2.91-3.07 (5H, m), 3.17-3.23 (2H, m), 3.49 (3H, s), 3.52-3.61 (3H, m), 3.63-3.74 (3H, m), 4.18 (1H, t, J=7.4 Hz), 4.22-4.28 (3H, m), 4.33-4.40 (1H, m), 4.44-4.51 (2H, m), 4.74 (1H, d, J=11.0 Hz), 4.81-4.89 (2H, m), 5.83-5.87 (1H, m), 7.10-7.33 (14H, m), 7.34-7.39 (2H, m), 7.49 (1H, d, J=9.3 Hz), 7.55 (1H, t, J=6.0 Hz), 7.66 (1H, d, J=7.3 Hz), 7.72-7.76 (1H, m), 7.79-7.87 (3H, m), 7.98 (1H, t, J=5.3 Hz), 8.06-8.14 (2H, m), 8.21-8.30 (1H, m), 8.44 (1H, t, J=6.4 Hz), 12.06 (1H, brs).
ESI-MS (m/z): 1255.98 $[M+H]^+$

[Production Example 203-2]

(S)-3-Acetamide-4-(((3-(5-(3-((1'-(4-((1S,10S)-16-amino-10-benzyl-1-cyclopropoxy-6,9,12,15-tetraoxo-1-phenyl-3-oxa-5,8,11,14-tetraazahexadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid

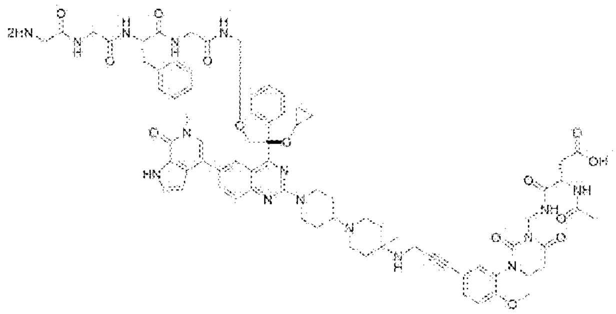

To a solution of the (9H-fluoren-9-yl)methyl((1S,10S)-10-benzyl-1-cyclopropoxy-1-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-6,9,12,15-tetraoxo-1-phenyl-3-oxa-5,8,11,14-tetraazahexadecan-16-yl)carbamate of Production Example 203-1 (100 mg, 0.080 mmol), the (S)-3-acetamide-4-(((3-(5-iodo-2-methoxyphenyl)-2,6-dioxotetrahydropyrimidine-1(2H)-yl)methyl)amino)-4-oxobutanoic acid of Production Example 201-19 (85 mg, 0.16 mmol), CuI (6.07 mg, 0.032 mmol) and tetrakis(triphenylphosphine)palladium(0) (36.8 mg, 0.032 mmol) in DMF (2 mL) there was added N,N-diisopropylethylamine (200 μL, 1.12 mmol), and a procedure of pressure reduction and nitrogen substitution was repeated 3 times. The reaction mixture was stirred for 3 hours at 60°C. After cooling to room temperature, diethylamine (200 μL, 1.93 mmol) was added to the reaction mixture, which was then stirred for 30 minutes. The reaction mixture was concentrated to 1/3 volume and purified by reversed-phase silica gel chromatography (0.1% ammonia water solution:0.1% ammonia-acetonitrile solution = 9:1 to 1:4) to obtain the title compound (43 mg).

$^1$H-NMR Spectrum (500 MHz, DMSO-$d_6$) δ(ppm): 0.18-0.26 (2H, m), 0.36-0.44 (1H, m), 0.63-0.71 (1H, m), 1.02 (3H, s), 1.35-1.60 (6H, m), 1.77, (3H, s), 1.83-1.95 (2H, m), 2.25-2.34 (1H, m), 2.36-2.44 (3H, m), 2.54-2.68 (3H, m), 2.70-2.79 (3H, m), 2.91-3.07 (5H, m), 3.08-3.16 (2H, m), 3.44-3.48 (6H, m), 3.55-3.62 (3H, m), 3.66 (2H, d, J=6.5 Hz), 3.68-3.75 (2H, m), 3.77 (3H, s), 4.25 (1H, d, J=11.0 Hz), 4.32-4.40 (1H, m), 4.41-4.52 (3H, m), 4.74 (1H, d, J=11.0 Hz), 4.80-4.92 (2H, m), 4.96-5.07 (2H, m), 5.87 (1H, d, J=2.5 Hz), 7.03-7.09 (1H, m), 7.11-7.28 (13H, m), 7.29-7.34 (2H, m), 7.49 (1H, d, J=9.2 Hz), 7.70-7.77 (1H, m), 8.08 (1H, d, J=8.0 Hz), 8.12 (1H, d, J=1.9 Hz), 8.21-8.32 (2H, m), 8.36 (1H, t, J=5.5 Hz), 8.47 (1H, t, J=6.4 Hz), 12.06 (1H, brs).
ESI-MS (m/z): 1438.19 [M+H]$^+$

[Example 204]

(S)-3-Acetamide-4-(((3-(5-(3-((1'-(4-((1S,7S)-7-(2-amino-2-oxoethyl)-1-cyclopropoxy-17-(2,5-dioxo-3,4-bis(phenylthio)-2,5-dihydro-1H-pyrrol-1-yl)-6,9-dioxo-1-phenyl-3,12,15-trioxa-5,8-diazaheptadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid

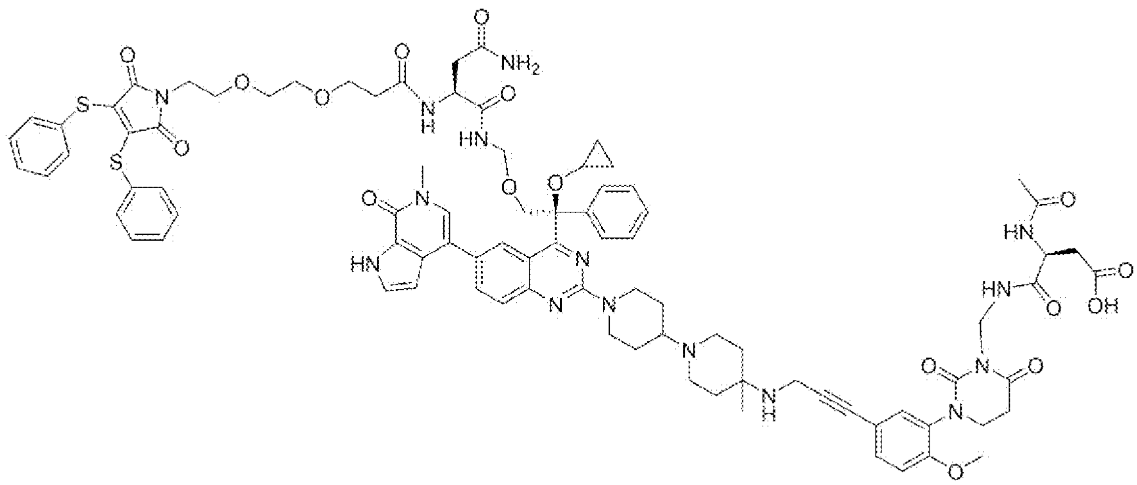

To a solution of the (S)-3-acetamide-4-(((3-(5-(3-((1'-(4-((S)-1-cyclopropoxy-2-(((S)-2,4-diamino-4-oxobutanamido)methoxy)-1-phenylethyl)-6-(6-methyl-7-oxo-6,7-dihydro-1H-pyrrolo[2,3-c]pyridin-4-yl)quinazoline-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl) amino)-4-oxobutanoic acid of Production Example 204-5 (5.0 mg, 0.0041 mmol) in DMF (0.12 mL) there were added N,N-diisopropylethylamine (3.5 μL, 0.020 mmol) and the 2,5-dioxopyrrolidin-1-yl 3-(2-(2-(2,5-dioxo-3,4-bis(phenylthio)-2,5-dihydro-1H-pyrrol-1-yl)ethoxy)ethoxy)propanoate of Production Example 201-23 (3.5 mg, 0.0061 mmol), and the mixture was stirred for 1 hour at room temperature (flask A). To a solution of the (S)-3-acetamide-4-(((3-(5-(3-((1 '-(4-((S)-1-cyclopropoxy-2-(((S)-2,4-diamino-4-oxobutanamido)methoxy)-1-phenylethyl)-6-(6-methyl-7-oxo-6,7-dihydro-1H-pyrrolo[2,3-c]pyridin-4-yl)quinazoline-2-yl)-4-methyl-[1,4'-bipiperidine] -4-yl)amino)prop-1-yn-1-yl)-2-methoxyphenyl)-2,6-dioxotetrahydropyrimidine-1(2H)-yl)methyl)amino)-4-oxobutanoic acid of Production Example 204-5 (130 mg, 0.105 mmol) in DMF (1.0 mL) there was added a solution of N,N-diisopropylethylamine (92.5 μL, 0.527 mmol) and the 2,5-dioxopyrrolidin-1-yl 3-(2-(2-(2,5-dioxo-3,4-bis(phenylthio)-2,5-dihydro-1H-pyrrol-1-yl)ethoxy)ethoxy)propanoate of Production Example 201-23 (90.5 mg, 0.159 mmol) in DMF (2.4 mL), and after stirring for 5 minutes at room temperature, the solution of flask A was added. The mixture was further stirred for 1.5 hours at room temperature, and then the 2,5-dioxopyrrolidin-1-yl 3-(2-(2-(2,5-dioxo-3,4-bis(phenylthio)-2,5-dihydro-1H-pyrrol-1-yl)ethoxy)ethoxy)propanoate of Production Example 201-23 (12 mg, 0.021 mmol) was further added and the mixture was stirred for 1 hour at room temperature. The reaction mixture was purified a total of 2 times by direct reversed-phase silica gel chromatography (0.1% formic acid aqueous solution:0.1% acetonitrile formate solution = 90:10 to 50:50), and then dissolved in a 10% methanol dichloromethane solution, after which *n*-heptane was added, and the mixture was concentrated to solidification to obtain a formate of the title compound (0.11 g).

ESI-MS (m/z): 845.23 [(M+2H)/2]$^+$.
$^1$H-NMR Spectrum (700 MHz, DMSO-$d_6$) δ(ppm): 0.14-0.20 (1H, m), 0.20-0.27 (1H, m), 0.38-0.44 (1H, m), 0.62-0.69 (1H, m), 1.10 (3H, s), 1.49-1.59 (4H, m), 1.62-1.70 (2H, m), 1.81 (3H, s), 1.96-2.03 (2H, m), 2.34 (2H, t, J=6.7 Hz), 2.37-2.44 (2H, m), 2.45-2.51 (1H, m), 2.56 (1H, dd, J=16.3 Hz, 5.8 Hz), 2.60-2.67 (2H, m), 2.75-2.87 (5H, m), 2.98-3.09 (3H, m), 3.36-3.40 (2H, m), 3.41-3.45 (2H, m), 3.45-3.50 (2H, m), 3.50-3.57 (11H, m), 3.80 (3H, s), 4.24 (1H, brd, J=11.2 Hz), 4.38 (1H, dd, J=10.0 Hz, 7.0 Hz), 4.45-4.56 (3H, m), 4.76 (1 H, brd, J=11.1 Hz), 4.88-4.96 (2H, m), 4.96-5.03 (1H, m), 5.07-5.13 (1H, m), 5.86 (1H, m), 6.84 (1H, brs), 7.10 (1H, d, J=8.6 Hz), 7.17-7.31 (18H, m), 7.32-7.38 (2H, m), 7.53 (1H, d, J=8.7 Hz), 7.77 (1H, dd, J=8.9, 1.9 Hz), 8.02 (1H, d, J=7.9 Hz), 8.06 (1H, m), 8.11-8.14 (2 H, m), 8.42 (1H, br t, J=6.6 Hz), 12.06 (1H, brs)

[Production Example 204-1]

*tert*-Butyl N2-(((9H-fluoren-9-yl)methoxy)carbonyl)N4-trityl-L-asparaginyl glycinate

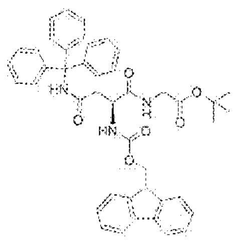

To a solution of FMOC-L-asparagine (TRT)-OH (26.4 g, 44.3 mmol) in DMF (100 mL) there were added H-glycine-OTBU hydrochloride (7.43 g, 44.3 mmol), HATU (18.5 g, 48.7 mmol) and N,N-diisopropylethylamine (23.2 mL, 133 mmol), and the mixture was stirred for 3.5 hours at room temperature. Water and ethyl acetate were added to the reaction mixture, and extraction was performed with ethyl acetate. The organic layer was washed with water and brine, dried over magnesium sulfate and filtered, and the filtrate was concentrated to obtain the title compound (32.7 g).
$^1$H-NMR Spectrum (500 MHz, $CDCl_3$) δ(ppm): 1.45 (9H, s), 2.57-2.69 (1H, m), 3.06-3.17 (1H, m), 3.64-3.81 (1H, m), 3.90-4.00 (1H, m), 4.18-4.20 (1H, m), 4.29-4.44 (2H, m), 4.51-4.64 (1H, m), 6.33-6.45 (1H, m), 6.84-6.93 (1H, m), 7.11-7.40(20H, m), 7.56 (2H, brd, J=6.9 Hz), 7.74 (2H, brd, J=6.9 Hz).

[Production Example 204-2]

(((9H-Fluoren-9-yl)methoxy)carbonyl)-L-asparaginylglycine

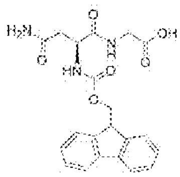

To a solution of the *tert-butyl* N2-(((9H-fluoren-9-yl)methoxy)carbonyl)N4-trityl-L-asparagine glycinate of Production Example 204-1 (25 g, 35 mmol) in dichloromethane (15 mL) there was added TFA (30 mL), and the mixture was stirred for 18 hours at room temperature. The reaction mixture was concentrated under reduced pressure, processed 3 times by azeotropic distillation with ethyl acetate, and then diluted with ethyl acetate. The reaction solution was filtered, and the obtained solid was washed 5 times with ethyl acetate to obtain the title compound (12 g).
$^1$H-NMR Spectrum (500 MHz, DMSO-$d_6$) δ(ppm): 2.29-2.42 (1H, m), 2.48-2.52 (1H, m), 3.61-3.80 (2H, m), 4.15-4.27 (3H, m), 4.31-4.40 (1H, m), 6.81-6.90 (1H, m), 7.21-7.33 (3H, m), 7.35-7.41 (2H, m), 7.45-7.57 (1H, m), 7.63-7.75 (2H, m), 7.82-7.89 (2H, m), 7.99-8.08 (1H, m), 12.39-12.64 (1H, m).
ESI-MS (m/z): 412.21 $[M+H]^+$.

[Production Example 204-3]

(S)-(2-((((9H-Fluoren-9-yl)methoxy)carbonyl)amino)-4-amino-4-oxobutanamide)methyl acetate

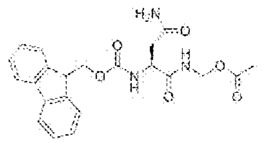

To a solution of the (((9H-fluoren-9-yl)methoxy)carbonyl)-L-asparaginylglycine of Production Example 204-2 (6.95 g, 16.9 mmol) in THF (550 mL), toluene (100 mL) and pyridine (1.64 mL) there was added lead tetraacetate (8.99 g, 20.3 mmol), and the mixture was stirred for 2 hours at 110°C. The mixture was cooled to room temperature and filtered with Celite (washing with ethyl acetate/THF), and the filtrate was concentrated. The obtained solid was suspended in ethyl acetate (220 mL) and filtered, and then washed with ethyl acetate to obtain the title compound (3.29 g).
$^1$H-NMR Spectrum (400 MHz, DMSO-$d_6$) δ(ppm): 1.95 (s, 3H), 2.32-2.39 (1H, m), 4.19-4.23 (4H, m), 4.28-4.37 (1H, m), 5.01-5.08 (2H, m), 6.82-6.91 (1H, m), 7.21-7.43 (5H, m), 7.49-7.57 (1H, m), 7.66-7.69 (2H, m), 7.84-7.87 (2H, m), 8.75-8.86 (1H, m).
ESI-MS (m/z): 731.33 $[2M-2OAc+H]^+$.

[Production Example 204-4]

(9H-Fluoren-9-yl)methyl ((S)-4-amino-1-((((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-1,4-dioxobutan-2-yl)carbamate

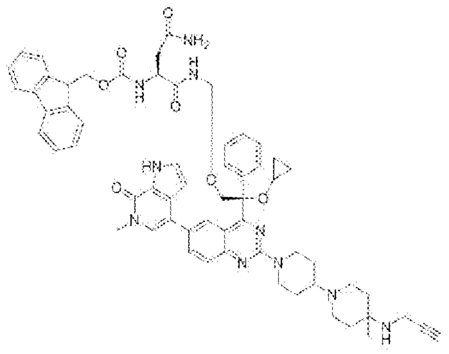

To a solution of the (S)-(2-((((9H-fluoren-9-yl)methoxy)carbonyl)amino)-4-amino-4-oxobutanamide)methyl acetate of Production Example 204-3 (248 mg, 0.583 mmol) in dichloromethane (4.0 mL) there was added TMSCl (93 μL, 0.73 mmol), and the mixture was stirred for 20 minutes at room temperature (flask A). The (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 201-9 (100 mg, 0.146 mmol) and CSA (135 mg, 0.581 mmol) were together processed by azeotropic distillation with dichloromethane/toluene, and the water was removed (flask B). Dichloromethane (4.0 mL) was added to flask B, and the mixture was added to flask A and stirred for 75 minutes at room temperature and then quenched with saturated sodium bicarbonate water (8 mL) (crude product 1). To a solution of the (S)-(2-((((9H-fluoren-9-yl)methoxy)carbonyl)amino)-4-amino-4-oxobutanamide)methyl acetate of Production Example 204-3 (496 mg, 1.17 mmol) in dichloromethane (8.0 mL) there was added TMSCl (187 μL, 1.46 mmol), and the mixture was stirred for 20 minutes at room temperature (flask C). The (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 201-9 (200 mg, 0.292 mmol) and CSA (271 mg, 1.17 mmol) were together processed by azeotropic distillation with dichloromethane/toluene, and the water was removed (flask D). Dichloromethane (8.0 mL) was added to flask D, and the mixture was added to flask C and stirred for 60 minutes at room temperature and then quenched with saturated sodium bicarbonate water (16 mL) (crude product 2). Crude products 1 and 2 were each diluted with a 10% methanol dichloromethane solution and water and then irradiated with ultrasonic waves, and the insoluble portions were filtered out (washing with 10% methanol dichloromethane solution and methanol). The filtrate was extracted with a 10% methanol dichloromethane solution, dried over sodium sulfate, filtered and concentrated. The residue was purified by reversed-phase silica gel chromatography (0.1% formic acid aqueous solution:0.1% acetonitrile formate solution = 9:1 to 6:4) to obtain a formate of the title compound (150 mg). Upon collecting the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 201-9 (100 mg), it was subj ected to the same reaction conditions to again obtain a formate of the title compound (50 mg). This was combined with 21 mg synthesized by the same method, and the total of 221 mg of the formate of the title compound was dissolved in a 10% methanol dichloromethane solution, sodium bicarbonate water was added for neutralization, and extraction was performed with a 10% methanol dichloromethane solution. The organic layer was dried over sodium sulfate and filtered, and the filtrate was concentrated to obtain the title compound (0.21 g).

ESI-MS (m/z): 1051.78 $[M+H]^+$.

$^1$H-NMR Spectrum (400 MHz, $CDCl_3$) δ(ppm): 0.18-0.28 (1H, m), 0.33-0.50 (2H, m), 0.75-0.87 (1H, m), 1.09 (3H, s), 1.41-1.74 (6H, m), 1.96-2.07 (2H, m), 2.15-2.21 (1H, m), 2.45-2.71 (7H, m), 2.90-3.07 (3H, m), 3.11-3.21 (1H, m), 3.30-3.38 (2H, m), 3.61 (3H, s), 4.16-4.22 (1H, m), 4.32-4.50 (4H, m), 4.58-4.71 (2H, m), 4.87-4.96 (1H, m) 4.99-5.09 (2H, m), 5.55-5.66 (1H, m), 5.89-6.02 (1H, m), 6.07-6.11 (1H, m), 6.25-6.31 (1H, m), 6.69 (1H, s), 7.09-7.30 (7H, m), 7.34-7.41 (4H, m), 7.51-7.59 (3H, m), 7.62-7.69 (1H, m), 7.74 (2H, d, J=7.3 Hz), 8.12 (1H, s), 9.80-9.95 (1H, m)

[Production Example 204-5]

(S)-3-Acetamide-4-(((3-(5-(3-((1'-(4-((S)-1-cyclopropoxy-2-(((S)-2,4-diamino-4-oxobutanamido)methoxy)-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid

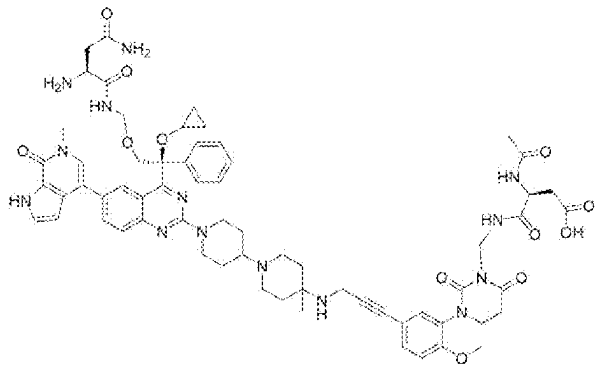

To a solution of the (9H-fluoren-9-yl)methyl ((S)-4-amino-1-((((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-1,4-dioxobutan-2-yl)carbamate of Production Example 204-4 (200 mg, 0.190 mmol), the (S)-2-acetamide-4-(((3-(5-iodo-2-methoxyphenyl)-2,6-dioxotetrahydropyrimidine-1(2H)-yl)methyl)amino)-4-oxobutanoic acid of Production Example 201-19 (152 mg, 0.286 mmol) and CuI (27.2 mg, 0.143 mmol) in DMF (2.95 mL) there were added N,N-diisopropylethylamine (332 μL, 1.90 mmol) and tetrakis(triphenylphosphine)palladium(0) (165 mg, 0.143 mmol), and after deaeration (pressure reduction/nitrogen substitution repeated 3 times), the mixture was stirred for 1 hour at 50°C under a nitrogen atmosphere. The (S)-2-acetamide-4-(((3-(5-iodo-2-methoxyphenyl)-2,6-dioxotetrahydropyrimidine-1(2H)-yl)methyl)amino)-4-oxobutanoic acid of Production Example 201-19 (15.2 mg, 0.0286 mmol was further added, and the mixture was stirred for 30 minutes at 50°C under a nitrogen atmosphere. The reaction mixture was cooled to room temperature, and then diethylamine (398 μL, 3.81 mmol) was added and the mixture was stirred for 30 minutes at room temperature. The reaction mixture was concentrated and purified by direct reversed-phase silica gel chromatography (0.1% ammonia water solution:0.1% ammonia-acetonitrile solution = 9:1 to 7:3) to obtain the title compound (147 mg).

ESI-MS (m/z): 617.67 $[(M+2H)/2]^+$.

$^1$H-NMR Spectrum (500 MHz, DMSO-$d_6$) δ(ppm): 0.20-0.33 (2H, m), 0.37-0.48 (1H, m), 0.64-0.76 (1H, m), 1.08 (3H, s), 1.41-1.66 (6H, m), 1.81 (3H, s), 1.89-1.99 (2H, m), 2.12-2.23 (1H, m), 2.25-3.68 (21H, m), 3.81 (3H, s), 4.26 (1H, brd, J=11.5 Hz), 4.35-4.49 (2H, m), 4.50-4.59 (1H, m), 4.71-4.82 (1H, m) 4.83-4.97 (2H, m), 5.00-5.14 (2H, m), 5.83-5.94 (1H, m), 6.80-6.88 (1H, m), 7.05-7.14 (1H, m), 7.15-7.31 (7H, m), 7.33-7.39 (2H, m), 7.39-7.47 (1H, m), 7.53 (1H, d, J=8.6 Hz), 7.77 (1H, dd, J=8.6, 1.7 Hz), 8.06-8.20 (2H, m), 8.36-8.76 (2H, m), 12.05-12.14 (1H, m).

[Example 205]

(S)-N-(2-((((S)-2-Cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)-2-(15-(2,5-dioxo-3,4-bis(phenylthio)-2,5-dihydro-1H-pyrrol-1-yl)-4,7-dioxo-10,13-dioxa-3,6-diazapentadecanamido)-3-phenylpropaneamide

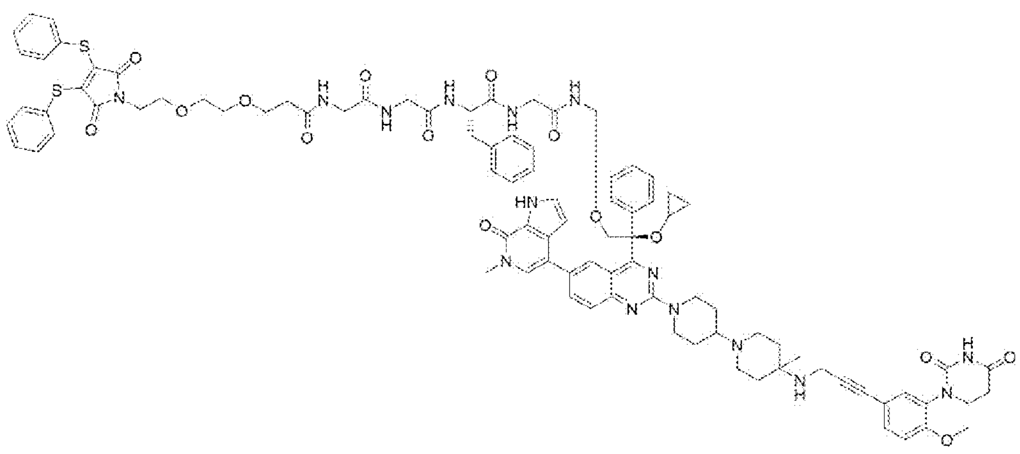

To a solution of the tert-butyl 3-(2-(2-(2,5-dioxo-3,4-bis(phenylthio)-2,5-dihydro-1H-pyrrol-1-yl)ethoxy)ethoxy) propanoate of Production Example 201-22 (8.0 mg, 0.015 mmol) in dichloromethane (0.30 mL) there was added TFA(0.30 mL)), and the mixture was stirred for 1 hour at room temperature. The reaction mixture was concentrated under reduced pressure to obtain crude product 1. Crude product 1 was dissolved in DMF (0.25 mL), N,N-diisopropylethylamine (24.5 μL, 0.137 mmol) and HATU (5.2 mg, 0.014 mmol) were added, and the mixture was stirred for 5 minutes at room temperature. This solution was added to a solution of the (S)-2-(2-(2-aminoacetamide)acetamide)-N-(2-((((S)-2-cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl) amino)-2-oxoethyl)-3-phenylpropaneamide of Production Example 205-1 (8.6 mg, 6.9 μmol) in DMF (0.25 mL), and the mixture was stirred for 1 hour at room temperature. The reaction mixture was purified by direct reversed-phase silica gel chromatography (0.1% formic acid aqueous solution:0.1% acetonitrile formate solution = 90:10 to 55:45) to obtain a formate of the title compound (7.6 mg).

ESI-MS (m/z): 854.80 [(M+2H)/2]$^+$.

[Production Example 205-1]

(S)-2-(2-(2-Aminoacetamide)acetamide)-N-(2-((((S)-2-cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)-3-phenylpropaneamide

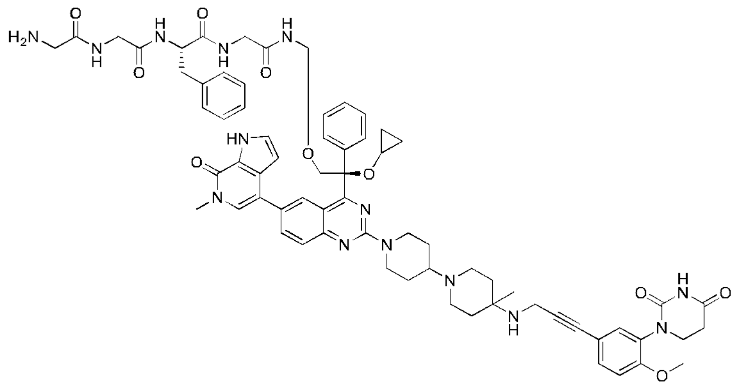

The title compound (10.5 mg) was obtained from the (9H-fluoren-9-yl)methyl((1S,10S)-10-benzyl-1-cyclopropoxy-1-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c] pyridin-4-yl)quinazolin-4-yl)-6,9,12,15-tetraoxo-1-phenyl-3-oxa-5,8,11,14-tetraazahexadecan-16-yl)carbamate of Production Example 203-1 (24.1 mg, 0.0192 mmol) and the 1-(5-iodo-2-methoxyphenyl)dihydropyrimidine-2,4(1H,3H)-dione of Production Example 201-16 (13.3 mg, 0.0384 mmol), by the same method as Production Example 204-5.

ESI-MS (m/z): 1251.78 [M+H]$^+$.

[Example 206]

(S)-N-(2-(((3-(5-(3-((1'-(4-((S)-1-Cyclopropoxy-2-hydroxy-1-phenylethyl)-6-(6-methyl-7-oxo-6,7-dihydro-1H-pyrrolo [2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-2-oxoethyl)-2-(15-(2,5-dioxo-3,4-bis(phenylthio)-2,5-dihydro-1H-pyrrol-1-yl)-4,7-dioxo-10,13-dioxa-3,6-diazapentadecanamido)-3-phenylpropaneamide

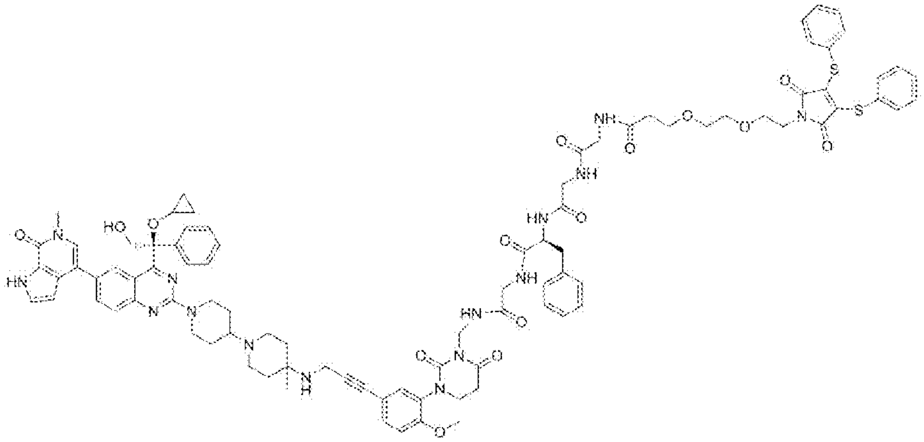

A formate of the title compound (3.2 mg) was obtained from the *tert-butyl* 3-(2-(2-(2,5-dioxo-3,4-bis(phenylthio)-2,5-dihydro-1H-pyrrol-1-yl)ethoxy)ethoxy)propanoate of Production Example 201-22 (4.7 mg, 0.0089 mmol) and a formate of the (S)-2-(2-(2-aminoacetamide)acetamide)-N-(2-(((3-(5-(3-((1 ’-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4’-bipiperidin]-4-yl)amino)prop-1-yn-1-yl)-2-methoxyphenyl)-2,6-dioxotetrahydropyrimidine-1(2H)-yl)methyl)amino)-2-oxoethyl)-3-phenylpropaneamide of Production Example 206-3 (6.0 mg), by the same method as Example 5.

ESI-MS (m/z): 854.52 $[(M+2H)/2]^+$.

[Production Example 206-1]

(9H-Fluoren-9-yl)methyl(2-(((3-(5-iodo-2-methoxyphenyl)-2,6-dioxotetrahydropyrimidine-1(2H)-yl)methyl)amino)-2-oxoethyl)carbamate

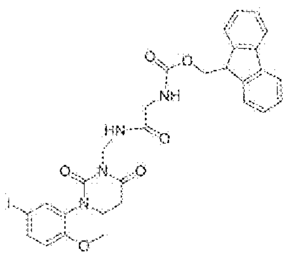

The title compound (350 mg) was obtained from the 1-(5-iodo-2-methoxyphenyl)dihydropyrimidine-2,4(1H,3H)-dione of Production Example 201-16 (250 mg, 0.722 mmol) and the (2-((((9H-fluoren-9-yl)methoxy)carbonyl)amino)acetamide)methyl acetate of Production Example 201-10 (293 mg, 0.795 mmol), by the same method as Production Example 201-18.

ESI-MS (m/z): 655.14 $[M+H]^+$.

[Production Example 206-2]

(9H-Fluoren-9-yl)methyl (S)-(7-benzyl-1-(3-(5-iodo-2-methoxyphenyl)-2,6-dioxotetrahydropyrimidine-1(2H)-yl)-3,6,9,12-tetraoxo-2,5,8,11-tetraazatridecan-13-yl)carbamate

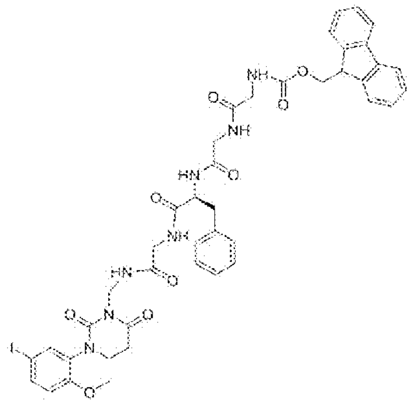

The title compound (79 mg) was obtained from the (9H-fluoren-9-yl)methyl (2-(((3-(5-iodo-2-methoxyphenyl)-2,6-dioxotetrahydropyrimidine-1(2H)-yl)methyl)amino)-2-oxoethyl)carbamate of Production Example 206-1 (70 mg, 0.11 mmol), by the same method as Production Example 203-1.
ESI-MS (m/z): 916.39 $[M+H]^+$.

[Production Example 206-3]

(S)-2-(2-(2-Aminoacetamide)acetamide)-N-(2-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-2-oxoethyl)-3-phenylpropaneamide

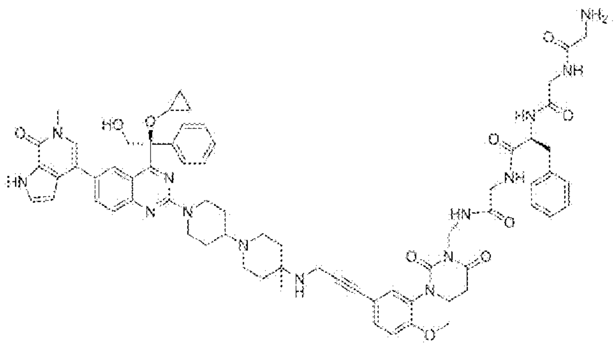

A formate of the title compound (13 mg) was obtained from the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo [2,3-c]pyridin-7-one of Production Example 201-9 (10 mg, 0.015 mmol) and the (9H-fluoren-9-yl)methyl (S)-(7-benzyl-1-(3-(5-iodo-2-methoxyphenyl)-2,6-dioxotetrahydropyrimidine-1(2H)-yl)-3,6,9,12-tetraoxo-2,5,8,11-tetraazatridecan-13-yl)carbamate of Production Example 206-2 (26.7 mg, 0.029 mmol), by the same method as Production Example 204-5.
ESI-MS (m/z): 1251.75 $[M+H]^+$.

[Example 207]

(S)-N-(((S)-2-Cyclopropoxy-2-(2-(4-((3-(3-(2,4-dioxotetrahydropyrimidine-1(2H)-yl)-4-methoxyphenyl)prop-2-yn-1-yl) amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)-2-(15-(2,5-dioxo-3,4-bis(phenylthio)-2,5-dihydro-1H-pyrrol-1-yl)-4,7-dioxo-10,13-dioxa-3,6-diazapentadecanamido)-3-phenylpropaneamide

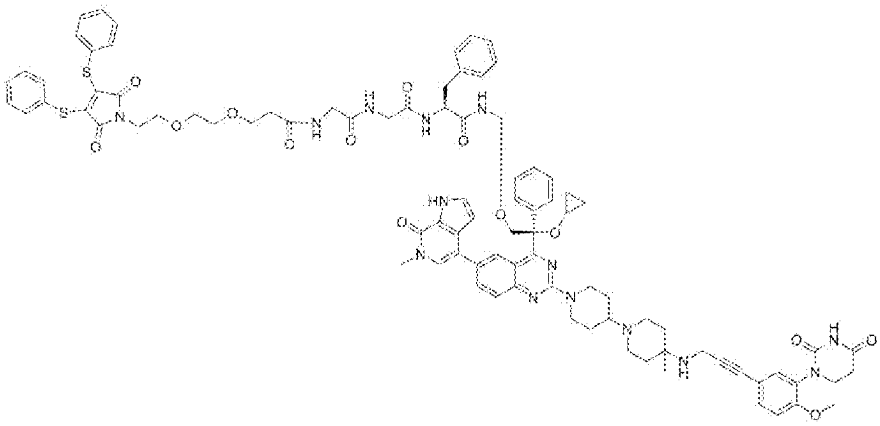

After obtaining a reaction intermediate from a formate of the (9H-fluoren-9-yl)methyl((S)-1-((((S)-2-cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl) amino)-1-oxo-3-phenylpropan-2-yl)carbamate of Production Example 207-3 (4.5 mg) and (((9H-fluoren-9-yl)methoxy) carbonyl)glycylglycine (1.7 mg, 0.0048 mmol) by the same method as Production Example 201-15, diethylamine (0.050 mL) was added and the mixture was stirred for 30 minutes at room temperature and then concentrated under reduced pressure to obtain crude product 1. A formate of the title compound (1.1 mg) was then obtained from crude product 1 and the tert-butyl 3-(2-(2-(2,5-dioxo-3,4-bis(phenylthio)-2,5-dihydro-1H-pyrrol-1-yl)ethoxy)ethoxy)propanoate of Production Example 201-22 (3.4 mg, 0.0065 mmol), by the same method as Example 205.
ESI-MS (m/z): 825.91 [(M+2H)/2]$^+$.

[Production Example 207-1]

(S)-(2-((((9H-Fluoren-9-yl)methoxy)carbonyl)amino)-3-phenylpropanamido)methyl acetate

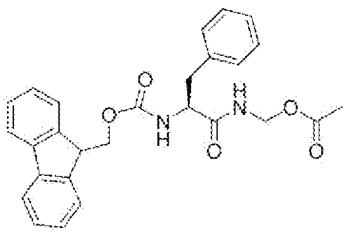

The title compound (0.72 g) was obtained from (((9H-fluoren-9-yl)methoxy)carbonyl)-L-phenylalanylglycine (2.4 g, 5.4 mmol), known from published literature, by the same method as Production Example 201-10.
ESI-MS (m/z): 797.40[2M-2OAc+H]$^+$.

[Production Example 207-2]

(S)-1-(5-(3-((1'-(4-(1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)dihydropyrimidine-2,4(1H,3H)-dione

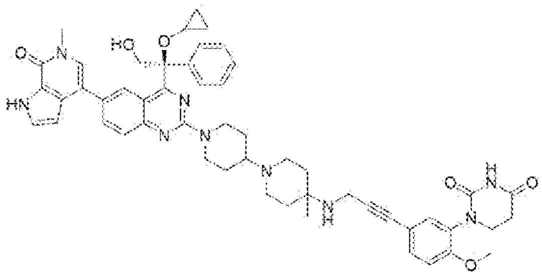

To a solution of the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 201-9 (250 mg, 0.364 mmol), the 1-(5-iodo-2-methoxyphenyl)dihydropyrimidine-2,4(1H,3H)-dione of Production Example 201-16 (252 mg, 0.729 mmol) and CuI (34.7 mg, 0.182 mmol) in DMF (5.65 mL) there were added N,N-diisopropylethylamine (0.637 mL, 3.65 mmol and tetrakis(triphenylphosphine)palladium(0) (211 mg, 0.182 mmol), and the mixture was stirred for 1 hour at 50°C under a nitrogen atmosphere. After cooling to room temperature, the reaction mixture was purified twice by direct ODS silica gel chromatography (0.1% formic acid aqueous solution:0.1% acetonitrile formate solution = 9:1 to 7:3), to obtain a crude product. The crude product was purified by NH silica gel column chromatography (n-heptane:ethyl acetate = 1: 1 to ethyl acetate to ethyl acetate:methanol = 7:3) to obtain the title compound (193 mg).
ESI-MS (m/z): 904.69 $[M+H]^+$.

[Production Example 207-3]

(9H-Fluoren-9-yl)methyl((S)-1-((((S)-2-cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-1-oxo-3-phenylpropan-2-yl)carbamate

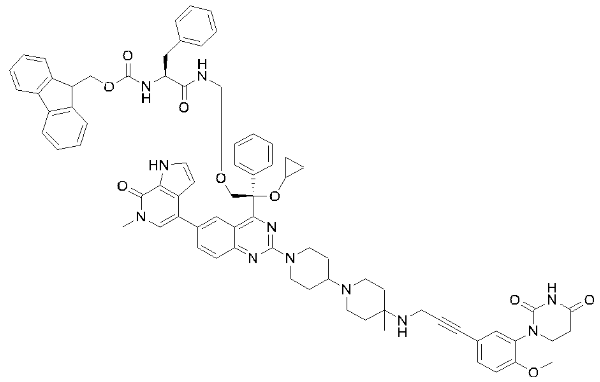

A formate of the title compound (4.5 mg) was obtained from the (S)-(2-((((9H-fluoren-9-yl)methoxy)carbonyl)amino)-3-phenylpropanamido)methyl acetate of Production Example 207-1 (37 mg, 0.080 mmol) and a formate of the (S)-1-(5-(3-((1'-(4-(1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)dihydropyrimidine-2,4(1H,3H)-dione of Production Example 207-2 (10 mg), by the same method as Production Example 201-11.
ESI-MS (m/z): 1302.74 $[M+H]^+$.

[Example 208]

(S)-N-((3-(5-(3-((1'-(4-((S)-1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)-2-(15-(2,5-dioxo-3,4-bis(phenylthio)-2,5-dihydro-1H-pyrrol-1-yl)-4,7-dioxo-10,13-dioxa-3,6-diazapentadecanamido)-3-phenylpropaneamide

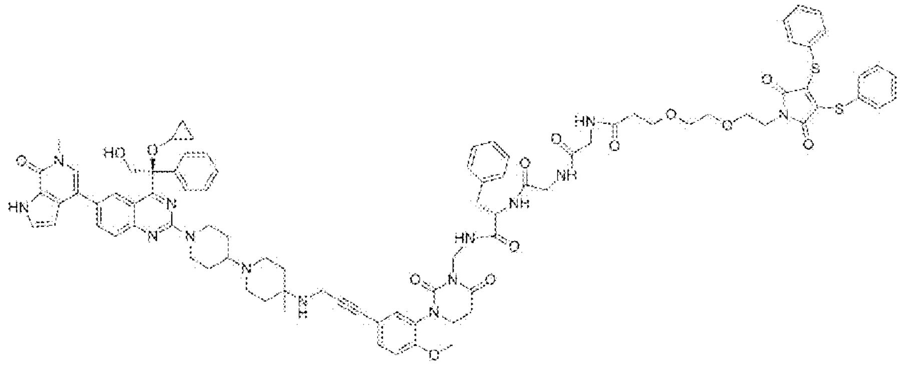

A formate of the title compound (2.1 mg) was obtained from the (S)-2-(2-(2-aminoacetamide)acetamide)-N-((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)-3-phenylpropaneamide of Production Example 208-3 (3.3 mg, 0.0026 mmol) and the tert-butyl 3-(2-(2-(2,5-dioxo-3,4-bis(phenylthio)-2,5-dihydro-1H-pyrrol-1-yl)ethoxy)ethoxy)propanoate of Production Example 201-22 (2.7 mg, 0.0051 mmol), by the same method as Example 205.

ESI-MS (m/z): 825.91 [(M+2H)/2]+.

[Production Example 208-1]

(9H-Fluoren-9-yl)methyl(S)-(1-(((3-(5-iodo-2-methoxyphenyl)-2,6-dioxotetrahydropyrimidine-1(2H)-yl)methyl)amino)-1-oxo-3-phenylpropan-2-yl)carbamate

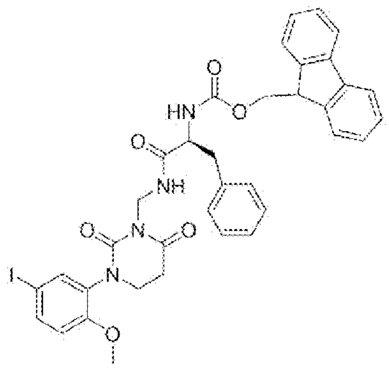

The title compound (70 mg) was obtained from the 1-(5-iodo-2-methoxyphenyl)dihydropyrimidine-2,4(1H,3H)-dione of Production Example 201-16 (100 mg, 0.289 mmol) and the (S)-(2-((((9H-fluoren-9-yl)methoxy)carbonyl)amino)-3-phenylpropanamido)methyl acetate of Production Example 207-1 (199 mg, 0.433 mmol), by the same method as Production Example 201-18.

ESI-MS (m/z): 745.24 [M+H]+.

[Production Example 208-2]

(S)-2-Amino-N-((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)-3-phenylpropaneamide

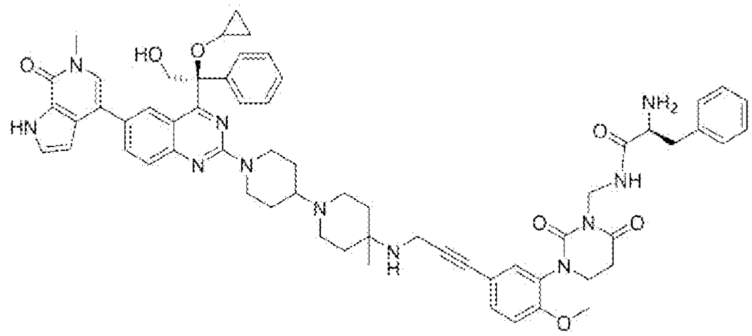

A formate of the title compound (13 mg) was obtained from the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo [2,3-c]pyridin-7-one of Production Example 201-9 (16 mg, 0.023 mmol) and the (9H-fluoren-9-yl)methyl(S)-(1-(((3-(5-iodo-2-methoxyphenyl)-2,6-dioxotetrahydropyrimidine-1(2H)-yl)methyl)amino)-1-oxo-3-phenylpropan-2-yl)carbamate of Production Example 208-1 (26 mg, 0.035 mmol), by the same method as Production Example 204-5.
ESI-MS (m/z): 1080.64 [M+H]$^+$.

[Production Example 208-3]

(S)-2-(2-(2-Aminoacetamide)acetamide)-N-((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidine] -4-yl)amino)prop-1-yn-1-yl)-2-methoxyphenyl)-2,6-dioxotetrahydropyrimidine-1(2H)-yl)methyl)-3-phenylpropaneamide

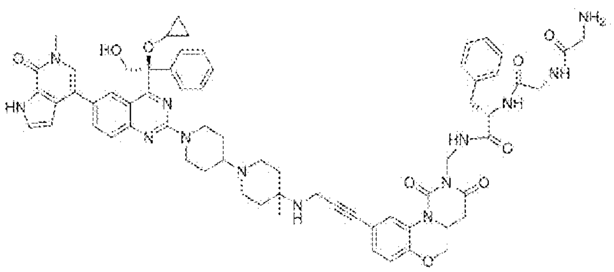

After obtaining a reaction intermediate from a formate of the (S)-2-amino-N-((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl) methyl)-3-phenylpropaneamide of Production Example 208-2 (6.0 mg) and commercially available (((9H-fluoren-9-yl) methoxy)carbonyl)glycylglycine (3.6 mg, 0.010 mmol) by the same method as Production Example 201-15, diethylamine (0.016 mL) was added and the mixture was stirred for 30 minutes at room temperature, and the reaction mixture was purified by direct reversed-phase silica gel chromatography (0.1% formic acid aqueous solution:0.1% acetonitrile formate solution = 95:5 to 70:30) to obtain a formate of the title compound (4.0 mg).
ESI-MS (m/z): 1194.74 [M+H]$^+$.

[Example 209]

(S)-N-(((S)-2-Cyclopropoxy-2-(2-(4-((3-(3-(2,4-dioxotetrahydropyrimidine-1(2H)-yl)-4-methoxyphenyl)prop-2-yn-1-yl) amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)-2-(2-(3-(2-(2-(2,5-dioxo-3,4-bis(phenylthio)-2,5-dihydro-1H-pyrrol-1-yl)ethoxy)ethoxy)propanamido)acetamide)-3-phenylpropaneamide

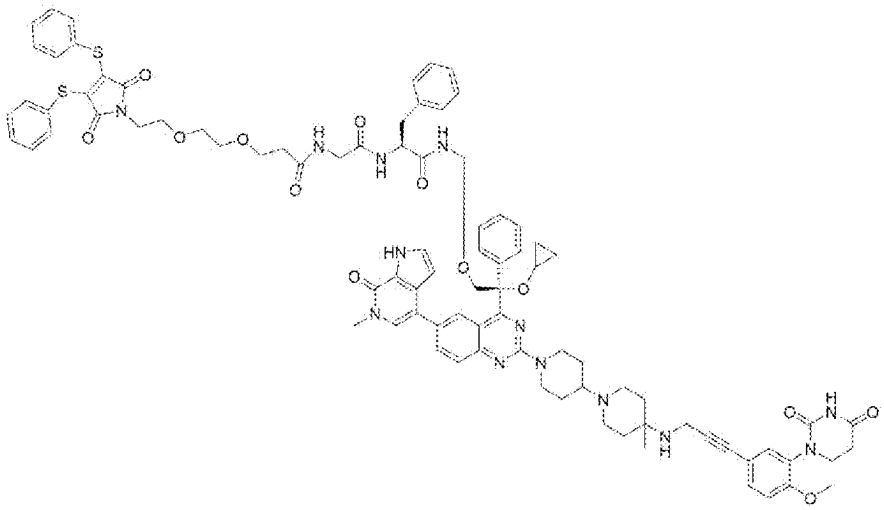

After obtaining a reaction intermediate from a formate of the (9H-fluoren-9-yl)methyl((S)-1-((((S)-2-cyclopropoxy-2-(2-(4-((3-(3-(2,4-dioxotetrahydropyrimidine- 1(2H)-yl)-4-methoxyphenyl)prop-2-yn-1-yl)amino)-4-methyl-[1,4'-bipiperidine] -1'-yl)-6-(6-methyl-7-oxo-6,7-dihydro-1H-pyrrolo[2,3-c]pyridin-4-yl)quinazolin-4-yl)-2-phenylethoxy)methyl)amino)-1-oxo-3-phenylpropan-2-yl)carbamate of Production Example 207-3 (5.5 mg) and (((9H-fluoren-9-yl)methoxy)carbonyl)glycine (1.8 mg, 0.0059 mmol) by the same method as Production Example 201-15, diethylamine (0.020 mL, 0.20 mmol) was added and the mixture was stirred for 30 minutes at room temperature and then concentrated under reduced pressure to obtain crude product 1. A formate of the title compound (3.0 mg) was then obtained from crude product 1 and the 2,5-dioxopyrrolidin-1-yl 3-(2-(2-(2,5-dioxo-3,4-bis(phenylthio)-2,5-dihydro-1H-pyrrol-1-yl)ethoxy)ethoxy)propanoate of Production Example 201-23 (3.6 mg, 0.0063 mmol), by the same method as Example 201.
ESI-MS (m/z): 797.47 [(M+2H)/2]$^+$.

[Example 210]

(S)-N-((3-(5-(3-((1'-(4-((S)-1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)-2-(2-(3-(2-(2-(2,5-dioxo-3,4-bis(phenylthio)-2,5-dihydro-1H-pyrrol-1-yl)ethoxy)ethoxy)propanamido)acetamide)-3-phenylpropaneamide

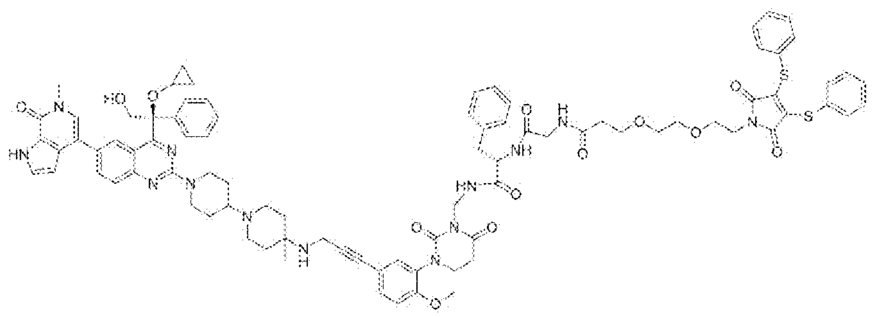

The same methods as in Production Example 208-3 and Example 205 were carried out consecutively to obtain a formate of the title compound (1.8 mg) from the formate of (S)-2-amino-N-((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)-3-phenylpropaneamide of Production Example 208-2 (3.0 mg), (((9H-fluoren-9-yl)methoxy)carbonyl)glycine (1.1 mg, 0.0038 mmol) and the *tert*-butyl 3-(2-(2-(2,5-dioxo-3,4-bis(phenylthio)-2,5-dihydro-1H-pyrrol-1-yl)ethoxy)ethoxy)propanoate of Production Example 201-22 (2.7 mg, 0.0051 mmol).
ESI-MS (m/z): 797.40 [(M+2H)/2]$^+$.

[Example 211]

(S)-N-(2-((((S)-2-Cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)-2-(3-(2-(2-(2,5-dioxo-3,4-bis(phenylthio)-2,5-dihydro-1H-pyrrol-1-yl)ethoxy)ethoxy)propaneamide)-3-phenylpropaneamide

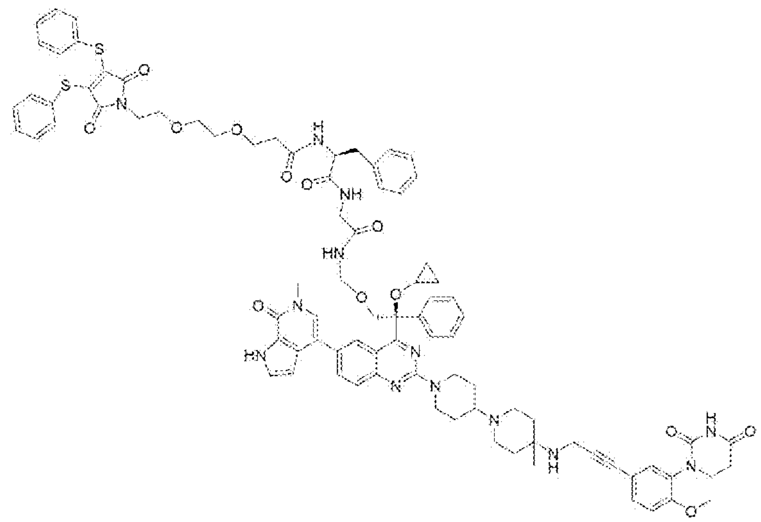

A formate of the title compound (0.89 mg) was obtained from a formate of the (9H-fluoren-9-yl)methyl (S)-(2-(((2-cyclopropoxy-2-(2-(4-((3-(3-(2,4-dioxotetrahydropyrimidine-1(2H))-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamate of Production Example 211-1 (10 mg), (((9H-fluoren-9-yl)oxy)carbonyl)-L-phenylalanine (4.3 mg, 0.011 mmol) and the 2,5-dioxopyrrolidin-1-yl 3-(2-(2-(2,5-dioxo-3,4-bis(phenylthio)-2,5-dihydro-1H-pyrrol-1-yl)ethoxy)ethoxy)propanoate of Production Example 201-23 (3.6 mg, 0.0063 mmol), by the same method as Example 209.

ESI-MS (m/z): 797.51 $[(M+2H)/2]^+$.

[Production Example 211-1]

(9H-Fluoren-9-yl)methyl (S)-(2-(((2-cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-bipiperidine] -1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamate

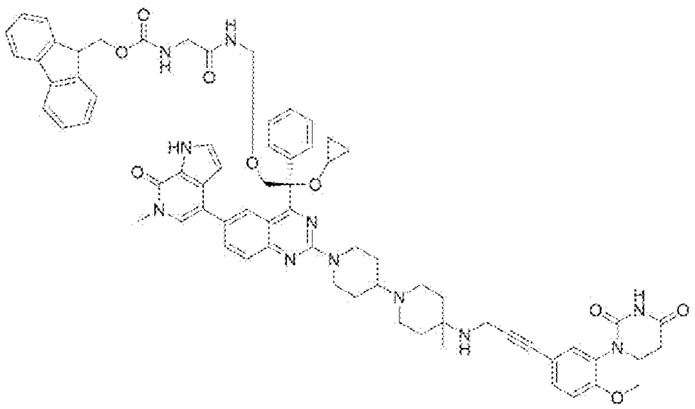

A formate of the title compound (19 mg) was obtained from the (2-((((9H-fluoren-9-yl)methoxy)carbonyl)amino) acetamide)methyl acetate of Production Example 201-10 (98 mg, 0.27 mmol) and the (S)-1-(5-(3-((1'-(4-(1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)dihydropyrimidine-2,4(1H,3H)-dione of Production Example 207-2 (30 mg, 0.033 mmol), by the same method as Production Example 201-11.
ESI-MS (m/z): 1212.59 $[M+H]^+$.

[Example 212]

(S)-N-(2-(((3-(5-(3-((1'-(4-((S)-1-Cyclopropoxy-2-hydroxy-1-phenylethyl)-6-(6-methyl-7-oxo-6,7-dihydro-1H-pyrrolo [2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-2-oxoethyl)-2-(3-(2-(2-(2,5-dioxo-3,4-bis(phenylthio)-2,5-dihydro-1H-pyrrol-1-yl)ethoxy)ethoxy)propaneamide)-3-phenylpropaneamide

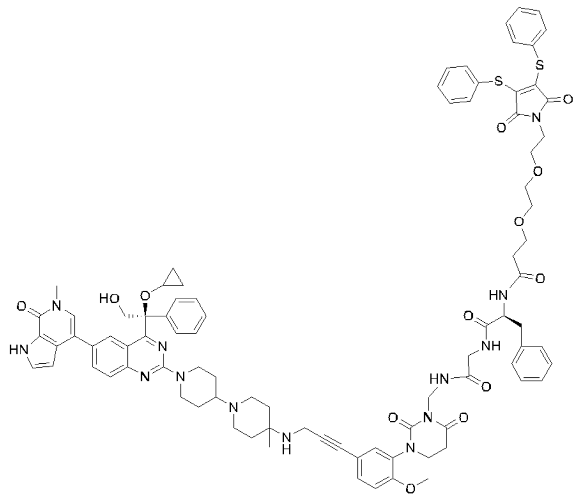

The same methods as in Production Example 208-3 and Example 205 were carried out consecutively to obtain a formate of the title compound (2 mg) from the formate of (S)-2-amino-N-((3-(5-(3-((1'-(4-(1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)acetamide of Production Example 212-1 (9 mg), (((9H-fluoren-9-yl)oxy)carbonyl)-L-phenylalanine (4.8 mg, 0.012 mmol) and the *tert-butyl* 3-(2-(2-(2,5-dioxo-3,4-bis(phenylthio)-2,5-dihydro-1H-pyrrol-1-yl)ethoxy)ethoxy)propanoate of Production Example 201-22 (3.5 mg, 0.0067 mmol).
ESI-MS (m/z): 797.40 $[(M+2H)/2]^+$.

[Production Example 212-1]

(S)-2-Amino-N-((3-(5-(3-((1'-(4-(1-cyclopropoxy-2-hydroxy-1-phenylethyl)-6-(6-methyl-7-oxo-6,7-dihydro-1H-pyrrolo [2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)acetamide

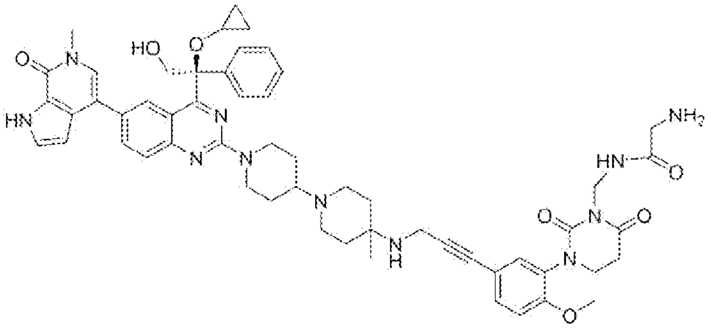

A formate of the title compound (16 mg) was obtained from the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo [2,3-c]pyridin-7-one of Production Example 201-9 (15 mg, 0.022 mmol) and the (9H-fluoren-9-yl)methyl(2-(((3-(5-iodo-2-methoxyphenyl)-2,6-dioxotetrahydropyrimidine-1(2H)-yl)methyl)amino)-2-oxoethyl)carbamate of Production Example 206-1 (29 mg, 0.044 mmol), by the same method as Production Example 204-5.
ESI-MS (m/z): 990.57 $[M+H]^+$.

[Example 213]

(S)-N-(2-((((S)-2-Cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)-2-(2-(3-(2-(2-(2,5-dioxo-3,4-bis(phenylthio)-2,5-dihydro-1H-pyrrol-1-yl) ethoxy)ethoxy)propanamido)acetamide)-3-phenylpropaneamide

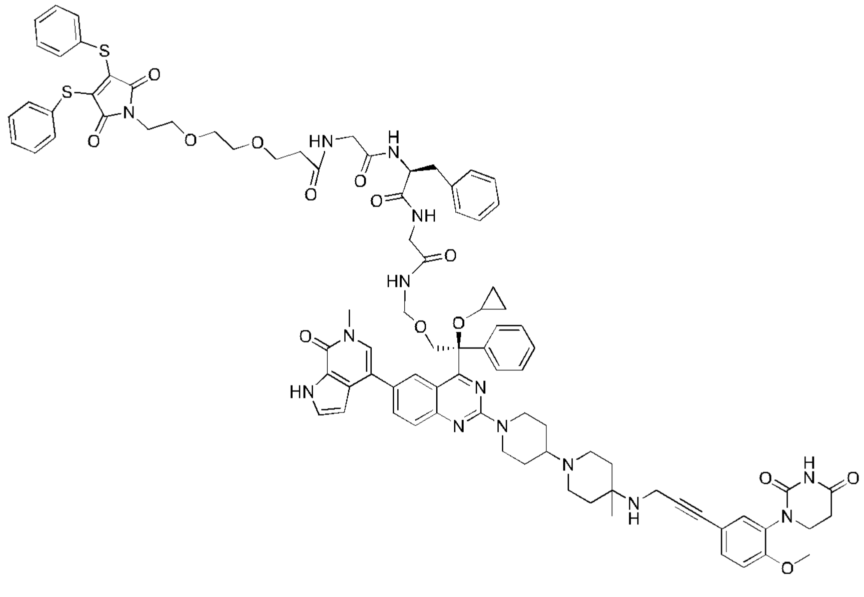

A formate of the title compound (1.6 mg) was obtained from the formate of (9H-fluoren-9-yl)methyl(S)-(2-(((2-cyclopropoxy-2-(2-(4-((3-(3-(2,4-dioxotetrahydropyrimidine-1(2H))-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamate of Production Example 211-1 (10 mg), (((9H-fluoren-9-yl)oxy)carbonyl)-L-phenylalanine (4.3 mg, 0.011 mmol), (((9H-fluoren-9-yl)methoxy)carbonyl)glycine (2.2 mg, 0.0077 mmol) and the 2,5-dioxopyrrolidin-1-yl 3-(2-(2-(2,5-dioxo-3,4-bis(phenylthio)-2,5-dihydro-1H-pyrrol-1-yl)ethoxy)ethoxy)propanoate of Production Example 201-23 (3.6 mg, 0.0063 mmol), by the same method as Example 209. ESI-MS (m/z): 826.14 $[(M+2H)/2]^+$.

[Example 214]

(S)-N-(2-(((3-(5-(3-((1'-(4-((S)-1-Cyclopropoxy-2-hydroxy-1-phenylethyl)-6-(6-methyl-7-oxo-6,7-dihydro-1H-pyrrolo [2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-2-oxoethyl)-2-(2-(3-(2-(2-(2,5-dioxo-3,4-bis(phenylthio)-2,5-dihydro-1H-pyrrol-1-yl)ethoxy)ethoxy)propanamido)acetamide)-3-phenylpropaneamide

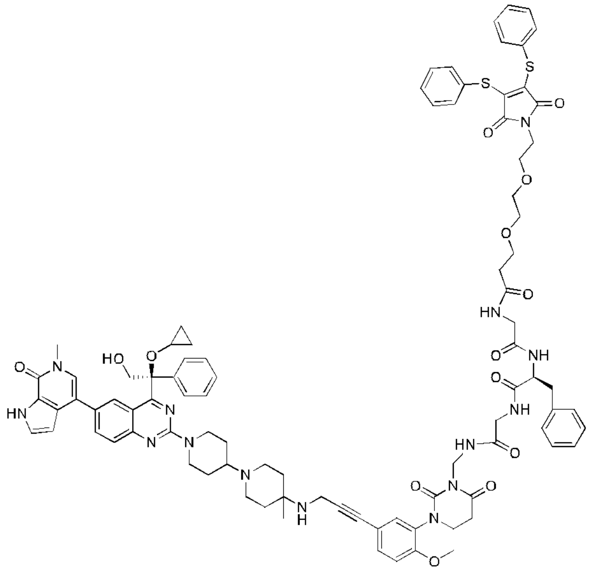

The same methods as in Production Example 208-3 and Example 205 were carried out consecutively to obtain a formate of the title compound (1 mg) from the formate of (S)-2-amino-N-((3-(5-(3-((1 '-(4-(1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidine] -4-yl)amino)prop-1-yn-1-yl)-2-methoxyphenyl)-2,6-dioxotetrahydropyrimidine-1(2H)-yl)methyl)acetamide of Production Example 212-1 (9 mg), (((9H-fluoren-9-yl)oxy)carbonyl)-L-phenylalanine (4.8 mg, 0.012 mmol), (((9H-fluoren-9-yl) methoxy)carbonyl)glycine (2.5 mg, 0.0083 mmol) and the tert-butyl 3-(2-(2-(2,5-dioxo-3,4-bis(phenylthio)-2,5-dihydro-1H-pyrrol-1-yl)ethoxy)ethoxy)propanoate of Production Example 201-22 (5.3 mg, 0.010 mmol).
ESI-MS (m/z): 825.87 [(M+2H)/2]$^+$.

[Example 215]

N-((1S,10S)-10-Benzyl-1-cyclopropoxy-1-(2-(4-((3-(3-(2,4-dioxotetrahydropyrimidine-1(2H)-yl)-4-methoxyphenyl) prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-6,9,12,15-tetraoxo-1-phenyl-3-oxa-5,8,11,14-tetraazahexadecan-16-yl)-6-(2,5-dioxo-3,4-bis(phenylthio)-2,5-dihydro-1H-pyrrol-1-yl)hexaneamide

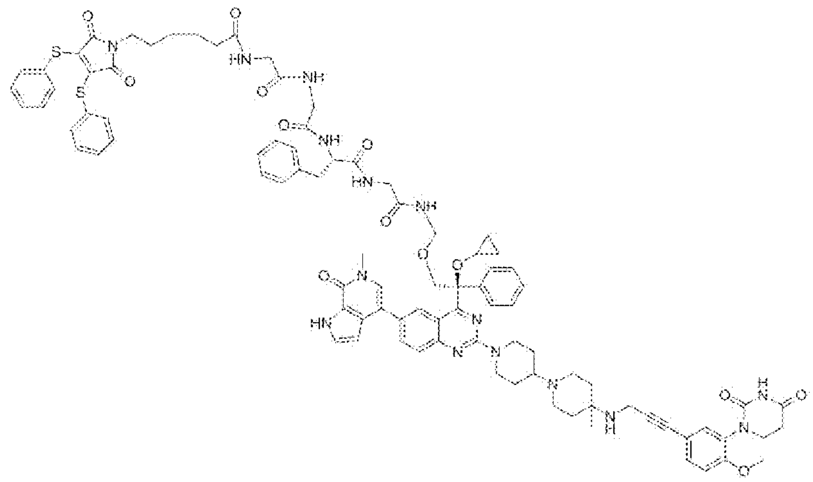

Diethylamine (0.013 mL, 0.128 mmol) was added to the formate of (9H-fluoren-9-yl)methyl ((1S,10S)-10-benzyl-1-cyclopropoxy-1-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-6,9,12,15-tetraoxo-1-phenyl-3-oxa-5,8,11,14-tetraazahexadecan-16-yl)carbamate of Production Example 215-1 (2.0 mg), and the mixture was stirred for 20 minutes at room temperature and concentrated under reduced pressure to obtain crude product 1. A formate of the title compound (1.4 mg) was then obtained from crude product 1 and the tert-butyl 6-(2,5-dioxo-3,4-bis(phenylthio)-2,5-dihydro-1H-pyrrol-1-yl)hexanoate of Production Example 215-2 (1.2 mg, 0.0026 mmol), by the same method as Example 205.

ESI-MS (m/z): 831.56 [(M+2H)/2]$^+$.

[Production Example 215-1]

(9H-Fluoren-9-yl)methyl((1S,10S)-10-benzyl-1-cyclopropoxy-1-(2-(4-((3-(3-(2,4-dioxotetrahydropyrimidine-1(2H))-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-6,9,12,15-tetraoxo-1-phenyl-3-oxa-5,8,11,14-tetraazahexadecan-16-yl)carbamate

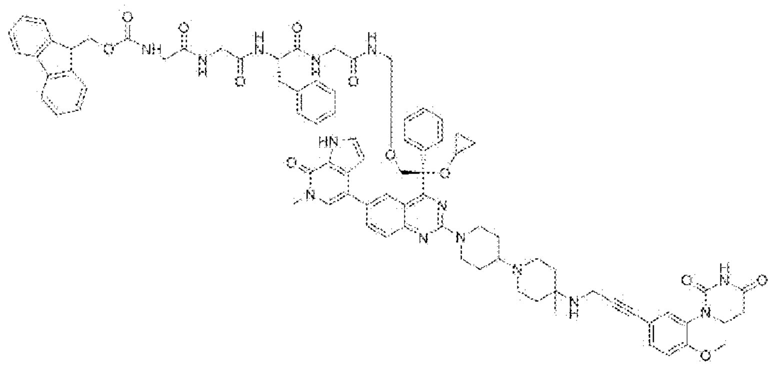

A formate of the title compound (18 mg) was obtained from the formate of (9H-fluoren-9-yl)methyl (S)-(2-(((2-cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamate of Production Example 211-1 (23 mg), by the same method as Production Example 203-1.

ESI-MS (m/z): 737.99 [(M+2H)/2]$^+$.

[Production Example 215-2]

*tert*-Butyl 6-(2,5-dioxo-3,4-bis(phenylthio)-2,5-dihydro-1H-pyrrol-1-yl)hexanoate

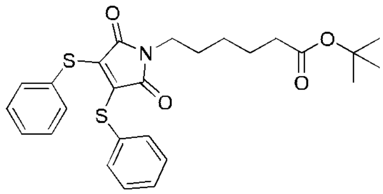

After adding commercially available tert-butyl 6-hydroxyhexanoate (0.99 mL, 5.1 mmol) to a solution of triphenylphosphine (1.6 g, 6.3 mmol) in THF (25 mL) at 0°C and stirring the mixture for 15 minutes, 3,4-dibromo-1H-pyrrole-2,5-dione (1.0 g, 3.9 mmol) was added. After the solution became transparent, diisopropyl (E)-diazene-1,2-dicarboxylate (1.9 M solution, 4.1 mL, 7.8 mmol) was added and the mixture was increased in temperature to 20°C and stirred for 3 hours. The reaction mixture was concentrated under reduced pressure, and then the residue was purified by silica gel column chromatography (n-heptane:ethyl acetate = 1:0 to 1:1) to obtain a crude product (1.3 g). The crude product (1.1 g) and thiophenol (0.53 mL, 5.2 mmol) were dissolved in dichloromethane (22 mL), N,N-diisopropylethylamine (2.3 mL, 12.9 mmol) was added at 20°C, and the mixture was stirred for 40 minutes. The reaction mixture was purified by direct silica gel column chromatography (*n*-heptane:ethyl acetate = 1:0 to 1:1) to obtain the title compound (0.21 g). $^1$H-NMR Spectrum (400 MHz, $CDCl_3$) $\delta$(ppm): 1.42 (9H, s), 1.51-1.61 (6H, m), 2.13-2.19 (2H, m), 3.45-3.50 (2H, m), 7.17-7.29(10H, m).

[Example 216]

(S)-N-(2-((((S)-2-Cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)-2-(2-(2-(3-(2,5-dioxo-3,4-bis(phenylthio)-2,5-dihydro-1H-pyrrol-1-yl)propanamido)acetamide)acetamide)-3-phenylpropaneamide

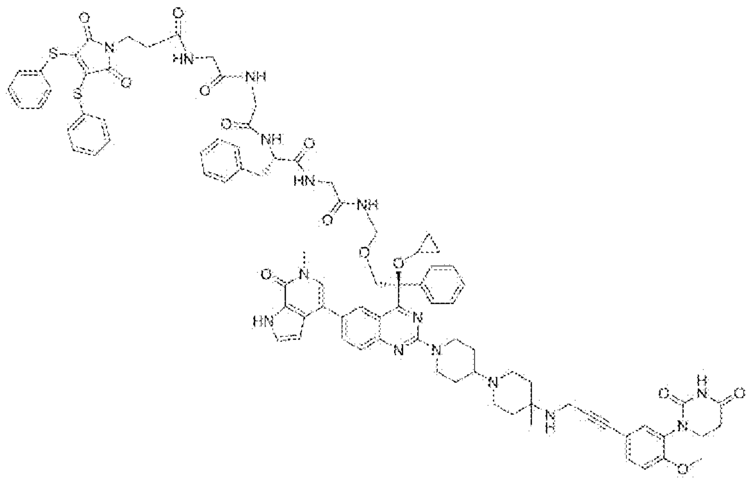

A formate of the title compound (1.3 mg) was obtained from the formate of (9H-fluoren-9-yl)methyl((1S,10S)-10-benzyl-1-cyclopropoxy-1-(2-(4-((3-(3-(2,4-dioxotetrahydropyrimidine-1(2H))-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-6,9,12,15-tetraoxo-1-phenyl-3-oxa-5,8,11,14-tetraazahexadecan-16-yl)carbamate of Production Example 215-1 (2.0 mg) and the *tert*-butyl 3-(2,5-dioxo-3,4-bis(phenylthio)-2,5-dihydro-1H-pyrrol-1-yl)propanoate of Production Example 216-2 (1.1 mg, 0.0026 mmol), by the same method as Example 215.

ESI-MS (m/z): 810.52 $[(M+2H)/2]^+$.

[Production Example 216-1]

*tert-Butyl* 3-(3,4-dibromo-2,5-dioxo-2,5-dihydro-1H-pyrrol-1-yl)propanoate

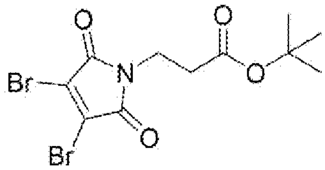

The title compound (1.57 g) was obtained from 2,3-dibromomaleimide (2.00 g, 7.85 mmol) and *tert-butyl* 3-aminopropanoate hydrochloride (1.57 g, 8.63 mmol) by the same method as Production Example 201-21.

$^1$H-NMR Spectrum (500 MHz, $CDCl_3$) $\delta$(ppm): 1.41 (9H, s), 2.57 (2H, t, J=7.03 Hz), 3.80-3.95 (2H, m).

[Production Example 216-2]:

*tert*-Butyl 3-(2,5-dioxo-3,4-bis(phenylthio)-2,5-dihydro-1H-pyrrol-1-yl)propanoate

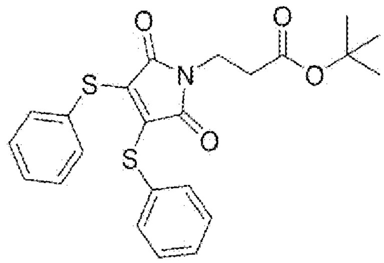

The title compound (595 mg) was obtained from the *tert*-butyl 3-(3,4-dibromo-2,5-dioxo-2,5-dihydro-1H-pyrrol-1-yl)propanoate of Production Example 216-1 (532 mg, 1.39 mmol) by the same method as Production Example 201-22.

$^1$H-NMR Spectrum (500 MHz, $CDCl_3$) $\delta$(ppm): 1.41 (9H, s), 2.51 (2H, t, J=7.34 Hz), 3.75 (2H, t, J=7.34 Hz), 7.18-7.32(10H, m).

[Example 217]

(S)-N-(2-((((S)-2-Cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)-2-(2-(2-(3-(2-(2,5-dioxo-3,4-bis(phenylthio)-2,5-dihydro-1H-pyrrol-1-yl)ethoxy)propanamido)acetamide)acetamide)-3-phenylpropaneamide

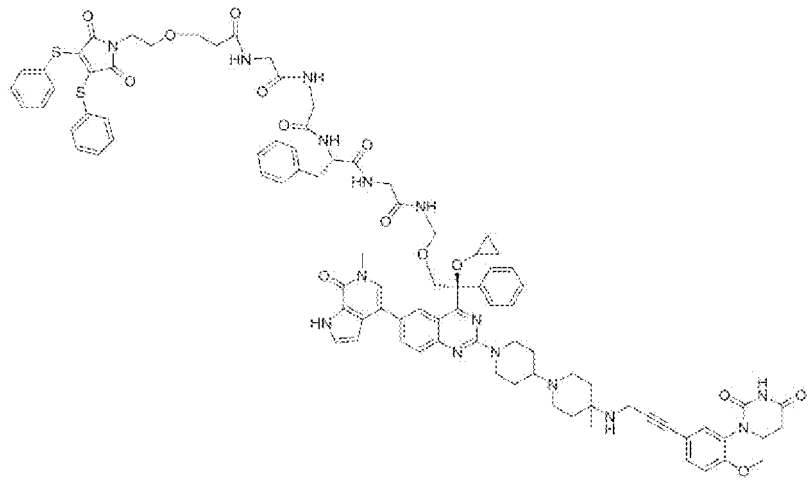

A formate of the title compound (1.3 mg) was obtained from the formate of (9H-fluoren-9-yl)methyl ((1S,10S)-10-benzyl-1-cyclopropoxy-1-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-6,9,12,15-tetraoxo-1-phenyl-3-oxa-5,8,11,14-tetraazahexadecan-16-yl)carbamate of Production Example 215-1 (2.0 mg) and the *tert-butyl* 3-(2-(2,5-dioxo-3,4-bis(phenylthio)-2,5-dihydro-1H-pyrrol-1-yl)ethoxy)propanoate of Production Example 217-2 (1.2 mg, 0.0026 mmol), by the same method as Example 215.
ESI-MS (m/z): 832.50 [(M+2H)/2]$^+$.

[Production Example 217-1]

*tert*-Butyl 3-(2-(3,4-dibromo-2,5-dioxo-2,5-dihydro-1H-pyrrol-1-yl)ethoxy)propanoate

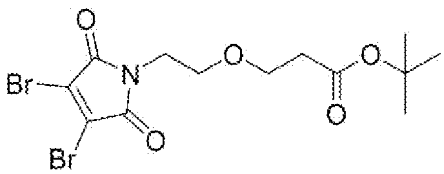

The title compound (2.63 g) was obtained from 2,3-dibromomaleimide (2.00 g, 7.85 mmol) and *tert*-butyl 3-(2-aminoethoxy)propanoate (1.49 g, 7.85 mmol) by the same method as Production Example 201-21.
$^1$H-NMR Spectrum (500 MHz, $CDCl_3$) δ(ppm): 1.42 (9H, s), 2.42 (2H, t, J=6.4 Hz), 3.61-3.67 (4H, m), 3.77-3.80 (2H, m).

[Production Example 217-2]:

*tert*-Butyl 3-(2-(2,5-dioxo-3,4-bis(phenylthio)-2,5-dihydro-1H-pyrrol-1-yl)ethoxy)propanoate

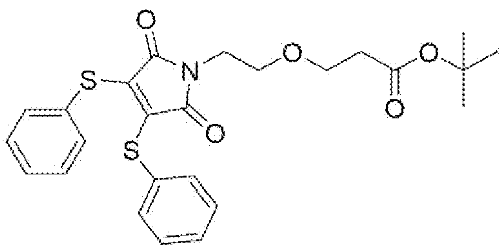

The title compound (474 mg) was obtained from the *tert*-butyl 3-(2-(3,4-dibromo-2,5-dioxo-2,5-dihydro-1H-pyrrol-1-yl)ethoxy)propanoate of Production Example 217-1 (600 mg, 1.41 mmol) by the same method as Production Example 201-22.

$^1$H-NMR Spectrum (400 MHz, $CDCl_3$) δ(ppm): 1.43 (9H, s), 2.40 (2H, t, J=6.6 Hz), 3.58 (2H, d, J=5.4 Hz), 3.62-3.69 (4H, m), 7.17-7.30(10H, m).

[Example 218]

(S)-N1-((3-(5-(3-((1'-(4-((S)-1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)-2-(3-(2-(2-(2,5-dioxo)-3,4-bis(phenylthio)-2,5-dihydro-1H-pyrrol-1-yl)ethoxy)ethoxy)propanamido) succinamide

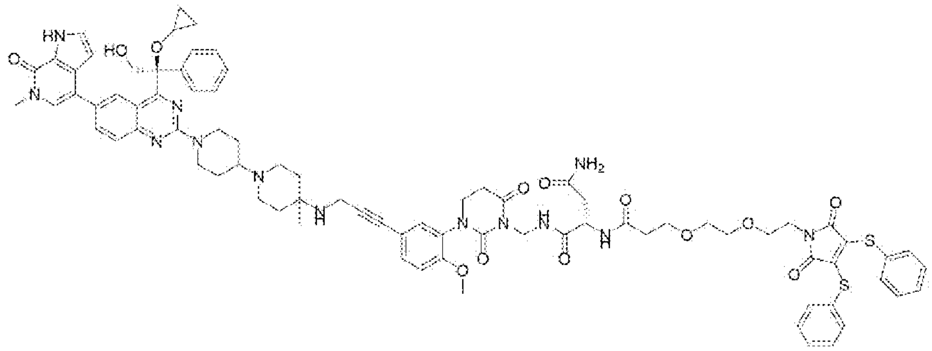

To a solution of the (9H-fluoren-9-yl)methyl ((S)-4-amino-1-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-1,4-dioxobutan-2-yl)carbamate of Production Example 218-2 (5.1 mg, 4.0 μmol) in DMF (2 mL) there was added diethylamine (200 μL), and the mixture was stirred for 30 minutes at room temperature. The reaction mixture was concentrated under reduced pressure to obtain intermediate 1. To a solution of the *tert*-butyl 3-(2-(2-(2,5-dioxo-3,4-bis(phenylthio)-2,5-dihydro-1H-pyrrol-1-yl)ethoxy)ethoxy)propionate of Production Example 201-22 (2.77 mg, 5.22 μmol) in dichloromethane (2 mL) there was added TFA(400 μL), and the mixture was stirred for 45 minutes at room temperature. The reaction mixture was concentrated under reduced pressure, and then N,N-diisopropylethylamine (200 μL, 1.15 mmol) and HATU (1.99 mg, 5.22 μmol) were added to a solution of the residue in DMF (2 mL). A solution of intermediate 1 in DMF (2 mL) was added to the reaction mixture at room temperature, and the mixture was stirred for 75 minutes. The reaction mixture was concentrated under reduced pressure and the residue was purified by preparative HPLC (0.1% formic acid aqueous solution:0.1% acetonitrile formate solution = 7:3 to 2:3) to obtain the title compound (1.5 mg).
ESI-MS (m/z): 1503.69 [M+H]$^+$

[Production Example 218-1]

(9H-Fluoren-9-yl)methyl (S)-(4-amino-1-(((3-(5-iodo-2-methoxyphenyl)-2,6-dioxotetrahydropyrimidine-1(2H)-yl)methyl)amino)-1,4-dioxobutan-2-yl)carbamate

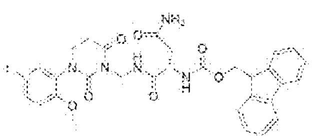

The title compound (88 mg) was obtained from the (S)-(2-((((9H-fluoren-9-yl)methoxy)carbonyl)amino)-4-amino-4-oxobutanamido)methyl acetate of Production Example 204-3 (200 mg, 0.578 mmol) by the same method as Production Example 201-18. ESI-MS (m/z): 712.26 [M+H]$^+$

[Production Example 218-2]

(9H-Fluoren-9-yl)methyl ((S)-4-amino-1-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-1,4-dioxobutan-2-yl)carbamate

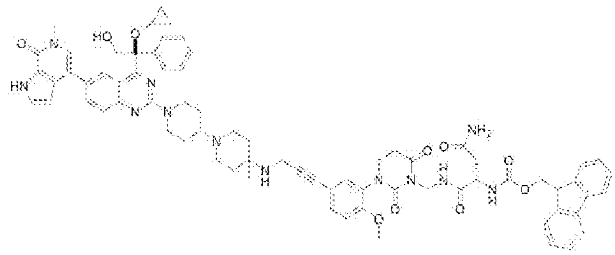

To a solution of the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 201-9 (16 mg, 0.023 mmol) and the (9H-fluoren-9-yl)methyl (S)-(4-amino-1-(((3-(5-iodo-2-methoxyphenyl)-2,6-dioxotetrahydropyrimidine-1(2H)-yl)methyl)amino)-1,4-dioxobutan-2-yl)carbamate of Production Example 218-1 (19.9 mg, 0.028 mmol) in DMF (2 mL) there were added CuI (1.78 mg, 9.33 μmol), N,N-diisopropylethylamine (40 μL, 0.23 mmol) and tetrakis(triphenylphosphine)palladium(0) (10.8 mg, 9.33 μmol), and the mixture was stirred for 2 hours at 50°C. The reaction mixture was purified by direct reversed-phase silica gel chromatography (0.1% formic acid aqueous solution:0.1% acetonitrile formate solution = 95:5 to 1:4) to obtain the title compound (13 mg).
ESI-MS (m/z): 1269.68 $[M+H]^+$

[Example 219]

(S)-N1-(((S)-2-Cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)-2-(3-(2-(2-(2,5-dioxo-3,4-bis(phenylthio)-2,5-dihydro-1H-pyrrol-1-yl)ethoxy)ethoxy)propanamido) succinamide

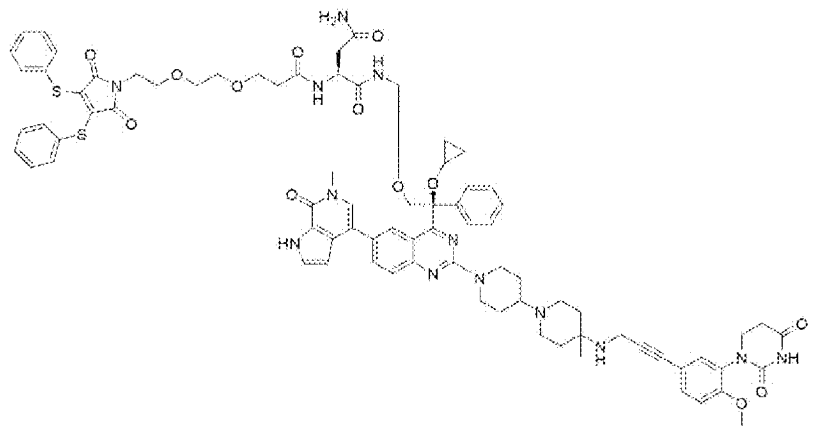

The title compound (1.2 mg) was obtained from the (9H-fluoren-9-yl)methyl((S)-4-amino-1-((((S)-2-cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-1,4-dioxobutan-2-yl)carbamate of Production Example 219-1 (4.6 mg, 3.6 μmol), by same method as Example 218.
ESI-MS (m/z): 1503.61 $[M+H]^+$

[Production Example 219-1]

(9H-Fluoren-9-yl)methyl((S)-4-amino-1-((((S)-2-cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-1,4-dioxobutan-2-yl)carbamate

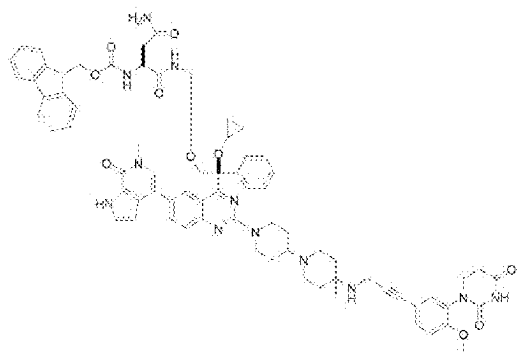

To a solution of the (S)-1-(5-(3-((1'-(4-(1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)dihydropyrimidine-2,4(1H,3H)-dione of Production Example 207-2 (12 mg, 0.013 mmol) and the (S)-(2-((((9H-fluoren-9-yl)methoxy)carbonyl)amino)-4-amino-4-oxobutanamido)methyl acetate of Production Example 204-3 (22.6 mg, 0.053 mmol) in THF (1 mL)/dichloromethane (1 mL) there was added CSA (12.0 mg, 0.053 mmol), and the mixture was stirred for 3 hours at 50°C. The reaction mixture was purified by direct reversed-phase silica gel chromatography (0.1% formic acid aqueous solution:0.1% acetonitrile formate solution = 95:5 to 0:100) to obtain the title compound (4.6 mg).
ESI-MS (m/z): 1269.87 $[M+H]^+$.

[Example 220]

(S)-N1-((3-(5-(3-((1'-(4-((S)-1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)-2-(2-(3-(2-(2-(2,5-dioxo-3,4-bis(phenylthio)-2,5-dihydro-1H-pyrrol-1-yl)ethoxy)ethoxy)propanamido)acetamide) succinamide

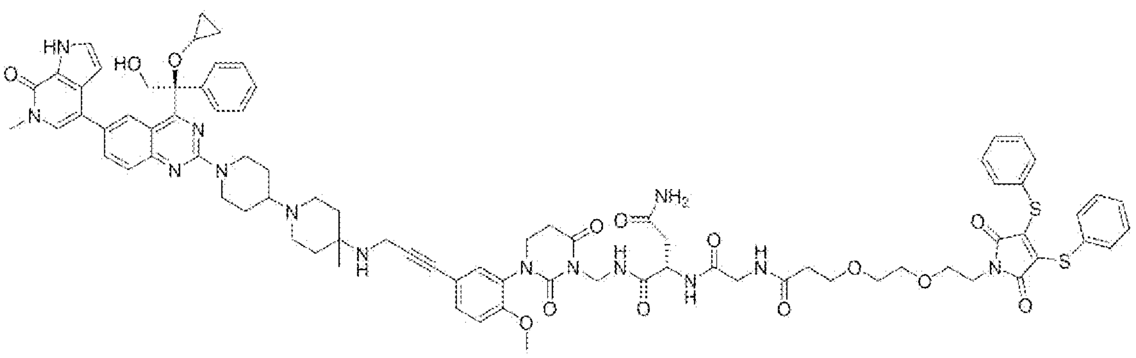

To a solution of the (9H-fluoren-9-yl)methyl ((S)-4-amino-1-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-1,4-dioxobutan-2-yl)carbamate of Production Example 218-2 (8.0 mg, 6.3 μmol) in DMF (1 mL) there was added diethylamine (100 μL), and the mixture was stirred for 1 hour at room temperature. The reaction mixture was concentrated under reduced pressure, and then (((9H-fluoren-9-yl)methoxy)carbonyl)glycine (1.87 mg, 6.30 μmol), N,N-diisopropylethylamine (100 μL, 0.573 mmol) and HATU (3.12 mg, 8.19 μmol) were added to a solution of the residue in DMF (1 mL), and the mixture was stirred

for 1 hour at room temperature. Diethylamine (100 μL) was added to the reaction mixture, which was then stirred for 30 minutes at room temperature and subsequently concentrated under reduced pressure, to obtain intermediate 1. The title compound (0.93 mg) was then obtained from intermediate 1 by the same method as Example 218.

ESI-MS (m/z): 1559.79 [M+H]+.

[Example 221]

(S)-4-(((3-(5-(3-((1'-(4-((S)-1-Cyclopropoxy-2-hydroxy-1-phenylethyl)-6-(6-methyl-7-oxo-6,7-dihydro-1H-pyrrolo[2,3-C]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-3-(3-(2-(2-(2,5-dioxo-3,4-bis(phenylthio)-2,5-dihydro-1H-pyrrol-1-yl)ethoxy)ethoxy)propanamido)-4-oxobutanoic acid

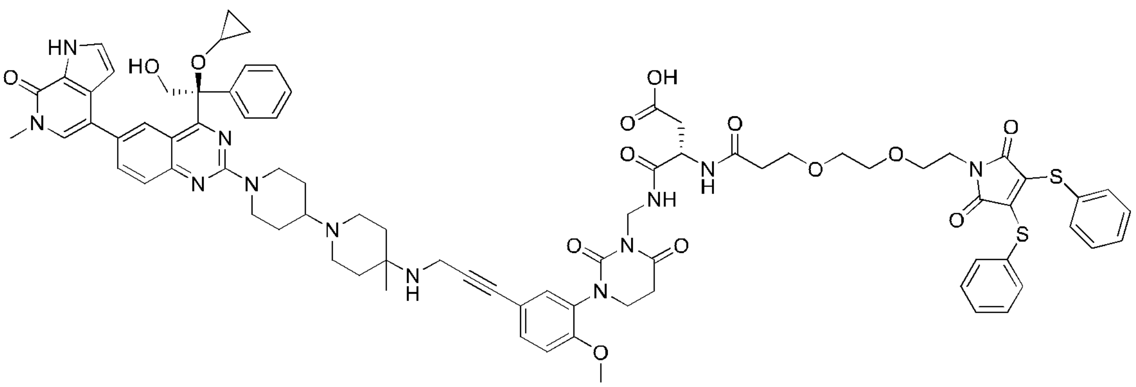

The title compound (9.6 mg) was obtained from the (S)-3-((((9H-fluoren-9-yl)methoxy)carbonyl)amino)-4-(((3-(5-(3-((1'-(4-((S))-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid of Production Example 221-2 (32 mg, 0.025 mmol), by the same method as Example 218.

ESI-MS (m/z): 1503.88 [M+H]+.

[Production Example 221-1]

(S)-3-((((9H-Fluoren-9-yl)methoxy)carbonyl)amino)-4-(((3-(5-iodo-2-methoxyphenyl)-2,6-dioxotetrahydropyrimidine-1(2H)-yl)methyl)amino)-4-oxobutanoic acid

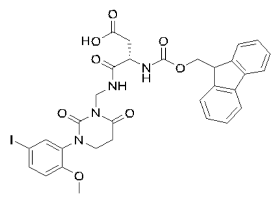

To a solution of the *tert-butyl* (S)-3((((9H-fluoren-9-yl)methoxy)carbonyl)amino)-4-(((3-(5-iodo-2-methoxyphenyl)-2,6-dioxotetrahydropyrimidine-1(2H)-yl)methyl)amino)-4-oxobutanoate of Production Example 201-18 (125 mg, 0.163 mmol) in dichloromethane (2 mL) there was added TFA (1 mL), and the mixture was stirred for 1 hour and 30 minutes. The reaction mixture was purified by direct reversed-phase silica gel chromatography (0.1% formic acid aqueous solution:0.1% acetonitrile formate solution = 95:5 to 0:100) to obtain the title compound (111 mg).

ESI-MS (m/z): 713.28 [M+H]+.

[Production Example 221-2]

(S)-3-((((9H-Fluoren-9-yl)methoxy)carbonyl)amino)-4-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid

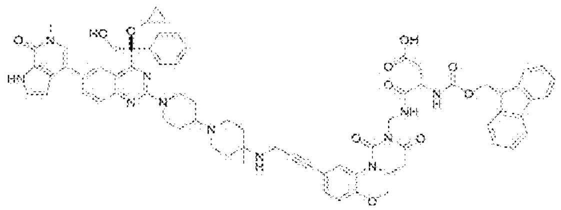

**The title** compound (68 mg) was obtained from the (S)-4-(4-(1-Cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 201-9 (60 mg, 0.087 mmol) and the (S)-3-((((9H-fluoren-9-yl)methoxy)carbonyl)amino)-4-(((3-(5-iodo-2-methoxyphenyl)-2,6-dioxotetrahydropyrimidine-1(2H)-yl)methyl)amino)-4-oxobutanoic acid of Production Example 221-1 (74.8 mg, 0.105 mmol), by the same method as Production Example 218-2.
ESI-MS (m/z): 1270.95 $[M+H]^+$

[Example 222]

(S)-4-((((S)-2-Cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-3-(3-(2-(2-(2,5-dioxo-3,4-bis(phenylthio)-2,5-dihydro-1H-pyrrol-1-yl)ethoxy)ethoxy)propanamido)-4-oxobutanoic acid

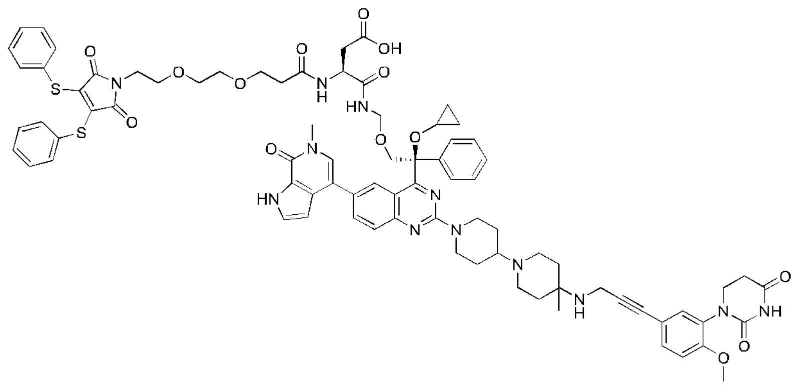

To a solution of the *tert*-butyl(S)-3-((((9H-fluoren-9-yl)methoxy)carbonyl)amino)-4-((((S)-2-cyclopropoxy-2-(2-(4-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-4-oxobutanoate of Production Example 222-1 (10 mg, 7.5 μmol) in THF (1.5 mL) there was added potassium-t-butoxide (8.46 mg, 0.075 mmol) at 0°C, and the mixture was stirred for 30 minutes. The reaction mixture was purified by direct reversed-phase silica gel chromatography (0.1% formic acid aqueous solution:0.1% acetonitrile formate solution = 95:5 to 0:100), to obtain a reaction intermediate. To a solution of the obtained reaction intermediate in DMF (1.5 mL) there were added the 2,5-dioxopyrrolidin-1-yl 3-(2-(2-(2,5-dioxo-3,4-bis(phenylthio)-2,5-dihydro-1H-pyrrol-1-yl)ethoxy)ethoxy)propanoate of Production Example 201-23 (4.30 mg, 7.54 μmol) and N,N-diisopropylethylamine (40 μL, 0.23 mmol), and the mixture was stirred for 30 minutes at 50°C. The reaction mixture was directly purified by preparative HPLC (0.1% formic acid aqueous solution:0.1% acetonitrile formate solution = 4:1 to 1:1), to obtain the title compound (1.8 mg).
ESI-MS (m/z): 1504.14 $[M+H]^+$.

[Production Example 222-1]

*tert*-Butyl (S)-3-((((9H-fluoren-9-yl)methoxy)carbonyl)amino)-4-((((S)-2-cyclopropoxy-2-(2-(4-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-4-oxobutanoate

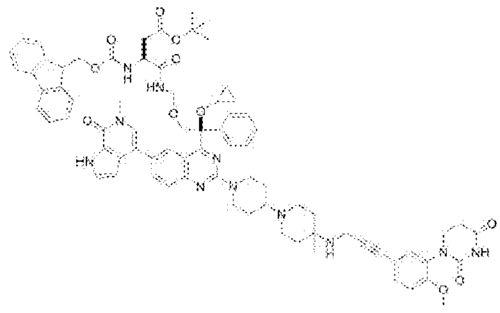

The title compound (10 mg) was obtained from the (S)-1-(5-(3-((1'-(4-(1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)dihydropyrimidine-2,4(1H,3H)-dione of Production Example 207-2 (20 mg, 0.022 mmol) and the *tert-butyl* (S)-3-((((9H-fluoren-9-yl)methoxy)carbonyl)amino-4-((acetoxymethyl)amino)-4-oxobutanoate of Production Example 201-17 (42.7 mg, 0.088 mmol), by the same method as Production Example 219-1.
ESI-MS (m/z): 1326.97 $[M+H]^+$.

[Example 223]

(S)-N1-((3-(5-(3-((1'-(4-((S)-1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)-2-((S)-2-(3-(2-(2-(2,5-dioxo-3,4-bis(phenylthio)-2,5-dihydro-1H-pyrrol-1-yl)ethoxy)ethoxy)propanamido)-3-hydroxypropanamido) succinamide

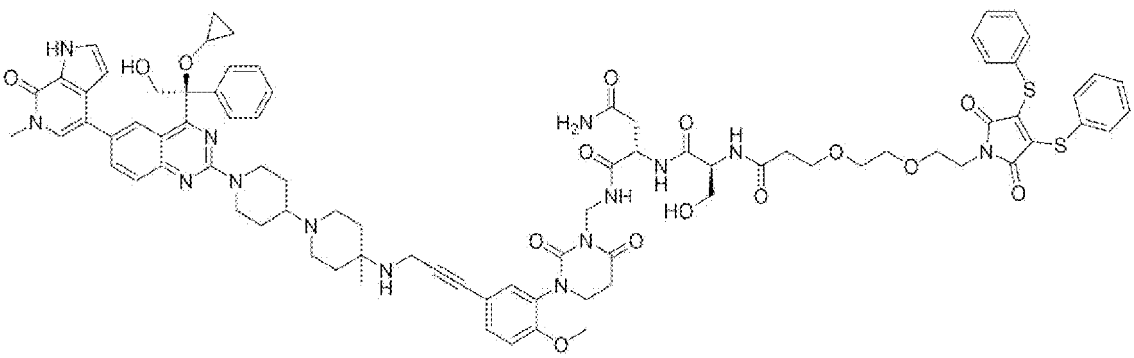

To a solution of the formate of (9H-fluoren-9-yl)methyl ((S)-1-(((S)-4-amino-1-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-1,4-dioxobutan-2-yl)amino)-3-hydroxy-1-oxopropan-2-yl)carbamate of Production Example 223-2 (8.4 mg) in DMF (2 mL) there was added diethylamine (200 μL, 6.2 μmol), and the mixture was stirred for 30 minutes at room temperature. The reaction mixture was concentrated under reduced pressure to obtain intermediate 1. The title compound (1.2 mg) was obtained from intermediate 1 at a reaction temperature of 50°C, by the same method as Example 201.
ESI-MS (m/z): 1590.91 $[M+H]^+$.

[Production Example 223-1]

(9H-Fluoren-9-yl)methyl ((S)-1-(((S)-4-amino-1-(((3-(5-iodo-2-methoxyphenyl)-2,6-dioxotetrahydropyrimidine-1(2H)-yl)methyl)amino)-1,4-dioxobutan-2-yl)amino)-3-hydroxy-1-oxopropan-2-yl)carbamate

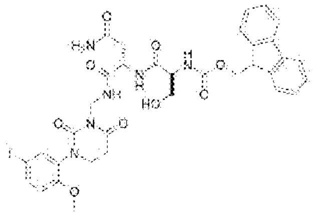

To a solution of the (9H-fluoren-9-yl)methyl (S)-(4-amino-1-(((3-(5-iodo-2-methoxyphenyl)-2,6-dioxotetrahydropyrimidine-1(2H)-yl)methyl)amino)-1,4-dioxobutan-2-yl)carbamate of Production Example 218-1 (60 mg, 0.084 mmol) in DMF (2 mL) there was added diethylamine (200 μL, 1.91 mmol), and the mixture was stirred for 1 hour at room temperature. The reaction mixture was concentrated under reduced pressure, and then N-(((9H-fluoren-9-yl)methoxy)carbonyl)-O-(*tert*-butyl)-L-serine (35.6 mg, 0.093 mmol), HATU (35.3 mg, 0.093 mmol) and N,N-diisopropylethylamine (40 μL, 0.229 mmol) were added to a solution of the residue in DMF (2 mL), and the mixture was stirred for 2 hours at room temperature. The reaction mixture was quenched with water and extracted with ethyl acetate. The collected organic layer was washed with brine, dried over sodium sulfate and concentrated to obtain an intermediate. To a solution of the obtained intermediate in dichloromethane (3 mL) there was added TFA (1 mL), and the mixture was stirred for 3 hours. The reaction mixture was purified by direct reversed-phase silica gel chromatography (0.1% formic acid aqueous solution:0.1% acetonitrile formate solution = 95:5 to 0:100) to obtain the title compound (34 mg).
ESI-MS (m/z): 799.36 $[M+H]^+$.

[Production Example 223-2]

(9H-Fluoren-9-yl)methyl ((S)-1-(((S)-4-amino-1-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-1,4-dioxobutan-2-yl)amino)-3-hydroxy-1-oxopropan-2-yl)carbamate

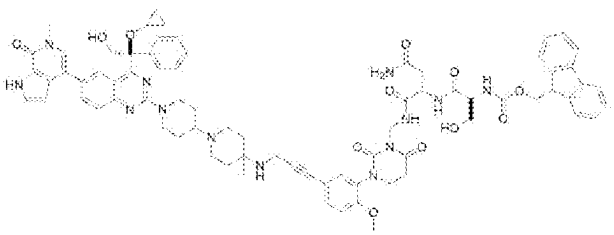

A formate of the title compound (8.4 mg) was obtained from the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 201-9 (10 mg, 0.015 mmol) and the (9H-fluoren-9-yl)methyl ((S)-1-(((S)-4-amino-1-(((3-(5-iodo-2-methoxyphenyl)-2,6-dioxotetrahydropyrimidine-1(2H)-yl)methyl)amino)-1,4-dioxobutan-2-yl)amino)-3-hydroxy-1-oxopropan-2-yl)carbamate of Production Example 223-1 (15.1 mg, 0.019 mmol), by the same method as Production Example 218-2.
ESI-MS (m/z): 1357.03 $[M+H]^+$.

[Example 224]

(11S, 14S)-14-(((3-(5-(3-((1'-(4-((S)-1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)-1-(2,5-dioxo-3,4-bis(phenylthio)-2,5-dihydro-1H-pyrrol-1-yl)-11-(hydroxymethyl)-9,12-dioxo-3,6-dioxa-10,13-diazahexadecan-16-oic acid

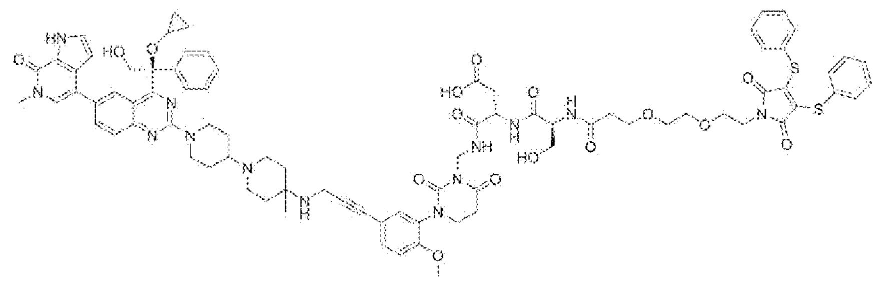

A formate of the title compound (1.2 mg) was obtained from the formate of (S)-3-((S)-2-((((OH-fluoren-9-yl)methoxy)carbonyl)amino)-3-hydroxypropanamido)-4-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid of Production Example 224-5 (4.5 mg, 3.3 μmol), by the same method as Example 223.
ESI-MS (m/z): 1591.14 $[M+H]^+$.

[Production Example 224-1]

(S)-3-((S)-2-((((9H-Fluoren-9-yl)methoxy)carbonyl)amino)-3-hydroxypropanamido)-4-(((3-(5-iodo-2-methoxyphenyl)-2,6-dioxotetrahydropyrimidine-1(2H)-yl)methyl)amino)-4-oxobutanoic acid

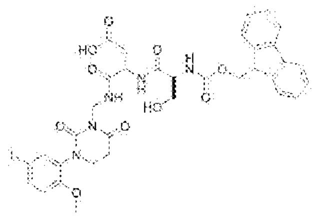

The title compound (33 mg) was obtained from the *tert-butyl* (S)-3((((9H-fluoren-9-yl)methoxy)carbonyl)amino)-4-(((3-(5-iodo-2-methoxyphenyl)-2,6-dioxotetrahydropyrimidine-1(2H)-yl)methyl)amino)-4-oxobutanoate of Production Example 201-18 (50 mg, 0.065 mmol) and N-(((9H-fluoren-9-yl)methoxy)carbonyl)-O-(tert-butyl)-L-serine (27.4 mg, 0.072 mmol), by the same method as Production Example 223-1.
ESI-MS (m/z): 800.31 $[M+H]^+$.

[Production Example 224-2]

2-Chloro-4-((1S)-1-cyclopropoxy-1-phenyl-2-((tetrahydro-2H-pyran-2-yl)oxy)ethyl)-6-iodoquinazoline

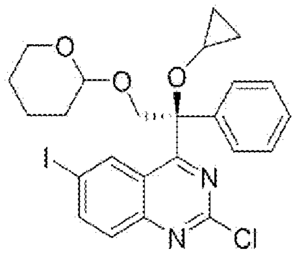

To a solution of the (S)-2-(2-chloro-6-iodoquinazolin-4-yl)-2-cyclopropoxy-2-phenylethan-1-ol of Production Example 201-4 (20 mg, 0.043 mmol) in dichloromethane (2 mL) at room temperature there were added DHP (39 μL, 0.43 mmol) and *p*-toluenesulfonic acid monohydrate (4.1 mg, 0.021 mmol) at 0°C, and the mixture was stirred for 2 hours at room temperature. A saturated aqueous sodium hydrogencarbonate solution was added to the reaction mixture for quenching, and extraction was performed with dichloromethane. The organic layer was dried over anhydrous sodium sulfate and filtered, and the filtrate was concentrated under reduced pressure. The residue was purified by silica gel column chromatography (n-heptane:ethyl acetate = 99:1 to 7:3) to obtain the title compound (22 mg). ESI-MS (m/z): 551.18 $[M+H]^+$.

[Production Example 224-3]

4-(2-Chloro-4-((1S)-1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one

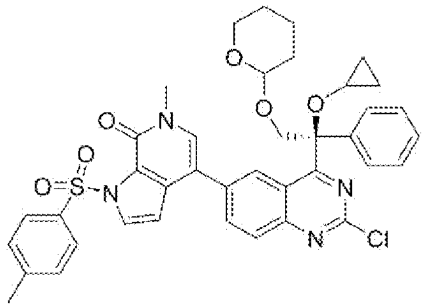

The title compound (357 mg) was obtained from the 2-chloro-4-((1S)-1-cyclopropoxy-1-phenyl-2-((tetrahydro-2H-pyran-2-yl)oxy)ethyl)-6-iodoquinazoline of Production Example 224-2 (250 mg, 0.454 mmol) by the same method as Production Example 201-5.

ESI-MS (m/z): 726.15 $[M+H]^+$.

[Production Example 224-4]

4-(4-((1S)-1-Cyclopropoxy-1-phenyl-2-((tetrahydro-2H-pyran-2-yl)oxy)ethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one

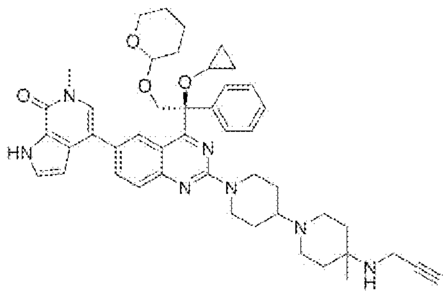

The same methods as in Production Example 201-8 and Production Example 201-9 were carried out consecutively to obtain the title compound (159 mg) from the 4-(2-chloro-4-((1S)-1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 224-3 (150 mg, 0.207 mmol).

ESI-MS (m/z): 770.59 [M+H]+.

[Production Example 224-5]

(S)-3-((S)-2-((((9H-Fluoren-9-yl)methoxy)carbonyl)amino)-3-hydroxypropanamido)-4-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid

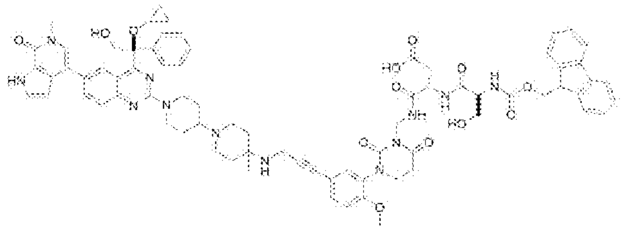

The 4-(4-((1 S)-1-cyclopropoxy-1-phenyl-2-((tetrahydro-2H-pyran-2-yl)oxy)ethyl)-2-(4-methyl-4-(prop-2-yn-1-ylamino)-[1,4'-bipiperidin]-1'-yl)quinazoline-6-methyl-1,6-dihydro-7H-pyrrolo[2,3-c]pyridin-7-one of Example 224-4 (15.0 mg, 0.019 mmol) was treated with dichloromethane (1 mL) and TFA (1 mL) to obtain intermediate 1. The title compound (9.3 mg) was obtained from intermediate 1 and the (S)-3-((S)-2-((((9H-fluoren-9-yl)methoxy)carbonyl)amino)-3-hydroxypropanamido)-4-(((3-(5-iodo-2-methoxyphenyl)-2,6-dioxotetrahydropyrimidine-1(2H)-yl)methyl)amino)-4-oxobutanoic acid of Production Example 224-1 (17.1 mg, 0.021 mmol), by the same method as Production Example 218-2. ESI-MS (m/z): 1358.89 [M+H]+.

[Example 225]

(11R,14S,17S)-17-(((3-(5-(3-((1'-(4-((S)-1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)-1-(2,5-dioxo-3,4-bis(phenylthio)-2,5-dihydro-1H-pyrrol-1-yl)-14-(hydroxymethyl)-11-methyl-9,12,15-trioxo-3,6-dioxa-10,13,16-triazanonadecan-19-oic acid

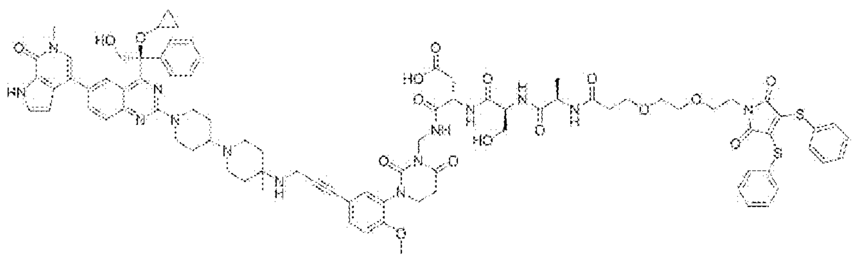

The title compound (0.96 mg) was obtained from the (5R, 8S, 11S)-11-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)-1-(9H-fluoren-9-yl)-8-(hydroxymethyl)-5-methyl-3,6,9-trioxo-2-oxa-4,7,10-triazatridecan-13-oic acid of Production Example 225-2 (10 mg, 7.0 μmol) by the same method as Example 235. ESI-MS (m/z): 1662.21 [M+H]+.

[Production Example 225-1]

(5R,8S,11S)-1-(9H-Fluoren-9-yl)-8-(hydroxymethyl)-11-(((3-(5-iodo-2-methoxyphenyl)-2,6-dioxotetrahydropyrimidine-1(2H)-yl)methyl)carbamoyl)-5-methyl-3,6,9-trioxo-2-oxa-4,7,10-triazatridecan-13-oic acid

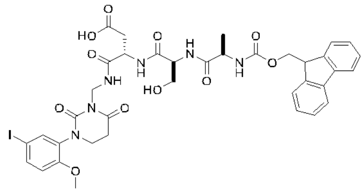

To a solution of the tert-butyl(S)-3((((9H-fluoren-9-yl)methoxy)carbonyl)amino)-4-(((3-(5-iodo-2-methoxyphenyl)-2,6-dioxotetrahydropyrimidine-1(2H)-yl)methyl)amino)-4-oxobutanoate of Production Example 201-18 (40 mg, 0.052 mmol) in DMF (2 mL) there was added diethylamine (200 μL, 1.91 mmol), and the mixture was stirred for 1 hour at room temperature. The reaction mixture was concentrated under reduced pressure, and then N-(((9H-fluoren-9-yl)methoxy)carbonyl)-O-(*tert*-butyl)-L-serine (22.0 mg, 0.057 mmol), HATU (21.8 mg, 0.057 mmol) and N,N-diisopropylethylamine (100 μL, 0.573 mmol) were added to a solution of the residue in DMF (2 mL), and the mixture was stirred for 2 hours at room temperature. Diethylamine (200 μL, 1.91 mmol) was added to the reaction mixture, which was then stirred for 1 hour at room temperature. The reaction mixture was concentrated under reduced pressure, and then ((9H-fluoren-9-yl)methoxy)carbonyl)-D-alanine (17.8 mg, 0.057 mmol), HATU (21.8 mg, 0.057 mmol) and N,N-diisopropylethylamine (100 μL, 0.573 mmol) were added to a solution of the residue in DMF (2 mL), and the mixture was stirred for 1 hour at room temperature. Water was added to the reaction mixture for quenching, and the mixture was extracted with dichloromethane. The organic layer was concentrated to obtain an intermediate. To a solution of the intermediate in dichloromethane (1 mL) there was added TFA(1 mL), and the mixture was stirred for 5 hours at room temperature. The reaction mixture was purified by direct reversed-phase silica gel chromatography (0.1% formic acid aqueous solution:0.1% acetonitrile formate solution = 95:5 to 0:100) to obtain the title compound (21 mg). ESI-MS (m/z): 871.39 [M+H]+.

[Production Example 225-2]

(5R,8S,11S)-11-(((3-(5-(3-((1'-(4-((S)-1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)-1-(9H-fluoren-9-yl)-8-(hydroxymethyl)-5-methyl-3,6,9-trioxo-2-oxa-4,7,10-triazatridecan-13-oic acid

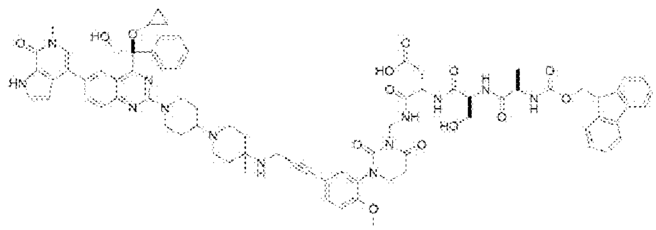

The title compound (10 mg) was obtained from the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 201-9 (15 mg, 0.022 mmol) and the (5R,8S,11S)-1-(9H-fluoren-9-yl)-8-(hydroxymethyl)-11-(((3-(5-iodo-2-methoxyphenyl)-2,6-dioxotetrahydropyrimidine-1(2H)-yl)methyl)carbamoyl)-5-methyl-3,6,9-trioxo-2-oxa-4,7,10-triazatridecan-13-oic acid of Production Example 225-1 (19 mg, 0.022 mmol), by the same method as Production Example 218-2.

ESI-MS (m/z): 1429.20 [M+H]+.

[Example 226]

(11S,14S)-14-(((3-(5-(3-((1'-(4-((S)-1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)-1-(2,5-dioxo-3,4-bis(phenylthio)-2,5-dihydro-1H-pyrrolo-1-yl)-11-methyl-9,12-dioxo-3,6-dioxa-10,13-diazahexadecan-16-oic acid

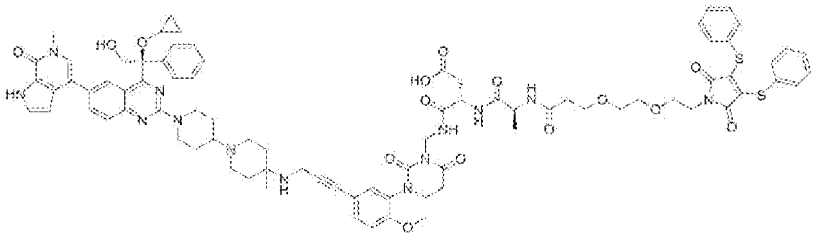

A formate of the title compound (3.0 mg) was obtained from the formate of (S)-3-((S)-2-aminopropaneamide)-4-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)-amino)prop-1-yn-1-yl)-2-methoxyphenyl)-2,6-dioxotetrahydropyrimidine-1(2H)-yl)methyl)amino)-4-oxobutanoic acid of Production Example 226-4 (10.0 mg) and the 2,5-dioxopyrrolidin-1-yl 3-(2-(2-(2,5-dioxo-3,4-bis(phenylthio)-2,5-dihydro-1H-pyrrol-1-yl)ethoxy)ethoxy)propanoate of Production Example 201-23 (5.61 mg, 9.83 μmol), by the same method as Example 201.
ESI-MS (m/z): 1575.86 $[M+H]^+$.

[Production Example 226-1]

*tert-Butyl* (S)-3-((S)-2-((((9H-fluoren-9-yl)methoxy)carbonyl)amino)propaneamide)-4-(((3-(5-iodo-2-methoxyphenyl)-2,6-dioxotetrahydropyrimidine-1(2H)-yl)methyl)amino)-4-oxobutanoate

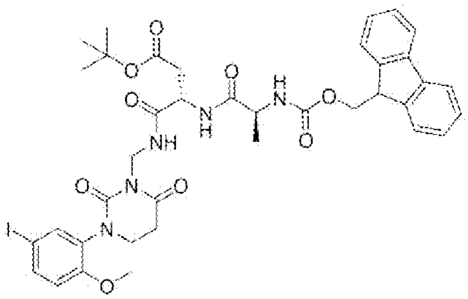

The title compound (47 mg) was obtained from the *tert-butyl* (S)-3((((9H-fluoren-9-yl)methoxy)carbonyl)amino)-4-(((3-(5-iodo-2-methoxyphenyl)-2,6-dioxotetrahydropyrimidine-1(2H)-yl)methyl)amino)-4-oxobutanoate of Production Example 201-18 (50 mg, 65 μmol) and (2S)-2-({[(9H-fluoren-9-yl)methoxy]carbonyl}amino)propanoic acid hydrate (25.7 mg, 78.0 μmol), by the same method as Production Example 229-1.
ESI-MS (m/z): 840.27 $[M+H]^+$.

[Production Example 226-2]

(S)-3-((S)-2-((((9H-Fluoren-9-yl)methoxy)carbonyl)amino)propaneamide)-4-(((3-(5-iodo-2-methoxyphenyl)-2,6-dioxotetrahydropyrimidine-1(2H)-yl)methyl)amino)-4-oxobutanoic acid

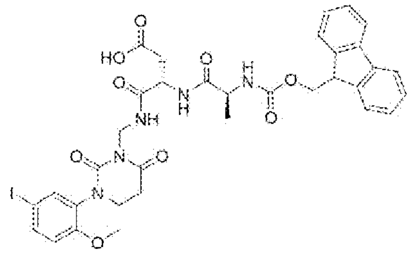

The title compound (43 mg) was obtained from the *tert-butyl* (S)-3-((S)-2-((((9H-fluoren-9-yl)methoxy)carbonyl) amino)propaneamide)-4-(((3-(5-iodo-2-methoxyphenyl)-2,6-dioxotetrahydropyrimidine-1(2H)-yl)methyl)amino)-4-oxobutanoate of Production Example 226-1 (47 mg, 56 μmol), by the same method as Production Example 221-1.
ESI-MS (m/z): 784.21 [M+H]+.

[Production Example 226-3]

(S)-3-((S)-2-((((9H-Fluoren-9-yl)methoxy)carbonyl)amino)propaneamide)-4-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid

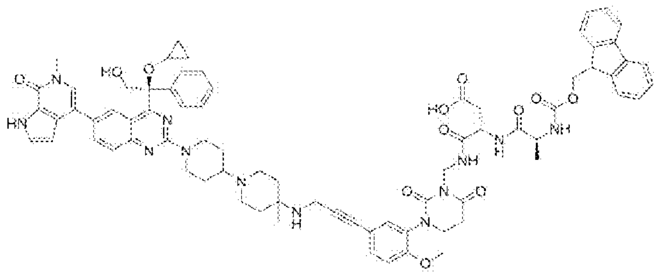

A formate of the title compound (11 mg) was obtained from the (S)-3-((S)-2-((((9H-fluoren-9-yl)methoxy) carbonyl)propaneamide)-4-(((3-(5-iodo-2-methoxyphenyl)-2,6-dioxotetrahydropyrimidine-1(2H)-yl)methyl)amino)-4-oxobutanoic acid of Production Example 226-2 (15 mg, 19 μmol) and the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 201-9 (12 mg, 17 μmol), by the same method as Production Example 218-2.
ESI-MS (m/z): 1342.54 [M+H]+.

[Production Example 226-4]

(S)-3-((S)-2-Aminopropaneamide)-4-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid

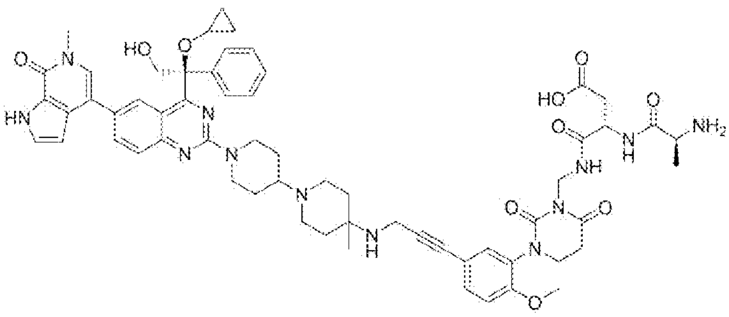

To a solution of the formate of (S)-3-((S)-2-((((9H-fluoren-9-yl)methoxy)carbonyl)amino)propaneamide)-4-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino-4-oxobutanoic acid of Production Example 226-3 (11 mg) in DMF (2.00 mL) there was added diethylamine (200 μL) at room temperature, and the mixture was stirred for 1 hour at room temperature. After concentrating the reaction mixture under reduced pressure, it was purified by ODS silica gel column chromatography (0.1% formic acid-containing water:0.1% formic acid-containing acetonitrile = 95:5 to 3:7), to obtain a formate of the title compound (10 mg). ESI-MS (m/z): 1119.69 $[M+H]^+$.

[Example 227]

(11S,14S)-11-(2-Amino-2-oxoethyl)-14-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)-1-(2,5-dioxo-3,4-bis(phenylthio)-2,5-dihydro-1H-pyrrol-1-yl)-9,12-dioxo-3,6-dioxa-10,13-diazahexadecan-16-oic acid

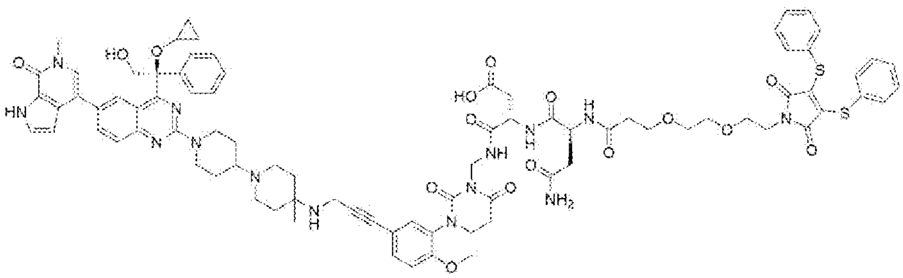

A formate of the title compound (2.5 mg) was obtained from the formate of (S)-4-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-3-((S)-2,4-diamino-4-oxobutanamide)-4-oxobutanoic acid of Production Example 227-2 (9.0 mg) and the 2,5-dioxopyrrolidin-1-yl 3-(2-(2-(2,5-dioxo-3,4-bis(phenylthio)-2,5-dihydro-1H-pyrrol-1-yl)ethoxy)ethoxy)propanoate of Production Example 201-23 (8.2 mg, 0.014 mmol), by the same method as Example 201.
ESI-MS (m/z): 809.83 $[(M+2H)/2]^+$.

[Production Example 227-1]

(S)-3-((S)-2-((((9H-Fluoren-9-yl)methoxy)carbonyl)amino)-4-amino-4-oxobutanamide)-4-(((3-(5-iodo-2-methoxyphenyl)-2,6-dioxotetrahydropyrimidine-1(2H)-yl)methyl)amino)-4-oxobutanoic acid

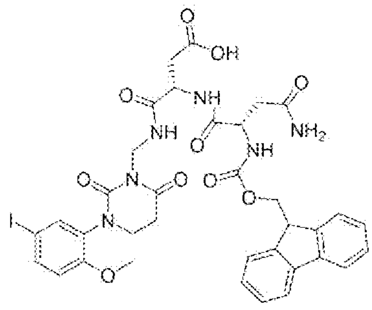

A reaction intermediate was obtained from the *tert-butyl* (S)-3((((9H-fluoren-9-yl)methoxy)carbonyl)amino)-4-(((3-(5-iodo-2-methoxyphenyl)-2,6-dioxotetrahydropyrimidine-1(2H)-yl)methyl)amino)-4-oxobutanoate of Production Example 201-18 (50 mg, 0.065 mmol) and (((9H-fluoren-9-yl)methoxy)carbonyl)-L-asparagine (27.7 mg, 0.078 mmol), by the same method as Production Example 203-1. After adding dichloromethane (1.0 mL) and TFA(0.50 mL) and stirring for 1 hour at room temperature, the mixture was concentrated under reduced pressure and the residue was purified by ODS silica gel chromatography (0.1% formic acid aqueous solution:0.1% acetonitrile formate solution = 9:1 to 1:1), to obtain the title compound (26 mg).

ESI-MS (m/z): 827.34 $[M+H]^+$.

[Production Example 227-2]

(S)-4-(((3-(5-(3-((1'-(4-((S)-1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-3-((S)-2,4-diamino-4-oxobutanamide)-4-oxobutanoic acid

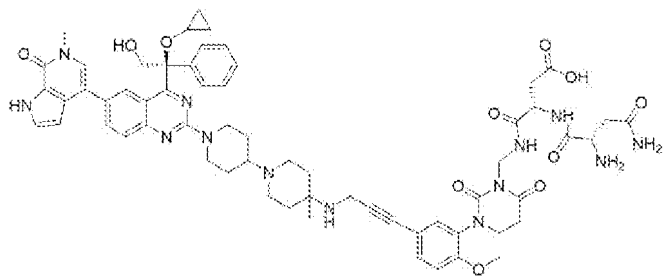

A formate of the title compound (9.0 mg) was obtained from the (S)-3-((S)-2-((((9H-fluoren-9-yl)methoxy)carbonyl)amino)-4-amino-4-oxobutanamide)-4-(((3-(5-iodo-2-methoxyphenyl)-2,6-dioxotetrahydropyrimidine-1(2H)-yl)methyl)amino)-4-oxobutanoic acid of Production Example 227-1 (13 mg, 0.016 mmol) and the (S)-4-(4-(1-cyclopropoxy-2-hydroxy-1-phenylethyl)-2(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 201-9 (9.0 mg, 0.013 mmol), by the same method as Production Example 204-5. ESI-MS (m/z): 582.25 $[(M+2H)/2]^+$.

[Example 228]

(11R,14S)-11-(Carboxymethyl)-14(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)-1-(2,5-dioxo-3,4-bis(phenylthio)-2,5-dihydro-1H-pyrrolo-1-yl)-9,12-dioxo-3,6-dioxa-10,13-diazahexadecan-16-oic acid

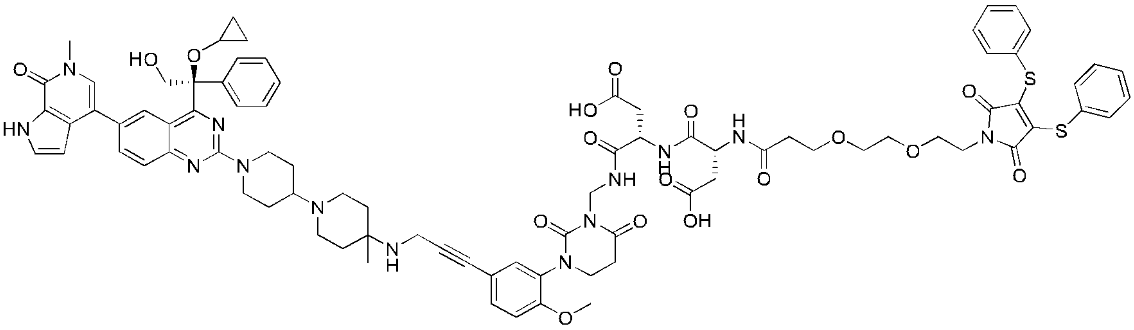

A formate of the title compound (2.1 mg) was obtained from the formate of (R)-3-amino-4-(((S)-3-carboxy-1-(((3-5-3-((1'-4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-1-oxopropan-2-yl)amino)-4-oxobutanoic acid of Production Example 228-3 (8.0 mg) and the 2,5-dioxopyrrolidin-1-yl 3-(2-(2-(2,5-dioxo-3,4-bis(phenylthio)-2,5-dihydro-1H-pyrrol-1-yl)ethoxyethoxy)propanoate of Production Example 201-23 (4.71 mg, 8.25 μmol), by the same method as Example 201.
ESI-MS (m/z): 1619.08 [M+H]+.

[Production Example 228-1]

*tert-Butyl* (R)-3-((((9H-fluoren-9-yl)methoxy)carbonyl)amino)-4-(((S)-4-(*tert*-butoxy)-1-(((3-(5-iodo-2-methoxyphenyl)-2,6-dioxotetrahydropyrimidine-1(2H)-yl)methyl)amino)-1,4-dioxobutan-2-yl)amino)-4-oxobutanoate

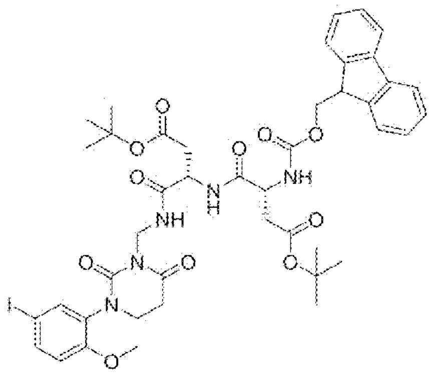

The title compound (55 mg) was obtained from the *tert-butyl* (S)-3((((9H-fluoren-9-yl)methoxy)carbonyl)amino)-4-(((3-(5-iodo-2-methoxyphenyl)-2,6-dioxotetrahydropyrimidine-1(2H)-yl)methyl)amino)-4-oxobutanoate of Production Example 201-18 (50 mg, 65 μmol) and (2R) -4-(tert-butoxy)-2-({ [(9H-fluoren-9-yl)methoxy]carbonyl}amino)-4-oxobutanoic acid (32 mg, 78 μmol), by the same method as Production Example 229-1.
ESI-MS (m/z): 940.31 [M+H]+.

[Production Example 228-2]

(R)-3-((((9H-Fluoren-9-yl)methoxy)carbonyl)amino)-4-(((S)-3-carboxy-1-(((3-(5-iodo-2-methoxyphenyl)-2,6-dioxotetrahydropyrimidine-1(2H)-yl)methyl)amino)-1-oxopropan-2-yl)amino)-4-oxobutanoic acid

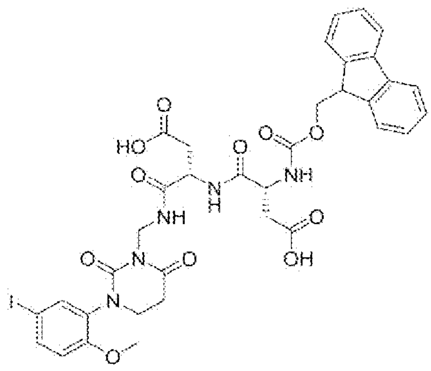

The title compound (47 mg) was obtained from the *tert-butyl* (R)-3-((((9H-fluoren-9-yl)methoxy)carbonyl) amino)-4-(((S)-4-(tert-butoxy)-1-(((3-(5-iodo-2-methoxyphenyl)-2,6-dioxotetrahydropyrimidine-1(2H)-yl)methyl)amino)-1,4-dioxobutan-2-yl)amino)-4-oxobutanoate of Production Example 228-1 (55 mg, 59 μmol) by the same method as Production Example 221-1.
ESI-MS (m/z): 828.18 $[M+H]^+$.

[Production Example 228-3]

(R)-3-Amino-4-(((S)-3-carboxy-1-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-1-oxopropan-2-yl)amino)-4-oxobutanoic acid

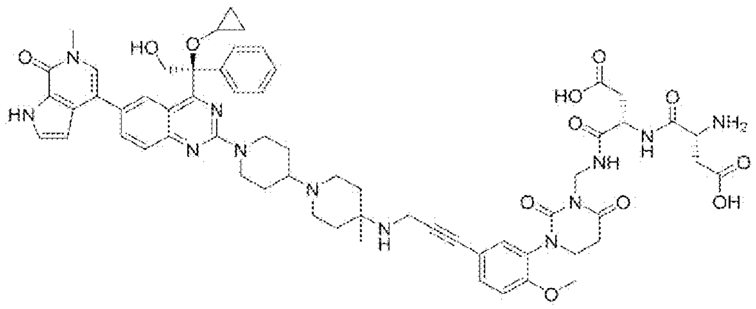

A formate of the title compound (8.0 mg) was obtained from the (R)-3-((((9H-fluoren-9-yl)methoxy)carbonyl) amino)-4-(((S)-3-carboxy-1-(((3-(5-iodo-2-methoxyphenyl)-2,6-dioxotetrahydropyrimidine-1(2H)-yl)methyl)amino)-1-oxopropan-2-yl)amino)-4-oxobutanoic acid of Production Example 228-2 (15.9 mg, 19 μmol) and the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 201-9 (12 mg, 17 μmol), by the same methods as Production Example 218-2 and Production Example 226-4.
ESI-MS (m/z): 1164.56 $[M+H]^+$.

[Example 229]

(S)-N1-(2-(((3-(5-(3-((1'-(4-((S)-1-Cyclopropoxy-2-hydroxy-1-phenylethyl)-6-(6-methyl-7-oxo-6,7-dihydro-1H-pyrrolo [2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-2-oxoethyl)-2-(3-(2-(2-(2,5-dioxo-3,4-bis(phenylthio)-2,5-dihydro-1H-pyrrol-1-yl)ethoxy)ethoxy)propanamido) succinamide

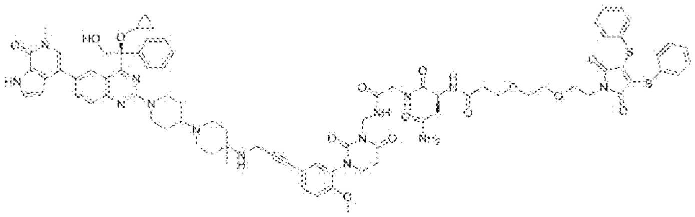

To a solution of the (9H-fluoren-9-yl)methyl ((S)-4-amino-1-((2-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-2-oxoethyl)amino)-1,4-dioxobutan-2-yl)carbamate of Production Example 229-2 (2.8 mg, 2.1 μmol) in DMF (500 μL) there was added diethylamine (100 μL), and the mixture was stirred for 30 minutes at room temperature. The reaction mixture was concentrated under reduced pressure to obtain intermediate 1. To a solution of the tert-butyl 3-(2-(2-(2,5-dioxo-3,4-bis(phenylthio)-2,5-dihydro-1H-pyrrol-1-yl)ethoxy)ethoxy)propionate of Production Example 201-22 (1.4 mg, 2.7 μmol) in dichloromethane (500 μL) there was added TFA(100 μL), and the mixture was stirred for 30 minutes at room temperature. The reaction mixture was concentrated under reduced pressure, and then N,N-diisopropylethylamine (3.7 μL, 0.021 mmol) and HATU (1.04 mg, 2.74 μmol) were added to a solution of the residue in DMF (500 μL). A solution of intermediate 1 in DMF (500 μL) was added to the reaction mixture at room temperature, and the mixture was stirred for 1 hour. The reaction mixture was purified by preparative HPLC (0.1% formic acid aqueous solution:0.1% acetonitrile formate solution = 80:20 to 50:50), to obtain the title compound (0.45 mg).
ESI-MS (m/z): 1559.79 $[M+H]^+$.

[Production Example 229-1]

(9H-Fluoren-9-yl)methyl (S)-(4-amino-1-((2-(((3-(5-iodo-2-methoxyphenyl)-2,6-dioxotetrahydropyrimidine-1(2H)-yl)methyl)amino)-2-oxoethyl)amino)-1,4-dioxobutan-2-yl)carbamate

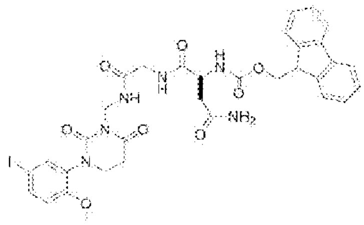

To a solution of the (9H-fluoren-9-yl)methyl (2-(((3-(5-iodo-2-methoxyphenyl)-2,6-dioxotetrahydropyrimidine-1(2H)-yl)methyl)amino)-2-oxoethyl)carbamate of Production Example 206-1 (40 mg, 0.061 mmol) in DMF (1 mL) there was added diethylamine (200 μL, 1.91 mmol), and the mixture was stirred for 2 hours at room temperature. The reaction mixture was concentrated under reduced pressure, and then (((9H-fluoren-9-yl)methoxy)carbonyl)-L-asparagine (32.5 mg, 0.092 mmol), HATU (34.9 mg, 0.092 mmol) and N,N-diisopropylethylamine (32.0 μL, 0.183 mmol) were added to a solution of the residue in DMF (1 mL), and the mixture was stirred for 1 hour at room temperature. The reaction mixture was purified by direct reversed-phase silica gel chromatography (0.1% formic acid aqueous solution:0.1% acetonitrile formate solution = 95:5 to 20:80) to obtain the title compound (38 mg).
ESI-MS (m/z): 769.09 $[M+H]^+$.

[Production Example 229-2]

(9H-Fluoren-9-yl)methyl ((S)-4-amino-1-((2-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-2-oxoethyl)amino)-1,4-dioxobutan-2-yl)carbamate

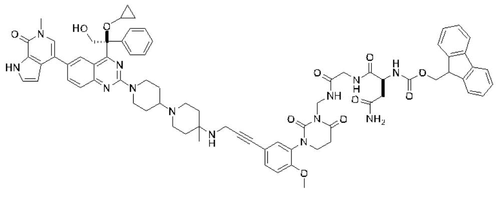

**The title** compound (2.8 mg) was obtained from the (S)-4-(4-(1-cyclopropoxy-2-hydroxy-1-phenylethyl)-2-(4-methyl-4-(prop-2-yn-1-ylamino)-[1,4'bipiperidin]-1'-yl)quinazolin-6-yl)-6-methyl-1,6-dihydro-7H-pyrrolo[2,3-c]pyridin-7-one of Production Example 201-9 (6.0 mg, 8.7 μmol) and the (9H-fluoren-9-yl)methyl (S)-(4-amino-1-((2-(((3-(5-iodo-2-methoxyphenyl)-2,6-dioxotetrahydropyrimidine-1(2H)-yl)methyl)amino)-2-oxoethyl)amino)-1,4-dioxobutan-2-yl)carbamate of Production Example 229-1 (7.4 mg, 9.6 μmol), by the same method as Production Example 218-2.
ESI-MS (m/z): 1326.90 $[M+H]^+$.

[Example 230]

(11S,14S)-11-(2-Carboxyethyl)-14-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)-1-(2,5-dioxo-3,4-bis(phenylthio)-2,5-dihydro-1H-pyrrol-1-yl)-9,12-dioxo-3,6-dioxa-10,13-diazahexadecan-16-oic acid

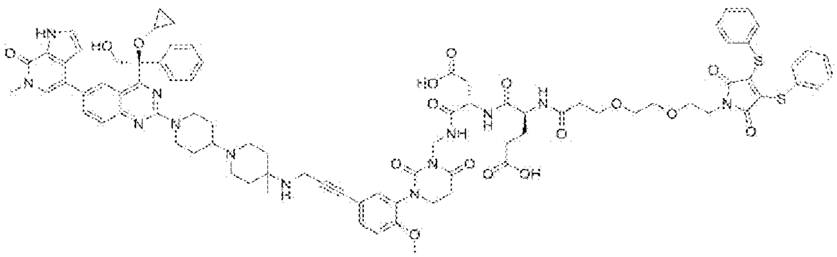

The title compound (1.5 mg) was obtained from the (S)-4-((((9H-fluoren-9-yl)methoxy)carbonyl)amino)-5-(((S)-3-carboxy-1-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidine] -4-yl)amino)prop-1-yn-1-yl)-2-methoxyphenyl)-2,6-dioxotetrahydropyrimidine-1(2H)-yl)methyl)amino)-1-oxopropan-2-yl)amino)-5-oxopentanoic acid of Production Example 230-2 (8.5 mg, 6.1 μmol) by the same method as Example 235.
ESI-MS (m/z): 1633.06 $[M+H]^+$.

[Production Example 230-1]

(S)-4-((((9H-Fluoren-9-yl)methoxy)carbonyl)amino)-5-(((S)-3-carboxy-1-(((3-(5-iodo-2)-methoxyphenyl)-2,6-dioxotetrahydropyrimidine-1(2H)-yl)methyl)amino)-1-oxopropan-2-yl)amino)-5-oxopentanoic acid

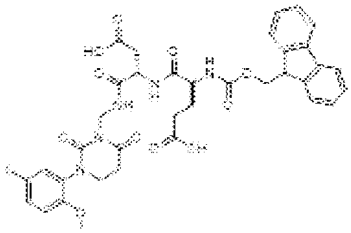

The title compound (69 mg) was obtained from the *tert-butyl* (S)-3((((9H-fluoren-9-yl)methoxy)carbonyl)amino)-4-(((3-(5-iodo-2-methoxyphenyl)-2,6-dioxotetrahydropyrimidine-1(2H)-yl)methyl)amino)-4-oxobutanoate of Production Example 201-18 (170 mg, 0.091 mmol) and (S)-2-((((9H-fluoren-9-yl)methoxy)carbonyl)amino)-5-*(tert-butoxy)-5-*

*oxopentanoic* acid (50.4 mg, 0.118 mmol), by the same method as Production Example 223-1.

ESI-MS (m/z): 842.31 [M+H]+.

[Production Example 230-2]

(S)-4-((((9H-Fluoren-9-yl)methoxy)carbonyl)amino)-5-(((S)-3-carboxy-1-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-1-oxopropan-2-yl)amino)-5-oxopentanoic acid

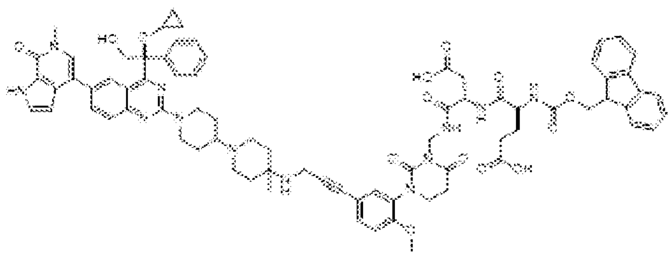

The title compound (17 mg) was obtained from the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 201-9 (15 mg, 0.022 mmol) and the (S)-4-((((9H-fluoren-9-yl)methoxy)carbonyl)amino)-5-(((S)-3-carboxy-1-(((3-(5-iodo-2)-methoxyphenyl)-2,6-dioxotetrahydropyrimidine-1(2H)-yl)methyl)amino)-1-oxopropan-2-yl)amino)-5-oxopentanoic acid of Production Example 230-1 (20.3 mg, 0.024 mmol), by the same method as Production Example 218-2.

ESI-MS (m/z): 1400.09 [M+H]+.

[Example 231]

(44S,50S)-50-(((3-(5-(3-((1'-(4-((S)-1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)-44-(3-(2-(2-(2,5-dioxo-3,4-bis(phenylthio)-2,5-dihydro-1H-pyrrol-1-yl)ethoxy)ethoxy)propanamido)-38,45,48-trioxo-2,5,8,11,14,17,20,23,26,29,32,35-dodecaoxa-39,46,49-triazadopentacontan-52-oic acid

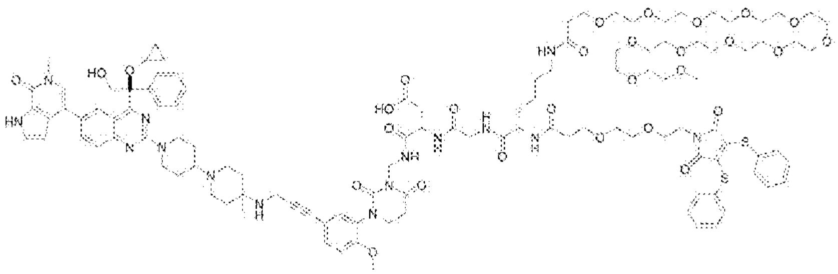

After obtaining intermediate 1 (7.5 mg) from the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 201-9 (7 mg, 10.2 μmol and the (44S,50S)-44-((((9H-fluoren-9-yl)methoxy)carbonyl)amino)-50-(((3-(5-iodo-2-methoxyphenyl)-2,6-dioxotetrahydropyrimidine- 1(2H)-yl)methyl)carbamoyl)-38,45,48-trioxo-2,5,8,11,14,17,20,23,26,29,32,35-dodecaoxa-39,46,49-triazadopentacontan-52-oic acid of Production Example 231-3 (15.0 mg, 10.2 μmol) by the same method as Production Example 201-20, the title compound (1.5 mg) was obtained by the same method as Example 201.

ESI-MS (m/z): 1131.27 [(M+2H)/2]+.

[Production Example 231-1]

*tert*-Butyl N2-(((9H-fluoren-9-yl)methoxy)carbonyl)-N6-((benzyloxy)carbonyl)-L-lysylglycinate

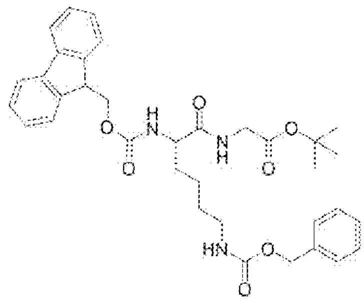

To a solution of (2S)-6-{[(benzyloxy)(hydroxy)methylidene]amino}-2-({[(9H-fluoren-9-yl)methoxy](hydroxy)methylidene}amino)hexanoic acid (1.00 g, 1.99 mmol) in DMF (7.7 mL) there were added N,N-diisopropylethylamine (1.04 mL, 5.97 mmol), glycine *tert-butyl* ester hydrochloride (0.500 g, 2.99 mmol) and HATU (1.14 g, 2.99 mmol), and the mixture was stirred for 2 hours. An aqueous ammonium chloride solution was added for dilution, and extraction was performed with ethyl acetate. After drying over sodium sulfate and concentration under reduced pressure, the residue was purified by silica gel column chromatography (heptane:ethyl acetate = 1:0 to 0:1) to obtain the title compound (570 mg). ESI-MS (m/z): 616.49 $[M+H]^+$.

[Production Example 231-2]

*tert*-Butyl (S)-(44-((((9H-fluoren-9-yl)methoxy)carbonyl)amino)-38-oxo-2,5,8,11,14,17,20,23,26,29,32,35-dodecaoxa-39-azapentatetracontan-45-oyl) glycinate

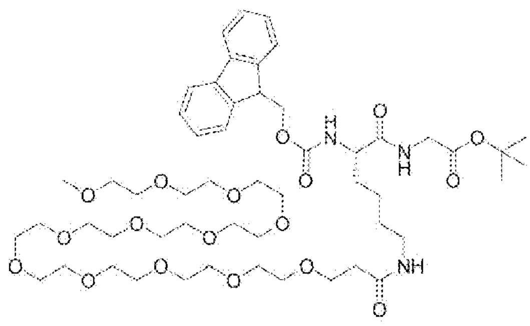

To a solution of the *tert-butyl* N2-(((9H-fluoren-9-yl)methoxy)carbonyl)-N6-((benzyloxy)carbonyl)-L-lysylglycinate of Production Example 231-1 (300 mg, 0.487 mmol) in methanol (11.1 mL) there was added 10% palladium-carbon (50% water, 33.3 mg), and the mixture was stirred for 2 hours at room temperature under a hydrogen atmosphere. The reaction mixture was filtered with Celite and concentrated under reduced pressure. To a solution of the residue in DMF (2.22 mL) there were added N,N-diisopropylethylamine (849 μL, 4.87 mmol) and 1-[(38-oxo-2,5,8,11,14,17,20,23,26,29,32,35-dodecaoxaoctatriacontan-38-yl)oxy]-2,5-pyrrolidinedione (334 mg, 0.487 mmol), and the mixture was stirred for 2 hours. The reaction mixture was purified by reversed-phase silica gel column chromatography (0.1% formic acid aqueous solution:0.1% acetonitrile formate solution = 1:0 to 0:1) to obtain the title compound (156 mg).

ESI-MS (m/z): 1053.61 $[M+H]^+$.

[Production Example 231-3]

(44S,50S)-44-((((9H-Fluoren-9-yl)methoxy)carbonyl)amino)-50-(((3-(5-iodo-2-methoxyphenyl)-2,6-dioxotetrahydropyrimidine-1(2H)-yl)methyl)carbamoyl)-38,45,48-trioxo-2,5,8,11,14,17,20,23,26,29,32,35-dodecaoxa-39,46,49-triazadopentacontan-52-oic acid

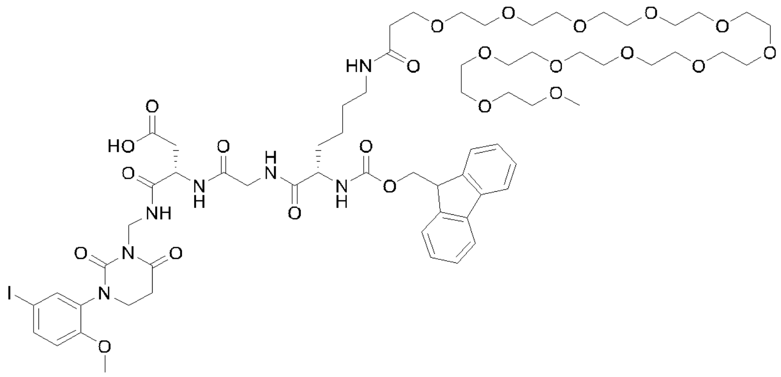

A solution of the *tert-butyl* (S)-(44-((((9H-fluoren-9-yl)methoxy)carbonyl)amino)-38-oxo-2,5,8,11,14,17,20,23,26,29,32,35-dodecaoxa-39-azapentatetracontan-45-oyl) glycinate of Production Example 231-2 (15.1 mg, 0.014 mmol) in dichloromethane (1 mL) and TFA(1 mL) was stirred for 1 hour and then concentrated under reduced pressure, to obtain intermediate 1. The title compound (15 mg) was obtained from the *tert-butyl* (S)-3((((9H-fluoren-9-yl)methoxy)carbonyl)amino)-4-(((3-(5-iodo-2-methoxyphenyl)-2,6-dioxotetrahydropyrimidine-1(2H)-yl) methyl)amino)-4-oxobutanoate of Production Example 201-18 (10 mg, 0.013 mmol) and intermediate 1, by the same method as Production Example 223-1.

ESI-MS (m/z): 1468.97 $[M+H]^+$.

[Example 232]

(S)-2-(3-(2-(2-((S)-3-Amino-2-(2,5-dioxo-3,4-bis(phenylthio)-2,5-dihydro-1H-pyrrol-1-yl)propanamido)ethoxy)ethoxy) propanamido)-N1-(((S)-2-cyclopropoxy-2-(2-(4-((3-(3-(2,4-dioxotetrahydropyrimidine-1(2H)-yl)-4-methoxyphenyl) prop-2-yn-1-yl)amino)-4-methyl-[1,4'-bipiperidin]-1-yl)-6-(6-methyl-7-oxo-6,7-dihydro-1H-pyrrolo[2,3-c]pyridin-4-yl)quinazolin-4-yl)-2-phenylethoxy)methyl) succinamide

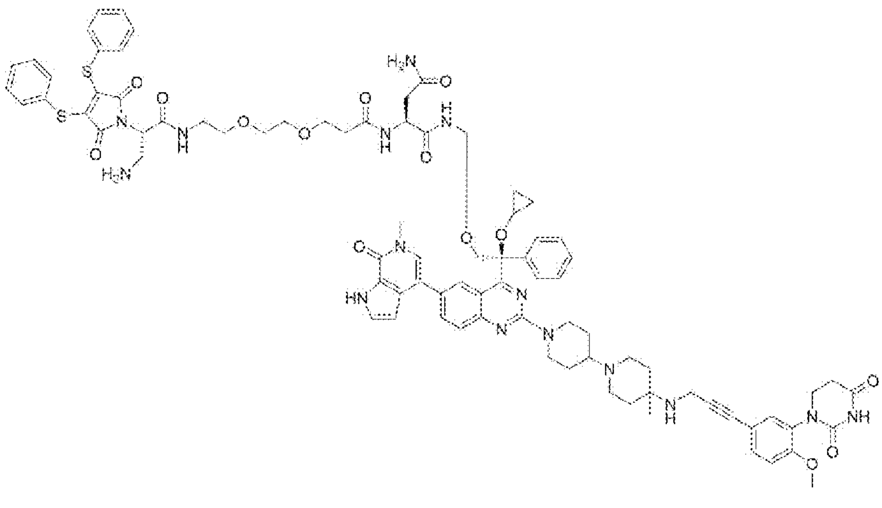

To a solution of the *tert-butyl* (S)-6-(2,5-dioxo-3,4-bis(phenylthio)-2,5-dihydro-1H-pyrrol-1-yl)-1-(9H-fluoren-9-yl)-3,7-dioxo-2,11,14-trioxa-4,8-diazaheptadecan-17-oate of Production Example 232-4 (6.93 mg, 8.27 μmol) in dichloromethane (500 μL) there was added TFA (100 μL), and the mixture was stirred for 30 minutes at room temperature.. The reaction mixture was processed 3 times by azeotropic distillation with toluene and concentrated under reduced pressure,

to obtain intermediate 1. To a solution of the (9H-fluoren-9-yl)methyl((S)-4-amino-1-((((S)-2-cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl) amino)-1,4-dioxobutan-2-yl)carbamate of Production Example 219-1 (7.0 mg, 5.5 μmol) in DMF (500 μL) there was added diethylamine (100 μL), and the mixture was stirred for 15 minutes at room temperature. The reaction mixture was processed 3 times by azeotropic distillation with toluene and concentrated under reduced pressure, after which a solution of N,N-diisopropylethylamine (4.8 μL, 0.028 mmol), HATU (2.52 mg, 6.62 μmol) and intermediate 1 in DMF (500 μL) was added to the residue at room temperature, and the mixture was stirred for 1 hour. It was then purified by reversed-phase silica gel column chromatography (0.1% formic acid aqueous solution:0.1% acetonitrile formate solution = 95:5 to 30:70), to obtain intermediate 2. To a solution of the obtained intermediate 2 in DMF (600 μL) there was added diethylamine (11.4 μL, 0.11 mmol), and the mixture was stirred for 40 minutes at room temperature. The reaction mixture was concentrated under reduced pressure and then purified by preparative HPLC (0.1% formic acid aqueous solution:0.1% acetonitrile formate solution = 80:20 to 50:50) to obtain a formate of the title compound (1.3 mg).

ESI-MS (m/z): 1589.70 $[M+H]^+$.

[Production Example 232-1]

3,4-bis(Phenylthio)-1H-pyrrole-2,5-dione

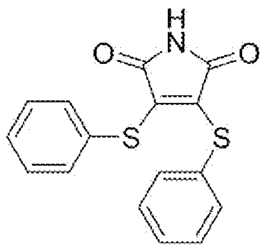

To a solution of 2,3-dibromomaleimide (1.0 g, 3.9 mmol) in methanol (40 mL) there were added sodium hydrogencarbonate (1.65 g, 19.6 mmol) and thiophenol (0.961 mL, 9.42 mmol) at 0°C, and the mixture was stirred for 30 minutes at room temperature. The reaction mixture was concentrated and purified by silica gel chromatography (heptane:ethyl acetate = 9:1 to 3:7) to obtain the title compound (1.3 g).

1H-NMR Spectrum (400 MHz, $CD_3OD$) δ(ppm): 7.14-7.16 (4H, m), 7.22-7.27 (6H, m). ESI-MS (m/z): 314.11 $[M+H]^+$.

[Production Example 232-2]

(S)-3-((*tert*-Butoxycarbonyl)amino)-2-(2,5-dioxo-3,4-bis(phenylthio)-2,5-dihydro-1H-pyrrol-1-yl)propanoic acid

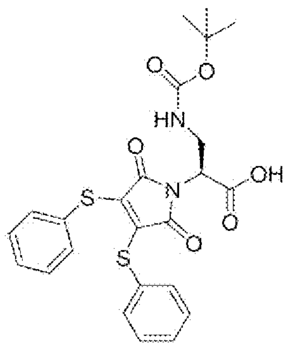

To a solution of the 3,4-bis(phenylthio)-1H-pyrrole-2,5-dione of Production Example 232-1 (760 mg, 2.43 mmol) in THF (10 mL) there were added N-methylmorpholine (348 μL, 3.15 mmol) and methyl chloroformate (243 μL, 3.15 mmol) at room temperature, and the mixture was stirred for 30 minutes at room temperature. Ethyl acetate and water were added

to the reaction mixture, and extraction was performed with ethyl acetate. The organic layer was washed with water and brine. The organic layer was dried over magnesium sulfate and filtered. After concentrating the filtrate under reduced pressure, (S)-2-amino-3-((tert-butoxycarbonyl)amino)propanoic acid (545 mg, 2.67 mmol) and N,N-diisopropylethylamine (847 μL, 4.85 mmol) were added to a solution of the residue in dichloromethane (10 mL), and the mixture was stirred for 40 minutes at room temperature. The reaction mixture was purified by silica gel column chromatography (n-heptane:ethyl acetate = 1:0 to 1:1) to obtain the title compound (315 mg).

ESI-MS (m/z): 1001.26 [2M+H]+.

[Production Example 232-3]

(S)-3-((((9H-Fluoren-9-yl)methoxy)carbonyl)amino)-2-(2,5-dioxo-3,4-bis(phenylthio)-2,5-dihydro-1H-pyrrol-1-yl)propanoic acid

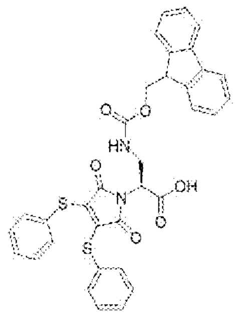

To a solution of the (S)-3-((*tert*-butoxycarbonyl)amino)-2-(2,5-dioxo-34-bis(phenylthio)-2,5-dihydro-1H-pyrrol-1-yl)propanoic acid of Production Example 232-2 (33 mg, 0.066 mmol) in dichloromethane (500 μL) there was added TFA (100 μL), and the mixture was stirred for 30 minutes at room temperature. The reaction mixture was processed 3 times by azeotropic distillation with toluene and concentrated under reduced pressure, after which (9H-fluoren-9-yl)methyl (2,5-dioxopyrrolidin-1-yl)carbonate (26.7 mg, 0.079 mmol) and N,N-diisopropylethylamine (58 μL, 0.33 mmol) were added to a solution of the residue in DMF (500 μL), and the mixture was stirred for 15 minutes at room temperature. The reaction mixture was purified by reversed-phase silica gel column chromatography (0.1% formic acid aqueous solution:0.1% acetonitrile formate solution = 95:5 to 0:1) to obtain a crude product containing the title compound (50 mg).

ESI-MS (m/z): 623.31 [M+H]+.

[Production Example 232-4]

*tert*-Butyl (S)-6-(2,5-dioxo-3,4-bis(phenylthio)-2,5-dihydro-1H-pyrrol-1-yl)-1-(9H-fluoren-9-yl)-3,7-dioxo-2,11,14-trioxa-4,8-diazaheptadecan-17-oate

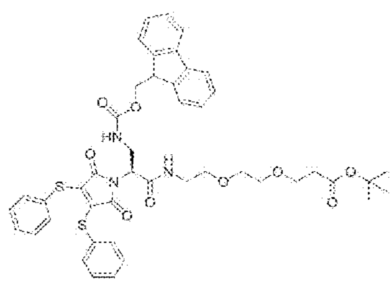

To a solution of the (S)-3-((((9H-fluoren-9-yl)methoxy)carbonyl)amino)-2-(2,5-dioxo-3,4-bis(phenylthio)-2,5-dihydro-1H-pyrrol-1-yl)propanoic acid of Production Example 232-3 (50 mg, 0.080 mmol) in DMF (0.5 mL) there were added 3-(2-(2-aminoethoxy)propanoic acid *tert-butyl* ester (20.6 mg, 0.88 mmol), HATU (30.5 mg, 0.080 mmol) and N,N-diisopropylethylamine (70 μL, 0.40 mmol) at room temperature, and the mixture was stirred for 1 hour at room temperature. The reaction mixture was purified by reversed-phase silica gel column chromatography (0.1% formic acid

aqueous solution:0.1% acetonitrile formate solution = 95:5 to 30:70) to obtain the title compound (31 mg).
ESI-MS (m/z):838.20 $[M+H]^+$.

[Example 233]

(S)-N1-((S)-4-Amino-1-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-1,4-dioxobutan-2-yl)-2-(3-(2-(2-(2,5-dioxo-3,4-bis(phenylthio)-2,5-dihydro-1H-pyrrol-1-yl)ethoxy)ethoxy)propanamido) succinamide

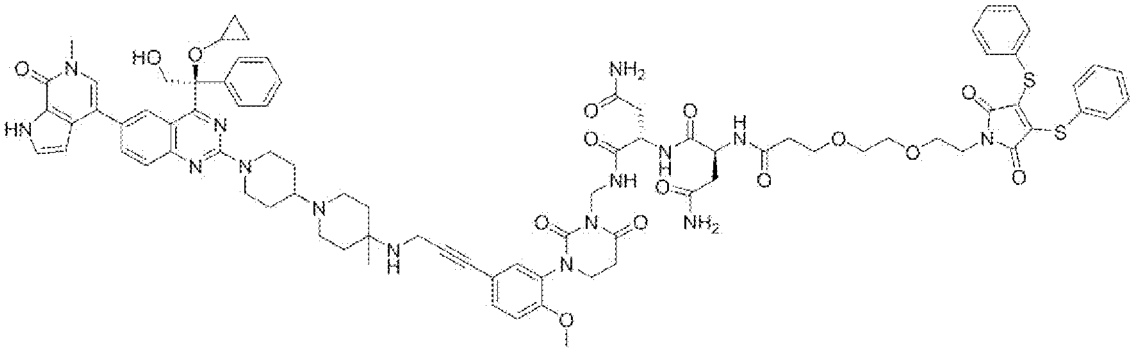

To a solution of the (9H-fluoren-9-yl)methyl((S)-4-amino-1-(((S)-4-amino-1-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl) amino)-1,4-dioxobutan-2-yl)amino)-1,4-dioxobutan-2-yl)carbamate of Production Example 233-2 (5.5 mg, 4.0 μmol) in DMF (500 μL) there was added diethylamine (100 μL), and the mixture was stirred for 30 minutes at room temperature. The reaction mixture was concentrated under reduced pressure to obtain intermediate 1. To a solution of the *tert-butyl* 3-(2-(2-(2,5-dioxo-3,4-bis(phenylthio)-2,5-dihydro-1H-pyrrol-1-yl)ethoxy)ethoxy)propionate of Production Example 201-22 (2.74 mg, 5.17 μmol) in dichloromethane (500 μL) there was added TFA(100 μL), and the mixture was stirred for 30 minutes at room temperature. The reaction mixture was concentrated under reduced pressure, and then N,N-diisopropylethylamine (6.94 μL, 0.040 mmol) and HATU (1.97 mg, 5.17 μmol) were added to a solution of the residue in DMF (500 μL). A solution of intermediate 1 in DMF (500 μL) was added to the reaction mixture at room temperature, and the mixture was stirred for 1 hour. The reaction mixture was purified by preparative HPLC (0.1% formic acid aqueous solution:0.1% acetonitrile formate solution = 4:1 to 1:1), to obtain the title compound (0.87 mg).
ESI-MS (m/z): 1617.87 $[M+H]^+$.

[Production Example 233-1]

(9H-Fluoren-9-yl)methyl((S)-4-amino-1-(((S)-4-amino-1-(((3-(5-iodo-2-methoxyphenyl)-2,6-dioxotetrahydropyrimidine-1(2H)-yl)methyl)amino)-1,4-dioxobutan-2-yl)amino)-1,4-dioxobutan-2-yl)carbamate

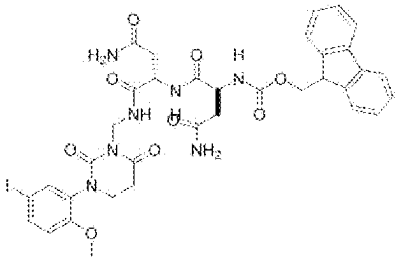

To a solution of the (9H-fluoren-9-yl)methyl(S)-(4-amino-1-(((3-(5-iodo-2-methoxyphenyl)-2,6-dioxotetrahydropyrimidine-1(2H)-yl)methyl)amino)-1,4-dioxobutan-2-yl)carbamate of Production Example 218-1 (20 mg, 0.028 mmol) in

DMF (1 mL) there was added diethylamine (200 μL, 1.91 mmol), and the mixture was stirred for 30 minutes at room temperature. The reaction mixture was concentrated under reduced pressure, and then (((9H-fluoren-9-yl)methoxy) carbonyl)-L-asparagine (11.0 mg, 0.0309 mmol), HATU (11.8 mg, 0.0309 mmol) and N,N-diisopropylethylamine (24.6 μL, 0.141 mmol) were added to a solution of the residue in DMF (1 mL), and the mixture was stirred for 2 hours at room temperature. It was then directly purified by reversed-phase silica gel column chromatography (0.1% formic acid aqueous solution:0.1% acetonitrile formate solution = 95:5 to 0:1), to obtain intermediate 1. To a solution of intermediate 1 (11.8 mg, 0.0241 mmol) in DMF (1 mL) there were added N2-(((9H-fluoren-9-yl)methoxy)carbonyl)-N4-trityl-L-asparagine (17.3 mg, 0.0289 mmol), HATU (11.0 mg, 0.0289 mmol) and N,N-diisopropylethylamine (21.1 μL, 0.121 mmol), and the mixture was stirred for 1 hour at room temperature. Water was added to the reaction mixture for quenching, and the mixture was extracted with dichloromethane. The collected organic layer was washed with water and brine, and the organic layer was dried over magnesium sulfate and filtered. The filtrate was concentrated under reduced pressure to obtain a residue. To a solution of the residue in dichloromethane (1 mL) there was added TFA(200 μL), and the mixture was stirred overnight. The reaction mixture was concentrated under reduced pressure and the residue was purified by reversed-phase silica gel column chromatography (0.1% formic acid aqueous solution:0.1% acetonitrile formate solution = 95:5 to 0:1) to obtain the title compound (9.0 mg).

ESI-MS (m/z): 826.35 $[M+H]^+$.

[Production Example 233-2]

(9H-Fluoren-9-yl)methyl((S)-4-amino-1-(((S)-4-amino-1-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-1,4-dioxobutan-2-yl)amino)-1,4-dioxobutan-2-yl)carbamate

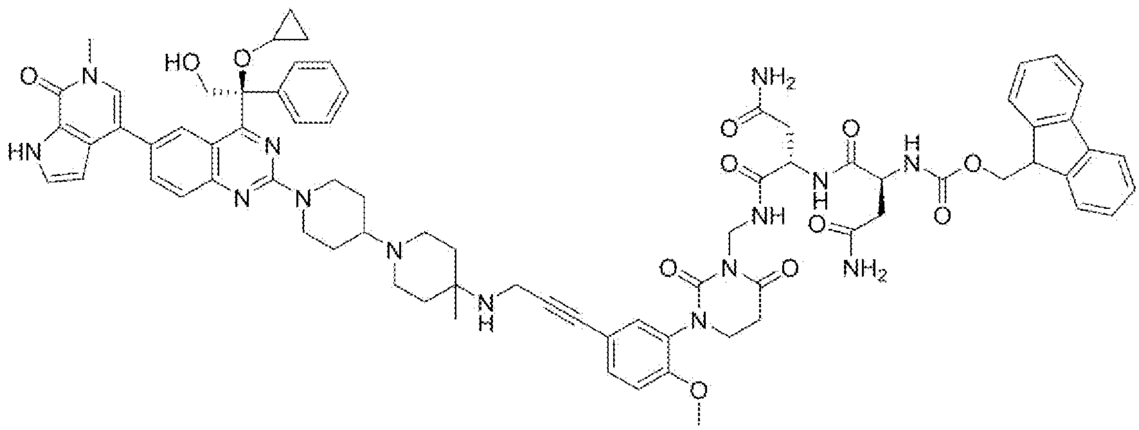

The title compound (5.5 mg) was obtained from the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 201-9 (9.0 mg, 0.014 mmol) and the (9H-fluoren-9-yl)methyl((S)-4-amino-1-(((S)-4-amino-1-(((3-(5-iodo-2-methoxyphenyl)-2,6-dioxotetrahydropyrimidine-1(2H)-yl)methyl)amino)-1,4-dioxobutan-2-yl)amino)-1,4-dioxobutan-2-yl)carbamate of Production Example 233-1 (11.9 mg, 0.0144 mmol), by the same method as Production Example 218-2.

ESI-MS (m/z): 1385.04 $[M+H]^+$.

[Example 234]

(11R, 14S)-11-(2-Carboxyethyl)-14-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidine] -4-yl)amino)prop-1-yn-1-yl)-2-methoxyphenyl)-2,6-dioxotetrahydropyrimidine-1(2H)-yl)methyl)carbamoyl)-1-(2,5-dioxo-3,4-bis(phenylthio)-2,5-dihydro-1H-pyrrol-1-yl)-9,12-dioxo-3,6-dioxa-10,13-diazahexadecan-16-oic acid

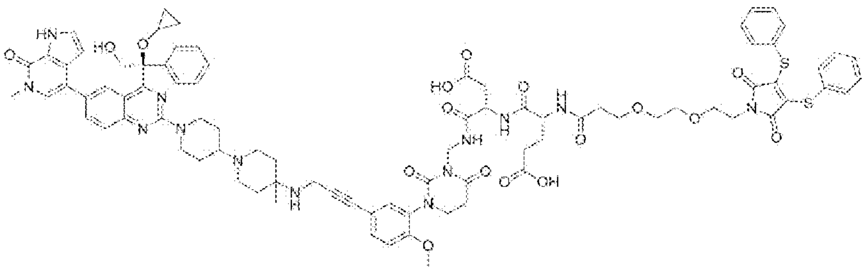

The title compound (2.43 mg) was obtained from the (R)-4-((((9H-fluoren-9-yl)methoxy)carbonyl)amino)-5-(((S)-3-carboxy-1-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-1-oxopropan-2-yl)amino)-5-oxopentanoic acid of Production Example 234-2 (10.0 mg, 7.15 μmol), by the same method as Example 235.
ESI-MS (m/z): 1633.33 $[M+H]^+$.

[Production Example 234-1]

(R)-4-((((9H-Fluoren-9-yl)methoxy)carbonyl)amino)-5-(((S)-3-carboxy-1-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-1-oxopropan-2-yl)amino)-5-oxopentanoic acid

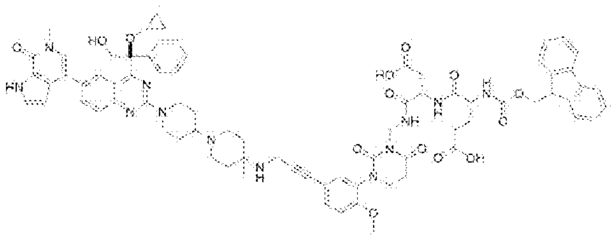

Intermediate 1 (56 mg) was obtained from the *tert-butyl(S)-3((((9H-fluoren-9*-yl)methoxy)carbonyl)amino)-4-(((3-(5-iodo-2-methoxyphenyl)-2,6-dioxotetrahydropyrimidine-1(2H)-yl)methyl)amino)-4-oxobutanoate of Production Example 201-18 (70 mg, 0.091 mmol) and *5-tert-butyl* N-[(9H-fluoren-9-ylmethoxy)carbonyl]-D-glutamic acid (46.5 mg, 0.109 mmol), by the same method as Production Example 223-1. The title compound (19 mg) was obtained from the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 201-9 (15 mg, 0.022 mmol) and intermediate 1 (20.3 mg, 0.0241 mmol), by the same method as Production Example 218-2.
ESI-MS (m/z): 1401.15 $[M+H]^+$.

[Example 235]

(S)-4-((2-(((3-(5-(3-((1'-(4-((S)-1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl}-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-2-oxoethyl)amino)-3-(3-(2-(2-(2,5-dioxo-3,4-bis(phenylthio)-2,5-dihydro-1H-pyrrol-1-yl)ethoxy)ethoxy)propanamido)-4-oxobutanoic acid

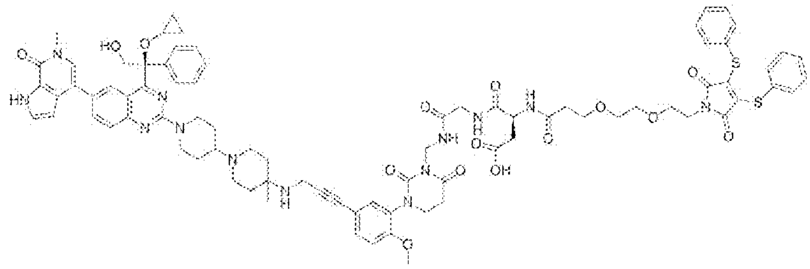

To a solution of the (S)-3-((((9H-fluoren-9-yl)methoxy)carbonyl)amino)-4-((2-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-2-oxoethyl)amino)-4-oxobutanoic acid of Production Example 235-2 (15.3 mg, 0.0115 mmol) in DMF (500 μL) there was added diethylamine (100 μL, 0.0115 mmol), and the mixture was stirred for 20 minutes at room temperature. The reaction mixture was concentrated under reduced pressure to obtain intermediate 1. To a solution of the residue in DMF (500 μL) there were added N,N-diisopropylethylamine (20.1 μL, 0.115 mmol) and the 2,5-dioxopyrrolidin-1-yl 3-(2-(2-(2,5-dioxo-3,4-bis(phenylthio)-2,5-dihydro-1H-pyrrol-1-yl)ethoxy)ethoxy)propanoate of Production Example 201-23 (8.55 mg, 0.0150 mmol) at 50°C, and the mixture was stirred for 4 hours. The reaction mixture was purified by preparative HPLC (0.1% formic acid aqueous solution:0.1% acetonitrile formate solution = 70:30 to 40:60), to obtain the title compound (7.7 mg).

ESI-MS (m/z): 1561.50 $[M+H]^+$.

[Production Example 235-1]

(S)-3-((((9H-Fluoren-9-yl)methoxy)carbonyl)amino)-4-((2-(((3-(5-iodo-2-methoxyphenyl)-2,6-dioxotetrahydropyrimidine-1(2H)-yl)methyl)amino)-2-oxoethyl)amino)-4-oxobutanoic acid

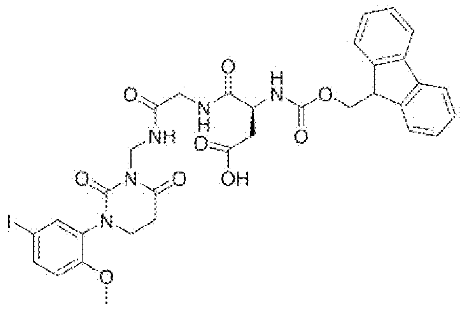

Intermediate 1 was obtained from the (9H-fluoren-9-yl)methyl (2-(((3-(5-iodo-2-methoxyphenyl)-2,6-dioxotetrahydropyrimidine-1(2H)-yl)methyl)amino)-2-oxoethyl)carbamate of Production Example 206-1 (15 mg, 0.023 mmol) and (S)-2-((((9H-fluoren-9-yl)methoxy)carbonyl)amino)-4-(*tert*-butoxy)-4-oxobutanoic acid (10.4 mg, 0.0252 mmol), by the same method as Production Example 229-2. To a solution of intermediate 1 in dichloromethane (1 mL) there was added TFA(200 μL), and the mixture was stirred for 1 hour at room temperature. The reaction mixture was purified by direct reversed-phase silica gel chromatography (0.1% formic acid aqueous solution:0.1% acetonitrile formate solution = 95:5 to 0:1) to obtain the title compound (12 mg).

ESI-MS (m/z): 770.22 $[M+H]^+$.

[Production Example 235-2]

(S)-3-((((9H-Fluoren-9-yl)methoxy)carbonyl)amino)-4-((2-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-2-oxoethyl)amino)-4-oxobutanoic acid

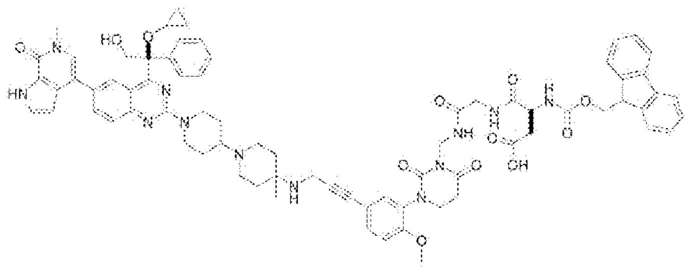

The title compound (15 mg) was obtained from the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 201-9 (15 mg, 0.022 mmol) and the (S)-3-((((9H-fluoren-9-yl)methoxy)carbonyl)amino)-4-((2-(((3-(5-iodo-2-methoxyphenyl)-2,6-dioxotetrahydropyrimidine-1(2H)-yl)methyl)amino)-2-oxoethyl)amino)-4-oxobutanoic acid of Production Example 235-1 (18.5 mg, 0.0241 mmol), by the same method as Production Example 218-2.

ESI-MS (m/z): 1327.96 $[M+H]^+$.

[Example 236]

(S)-N1-(2-((((S)-2-Cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)-2-(3-(2-(2-(2,5-dioxo-3,4-bis(phenylthio)-2,5-dihydro-1H-pyrrol-1-yl)ethoxy)ethoxy)propanamido) succinamide

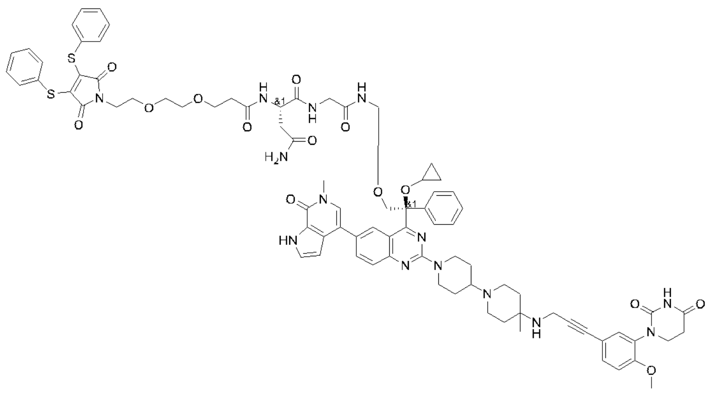

A formate of the title compound (0.35 mg) was obtained from the (9H-fluoren-9-yl)methyl ((S)-4-amino-1-((2-((((S)-2-cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)amino)-1,4-dioxobutan-2-yl)carbamate of Production Example 236-2 (1.3 mg, 0.98 μmol), by the same method as Example 229. ESI-MS (m/z): 1560.51 $[M+H]^+$.

[Production Example 236-1]

(9H-Fluoren-9-yl)methyl ((S)-4-amino-1-((2-(((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)amino)-1,4-dioxobutan-2-yl)carbamate

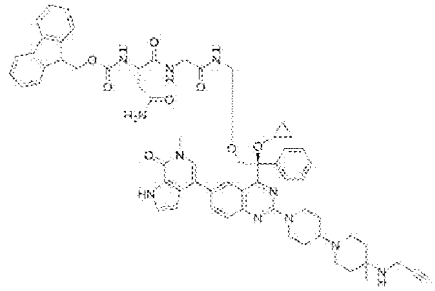

To a solution of the (9H-fluoren-9-yl)methyl(S)-(2-(((2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin4-yl)quinazolin-4-yl)-2-phenylethoxy)methyl)amino)-2-oxoethyl)carbamate of Production Example 201-11 (20 mg, 0.20 mmol) in DMF (1 mL) there was added diethylamine (200 μL), and the mixture was stirred for 30 minutes at room temperature. After processing the reaction mixture 3 times by azeotropic distillation with toluene and concentrating under reduced pressure, the residue was again dissolved in DMF (1 mL), and then HATU (9.9 mg, 0.026 mmol), HOBt (4.0 mg, 0.026 mmol), N,N-diisopropylamine (18 μL, 0.10 mmol) and (((9H-fluoren-9-yl)methoxy)carbonyl)-L-asparagine (9.3 mg, 0.026 mmol) were added and the mixture was stirred for 15 minutes at room temperature. The reaction mixture was purified by direct reversed-phase silica gel chromatography (0.1% formic acid aqueous solution:0.1% acetonitrile formate solution = 70:30 to 30:70) to obtain the title compound (3.3 mg).

ESI-MS (m/z): 1108.87 $[M+H]^+$.

[Production Example 236-2]

(9H-Fluoren-9-yl)methyl ((S)-4-amino-1-((2-((((S)-2-cyclopropoxy-2-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)amino)-1,4-dioxobutan-2-yl)carbamate

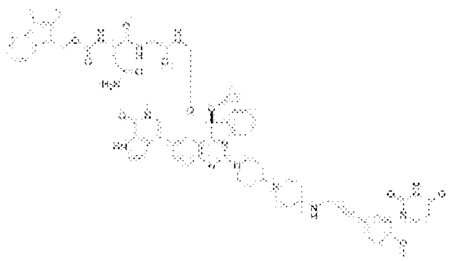

The title compound (1.3 mg) was obtained from the (9H-fluoren-9-yl)methyl ((S)-4-amino-1-((2-((((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)amino)-1,4-dioxobutan-2-yl)carbamate of Production Example 236-1 (6.8 mg, 6.1 μmol) and the 1-(5-iodo-2-methoxyphenyl)dihydropyrimidine-2,4(1H,3H)-dione of Production Example 201-16 (2.4 mg, 6.7 μmol), by the same method as Production Example 218-2.

ESI-MS (m/z): 1327.05 $[M+H]^+$.

[Example 237]

(3S)-3-[({[(2-Cyclopropoxy-2-{2-[4-({3-[3-(2,4-dioxo-1,3-diazinane-1-yl)-4-methoxyphenyl]prop-2-yn-1-yl}amino)-4-methyl-[1,4'-bipiperidin]-1'-yl]-6-{6-methyl-7-oxo-1H,6H,7H-pyrrolo[2,3-c]pyridin-4-yl}quinazolin-4-yl}-2-phenylethoxy)methyl]carbamoyl}methyl)carbamoyl]-3-[3-(2-{2-[2,5-dioxo-3,4-bis(phenylsulfanyl)-2,5-dihydro-1H-pyrrol-1-yl]ethoxy}ethoxy)propaneamide]propanoic acid

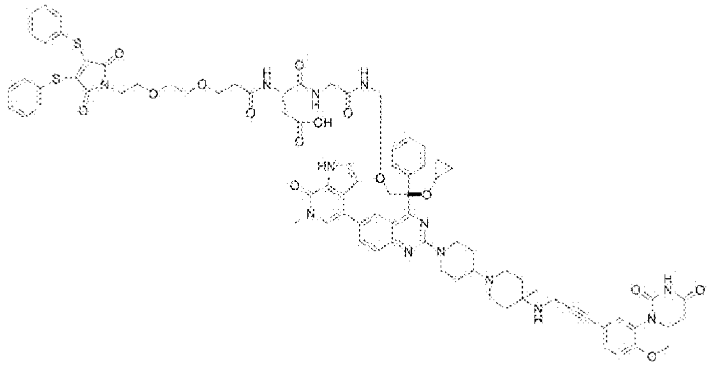

To a solution of the formate of *tert-butyl* (3S) -3-[({[(2-cyclopropoxy-2-{2-[4-({3-[3-(2,4-dioxo-1,3-diazinane-1-yl)-4-methoxyphenyl]prop-2-yn-1-yl}amino)-4-methyl-[1,4'-bipiperidin]-1'-yl]-6-{6-methyl-7-oxo-1H,6H,7H-pyrrolo[2,3-c]pyridin-4-yl}quinazolin-4-yl}-2-phenylethoxy)methyl]carbamoyl}methyl)carbamoyl]-3-({[(9H-fluoren-9-yl)methoxy]carbonyl}amino)propanoate of Production Example 237-2 (3.0 mg) in THF (1.1 mL) there was added potassium *tert-butoxide* (2.4 mg, 22 μmol) at 0°C, and after stirring for 20 minutes, a solution of 1 N hydrochloric acid (0.022 mL, 0.022 mmol) and triethylamine (0.20 mL, 1.4 mmol) in THF (0.5 mL) was added to halt the reaction. Toluene (1.0 mL) and methanol (1.0 mL) were added and azeotropic distillation was carried out to obtain an intermediate. In a separate vessel, TFA(0.50 mL) was added to a solution of the *tert-butyl* 3-(2-(2-(2,5-dioxo-3,4-bis(phenylthio)-2,5-dihydro-1H-pyrrol-1-yl)ethoxy)ethoxy) propionate of Production Example 201-22 (7.0 mg, 0.013 mmol) in dichloromethane (0.50 mL), and the mixture was stirred for 1 hour at room temperature and then concentrated. Toluene (1.0 mL) was added to the obtained residue and azeotropic distillation was repeated 3 times, after which it was dissolved in DMF (0.50 mL). To the reaction mixture there were added triethylamine (20 μL, 1.4 mmol) and HATU (5.0 mg, 0.13 mmol), and after stirring the mixture for 3 minutes at room temperature, it was combined with a solution of the obtained intermediate in DMF (0.50 mL). The reaction mixture was stirred for 30 minutes at room temperature and then purified by UFPLC to obtain a formate of the title compound (0.70 mg).

ESI-MS (m/z): 1560.47 $[M+H]^+$.

[Production Example 237-1]

(9H-Fluoren-9-yl)methyl N-({[(2-cyclopropoxy-2-{2-[4-({3-[3-(2,4-dioxo-1,3-diazinane-1-yl)-4-methoxyphenyl]prop-2-yn-1-yl}amino)-4-methyl-[1,4'-bipiperidin]-1'-yl]-6-{6-methyl-7-oxo-1H,6H,7H-pyrrolo[2,3-c]pyridin-4-yl}quinazolin-4-yl}-2-phenylethoxy)methyl]carbamoyl}methyl)carbamate

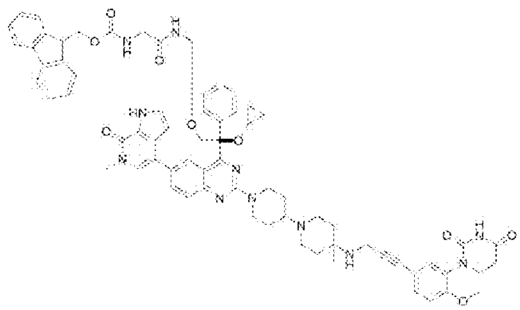

To a mixture of the (S)-1-(5-(3-((1'-(4-(1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)dihydropyrimidine-2,4(1H,3H)-dione of Production Example 207-2 (50 mg, 55 μmol) and the (2-((((9H-fluoren-9-yl) methoxy)carbonyl)amino)acetamide)methyl acetate of Production Example 201-10 (41 mg, 0.11 mmol) there was added toluene (2.0 mL), and azeotropic distillation was repeated twice. After adding CSA (26 mg, 0.11 mmol), dichloromethane (712 μL) and THF (906 μL) to the mixture, it was stirred for 1 hour at room temperature, and then (2-((((9H-fluoren-9-yl) methoxy)carbonyl)amino)acetamide)methyl acetate (41 mg, 0.11 mmol) and CSA(26 mg, 0.11 mmol) were added and the mixture was stirred for 1 hour at room temperature. To this mixture there were added (2-((((9H-fluoren-9-yl)methoxy) carbonyl)amino)acetamide)methyl acetate (41 mg, 0.11 mmol) and CSA(26 mg, 0.11 mmol), and the mixture was stirred

for 30 minutes at room temperature. Triethylamine (400 μL) was added to the reaction mixture to halt the reaction, and after concentration, the residue was purified by silica gel column chromatography (ethyl acetate:heptane = 50:50 to 0:100, followed by methanol:ethyl acetate = 0:100 to 70:30) to obtain a crude product. The obtained crude product was purified by ODS silica gel column chromatography (0.1% acetonitrile formate solution:0.1% formic acid aqueous solution = 2:98 to 70:30) to obtain a formate of the title compound (15 mg).

ESI-MS (m/z): 1212.55 [M+H]+.

[Production Example 237-2]

*tert-Butyl* (3S)-3-[({[(2-cyc1opropoxy-2-{2-[4-({3-[3-(2,4-dioxo-1,3-diazinane-1-yl)-4-methoxyphenyl]prop-2-yn-l-yl}amino)-4-methyl-[1,4'-bipiperidine]-1'-yl]-6-{6-methyl-7-oxo-1H,6H,7H-pyrrolo[2,3-c]pyridin4-yl}quinazolin-4-yl}-2-phenylethoxy)methyl]carbamoyl }methyl)carbamoyl]-3-({ [(9H-fluoren-9-yl)methoxy]carbonyl}amino)propanoate

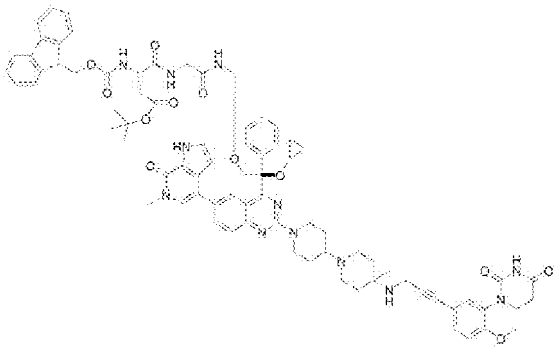

To a solution of the formate of (9H-fluoren-9-yl)methyl N-({[(2-cyclopropoxy-2- {2-[4-({3-[3-(2,4-dioxo-1,3-diazinane-1-yl)-4-methoxyphenyl]prop-2-yn-1-yl}amino)-4-methyl-[1,4'-bipiperidin]-l'-yl]-6-{6-methyl-7-oxo-1H,6H,7H-pyrrolo[2,3-c]pyridin-4-yl}quinazolin-4-yl}-2-phenylethoxy)methyl]carbamoyl}methyl)carbamate of Production Example 237-1 (7.5 mg) in DMF (480 μL) there was added diethylamine (0.10 mL, 0.97 mmol), and the mixture was stirred for 20 minutes at room temperature. The reaction mixture was concentrated and processed 3 times by azeotropic distillation with toluene (1 mL). To the obtained residue there were added DMF (480 μL), (2S)-4-(*tert*-butoxy)-2-({[(9H-fluoren-9-yl)methoxy]carbonyl}amino)-4-oxobutanoic acid (3.8 mg, 9.3 μmol), N,N-diisopropylethylamine (11 μL, 62 μmol) and HATU (3.3 mg, 8.7 μmol), and the mixture was stirred for 3 hours at room temperature. The reaction mixture was purified by ODS silica gel column chromatography (0.1% acetonitrile formate solution:0.1% formic acid aqueous solution = 2:98 to 70:30) to obtain a formate of the title compound (3.0 mg).

ESI-MS (m/z): 1384.58 [M+H]+.

[Example 238]

(S)-15-(((3-(5-(3-((1'-(4-((S)-1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)-1-(2,5-dioxo-3,4-bis(phenylthio)-2,5-dihydro-1H-pyrrol-1-yl)-9,13-dioxo-3,6-dioxa-10,14-diazaheptadecan-17-oic acid

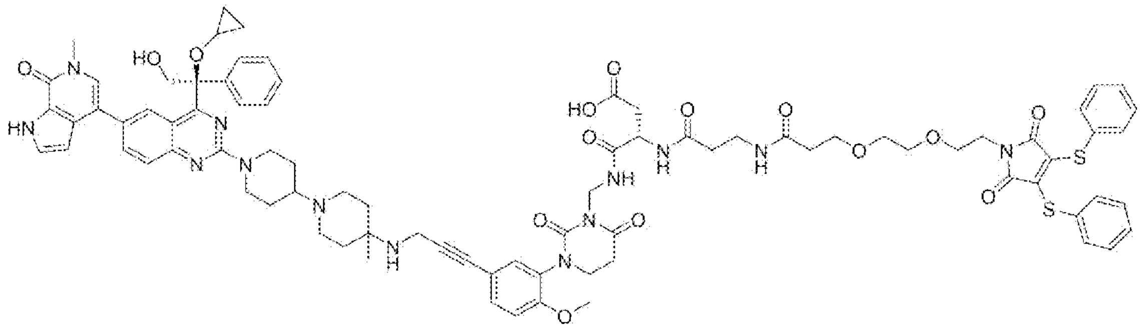

To a solution of the (S)-3-((((9H-fluoren-9-yl)methoxy)carbonyl)amino)-4-(((3-(5-(3-((1'-(4-((S))-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidine]-4-yl)amino)prop-1-yn-1-yl)-2-methoxyphenyl)-2,6-dioxotetrahydropyrimidine-1(2H)-yl)methyl)amino)-4-oxobutanoic acid of Production Example 221-2 (9 mg, 7 μmol) in DMF (2 mL) there was added diethylamine (100 μL), and the mixture was stirred for 30 minutes at room temperature and then concentrated under reduced pressure to obtain intermediate 1. To a solution of the 3-(3-(2-(2-(2,5-dioxo-3,4-bis(phenylthio)-2,5-dihydro-1H-pyrrol-1-yl)ethoxy)ethoxy)propanamido)propanoic acid of Production Example 238-1 (4.24 mg, 7.79 μmol) in DMF (2 mL) there were added HATU (2.83 mg, 7.44 μmol) and N,N-diisopropylethylamine (100 μL, 0.573 mmol), and the mixture was stirred for 10 minutes at room temperature, after which intermediate 1 was further added and the mixture was stirred for 1 hour. The reaction mixture was purified by preparative HPLC (0.1% formic acid aqueous solution:0.1% acetonitrile formate solution = 4:1 to 1:1), to obtain the title compound (2 mg).
ESI-MS (m/z): 1575.94 [M+H]+.

[Production Example 238-1]

3-(3-(2-(2-(2,5-Dioxo-3,4-bis(phenylthio)-2,5-dihydro-1H-pyrrol-1-yl)ethoxy)ethoxy)propanamido)propanoic acid

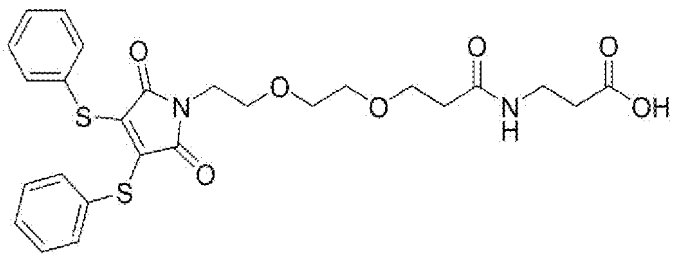

To a solution of the *tert*-butyl 3-(2-(2-(2,5-dioxo-3,4-bis(phenylthio)-2,5-dihydro-1H-pyrrol-1-yl)ethoxy)ethoxy) propionate of Production Example 201-22 (20 mg, 0.038 mmol) in dichloromethane (2 mL) there was added TFA (1 mL), and after stirring for 30 minutes at room temperature, the reaction mixture was concentrated under reduced pressure to obtain intermediate 1. To a solution of intermediate 1 in DMF (2 mL) there were added β-alanine *tert-butyl* ester hydrochloride (7.55 mg, 0.042 mmol), HATU (15.8 mg, 0.042 mmol) and N,N-diisopropylethylamine (20 μL, 0.113 mmol), and the mixture was stirred for 1 hour at room temperature. Water and ethyl acetate were added to the reaction mixture, and the organic layer was collected, dried over sodium sulfate and concentrated under reduced pressure to obtain intermediate 2. TFA (1 mL) was added to a solution of intermediate 2 in dichloromethane (2 mL), and the mixture was stirred for 2 hours. The reaction mixture was purified by reversed-phase silica gel column chromatography (0.1% formic acid aqueous solution:0.1% acetonitrile formate solution = 1: 1 to 0:1) to obtain the title compound (14 mg).
ESI-MS (m/z): 545.26 [M+H]+.

[Example 239]

(44S,51S)-51-(((3-(5-(3-((1'-(4-((S)-1-Cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)-44-(3-(2-(2-(2-(2,5-dioxo-3,4-bis(phenylthio)-2,5-dihydro-1H-pyrrol-1-yl)ethoxy)ethoxy)propanamido)-38,45,49-trioxo-2,5,8,11,14,17,20,23,26,29,32,35-dodecaoxa-39,46,50-triazatripentacontan-53-oic acid

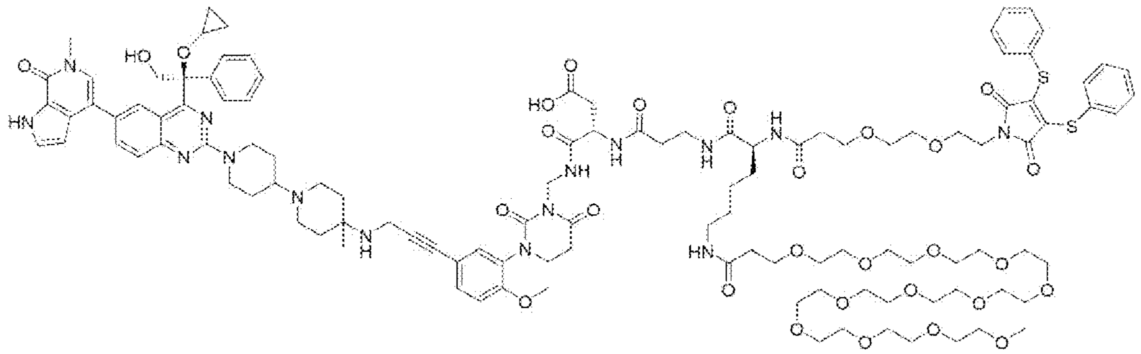

A solution of the *tert-butyl* (S)-44-(3-(2-(2-(2,5-dioxo-3,4-bis(phenylthio)-2,5-dihydro-1H-pyrrol-1-yl)ethoxy)ethoxy)propanamido)-38,45-dioxo-2,5,8,11,14,17,20,23,26,29,32,35-dodecaoxa-39,46-diazanonatetracontan-49-oate of Production Example 239-3 (20 mg, 0.015 mmol) in dichloromethane (2 mL) and TFA (1 mL) was stirred for 30 minutes and then concentrated under reduced pressure to obtain intermediate 1. The title compound (7.6 mg) was obtained from the (S)-3-((((9H-fluoren-9-yl)methoxy)carbonyl)amino)-4-(((3-(5-(3-((1'-(4-((S))-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid of Production Example 221-2 (15 mg, 0.012 mmol) and intermediate 1, by the same method as Example 238.

ESI-MS (m/z): 1137.94 [1/2 M+H]$^+$.

[Production Example 239-1]

*tert-Butyl* (S)-44-((((9H-fluoren-9-yl)methoxy)catbonyl)amino)-38,45-dioxo-2,5,8,11,14,17,20,23,26,29,32,35-dodecaoxa-39,46-diazanonatetracontan-49-oate

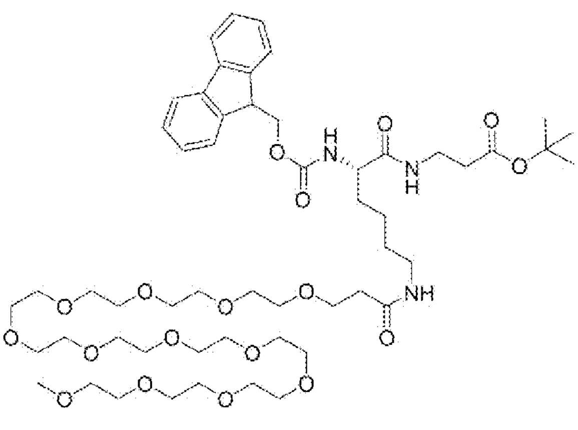

To a solution of the (S)-44-((((9H-fluoren-9-yl)methoxy)carbonyl)amino)-38-oxo-2,5,8,11,14,17,20,23,26,29,32,35-dodecaoxa-39-azapentatetracontan-45-oic acid of Production Example 201-12 (464 mg, 0.494 mmol) in DMF (2 mL) there were added β-alanine *tert-butyl* ester hydrochloride (90 mg, 0.494 mmol), HATU (207 mg, 0.543 mmol) and N,N-diisopropylethylamine (173 μL, 0.988 mmol), and the mixture was stirred for 2 hours at room temperature. The reaction mixture was purified by reversed-phase silica gel column chromatography (0.1% formic acid aqueous solution:0.1% acetonitrile formate solution = 95:5 to 0:1) to obtain the title compound (459 mg).

ESI-MS (m/z): 1066.87 [M+H]$^+$.

[Production Example 239-2]

*tert*-Butyl (S)-44-(3-(2-(2-(2,5-dioxo-3,4-bis(phenylthio)-2,5-dihydro-1H-pyrrol-1-yl)ethoxy)ethoxy)propanamido)-38,45-dioxo-2,5,8,11,14,17,20,23,26,29,32,35-dodecaoxa-39,46-diazanonatetracontan-49-oate

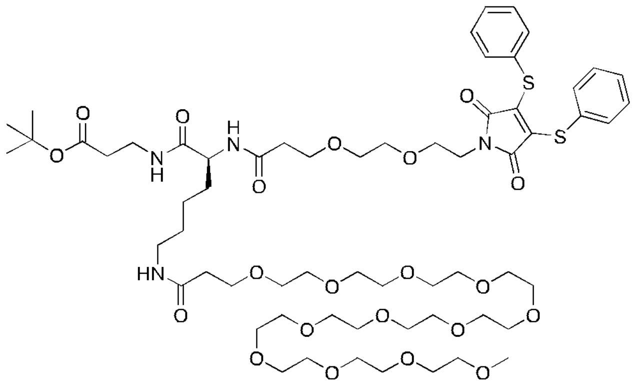

To a solution of the *tert*-butyl 3-(2-(2-(2,5-dioxo-3,4-bis(phenylthio)-2,5-dihydro-1H-pyrrol-1-yl)ethoxy)ethoxy) propionate of Production Example 201-22 (27.3 mg, 0.052 mmol) in dichloromethane (2 mL) there was added TFA (1 mL), and after stirring for 30 minutes at room temperature, the reaction mixture was concentrated under reduced pressure to obtain intermediate 1. To a solution of the *tert-butyl* (S)-44-((((9H-fluoren-9-yl)methoxy)carbonyl)amino)-38,45-dioxo-2,5,8,11,14,17,20,23,26,29,32,35-dodecaoxa-39,46-diazanonatetracontan-49-oate of Production Example 239-1 (55 mg, 0.052 mmol) in DMF (2 mL) there was added diethylamine (200 μL), and the mixture was stirred for 20 minutes at room temperature and then concentrated under reduced pressure to obtain intermediate 2. To a solution of intermediate 1 in DMF (2 mL) there were added HATU (21.6 mg, 0.057 mmol) and N,N-diisopropylethylamine (200 μL, 1.15 mmol), and the mixture was stirred for 10 minutes at room temperature. Intermediate 2 was added to the reaction mixture, which was then stirred for 30 minutes. The reaction mixture was purified by reversed-phase silica gel column chromatography (0.1% formic acid aqueous solution:0.1% acetonitrile formate solution = 95:5 to 1:9) to obtain the title compound (27 mg).
ESI-MS (m/z): 1300.02 [M+H]+.

[Example 240]

(S)-N1-(2-(((3-(5-(3-((1'-(4-((S)-1-Cyclopropoxy-2-hydroxy-1-phenylethyl)-6-(6-methyl-7-oxo-6,7-dihydro-1H-pyrrolo [2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-2-oxoethyl)-2-((S)44-(3-(2-(2-(2,5-dioxo-3,4-bis(phenylthio)-2,5-dihydro-1H-pyrrol-1-yl)ethoxy)ethoxy)propanamido)-38-oxo-2,5,8,11,14,17,20,23,26,29,32,35-dodecaoxa-39-azapentatetracontan-45-amide) succinamide

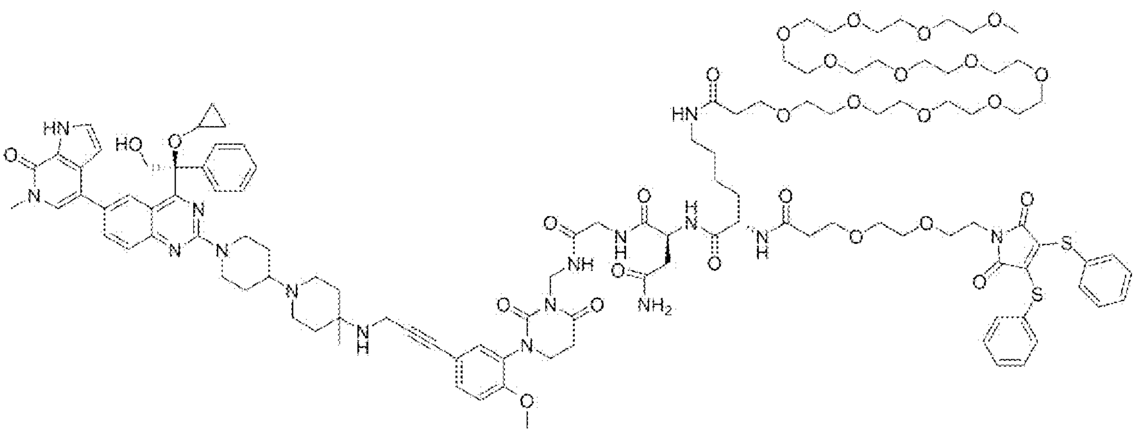

A formate of the title compound (1.3 mg) was obtained from the (9H-fluoren-9-yl)methyl ((44S, 47S)-47-(2-amino-2-oxoethyl)-53-(3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)-38,45,48,51-tetraoxo-2,5,8,11,14,17,20,23,26,29,32,35-dodecaoxa-39,46,49,52-tetraazatripentacontan-44-yl)carbamate of Production Example 240-1 (8.0 mg, 4.0 μmol), by the same method as Example 229.

ESI-MS (m/z): 1130.33 $[(M+2H)/2]^+$.

[Production Example 240-1]

(9H-Fluoren-9-yl)methyl ((44S, 47S)-47-(2-amino-2-oxoethyl)-53-(3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)-38,45,48,51-tetraoxo-2,5,8,11,14,17,20,23,26,29,32,35-dodecaoxa-39,46,49,52-tetraazatripentacontan-44-yl)carbamate

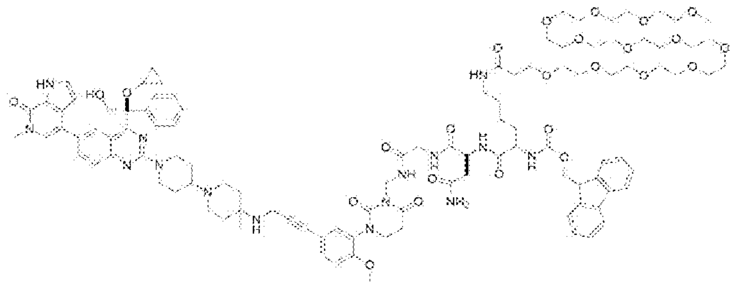

To a solution of the (9H-fluoren-9-yl)methyl ((S)-4-amino-1-((2-(((3-(5-(3-((1'-(4-((S)-1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidine]-4-yl)amino)prop-1-yn-1-yl)-2-methoxyphenyl)-2,6-dioxotetrahydropyrimidine-1(2H)-yl)methyl)amino)-2-oxoethyl)amino)-1,4-dioxobutan-2-yl)carbamate of Production Example 229-2 (10.9 mg, 8.22 μmol) in DMF (1 mL) there was added diethylamine (200 μL), and the mixture was stirred for 20 minutes at room temperature. The reaction solution was processed 3 times by azeotropic distillation with toluene and concentrated under reduced pressure, and then the (S)-44-((((9H-fluoren-9-yl)methoxy)carbonyl)amino)-38-oxo-2,5,8,11,14,17,20,23,26,29,32,35-dodecaoxa-39-azapentatetracontan-45-oic acid of Production Example 201-12 (10.0 mg, 10.7 μmol), HATU (4.1 mg, 11 μmol) and N,N-diisopropylethylamine (7.2 μL, 0.041 mmol) were added to a solution of the residue in DMF (1 mL), and the mixture was stirred for 1 hour at room temperature. The reaction mixture was purified by reversed-phase silica gel column chromatography (0.1% formic acid aqueous solution:0.1% acetonitrile formate solution = 70:30 to 20:80) to obtain a formate of the title compound (4.7 mg).

ESI-MS (m/z): 1013.84 $[(M+2H)/2]^+$.

[Example 241]

(S)-2-Acetamide-N1-((3-(5-(3-((1'-(4-((1S,10S)-10-benzyl-1-cyclopropoxy-26-(2,5-dioxo-3,4-bis(phenylthio)-2,5-dihydro-1H-pyrrol-1-yl)-6,9,12,15,18-pentaoxo-1-phenyl-3,21,24-trioxa-5,8,11,14,17-pentaazahexacosyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl) succinamide

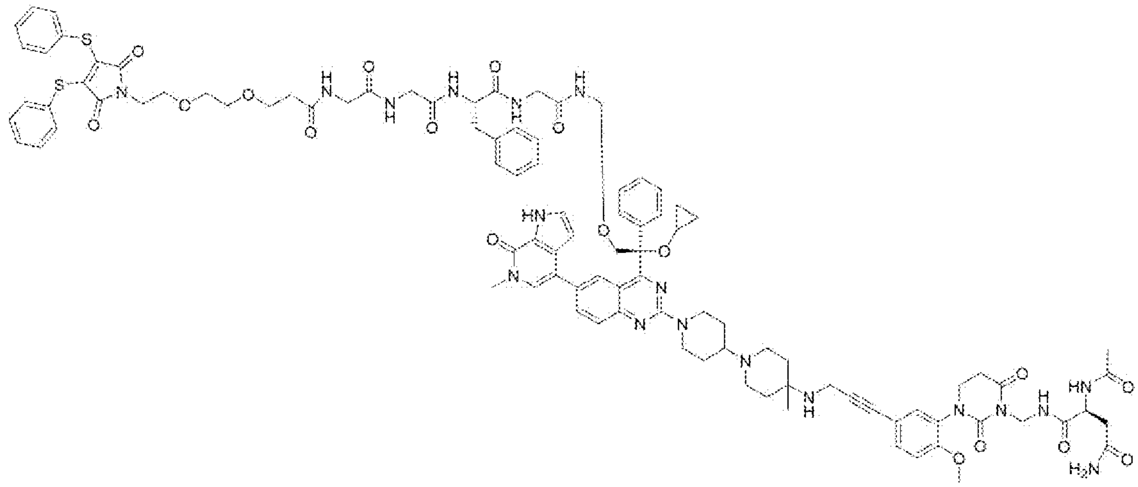

A formate of the title compound (14 mg) was obtained from the formate of (S)-2-acetamide-N1-((3-(5-(3-((1'-(4-((1S,10S)-16-amino-10-benzyl-1-cyclopropoxy-6,9,12,15-tetraoxo-1-phenyl-3-oxa-5,8,11,14-tetraazahexadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl) succinamide of Production Example 241-2 (22 mg) and the 2,5-dioxopyrrolidin-1-yl 3-(2-(2-(2,5-dioxo-3,4-bis(phenylthio)-2,5-dihydro-1H-pyrrol-1-yl)ethoxy)ethoxy)propanoate of Production Example 201-23 (14.8 mg, 0.028 mmol), by the same method as Example 201.
ESI-MS (m/z):947.02 [(M+2H)/2]$^+$.

[Production Example 241-1]

(S)-2-Acetamide-N1-((3-(5-iodo-2-methoxyphenyl)-2,6-dioxotetrahydropyrimidine-1(2H)-yl)methyl) succinamide

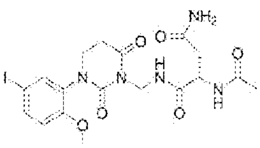

To a solution of the (9H-fluoren-9-yl)methyl (S)-(4-amino-1-(((3-(5-iodo-2-methoxyphenyl)-2,6-dioxotetrahydropyrimidine-1(2H)-yl)methyl)amino)-1,4-dioxobutan-2-yl)carbamate of Production Example 218-1 (100 mg, 0.141 mmol) in DMF (1.0 mL) there was added diethylamine (218 μL, 2.11 mmol), and the mixture was stirred for 30 minutes at room temperature. The reaction mixture was concentrated under reduced pressure and the residue was dissolved in THF (1.2 mL) and dichloromethane (1.2 mL), and then N,N-diisopropylethylamine (36.8 μL, 0.211 mmol) and acetic anhydride (15.9 μL, 0.169 mmol) were added and the mixture was stirred for 10 minutes at room temperature. The reaction mixture was diluted with DMF (0.4 mL) and stirred for 10 minutes, after which it was concentrated under reduced pressure to 1/6 volume. The residue was purified by reversed-phase silica gel chromatography (0.1% formic acid aqueous solution:0.1% acetonitrile formate solution = 9:1 to 6:4) to obtain the title compound (60 mg).
ESI-MS (m/z): 532.14 [M+H]$^+$.

[Production Example 241-2]

(S)-2-Acetamide-N1-((3-(5-(3-((1'-(4-((1S,10S)-16-amino-10-benzyl-1-cyclopropoxy-6,9,12,15-tetraoxo-1-phenyl-3-oxa-5,8,11,14-tetraazahexadecyl)-6-(6-methyl-7-oxo-6,7-dihydro-1H-pyrrolo[2,3-c]pyridin-4-yl)quinazolin-2-yl)-4-methyl-[1,4''-bipiperidin]-4-yl)amino)prop-1-yn-1-yl)-2-methoxyphenyl)-2,6-dioxotetrahydropyrimidine-1(2H)-yl)methyl) succinamide

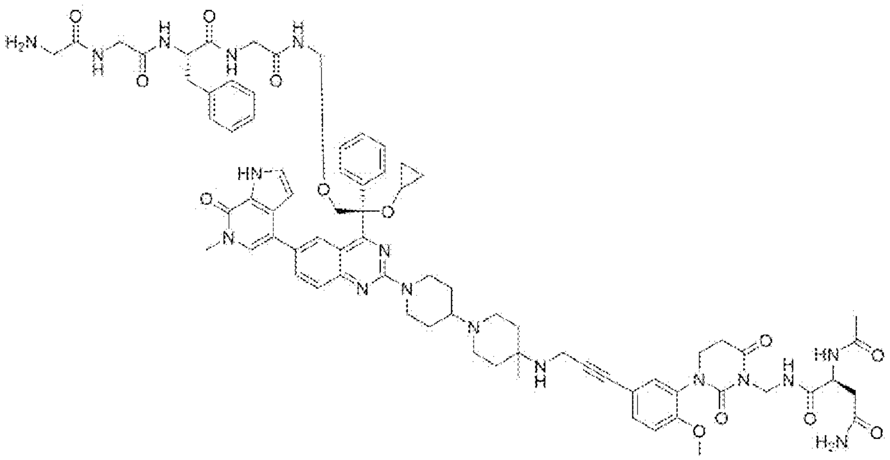

A formate of the title compound (22 mg) was obtained from the formate of (F9H-fluoren-9-yl)methyl((1S,10S)-10-benzyl-1-cyclopropoxy-1-(2-(4-methyl-4-(prop-2-yn-1-ylamino)-[1,4'-bipiperidine]-1'-yl)-6-(6-methyl-7-oxo-6,7-dihydro-1H-pyrrolo[2,3-c]pyridin-4-yl)quinazolin-4-yl)-6,9,12,15-tetraoxo-1-phenyl-3-oxa-5,8,11,14-tetraazahexadecan-16-yl)carbamate of Production Example 203-1 (35 mg) and the (S)-2-acetamide-N1-((3-(5-iodo-2-methoxyphenyl)-2,6-dioxotetrahydropyrimidine-1(2H)-yl)methyl) succinamide of Production Example 241-1 (21 mg, 0.039 mmol), by the same method as Production Example 203-2.

ESI-MS (m/z): 719.24 $[(M+2H)/2]^+$.

[Example 242]

(S)-2-Acetamide-N1-(((S)-2-(2-(4-((3-(3-(3-((S)-7-benzyl-23-(2,5-dioxo-3,4-bis(phenylthio)-2,5-dihydro-1H-pyrrol-1-yl)-3,6,9,12,15-pentaoxo-18,21-dioxa-2,5,8,11,14-pentaazatricosyl)-2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-cyclopropoxy-2-phenylethoxy)methyl) succinamide

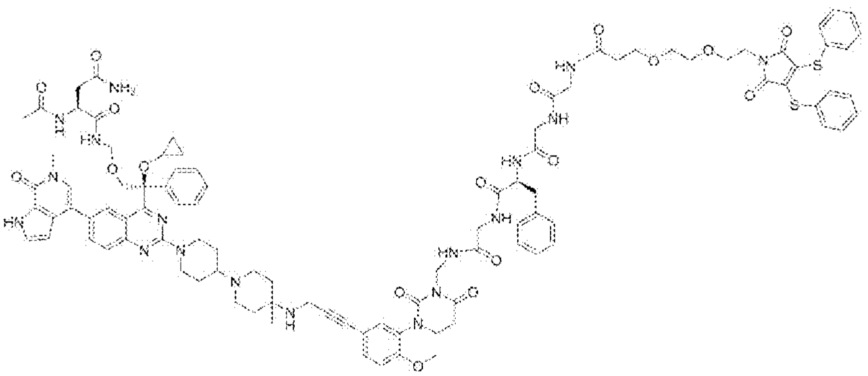

A formate of the title compound (1.6 mg) was obtained from the formate of (S)-2-acetamide-N1-(((S)-2-(2-(4-((3-(3-(3-((S)-13-amino-7-benzyl-3,6,9,12-tetraoxo-2,5,8,11-tetraazatridecyl)-2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-cyclopropoxy-2-phenylethoxy)methyl) succinamide of Production Example 242-2 (3.0 mg) and the 2,5-dioxopyrrolidin-1-yl 3-(2-(2-(2,5-dioxo-3,4-bis(phenylthio)-2,5-dihydro-1H-pyrrol-1-yl)ethoxy)ethoxy)propanoate of Production Example 201-23 (2.2 mg, 0.0039 mmol), by the same method as Example 201.

ESI-MS (m/z): 947.05 $[(M+2H)/2]^+$.

[Production Example 242-1]

(S)-2-Acetamide-N1-(((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl) succinamide

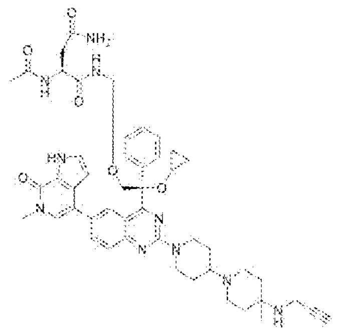

To a solution of a formate of the (9H-fluoren-9-yl)methyl ((S)-4-amino-1-((((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-1,4-dioxobutan-2-yl)carbamate of Production Example 204-4 (14.2 mg) in DMF (260 μL) there was added diethylamine (26.0 μL, 0.248 mmol), and the mixture was stirred for 30 minutes at room temperature. The reaction mixture was concentrated under reduced pressure and the residue was dissolved in THF (300 μL) and dichloromethane (300 μL), and then N,N-diisopropylethylamine (4.3 μL, 0.025 mmol) and acetic anhydride (1.4 μL, 0.015 mmol) were added and the mixture was stirred for 30 minutes at room temperature. The reaction mixture was concentrated under reduced pressure, and the residue was purified by NH silica gel column chromatography (ethyl acetate:methanol = 1:0 to 7:3) to obtain the title compound (7.5 mg).
ESI-MS (m/z): 871.55 $[M+H]^+$.

[Production Example 242-2]

(S)-2-Acetamide-N1-(((S)-2-(2-(4-((3-(3-(3-((S)-13-amino-7-benzyl-3,6,9,12-tetraoxo-2,5,8,11-tetraazatridecyl)-2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-cyclopropoxy-2-phenylethoxy)methyl) succinamide

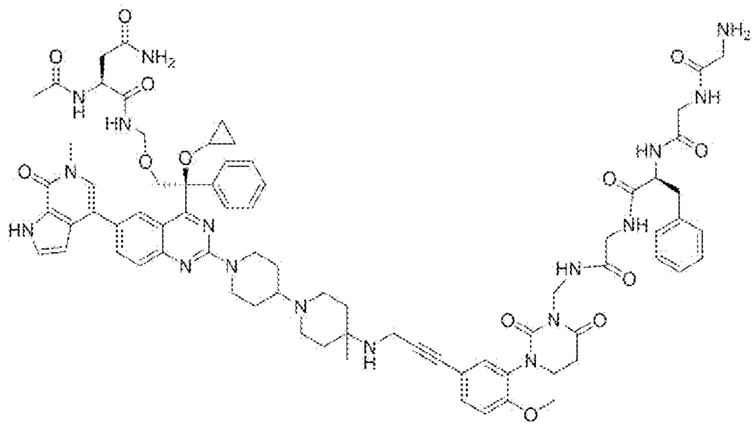

A formate of the title compound (3.0 mg) was obtained from the (S)-2-acetamide-N1-(((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl) succinamide of Production Example 242-1 (3.0 mg, 0.0034 mmol) and the (9H-fluoren-9-yl)methyl (S)-(7-benzyl-1-(3-(5-iodo-2-methoxyphenyl)-2,6-dioxotetrahydropyrimidine-1(2H)-yl)-3,6,9,12-tetraoxo-2,5,8,11-tetraazatridecan-13-yl)carbamate of Production Example 206-2 (6.3 mg, 0.0069 mmol), by the same method as Production Example 204-5. ESI-MS (m/z): 719.28 $[(M+2H)/2]^+$.

[Example 243]

(S)-2-Acetamide-N1-(((S)-2-(2-(4-((3-(3-(3-((S)-4-(2-amino-2-oxoethyl)-14-(2,5-dioxo-3,4-bis(phenylthio)-2,5-dihydro-1H-pyrrol-1-yl)-3,6-dioxo-9,12-dioxa-2,5-diazatetradecyl)-2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-cyclopropoxy-2-phenylethoxy)methyl) succinamide

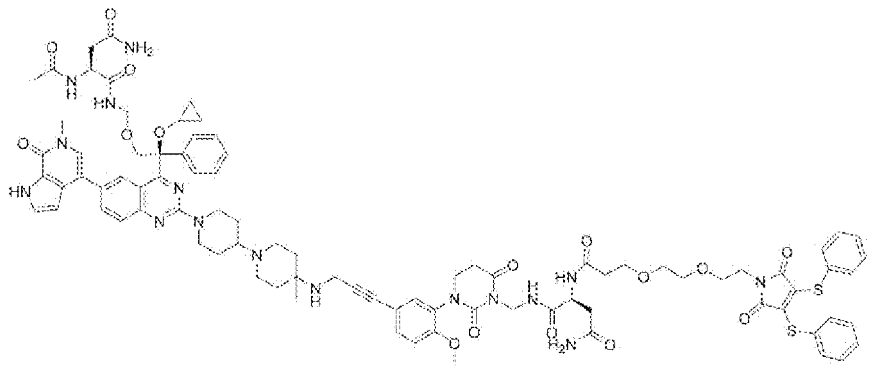

A formate of the title compound (0.45 mg) was obtained from the formate of (S)-2-acetamide-N1-(((S)-2-cyclopropoxy-2-(2-(4-((3-(3-(3-(((S)-2,4-diamino-4-oxobutanamide)methyl)-2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl) succinamide of Production Example 243-1 (1.8 mg) and the 2,5-dioxopyrrolidin-1-yl 3-(2-(2-(2,5-dioxo-3,4-bis(phenylthio)-2,5-dihydro-1H-pyrrol-1-yl)ethoxy)ethoxy)propanoate of Production Example 201-23 (2.2 mg, 0.0039 mmol) at a reaction temperature of 50°C, by the same method as Example 201.

ESI-MS (m/z): 844.90 [(M+2H)/2]$^+$.

[Production Example 243-1]

(S)-2-Acetamide-N1-(((S)-2-cyclopropoxy-2-(2-(4-((3-(3-(3-(((S)-2,4-diamino-4-oxobutanamide)methyl)-2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl) succinamide

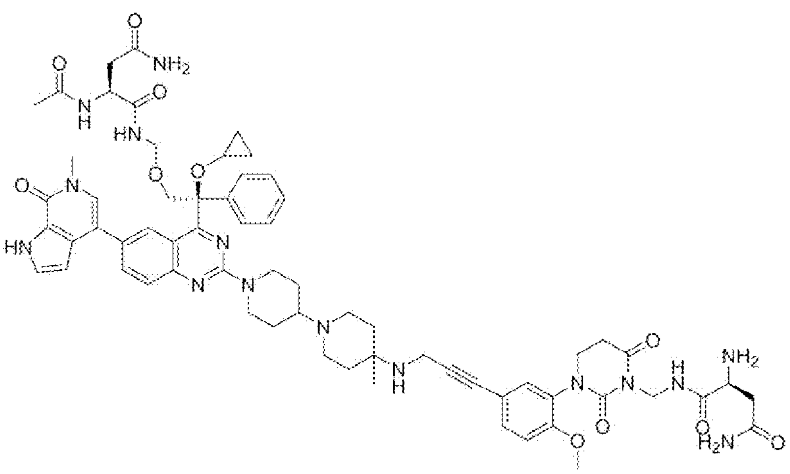

A formate of the title compound (1.8 mg) was obtained from the formate of (S)-2-acetamide-N1-(((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl) succinamide of Production Example 242-1 (3.0 mg) and the (9H-fluoren-9-yl)methyl(S)-(4-amino-1-(((3-(5-iodo-2-methoxyphenyl)-2,6-dioxotetrahydropyrimidine-1(2H)-yl)methyl)amino)-1,4-dioxobutan-2-yl)carbamate of Production Example 218-1(4.9 mg, 0.0069 mmol), by the same method as Production Example 204-5.

ESI-MS (m/z): 617.05 [(M+2H)/2]+.

[Example 244]

(S)-2-Acetamide-N1-(2-(((3-(5-(3-((1'-(4-((1S,10S)-10-benzyl-1-cyclopropoxy-26-(2,5-dioxo-3,4-bis(phenylthio)-2,5-dihydro-1H-pyrrol-1-yl)-6,9,12,15,18-pentaoxo-1-phenyl-3,21,24-trioxa-5,8,11,14,17-pentaazahexacosyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-2-oxoethyl)succinamide

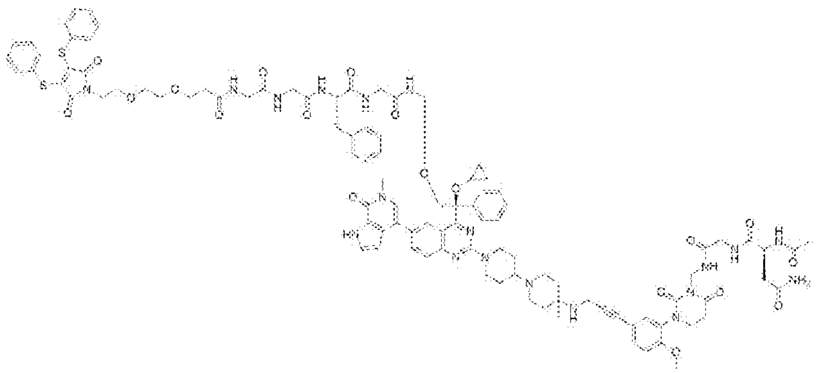

The title compound (4.3 mg) was obtained from the (9H-fluoren-9-yl)methyl ((1S,10S)-1-(2-(4-((3-(3-(3-((2-((S)-2-acetamide-4-amino-4-oxobutanamide)acetamide)methyl)-2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-10-benzyl-1-cyclopropoxy-6,9,12,15-tetraoxo-1-phenyl-3-oxa-5,8,11,14-tetraazahexadecan-16-yl)carbamate of Production Example 244-2 (7.3 mg, 4.3 μmol) and the 2,5-dioxopyrrolidin-1-yl 3-(2-(2-(2,5-dioxo-3,4-bis(phenylthio)-2,5-dihydro-1H-pyrrol-1-yl)ethoxy)ethoxy)propanoate of Production Example 201-23 (7.28 mg, 12.8 μmol), by the same method as Example 235.

ESI-MS (m/z): 1950.86 [M+H]+.

[Production Example 244-1]

(S)-2-Acetamide-N1-(2-(((3-(5-iodo-2-methoxyphenyl)-2,6-dioxotetrahydropyrimidine-1(2H)-yl)methyl)amino)-2-oxoethyl) succinamide

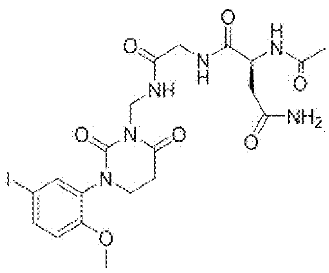

To a solution of the (9H-fluorolen-9-yl)methyl (S)-(4-amino-1-((2-(((3-(5-iodo-2-methoxyphenyl)-2,6-dioxotetrahydropyrimidine-1(2H)-yl)methyl)amino)-2-oxoethyl)amino)-1,4-dioxobutan-2-yl)carbamate of Production Example 229-1 (60 mg, 78 μmol) in DMF (500 μL) there was added diethylamine (81 μL, 0.78 mmol), and the mixture was stirred for 40 minutes at room temperature. The reaction mixture was then concentrated under reduced pressure. To a solution of the residue in DMF (500 μL) there were added triethylamine (32.6 μL, 234 μmol) and acetic anhydride (11.1 μL, 117 μmol), and the mixture was stirred for 1 hour at room temperature. The reaction mixture was purified by direct reversed-phase

silica gel chromatography (0.1% formic acid aqueous solution:0.1% acetonitrile formate solution = 90:10 to 65:35) to obtain the title compound (38 mg).

ESI-MS (m/z): 589.11 $[M+H]^+$.

[Production Example 244-2]

(9H-Fluoren-9-yl)methyl ((1S,10S)-1-(2-(4-((3-(3-(3-((2-((S)-2-acetamide-4-amino-4-oxobutanamide)acetamide) methyl)-2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-10-benzyl-1-cyclopropoxy-6,9,12,15-tetraoxo-1-phenyl-3-oxa-5,8,11,14-tetraazahexadecan-16-yl)carbamate

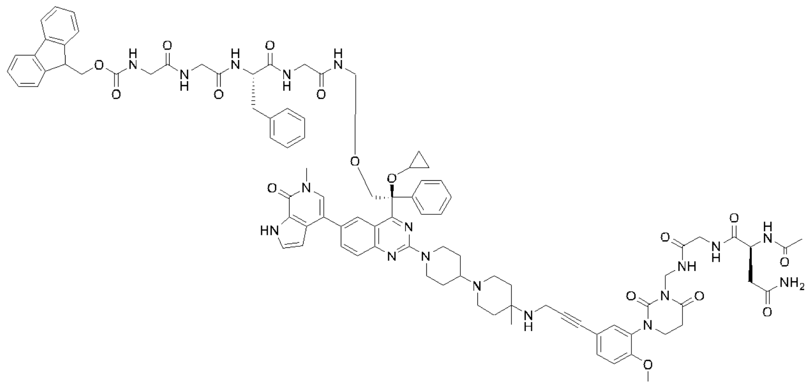

The title compound (7.3 mg) was obtained from the (9H-fluoren-9-yl)methyl ((1S,10S)-10-benzyl-1-cyclopropoxy-1-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c] pyridin-4-yl)quinazolin-4-yl)-6,9,12,15-tetraoxo-1-phenyl-3-oxa-5,8,11,14-tetraazahexadecan-16-yl)carbamate of Production Example 203-1 (8.5 mg, 6.8 μmol) and the (S)-2-acetamide-N1-(2-(((3-(5-iodo-2-methoxyphenyl)-2,6-dioxotetrahydropyrimidine-1(2H)-yl)methyl)amino)-2-oxoethyl) succinamide of Production Example 244-1 (3.98 mg, 6.77 μmol), by the same method as Production Example 218-2.

ESI-MS (m/z): 858.58 $[(M+2H)/2]^+$.

[Example 245]

(2S)-N-{[3-(5-{3-[(1'-{4-[1-Cyclopropoxy-2-({2-[(2S)-2-{2-[(2S)-2-[3-(2-{2-[2,5-dioxo-3,4-bis(phenylsulfanyl)-2,5-dihydro-1H-pyrrol-1-yl]ethoxy}ethoxy)propanamido]-6-(2,5,8,11,14,17,20,23-octaoxahexacosan-26-amide)hexanamido] acetamide}-3-phenylpropanamido]acetamide}methoxy)-1-phenylethyl]-6-{6-methyl-7-oxo-1H,6H,7H-pyrrolo[2,3-c]pyridin-4-yl}quinazolin-2-yl}-4-methyl-[1,4'-bipiperidin]-4-yl)amino]prop-1-yn-1-yl}-2-methoxyphenyl)-2,6-dioxo-1,3-diazinane-1-yl]methyl}-2-acetamidebutanediamide

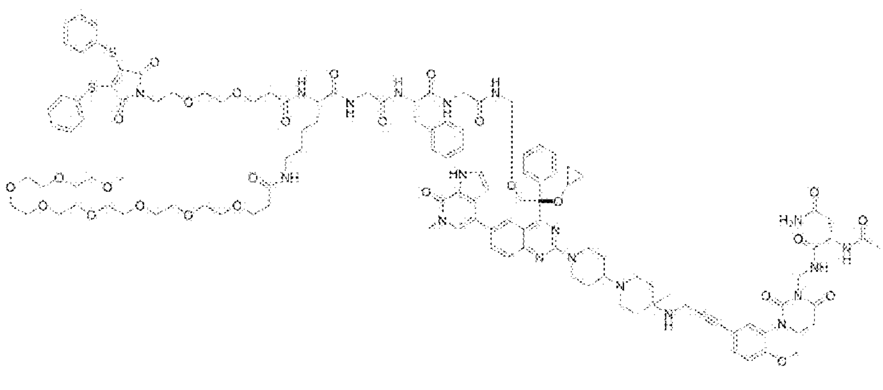

To a solution of the formate of (9H-fluoren-9-yl)methyl N-[(1S)-1-[({[(1S)-1-[({[(2-{2-[4-({3-[3-(3-{[(2S)-3-carbamoyl-2-acetamidepropanamido]methyl}-2,4-dioxo-1,3-diazinane-1-yl)-4-methoxyphenyl]prop-2-yn-1-yl}amino)-4-methyl-[1,4'-bipiperidin]-1'-yl]-6-{6-methyl-7-oxo-1H,6H,7H-pyrrolo[2,3-c]pyridin-4-yl}quinazolin-4-yl}-2-cyclopropoxy-2-phenylethoxy)methyl carbamoyl}methyl)carbamoyl]-2-phenylethyl]carbamoyl}methyl)carbamoyl]-5-(2,5,8,11,14,17,20,23-octaoxahexacosan-26-amide)pentyl]carbamate of Production Example 245-4 (4.0 mg) in DMF (730 μL) there was added N,N-diethylamine (200 μL, 1.9 mmol), and the mixture was stirred for 20 minutes at room temperature. The reaction mixture was concentrated and the residue was processed 3 times by azeotropic distillation with toluene (1.0 mL) to obtain an intermediate. In a separate vessel, TFA (0.36 mL) was added to a solution of the *tert*-butyl 3-(2-(2-(2,5-dioxo-3,4-bis(phenylthio)-2,5-dihydro-1H-pyrrol-1-yl)ethoxy)ethoxy)propionate of Production Example 201-22 (2.0 mg, 3.8 μmol) in dichloromethane (0.36 mL), and the mixture was stirred for 30 minutes at room temperature and then concentrated. After adding toluene (1 mL) to the obtained residue and repeating azeotropic distillation 3 times, DMF (730 μL), N,N-diisopropylethylamine (1.6 μL, 9.4 μmol) and HATU (1.4 mg, 3.8 μmol) were added and the mixture was stirred for 2 minutes at room temperature and combined with a solution of the previously obtained intermediate in DMF (730 μL). The reaction mixture was stirred for 1 hour at room temperature and then purified by UFPLC to obtain a formate of the title compound (1.7 mg).

ESI-MS (m/z): 1179.80[(M+2H)/2]$^+$.

[Production Example 245-1]

*tert-Butyl* 2-[(2S)-6-{[(benzyloxy)carbonyl]amino}-2-({[(9H-fluoren-9-yl)methoxy]carbonyl}amino)hexanamido]acetate

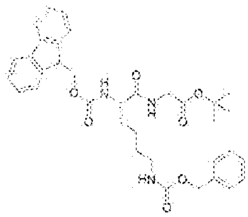

To a solution of (25)-6-{[(benzyloxy)carbonyl]amino}-2-({[(9H-fluoren-9-yl)methoxy]carbonyl}amino)hexanoic acid (1.0 g, 2.0 mmol) in DMF (7.7 mL)) there were added N,N-diisopropylethylamine (1.0 mL, 6.0 mmol), *tert*-butyl 2-aminoacetate hydrochloride (0.50 g, 3.0 mmol) and HATU (1.1 g, 3.0 mmol). The reaction mixture was stirred for 2 hours at room temperature, and then an ammonium chloride aqueous solution was added to halt the reaction. The reaction mixture was extracted with ethyl acetate and the obtained organic layer was dried over anhydrous sodium sulfate and filtered, and then concentrated. The obtained residue was purified by silica gel column chromatography (ethyl acetate:heptane = 0: 1 to 1:0) to obtain the title compound (570 mg).

ESI-MS (m/z): 616.49 [M+H]$^+$.

[Production Example 245-2]

*tert*-Butyl 2-[(2S)-2-({[(9H-fluoren-9-yl)methoxy]carbonyl}amino)-6-(2,5,8,11,14,17,20,23-octaoxahexacosan-26-amide)hexanamido]acetate

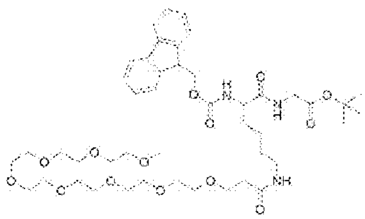

To a solution of the *tert-butyl* 2-[(2S)-6-{[(benzyloxy)carbonyl]amino}-2-({[(9H-fluoren-9-yl)methoxy]carbonyl}amino)hexanamido]acetate of Production Example 245-1 (270 mg, 0.44 mmol) in methanol (5.3 mL) there was added 10% palladium-carbon (50% water, 46.7 mg), and the mixture was stirred for 2 hours at room temperature, ordinary pressure under a hydrogen atmosphere. The reaction mixture was filtered with Celite and concentrated. To a solution of the obtained

residue in DMF (17 mL) there were added N,N-diisopropylethylamine (380 μL, 2.2 mmol) and 2,5-dioxopyrrolidin-1-yl 2,5,8,11,14,17,20,23-octaoxahexacosan-26-oate (290 mg, 0.57 mmol), and the mixture was stirred for 2 hours at room temperature. The reaction mixture was concentrated and the obtained residue was purified by ODS silica gel column chromatography (0.1% acetonitrile formate solution:0.1% formic acid aqueous solution = 3:97 to 70:30) to obtain the title compound (75 mg).

ESI-MS (m/z): 877.00 $[M+H]^+$.

[Production Example 245-3]

(9H-Fluoren-9-yl)methyl N-[(1S)-1-[({[(1S)-1-{[({[2-cyclopropoxy-2-(2-{4-methyl-4-[(prop-2-yn-1-yl)amino]-[1,4'-bipiperidin]-1'-yl}-6-{6-methyl-7-oxo-1H,6H,7H-pyrrolo[2,3-c]pyridin-4-yl}quinazolin-4-yl)-2-phenylethoxy]methyl}carbamoyl) methyl]carbamoyl}-2-phenylethyl]carbamoyl}methyl)carbamoyl]-5-(2,5,8,11,14,17,20,23-octaoxahexacosan-26-amide)pentyl]carbamate

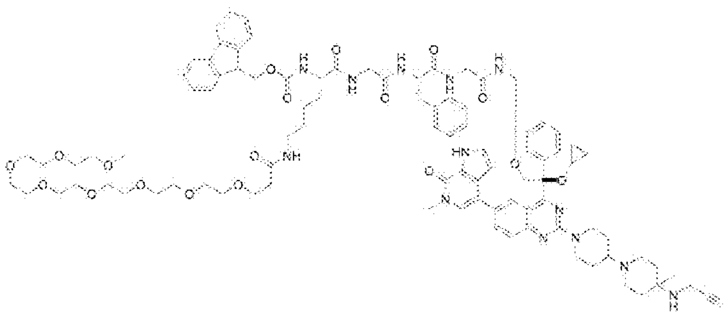

To a solution of the (9H-fluoren-9-yl)methyl (S)-(2-(((2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamate of Production Example 201-11 (50 mg, 0.050 mmol) in DMF (1.2 mL) there was added diethylamine (210 μL, 2.0 mmol), and the mixture was stirred for 20 minutes at room temperature. The reaction mixture was concentrated and processed 3 times by azeotropic distillation with addition of toluene (2 mL). To the obtained residue there was added a solution of (2S)-2-({[(9H-fluoren-9-yl)methoxy]carbonyl}amino)-3-phenylpropanoic acid (25 mg, 0.065 mmol), N,N-diisopropylethylamine (26 μL, 0.15 mmol) and HATU (25 mg, 0.065 mmol) in DMF (1.2 mL), and the mixture was stirred for 1 hour at room temperature. Diethylamine (210 μL, 2.0 mmol) was added to the reaction mixture, which was then stirred for 15 minutes at room temperature and subsequently concentrated. The obtained residue was processed 3 times by azeotropic distillation with toluene (2 mL) to obtain an intermediate. In a separate vessel, TFA (770 μL) was added to a solution of the *tert*-butyl 2-[(2S)-2-({[(9H-fluoren-9-yl)methoxy]carbonyl}amino)-6-(2,5,8,11,14,17,20,23-octaoxahexacosan-26-amide)hexanamido]acetate of Production Example 245-2 (66 mg, 0.075 mmol) in dichloromethane (650 μL). The reaction mixture was stirred for 1.5 hours at room temperature and then concentrated, and the obtained residue was processed 3 times by azeotropic distillation with toluene (1.0 mL). To a solution of the obtained residue in DMF (1.2 mL) there were added N,N-diisopropylethylamine (26 μL, 0.15 mmol) and HATU (25 mg, 0.065 mmol), and the mixture was combined with the previously obtained intermediate. The reaction mixture was stirred for 1 hour at room temperature and then purified by ODS silica gel column chromatography (0.1% acetonitrile formate solution:0.1% formic acid aqueous solution = 0:100 to 50:50) to obtain a formate of the title compound (34 mg).

ESI-MS (m/z): 1720.75 $[M+H]^+$.

[Production Example 245-4]

(9H-Fluoren-9-yl)methyl N-[(1S)-1-[({[(1S)-1-[({[(2-{2-[4-({3-[3-(3-{[(2S)-3-carbamoyl-2-acetamidepropanamido] methyl}-2,4-dioxo-1,3-diazinane-1-yl)-4-methoxyphenyl]prop-2-yn-1-yl}amino)-4-methyl-[1,4'-bipiperidin]-1'-yl]-6-{6-methyl-7-oxo-1H,6H,7H-pyrrolo[2,3-c]pyridin-4-yl}quinazolin-4-yl}-2-cyclopropoxy-2-phenylethoxy)methyl carbamoyl} methyl)carbamoyl]-2-phenylethyl]carbamoyl}methyl)carbamoyl]-5-(2,5,8,11,14,17,20,23-octaoxahexacosan-26-amide)pentyl]carbamate

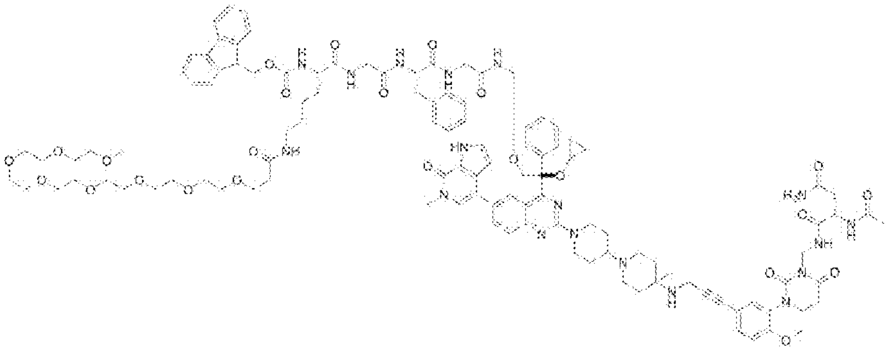

To a solution of the formate of (9H-fluoren-9-yl)methyl N-[(1S)-1-[({[(1S)-1-{[({[2-cyclopropoxy-2-(2-{4-methyl-4-[(prop-2-yn-1-yl)amino]-[1,4'-bipiperidin]-1'-yl}-6-{6-methyl-7-oxo-1H,6H,7H-pyrrolo[2,3-c]pyridin-4-yl}quinazolin-4-yl)-2-phenylethoxy]methyl}carbamoyl)methyl]carbamoyl}-2-phenylethyl]carbamoyl}methyl)carbamoyl]-5-(2,5,8,11,14,17,20,23-octaoxahexacosan-26-amide)pentyl]carbamate of Production Example 245-3 (13 mg), the (S)-2-acetamide-N1-((3-(5-iodo-2-methoxyphenyl)-2,6-dioxotetrahydropyrimidine-1(2H)-yl)methyl) succinamide of Production Example 241-1 (8.0 mg, 0.015 mmol), tetrakis(triphenylphosphine)palladium(0) (3.5 mg, 3.0 μmol) and N,N-diisopropylethylamine (130 μL, 0.76 mmol) in DMF (400 μL) there was added copper(I) iodide (0.29 mg, 1.5 μmol) under a nitrogen atmosphere. The reaction mixture was stirred for 2 hours at 40°C and then concentrated. The obtained residue was purified by ODS silica gel column chromatography (0.1% acetonitrile formate solution:0.1% formic acid aqueous solution = 3:97 to 70:30) to obtain a formate of the title compound (4.0 mg).

ESI-MS (m/z): 1063.12[(M+2H)/2]$^+$.

[Example 246]

(3 S)-3-({[3-(5-{3-[(1'-{4-[1-Cyclopropoxy-2-({2-[(2S)-2-{2-[(2S)-2-[3-(2-{2-[2,5-dioxo-3,4-bis(phenylsulfanyl)-2,5-dihydro-1H-pyrrol-1-yl]ethoxy}ethoxy)propanamido]-6-(2,5,8,11,14,17,20,23-octaoxahexacosan-26-amide)hexanamido] acetamide}-3-phenylpropanamido]acetamide}methoxy)-1-phenylethyl]-6-{6-methyl-7-oxo-1H,6H,7H-pyrrolo[2,3-c]pyridin4-yl}quinazolin-2-yl}-4-methyl-[1,4"-bipiperidin]-4-yl)amino]prop-1-yn-1-yl}-2-methoxyphenyl)-2,6-dioxo-1,3-diazinane-1-yl]methyl}carbamoyl)-3-acetamidepropanoic acid

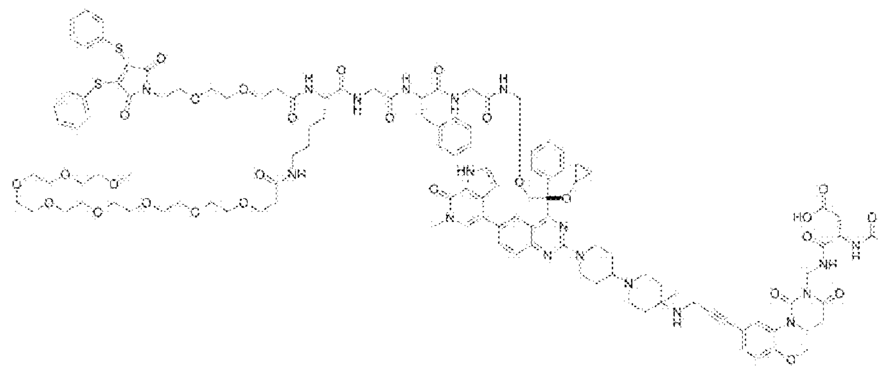

To a solution of the (3S)-3-({[3-(5-{3-[(1'-{ 4-[1-cyc1opropoxy-2-({2-[(2S)-2-{2-[(2S)-2-({[(9H-fluoren-9-yl)methoxy]carbonyl}amino)-6-(2,5,8,11,14,17,20,23-octaoxahexacosan-26-amide)hexanamido]acetamide}-3-phenylpropanamido]acetamide}methoxy)-1-phenylethyl]-6-{6-methyl-7-oxo-1H,6H,7H-pyrrolo[2,3-c]pyridin-4-yl}quinazolin-2-yl}-4-methyl-[1,4'-bipiperidin]-4-yl)amino]prop-1-yn-1-yl}-2-methoxyphenyl)-2,6-dioxo-1,3-diazinane-1-yl]methyl}carbamoyl)-3-acetamidepropanoic acid of Production Example 246-1 (4.0 mg, 1.9 μmol) in N,N-dimethylformamide (440 μL) there was added diethylamine (200 μL, 1.9 mmol), and the mixture was stirred for 20 minutes at room temperature and then concentrated. The obtained residue was processed 3 times by azeotropic distillation with toluene (1.0 mL). In a separate vessel, TFA (0.43 mL) was added to a solution of the *tert*-butyl 3-(2-{2-[2,5-dioxo-3,4-bis(phenylthio)-2,5-dihydro-1H-pyrrol-1-yl]ethoxy }ethoxy)propanoate of Production Example 201-22 (1.5 mg, 2.8 μmol) in dichloromethane (0.36 mL), and the mixture was stirred for 1 hour at room temperature and then concentrated. Toluene (1.0 mL) was added to the obtained residue and azeotropic distillation was carried out 3 times. To a solution of the residue in N,N-dimethylformamide (0.44 mL) there were added N,N-diisopropylethylamine (1.6 μL, 9.4 μmol) and HATU (1.1 mg, 2.8 μmol), and the mixture was stirred for 5 minutes at room temperature and then combined with the previously prepared amine residue. After stirring for 1 hour at room temperature, the mixture was purified by preparative HPLC to obtain the title compound (1.3 mg).

ESI-MS (m/z): 1180.26 [(M+2H)/2]$^+$.

[Production Example 246-1]

(3S)-3-({[3-(5-{3-[(1'-{4-[1-Cyclopropoxy-2-({2-[(2S)-2-{2-[(2S)-2-({[(9H-fluoren-9-yl)methoxy]carbonyl}amino)-6-(2,5,8,11,14,17,20,23-octaoxahexacosan-26-amide)hexanamido]acetamide}-3-phenylpropanamido]acetamide}methoxy)-1-phenylethyl]-6-{6-methyl-7-oxo-1H,6H,7H-pyrrolo[2,3-c]pyridin-4-yl}quinazolin-2-yl}-4-methyl-[1,4'-bipiperidin]-4-yl)amino]prop-1-yn-1-yl}-2-methoxyphenyl)-2,6-dioxo-1,3-diazinane-1-yl]methyl}carbamoyl)-3-acetamidepropanoic acid

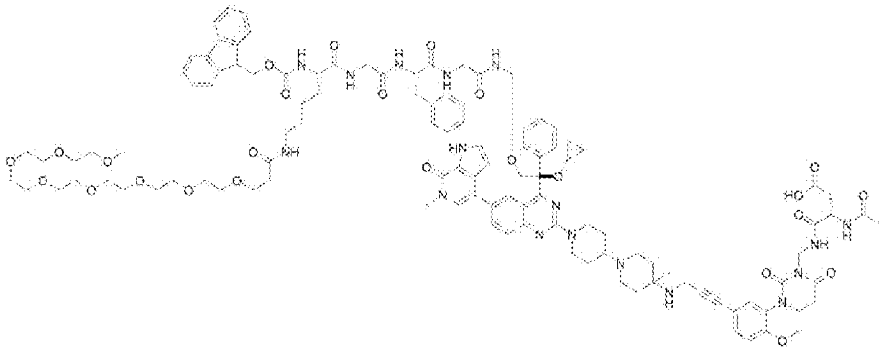

To a solution of the (9H-fluoren-9-yl)methylN-[(1S)-1-[({[(1S)-1-{[({[2-cyclopropoxy-2-(2-{4-methyl-4-[(prop-2-yn-1-yl)amino]-[1,4'-bipiperidin]-1'-yl}-6-{6-methyl-7-oxo-1H,6H,7H-pyrrolo[2,3-c]pyridin-4-yl}quinazolin-4-yl)-2-phenylethoxy]methyl}carbamoyl)methyl]carbamoyl}-2-phenylethyl]carbamoyl}methyl)carbamoyl]-5-(2,5,8,11,14,17,20,23-octaoxahexacosan-26-amide)pentyl]carbamate of Production Example 245-3 (14 mg, 8.1 μmol), the (S)-3-acetamide-4-(((3-(5-iodo-2-methoxyphenyl)-2,6-dioxotetrahydropyrimidine-1(2H)-yl)methyl)amino)-4-oxobutanoic acid of Production Example 201-19 (8.7 mg, 0.016 mmol), tetrakistriphenylphosphinepalladium (3.8 mg, 3.3 μmol) and N,N-diisopropylethylamine (150 μL, 0.81 mmol) in N,N-dimethylformamide (430 μL) there was added copper(I) iodide (0.31 mg, 1.6 μmol) under a nitrogen atmosphere. After heating to 40° and stirring for 2 hours, the reaction mixture was concentrated and the obtained residue was purified by ODS silica gel column chromatography (0.1% acetonitrile formate solution:0.1% formic acid aqueous solution = 3:97 to 70:30) to obtain a formate of the title compound (4.0 mg). ESI-MS (m/z): 1065.10[(M+2H)/2]$^+$.

[Example 247]

(3S)-3-({[3-(5-{3-[(1'-{4-[1-Cyc1opropoxy-2-({2-[(2S)-2-(2-{2-[3-(2-{2-[2,5-dioxo-3,4-bis(phenylsulfanyl)-2,5-dihydro-1H-pyrrol-1-yl]ethoxy}ethoxy)propanamido]acetamide}acetamide)-3-phenylpropanamido]acetamide}methoxy)-1-phenylethyl]-6-{6-methyl-7-oxo-1H,6H,7H-pyrrolo[2,3-c]pyridin-4-yl}quinazolin-2-yl}-4-methyl-[1,4'-bipiperidin]-4-yl)amino]prop-1-yn-1-yl}-2-methoxyphenyl)-2,6-dioxo-1,3-diazinane-1-yl]methyl}carbamoyl)-3-(2,5,8,11,14,17,20,23,26,29,32,35-dodecaoxaoctatriacontan-38-amide)propanoic acid

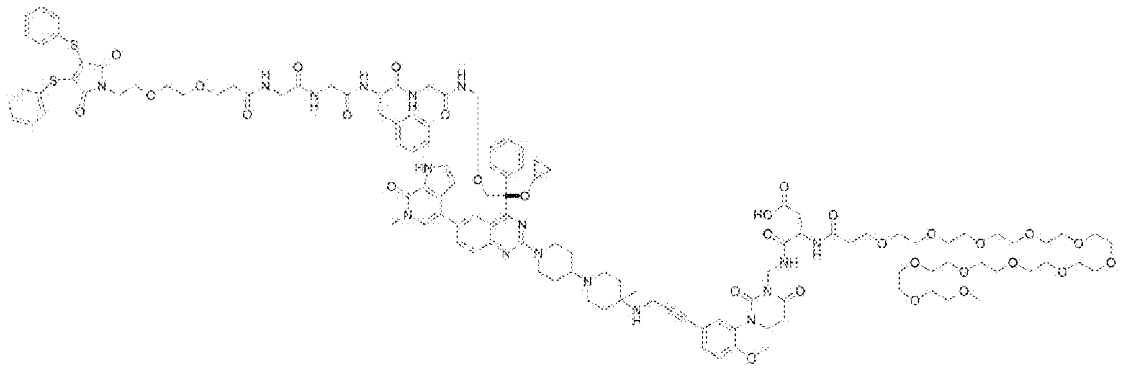

To a solution of the (3S)-3-({[3-(5-{3-[(1'-{4-[1-cyclopropoxy-2-({2-[(2S)-2-{2-[2-({[(9H-fluoren-9-yl)methoxy]carbonyl}amino)acetamide]acetamide}-3-phenylpropanamido]acetamide}methoxy)-l-phenylethyl]-6-{6-methyl-7-oxo-1H,6H,7H-pyrrolo[2,3-c]pyridin4-yl}quinazolin-2-yl}-4-methyl-[1,4"-bipiperidin]-4-yl)amino]prop-1-yn-1-yl }-2-methoxyphenyl)-2,6-dioxo-1,3-diazinane-1-yl]methyl }carbamoyl)-3-(2,5,8,11,14,17,20,23,26,29,32,35-dodecaoxaoctatriacontan-38-amide)propanoic acid of Production Example 247-3 (10 mg, 4.6 μmol) in N,N-dimethylformamide (350 μL) there was added diethylamine (95 μL, 0.91 mmol), and the mixture was stirred for 20 minutes at room temperature and then concentrated. The obtained residue was processed 3 times by azeotropic distillation with toluene (1.0 mL) and the residue was dissolved in N,N-dimethylformamide (350 μL, 4.6 mmol). After adding N,N-diisopropylethylamine (16 μL, 0.091 mmol) and the 2,5-dioxopyrrolidin-1-yl 3-(2-(2-(2,5-dioxo-3,4-bis(phenylthio)-2,5-dihydro-1H-pyrrol-1-yl)ethoxy)ethoxy)propanoate of Production Example 201-23 (3.4 mg, 6.0 μmol), the mixture was stirred for 2 hours at room temperature and then purified by preparative HPLC to obtain a formate of the title compound (0.76 mg).

ESI-MS (m/z): 1212.33 [(M+2H)/2]$^+$.

[Production Example 247-1]

(9H-Fluoren-9-yl)methyl N-{[({[(1S)-1-{[({[2-cyclopropoxy-2-(2-{4-methyl-4-[(prop-2-yn-1-yl)amino]-[1,4'-bipiperidin]-1'-yl}-6- f 6-methyl-7-oxo-1H,6H,7H-pyrrolo[2,3-c]pyridin-4-yl}quinazolin-4-yl)-2-phenylethoxy]methyl}carbamoyl)methyl]carbamoyl}-2-phenylethyl]carbamoyl}methyl)carbamoyl]methyl}carbamate

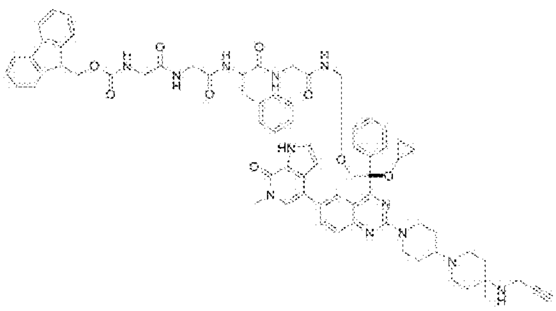

To a solution of the (9H-fluoren-9-yl)methyl(S)-(2-(((2-cyclopropoxy-2-(2-(4-methyl-4-(prop-2-yn-1-ylamino)-[1,4'-bipiperidine]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamate of Production Example 201-11 (20 mg, 0.02 mmol) in DMF (0.47 mL) there was added diethylamine (210 μL, 2.0 mmol). After stirring for 20 minutes at room temperature, the reaction mixture was concentrated and processed 3 times by azeotropic distillation with toluene (3.0 mL). The obtained residue was dissolved in N,N-dimethylformamide (470 μL), and then (2S) -2-{2-[2-({[(9H-fluoren-9-yl)methoxy]carbonyl}amino)acetamide]acetamide}-3-phenylpropanoic acid (15 mg, 0.03 mmol), N,N-diisopropylethylamine (70 μL, 0.40 mmol) and HATU (15 mg, 0.040 mmol) were added. After stirring for 1 hour at room temperature, the reaction mixture was concentrated and the obtained residue was purified by ODS silica gel column chromatography (0.1% acetonitrile formate solution:0.1% formic acid aqueous solution = 2:98 to 70:30) to obtain a formate of the title compound (18 mg).

ESI-MS (m/z): 1255.62 [M+H]$^+$.

[Production Example 247-2]

(S)-40-(((3-(5-Iodo-2-methoxyphenyl)-2,6-dioxotetrahydropyrimidine-1(2H)-yl)methyl)carbamoyl)-38-oxo-2,5,8,11,14,17,20,23,26,29,32,35-dodecaoxa-39-azadotetracontan-42-oic acid

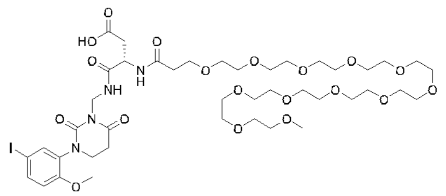

Diethylamine (340 μL, 3.3 mmol) was added to a solution of the *tert*-butyl(S)-3((((9H-fluoren-9-yl)methoxy) carbonyl)amino)-4-(((3-(5-iodo-2-methoxyphenyl)-2,6-dioxotetrahydropyrimidine-1(2H)-yl)methyl)amino)-4-oxobutanoate of Production Example 201-18 (100 mg, 0.13 mmol) in N,N-dimethylformamide (1.0 mL). After stirring for 30 minutes at room temperature, the reaction mixture was concentrated and the obtained residue was processed 3 times by azeotropic distillation with toluene (1.0 mL). The obtained residue was dissolved in DMF (1.0 mL), and then N,N-diisopropylethylamine (220 μL, 1.3 mL) and 2,5-dioxopyrrolidin-1-yl 2,5,8,11,14,17,20,23,26,29,32,35-dodecaoxaoctatriacontan-38-oate (120 mg, 0.17 mmol) were added and the mixture was stirred for 1 hour at room temperature. Diethylamine (340 μL, 3.3 mmol) was added to the reaction mixture to halt the reaction, and the mixture was concentrated under reduced pressure and processed 3 times by azeotropic distillation with toluene (1.0 mL). The obtained residue was dissolved in dichloromethane (1.3 mL), TFA (0.70 mL) was added and the mixture was stirred for 1 hour at room temperature and then concentrated, after which the obtained residue was purified by ODS silica gel column chromatography (0.1% acetonitrile formate solution:0.1% formic acid aqueous solution = 2:98 to 100:0) to obtain the title compound (100 mg).
ESI-MS (m/z): 1061.38 $[M+H]^+$.

[Production Example 247-3]

(3S)-3-({[3-(5-{3-[(1'-{4-[1-Cyclopropoxy-2-({2-[(2S)-2-{2-[2-({[(9H-fluoren-9-yl)methoxycarbonyl}amino)acetamide] acetamide}-3-phenylpropanamido]acetamide}methoxy)-1-phenylethyl]-6-{6-methyl-7-oxo-1H,6H,7H-pyrrolo[2,3-c]pyridin4-yl}quinazolin-2-yl}-4-methyl-[1,4'-bipiperidin]-4-yl)amino]prop-1-yn-1-yl}-2-methoxyphenyl)-2,6-dioxo-1,3-diazinane-1-yl]methyl}carbamoyl)-3-(2,5,8,11,14,17,20,23,26,29,32,35-dodecaoxaoctatriacontan-38-amide)propanoic acid

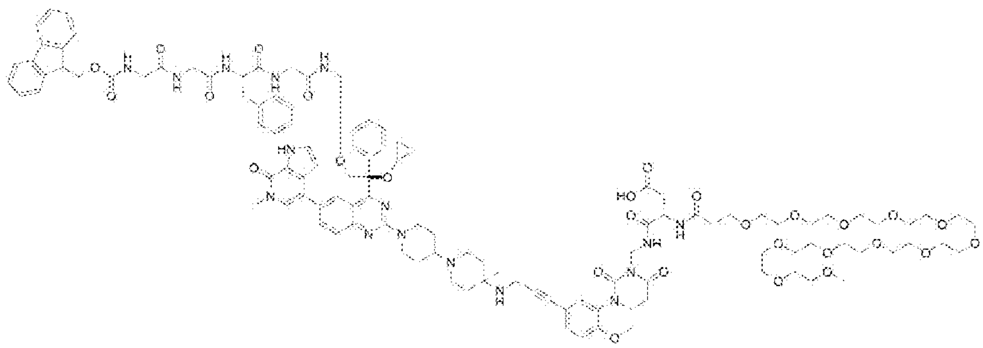

To a solution of the (9H-fluoren-9-yl)methylN-{[({[(1S)-1-{[({[2-cyclopropoxy-2-(2-{4-methyl-4-[(prop-2-yn-1-yl) amino]-[1,4'-bipiperidin]-1'-yl}-6-{6-methyl-7-oxo-1H,6H,7H-pyrrolo[2,3-c]pyridin-4-yl}quinazolin-4-yl)-2-phenylethoxy] methyl}carbamoyl)methyl]carbamoyl}-2-phenylethyl]carbamoyl}methyl)carbamoyl]methyl}carbamate of Production Example 247-1 (15 mg, 0.012 mmol) and the (S)-40-(((3-(5-iodo-2-methoxyphenyl)-2,6-dioxotetrahydropyrimidine-1(2H)-yl)methyl)carbamoyl)-3 8-oxo-2,5,8,11,14,17,20,23,26,29,32,35-dodecaoxa-39-azadotetracontan-42-oic acid of Production Example 247-2 (19.0 mg, 0.018 mmol) in DMF (720 μL) there were added N,N-diisopropylethylamine (210 μL, 1.20 mmol), copper(I) iodide (0.91 mg, 4.80 μmol) and tetrakistriphenylphosphinepalladium(0) (5.5 mg, 4.8 μmol), and the mixture was stirred for 2 hours at 55°C under a nitrogen atmosphere. The reaction mixture was purified by ODS silica gel column chromatography (0.1% acetonitrile formate solution:0.1% formic acid aqueous solution = 2:98 to 70:30), to obtain a formate of the title compound (10 mg). ESI-MS (m/z): 1095.16 $[(M+2H)/2]^+$.

[Example 248]

(S)-3-Acetamide-4-((2-(((3-(5-(3-((1'-(4-((1S,10S)-10-benzyl-1-cyclopropoxy-26-(2,5-dioxo-3,4-bis(phenylthio)-2,5-dihydro-1H-pyrrol-1-yl)-6,9,12,15,18-pentaoxo-1-phenyl-3,21,24-trioxa-5,8,11,14,17-pentaazahexacosyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-2-oxoethyl)amino)-4-oxobutanoic acid

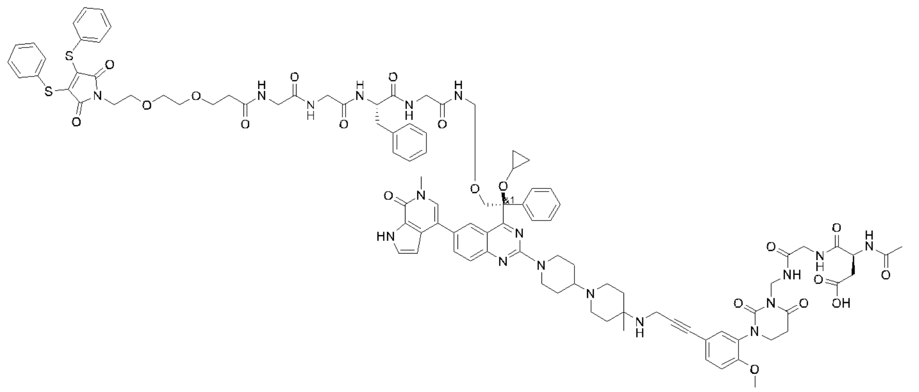

To a solution of the tert-butyl 3-(2-(2-(2,5-dioxo-3,4-bis(phenylthio)-2,5-dihydro-1H-pyrrol-1-yl)ethoxy)ethoxy) propionate of Production Example 201-22 (2.08 mg, 3.91 μmol) in dichloromethane (500 μL) there was added TFA (100 μL), and the mixture was stirred for 30 minutes at room temperature. The reaction mixture was concentrated under reduced pressure to obtain intermediate 1. To a solution of the (S)-3-acetamide-4-((2-(((3-(5-(3-((1'-(4-((11S,20S)-11-benzyl-20-cyclopropoxy-1-(9H-fluoren-9-yl)-3,6,9,12,15-pentaoxo-20-phenyl-2,18-dioxa-4,7,10,13,16-pentaazaeicosan-20-yl)-6-(6-methyl-7-oxo-6,7-dihydro-1H-pyrrolo[2,3-c]pyridin-4-yl)quinazolin-2-yl)-4-methyl-[1,4'-bipiperidin]-4-yl) amino)prop-1-yn-1-yl)-2-methoxyphenyl)-2,6-dioxotetrahydropyrimidine-1(2H)-yl)methyl)amino)-2-oxoethyl)amino)-4-oxobutanoic acid of Production Example 248-3 (4.5 mg, 2.6 μmol) in DMF (500 μL) there was added diethylamine (100 μL), and the mixture was stirred for 15 minutes at room temperature. The reaction mixture was concentrated under reduced pressure, and then a solution of N,N-diisopropylethylamine (9.2 μL, 0.052 mmol), HATU (1.5 mg, 3.9 μmol) and intermediate 1 in DMF (500 μL) was added to the residue at room temperature and the mixture was stirred for 1 hour. The reaction mixture was purified by preparative HPLC (0.1% formic acid aqueous solution:0.1% acetonitrile formate solution = 70:30 to 40:60), to obtain a formate of the title compound (1.6 mg).

ESI-MS (m/z): 1950.52 $[M+H]^+$.

[Production Example 248-1]

*tert*-Butyl (S)-3-((((9H-fluoren-9-yl)methoxy)carbonyl)amino)-4-((2-(((3-(5-iodo-2-methoxyphenyl)-22,6-dioxotetrahydropyrimidine-1(2H)-yl)methyl)amino)-2-oxoethyl)amino)-4-oxobutanoate

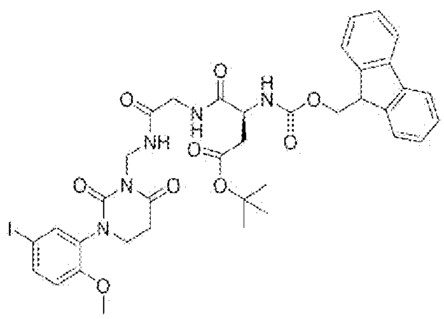

The title compound (25.5 mg) was obtained from the (9H-fluoren-9-yl)methyl (2-(((3-(5-iodo-2-methoxyphenyl)-2,6-dioxotetrahydropyrimidine-1(2H)-yl)methyl)amino)-2-oxoethyl)carbamate of Production Example 206-1 (50 mg,

0.076 mmol) and (S)-2-((((9H-fluoren-9-yl)methoxy)carbonyl)amino)-4-(*tert*-butoxy)-4-oxobutanoic acid (34.6 mg, 0.0841 mmol), by the same method as Production Example 235-1.

ESI-MS (m/z): 826.11 $[M+H]^+$.

[Production Example 248-2]

(S)-3-Acetamide-4-((2-(((3-(5-iodo-2-methoxyphenyl)-2,6-dioxotetrahydropyrimidine-1(2H)-yl)methyl)amino)-2-oxoethyl)amino)-4-oxobutanoic acid

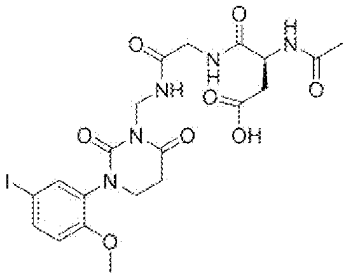

The title compound (11.7 mg) was obtained from the (S)-3-((((9H-fluoren-9-yl)methoxy)carbonyl)amino)-4-((2-(((3-(5-iodo-2-methoxyphenyl)-2,6-dioxotetrahydropyrimidine-1(2H)-yl)methyl)amino)-2-oxoethyl)amino)-4-oxobutanoic acid of Production Example 248-1 (25.5 mg, 0.0309 mmol), by the same method as Production Example 201-19.

ESI-MS (m/z):590.22 $[M+H]^+$.

[Production Example 248-3]

(S)-3-Acetamide-4-((2-(((3-(5-(3-((1'-(4-((11S,20S)-11-benzyl-20-cyclopropoxy-1-(9H-fluoren-9-yl)-3,6,9,12,15-pentaoxo-20-phenyl-2,18-dioxa-4,7,10,13,16-pentaazaeicosan-20-yl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-2-oxoethyl)amino)-4-oxobutanoic acid

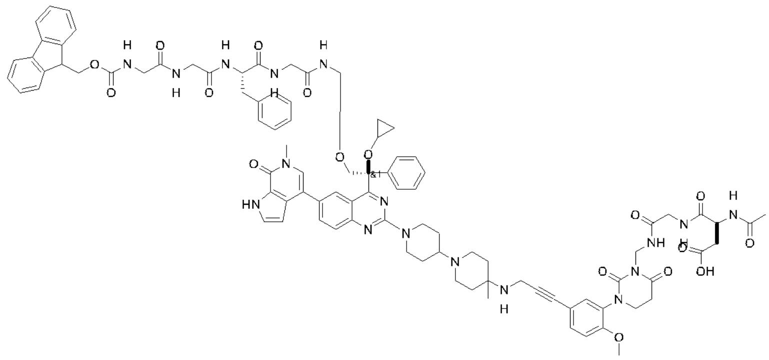

The title compound (4.5 mg) was obtained from the (S)-3-acetamide-4-((2-(((3-(5-iodo-2-methoxyphenyl)-2,6-dioxotetrahydropyrimidine-1(2H)-yl)methyl)amino)-2-oxoethyl)amino)-4-oxobutanoic acid of Production Example 248-2 (6.3 mg, 11 μmol) and the (9H-fluoren-9-yl)methyl((1S,10S)-10-benzyl-1-cyclopropoxy-1-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-6,9,12,15-tetraoxo-1-phenyl-3-oxa-5,8,11,14-tetraazahexadecan-16-yl)carbamate of Production Example 203-1 (9.0 mg, 7.2 μmol), by the same method as Production Example 218-2.

ESI-MS (m/z): 859.82$[(M+2H)/2]^+$.

[Example 249]

(S)-4-(((3-(5-(3-((1'-(4-((1S,10S)-10-(2-Amino-2-oxoethyl)-1-cyclopropoxy-6,9,12-trioxo-1-phenyl-3-oxa-5,8,11-triazatridecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-3-(3-(2-(2-(2,5-dioxo-3,4-bis(phenylthio)-2,5-dihydro-1H-pyrrol-1-yl)ethoxyethoxy)propanamido)-4-oxobutanoic acid

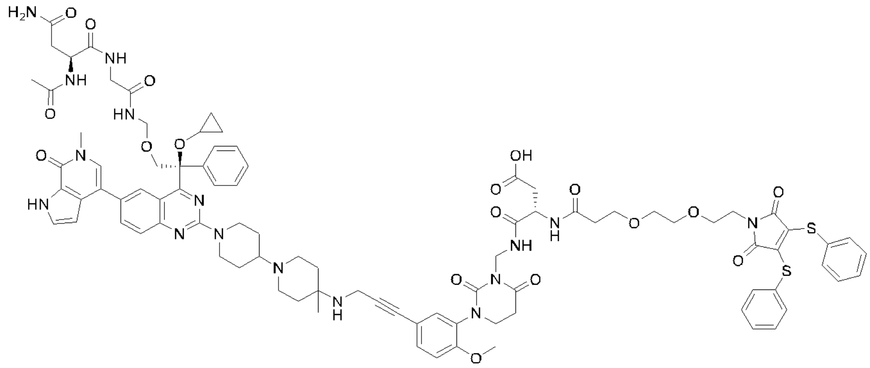

A formate of the title compound (1 mg) was obtained from the formate of (S)-3-((((9H-fluoren-9-yl)methoxy)carbonyl)amino)-4-(((3-(5-(3-((1'-(4-((1S,10S))-10-(2-amino-2-oxoethyl)-1-cyclopropoxy6,9,12-trioxo-1-phenyl-3-oxa-5,8,11-triazatridecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid of Production Example 249-2 (5 mg), by the same method as Example 218.
ESI-MS (m/z): 1746.82 $[M+H]^+$.

[Production Example 249-1]

(S)-2-Acetamide-N1-(2-((((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)succinamide

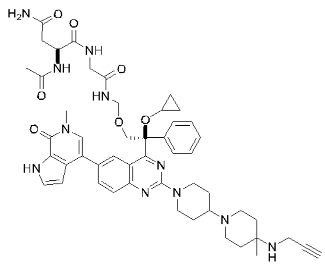

To a solution of the (9H-fluoren-9-yl)methyl (S)-(2-(((2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamate of Production Example 201-11 (28 mg, 0.028 mmol) in DMF (2 mL) there was added diethylamine (200 μL), and the mixture was stirred for 20 minutes at room temperature and then concentrated under reduced pressure to obtain intermediate 1. To a solution of *tert*-butyl acetyl-L-asparaginate (8.43 mg, 0.037 mmol) in dichloromethane (1 mL) there was added TFA (0.5 mL), and the mixture was stirred for 1 hour and then concentrated under reduced pressure to obtain intermediate 2. To a solution of intermediate 1 and intermediate 2 in DMF (2 mL) there were added HATU (13.9 mg, 0.037 mmol) and N,N-diisopropylethylamine (100 μL, 0.573 mmol), and the mixture was stirred for 1 hour at room temperature. The reaction mixture was purified by reversed-phase silica gel column chromatography (0.1% formic acid aqueous solution:0.1% acetonitrile formate solution = 95:5 to 0:1) to obtain a formate of the title compound (8.8 mg).
ESI-MS (m/z): 928.66 $[M+H]^+$.

[Production Example 249-2]

(S)-3-((((9H-Fluoren-9-yl)methoxy)carbonyl)amino)-4-(((3-(5-(3-((1'-(4-((1S,10S)-10-(2-amino-2-oxoethyl)-1-cyclopropoxy-6,9,12-trioxo-1-phenyl-3-oxa-5,8,11-triazatridecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid

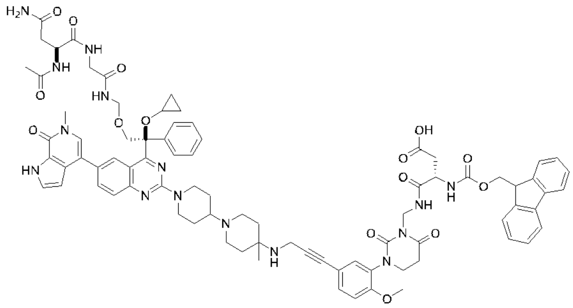

A formate of the title compound (5 mg) was obtained from the formate of (S)-2-acetamide-N1-(2-((((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl) succinamide of Production Example 249-1 (8.8 mg) and the (S)-3-((((9H-fluoren-9-yl)methoxy)carbonyl)amino)-4-(((3-(5-iodo-2-methoxyphenyl)-2,6-dioxotetrahydropyrimidine-1(2H)-yl)methyl)amino)-4-oxobutanoic acid of Production Example 221-1 (8.11 mg, 0.011 mmol), by the same method as Production Example 218-2.

ESI-MS (m/z): 1513.20 $[M+H]^+$.

[Example 250]

(S)-N1-((3-(5-(3-((1'-(4-((1S,10S)-10-Benzyl-1-cyclopropoxy-26-(2,5-dioxo-3,4-bis(phenylthio)-2,5-dihydro-1H-pyrrol-1-yl)-6,9,12,15,18-pentaoxo-1-phenyl-3,21,24-trioxa-5,8,11,14,17-pentaazahexacosyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)-2-(2,5,8,11,14,17,20,23-octaoxahexacosan-26-amide) succinamide

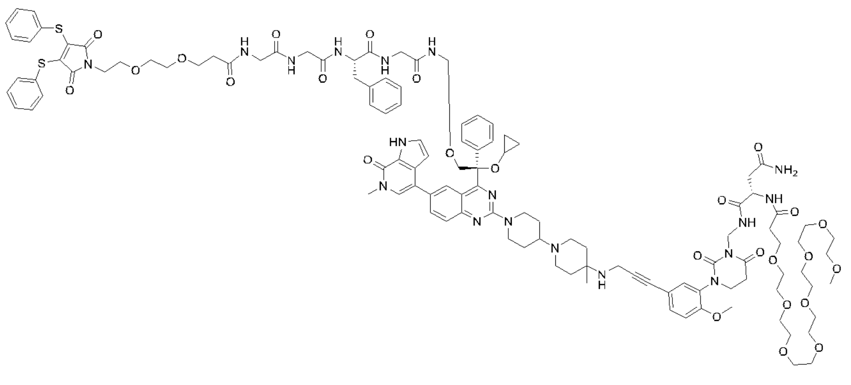

A formate of the title compound (3.4 mg) was obtained from the formate of (S)-N1-((3-(5-(3-((1'-(4-((1S,10S)-16-amino-10-benzyl-1-cyclopropoxy-6,9,12,15-tetraoxo-1-phenyl-3-oxa-5,8,11,14-tetraazahexadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)-2-(2,5,8,11,14,17,20,23-octaoxahexacosan-26-amide) succinamide of Production Example 250-2 (5.0 mg) and the *tert-butyl* 3-(2-(2-(2,5-dioxo-3,4-bis(phenylthio)-2,5-dihydro-1H-pyrrol-1-yl)ethoxy)ethoxy)propanoate of Production Example 201-22 (2.7 mg, 0.0052 mmol), by the same method as Example 205.

ESI-MS (m/z): 1123.13 $[(M+2H)/2]^+$.

[Production Example 250-1]

(S)-2-(2,5,8,11,14,17,20,23-Octaoxahexacosan-26-amide)-N1-((3-(5-iodo-2-methoxyphenyl)-2,6-dioxotetrahydropyrimidine-1(2H)-yl)methyl) succinamide

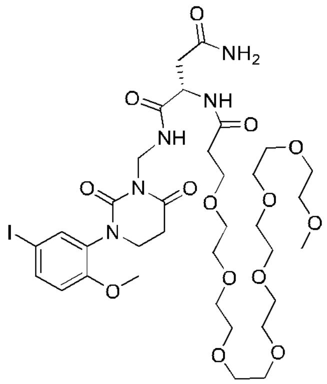

To a solution of the (9H-fluoren-9-yl)methyl (S)-(4-amino-1-(((3-(5-iodo-2-methoxyphenyl)-2,6-dioxotetrahydropyrimidine-1(2H)-yl)methyl)amino)-1,4-dioxobutan-2-yl)carbamate of Production Example 218-1 (30 mg, 0.042 mmol) in DMF (0.22 mL) there was added diethylamine (44 μL, 0.42 mmol), and the mixture was stirred for 30 minutes at room temperature. After concentration under reduced pressure, DMF (0.22 mL), N,N-diisopropylethylamine (18 μL, 0.10 mmol) and 2,5-dioxopyrrolidin-1-yl 2,5,8,11,14,17,20,23-octaoxahexacosan-26-oate (26 mg, 0.051 mmol) were added to the crude product, and the mixture was stirred for 1 hour at room temperature. The reaction mixture was purified by direct reversed-phase silica gel chromatography (0.1% formic acid aqueous solution:0.1% acetonitrile formate solution = 90:10 to 50:50) to obtain the title compound (18 mg).

ESI-MS (m/z): 884.32 $[M+H]^+$.

[Production Example 250-2]

(S)-N1-((3-(5-(3-((1'-(4-((1S,10S)-16-Amino-10-benzyl-1-cyclopropoxy-6,9,12,15-tetraoxo-1-phenyl-3-oxa-5,8,11,14-tetraazahexadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl) methyl)-2-(2,5,8,11,14,17,20,23-octaoxahexacosan-26-amide) succinamide

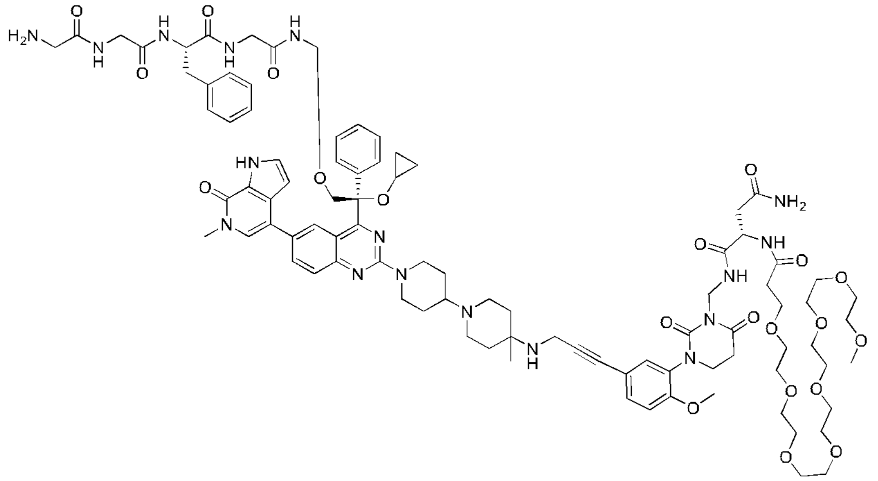

A formate of the title compound (5.0 mg) was obtained from the formate of (9H-fluoren-9-yl)methyl((1S,10S)-10-benzyl-1-cyclopropoxy-1-(2-(4-methyl-4-(prop-2-yn-1-ylamino)-[1,4'-bipiperidine]-1'-yl)-6-(6-methyl-7-oxo-6,7-dihydro-1H-pyrrolo[2,3-c]pyridin-4-yl)quinazolin-4-yl)-6,9,12,15-tetraoxo-1-phenyl-3-oxa-5,8,11,14-tetraazahexadecan-16-yl)carbamate of Production Example 203-1 (8.0 mg) and the (S)-2-(2,5,8,11,14,17,20,23-octaoxahexacosan-26-amide)-N1-((3-(5-iodo-2-methoxyphenyl)-2,6-dioxotetrahydropyrimidine-1(2H)-yl)methyl) succinamide of Production Example 250-1 (10.5 mg, 0.012 mmol), by the same method as Production Example 204-5.

ESI-MS (m/z): 895.43 [(M+2H)/2]+.

[Example 251]

(S)-28-(((3-(5-(3-((1'-(4-((1S,10S)-10-Benzyl-1-cyclopropoxy-26-(2,5-dioxo-3,4-bis(phenylthio)-2,5-dihydro-1H-pyrrol-1-yl)-6,9,12,15,18-pentaoxo-1-phenyl-3,21,24-trioxa-5,8,11,14,17-pentaazahexacosyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)-26-oxo-2,5,8,11,14,17,20,23-octaoxa-27-azatriacontan-30-oic acid

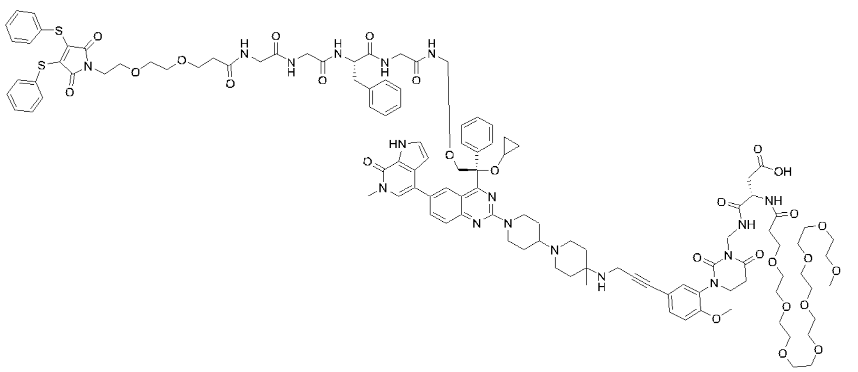

A formate of the title compound (3.4 mg) was obtained from the formate of (S)-28-(((3-(5-(3-((1'-(4-((1S,10S)-16-amino-10-benzyl-1-cyclopropoxy-6,9,12,15-tetraoxo-1-phenyl-3-oxa-5,8,11,14-tetraazahexadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)-26-oxo-2,5,8,11,14,17,20,23-octaoxa-27-azatriacontan-30-oic acid of Production Example 251-2 (3.3 mg) and the tert-butyl 3-(2-(2-(2,5-dioxo-3,4-bis(phenylthio)-2,5-dihydro-1H-pyrrol-1-yl)ethoxy)ethoxy)propanoate of Production Example 201-22 (2.7 mg, 5.2 μmol), by the same method as Example 205.

ESI-MS (m/z): 1123.62 [(M+2H)/2]+.

[Production Example 251-1]

(S)-28-(((3-(5-Iodo-2-methoxyphenyl)-2,6-dioxotetrahydropyrimidine-1(2H)-yl)methyl)carbamoyl)-26-oxo-2,5,8,11,14,17,20,23-octaoxa-27-azatriacontan-30-oic acid

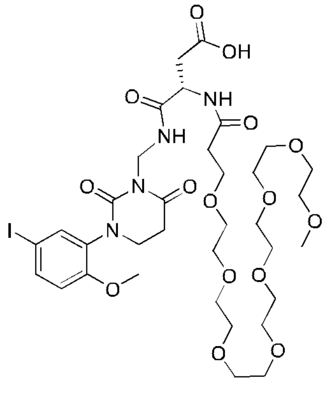

To a solution of the tert-butyl(S)-3((((9H-fluoren-9-yl)methoxy)carbonyl)amino)-4-(((3-(5-iodo-2-methoxyphenyl)-2,6-dioxotetrahydropyrimidine-1(2H)-yl)methyl)amino)-4-oxobutanoate of Production Example 201-18 (75 mg, 0.098 mmol) in DMF (0.50 mL) there was added diethylamine (0.10 mL, 0.98 mmol), and the mixture was stirred for 30 minutes at room temperature. After concentration under reduced pressure, the crude product was divided in half, and then DMF (0.25 mL), N,N-diisopropylethylamine (21 μL, 0.12 mmol) and 2,5-dioxopyrrolidin-1-yl 2,5,8,11,14,17,20,23-octaoxahexacosan-26-oate (30 mg, 0.059 mmol) were added and the mixture was stirred for 2 hours at room temperature. The reaction mixture was concentrated under reduced pressure, and then dichloromethane (0.80 mL) and TFA (0.20 mL) were added and the mixture was stirred for 1 hour at room temperature. The reaction mixture was concentrated under reduced pressure and then purified by direct reversed-phase silica gel chromatography (0.1% formic acid aqueous solution:0.1% acetonitrile formate solution = 9:1 to 1:1) to obtain the title compound (30 mg).
ESI-MS (m/z): 885.31 $[M+H]^+$.

[Production Example 251-2]

(S)-28-(((3-(5-(3-((1'-(4-((1S,10S)-16-Amino-10-benzyl-1-cyclopropoxy-6,9,12,15-tetraoxo-1-phenyl-3-oxa-5,8,11,14-tetraazahexadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)-26-oxo-2,5,8,11,14,17,20,23-octaoxa-27-azatriacontan-30-oic acid

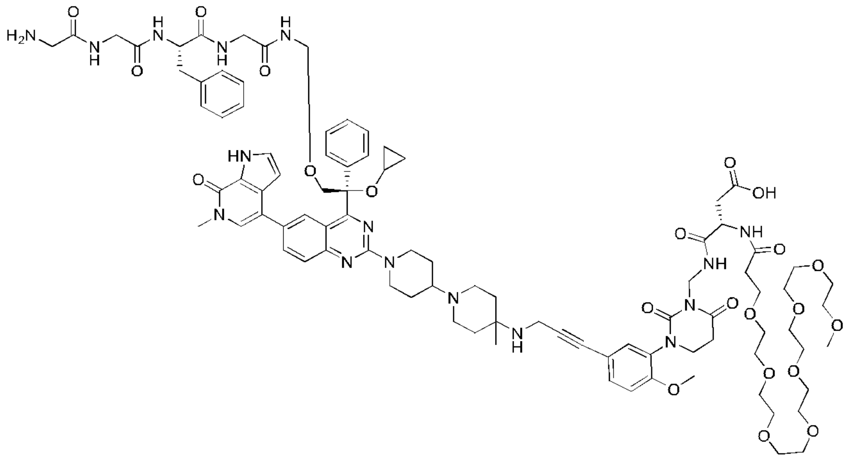

A formate of the title compound (3.3 mg) was obtained from the formate of (9H-fluoren-9-yl)methyl((1S,10S)-10-benzyl-1-cyclopropoxy-1-(2-(4-methyl-4-(prop-2-yn-1-ylamino)-[1,4'-bipiperidine]-1'-yl)-6-(6-methyl-7-oxo-6,7-dihydro-1H-pyrrolo[2,3-c]pyridin-4-yl)quinazolin-4-yl)-6,9,12,15-tetraoxo-1-phenyl-3-oxa-5,8,11,14-tetraazahexadecan-16-yl)carbamate of Production Example 203-1 (8.0 mg) and the (S)-28-(((3-(5-iodo-2-methoxyphenyl)-2,6-dioxotetrahydropyrimidine-1(2H)-yl)methyl)catbamoyl)-26-oxo-2,5,8,11,14,17,20,23-octaoxa-27-azatriacontan-30-oic acid of Production Example 251-1 (10.5 mg, 0.012 mmol), by the same method as Production Example 204-5.
ESI-MS (m/z): 895.80 $[(M+2H)/2]^+$.

[Example 252]

(S)-N1-((3-(5-(3-((1'-(4-((1S,10S)-10-Benzyl-1-cyclopropoxy-26-(2,5-dioxo-3,4-bis(phenylthio)-2,5-dihydro-1H-pyrrol-1-yl)-6,9,12,15,18-pentaoxo-1-phenyl-3,21,24-trioxa-5,8,11,14,17-pentaazahexacosyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)-2-(2,5,8,11,14,17,20,23,26,29,32,35-dodecaoxaoctatriacontan-38-amide) succinamide

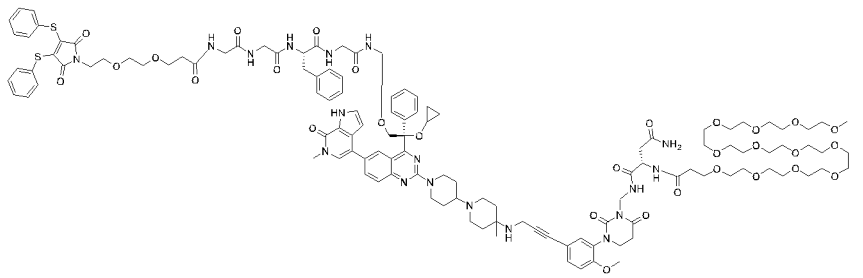

A formate of the title compound (3.0 mg) was obtained from the formate of (S)-N1-((3-(5-(3-((1'-(4-((1S,10S)-16-amino-10-benzyl-1-cyclopropoxy-6,9,12,15-tetraoxo-1-phenyl-3-oxa-5,8,11,14-tetraazahexadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)-2-(2,5,8,11,14,17,20,23,26,29,32,35-dodecaoxaoctatriacontan-38-amide) succinamide of Production Example 252-2 (4.2 mg) and the tert-butyl 3-(2-(2-(2,5-dioxo-3,4-bis(phenylthio)-2,5-dihydro-1H-pyrrol-1-yl)ethoxy)ethoxy)propanoate of Production Example 201-22 (2.2 mg, 0.0041 mmol), by the same method as Example 205.

ESI-MS (m/z): 1211.30[(M+2H)/2]+.

[Production Example 252-1]

(S)-N1-((3-(5-Iodo-2-methoxyphenyl)-2,6-dioxotetrahydropyrimidine-1(2H)-yl) methyl)-2-(2,5,8,11,14,17,20,23,26,29,32,35-dodecaoxaoctatriacontan-38-amide) succinamide

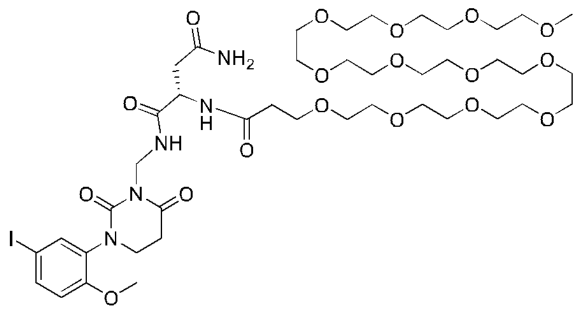

The title compound (17 mg) was obtained from the (9H-fluoren-9-yl)methyl (S)-(4-amino-1-(((3-(5-iodo-2-methoxyphenyl)-2,6-dioxotetrahydropyrimidine-1(2H)-yl)methyl)amino)-1,4-dioxobutan-2-yl)carbamate of Production Example 218-1 (20 mg, 0.028 mmol) and 2,5-dioxopyrrolidin-1-yl 2,5,8,11,14,17,20,23,26,29,32,35-dodecaoxaoctatriacontan-38-oate (23 mg, 0.034 mmol), by the same method as Production Example 250-1.

ESI-MS (m/z): 1060.43 [M+H]+.

[Production Example 252-2]

(S)-N1-((3-(5-(3-((1'-(4-((1S,10S)-16-Amino-10-benzyl-1-cyclopropoxy-6,9,12,15-tetraoxo-1-phenyl-3-oxa-5,8,11,14-tetraazahexadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl) methyl)-2-(2,5,8,11,14,17,20,23,26,29,32,35-dodecaoxaoctatriacontan-38-amide) succinamide

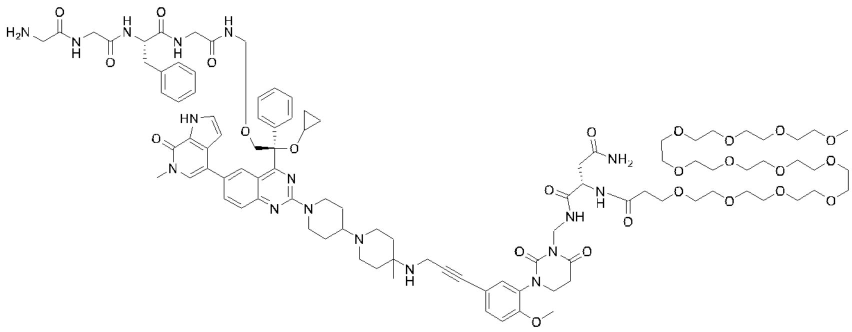

A formate of the title compound (4.2 mg) was obtained from the formate of (9H-fluoren-9-yl)methyl ((1S,10S)-10-benzyl-1-cyclopropoxy-1-(2-(4-methyl-4-(prop-2-yn-1-ylamino)-[1,4'-bipiperidine]-1'-yl)-6-(6-methyl-7-oxo-6,7-dihydro-1H-pyrrolo[2,3-c]pyridin-4-yl)quinazolin-4-yl)-6,9,12,15-tetraoxo-1-phenyl-3-oxa-5,8,11,14-tetraazahexadecan-16-yl)carbamate of Production Example 203-1 (7.0 mg) and the (S)-N1-((3-(5-iodo-2-methoxyphenyl)-2,6-dioxotetrahydropyrimidine-1(2H)-yl)methyl)-2-(2,5,8,11,14,17,20,23,26,29,32,35-dodecaoxaoctatriacontan-38-amide) succinamide of Production Example 252-1 (8.3 mg, 0.0078 mmol), by the same method as Production Example 204-5.
ESI-MS (m/z): 983.56 [(M+2H)/2]$^+$.

[Example 253]

(S)-3-Acetamide-4-(((3-(5-(3-((1'-(4-((1S,10S)-10-benzyl-1-cyclopropoxy-20-(2,5-dioxo-3,4-bis(phenylthio)-2,5-dihydro-1H-pyrrol-1-yl)-6,9,12,15,18-pentaoxo-1-phenyl-3-oxa-5,8,11,14,17-pentaazaeicosyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid

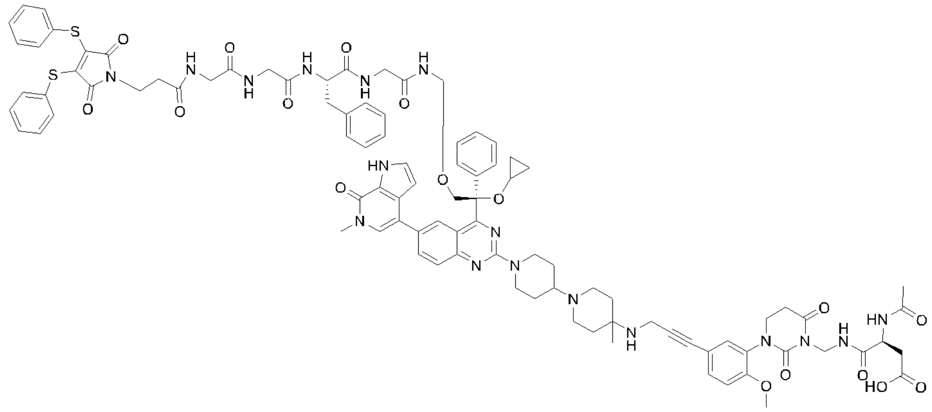

A formate of the title compound (2.0 mg) was obtained from the formate of (S)-3-acetamide-4-(((3-(5-(3-((1'-(4-((1S,10S)-16-amino-10-benzyl-1-cyclopropoxy-6,9,12,15-tetraoxo-1-phenyl-3-oxa-5,8,11,14-tetraazahexadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid of Production Example 203-2 (6.3 mg) and the tert-butyl 3-(2,5-dioxo-3,4-bis(phenylthio)-2,5-dihydro-1H-pyrrol-1-yl)propanoate of Production Example 216-2 (3.5 mg, 0.0080 mmol), by the same method as Example 205.
ESI-MS (m/z): 903.39 [(M+2H)/2]$^+$.

[Example 254]

(S)-3-Acetamide-4-(((3-(5-(3-((1'-(4-((1S,10S)-10-benzyl-1-cyclopropoxy-23-(2,5-dioxo-3,4-bis(phenylthio)-2,5-dihydro-1H-pyrrol-1-yl)-6,9,12,15,18-pentaoxo-1-phenyl-3,21-dioxa-5,8,11,14,17-pentaazatricosyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid

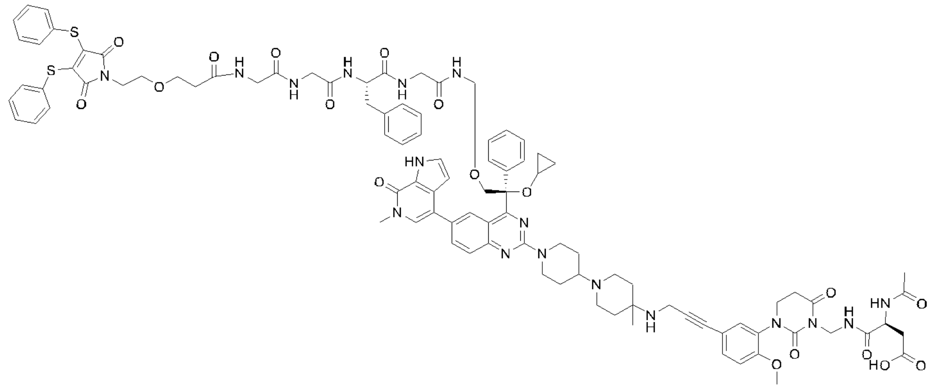

A formate of the title compound (2.2 mg) was obtained from the formate of (S)-3-acetamide-4-(((3-(5-(3-((1'-(4-((1S,10S)-16-amino-10-benzyl-1-cyclopropoxy-6,9,12,15-tetraoxo-1-phenyl-3-oxa-5,8,11,14-tetraazahexadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid of Production Example 203-2 (6.3 mg) and the tert-butyl 3-(2-(2,5-dioxo-3,4-bis(phenylthio)-2,5-dihydro-1H-pyrrol-1-yl)ethoxy)propanoate of Production Example 217-2 (3.9 mg, 0.0080 mmol), by the same method as Example 205.

ESI-MS (m/z): 925.63 [(M+2H)/2]$^+$.

[Example 255]

(S)-3-Acetamide-4-(((3-(5-(3-((1'-(4-((44S,50S,59S)-50-benzyl-59-cyclopropoxy-44-(3-(2-(2-(2,5-dioxo-2,5-dihydro-1H-pyrrol-1-ylethoxy)ethoxy)propanamido)-38,45,48,51,54-pentaoxo-59-phenyl-2,5,8,11,14,17,20,23,26,29,32,35,57-tridecaoxa-39,46,49,52,55-pentaazanonapentacontan-59-yl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid

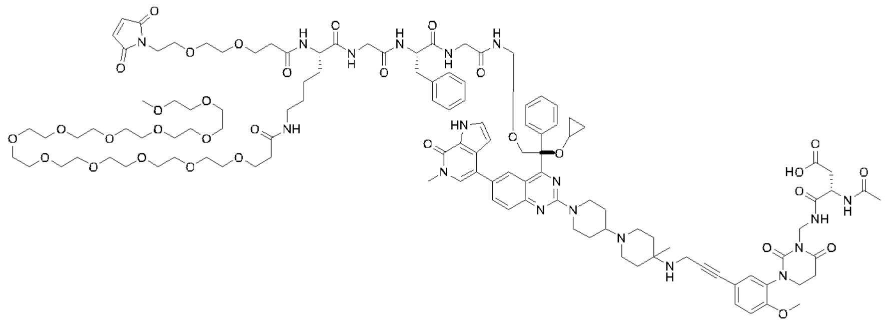

The title compound (6.9 mg) was obtained from the (S)-3-acetamide-4-(((3-(5-(3-((1'-(4-((44S,50S,59S)-44-amino-50-benzyl-59-cyclopropoxy-38,45,48,51,54-pentaoxo-59-phenyl-2,5,8,11,14,17,20,23,26,29,32,35,57-tridecaoxa-39,46,49,52,55-pentaazanonapentacontan-59-yl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid of Production Example 201-20 (9.8 mg, 4.7 μmol) and N-succinimidyl 13-maleimide-11-oxo-4,7-dioxa-10-azatridecanoate (3.34 mg, 9.43 μmol), by the same method as Example 201.

ESI-MS (m/z): 1160.42 [(M+2H)/2]$^+$.

[Example 256]

(S)-40-(((3-(5-(3-((1'-(4-((1S,7S)-7-(2-Amino-2-oxoethyl)-1-cyclopropoxy-17-(2,5-dioxo-3,4-bis(phenylthio)-2,5-dihydro-1H-pyrrol-1-yl)-6,9-dioxo-1-phenyl-3,12,15-trioxa-5,8-diazaheptadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)-3 8-oxo-2,5,8,11,14,17,20,23,26,29,32,35-dodecaoxa-39-azadotetracontan-42-oic acid

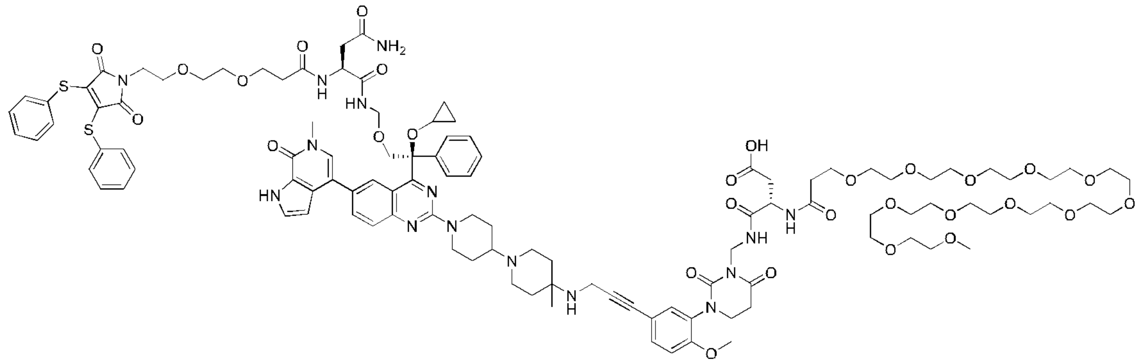

To a solution of the *tert*-butyl 3-(2-(2-(2,5-dioxo-3,4-bis(phenylthio)-2,5-dihydro-1H-pyrrol-1-yl)ethoxy)ethoxy) propionate of Production Example 201-22 (14.0 mg, 0.026 mmol) in dichloromethane (2 mL) there was added TFA (0.5 mL), and the mixture was stirred for 30 minutes at room temperature. The reaction mixture was concentrated under reduced pressure, and then N,N-diisopropylethylamine (100 μL, 0.573 mmol) and HATU (10.0 mg, 0.026 mmol) were added to a solution of the residue in DMF (2 mL), and the mixture was stirred for 10 minutes at 0°C. The (S)-40-(((3-(5-(3-((1'-(4-((S)-1-cyclopropoxy-2-(((S)-2,4-diamino-4-oxobutanamido)methoxy)-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)-38-oxo-2,5,8,11,14,17,20,23,26,29,32,35-dodecaoxa-39-azadotetracontan-42-oic acid of Production Example 256-1 (31 mg, 0.018 mmol) was added to the reaction mixture, and the mixture was stirred for 1 hour at 0°C. The reaction mixture was purified by reversed-phase silica gel column chromatography (0.1% formic acid aqueous solution:0.1% acetonitrile formate solution = 95:5 to 3:7) to obtain a formate of the title compound (22 mg).

ESI-MS (m/z): 1109.93 [(M+2H)/2]$^+$.

[Production Example 256-1]

(S)-40-(((3-(5-(3-((1'-(4-((S)-1-Cyclopropoxy-2-(((S)-2,4-diamino-4-oxobutanamido)methoxy)-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)- 3 8-oxo-2,5,8,11,14,17,20,23,26,29,32,35-dodecaoxa-39-azadotetracontan-42-oic acid

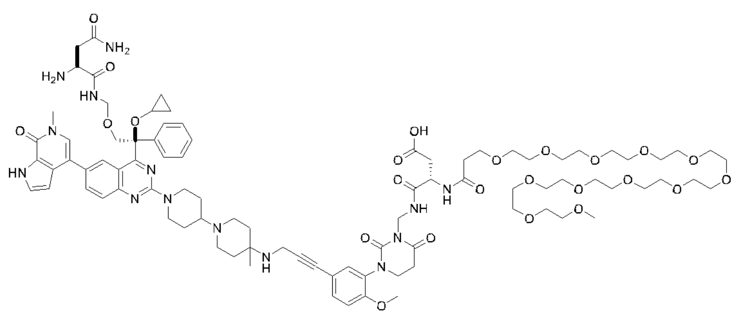

The title compound (31 mg) was obtained from the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 201-9 (55 mg, 0.052 mmol) and the (S)-40-(((3-(5-iodo-2-methoxyphenyl)-2,6-dioxotetrahydropyrimidine-1(2H)-yl)methyl)carbamoyl)-38-oxo-2,5,8,11,14,17,20,23,26,29,32,35-dodecaoxa-39-azadotetracontan-42-oic acid of Production Example 247-2 (72.2 mg, 0.068 mmol), by the same methods as Production Example 218-2 and Production Example 226-4.

ESI-MS (m/z): 1762.89 $[M+H]^+$.

[Example 257]

(3S)-3-[({[(2-{2-[4-({3-[3-(3-{[(2S)-3-Carboxy-2-(2,5,8,11,14,17,20,23,26,29,32,35-dodecaoxaoctatriacontan-38-amide) propanamido]methyl}-2,4-dioxo-1,3-diazinane-1-yl)-4-methoxyphenyl]prop-2-yn-1-yl}amino)-4-methyl-[1,4'-bipiperidin]-1'-yl]-6-{6-methyl-7-oxo-1H,6H,7H-pyrrolo[2,3-c]pyridin4-yl}quinazolin-4-yl}-2-cyclopropoxy-2-phenylethoxy) methyl]carbamoyl}methyl)carbamoyl]-3-[3-(2-{2-[2,5-dioxo-3,4-bis(phenylsulfanyl)-2,5-dihydro-1H-pyrrol-1-yl]ethoxy} ethoxy)propaneamide]propanoic acid

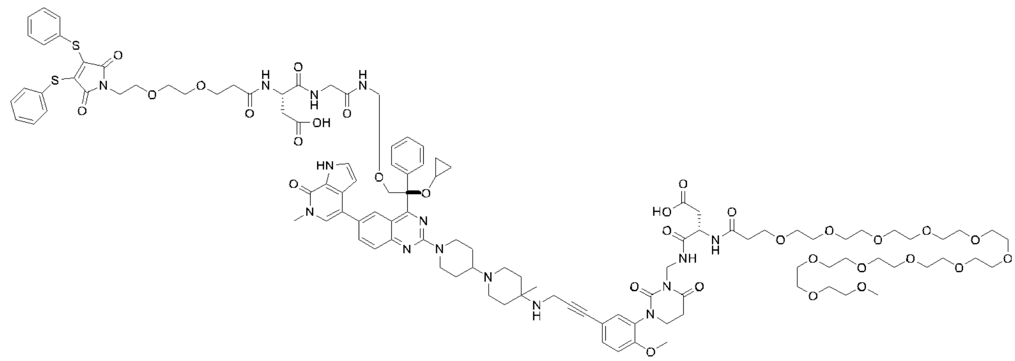

To a solution of the (S)-40-(((3-(5-(3-((1'-(4-((S)-2-((2-((S)-2-Amino-3-carboxypropanamido)acetamide))methoxy)-1-cyclopropoxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidine] -4-yl)amino)prop-1-yn-1-yl)-2-methoxyphenyl)-2,6-dioxotetrahydropyrimidine-1(2H)-yl)methyl)carbamoyl)-38-oxo-2,5,8,11,14,17,20,23,26,29,32,35-dodecaoxa-39-azadotetracontan-42-oic acid (13 mg, 7.1 μmol) in N,N-dimethylformamide (0.64 mL) there was added diethylamine (170 μL, 1.6 mmol), and after stirring for 20 minutes at room temperature, the reaction mixture was concentrated. The obtained residue was processed 3 times by azeotropic distillation with toluene (1.0 mL). In a separate vessel, trifluoroacetic acid (1.0 mL) was added to a solution of the tert-butyl 3-(2-(2-(2,5-dioxo-3,4-bis(phenylthio)-2,5-dihydro-1H-pyrrol-1-yl)ethoxy)ethoxy)propionate synthesized in Production Example 201-22 (6.6 mg, 12 μmol) in dichloromethane (1.0 mL), and the reaction mixture was stirred for 1.5 hours at room temperature and then concentrated. Toluene (1.0 mL) was added to the obtained residue and azeotropic distillation was repeated 3 times, after which it was dissolved in DMF (0.64 mL). After adding N,N-diisopropylethylamine (14 μL, 82 μmol) and HATU (4.7 mg, 0.012 mmol), the mixture was stirred for 5 minutes at room temperature and then combined with the previously prepared amine residue. After stirring for 1 hour at room temperature, the mixture was purified by preparative HPLC to obtain the title compound (3.4 mg).

ESI-MS (m/z): 1138.63 $[(M+2H)/2]^+$.

[Production Example 257-1]

*tert*-Butyl (3S) -3-{[({[2-cyclopropoxy-2-(2-{4-methyl-4-[(prop-2-yn-1-yl)amino]-[1,4'-bipiperidin]-1'-yl }-6-{ 6-methyl-7-oxo-1H,6H, 7H-pyrrolo[2,3-c]pyridin-4-yl } quinazolin-4-yl)-2-phenylethoxy]methyl}carbamoyl)methyl]carbamoyl}-3-({[(9H-fluoren-9-yl)methoxy]carbonyl}amino)propanoate

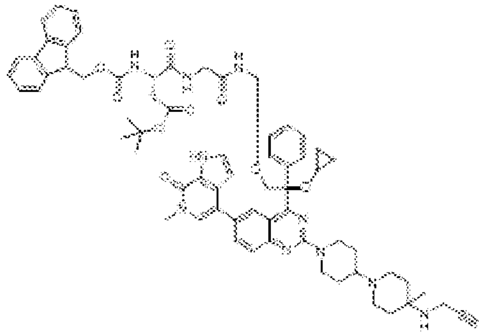

To a solution of the (9H-fluoren-9-yl)methyl (S)-(2-(((2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamate of Production Example 201-11 (45 mg, 0.045 mmol) in DMF (3.5 mL) there was added diethylamine (702 μL, 6.8 mmol), and the mixture was stirred for 20 minutes at room temperature and concentrated, and then processed 3 times by azeotropic distillation with toluene (2.0 mL). The obtained residue was dissolved in DMF (3.5 mL), and then (2*S*)-4-(*tert*-butoxy)-2-({[(9H-fluoren-9-yl)methoxy]carbonyl}amino)-4-oxobutanoic acid (28 mg, 0.068 mmol), N,N-diisopropylethylamine (79 μL, 0.45 mmol) and HATU (24 mg, 0.063 mmol) were added. The reaction mixture was stirred for 1 hour at room temperature and then purified by ODS silica gel column chromatography (0.1% acetonitrile formate solution:0.1% formic acid aqueous solution = 2:98 to 70:30) to obtain a formate of the title compound (30 mg).

ESI-MS (m/z): 1165.59 $[M+H]^+$.

[Production Example 257-2]

(S)-40-(((3-(5-(3-((1'-(4-((S)-2-((2-((S)-2-Amino-3-carboxypropanamido)acetamide))methoxy)-1-cyclopropoxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidine] -4-yl) amino)prop-1-yn-1-yl)-2-methoxyphenyl)-2,6-dioxotetrahydropyrimidine-1(2H)-yl)methyl)carbamoyl)-38-oxo-2,5,8,11,14,17,20,23,26,29,32,35-dodecaoxa-39-azadotetracontan-42-oic acid

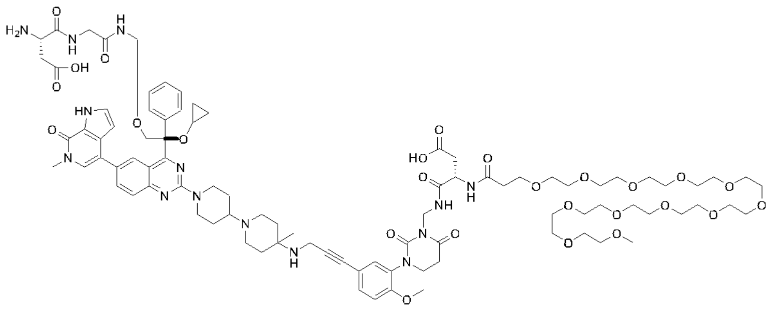

To a solution of the formate of *tert-butyl* (3S) -3-{[({[2-cyclopropoxy-2-(2-{4-methyl-4-[(prop-2-yn-1-yl)amino]-[1,4'-bipiperidin]-1'-yl}-6- f 6-methyl-7-oxo-1H,6H,7H-pyrrolo[2,3-c]pyridin-4-yl}quinazolin-4-yl)-2-phenylethoxy] methyl}carbamoyl)methyl]carbamoyl}-3-({[(9H-fluoren-9-yl)methoxy]carbonyl}amino)propanoate of Production Example 257-1 (30 mg) in THF (1.3 mL) there was added potassium *tert*-butoxide (29 mg, 0.26 mmol) at 0°C, and the mixture was stirred for 20 minutes. After adding a solution of 1 N hydrochloric acid (257 μL) and triethylamine (54 μL, 0.39 mmol) in THF (0.50 mL) to the reaction mixture to halt the reaction, the mixture was concentrated under reduced pressure and then processed by azeotropic distillation with toluene (1.0 mL) and methanol (1.0 mL) to obtain an intermediate. To a solution of the obtained intermediate and the (S)-40-(((3-(5-iodo-2-methoxyphenyl)-2,6-dioxotetrahydropyrimidine-1(2H)-yl)methyl) carbamoyl)-38-oxo-2,5,8,11,14,17,20,23,26,29,32,35-dodecaoxa-39-azadotetracontan-42-oic acid of Production Example 247-2 (40 mg, 0.038 mmol) in DMF (1.5 mL) there were added N,N-diisopropylethylamine (450 μL, 2.5 mmol), copper iodide (1.9 mg, 10 μmol) and tetrakistriphenylphosphinepalladium (12 mg, 10 μmol). The reaction mixture was stirred for 6 hours at 55°C under a nitrogen atmosphere. The reaction mixture was returned to room temperature and then purified by ODS silica gel column chromatography (0.1% acetonitrile formate solution:0.1% formic acid aqueous solution = 2:98 to 70:30) to obtain a formate of the title compound (15 mg).

ESI-MS (m/z): 1820.98 [M+H]$^+$.

[Example 258]

(44S,51S)-51-(((3-(5-(3-((1'-(4-((1S,10S)-10-(2-Amino-2-oxoethyl)-1-cyclopropoxy-6,9,12-trioxo-1-phenyl-3-oxa-5,8,11-triazatridecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)-44-(3-(2-(2-(2,5-dioxo-3,4-bis(phenylthio)-2,5-dihydro-1H-pyrrol-1-yl)ethoxy)ethoxy)propanamido)-38,45,49-trioxo-2,5,8,11,14,17,20,23,26,29,32,35-dodecaoxa-39,46,50-triazatripentacontan-53-oic acid

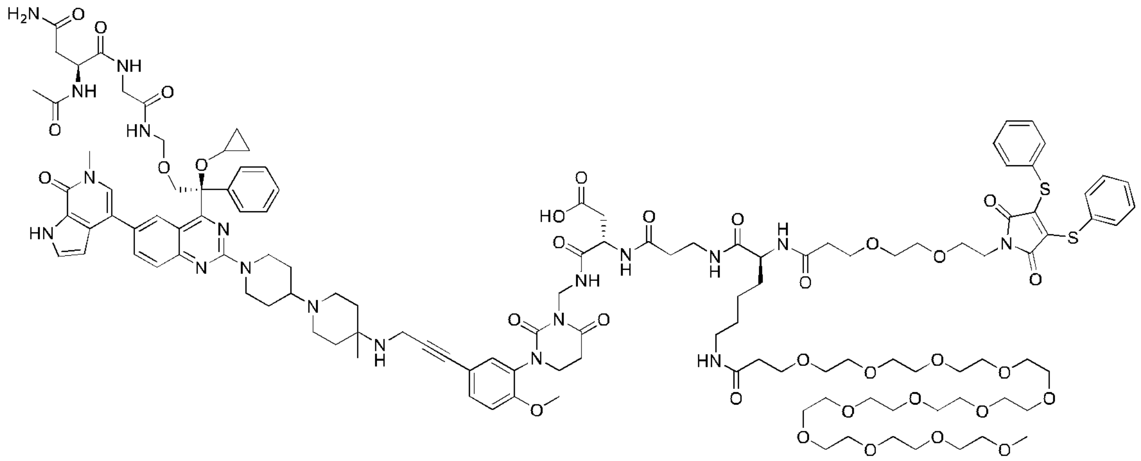

The title compound (3.07 mg) was obtained from the (S)-3-((((9H-fluoren-9-yl)methoxy)carbonyl)amino)-4-(((3-(5-(3-((1'-(4-((1S,10S)-10-(2-amino-2-oxoethyl)-1-cyclopropoxy-6,9,12-trioxo-1-phenyl-3-oxa-5,8,11-triazatridecyl)-6-(6-methyl-7-oxo-6,7-dihydro-1H-pyrrolo[2,3-c]pyridin-4-yl)quinazolin-2-yl)-4-methyl-[1,4'-bipiperidine] -4-yl)amino)prop-1-yn-1-yl)-2-methoxyphenyl)-2,6-dioxotetrahydropyrimidine-1(2H)-yl)methyl)amino)-4-oxobutanoic acid of Production Example 249-2 (11 mg, 7.3 μmol) and the tert-butyl(S)-44-(3-(2-(2-(2,5-dioxo-3,4-bis(phenylthio)-2,5-dihydro-1H-pyrrol-1-yl)ethoxy)ethoxy)propanamido)-38,45-dioxo-2,5,8,11,14,17,20,23,26,29,32,35-dodecaoxa-39,46-diazanonatetracontan-49-oate of Production Example 239-2 (12.3 mg, 9.45 μmol), by the same method as Example 239.

ESI-MS (m/z): 1259.08 [(M+2H)/2]$^+$.

[Example 259]

(S)-N1-((3-(5-(3-((1'-(4-((1S,10S)-10-Benzyl-1-cyclopropoxy-26-(2,5-dioxo-3,4-bis(phenylthio)-2,5-dihydro-1H-pyrrol-1-yl)-6,9,12,15,18-pentaoxo-1-phenyl-3,21,24-trioxa-5,8,11,14,17-pentaazahexacosyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)
methyl)-2-(2,5,8,11,14,17,20,23,26,29,32,35,38,41,44,47,50,53,56,59,62,65,68,71-tetracosaoxatetraheptacontan-74-amide) succinamide

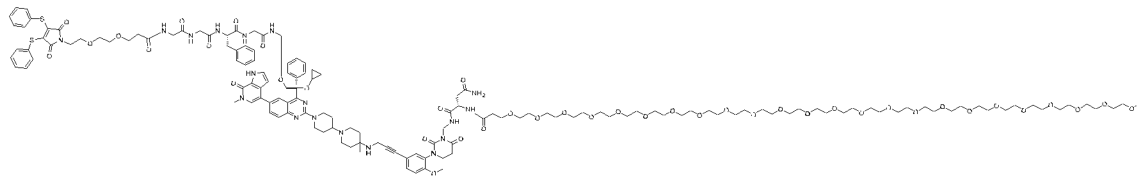

A formate of the title compound (3.6 mg) was obtained from the formate of (S)-N1-((3-(5-(3-((1'-(4-((1S,10S)-16-amino-10-benzyl-1-cyclopropoxy-6,9,12,15-tetraoxo-1-phenyl-3-oxa-5,8,11,14-tetraazahexadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl) methyl)-2-(2,5,8,11,14,17,20,23,26,29,32,35,38,41,44,47,50,53,56,59,62,65,68,71-tetracosaoxatetraheptacontan-74-amide) succinamide of Production Example 259-2 (5.4 mg) and the tert-butyl 3-(2-(2-(2,5-dioxo-3,4-bis(phenylthio)-2,5-dihydro-1H-pyrrol-1-yl)ethoxy)ethoxy)propanoate of Production Example 201-22 (2.2 mg, 0.0041 mmol), by the same method as Example 205.

ESI-MS (m/z): 1475.63[(M+2H)/2]+.

[Production Example 259-1]

(S)-N1-((3-(5-Iodo-2-methoxyphenyl)-2,6-dioxotetrahydropyrimidine-1(2H)-yl) methyl)-2-(2,5,8,11,14,17,20,23,26,29,32,35,38,41,44,47,50,53,56,59,62,65,68,71-tetracosaoxatetraheptacontan-74-amide) succinamide

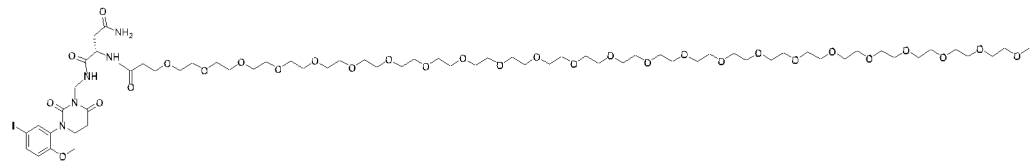

The title compound (26 mg) was obtained from the (9H-fluoren-9-yl)methyl (S)-(4-amino-1-(((3-(5-iodo-2-methoxyphenyl)-2,6-dioxotetrahydropyrimidine-1(2H)-yl)methyl)amino)-1,4-dioxobutan-2-yl)carbamate of Production Example 218-1 (20 mg, 0.028 mmol) and 2,5-dioxopyrrolidin-1-yl 2,5,8,11,14,17,20,23,26,29,32,35,38,41,44,47,50,53,56,59,62,65,68,71-tetracosaoxatetraheptacontan-74-oate (41 mg, 0.034 mmol), by the same method as Production Example 250-1.

ESI-MS (m/z): 795.31 [(M+2H)/2]+.

[Production Example 259-2]

(S)-N1-((3-(5-(3-((1'-(4-((1S,10S)-16-Amino-10-benzyl-1-cyclopropoxy-6,9,12,15-tetraoxo-1-phenyl-3-oxa-5,8,11,14-tetraazahexadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl) methyl)-2-(2,5,8,11,14,17,20,23,26,29,32,35,38,41,44,47,50,53,56,59,62,65,68,71-tetracosaoxatetraheptacontan-74-amide) succinamide

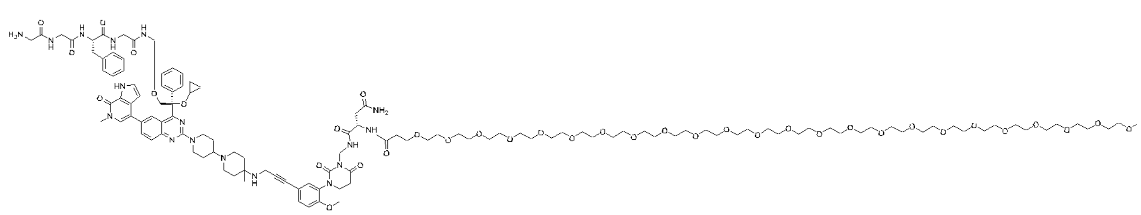

A formate of the title compound (5.4 mg) was obtained from the formate of (9H-fluoren-9-yl)methyl((1S,10S)-10-benzyl-1-cyclopropoxy-1-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-6,9,12,15-tetraoxo-1-phenyl-3-oxa-5,8,11,14-tetraazahexadecan-16-yl)carbamate of Production Example 203-1 (7.0 mg) and the (S)-N1-((3-(5-iodo-2-methoxyphenyl)-2,6-dioxotetrahydropyrimidine-1(2H)-yl)methyl)-2-(2,5,8,11,14,17,20,23,26,29,32,35,38,41,44,47,50,53,56,59,62,65,68,71-tetracosaoxatetraheptacontan-74-amide) succinamide of Production Example 259-1 (12 mg, 0.0078 mmol), by the same method as Production Example 204-5.

ESI-MS (m/z): 1247.77 [(M+2H)/2]+.

[Example 260]

(S)-4-(((3-(5-(3-((1'-(4-((28S, 37S)-28-(2-Amino-2-oxoethyl)-37-cyclopropoxy-26,29,32-trioxo-37-phenyl-2,5,8,11,14,17,20,23,35-nonaoxa-27,30,33-triazaheptatriacontan-37-yl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-3-(3-(2-(2-(2,5-dioxo-3,4-bis(phenylthio)-2,5-dihydro-1H-pyrrol-1-yl)ethoxy)ethoxy)propanamido)-4-oxobutanoic acid

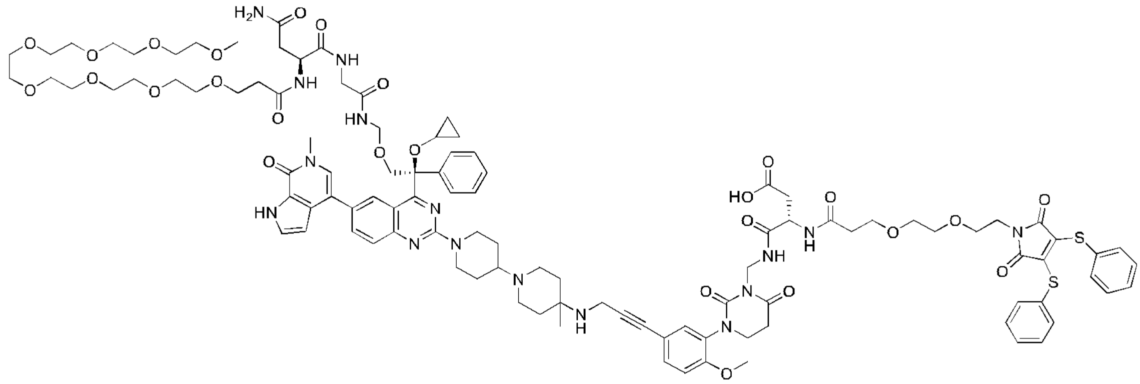

A formate of the title compound (1.2 mg) was obtained from the formate of (S)-3-((((9H-fluoren-9-yl)methoxy)carbonyl)amino)-4-(((3-(5-(3-((1'-(4-((28S, 37S)-28-(2-amino-2-oxoethyl)-37-cyclopropoxy-26,29,32-trioxo-37-phenyl-2,5,8,11,14,17,20,23,35-nonaoxa-27,30,33-triazaheptatriacontan-37-yl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid of Production Example 260-3 (9.3 mg), by the same method as Example 218. ESI-MS (m/z): 1050.19 $[(M+2H)/2]^+$.

[Production Example 260-1]

*tert*-Butyl (2,5,8,11,14,17,20,23-octaoxahexacosan-26-oyl)-L-asparaginate

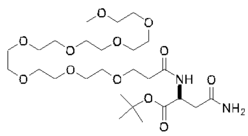

To a solution of *tert*-butyl (2S)-2-amino-3-carbamoyl propanoate (50 mg, 0.266 mmol) and N-succinimidyl 4,7,10,13,16,19,22,25-octaoxahexacosanoate (135 mg, 0.266 mmol) in DMF (2 mL) there was added N,N-diisopropylethylamine (186 μL, 1.06 mmol), and the mixture was stirred for 3 hours at 50°C. The reaction mixture was concentrated under reduced pressure to obtain a crude product of the title compound (170 mg).
ESI-MS (m/z): 583.48 $[M+H]^+$.

[Production Example 260-2]

(S)-N1-(2-((((S)-2-Cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)-2-(2,5,8,11,14,17,20,23-octaoxahexacosan-26-amide) succinamide

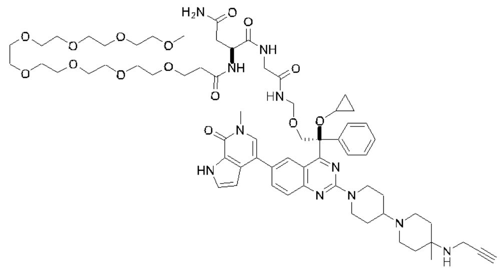

A formate of the title compound (25 mg) was obtained from the (9H-fluoren-9-yl)methyl(S)-(2-(((2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamate of Production Example 201-11 (40 mg, 0.040 mmol) and the *tert-butyl* (2,5,8,11,14,17,20,23-octaoxahexacosan-26-oyl)-L-asparaginate of Production Example 260-1 (46.9 mg, 0.080 mmol), by the same method as Production Example 249-1.

ESI-MS (m/z): 1281.11 $[M+H]^+$.

[Production Example 260-3]

(S)-3-((((9H-Fluoren-9-yl)methoxy)carbonyl)amino)-4-(((3-(5-(3-((1'-(4-((28S, 37S)-28-(2-amino-2-oxoethyl)-37-cyclopropoxy-26,29,32-trioxo-37-phenyl-2,5,8,11,14,17,20,23,35-nonaoxa-27,30,33-triazaheptatriacontan-37-yl)-6-(6-methyl-7-oxo-6,7-dihydro-1H-pyrrolo[2,3-c]pyridin-4-yl)quinazoline-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid

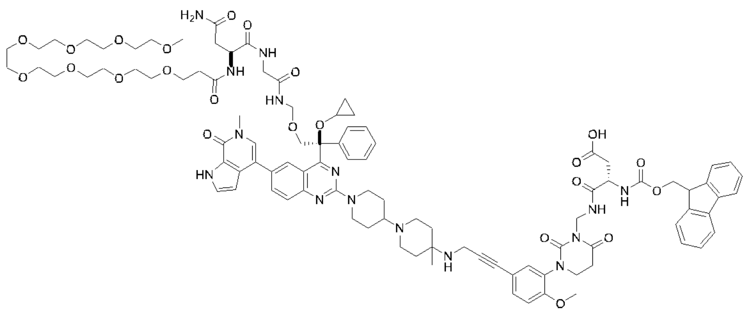

A formate of the title compound (9.3 mg) was obtained from the formate of (S)-N1-(2-((((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)-2-(2,5,8,11,14,17,20,23-octaoxahexacosan-26-amide) succinamide of Production Example 260-2 (25 mg) and the (S)-3-((((9H-fluoren-9-yl)methoxy)carbonyl)amino)-4-(((3-(5-iodo-2-methoxyphenyl)-2,6-dioxotetrahydropyrimidine-1(2H)-yl)methyl)amino)-4-oxobutanoic acid of Production Example 221-1 (16.7 mg, 0.023 mmol), by the same method as Production Example 218-2. ESI-MS (m/z): 933.67 $[(M+2H)/2]^+$.

[Example 261]

(S)-76-(((3-(5-(3-((1'-(4-((1S,10S)-10-Benzyl-1-cyclopropoxy-26-(2,5-dioxo-3,4-bis(phenylthio)-2,5-dihydro-1H-pyrrol-1-yl)-6,9,12,15,18-pentaoxo-1-phenyl-3,21,24-trioxa-5,8,11,14,17-pentaazahexacosyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)-74-oxo-2,5,8,11,14,17,20,23,26,29,32,35,38,41,44,47,50,53,56,59,62,65,68,71-tetracosaoxa-75-azaoctaheptacontan-78-oic acid

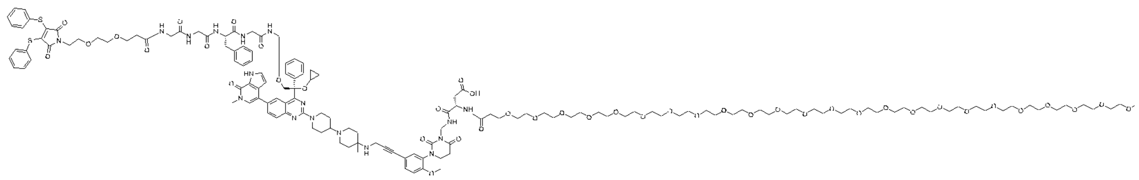

A formate of the title compound (3.6 mg) was obtained from the formate of (S)-76-(((3-(5-(3-((1'-(4-((1S,10S)-16-amino-10-benzyl-1-cyclopropoxy-6,9,12,15-tetraoxo-1-phenyl-3-oxa-5,8,11,14-tetraazahexadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)-74-oxo-2,5,8,11,14,17,20,23,26,29,32,35,38,41,44,47,50,53,56,59,62,65,68,71-tetracosaoxa-75-azaoctaheptacontan-78-oic acid of Production Example 261-2 (8.8 mg) and the *tert*-butyl 3-(2-(2-(2,5-dioxo-3,4-bis(phenylthio)-2,5-dihydro-1H-pyrrol-1-yl)ethoxy)ethoxy)propanoate of Production Example 201-22 (3.5 mg, 0.0067 mmol), by the same method as Example 205.

ESI-MS (m/z): 1476.13[(M+2H)/2]$^+$.

[Production Example 261-1]

(S)-76-(((3-(5-Iodo-2-methoxyphenyl)-2,6-dioxotetrahydropyrimidine-1(2H)-yl)methyl)carbamoyl)-74-oxo-2,5,8,11,14,17,20,23,26,29,32,35,38,41,44,47,50,53,56,59,62,65,68,71-tetracosaoxa-75-azaoctaheptacontan-78-oic acid

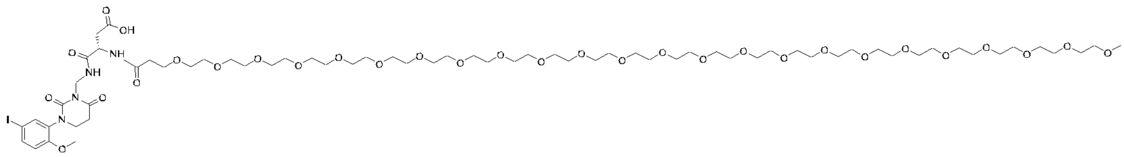

The title compound (85 mg) was obtained from the *tert-butyl* (S)-3((((9H-fluoren-9-yl)methoxy)carbonyl)amino)-4-(((3-(5-iodo-2-methoxyphenyl)-2,6-dioxotetrahydropyrimidine-1(2H)-yl)methyl)amino)-4-oxobutanoate of Production Example 201-18 (90 mg, 0.12 mmol) and 2,5-dioxopyrrolidin-1-yl 2,5,8,11,14,17,20,23,26,29,32,35,38,41,44,47,50,53,56,59,62,65,68,71-tetracosaoxatetraheptacontan-74-oate (114 mg, 0.094 mmol), by the same method as Production Example 251-1.

ESI-MS (m/z): 795.84 [(M+2H)/2]$^+$.

[Production Example 261-2]

(S)-76-(((3-(5-(3-((1'-(4-((1S,10S)-16-Amino-10-benzyl-1-cyclopropoxy-6,9,12,15-tetraoxo-1-phenyl-3-oxa-5,8,11,14-tetraazahexadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)carbamoyl)-74-oxo-2,5,8,11,14,17,20,23,26,29,32,35,38,41,44,47,50,53,56,59,62,65,68,71-tetracosaoxa-75-azaoctaheptacontan-78-oic acid

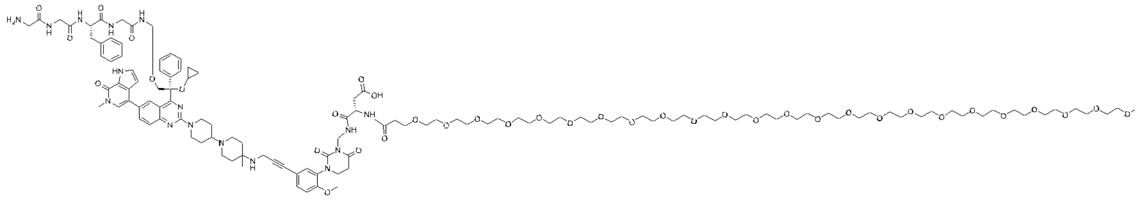

A formate of the title compound (8.8 mg) was obtained from the formate of (9H-fluoren-9-yl)methyl((1S,10S)-10-benzyl-1-cyclopropoxy-1-(2-(4-methyl-4-(prop-2-yn-1-ylamino)-[1,4'-bipiperidine]-1'-yl)-6-(6-methyl-7-oxo-6,7-dihydro-1H-pyrrolo[2,3-c]pyridin-4-yl)quinazolin-4-yl)-6,9,12,15-tetraoxo-1-phenyl-3-oxa-5,8,11,14-tetraazahexadecan-16-yl)carbamate of Production Example 203-1 (18 mg) and the (S)-76-(((3-(5-iodo-2-methoxyphenyl)-2,6-dioxotetrahydropyrimidine-1(2H)-yl)methyl)carbamoyl)-74-oxo-2,5,8,11,14,17,20,23,26,29,32,35,38,41,44,47,50,53,56,59,62,65,68,71-tetracosaoxa-75-azaoctaheptacontan-78-oic acid of Production Example 261-1 (21 mg, 0.013 mmol), by the same method as Production Example 204-5.

ESI-MS (m/z): 1248.34 [(M+2H)/2]$^+$.

[Example 262]

(S)-40-((2-((((S)-2-(2-(4-((3-(3-(3-((S)-4-(Carboxymethyl)-14-(2,5-dioxo-3,4-bis(phenylthio)-2,5-dihydro-1H-pyrrol-1-yl)-3,6-dioxo-9,12-dioxa-2,5-diazatetradecyl)-2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-cyclopropoxy-2-phenylethoxy)methyl)amino)-2-oxoethyl)carbamoyl)-38-oxo-2,5,8,11,14,17,20,23,26,29,32,35-dodecaoxa-39-azadotetracontan-42-oic acid

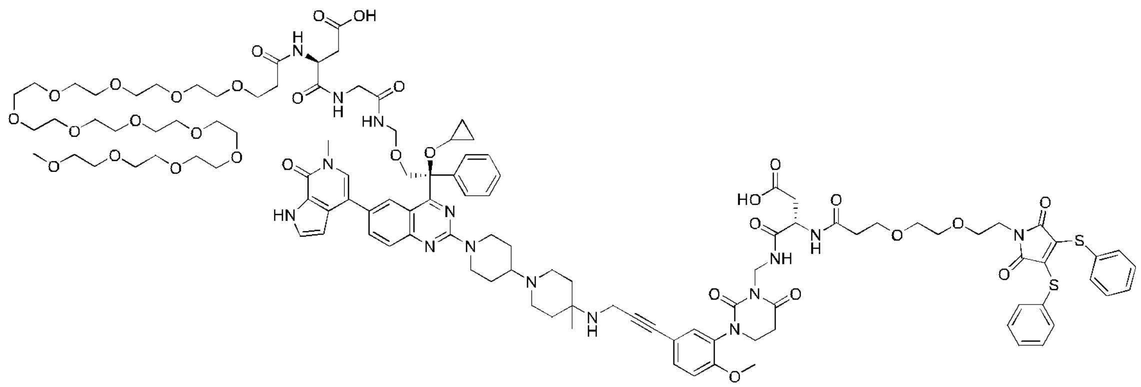

The title compound (1.51 mg) was obtained from the (S)-40-((2-((((S)-2-(2-(4-((3-(3-(3-(((S)-2-((((9H-fluoren-9-yl)methoxy)carbonyl)amino)-3-carboxypropanamido)methyl)-2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-cyclopropoxy-2-phenylethoxy)methyl)amino)-2-oxoethyl)carbamoyl)-38-oxo-2,5,8,11,14,17,20,23,26,29,32,35-dodecaoxa-39-azadotetracontan-42-oic acid of Production Example 262-3 (6.00 mg, 2.94 µmol), by the same method as Example 218. ESI-MS (m/z): 1138.85 [1/2 M+H]$^+$.

[Production Example 262-1]

(S)-40-(2-(*tert*-Butoxy)-2-oxoethyl)-38-oxo-2,5,8,11,14,17,20,23,26,29,32,35-dodecaoxa-39-azahentetracontan-41-oic acid

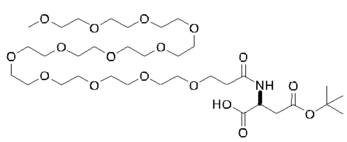

To a solution of (S)-2-((((9H-fluoren-9-yl)methoxy)carbonyl)amino)-4-(*tert*-butoxy)-4-oxobutanoic acid (120 mg, 0.292 mmol) in DMF (2 mL) there was added diethylamine (200 µL), and the mixture was stirred for 30 minutes at room temperature. The reaction mixture was concentrated under reduced pressure to obtain intermediate 1. A crude product of the title compound (220 mg) was obtained from 1-[(38-oxo-2,5,8,11,14,17,20,23,26,29,32,35-dodecaoxaoctatriacontan-38-yl)oxy]-2,5-pyrrolidinedione (200 mg, 0.292 mmol) and intermediate 1, by the same method as Production Example 260-1.

ESI-MS (m/z): 760.62 [M+H]$^+$.

[Production Example 262-2]

(S)-40-((2-((((S)-2-Cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamoyl)-38-oxo-2,5,8,11,14,17,20,23,26,29,32,35-dodecaoxa-39-azadotetracontan-42-oic acid

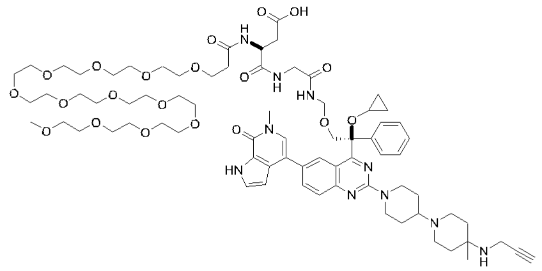

To a solution of the (9H-fluoren-9-yl)methyl(S)-(2-(((2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamate of Production Example 201-11 (40 mg, 0.040 mmol) in DMF (2 mL) there was added diethylamine (200 μL), and the mixture was stirred for 20 minutes at room temperature. The reaction mixture was concentrated under reduced pressure to obtain an intermediate. To a solution of the obtained intermediate and the (S)-40-(2-(*tert*-butoxy)-2-oxoethyl)-38-oxo-2,5,8,11,14,17,20,23,26,29,32,35-dodecaoxa-39-azahentetracontan-41-oic acid of Production Example 262-1 (30.6 mg, 0.040 mmol) in DMF (2 mL) there were added HATU (15.3 mg, 0.040 mmol) and N,N-diisopropylethylamine (100 μL, 0.573 mmol), and the mixture was stirred for 1 hour at room temperature. It was then purified by reversed-phase silica gel column chromatography (0.1% formic acid aqueous solution:0.1% acetonitrile formate solution = 95:5 to 0:1), to obtain intermediate 2. To a solution of intermediate 2 in DMF (2 mL) there was added potassium *tert*-butoxide (9.03 mg, 0.080 mmol) at 0°C, and the mixture was stirred for 1 hour at 0°C. The reaction mixture was purified by reversed-phase silica gel column chromatography (0.1% formic acid aqueous solution:0.1% acetonitrile formate solution = 95:5 to 0:1) to obtain the title compound (20 mg).

ESI-MS (m/z): 1459.11 $[M+H]^+$.

[Production Example 262-3]

(S)-40-((2-((((S)-2-(2-(4-((3-(3-(3-(((S)-2-((((9H-Fluoren-9-yl)methoxy)carbonyl)amino)-3-carboxypropanamido)methyl)-2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-2-cyclopropoxy-2-phenylethoxy)methyl)amino)-2-oxoethyl)carbamoyl)-38-oxo-2,5,8,11,14,17,20,23,26,29,32,35-dodecaoxa-39-azadotetracontan-42-oic acid

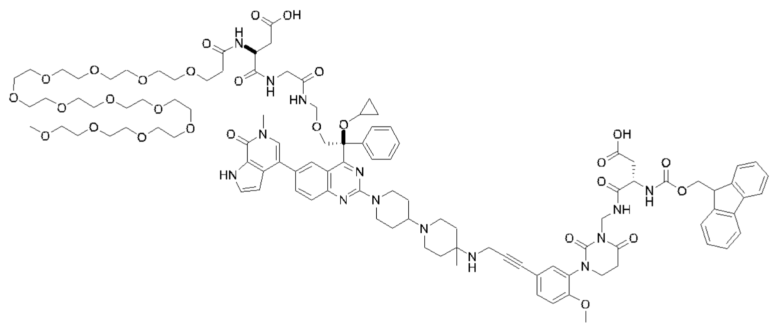

The title compound (6 mg) was obtained from the (S)-40-((2-((((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)amino)-2-oxoethyl)carbamoyl)-38-oxo-2,5,8,11,14,17,20,23,26,29,32,35-dodecaoxa-39-azadotetracontan-42-oic acid of Production Example 262-2 (17 mg, 0.012 mmol) and the (S)-3-((((9H-fluoren-9-yl)methoxy)carbonyl)amino)-4-(((3-(5-iodo-2-methoxyphenyl)-2,6-dioxotetrahydropyrimidine-1(2H)-yl)methyl)amino)-4-oxobutanoic acid of Production Example 221-1 (10.8 mg, 0.015 mmol), by the same method as Production Example 218-2.

ESI-MS (m/z): 1022.21 [1/2 M+H]+.

[Example 263]

(S)-3-Acetamide-4-(((3-(5-(3-((1'-(4-((44S,51S,57S)-51-(2-amino-2-oxoethyl)-57-cyclopropoxy-44-(3-(2-(2-(2,5-dioxo-3,4-bis(phenylthio)-2,5-dihydro-1H-pyrrol-1-yl)ethoxy)ethoxy)propanamido)-38,45,49,52-tetraoxo-57-phenyl-2,5,8,11,14,17,20,23,26,29,32,35,55-tridecaoxa-39,46,50,53-tetraazaheptapentacontan-57-yl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid

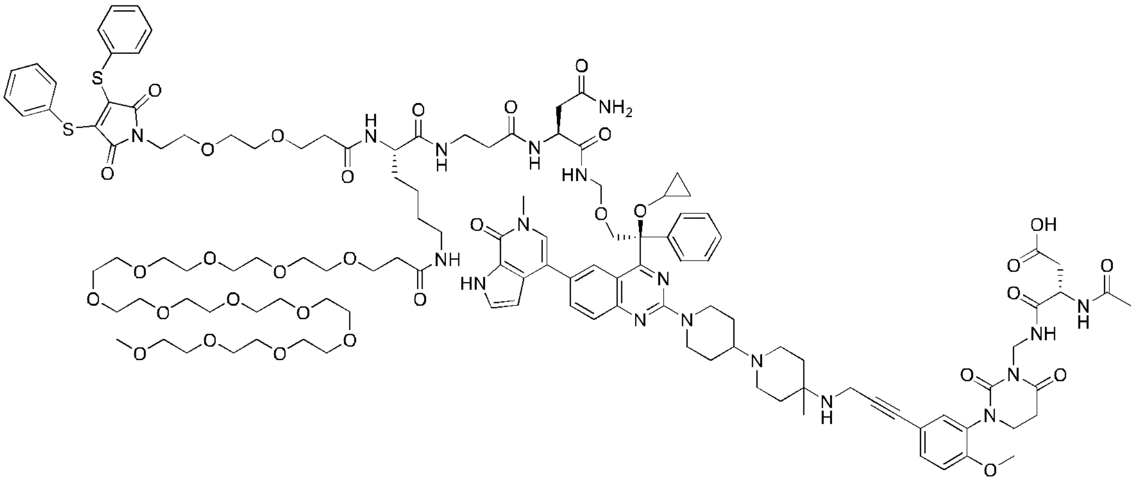

The title compound (18 mg) was obtained from the (S)-3-acetamide-4-(((3-(5-(3-((1'-(4-((S)-1-cyclopropoxy-2-(((S)-2,4-diamino-4-oxobutanamido)methoxy)-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid of Production Example 204-5 (35 mg, 0.028 mmol) and the *tert-butyl* (S)-44-(3-(2-(2-(2,5-dioxo-3,4-bis(phenylthio)-2,5-dihydro-1H-pyrrol-1-yl)ethoxy)ethoxy)propanamido)-38,45-dioxo-2,5,8,11,14,17,20,23,26,29,32,35-dodecaoxa-39,46-diazanonatetracontan-49-oate of Production Example 239-2 (51.6 mg, 0.040 mmol), by the same method as Example 256.

ESI-MS (m/z): 1230.92 [1/2 M+H]+.

[Example 264]

(2S,3S,4S,5R,6S)-6-(4-(11-Cyclopropoxy-11-(2-(4-(3-(2,4-dioxotetrahydropyrimidine-1(2H)-yl)-4-methoxybenzamide)-4-methyl-[1,4'-bipiperidin]-1'-yl)-6-(4-methoxy-1-methyl-6-oxo-1,6-dihydropyridin-3-yl)-quinazolin-4-yl)-4,7-dimethyl-3,8-dioxo-11-phenyl-2,9-dioxa4,7-diazaundecyl)-2-(3-(3-(2-(2-(2,5-dioxo-2,5-dihydro-1H-pyrrolo-1-yl)ethoxy)ethoxy)propaneamide)propaneamide)phenoxy)-3,4,5-trihydroxytetrahydro-2H-pyran-2-carboxylic acid

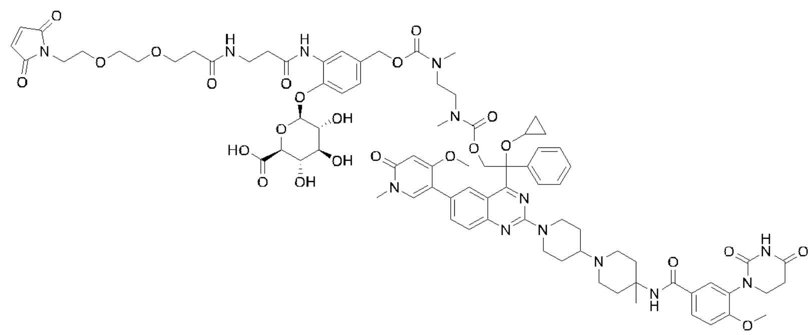

A formate of the title compound (18 mg) was obtained from the formate of (2S,3S,4S,5R,6S)-6-(4-(11-(2-(-((*tert*-butoxycarbonyl)amino)-4-methyl-[1,4'-bipiperidin]-1'-yl)-6-(4-methoxy-1-methyl-6-oxo-1,6-dihydropyridin-3-yl)quinazolin-4-yl)-11-cyclopropoxy-4,7-dimethyl-3,8-dioxo-11-phenyl-2,9-dioxa-4,7-diazaundecyl)-2-(3-(3-(2-(2-(2,5-dioxo-2,5-dihydro-1H-pyrrolo-1-yl)ethoxy)ethoxy)propaneamide)propaneamide)phenoxy)-3,4,5-trihydroxytetrahydro-2H-pyran-2-carboxylic acid of Production Example 264-11 (55 mg) and the 3-(2,4-dioxotetrahydropyrimidine-1(2H)-yl)-4-methoxybenzoic acid of Production Example 265-12 (9.7 mg, 37 μmol), by the same method as Example 265.
ESI-MS (m/z): 1651.87 $[M+H]^+$.

[Production Example 264-1]

Benzyl 4-((*tert*-butoxycarbonyl)amino)-4-methyl-[1,4'-bipiperidine]-1'-carboxylate

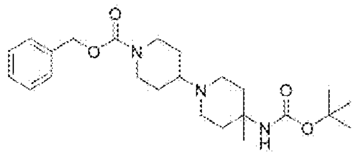

To a solution of *tert-butyl* (4-methylpiperidin-4-yl)carbamate (1286 mg, 6.00 mmol) in dichloromethane (10 mL) and acetic acid (172 μL, 3.00 mmol) there was added 1-(benzyloxycarbonyl)-4-piperidinone (700 mg, 3.00 mmol) at room temperature, and the mixture was stirred for 5 minutes at room temperature. Sodium triacetoxyborohydride (>80 wt%, 827 mg) was then added to the reaction mixture at room temperature, and the mixture was stirred for 19 hours and 25 minutes at room temperature. Water was added to the reaction mixture at room temperature, and extraction was performed with ethyl acetate.
The organic layer was washed with brine, dried over anhydrous magnesium sulfate and filtered, and the filtrate was concentrated under reduced pressure. The residue was purified by NH silica gel column chromatography (*n*-heptane:ethyl acetate = 6:4 to ethyl acetate) to obtain the title compound (941 mg).
ESI-MS (m/z): 432.41 $[M+H]^+$.

[Production Example 264-2]

*tert*-Butyl (4-methyl-[1,4'-bipiperidin]-4-yl)carbamate

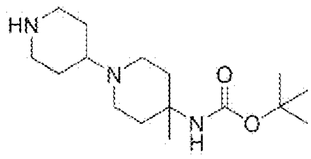

To a solution of the benzyl 4-((*tert*-butoxycarbonyl)amino)-4-methyl-[1,4'-bipiperidine]-1'-carboxylate of Production Example 264-1 (279 mg, 646 μmol) in methanol (10 mL) there was added 10% palladium-carbon (50% water, 138 mg) at room temperature, and the mixture was stirred for 2 hours and 25 minutes at room temperature and ordinary pressure, under a hydrogen atmosphere. After switching the reaction mixture to a nitrogen atmosphere, it was filtered with Celite and washed with ethyl acetate. The filtrate was concentrated under reduced pressure to obtain the title compound (192 mg).
ESI-MS (m/z): 298.30 $[M+H]^+$.

[Production Example 264-3]

Ethyl 2-(2-chloro-6-iodoquinazolin-4-yl)-2-cyclopropoxy-2-phenylacetate

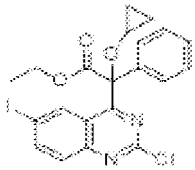

To a solution of the ethyl 2-cyclopropoxy-2-phenylacetate of Production Example 201-1 (661 mg, 3.00 mmol) and 2,4-dichloro-6-iodoquinazoline (1.17 g, 3.60 mmol) in THF (10 mL) there was added dropwise LHMDS (1.09 M THF solution, 3.30 mL, 3.60 mmol) at - 78°C under a nitrogen atmosphere, and the mixture was stirred for 10 minutes at -78°C. The reaction mixture was then stirred for 1 hour at room temperature. Saturated aqueous ammonium chloride was added to the reaction mixture, and extraction was performed with ethyl acetate. The organic layer was washed with brine and then dried over sodium sulfate and filtered. The filtrate was concentrated under reduced pressure and the residue was purified by NH silica gel column chromatography (*n*-heptane to *n*-heptane:ethyl acetate = 7:3) to obtain the title compound (1.35 g).

ESI-MS (m/z): 509.25 $[M+H]^+$.

[Production Example 264-4]

2-Chloro-4-(1-cyclopropoxy-1-phenyl-2-((tetrahydro-2H-pyran-2-yl)oxy)ethyl-6-iodoquinazoline

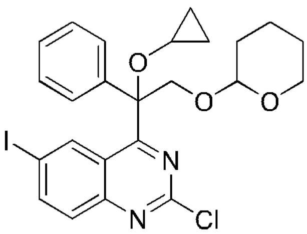

To a solution of the ethyl 2-(2-chloro-6-iodoquinazolin-4-yl)-2-cyclopropoxy-2-phenylacetate of Production Example 264-3 (1.08 g, 2.12 mmol) in dichloromethane (10 mL) there was added DIBAL-H (1.0 M toluene solution, 4.67 mL, 4.67 mmol) at -78°C, and the mixture was stirred for 10 minutes. The reaction mixture was increased in temperature to 0°C and stirred for 10 minutes. A saturated aqueous Rochelle salt solution was added to the reaction mixture, which was then stirred overnight at room temperature. The reaction mixture was extracted with ethyl acetate. The organic layer was washed with brine, dried over anhydrous sodium sulfate and filtered, and the filtrate was concentrated under reduced pressure. To a solution of the residue in dichloromethane (10 mL) there were added DHP (576 mL, 6.37 mmol) and CSA (99 mg, 0.43 mmol) at room temperature, and the mixture was stirred for 1 hour at room temperature. A saturated aqueous sodium hydrogencarbonate solution was added to the reaction mixture, and extraction was performed with ethyl acetate. The organic layer was washed with brine, dried over anhydrous sodium sulfate and filtered, and the filtrate was concentrated under reduced pressure. The residue was purified by NH silica gel column chromatography (*n*-heptane:ethyl acetate = 95:5 to 8:2) to obtain the title compound (733 mg).

ESI-MS (m/z): 551.25 $[M+H]^+$.

[Production Example 264-5]

5-(2-Chloro-4-(1-cyclopropoxy-1-phenyl-2-((tetrahydro-2H-pyran-2-yl)oxy)ethyl)quinazolin-6-yl)-4-methoxy-1-methylpyridin-2(1H)-one

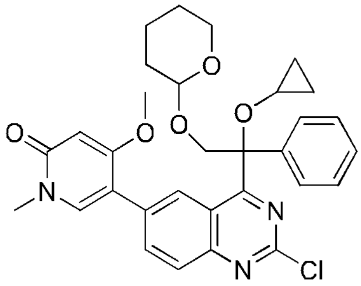

The title compound (99 mg) was obtained from the 2-chloro-4-(1-cyclopropoxy-1-phenyl-2-((tetrahydro-2H-pyran-2-yl)oxy)ethyl-6-iodoquinazoline of Production Example 264-4 (150 mg, 272 μmol) and the known compound 4-methoxy-1-methyl-5-(4,4,5,5-tetramethyl-1,3,2-dioxaborolan-2-yl)pyridin-2(1H)-one (94 mg, 0.35 mmol), by the same method as Production Example 201-5.
ESI-MS (m/z): 562.26 $[M+H]^+$.

[Production Example 264-6]

*tert*-Butyl (1'-(4-(1-cyclopropoxy-1-phenyl-2-((tetrahydro-2H-pyran-2-yl)oxyethyl)-6-(4-methoxy-1-methyl-6-oxo-1,6-dihydropyridin-3-yl)quinazolin-2-yl)-4-methyl-[1,4'-bipiperidin]-4-yl)carbamate

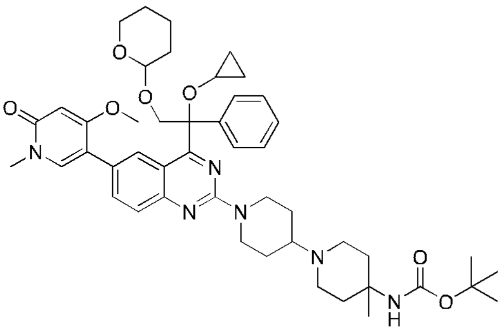

The title compound (19 mg) was obtained from the 5-(2-chloro-4-(1-cyclopropoxy-1-phenyl-2-((tetrahydro-2H-pyran-2-yl)oxy)ethyl)quinazolin-6-yl)-4-methoxy-1-methylpyridin-2(1H)-one of Production Example 264-5 (30 mg, 53 μmol) and the *tert*-butyl (4-methyl-[1,4'-bipiperidin]-4-yl)carbamate of Production Example 264-2 (28 mg, 95 μmol), by the same method as Production Example 265-7.
ESI-MS (m/z): 823.50 $[M+H]^+$.

[Production Example 264-7]

*tert*-Butyl (1'-(4-(1-cyclopropoxy-2-hydroxy-1-phenylethyl)-6-(4-methoxy-1-methyl-6-oxo-1,6-dihydropyrimidin-3-yl)quinazolin-2-yl}-4-methyl-[1,4'-bipiperidin]-4-yl)carbamate

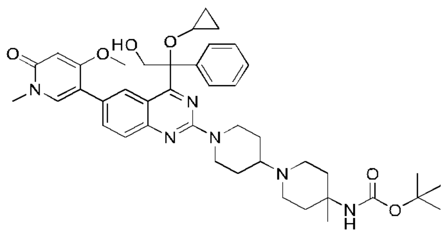

The title compound (239 mg) was obtained from the *tert*-butyl (1'-(4-(1-cyclopropoxy-1-phenyl-2-((tetrahydro-2H-pyran-2-yl)oxy)ethyl)-6-(4-methoxy-1-methyl-6-oxo-1,6-dihydropyridin-3-yl)quinazolin-2-yl)-4-methyl-[1,4'-bipiperidin]-4-yl)carbamate of Production Example 264-6 (288 mg, 350 μmol), by the same method as Production Example 268-2.

ESI-MS (m/z): 739.56 [M+H]+.

[Production Example 264-8]

2-(2-(4-((*tert*-Butoxycarbonyl)amino)-4-methyl-[1,4'-bipiperidin]-1'-yl)-6-(4-methoxy-1-methyl-6-oxo-1,6-dihydropyrimidin-3-yl)quinazolin-4-yl)-2-cyclopropoxy-2-phenylethyl methyl(2-(methylamino)ethyl)carbamate

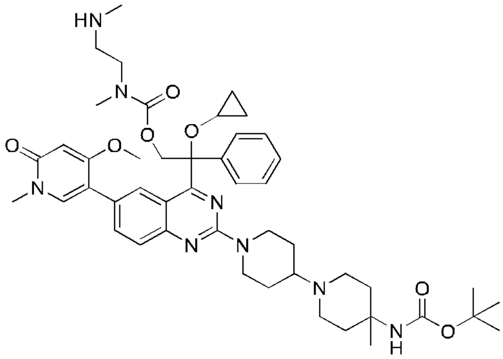

To a solution of the *tert*-butyl (1'-(4-(1-cyclopropoxy-2-hydroxy-1-phenylethyl)-6-(4-methoxy-1-methyl-6-oxo-1,6-dihydropyrimidin-3-yl)quinazolin-2-yl)-4-methyl-[1,4'-bipiperidin]4-yl)carbamate of Production Example 264-7 (189 mg, 256 μmol) and 4-dimethylaminopyridine (94 mg, 0.77 mmol) in dichloromethane (3 mL) there was added triphosgene (76 mg, 0.26 mmol) at room temperature, and the mixture was stirred for 10 minutes at room temperature. The reaction mixture was then added to a solution of N,N'-dimethylethylenediamine (550 μL, 5.12 mmol) in dichloromethane (3 mL) at room temperature, and the mixture was stirred for 35 minutes at room temperature. The reaction mixture was concentrated under reduced pressure and purified by NH silica gel column chromatography (*n*-heptane:ethyl acetate = 8:2 to ethyl acetate to ethyl acetate:methanol = 8:2) to obtain the title compound (151 mg).

ESI-MS (m/z): 853.69 [M+H]+.

[Production Example 264-9]

(2S,3R,4S,5S,6S)-2-(2-(3-((((9H-Fluoren-9-yl)methoxy)carbonyl)amino)propaneamide)-4-(11-(2-(4-((*tert*-butoxycarbonyl)amino)-4-methyl-[1,4'-bipiperidin]-1'-yl)-6-(4-methoxy-1-methyl-6-oxo-1,6-dihydropyrimidin-3-yl)quinazolin-4-yl)-11-cyclopropoxy-4,7-dimethyl-3,8-dioxo-11-phenyl-2,9-dioxa-4,7-diazaundecyl)phenoxy-6-(methoxycarbonyl)tetrahydro-2H-pyrane-3,4,5-triyl triacetate

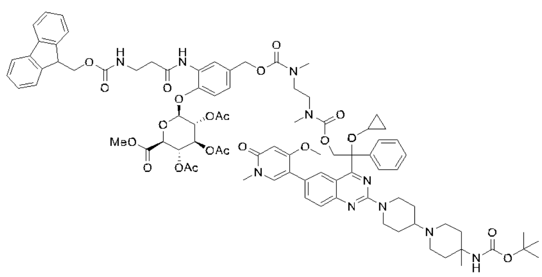

To a solution of the 2-(2-(4-((*tert*-butoxycarbonyl)amino)-4-methyl-[1,4'-bipiperidin]-1'-yl)-6-(4-methoxy-1-methyl-6-oxo-1,6-dihydropyrimidin-3-yl)quinazolin-4-yl)-2-cyclopropoxy-2-phenylethyl methyl(2-(methylamino)ethyl)carbamate of Production Example 264-8 (40 mg, 47 μmol) in DMF (2 mL) there was added N,N-diisopropylethylamine (82 μL, 0.47 mmol) and the known compound (2S,3R,4S,5S,6S)-2-(2-(3-((((9H-fluoren-9-yl)methoxy)carbonyl)amino)propaneamide)-4-((((4-nitrophenoxy)carbonyl)oxy)methyl)phenoxy)-6-(methoxycarbonyl)tetrahydro-2H-pyrane-3,4,5-triyl triacetate (47 mg, 52 μmol) at room temperature, and the mixture was stirred for 1 hour at room temperature. To the

reaction mixture there was added (2S,3R,4S,5S,6S)-2-(2-(3-((((9H-fluoren-9-yl)methoxy)carbonyl)amino)propaneamide)-4-((((4-nitrophenoxy)carbonyl)oxy)methyl)phenoxy)-6-(methoxycarbonyl)tetrahydro-2H-pyrane-3,4,5-triyl triacetate (47 mg, 52 μmol) at room temperature, and the mixture was stirred for 70 minutes at room temperature. Water was added to the reaction mixture, and extraction was performed with ethyl acetate. The organic layer was washed with water and brine, dried over anhydrous magnesium sulfate and filtered, and the filtrate was concentrated under reduced pressure. The residue was purified by NH silica gel column chromatography (*n*-heptane:ethyl acetate = 1:1 to ethyl acetate to ethyl acetate:methanol = 8:2) to obtain the title compound (72 mg).

ESI-MS (m/z): 1628.94 $[M+H]^+$.

[Production Example 264-10]

(2S,3S,4S,5R,6S)-6-(2-(3-Aminopropaneamide)-4-(11-(2-(4-((*tert*-butoxycarbonyl)amino)-4-methyl-[1,4'-bipiperidin]-1'-yl)-6-(4-methoxy-1-methyl-6-oxo-1,6-dihydropyridin-3-yl)quinazolin-4-yl)-11-cyclopropoxy-4,7-dimethyl-3,8-dioxo-11-phenyl-2,9-dioxa-4,7-diazaundecyl)phenoxy)-3,4,5-trihydroxytetrahydro-2H-pyran-2-carboxylic acid

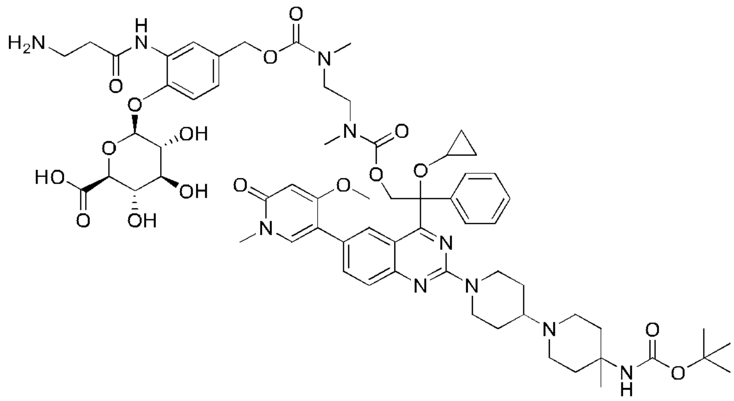

To a solution of the (2S,3R,4S,5S,6S)-2-(2-(3-((((9H-fluoren-9-yl)methoxy)carbonyl)amino)propaneamide)-4-(11-(2-(4-((*tert*-butoxycarbonyl)amino)-4-methyl-[1,4'-bipiperidin]-1'-yl)-6-(4-methoxy-1-methyl-6-oxo-1,6-dihydropyrimidin-3-yl)quinazolin-4-yl)-11-cyclopropoxy-4,7-dimethyl-3,8-dioxo-11-phenyl-2,9-dioxa-4,7-diazaundecyl)phenoxy)-6-(methoxycarbonyl)tetrahydro-2H-pyrane-3,4,5-triyl triacetate of Production Example 264-9 (72 mg, 44 μmol) in THF (1 mL), methanol (1 mL) and water (250 μL) there was added lithium hydroxide (10.6 mg, 442 μmol) at room temperature, and the mixture was stirred for 30 minutes at room temperature. After concentrating the reaction mixture under reduced pressure, the residue was purified by ODS silica gel chromatography (0.1% formic acid-containing water:0.1% formic acid-containing acetonitrile = 95:5 to 1:1), to obtain a formate of the title compound (49 mg).

ESI-MS (m/z): 1266.95 $[M+H]^+$.

[Production Example 264-11]

(2S,3S,4S,5R,6S)-6-(4-(11-(2-(4-((*tert*-Butoxycarbonyl)amino)-4-methyl-[1,4'-bipiperidin]-1'-yl)-6-(4-methoxy-1-methyl-6-oxo-1,6-dihydropyridin-3-yl)quinazolin-4-yl)-11-cyclopropoxy-4,7-dimethyl-3,8-dioxo-11-phenyl-2,9-dioxa-4,7-diazaundecyl)-2-(3-(3-(2-(2-(2,5-dioxo-2,5-dihydro-1H-pyrrolo-1-yl)ethoxy)ethoxy)propaneamide)propaneamide)phenoxy)-3,4,5-trihydroxytetrahydro-2H-pyran-2-carboxylic acid

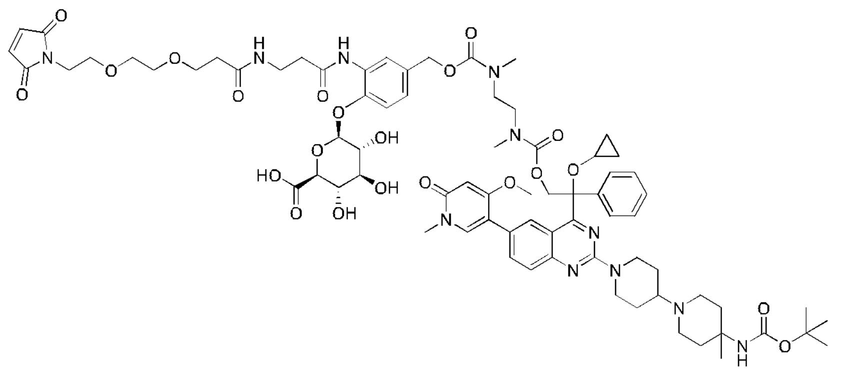

To a solution of the formate of (2S,3 S,4S,5R,6S)-6-(2-(3-aminopropaneamide)-4-(11-(2-(4-((*tert*-butoxycarbonyl)amino)-4-methyl-[1,4'-bipiperidin]-1'-yl)-6-(4-methoxy-1-methyl-6-oxo-1,6-dihydropyridin-3-yl)quinazolin-4-yl)-11-cyclopropoxy-4,7-dimethyl-3,8-dioxo-11-phenyl-2,9-dioxa-4,7-diazaundecyl)phenoxy)-3,4,5-trihydroxytetrahydro-2H-pyran-2-carboxylic acid of Production Example 264-10 (49 mg) in DMF (2 mL) there were added N,N-diisopropylethylamine (61 μL, 0.35 mmol) and 2,5-dioxopyrrolidin-1-yl 3-{2-[2-(2,5-dioxo-2,5-dihydro-1H-pyrrolo-1-yl)ethoxy]ethoxy} propanoate (12 mg, 35 μmol) at room temperature, and the mixture was stirred for 65 minutes at room temperature. The reaction mixture was purified by ODS silica gel column chromatography (0.1% formic acid-containing water:0.1% formic acid-containing acetonitrile = 95:5 to 1:1) to obtain a formate of the title compound (55 mg).

ESI-MS (m/z): 1505.85 $[M+H]^+$.

[Example 265]

4-(1-Cyclopropyl-2-(2-(4-(((2S,5R)-4-(3-(2,4-dioxotetrahydropyrimidine-1(2H)-yl)-4-methoxybenzoyl)-2,5-dimethylpiperazin-1-yl)methyl)piperidin-1-yl)-6-(4-methoxy-1-methyl-6-oxo-1,6-dihydropyridin-3-yl)quinazolin-4-yl)-3-hydroxypropan-2-yl)phenyl (4-((2S,5S)-15-(2,5-dioxo-2,5-dihydro-1H-pyrrol-1-yl)-5-isopropyl-4,7-dioxo-2-(3-ureidopropyl)-10,13-dioxa-3,6-diazapentadecanamido)benzyl)ethane-1,2-diylbis(methylcarbamate)

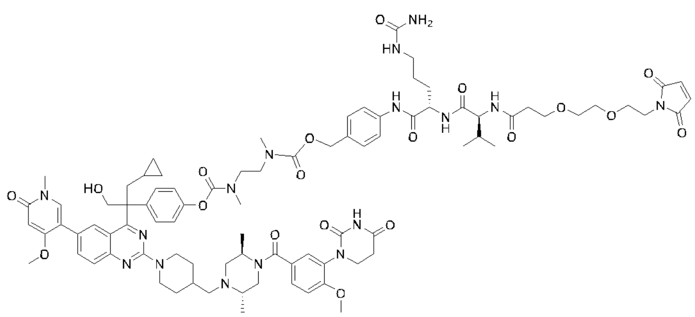

To a solution of the *tert*-butyl (2R,5S)-4-((1-(4-(1-cyclopropyl-2-(4-(((2-((((4-((2S,5S)-15-(2,5-dioxo-2,5-dihydro-1H-pyrrol-1-yl)-5-isopropyl-4,7-dioxo-2-(3-ureidopropyl)-10,13-dioxa-3,6-diazapentadecanamido)benzyl)oxy)carbonyl) (methyl)amino)ethyl) (methyl)carbamoyl)oxy)phenyl)-3-((tetrahydro-2H-pyran-2-yl)oxy)propan-2-yl)-6-(4-methoxy-1-methyl-6-oxo-1,6-dihydropyridin-3-yl)quinazolin-2-yl)piperidin-4-yl)methyl)-2,5-dimethylpiperazine-1-carboxylate of Production Example 265-11 (18.8 mg, 11.7 μmol) in dichloromethane (0.5 mL) there was added TFA (0.5 mL) at room temperature, and the mixture was stirred for 30 minutes at room temperature. The reaction mixture was concentrated under reduced pressure, and then dichloromethane and toluene were added to the residue and the mixture was again concentrated under reduced pressure. After repeating the same procedure again, N,N-diisopropylethylamine (20.4 μL, 117 μmol), the 3-(2,4-dioxotetrahydropyrimidine-1 (2H)-yl)-4-methoxybenzoic acid of Production Example 265-12 (4.6 mg, 18 μmol) and HATU (6.7 mg, 18 μmol) were added to a solution of the residue in DMF (0.5 mL) at room temperature, and the mixture was stirred overnight at room temperature. The reaction mixture was purified by direct reversed-phase silica gel chromatography (0.1% formic acid aqueous solution:0.1% acetonitrile formate solution = 90:10 to 65:35) to obtain a formate of the title compound (5.37 mg).

ESI-MS (m/z): 1673.03 $[M+H]^+$.

[Production Example 265-1]

Methyl 2-(4-(benzyloxy)phenyl)-3-cyclopropyl propanoate

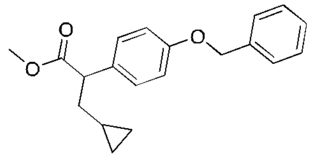

To a solution of methyl 2-[4-(benzyloxy)phenyl]acetate (2.50 g, 9.75 mmol) and cyclopropylmethyl bromide (1.14 mL, 11.7 mmol) in DMF (40 mL) there was added 50% to 72% sodium hydride oil (507 mg) while cooling on ice, and after stirring for 15 minutes, the mixture was further stirred for 4 hours at room temperature. Water was added to the reaction mixture, extraction was performed with ethyl acetate, and the organic layer was washed with water and brine. The organic layer was dried over magnesium sulfate and filtered, and the filtrate was concentrated under reduced pressure. The residue was purified by silica gel column chromatography (n-heptane to n-heptane:ethyl acetate = 94:6) to obtain the title compound (535 mg).

ESI-MS (m/z): 311.23 $[M+H]^+$.

[Production Example 265-2]

Methyl 2-(4-(benzyloxy)phenyl)-2-(2-chloro-6-iodoquinazolin-4-yl)-3-cyclopropyl propanoate

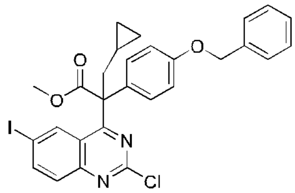

The title compound (715 mg) was obtained from the methyl 2-(4-(benzyloxy)phenyl)-3-cyclopropyl propanoate of Production Example 265-1 (543 mg, 1.75 mmol), by the same method as Production Example 201-2.

ESI-MS (m/z): 599.1 $[M+H]^+$.

[Production Example 265-3]

4-(2-(4-(Benzyloxy)phenyl)-1-cyclopropyl-3-((tetrahydro-2H-pyran-2-yl)oxy)propan-2-yl)-2-chloro-6-iodoquinazoline

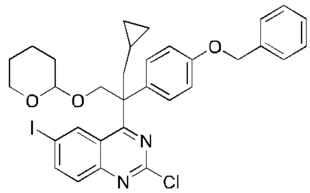

To a solution of the methyl 2-(4-(benzyloxy)phenyl)-2-(2-chloro-6-iodoquinazolin-4-yl)-3-cyclopropyl propanoate of Production Example 265-2 (715 mg, 1.19 mmol) in toluene (12 mL) there was added dropwise DIBAL (1.0 M toluene solution, 3.58 mL) at -78°C, and after directly stirring for 10 minutes, the reaction mixture was warmed to 0°C and further stirred for 2 hours. The reaction mixture was quenched with a saturated aqueous Rochelle salt solution, ethyl acetate was added, and the mixture was stirred for 2 hours at room temperature. The organic layer was separated off and the aqueous layer was extracted with ethyl acetate. The combined organic layers were washed with water and brine and then dried over

magnesium sulfate. After filtering the organic layer, it was concentrated under reduced pressure and the residue was dissolved in dichloromethane (15 mL). DHP (327 μL, 3.58 mmol) and p-toluenesulfonic acid monohydrate (91 mg, 0.478 mmol) were added to the reaction mixture, and the mixture was stirred for 2 hours at room temperature. The reaction mixture was purified by direct silica gel column chromatography (n-heptane:ethyl acetate = 83:17 to 66:34) to obtain the title compound (535 mg).

$^1$H-NMR Spectrum (500 MHz, $CDCl_3$) δ(ppm): -0.42 (1H, tq, J=10.1, 4.9 Hz), 0.02-0.19 (2H, m), 0.23-0.40 (2H, m), 1.09-1.53 (6H, m) 2.12-2.38 (1H, m), 2.53-2.69 (1H, m), 2.79-2.90 (0.5H, m), 3.14-3.24 (0.5H, m), 3.32-3.47 (1H, m), 4.10 (0.5H, d, J=9.8 Hz), 4.26-4.33 (1H, m), 4.48 (0.5H, d, J=9.8 Hz), 4.69 (0.5H, brs), 4.81 (0.5H, d, J=10.4 Hz), 5.09 (2H, s), 6.89-6.97 (2H, m), 7.09 (2H, d, J=8.6 Hz), 7.29-7.47 (5H, m), 7.63 (1H, dd, J=8.6, 1.8 Hz), 7.88-7.96 (2H, m).

[Production Example 265-4]

5-(4-(2-(4-(Benzyloxy)phenyl)-1-cyclopropyl-3-((tetrahydro-2H-pyran-2-yl)oxy)propan-2-yl)-2-chloroquinazolin-6-yl)-4-methoxy-1-methylpyridin-2(1H)-one

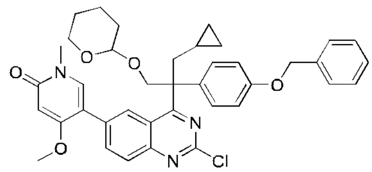

The title compound (105 mg) was obtained from the 4-(2-(4-(benzyloxy)phenyl)-1-cyclopropyl-3-((tetrahydro-2H-pyran-2-yl)oxy)propan-2-yl)-2-chloro-6-iodoquinazoline of Production Example 265-3 (300 mg, 458 μmol) and the known compound 4-methoxy-1-methyl-5-(4,4,5,5-tetramethyl-1,3,2-dioxaborolan-2-yl)pyridin-2(1H)-one (146 mg, 551 μmol), by the same method as Production Example 201-5.

ESI-MS (m/z): 667.46 $[M+H]^+$.

[Production Example 265-5]

*tert*-Butyl (2R,5S)-4-((1-((benzyloxy)carbonyl)piperidin-4-yl)methyl)-2,5-dimethylpiperazine-1-carboxylate

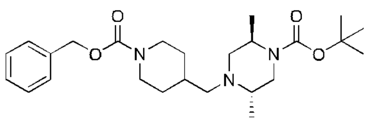

To a solution of *tert*-butyl (2R,5S)-2,5-dimethylpiperazine-1-carboxylate (3.90 g, 18.2 mmol) in dichloromethane (90 mL) and acetic acid (69 μL, 1.2 mmol) there was added 4-formyl-N-CBZ-piperidine (3.00 g, 12.1 mmol) at room temperature, and the mixture was stirred for 30 minutes at room temperature. Sodium triacetoxyborohydride (7.71 g, 36.4 mmol) was then added and the mixture was stirred for 36 hours and 20 minutes at room temperature. A saturated aqueous sodium hydrogencarbonate solution was added to the reaction mixture while cooling on ice, and the mixture was stirred for 30 minutes at room temperature and then extracted with chloroform. The organic layer was dried over magnesium sulfate and filtered, and the filtrate was concentrated under reduced pressure. The residue was purified by NH silica gel column chromatography (*n*-heptane:ethyl acetate = 4:1 to 1:1) to obtain the title compound (5.12 g).

ESI-MS (m/z): 446.37 $[M+H]^+$.

[Production Example 265-6]

*tert*-Butyl (2R,5S)-2,5-dimethyl-4-(piperidin-4-ylmethyl)piperazine-1-carboxylate

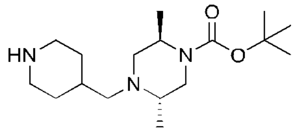

To a solution of the *tert*-butyl (2R,5S)-4-((1-((benzyloxy)carbonyl)piperidin-4-yl)methyl-2,5-dimethylpiperazine-1-carboxylate of Production Example 265-5 (5.12 g, 11.5 mmol) in THF (100 mL) there was added 10% palladium-carbon (50% water, 9.78 g) at room temperature, and the mixture was stirred for 66 hours at room temperature and ordinary pressure under a hydrogen atmosphere. The reaction mixture was filtered with Celite and washed with methanol. The filtrate was concentrated under reduced pressure to obtain a crude product of the title compound (3.02 g).
ESI-MS (m/z): 312.34 $[M+H]^+$.

[Production Example 265-7]

*tert*-Butyl(2R,5S)-4-((1-(4-(2-(4-(benzyloxy)phenyl)-1-cyclopropyl-3-((tetrahydro-2H-pyran-2-yl)oxy)propan-2-yl)-6-(4-methoxy-1-methyl-6-oxo-1,6-dihydropyridin-3-yl)quinazolin-2-yl)piperidin-4-yl)methyl)-2,5-dimethylpiperazine-1-carboxylate

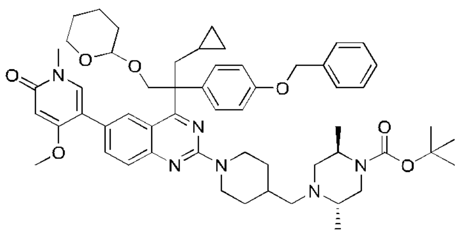

To a solution of the 5-(4-(2-(4-(benzyloxy)phenyl)-1-cyclopropyl-3-((tetrahydro-2H-pyran-2-yl)oxy)propan-2-yl)-2-chloroquinazolin-6-yl)-4-methoxy-1-methylpyridin-2(1H)-one of Production Example 265-4 (90 mg, 0.14 mmol) in DMF (1 mL) there were added N,N-diisopropylamine (71 μL, 0.41 mmol) and the *tert*-butyl (2R,5S)-2,5-dimethyl-4-(piperidin-4-ylmethyl)piperazine-1-carboxylate of Production Example 265-6 (58.9 mg, 189 μmol), and the mixture was stirred for 6 hours at 80°C. The reaction mixture was diluted with ethyl acetate and water, and then the aqueous layer was extracted with ethyl acetate. The organic layer was washed with water and brine and then dried over magnesium sulfate. The organic layer was filtered, and the filtrate was concentrated under reduced pressure. The residue was purified by NH silica gel column chromatography (n-heptane:ethyl acetate = 1:3 to ethyl acetate to ethyl acetate:methanol = 9: 1) to obtain the title compound (111 mg). ESI-MS (m/z): 942.81 $[M+H]^+$.

[Production Example 265-8]

*tert*-Butyl (2R,5 S)-4-((1-(4-(1-cyclopropyl-2-(4-hydroxyphenyl)-3-((tetrahydro-2H-pyran-2-yl)oxy)propan-2-yl)-6-(4-methoxy-1-methyl-6-oxo-1,6-dihydropyridin-3-yl)quinazolin-2-yl)piperidin-4-yl)methyl)-2,5-dimethylpiperazine-1-carboxylate

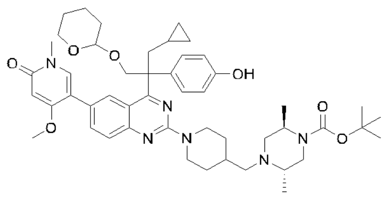

To a solution of the *tert*-butyl (2R,5S)-4-((1-(4-(2-(4-(benzyloxy)phenyl)-1-cyclopropyl-3-((tetrahydro-2H-pyran-2-yl)oxy)propan-2-yl)-6-(4-methoxy-1-methyl-6-oxo-1,6-dihydropyridin-3-yl)quinazolin-2-yl)piperidin-4-yl) methyl)-2,5-dimethylpiperazine-1-carboxylate of Production Example 265-7 (110 mg, 0.117 mmol) in methanol (2 mL) there was added 10% palladium-carbon (50% water, 49.7 mg), and the mixture was stirred for 26 hours at room

temperature and ordinary pressure under a hydrogen atmosphere. The reaction mixture was diluted with chloroform and methanol and then filtered with Celite, and the filtrate was concentrated under reduced pressure to obtain a crude product of the title compound (108 mg).

ESI-MS (m/z): 852.71 [M+H]+.

[Production Example 265-9]

*tert*-Butyl (2R,5S)-4-((1-(4-(1-cyclopropyl-2-(4-(((4-nitrophenoxy)carbonyl)oxy)phenyl)-3-((tetrahydro-2H-pyran-2-yl)oxy)propan-2-yl)-6-(4-methoxy-1-methyl-6-oxo-1,6-dihydropyridin-3-yl)quinazolin-2-yl)piperidin-4-yl)methyl)-2,5-dimethylpiperazine-1-carboxylate

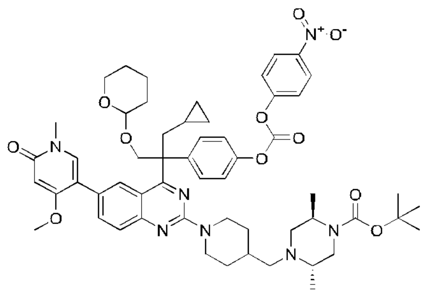

To a solution of the *tert*-butyl (2R,5S)-4-((1-(4-(1-cyclopropyl-2-(4-hydroxyphenyl)-3-((tetrahydro-2H-pyran-2-yl)oxy)propan-2-yl)-6-(4-methoxy-1-methyl-6-oxo-1,6-dihydropyridin-3-yl)quinazolin-2-yl)piperidin-4-yl)methyl)-2,5-dimethylpiperazine-1-carboxylate of Production Example 265-8 (25 mg, 29 μmol) in DMF (0.5 mL) there were added bis(4-nitrophenyl) carbonate (17.9 mg, 58.7 μmol) and N,N-diisopropylethylamine (20.5 μL, 117 μmol), and the mixture was stirred for 4 hours at room temperature. The reaction mixture was diluted with ethyl acetate and water, the aqueous layer was extracted with ethyl acetate, and the organic layer was washed with water and brine. The organic layer was dried over magnesium sulfate and filtered, and the filtrate was concentrated under reduced pressure. The residue was purified by silica gel thin-layer chromatography (chloroform:methanol = 20: 1) to obtain a crude product of the title compound (32 mg). ESI-MS (m/z): 1017.67 [M+H]+.

[Production Example 265-10]

*tert*-Butyl (4-((2S,5S)-15-(2,5-dioxo-2,5-dihydro-1H-pyrrol-1-yl)-5-isopropyl-4,7-dioxo-2-(3-ureidopropyl)-10,13-dioxa-3,6-diazapentadecanamido)benzyl)ethane-1,2-diylbis(methylcarbamate)

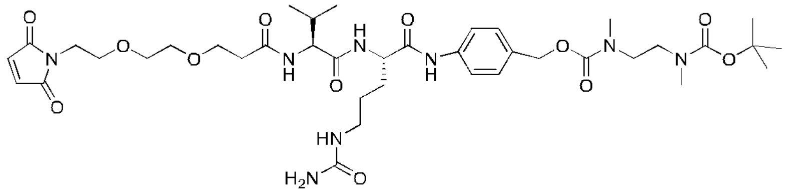

To a solution of the known compound (4-((2S)-5-(carbamoylamino)-2-((2S)-2-(3-(2-(2-(2,5-dioxo-2,5-dihydro-1H-pyrrol-1-yl)ethoxy)ethoxy)propanamido)-3-methyl butanamide)pentanamido)phenyl)methyl 4-nitrophenyl carbonate (50 mg, 64 μmol) in DMF (0.5 mL)) there were added *tert*-butyl methyl(2-(methylamino)ethyl)carbamate (12 mg, 64 μmol) and N,N-diisopropylethylamine (33.4 μl, 191 μmol) while cooling on ice, and the mixture was stirred for 30 minutes. The reaction mixture was purified by direct reversed-phase silica gel chromatography (0.1% formic acid aqueous solution:0.1% acetonitrile formate solution = 9:1 to 1:1) to obtain a crude product of the title compound (40 mg). ESI-MS (m/z): 834.55 [M+H]+.

[Production Example 265-11]

*tert*-Butyl (2R,5S)-4-((1-(4-(1-cyclopropyl-2-(4-(((2-((((4-((2S,5S)-15-(2,5-dioxo-2,5-dihydro-1H-pyrrol-1-yl)-5-isopropyl-4,7-dioxo-2-(3-ureidopropyl)-10,13-dioxa-3,6-diazapentadecanamido)benzyl)oxy)carbonyl) (methyl)amino)ethyl) (methyl)carbamoyl)oxy)phenyl)-3-((tetrahydro-2H-pyran-2-yl)oxy)propan-2-yl)-6-(4-methoxy-1-methyl-6-oxo-1,6-dihydropyridin-3-yl)quinazolin-2-yl)piperidin-4-yl)methyl)-2,5-dimethylpiperazine-1-carboxylate

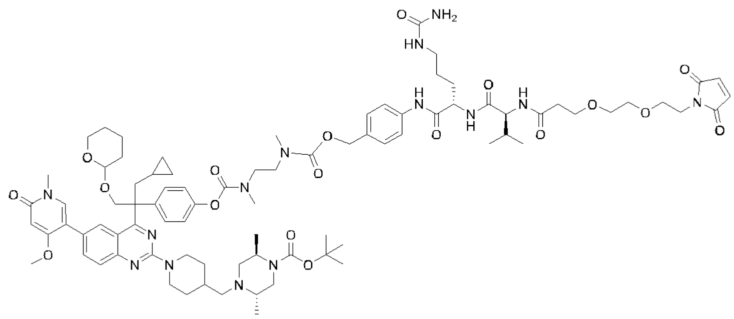

To a solution of the *tert*-butyl (4-((2S,5S)-15-(2,5-dioxo-2,5-dihydro-1H-pyrrol-1-yl)-5-isopropyl-4,7-dioxo-2-(3-ureidopropyl)-10,13-dioxa-3,6-diazapentadecanamido)benzyl)ethane-1,2-diylbis(methylcarbamate) of Production Example 265-10 (38 mg, 46 μmol) in dichloromethane (0.5 mL) there was added TFA(0.5 mL, 6.5 mmol), and the mixture was stirred for 30 minutes at room temperature. The reaction mixture was concentrated under reduced pressure and the residue was dissolved in toluene and dichloromethane and again concentrated under reduced pressure. The same procedure was repeated twice, and the residue was dissolved in DMF (0.5 mL). To the reaction mixture there was added a solution of *tert*-butyl (2R,5S)-4-((1-(4-(1-cyclopropyl-2-(4-(((4-nitrophenoxy)carbonyl)oxy)phenyl)-3-((tetrahydro-2H-pyran-2-yl)oxy)propan-2-yl)-6-(4-methoxy-1-methyl-6-oxo-1,6-dihydropyridin-3-yl)quinazolin-2-yl)piperidin-4-yl) methyl)-2,5-dimethylpiperazine-1-carboxylate (25 mg, 25 μmol) and N,N-diisopropylethylamine (43.0 μL, 246 μmol) mixed in DMF (0.5 mL) while cooling on ice, and the mixture was stirred for 40 minutes at room temperature. The reaction mixture was purified by direct reversed-phase silica gel chromatography (0.1% formic acid aqueous solution:0.1% acetonitrile formate solution = 9:1 to 1:1) to obtain a formate of the title compound (19 mg). ESI-MS (m/z): 1611.08 [M+H]+.

[Production Example 265-12]

3-(2,4-Dioxotetrahydropyrimidine-1(2H)-yl)-4-methoxybenzoic acid

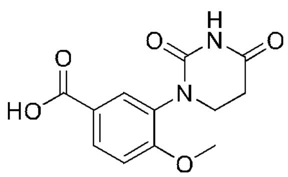

A solution of 3-amino-4-methoxybenzoic acid (2.5 g, 15 mmol) in acrylic acid (4 mL) was stirred for 3 hours at 100°C. The reaction mixture was cooled to room temperature, and then acetic acid (16 mL) was added and the mixture was stirred for 20 minutes at 100°C. Urea (5.5 g, 92 mmol) was then added and the mixture was stirred overnight at 110°C. The reaction mixture was cooled on ice, concentrated hydrochloric acid was added, and the mixture was stirred for 20 minutes while cooling on ice. The reaction mixture was allowed to stand for 2 hours at 4°C, and the obtained solid was filtered and washed with water, and then dried. After adding an aqueous 1 M hydrochloric acid solution to the obtained solid and crushing and filtering the solid, it was washed with TBME and dried to obtain the title compound (2.36 g).

ESI-MS (m/z): 265.12 [M+H]+.

[Example 266]

(2S)-N-(2-Cyclopropoxy-2-(2-(4-((2S,5R)-4-(3-(2,4-dioxotetrahydropyrimidine-1(2H)-yl)-4-methoxybenzoyl)-2,5-dimethylpiperazin-1-yl)piperidin-1-yl)-6-(6-methyl-7-oxo-6,7-dihydro-1H-pyrrolo[2,3-c]pyridin-4-yl)quinazolin-4-yl)-2-phenylethyl)-2-((S)-2-(3-(2-(2-(2,5-dioxo-2,5-dihydro-1H-pyrrol-1-yl)ethoxy)ethoxy)propanamido)-3-methyl butanamide)-5-ureidopentaneamide

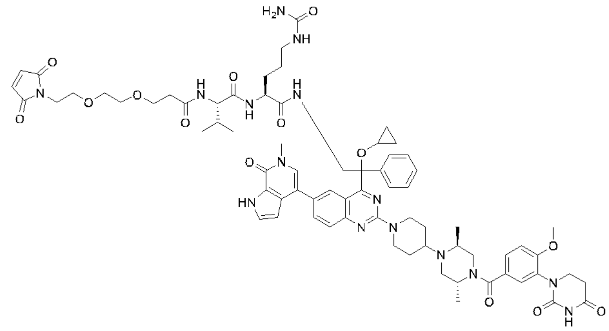

To a solution of a crude product of the 1-(5-((2R,5S)-4-(1-(4-(2-amino-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carbonyl)-2-methoxyphenyl)dihydropyrimidine-2,4(1H,3H)-dione of Production Example 266-7 (5.0 mg), Fmoc-Val-Cit-OH (3.34 mg, 6.72 μmol) and HATU (2.55 mg, 6.72 μmol) in DMF (300 μL) there was added N,N-diisopropylethylamine (2.45 μL, 14.0 μmol), and the mixture was stirred for 1 hour at room temperature. Diethylamine (5.8 μL, 56 μmol) was added to the reaction mixture, and stirring was continued for 30 minutes at room temperature. The reaction mixture was concentrated under reduced pressure, and then N,N-diisopropylethylamine (2.45 μL, 14.0 μmol) and MAL-PEG2-NHS ester (4.96 mg, 14.0 μmol) were added to a solution of the residue in DMF (300 μL), and the mixture was stirred for 1 hour at room temperature. The reaction mixture was purified by direct reversed-phase silica gel chromatography (0.1% formic acid aqueous solution:0.1% acetonitrile formate solution = 85:15 to 1:1), and the obtained crude product of the title compound was further purified by silica gel thin-layer chromatography (chloroform:methanol = 10:1) to obtain a crude product of the title compound (2.7 mg).
ESI-MS (m/z): 1389.51 $[M+H]^+$.

[Production Example 266-1]

*tert*-Butyl (2R,5S)-4-(1-((benzyloxy)carbonyl)piperidin-4-yl)-2,5-dimethylpiperazine-1-carboxylate

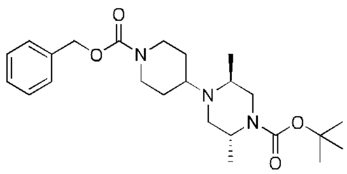

To a solution of *tert*-butyl (2R,5S)-2,5-dimethylpiperazine-1-carboxylate (4.82 g, 22.5 mmol) in dichloromethane (100 mL) and acetic acid (86 μL, 1.50 mmol) there was added 1-(benzyloxycarbonyl)-4-piperidinone (3.50 g, 15.0 mmol) at room temperature, and the mixture was stirred for 10 minutes at room temperature. Sodium triacetoxyborohydride (9.54 g, 45.0 mmol) was then added and the mixture was stirred for 99 hours at room temperature. A saturated aqueous sodium hydrogencarbonate solution was added to the reaction mixture at 0°C, and the mixture was stirred for 30 minutes and then extracted with chloroform. The organic layer was dried over magnesium sulfate and filtered, and the filtrate was concentrated under reduced pressure. The residue was purified by NH silica gel column chromatography (n-heptane:ethyl acetate = 4:1 to 1:1) to obtain the title compound (4.29 g).
ESI-MS (m/z): 432.79 $[M+H]^+$.

[Production Example 266-2]

*tert*-Butyl (2R,5S)-2,5-dimethyl-4-(piperidin-4-yl)piperazine-1-carboxylate

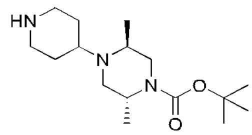

To a solution of the *tert-butyl* (2R,5S)-4-(1-((benzyloxy)carbonyl)piperidin-4-yl)-2,5-dimethylpiperazine-1-carboxylate of Production Example 266-1 (4.29 g, 9.94 mmol) in THF (25 mL) and ethyl acetate (25 mL) there was added 10% palladium-carbon (50% water, 8.46 g) at room temperature, and the mixture was stirred for 22 hours and 35 minutes at room temperature and ordinary pressure under a hydrogen atmosphere. After switching the reaction mixture to a nitrogen atmosphere, it was filtered with Celite and washed with methanol. The filtrate was concentrated under reduced pressure to obtain the title compound (2.81 g).
ESI-MS (m/z): 298.22 $[M+H]^+$.

[Production Example 266-3]

*tert*-Butyl (2R,5S)-4-(1-(4-(1-cyclopropoxy-2-hydroxy-1-phenylethyl)-6-iodoquinazolin-2-yl)piperidin-4-yl)-2,5-dimethylpiperazine-1-carboxylate

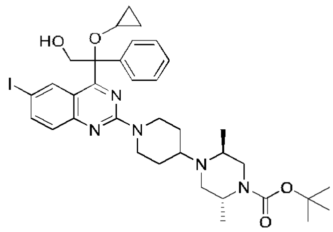

To a solution of the 2-chloro-4-(1-cyclopropoxy-1-phenyl-2-((tetrahydro-2H-pyran-2-yl)oxy)ethyl)-6-iodoquinazoline of Production Example 264-4 (4.6 g, 8.4 mmol) and the *tert*-butyl (2R,5 S)-2,5-dimethyl-4-(piperidin-4-yl)piperazine-1-carboxylate of Production Example 266-2 (2.86 g, 9.60 mmol) in DMF (30 mL) there was added N,N-diisopropylethylamine (3.65 mL, 20.9 mmol), and the mixture was stirred overnight at 80°C. Water was added to the reaction mixture, and extraction was performed with ethyl acetate. The organic layer was washed with water and brine, and then the organic layer was dried over anhydrous magnesium sulfate and filtered, and the filtrate was concentrated under reduced pressure to obtain a crude product. To a solution of the obtained crude product in methanol (30 mL)/dichloromethane (5 mL) there was added *p*-toluenesulfonic acid monohydrate (3.97 g, 20.9 mmol), and the mixture was stirred for 4 hours at room temperature. After adding N,N-diisopropylethylamine (7.29 mL, 41.8 mmol) to the reaction mixture, the mixture was concentrated under reduced pressure. The residue was purified by NH silica gel column chromatography (*n*-heptane:ethyl acetate = 1:1 to 1:3) to obtain the title compound (6.1 g).
ESI-MS (m/z): 728.91 $[M+H]^+$.

[Production Example 266-4]

*tert*-Butyl (2R,5 S)-4-(1-(4-(1-cyclopropoxy-2-hydroxy-1-phenylethyl)-6-(6-methyl-7-oxo-1-tosyl-6,7-dihydro-1H-pyrrolo [2,3-c]pyridin-4-yl)quinazolin-2-yl)piperidin-4-yl)-2,5-dimethylpiperazine-1-carboxylate

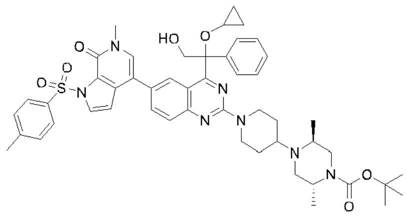

The title compound (983 mg) was obtained from the *tert*-butyl (2R,5S)-4-(1-(4-(1-cyclopropoxy-2-hydroxy-1-phenylethyl)-6-iodoquinazolin-2-yl)piperidin-4-yl)-2,5-dimethylpiperazine-1-carboxylate of Production Example 266-3 (1.00 g, 1.37 mmol), by the same method as Production Example 201-5.

ESI-MS (m/z): 902.97 $[M+H]^+$.

[Production Example 266-5]

*tert*-Butyl (2R,5S)-4-(1-(4-(2-amino-1-cyclopropoxy-1-phenylethyl)-6-(6-methyl-7-oxo-1-tosyl-6,7-dihydro-1H-pyrrolo [2,3-c]pyridin-4-yl)quinazolin-2-yl)piperidin-4-yl)-2,5-dimethylpiperazine-1-carboxylate

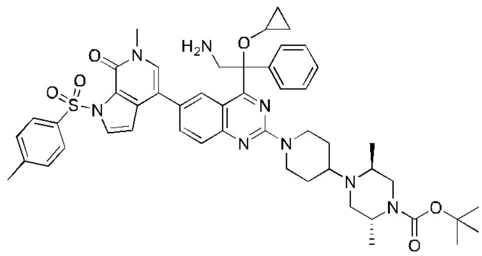

To a solution of the *tert-butyl* (2R,5S)-4-(1-(4-(1-cyclopropoxy-2-hydroxy-1-phenylethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-carboxylate of Production Example 266-4 (650 mg, 0.721 mmol) in dichloromethane (3.5 mL) there was added methanesulfonyl chloride (112 μL, 1.44 mmol) while cooling on ice, and the mixture was stirred for 10 minutes, and then further stirred for 20 minutes at room temperature. The reaction mixture was quenched with water and extracted with dichloromethane. The organic layer was dried over sodium sulfate and filtered, and the filtrate was concentrated under reduced pressure. The residue was dissolved in NMP (7 mL), 28% ammonia water (5 mL) was added, and reaction was conducted for 30 minutes at 130°C using a microwave synthesizer. The microwave synthesizer was again used for reaction for 15 minutes at 130°C, and the reaction mixture was diluted with ethyl acetate and water. After extraction with ethyl acetate, the organic layer was washed with water and brine and dried over magnesium sulfate. After filtration, the filtrate was concentrated under reduced pressure to obtain a partially purified product of the title compound (645 mg).

ESI-MS (m/z): 902.42 $[M+H]^+$.

[Production Example 266-6]

*tert*-Butyl (2R,5S)-4-(1-(4-(2-((((9H-fluoren-9-yl)methoxy)carbonyl)amino)-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate

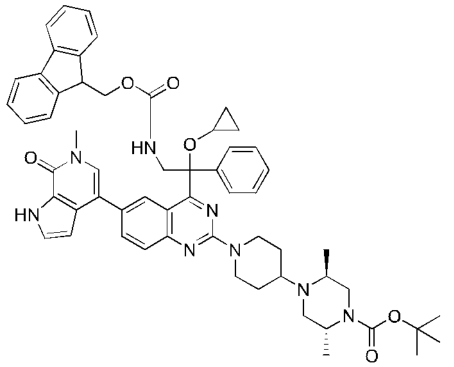

To a solution of the partially purified *tert*-butyl(2R,5S)-4-(1-(4-(2-amino-1-cyclopropoxy-1-phenylethyl)-6-(6-methyl-7-oxo-1-tosyl-6,7-dihydro-1H-pyrrolo[2,3-c]pyridin-4-yl)quinazolin-2-yl)piperidin-4-yl)-2,5-dimethylpiperazine-1-carboxylate of Production Example 266-5 (645 mg) in THF (2 mL)/methanol (2 mL) there was added a 2 M sodium hydroxide aqueous solution (2 mL), and the mixture was stirred for 3 hours at 50°C. The reaction mixture was diluted with dichloromethane and water and extracted with dichloromethane, and then the organic layer was dried over magnesium sulfate. After filtration, the filtrate was concentrated under reduced pressure. The residue was dissolved in dichloromethane (5 mL), and then 9-fluorenemethyl succinimidyl carbonate (314 mg, 931 μmol) and N,N-diisopropylethylamine (250 μL, 1.43 mmol) were added and the mixture was stirred overnight at room temperature. The reaction mixture was purified by direct NH-silica gel column chromatography (*n*-heptane:ethyl acetate = 30:70 to ethyl acetate to ethyl acetate:methanol(95:5)) to obtain the title compound (515 mg).
ESI-MS (m/z): 970.28 $[M+H]^+$.

[Production Example 266-7]

1-(5-((2R,5S)-4-(1-(4-(2-Amino-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carbonyl)-2-methoxyphenyl)dihydropyrimidine-2,4(1H,3H)-dione

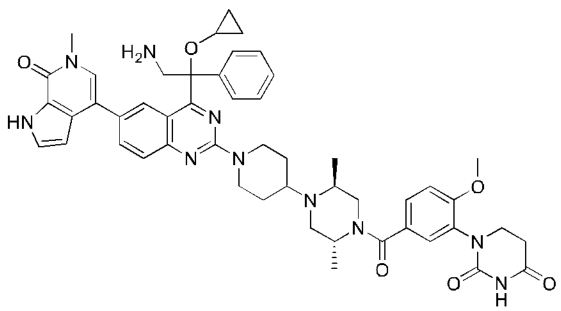

After reacting the *tert*-butyl(2R,5S)-4-(1-(4-(2-((((9H-fluoren-9-yl)methoxy)carbonyl)amino)-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)-2,5-dimethylpiperazine-1-carboxylate of Production Example 266-6 (410 mg, 0.423 mmol) and the 3-(2,4-dioxotetrahydropyrimidine-1(2H)-yl)-4-methoxybenzoic acid of Production Example 265-12 (168 mg, 635 μmol) by the same method as Example 265, diethylamine (656 μL, 6.35 mmol) was added to the reaction mixture, which was then stirred for 1 hour at room temperature. The reaction mixture was purified by direct reversed-phase silica gel chromatography (0.1% formic acid aqueous solution:0.1% acetonitrile formate solution = 1:9 to 1:1), and further purified by NH silica gel thin-layer chromatography (chloroform:methanol = 20:1), to obtain a partially purified product of the title compound (77 mg).
ESI-MS (m/z): 447.43 $[(M+2H)/2]^+$.

[Example 267]

{2-Cyclopropoxy-2-[2-(4-{[(2S,5R)-4-[3-(2,4-dioxo-1,3-diazinane-1-yl)-4-methoxybenzoyl]-2,5-dimethylpiperazin-1-yl]methyl}piperidin-1-yl)-6-{6-methyl-7-oxo-1H,6H,7H-pyrrolo[2,3-c]pyridin-4-yl}quinazolin-4-yl]-2-phenylethoxy}[({2-[6-(2,5-dioxo-2,5-dihydro-1H-pyrrol-1-yl)hexanamido]ethoxy}(hydroxy)phosphoryl)oxy]phosphinic acid

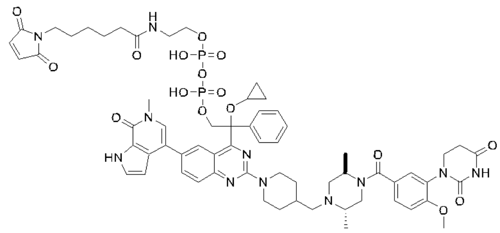

To a solution of the crude (9H-fluoren-9-yl)methyl (2-(((((2-cyclopropoxy-2-(2-(4-(((2S,5R)-4-(3-(2,4-dioxotetrahydropyrimidine-1(2H)-yl)-4-methoxybenzoyl)-2,5-dimethylpiperazin-1-yl)methyl)piperidin-1-yl)-6-(6-methyl-7-oxo-6,7-dihydro-1H-pyrrolo[2,3-c]pyridin-4-yl)quinazolin-4-yl)-2-phenylethoxy) (hydroxy)phosphoryl)oxy) (hydroxy) phosphoryl)oxy)ethyl)carbamate of Production Example 267-4 (12.2 mg) in DMF (1.0 mL) there was added diethylamine (38.2 μL, 0.366 mmol), and the mixture was stirred for 20 minutes. The reaction mixture was concentrated under reduced pressure and processed twice by azeotropic distillation with toluene (1 mL) to obtain an intermediate. To a solution of the obtained intermediate in DMF (1.0 mL) there were added N,N-diisopropylethylamine (15.9 μL, 0.091 mmol) and N-succinimidyl 6-maleimidecaproate (3.67 mg, 0.012 mmol) at room temperature, and the mixture was stirred for 40 minutes at room temperature. The reaction mixture was concentrated under reduced pressure and the residue was purified by ODS silica gel column chromatography (0.1% formic acid-containing water:0.1% formic acid-containing acetonitrile = 99:1 to 3:7), and then purified by silica gel thin-layer chromatography ((chloroform:methanol = 3:1) to obtain the title compound (1.52 mg).

ESI-MS (m/z): 1304.07 $[M+H]^+$.

[Production Example 267-1]

*tert-Butyl* (2R,5 S)-4-((1-(4-(1-cyclopropoxy-1-phenyl-2-(phosphonooxy)ethyl)-6-(6-methyl-7-oxo-1-tosyl-6,7-dihydro-1H-pyrrolo[2,3-c]pyridin-4-yl)quinazolin-2-yl)piperidin-4-yl)methyl)-2,5-dimethylpiperazine-1-carboxylate

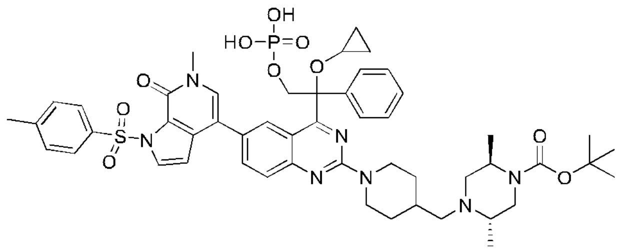

To a solution of the *tert*-butyl (2R,5S)-4((1-(4-(1-cyclopropoxy-2-hydroxy-1-phenylethyl)-6-(6-methyl-7-oxo-1-tosyl-6,7-dihydro-1H-pyrrolo[2,3-c]pyridin-4-yl)quinazolin-2-yl)piperidin-4-yl)methyl)-2,5-dimethylpiperazine-1-carboxylate of Production Example 268-2 (70 mg, 0.076 mmol) and pyridine (61.8 μL, 0.764 mmol) in THF (1.0 mL) there was added phosphoryl chloride (71.2 μL, 0.764 mmol), and the mixture was stirred for 1 hour and 30 minutes. Cold water was added to the reaction mixture to halt the reaction, and then extraction was performed with dichloromethane and the organic layer was dried over sodium sulfate and concentrated to obtain a crude product of the title compound (88 mg).

ESI-MS (m/z): 999.66 $[M+H]^+$.

[Production Example 267-2]

*tert*-Butyl (2R,5S)-4-((1-(4-(1-cyclopropoxy-1-phenyl-2-(phosphonooxy)ethyl)-6-(6-methyl-7-oxo-6,7-dihydro-1H-pyrrolo[2,3-c]pyridin-4-yl)quinazolin-2-yl)piperidin-4-yl)methyl)-2,5-dimethylpiperazine-1-carboxylate

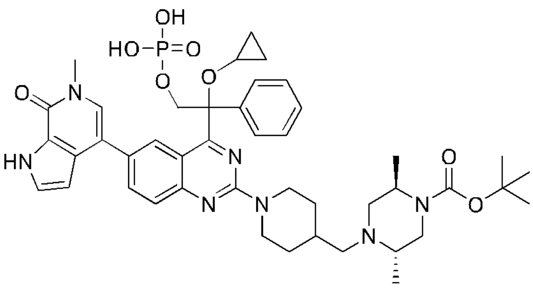

A formate of the title compound (53 mg) was obtained from the crude *tert*-butyl (2R,5 S)-4-((1-(4-(1-cyclopropoxy-1-phenyl-2-(phosphonooxyethyl)-6-(6-methyl-7-oxo-1-tosyl-6,7-dihydro-1H-pyrrolo[2,3-c]pyridin-4-yl)quinazolin-2-yl)piperidin-4-yl)methyl)-2,5-dimethylpiperazine-1-carboxylate of Production Example 267-1 (76 mg) by the same method as Production Example 201-9.
ESI-MS (m/z): 843.00 $[M+H]^+$.

[Production Example 267-3]

*tert*-Butyl (2R,5S)-4-((1-(4-(2-(((((2-((((9H-fluoren-9-yl)methoxy)carbonyl)amino)ethoxy) (hydroxy)phosphoryl)oxy) (hydroxy)phosphoryl)oxy)-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)methyl)-2,5-dimethylpiperazine-1-carboxylate

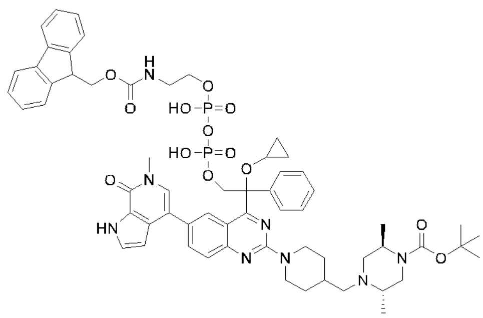

To a solution of (9H-fluoren-9-yl)methyl (2-(phosphonooxy)ethyl)carbamate (56.1 mg, 0.154 mmol) in DMF (1.0 mL) there were added 1,1-carbonyldiimidazole (50.1 mg, 0.309 mmol) and triethylamine (43.0 μL, 0.309 mmol), and the mixture was stirred for 30 minutes. Methanol (125 μL, 3.09 mmol) was added to the reaction mixture, which was then stirred for 20 minutes and subsequently concentrated under reduced pressure. A solution of the formate of *tert*-butyl (2R,5S)-4-((1-(4-(1-cyclopropoxy-1-phenyl-2-(phosphonooxy)ethyl)-6-(6-methyl-7-oxo-6,7-dihydro-1H-pyrrolo[2,3-c]pyridin-4-yl)quinazolin-2-yl)piperidin-4-yl)methyl)-2,5-dimethylpiperazine-1-carboxylate of Production Example 267-2 (26 mg) in DMF (1.0 mL) and zinc chloride (84 mg, 0.618 mmol) were added to the residue, and the mixture was stirred for 2 hours. The reaction mixture was purified by direct reversed-phase silica gel chromatography (0.1% formic acid aqueous solution:0.1% acetonitrile formate solution = 95:5 to 4:6) to obtain a formate of the title compound (10.6 mg).
ESI-MS (m/z): 1187.65 $[M+H]^+$.

[Production Example 267-4]

(9H-Fluoren-9-yl)methyl (2-(((((2-cyclopropoxy-2-(2-(4-(((2S,5R)-4-(3-(2,4-dioxotetrahydropyrimidine-1(2H)-yl)-4-methoxybenzoyl)-2,5-dimethylpiperazin-1-yl)methyl)piperidin-1-yl)-6-(6-methyl-7-oxo-6,7-dihydro-1H-pyrrolo[2,3-c]pyridin-4-yl)quinazolin-4-yl)-2-phenylethoxy) (hydroxy)phosphoryl)oxy) (hydroxy)phosphoryl)oxy)ethyl)carbamate

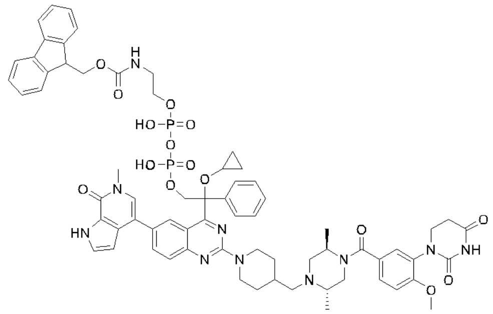

A crude product of the title compound (12.2 mg) was obtained from the formate of *tert*-butyl (2R,5S)-4-((1-(4-(2-(((((2-((((9H-fluoren-9-yl)methoxy)carbonyl)amino)ethoxy) (hydroxy)phosphoryl)oxy) (hydroxy) phosphoryl)oxy)-1-cyclopropoxy-1-phenylethyl)-6-(6-methyl-7-oxo-6,7-dihydro-1H-pyrrolo[2,3-c]pyridin-4-yl)quinazolin-2-yl)piperidin-4-yl)methyl)-2,5-dimethylpiperazine-1-carboxylate of Production Example 267-3 (10.6 mg), by the same method as Example 265.

ESI-MS (m/z): 1333.67 $[M+H]^+$.

[Example 268]

2-Cyclopropoxy-2-(2-(4-(((2S,5R)-4-(3-(2,4-dioxotetrahydropyrimidine-1(2H)-yl)-4-methoxybenzoyl)-2,5-dimethylpiperazin-1-yl)methyl)piperidin-1-yl)-6-(6-methyl-7-oxo-6,7-dihydro-1H-pyrrolo[2,3-c]pyridin-4-yl)quinazolin-4-yl)-2-phenylethyl (4-((80S,87S,90S)-80-(3-(2-(2-(2,5-dioxo-2,5-dihydro-1H-pyrrol-1-yl)ethoxy)ethoxy)propanamido)-87-isopropyl-74,81,85,88-tetraoxo-90-(3-ureidopropyl)-2,5,8,11,14,17,20,23,26,29,32,35,38,41,44,47,50,53,56,59,62,65,68,71-tetracosaoxa-75,82,86,89-tetraazahenonacontan-91-amide)benzyl)ethane-12-diylbis(methylcarbamate)

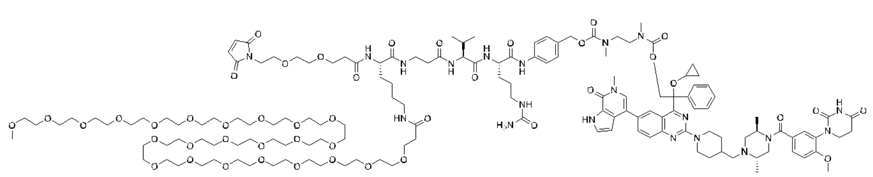

To a solution of the 2-cyclopropoxy-2-(2-(4-(((2S,5R)-4-(3-(2,4-dioxotetrahydropyrimidine-1(2H)-yl)-4-methoxybenzoyl)-2,5-dimethylpiperazin-1-yl)methyl)piperidin-1-yl)-6-(6-methyl-7-oxo-6,7-dihydro-1H-pyrrolo[2,3-c]pyridin-4-yl)quinazolin-4-yl)-2-phenylethyl methyl(2-(methylamino)ethyl)carbamate of Production Example 268-5 (8.00 mg, 7.83 μmol) in DMF (300 μL) there were added N,N-diisopropylethylamine (5.5 μL, 31 μmol), a TFA salt of the (9H-fluoren-9-yl) methyl ((80S,87S,90S)-95-amino-87-isopropyl-90-((4-((((4-nitrophenoxy)carbonyl)oxy)methyl)phenyl)carbamoyl)-74,81,85,88,95-pentaoxo-2,5,8,11,14,17,20,23,26,29,32,35,38,41,44,47,50,53,56,59,62,65,68,71-tetracosaoxa-75,82,86,89,94-pentaazapentanonacontan-80-yl)carbamate of Production Example 268-11 (18.8 mg, 8.61 μmol) and N,N-diisopropylethylamine (5.5 μL, 31 μmol), and the mixture was stirred for 1 hour at room temperature. Diethylamine (16.2 μL, 157 μmol) was added to the reaction mixture, and stirring was continued for 1 hour and 30 minutes at room temperature. The reaction mixture was concentrated under reduced pressure and the residue was dissolved in DMF (300 μL), after which MAL-PEG2-NHS ester (6.93 mg, 19.6 μmol) and N,N-diisopropylethylamine (5.5 μL, 31 μmol) were added and the mixture was stirred for 1 hour at room temperature. The reaction mixture was purified by direct reversed-phase silica gel chromatography (0.1% formic acid aqueous solution:0.1% acetonitrile formate solution = 9:1 to 6:4) to obtain a formate of the title compound (14.4 mg).

ESI-MS (m/z): 1483.45$[(M+2H)/2]^+$.

[Production Example 268-1]

*tert*-Butyl (2R,5S)-4((1-(4-(1-cyclopropoxy-1-phenyl-2-((tetrahydro-2H-pyran-2-yl)oxy)ethyl)-6-(6-methyl-7-oxo-1-tosyl-6,7-dihydro-1H-pyrrolo[2,3-c]pyridin-4-yl)quinazolin-2-yl)piperidin-4-yl)methyl)-2,5-dimethylpiperazine-1-carboxylate

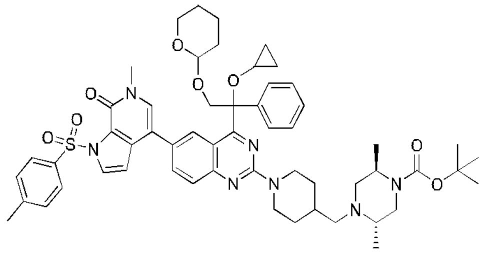

To a solution of the 4-(2-chloro-4-(1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 269-2 (400 mg, 552 μmol) in DMF (2 mL) there were added the *tert*-butyl (2R,5 S)-2,5-dimethyl-4-(piperidin-4-ylmethyl)piperazine-1-carboxylate of Production Example 265-6 (240 mg, 772 μmol) and N,N-diisopropylethylamine (295 μL, 1.66 mmol) at room temperature, and the mixture was stirred for 7 hours at 80°C. The reaction mixture was returned to room temperature, and then water was added and extraction was performed with ethyl acetate. The organic layer was washed with water and brine, dried over anhydrous magnesium sulfate and filtered, and the filtrate was concentrated under reduced pressure. The residue was purified by NH silica gel column chromatography (n-heptane:ethyl acetate = 1:3 to ethyl acetate) to obtain the title compound (389 mg).
ESI-MS (m/z): 1000.84 $[M+H]^+$.

[Production Example 268-2]

*tert*-Butyl (2R,5 S)-4-((1-(4-(1-cyclopropoxy-2-hydroxy-1-phenylethyl)-6-(6-methyl-7-oxo-1-tosyl-6,7-dihydro-1H-pyrrolo[2,3-c]pyridin-4-yl)quinazolin-2-yl)piperidin-4-yl)methyl)-2,5-dimethylpiperazine-1-carboxylate

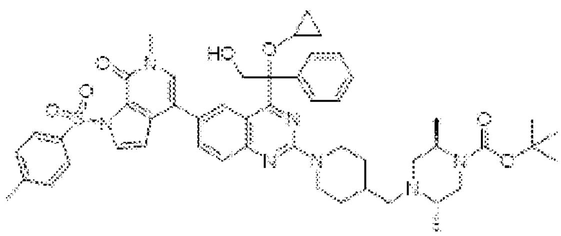

To a solution of the *tert*-butyl (2R,5S)-4-((1-(4-(1-cyclopropoxy-1-phenyl-2-((tetrahydro-2H-pyran-2-yl)oxy) ethyl)-6-(6-methyl-7-oxo-1-tosyl-6,7-dihydro-1H-pyrrolo[2,3-c]pyridin-4-yl)quinazolin-2-yl)piperidin-4-yl)methyl-2,5-dimethylpiperazine-1-carboxylate of Production Example 268-1 (250 mg, 250 μmol) in methanol (3 mL) there was added*p*-toluenesulfonic acid monohydrate (119 mg, 625 μmol), and the mixture was stirred for 2 hours at room temperature. The reaction mixture was diluted with ethyl acetate and saturated sodium hydrogencarbonate, and the aqueous layer was extracted with ethyl acetate. The organic layer was dried over magnesium sulfate and filtered, and the filtrate was concentrated under reduced pressure. The residue was purified by NH silica gel column chromatography (*n*-heptane:ethyl acetate = 40:60 to 15:85) to obtain the title compound (232 mg).
ESI-MS (m/z): 916.77 $[M+H]^+$.

[Production Example 268-3]

*tert*-Butyl (2R,5S)-4-((1-(4-(11-cyclopropoxy-4,7-dimethyl-3,8-dioxo-1,11-diphenyl-2,9-dioxa-4,7-diazaundecan-11-yl)-6-(6-methyl-7-oxo-6,7-dihydro-1H-pyrrolo[2,3-c]pyridin-4-yl)quinazolin-2-yl)piperidin-4-yl)methyl)-2,5-dimethylpiperazine-1-carboxylate

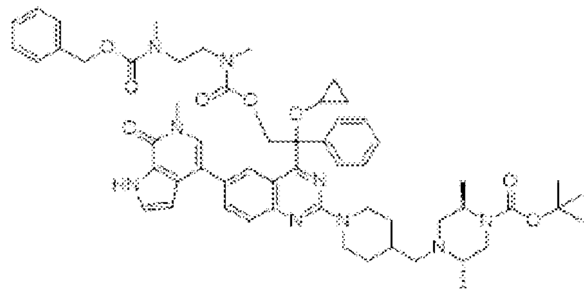

To a solution of the *tert*-butyl (2R,5S)-4-((1-(4-(1-cyclopropoxy-2-hydroxy-1-phenylethyl)-6-(6-methyl-7-oxo-1-tosyl-6,7-dihydro-1H-pyrrolo[2,3-c]pyridin-4-yl)quinazolin-2-yl)piperidin-4-yl)methyl)-2,5-dimethylpiperazine-1-carboxylate of Production Example 268-2 (120 mg, 131 μmol) and 4-dimethylaminopyridine (48 mg, 0.39 mmol) in dichloromethane (3 mL) there was added triphosgene (23.3 mg, 78.5 μmol) while cooling on ice, and after stirring for 5 minutes, the mixture was further stirred for 30 minutes at room temperature. The reaction mixture was again cooled on ice, and then benzyl methyl(2-methylamino)ethyl)carbamate (131 mg, 589 μmol) was added and the mixture was stirred for 10 minutes, after which it was further stirred for 30 minutes at room temperature. The reaction mixture was purified by NH silica gel column chromatography (*n*-heptane:ethyl acetate = 44:56 to ethyl acetate) to obtain a crude intermediate (220 mg). To a solution of the obtained crude intermediate (220 mg) in THF (400 μL)/methanol (800 μL) there was added a 2 M sodium hydroxide aqueous solution (400 μL, 800 μmol) at room temperature, and the mixture was stirred for 3 hours at 60°C. The reaction mixture was diluted with dichloromethane, the organic layer was dried over anhydrous magnesium sulfate and filtered, and the filtrate was concentrated under reduced pressure. The residue was purified by NH silica gel column chromatography (*n*-heptane:ethyl acetate = 15:85 to ethyl acetate to ethyl acetate:methanol = 95:5 to 9:1) to obtain a crude product of the title compound (106 mg). ESI-MS (m/z): 1010.74 $[M+H]^+$.

[Production Example 268-4]

Benzyl (2-cyclopropoxy-2-(2-(4-(((2S,5R)-4-(3-(2,4-dioxotetrahydropyrimidine-1(2H)-yl)-4-methoxybenzoyl)-2,5-dimethylpiperazin-1-yl)methyl)piperidin-1-yl)-6-(6-methyl-7-oxo-6,7-dihydro-1H-pyrrolo[2,3-c]pyridin-4-yl)quinazolin-4-yl)-2-phenylethyl)ethane-1,2-diylbis(methylcarbamate)

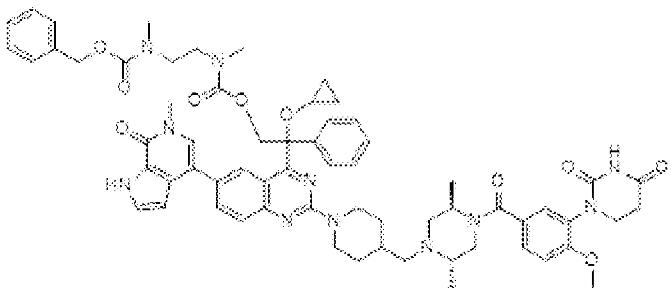

The title compound (57.5 mg) was obtained from the crude *tert*-butyl (2R,5S)-4((1-(4-(11-cyclopropoxy-4,7-dimethyl-3,8-dioxo-1,11-diphenyl-2,9-dioxa-4,7-diazaundecan-11-yl)-6-(6-methyl-7-oxo-6,7-dihydro-1H-pyrrolo[2,3-c]pyridin-4-yl)quinazolin-2-yl)piperidin-4-yl)methyl)-2,5-dimethylpiperazine-1-carboxylate of Production Example 268-3 (106 mg) and the 3-(2,4-dioxotetrahydropyrimidine-1(2H)-yl-4-methoxybenzoic acid of Production Example 265-12 (41.6 mg, 157 μmol), by the same method as Example 265.

ESI-MS (m/z): 1157.74 $[M+H]^+$.

[Production Example 268-5]

2-Cyclopropoxy-2-(2-(4-(((2S,5R)-4-(3-(2,4-dioxotetrahydropyrimidine-1(2H)-yl)-4-methoxybenzoyl)-2,5-dimethylpiperazin-1-yl)methyl)piperidin-1-yl)-6-(6-methyl-7-oxo-6,7-dihydro-1H-pyrrolo[2,3-c]pyridin-4-yl)quinazolin-4-yl)-2-phenylethyl methyl(2-(methylamino)ethyl)carbamate

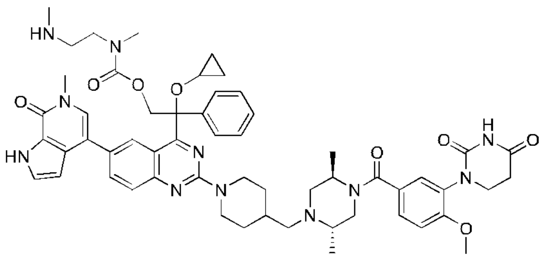

To a solution of the benzyl (2-cyclopropoxy-2-(2-(4-(((2S,5R)-4-(3-(2,4-dioxotetrahydropyrimidine-1(2H)-yl)-4-methoxybenzoyl)-2,5-dimethylpiperazin-1-yl)methyl)piperidin-1-yl)-6-(6-methyl-7-oxo-6,7-dihydro-1H-pyrrolo[2,3-c]pyridin-4-yl)quinazolin-4-yl)-2-phenylethyl ethane-1,2-diylbis(methylcarbamate) of Production Example 268-4 (57.5 mg, 49.7 μmol) in THF (2 mL)/ethyl acetate (2 mL) there was added 10% palladium-carbon (50% water, 42.3 mg) at room temperature, and the mixture was stirred for 1.5 hours at room temperature and ordinary pressure under a hydrogen atmosphere. After further adding 10% palladium-carbon (50% water, 106 mg), the mixture was stirred for 16 hours and 20 minutes at room temperature and ordinary pressure under a hydrogen atmosphere. The reaction mixture was filtered with Celite and washed with methanol. The filtrate was concentrated under reduced pressure and the residue was purified by NH silica gel thin-layer chromatography (chloroform:methanol = 20:1), to obtain the title compound (18.5 mg).
ESI-MS (m/z): 1022.72 $[M+H]^+$.

[Production Example 268-6]

(10S,17S,20S)-10-((((9H-Fluoren-9-yl)methoxy)carbonyl)amino)-17-isopropyl-2,2-dimethyl-4,11,15,18-tetraoxo-20-(3-ureidopropyl)-3-oxa-5,12,16,19-tetraazahenicosan-21-oic acid

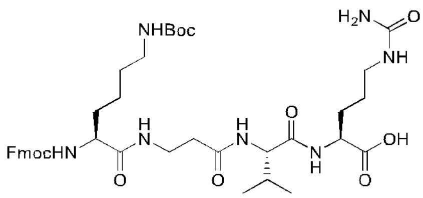

A 2-chlorotrityl chloride resin (50 mmol, 1.05 mmol/g) and dichloromethane were added to the reactor, allowing the resin to swell for 2 hours. A Fmoc-Cit-OH (19.9 g, 50 mmol) solution was added to the reaction mixture, and after mixing for 30 seconds, N,N-diisopropylethylamine (19.6 mL, 200 mmol) was added and nitrogen was passed through for bubbling for 1 hour. Methanol (50 mL) was added to the reaction mixture, and mixing was continued for 30 minutes. The reaction mixture was drained, and then the resin was washed 3 times with DMF, a 20% piperidine/DMF solution was added, and the mixture was stirred for 30 minutes. The reaction mixture was drained and the resin was washed 5 times with DMF. After adding an Fmoc-Val-OH (50.9 g, 150 mmol) solution to the reactor containing the resin and mixing for 30 seconds, N,N-diisopropylethylamine (29.5 mL, 300 mmol) and HBTU (54.0 g, 142.5 mmol) were added and nitrogen was passed through for bubbling for 1 hour. Methanol (50 mL) was added to the reaction mixture, and mixing was continued for 30 minutes. The reaction mixture was drained, and then the resin was washed 3 times with DMF, a 20% piperidine/DMF solution was added, and the mixture was stirred for 30 minutes. The reaction mixture was drained and the resin was washed 5 times with DMF. The same procedure was repeated two more times using an Fmoc-βAla-OH (46.7 g, 150 mmol) solution and an Fmoc-Lys(Boc)-OH (70.3 g, 150 mmol) solution. A 20% HFIP/dichloromethane solution was added to the reactor containing the resin, and after mixing for 30 minutes at room temperature, the mixture was filtered. The same procedure was repeated two more times, and the combined filtrates were concentrated under reduced pressure to obtain a crude product of the title compound (36.0 g).
ESI-MS (m/z): 796.4 $[M+H]^+$.

[Production Example 268-7]

(9H-Fluoren-9-yl)methyl *tert*-butyl((6S,9S,16S)-1-amino-6-((4-(hydroxymethyl)phenyl)carbamoyl)-9-isopropyl-1,8,11,15-tetraoxo-2,7,10,14-tetraazaeicosan-16,20-diyl)dicarbamate

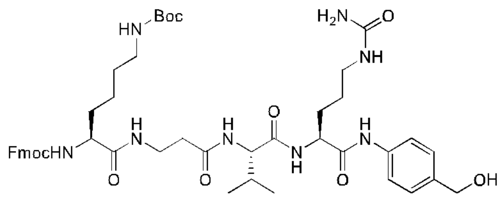

To a solution of the (10S, 17S, 20S)-10-((((9H-fluoren-9-yl)methoxy)carbonyl)amino)-17-isopropyl-2,2-dimethyl-4,11,15,18-tetraoxo-20-(3-ureidopropyl)-3-oxa-5,12,16,19-tetraazahenicosan-21-oic acid of Production Example 268-6 (36.0 g, 45.2 mmol) and (4-aminophenyl)methanol (16.7 g, 135 mmol) in dichloromethane (100 mL)/methanol (100 mL) there was added EEDQ (33.5 g, 135 mmol), and the mixture was stirred for 2 hours at 45°C while shielded from light. The reaction mixture was concentrated under reduced pressure and the residue was purified by silica gel chromatography (dichloromethane:methanol = 200:1 to 4:1), to obtain the title compound (30.0 g). ESI-MS (m/z): 901.5 $[M+H]^+$.

[Production Example 268-8]

(9H-Fluoren-9-yl)methyl ((6S,9S,16S)-1,20-diamino-6-((4-(hydroxymethyl)phenyl)carbamoyl)-9-isopropyl-1,8,11,15-tetraoxo-2,7,10,14-tetraazaeicosan-16-yl)carbamate

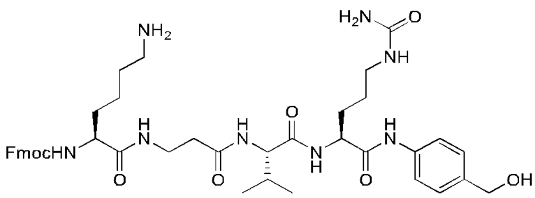

The (9H-fluoren-9-yl)methyl *tert*-butyl((6S, 9S, 16S)-1-amino-6-((4-(hydroxymethyl)phenyl)carbamoyl)-9-isopropyl-1,8,11,15-tetraoxo-2,7,10,14-tetraazaeicosan-16,20-diyl)dicarbamate of Production Example 268-7 (30.0 g, 33.2 mmol) was dissolved in a hydrochloric acid/dioxane solution (150 mL), and the mixture was stirred for 1 hour at 20 to 25°C. The reaction mixture was concentrated under reduced pressure and the residue was purified by preparative HPLC (0.1% acetonitrile/water) to obtain the title compound (8.0 g).
ESI-MS (m/z): 819.5 $[M+H2O]^+$.

[Production Example 268-9]

2,5-Dioxopyrrolidin-1-yl 2,5,8,11,14,17,20,23,26,29,32,35,38,41,44,47,50,53,56,59,62,65,68,71-tetracosaoxatetraheptacontan-74-oate

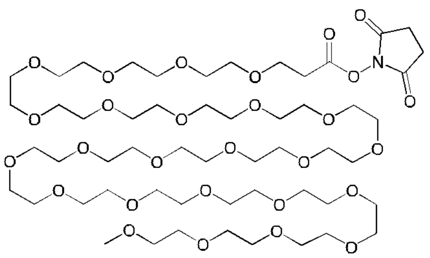

To a solution of m-PEG24-COOH (9.00 g, 8.06 mmol) in dichloromethane (90 mL) there were added N-hydroxysuccinimide (1.11 g, 9.67 mmol), EDCI (3.09 g, 16.1 mmol) and DMAP (394 mg, 3.22 mmol), and the mixture was stirred for 2 hours at 25°C. The reaction mixture was diluted with dichloromethane and then washed twice with 1 M hydrochloric acid and then with brine. The organic layer was dried over anhydrous sodium sulfate and filtered, and then the filtrate was concentrated under reduced pressure to obtain a crude product of the title compound (9.50 g).
ESI-MS (m/z): 1215.0 $[M+H]^+$.

[Production Example 268-10]

(9H-Fluoren-9-yl)methyl ((80S,87S,90S)-95-amino-90-((4-(hydroxymethyl)phenyl)carbamoyl)-87-isopropyl-74,81,85,88,95-pentaoxo-2,5,8,11,14,17,20,23,26,29,32,35,38,41,44,47,50,53,56,59,62,65,68,71-tetracosaoxa-75,82,86,89,94-pentaazapentanonacontan-80-yl)carbamate

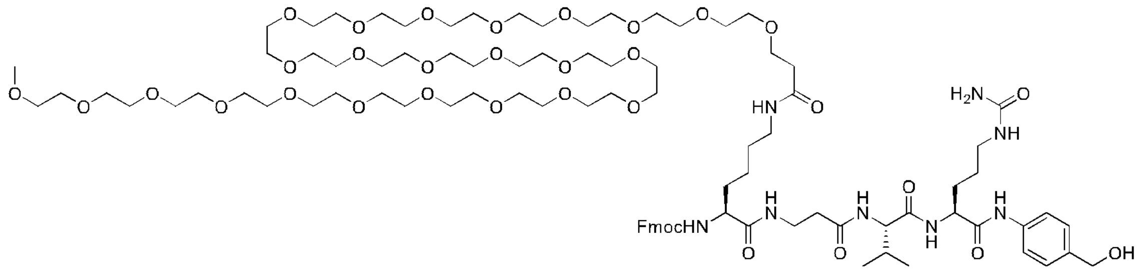

To a solution of the (9H-fluoren-9-yl)methyl ((6S, 9S, 16S)-1,20-diamino-6-((4-(hydroxymethyl)phenyl)carbamoyl)-9-isopropyl-1,8,11,15-tetraoxo-2,7,10,14-tetraazaeicosan-16-yl)carbamate of Production Example 268-8 (7.21 g, 9.00 mmol) in DMF (40 mL) there were added N,N-diisopropylethylamine (4.09 mL, 23.4 mmol) and the 2,5-dioxopyrrolidin-1-yl 2,5,8,11,14,17,20,23,26,29,32,35,38,41,44,47,50,53,56,59,62,65,68,71-tetracosaoxatetraheptacontan-74-oate of Production Example 268-9 (9.5 g, 7.82 mmol), and the mixture was stirred for 10 hours at 25°C. The reaction mixture was washed with TBME and then centrifuged (3 minutes, 3000 rpm) and dried under nitrogen to obtain the title compound (10.1 g).
ESI-MS (m/z): 951.3$[(M+2H)/2]^+$.

[Production Example 268-11]

(9H-Fluoren-9-yl)methyl ((80S,87S,90S)-95-amino-87-isopropyl-90-((4-((((4-nitrophenoxy)carbonyl)oxy)methyl)phenyl)carbamoyl)-74,81,85,88,95-pentaoxo-2,5,8,11,14,17,20,23,26,29,32,35,38,41,44,47,50,53,56,59,62,65,68,71-tetracosaoxa-75,82,86,89,94-pentaazapentanonacontan-80-yl)carbamate

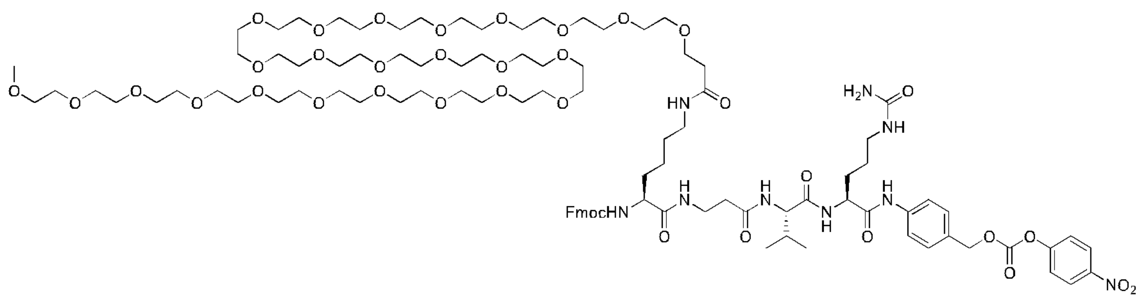

The title compound (460 mg) was obtained from the (9H-fluoren-9-yl)methyl((80S,87S,90S)-95-amino-90-((4-(hydroxymethyl)phenyl)carbamoyl)-87-isopropyl-74,81,85,88,95-pentaoxo-2,5,8,11,14,17,20,23,26,29,32,35,38,41,44,47,50,53,56,59,62,65,68,71-tetracosaoxa-75,82,86,89,94-pentaazapentanonacontan-80-yl)carbamate of Production Example 268-10 (650 mg, 342 μmol), by the same method as Production Example 265-9.
ESI-MS (m/z): 1033.7$[(M+2H)/2]^+$.

[Example 269]

(2S)-N-({[(2-Cyclopropoxy-2-{2-[4-({3-[3-(2,4-dioxo-1,3-diazinane-1-yl)-4-methoxyphenyl]prop-2-yn-1-yl}amino)-4-methyl-[1,4'-bipiperidin]-1'-yl]-6-{6-methyl-7-oxo-1H,6H,7H-pyrrolo[2,3-c]pyridin-4-yl}quinazolin-4-yl}-2-phenylethoxy)methyl]carbamoyl}methyl)-2-(2-{2-[3-(2-{2-[2,5-dioxo-3,4-bis(phenylsulfanyl)-2,5-dihydro-1H-pyrrol-1-yl]ethoxy}ethoxy)propanamido]acetamide}acetamide)-3-phenylpropaneamide

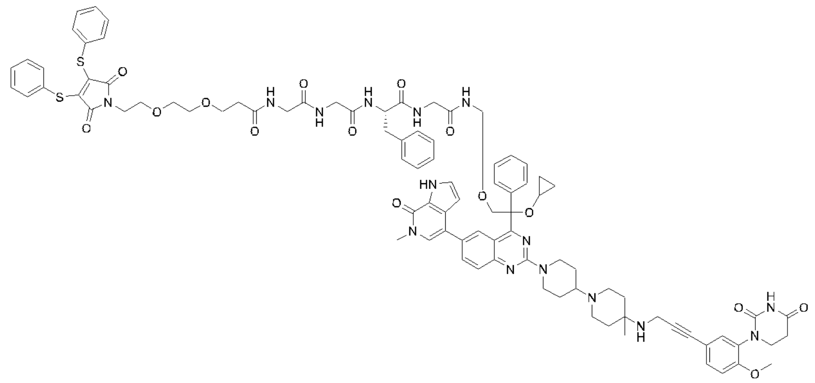

To a solution of the (9H-fluoren-9-yl)methyl N-({[(2-cyclopropoxy-2-{2-[4-({3-[3-(2,4-dioxo-1,3-diazinane-1-yl)-4-methoxyphenyl]prop-2-yn-1-yl}amino)-4-methyl-[1,4'-bipiperidin]-1'-yl]-6-{6-methyl-7-oxo-1H,6H,7H-pyrrolo[2,3-c]pyridin-4-yl}quinazolin-4-yl}-2-phenylethoxy)methyl]carbamoyl}methyl)carbamate of Production Example 269-7 (8.8 mg, 7.3 μmol) in DMF (561 μL) there was added piperidine (91 μL, 0.73 mmol), and the mixture was stirred for 15 minutes at room temperature and then concentrated under reduced pressure. After repeating azeotropic distillation of the obtained residue with toluene (1 mL) 3 times, a solution of (2S)-2-{2-[2-({[(9H-fluoren-9-yl)methoxy]carbonyl}amino)acetamide]acetamide}-3-phenylpropanoic acid (8.0 mg, 0.016 mmol), HATU (5.5 mg, 0.15 mmol) and N,N-diisopropylethylamine (6.3 μL, 0.036 mmol) in DMF (561 μL) was added. After stirring for 1 hour at room temperature, piperidine (91 μL, 0.73 mmol) was added and the mixture was stirred for 20 minutes. The reaction mixture was concentrated under reduced pressure and processed 3 times by azeotropic distillation with toluene (1.0 mL) to obtain an amine intermediate. In a separate vessel, the *tert*-butyl 3-(2-(2-(2,5-dioxo-3,4-bis(phenylthio)-2,5-dihydro-1H-pyrrol-1-yl)ethoxy)ethoxy)propionate of Production Example 201-22 (15 mg, 0.029 mmol) was dissolved in dichloromethane (233 μL), and TFA (111 μL, 1.45 mmol) was added. The reaction mixture was stirred for 20 minutes at room temperature and then concentrated under reduced pressure, and the residue was processed 3 times by azeotropic distillation with toluene (2 mL). To a solution of the residue in DMF (561 μL) there were added HATU (5.5 mg, 0.015 mmol) and N,N-diisopropylethylamine (6.3 μL, 0.036 mmol). The obtained solution was added to the previously synthesized amine intermediate, and the mixture was stirred for 30 minutes at room temperature. The reaction mixture was purified by UFPLC to obtain a formate of the title compound (3.6 mg).

ESI-MS (m/z): 1707.90 $[M+H]^+$.

[Production Example 269-1]

2-Chloro-4-(1-cyclopropoxy-1-phenyl-2-((tetrahydro-2H-pyran-2-yl)oxy)ethyl-6-iodoquinazoline

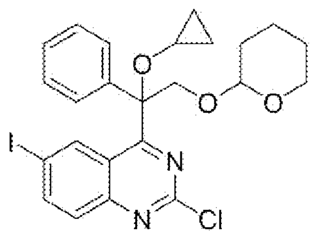

To a solution of the ethyl 2-(2-chloro-6-iodoquinazolin-4-yl)-2-cyclopropoxy-2-phenylacetate of Production Example 201-2 (1.08 g, 2.12 mmol) in dichloromethane (10 mL) there was added DIBAL-H (1.0 M toluene solution,

4.67 mL, 4.67 mmol) at -78°C, and the mixture was stirred for 10 minutes. The reaction mixture was increased in temperature to 0°C and stirred for 10 minutes. A saturated aqueous Rochelle salt solution was added to the reaction mixture, which was then stirred overnight at room temperature. The reaction mixture was extracted with ethyl acetate. The organic layer was washed with brine, dried over anhydrous sodium sulfate and filtered, and the filtrate was concentrated under reduced pressure. To a solution of the residue in dichloromethane (10 mL) there were added DHP (576 mL, 6.37 mmol) and CSA (99 mg, 0.43 mmol) at room temperature, and the mixture was stirred for 1 hour at room temperature. A saturated aqueous sodium hydrogencarbonate solution was added to the reaction mixture, and extraction was performed with ethyl acetate. The organic layer was washed with brine, dried over anhydrous sodium sulfate and filtered, and the filtrate was concentrated under reduced pressure. The residue was purified by NH silica gel column chromatography (n-heptane:ethyl acetate = 95:5 to 8:2) to obtain the title compound (733 mg).

ESI-MS (m/z): 551.25 [M+H]+.

[Production Example 269-2]

4-(2-Chloro-4-(1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one

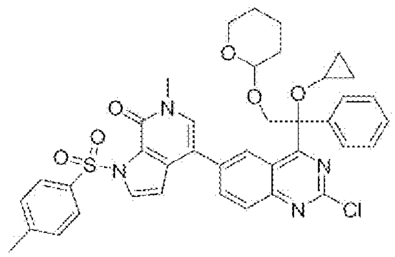

To a mixed solution of the 2-chloro-4-(1-cyclopropoxy-1-phenyl-2-((tetrahydro-2H-pyran-2-yl)oxy)ethyl-6-iodoquinazoline of Production Example 269-1 (4.00 g, 7.26 mmol) and 6-methyl-4-(4,4,5,5-tetramethyl-1,3,2-dioxaborolan-2-yl)-1-tosyl-1,6-dihydro-7H-pyrrolo[2,3-c]pyridin-7-one (3.73 g, 8.71 mmol) in toluene (70 mL), ethanol (17.5 mL) and 2 M aqueous sodium carbonate (17.5 mL) there was added tetrakis(triphenylphosphine)palladium(0) (839 mg, 726 μmol) under a nitrogen atmosphere, and the mixture was stirred for 18 hours at 70°C. The reaction mixture was returned to room temperature, and extraction was performed with ethyl acetate. The organic layer was washed with brine, dried over anhydrous magnesium sulfate and filtered, and the filtrate was concentrated under reduced pressure. The residue was purified by silica gel column chromatography (n-heptane:ethyl acetate = 1:1 to 3:7) to obtain the title compound (4.78 g).

ESI-MS (m/z): 725.38 [M+H]+.

[Production Example 269-3]

4-(2-Chloro-4-(1-cyclopropoxy-2-hydroxy-1-phenylethyl)quinazolin-6-yl)-6-methyl-1-tosyl-1,6-dihydro-7H-pyrrolo[2,3-c]pyridin-7-one

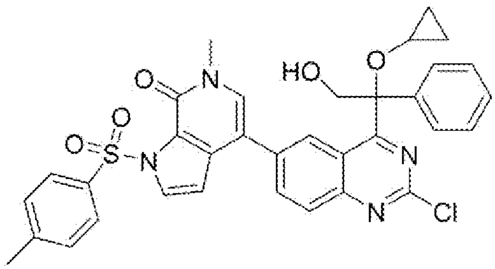

To a solution of the 4-(2-chloro-4-(1-cyclopropoxy-1-phenyl-2-((tetrahydro-2H-pyran-2-yl)oxy)ethyl)quinazolin-6-yl)-6-methyl-1-tosyl-1,6-dihydro-7H-pyrrolo[2,3-c]pyridin-7-one of Production Example 269-2 (100 mg, 138 μmol) in dichloromethane (5 mL) there was added TFA(1.06 mL), and the mixture was stirred for 1 hour. An aqueous sodium

hydrogencarbonate solution was added to the reaction mixture, which was then extracted twice with dichloromethane. The organic layer was dried over sodium sulfate and filtered. The filtrate was concentrated to obtain the title compound (109 mg).

ESI-MS (m/z): 641.16 $[M+H]^+$.

[Production Example 269-4]

4-(4-(1-Cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1-tosyl-1,6-dihydro-7H-pyrrolo[2,3-c]pyridin-7-one

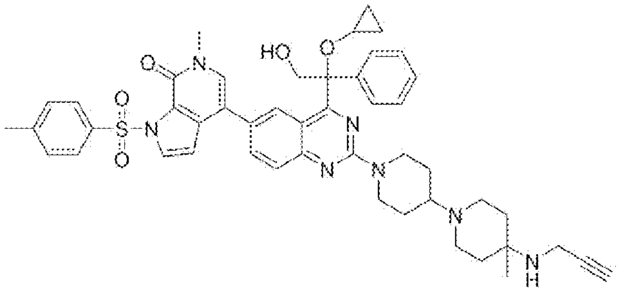

To a solution of the *tert*-butyl 4-methyl-4-(prop-2-yn-1-ylamino)-[1,4'-bipiperidine]-1'-carboxylate of Production Example 201-7 (610 mg, 90% purity, 1.64 mmol) in dichloromethane (3.44 mL) there was added TFA (3.37 mL), and the mixture was stirred for 30 minutes at room temperature. The reaction mixture was concentrated under reduced pressure and processed by azeotropic distillation with toluene, to obtain a crude product. To a solution of the obtained crude product in DMF (9.47 mL) there were added N,N-diisopropylethylamine (2.86 mL) and the 4-(2-chloro-4-(1-cyclopropoxy-2-hydroxy-1-phenylethyl)quinazolin-6-yl)-6-methyl-1-tosyl-1,6-dihydro-7H-pyrrolo[2,3-c]pyridin-7-one of Production Example 269-3 (700 mg, 1.09 mmol, azeotropic distillation with toluene before use), and the mixture was stirred for 1 hour at 85°C. To the reaction mixture there was added N,N-diisopropylethylamine (0.95 mL), and the mixture was stirred for 3 hours at 85°C. The reaction mixture was cooled to room temperature and concentrated under reduced pressure to 1/4 volume, and then ethyl acetate and an aqueous sodium hydrogencarbonate solution were added. After oil-water distribution, the aqueous layer was extracted with ethyl acetate. The organic layer was dried over sodium sulfate and filtered, and the filtrate was concentrated under reduced pressure. The residue was purified by NH silica gel column chromatography (*n*-heptane:ethyl acetate = 1:1 to ethyl acetate to ethyl acetate:methanol = 7:3). It was further purified by silica gel column chromatography (ethyl acetate to ethyl acetate:methanol = 1:1) to obtain the title compound (569 mg).

ESI-MS (m/z): 840.42 $[M+H]^+$.

[Production Example 269-5]

4-(4-(1-Cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one

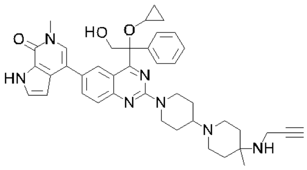

To a solution of the 4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1-tosyl-1,6-dihydro-7H-pyrrolo[2,3-c]pyridin-7-one of Production Example 269-4 (569 mg, 677 μmol) in ethanol (13.8 mL) there was added a sodium hydroxide aqueous solution (1 M, 2.71 mL, 2.71 mmol), and the mixture was stirred for 20 hours at room temperature. After diluting the reaction mixture with 5 mL of water, it was extracted twice with a 10% methanol/chloroform solution. After washing the organic layer with brine (10 mL), it was dried over sodium sulfate and filtered, and the filtrate was concentrated under reduced pressure. Ethyl acetate

(5 mL) was added to the residue and the precipitated solid was filtered. The obtained solid was further washed with ethyl acetate (10 mL) to obtain the title compound (360 mg). A 5.0 mg portion was purified by NH silica gel thin-layer chromatography (ethyl acetate:methanol = 9: 1) to obtain the title compound (4.3 mg).

ESI-MS (m/z): 686.41 [M+H]+.

[Production Example 269-6]

1-(5-(3-((1'-(4-(1-Cyclopropoxy-2-hydroxy-1-phenylethyl)-6-(6-methyl-7-oxo-6,7-dihydro-1H-pyrrolo[2,3-c]pyridin-4-yl) quinazolin-2-yl)-4-methyl-[1,4'-bipiperidin]-4-yl)amino)prop-1-yn-1-yl)-2-methoxyphenyl)dihydropyrimidine-2,4(1H,3H)-dione

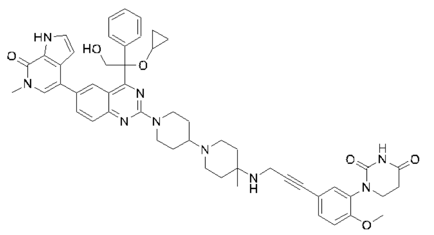

To a solution of the 4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 269-5 (35 mg, 51 μmol), the 1-(5-iodo-2-methoxyphenyl)dihydropyrimidine-2,4(1H,3H)-dione of Production Example 201-16 (35.3 mg, 102 μmol) and CuI (3.9 mg, 20 μmol) in DMF (385 μL) there were added N,N-diisopropylethylamine (454 μL, 2.55 mmol) and tetrakis(triphenylphosphine)palladium(0) (23.6 mg, 20 μmol), and the mixture was stirred for 2 hours at 40°C under a nitrogen atmosphere. The reaction mixture was concentrated under reduced pressure and the residue was purified by ODS silica gel chromatography (0.1% formic acid aqueous solution:0.1% acetonitrile formate solution = 97:3 to 0:100), to obtain a crude product. The crude product was purified with a fully automatic fractionation LC system (0.1% formic acid aqueous solution:0.1% acetonitrile formate solution = 7:3 to 1:1) to obtain a formate of the title compound (11 mg).

$^1$H-NMR Spectrum (500 MHz, $CD_3OD$) δ(ppm): 0.14-0.23 (1H, m), 0.24-0.37 (1H, m), 0.37-0.45 (1H, m), 0.71-0.83 (1H, m), 1.71-1.88 (4H, m), 1.93-2.05 (2H, m), 2.18-2.28 (2H, m), 2.72-2.76 (2H, m), 3.03-3.22 (5H, m), 3.26-3.28 (6H, m), 3.37-3.48 (3H, m), 3.60-3.66 (5H, m), 3.69 (2H, s), 3.81 (3H, s), 4.44 (1H, d, J=12.2 Hz), 5.16 (2H, d, J=12.9 Hz), 5.92 (1H, d, J=3.1 Hz), 7.00 (1H, s), 7.02 (1H, d, J=8.6 Hz), 7.20-7.28 (4H, m), 7.31-7.38 (4H, m), 7.58 (1H, d, J=9.2 Hz), 7.73-7.76 (1H, m), 8.20 (1H, d, J=1.9 Hz), 8.29-8.45 (2H, m) ESI-MS (m/z): 904.64 [M+H]+.

[Production Example 269-7]

(9H-Fluoren-9-yl)methyl N-({[(2-cyclopropoxy-2-{2-[4-({3-[3-(2,4-dioxo-1,3-diazinane-1-yl)-4-methoxyphenyl]prop-2-yn-1-yl}amino)-4-methyl-[1,4'-bipiperidin]-1'-yl]-6-{6-methyl-7-oxo-1H,6H,7H-pyrrolo[2,3-c]pyridin-4-yl}quinazolin-4-yl}-2-phenylethoxy)methyl]carbamoyl}methyl)carbamate

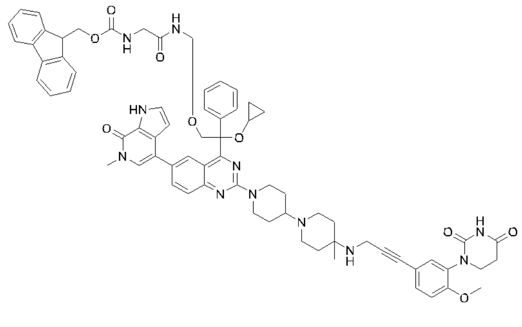

A mixture of the 1-(5-(3-((1'-(4-(1-cyclopropoxy-2-hydroxy-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)dihydropyrimidine-2,4(1H,3H)-dione of Production Example 269-6 (32 mg, 0.035 mmol) and the (2-((((9H-fluoren-9-yl) methoxy)carbonyl)amino)acetamide)methyl acetate of Production Example 201-10 (52 mg, 0.14 mmol was processed twice by azeotropic distillation with toluene (2.0 mL), and then CSA(33 mg, 0.14 mmol) and THF (1.2 mL) were added. The reaction mixture was stirred for 2 hours at room temperature, and then (2-((((9H-fluoren-9-yl)methoxy)carbonyl)amino) acetamide)methyl acetate (52 mg, 0.14 mmol) and CSA (33 mg, 0.14 mmol) were added and the mixture was stirred for 2 hours. Triethylamine (200 μL) was added to the reaction mixture to halt the reaction, and after concentration under reduced pressure, the residue was purified by silica gel column chromatography (ethyl acetate:heptane = 1: 1 to 0:1, followed by methanol:ethyl acetate = 0: 1 to 1:1) to obtain a crude product. This was followed by further purification by ODS silica gel column chromatography (0.1% acetonitrile formate solution:0.1% formic acid aqueous solution = 2:98 to 7:3) to obtain a formate of the title compound (9.0 mg).

ESI-MS (m/z): 1212.74 $[M+H]^+$.

[Example 270]

(S)-3-Acetamide-4-(((3-(5-(3-((1'-(4-((44S,50S,59S)-50-benzyl-59-cyclopropoxy-44-(3-(2-(2-(2,5-dioxo-3,4-bis(phenylthio)-2,5-dihydro-1H-pyrrol-1-yl)ethoxy)ethoxy)propanamido)-38,45,48,51,54-pentaoxo-59-phenyl-2,5,8,11,14,17,20,23,26,29,32,35,57-tridecaoxa-39,46,49,52,55-pentaazanonapentacontan-59-yl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxo-3,6-dihydropyrimidine-1(2H)-yl)methyl)amino)-4-oxobutanoic acid

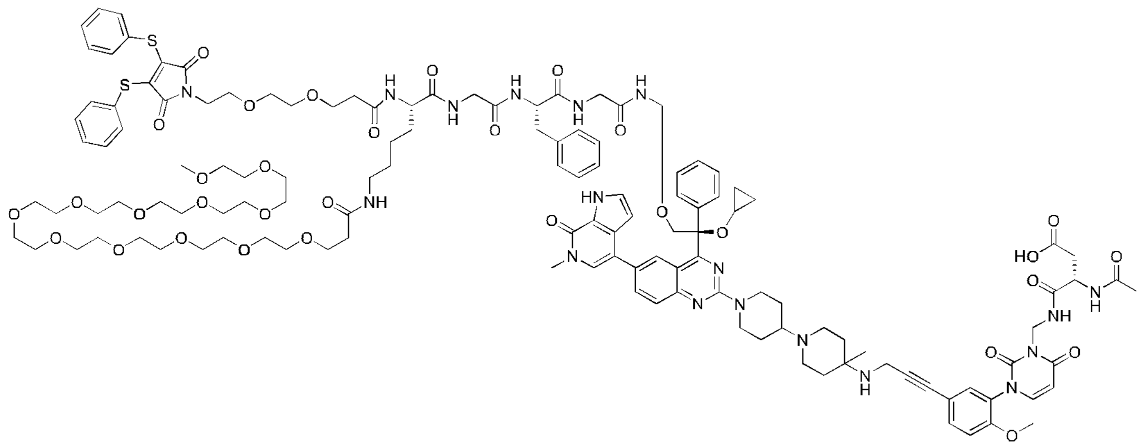

To a solution of the (S)-3-acetamide-4-(((3-(5-(3-((1'-(4-((44S,50S,59S)-44-amino-50-benzyl-59-cyclopropoxy-38,45,48,51,54-pentaoxo-59-phenyl-2,5,8,11,14,17,20,23,26,29,32,35,57-tridecaoxa-39,46,49,52,55-pentaazanonapentacontan-59-yl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxo-3,6-dihydropyrimidine-1(2H)-yl)methyl)amino)-4-oxobutanoic acid of Production Example 270-5 (13 mg, 0.0063 mmol) in DMF (1.5 mL) there were added the 2,5-dioxopyrrolidin-1-yl 3-(2-(2-(2,5-dioxo-3,4-bis(phenylthio)-2,5-dihydro-1H-pyrrol-1-yl)ethoxy)ethoxy)propanoate of Production Example 201-23 (7.1 mg, 0.15 mmol) and N,N-diisopropylethylamine (200 μL, 1.15 mmol), and the mixture was stirred for 1.5 hours at 50°C. The reaction mixture was directly purified by reversed-phase silica gel chromatography (0.1% formic acid aqueous solution:0.1% acetonitrile formate solution = 90:10 to 30:70) to obtain a formate of the title compound (3.4 mg).

ESI-MS (m/z): 1267.54$[(M+2H)/2]^+$.

[Production Example 270-1]

1-(5-Iodo-2-methoxyphenyl)urea

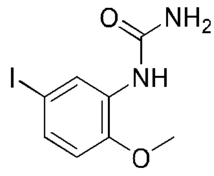

To a solution of 5-iodo-2-methoxyaniline (1.5 g, 6.0 mmol) in water (15 mL)/ethanol (10 mL)/2N-ethanol hydrochloride (15 mL) there was added a solution of potassium cyanate (0.98 g, 12 mmol) in water (5 mL) at 0°C using a dropping funnel, and the mixture was stirred for 1 hour at 0°C. The obtained solid was filtered out to obtain the title compound (1.3 g).
$^1$H-NMR Spectrum (500 MHz, DMSO-$d_6$) δ(ppm): 3.78 (3H, s), 6.26 (2H, brs), 6.75 (1H, d, J=8.3 Hz), 7.15 (1H, dd, J=8.3, 2.2 Hz), 7.97 (1H, s), 8.42 (1H, d, J=2.2 Hz).
ESI-MS (m/z): 292.95 $[M+H]^+$.

[Production Example 270-2]

1-(5-Iodo-2-methoxyphenyl)pyrimidine-2,4(1H,3H)-dione

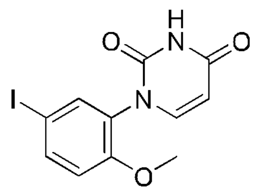

To a solution of the 1-(5-iodo-2-methoxyphenyl)urea of Production Example 270-1 (400 mg, 1.37 mmol) in DMF (10 mL) there was added 50% to 72% sodium hydride oil (110 mg) at room temperature, and after stirring the mixture at 50°C for about 3 minutes, methyl 3,3-dimethoxypropionate (383 μL, 2.74 mmol) was added at room temperature. The reaction mixture was stirred for 3 hours at 100°C and then cooled to room temperature, and water was added. After dilution with dichloromethane, the organic layer was extracted and dried over sodium sulfate. After filtration, the filtrate was concentrated under reduced pressure and the residue was purified by reversed-phase silica gel chromatography (0.1% formic acid aqueous solution:0.1% acetonitrile formate solution = 95:5 to 0:100) to obtain the title compound (140 mg).
$^1$H-NMR Spectrum (400 MHz, $CDCl_3$) δ(ppm): 3.83 (3H, s), 5.76 (1H, d, J=7.8 Hz), 6.79 (1H, d, J=8.8 Hz), 7.09 (1H, d, J=7.8 Hz), 7.57 (1H, d, J=2.0 Hz) 7.69 (1H, d, J=2.0, 8.8 Hz). ESI-MS (m/z): 345.01 $[M+H]^+$.

[Production Example 270-3]

*tert*-Butyl (S)-3((((9H-fluoren-9-yl)methoxy)carbonyl)amino)-4-(((3-(5-iodo-2-methoxyphenyl)-2,6-dioxo-3,6-dihydropyrimidine-1(2H)-yl)methyl)amino)-4-oxobutanoate

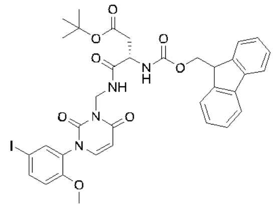

To a solution of the 1-(5-iodo-2-methoxyphenyl)pyrimidine-2,4(1H,3H)-dione of Production Example 270-2 (130 mg, 0.378 mmol) in THF (3.0 mL)/DMF (1.0 mL) there was added potassium *tert*-butoxide (55.1 mg, 0.491 mmol) at 0°C, and the mixture was stirred for 5 minutes. To the reaction mixture there was added a solution of the *tert*-butyl (S)-3-((((9H-fluoren-9-yl)methoxy)carbonyl)amino)-4-((acetoxymethyl)amino)-4-oxobutanoate of Production Example 201-17 (201 mg, 0.416 mmol) in THF (2.0 mL) at 0°C, and the mixture was stirred for 1 hour at 0°C. The reaction mixture was quenched with an ammonium chloride aqueous solution. The reaction mixture was extracted with dichloromethane and the organic

layer was dried over sodium sulfate, after which it was filtered and the filtrate was concentrated under reduced pressure. The residue was purified by silica gel chromatography (heptane:ethyl acetate = 90:10 to 0:1) to obtain the title compound (255 mg).

ESI-MS (m/z): 767.29 $[M+H]^+$.

[Production Example 270-4]

(S)-3-Acetamide-4-(((3-(5-iodo-2-methoxyphenyl)-2,6-dioxo-3,6-dihydropyrimidine-1(2H)-yl)methyl)amino)-4-oxobutanoic acid

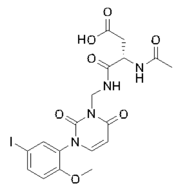

To a solution of the *tert*-butyl (S)-3((((9H-fluoren-9-yl)methoxy)carbonyl)amino)-4-(((3-(5-iodo-2-methoxyphenyl)-2,6-dioxo-3,6-dihydropyrimidine-1(2H)-yl)methyl)amino)-4-oxobutanoate of Production Example 270-3 (255 mg, 0.333 mmol) in DMF (4 mL) there was added diethylamine (200 μL, 1.93 mmol), and the mixture was stirred for 20 minutes at room temperature. The reaction mixture was concentrated under reduced pressure and the residue was processed 3 times by azeotropic distillation with toluene. The residue was dissolved in dichloromethane (4 mL), and then triethylamine (185 μL, 1.33 mmol) and acetic anhydride (62.9 μL, 0.665 mmol) were added and the mixture was stirred for 30 minutes at room temperature. The reaction mixture was quenched with an ammonium chloride aqueous solution. The reaction mixture was extracted with dichloromethane and the organic layer was dried over sodium sulfate, after which it was filtered and the filtrate was concentrated under reduced pressure. The residue was dissolved in dichloromethane (4 mL), TFA (2.0 mL) was added, and the mixture was stirred for 1 hour at room temperature. The reaction mixture was concentrated under reduced pressure and then purified by reversed-phase silica gel chromatography (0.1% formic acid aqueous solution:0.1% acetonitrile formate solution = 95:5 to 3:7) to obtain the title compound (160 mg).

ESI-MS (m/z): 531.16 $[M+H]^+$.

[Production Example 270-5]

(S)-3-Acetamide-4-(((3-(5-(3-((1'-(4-((44S,50S,59S)-44-amino-50-benzyl-59-cyclopropoxy-38,45,48,51,54-pentaoxo-59-phenyl-2,5,8,11,14,17,20,23,26,29,32,35,57-tridecaoxa-39,46,49,52,55-pentaazanonapentacontan-59-yl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxo-3,6-dihydropyrimidine-1(2H)-yl)methyl)amino)-4-oxobutanoic acid

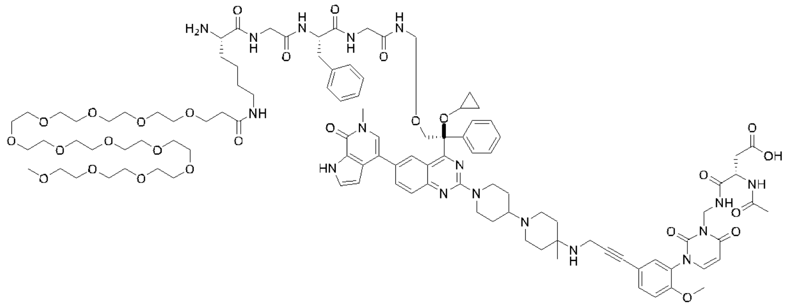

To a solution of the (9H-fluoren-9-yl)methyl ((44S,50S,59S)-50-benzyl-59-cyclopropoxy-59-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-38,45,48,51,54-pentaoxo-59-phenyl-2,5,8,11,14,17,20,23,26,29,32,35,57-tridecaoxa-39,46,49,52,55-pentaazanonapentacontan-44-yl)carbamate of Production Example 201-15 (30.6 mg, 0.016 mmol) in DMF (2.0 mL) there were added the (S)-3-acetamide-4-(((3-(5-iodo-2-methoxyphenyl)-2,6-dioxo-3,6-dihydropyrimidine-1(2H)-yl)methyl)amino)-4-oxobutanoic acid of Production Example 270-4 (11.1 mg, 0.021 mmol), CuI (1.23 mg, 6.45 μmol), N,N-diisopropylethylamine (100 μL, 0.562 mmol) and tetrakis(triphenylphosphine)palladium(0) (7.46 mg, 6.45 μmol), and the mixture was replaced with nitrogen 3 times under reduced pressure and stirred for 1.5 hours at 50°C. After cooling to room temperature, diethylamine(200 μL, 1.93 mmol) was added to the reaction mixture, which was then stirred for 30 minutes. The reaction mixture was concentrated to 1/3 volume and purified by reversed-phase silica gel chromatography (0.1% ammonia water solution:0.1% ammonia-acetonitrile solution = 9:1 to 3:7) to obtain the title compound (13 mg).
ESI-MS (m/z): 1039.69[(M+2H)/2]+.

[Example 271]

(S)-3-Acetamide-4-(((3-(5-(3-((1'-(4-((1S,10S)-10-benzyl-1-cyclopropoxy-26-(2,5-dioxo-3,4-bis(phenylthio)-2,5-dihydro-1H-pyrrol-1-yl)-6,9,12,15,18-pentaoxo-1-phenyl-3,21,24-trioxa-5,8,11,14,17-pentaazahexacosyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxo-3,6-dihydropyrimidine-1(2H)-yl)methyl)amino)-4-oxobutanoic acid

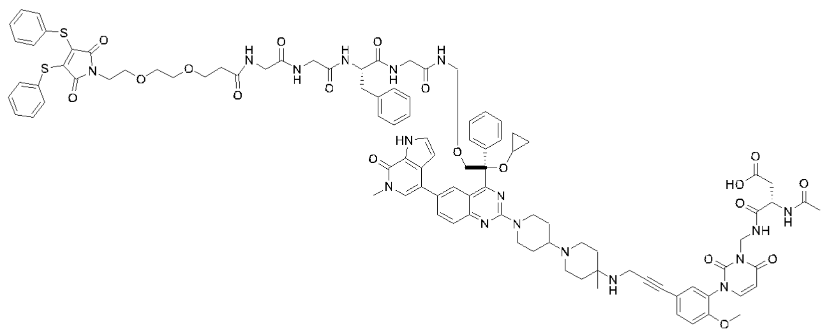

To a solution of the (S)-3-acetamide-4-(((3-(5-(3-((1'-(4-((1S,10S)-16-amino-10-benzyl-1-cyclopropoxy-6,9,12,15-tetraoxo-1-phenyl-3-oxa-5,8,11,14-tetraazahexadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxo-3,6-dihydropyrimidine-1(2H)-yl)methyl)amino)-4-oxobutanoic acid of Production Example 271-1 (14 mg, 0.0098 mmol) in DMF (2.0 mL) there were added the 2,5-dioxopyrrolidin-1-yl 3-(2-(2-(2,5-dioxo-3,4-bis(phenylthio)-2,5-dihydro-1H-pyrrol-1-yl)ethoxy)ethoxy)propanoate of Production Example 201-23 (11 mg, 0.019 mmol) and N,N-diisopropylethylamine (100 μL, 0.56 mmol), and the mixture was stirred for 1 hour at room temperature. The reaction mixture was concentrated to 1/3 volume and purified by reversed-phase silica gel chromatography (0.1% formic acid aqueous solution:0.1% acetonitrile formate solution = 90:10 to 30:70) to obtain a formate of the title compound (6.4 mg).
ESI-MS (m/z): 946.63[(M+2H)/2]+.

[Production Example 271-1]

(S)-3-Acetamide-4-(((3-(5-(3-((1'-(4-((1S,10S)-16-amino-10-benzyl-1-cyclopropoxy-6,9,12,15-tetraoxo-1-phenyl-3-oxa-5,8,11,14-tetraazahexadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxo-3,6-dihydropyrimidine-1(2H)-yl) methyl)amino)-4-oxobutanoic acid

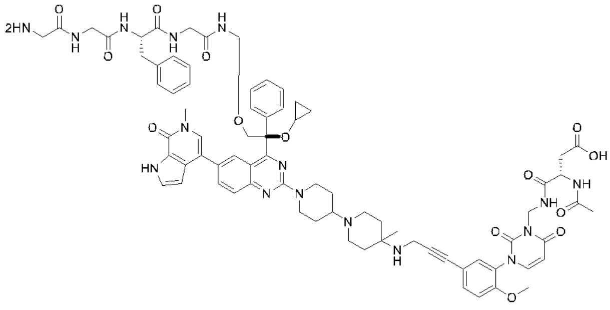

To a solution of the (9H-fluoren-9-yl)methyl((1S,10S)-10-benzyl-1-cyclopropoxy-1-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-6,9,12,15-tetraoxo-1-phenyl-3-oxa-5,8,11,14-tetraazahexadecan-16-yl)carbamate of Production Example 203-1 (47 mg, 0.037 mmol), the (S)-3-acetamide-4-(((3-(5-iodo-2-methoxyphenyl)-2,6-dioxo-3,6-dihydropyrimidine-1(2H)-yl)methyl)amino)-4-oxobutanoic acid of Production Example 270-4 (30 mg, 0.056 mmol), CuI (2.9 mg, 0.015 mmol) and tetrakis(triphenylphosphine)palladium(0) (17 mg, 0.015 mmol) in DMF (2 mL) there was added N,N-diisopropylethylamine (100 μL, 0.056 mmol), and the mixture was replaced with nitrogen 3 times under reduced pressure. The reaction mixture was stirred for 1 hour at 50°C. After cooling to room temperature, diethylamine (100 μL, 0.97 mmol) was added to the reaction mixture, which was then stirred for 30 minutes. The reaction mixture was concentrated to 1/3 volume and purified by reversed-phase silica gel chromatography (0.1% ammonia water solution:0.1% ammonia-acetonitrile solution = 95:5 to 0:100) to obtain the title compound (14 mg).

ESI-MS (m/z): 1436.14 $[M+H]^+$.

[Example 272]

(S)-3-Acetamido-4-(((3-(5-(3-((1'-(4-((1S, 7S)-7-(2-amino-2-oxoethyl)-17-(3,4-bis(4-(ethoxycarbonyl)phenoxy)-2,5-dioxo-2,5-dihydro-1H-pyrrol-1-yl)-1-cyclopropoxy-6,9-dioxo-1-phenyl-3,12,15-trioxa-5,8-diazaheptadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidin-1(2H)-yl)methyl)amino)-4-oxobutanoic acid

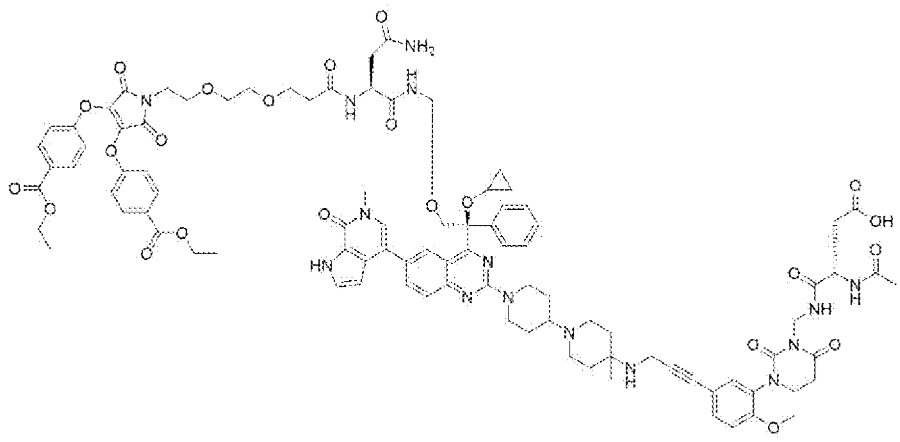

To a solution of the (S)-3-acetamido-4-(((3-(5-(3-((1'-(4-((S)-1-cyclopropoxy-2-(((S)-2,4-diamino-4-oxobutanamido)methoxy)-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidin-1(2H)-yl)methyl)amino)-4-oxobutanoic acid of Production Example 272-2 (180 mg, 0.127 mmol) in DMF (3.0 mL) there was added a solution of N,N-diisopropylethylamine (222 μL, 1.27 mmol) and the crude product of diethyl 4,4'-((1-(2-(2-(3-((2,5-dioxopyrrolidin-1-yl)oxy)-3-oxopropoxy)ethoxy)ethyl)-2,5-dioxo-2,5-dihydro-1H-pyrrole-3,4-diyl)bis(oxy))dibenzoate of Production Example 272-4 (174 mg) in DMF (1.0 mL) at room temperature, and the mixture was stirred for 2 hours at room temperature. The reaction mixture was concentrated to approximately 1/2 to 1/3 volume, and then purified by direct reversed-phase silica gel chromatography (0.1% formic acid aqueous solution:0.1% acetonitrile formate solution = 9:1 to 6:4). The same procedure was carried out again on a reaction scale, and the fractions containing the title compound were

combined and concentrated. The residue was dissolved in dichloromethane, heptane was added and the mixture was concentrated to obtain a formate of the title compound (304 mg).

ESI-MS (m/z): 901.60[(M+2H)/2] +.

1H-NMR Spectrum (700 MHz, DMSO-d6) δ(ppm): 0.16-0.19 (1H, m), 0.22-0.28 (1H, m), 0.39-0.44 (1H, m), 0.64-0.68 (1H, m), 1.12 (3H, s), 1.27 (6H, t, J=7.2 Hz), 1.53-1.60 (2H, m), 1.54-1.59 (2H, m), 1.61-1.78 (2H, m), 1.81 (3H, s), 1.95-2.09 (2H, m), 2.36 (2H, brt, J=6.6 Hz), 2.38-2.42 (1H, m), 2.39-2.44 (1H, m), 2.46-2.51 (1H, m), 2.57 (1H, dd, J=16.6, 5.7 Hz), 2.68-2.75 (2H, m), 2.78 (2H, br t, J=6.8 Hz), 2.87-2.92 (2H, m), 2.90-2.97 (1H, m), 3.00-3.06 (2H, m), 3.05-3.08 (1H, m), 3.42-3.45 (2H, m), 3.47-3.50 (2H, m), 3.51-3.54 (2H, m), 3.52 (3H, s), 3.51-3.54 (2H, m), 3.55-3.58 (2H, m), 3.57-3.59 (2H, m), 3.57-3.60 (2H, m), 3.81 (3H, s), 4.21-4.26 (1H, m), 4.25 (4H, q, J=7.2 Hz), 4.39 (1H, br dd, J=10.2, 6.8 Hz), 4.46-4.50 (1H, m), 4.48-4.52 (1H, m), 4.53 (1H, td, J=7.9, 5.3 Hz), 4.76 (1H, br d, J=11.2 Hz), 4.91-4.96 (2H, m), 4.99 (1H, br dd, J=12.3, 5.1 Hz), 5.10 (1H, br dd, J=12.3, 5.9 Hz), 5.86 (1H, t, J=2.3 Hz), 6.85 (1H, br s), 7.10 (1H, d, J=8.7 Hz), 7.19 (4H, d, J=8.9 Hz), 7.18-7.21 (1H, m), 7.20-7.22 (1H, m), 7.23-7.26 (2H, m), 7.25-7.28 (2H, m), 7.26-7.29 (2H, m), 7.33-7.35 (1H, m), 7.34-7.37 (1H, m), 7.53 (1H, d, J=8.9 Hz), 7.77 (1H, dd, J=8.9, 1.9 Hz), 7.80 (4H, d, J=8.9 Hz), 8.02 (1H, br d, J=7.9 Hz), 8.04-8.07 (1H, m), 8.12-8.14 (1H, m), 8.14 (2H, s), 8.14 (1H, m), 8.43 (1H, br t, J=6.8 Hz), 12.08 (1H, br s).

[Production Example 272-1]

(S)-2-Amino-N1-(((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)succinamide

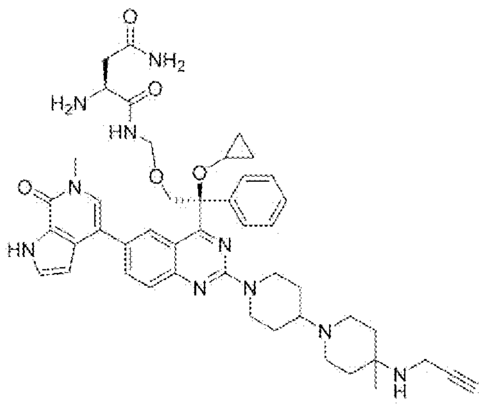

To a solution of the (S)-4-(4-(1-cyclopropoxy-2-hydroxy-1-phenylethyl)-2-(4-methyl-4-(prop-2-yn-1-ylamino)-[1,4'-bipiperidin]-1'-yl)quinazolin-6-yl)-6-methyl-1,6-dihydro-7H-pyrrolo[2,3-c]pyridin-7-one of Production Example 201-9 (550 mg, 0.802 mmol), the (S)-(2-((((9H-fluoren-9-yl)methoxy)carbonyl)amino)-4-amino-4-oxobutanamido)methyl acetate of Production Example 204-3 (1.37 g, 3.21 mmol) and CSA (745 mg, 3.21 mmol) in dichloromethane (44 mL), there was added dropwise TMSCl (430 μL, 3.37 mmol) at room temperature over a period of 5 minutes. The reaction mixture was stirred for 150 minutes at room temperature and then added to a solution of ice-cooled triethylamine (469 μL, 3.37 mmol) in methanol (88 mL). The mixture was stirred for 1 hour at room temperature and filtered, and the filtrate was then concentrated. The residue was dissolved in DMF (2.75 mL), and diethylamine (1.68 mL, 16.0 mmol) was added at room temperature. After stirring the reaction mixture at room temperature for 45 minutes, it was diluted with a 10% methanol/dichloromethane solution (16.5 mL), saturated sodium bicarbonate water (7.2 mL) and water (4.5 mL). The organic layer was separated off, and the aqueous layer was extracted 4 times with a 10% methanol/dichloromethane solution (16.5 mL). The combined organic layers were concentrated and then purified by reversed-phase silica gel chromatography (0.1% aqueous acetic acid solution:0.1% acetonitrile acetate solution = 10:0 to 8:2). The fractions containing the title compound were collected, the acetonitrile was removed by concentration under reduced pressure, and then a 10% methanol/dichloromethane solution (120 mL) and saturated sodium bicarbonate water (20 mL) were added. The organic layer was separated off, and the aqueous layer was extracted 3 times with a 10% methanol/dichloromethane solution (120 mL). The combined organic layers were concentrated to obtain the title compound (208 mg).

ESI-MS (m/z): 829.62[M+H] +.

1H-NMR Spectrum (500 MHz, CDCl3) δ(ppm): 0.18-0.31 (1H, m), 0.36-0.49 (2H, m), 0.77-0.88 (1H, m), 1.12 (3H, s), 1.42-1.80 (6H, m), 2.00-2.10 (2H, m), 2.16-2.21 (1H, m), 2.49-2.75 (7H, m), 2.97-3.08 (2H, m), 3.12-3.17 (1H, m), 3.38 (2H, s), 3.61-3.66 (4H, m), 4.31-4.38 (1H, m), 4.59-4.73 (2H, m), 4.89-4.98 (1H, m), 5.00-5.12 (2H, m), 5.37-5.46 (1H, m), 6.02-6.20 (2H, m), 6.64-6.77 (1H, m), 7.16-7.30 (4H, m), 7.35-7.42 (2H, m), 7.55-7.60 (1H, m), 7.66-7.71 (1H, m),

7.81-7.86 (1H, m), 8.08-8.17 (1H, m), 9.66-9.77 (1H, m).

[Production Example 272-2]

(S)-3-Acetamido-4-(((3-(5-(3-((1'-(4-((S)-1-cyclopropoxy-2-(((S)-2,4-diamino-4-oxobutanamido)methoxy)-1-phenylethyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidin-1(2H)-yl)methyl)amino)-4-oxobutanoic acid

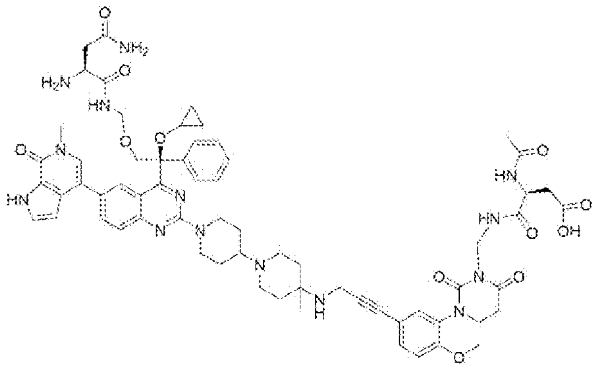

To the (S)-2-amino-N1-(((S)-2-cyclopropoxy-2-(2-(4-methyl-4-(prop-2-yn-1-ylamino)-[1,4'-bipiperidin]-1'-yl)-6-(6-methyl-7-oxo-6,7-dihydro-1H-pyrrolo[2,3-c]pyridin-4-yl)quinazolin-4-yl)-2-phenylethoxy)methyl)succinamide of Production Example 272-1 (200 mg, 0.241 mmol), the (S)-3-acetamido-4-(((3-(5-iodo-2-methoxyphenyl)-2,6-dioxotetrahydropyrimidin-1(2H)-yl)methyl)amino)-4-oxobutanoic acid of Production Example 201-19 (212 mg, 0.398 mmol), CuI (13.8 mg, 0.072 mmol) and tetrakis(triphenylphosphine)palladium(0) (27.9 mg, 0.024 mmol) there was added a solution of N,N-diisopropylethylamine (421 μL, 2.41 mmol) in DMF (3.74 mL) at room temperature under a nitrogen atmosphere, and the mixture was stirred for 2 hours at room temperature. The reaction mixture was concentrated and then purified by direct reversed-phase silica gel chromatography (0.1% acetic acid aqueous solution:0.1% acetonitrile acetate solution = 9:1 to 7:3) to obtain an acetate of the title compound (274 mg).

ESI-MS (m/z): 617.71 [(M+2H)/2] +.

1H-NMR Spectrum (500 MHz, DMSO-d6) δ(ppm): 0.18-0.33 (2H, m), 0.37-0.49 (1H, m), 0.68-0.75 (1H, m), 1.11 (3H, s), 1.49-1.61 (4H, m), 1.62-1.74 (2H, m), 1.81 (3H, s), 1.95-2.03 (2H, m), 2.17-2.30 (1H, m), 2.34-3.68 (21H, m), 3.81 (3H, s), 4.22-4.31 (1H, m), 4.38-4.46 (1H, m), 4.46-4.58 (2H, m), 4.73-4.79 (1H, m), 4.87-4.97 (2H, m), 4.97-5.05 (1H, m), 5.07-5.18 (1H, m), 5.84-5.91 (1H, m), 6.86-6.99 (1H, m), 7.07-7.13 (1H, m), 7.17-7.31 (7H, m), 7.33-7.40 (2H, m), 7.41-7.47 (1H, m), 7.50-7.55 (1H, m), 7.75-7.80 (1H, m), 8.05-8.10 (1H, m), 8.11-8.18 (2H, m), 8.65-8.76 (1H, m), 12.03-12.18 (1H, m) .

[Production Example 272-3]

Diethyl 4,4'-((1-(2-(2-(3-(tert-butoxy)-3-oxopropoxy)ethoxy)ethyl)-2,5-dioxo-2,5-dihydro-1H-pyrrole-3,4-diyl)bis(oxy))dibenzoate

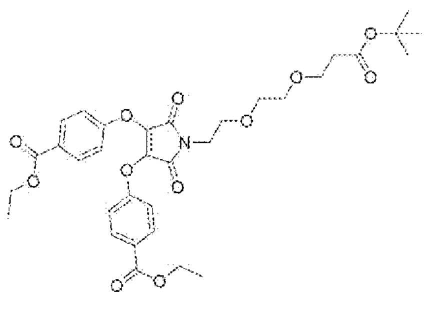

After adding potassium carbonate (704 mg, 5.09 mmol) to a solution of ethyl 4-hydroxybenzoate (635 mg, 3.82

mmol) in DMF (10 mL) and stirring for 10 minutes at room temperature, a solution of the tert-butyl 3-(2-(2-(3,4-dibromo-2,5-dioxo-2,5-dihydro-1H-pyrrol-1-yl)ethoxy)ethoxy)propanoate of Production Example 1-21 (600 mg, 1.27 mmol) in DMF (2 mL) was added and the mixture was stirred for 4 hours at room temperature. After adding MTBE to the reaction mixture and washing the organic layer with water, it was dried over sodium sulfate. The filtrate was concentrated under reduced pressure and the residue was purified by silica gel chromatography (heptane:ethyl acetate = 70:30 to 20:80) to obtain the title compound (348 mg).

ESI-MS (m/z): 659.41[M+18] +.

1H-NMR Spectrum (400 MHz, CDCl3) δ(ppm): 1.34-1.38(6H, m), 1.44(9H, s), 2.47-2.51(2H, m), 3.56-3.62 (4H, m), 3.67-3.72(4H, m), 3.75-3.77 (2H, m), 4.30-4.35(4H, m), 6.89-6.92(4H, m), 7.87-7.90(4H, m).

[Production Example 272-4]

Diethyl 4,4'-((1-(2-(2-(3-((2,5-dioxopyrrolidin-1-yl)oxy)-3-oxopropoxy)ethoxy)ethyl)-2,5-dioxo-2,5-dihydro-1H-pyrrole-3,4-diyl)bis(oxy))dibenzoate

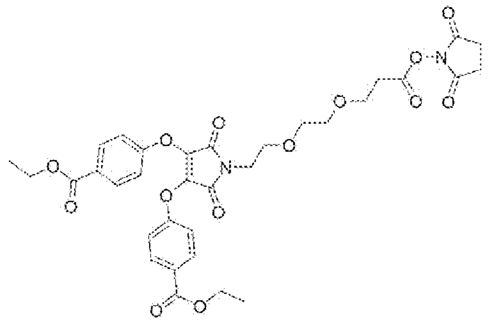

To a solution of the diethyl 4,4'-((1-(2-(2-(3-(tert-butoxy)-3-oxopropoxy)ethoxy)ethyl)-2,5-dioxo-2,5-dihydro-1H-pyrrole-3,4-diyl)bis(oxy))dibenzoate of Production Example 272-3 (348 mg, 0.542 mmol) in dichloromethane (2 mL) there was added trifluoroacetic acid (1 mL, 13.0 mmol), and the mixture was stirred for 1 hour at room temperature. The reaction mixture was concentrated under reduced pressure and the redissolved in dichloromethane (1 mL), after which it was again concentrated under reduced pressure and the residue was dissolved in dichloromethane (2 mL). After adding N,N-diisopropylethylamine (0.92 mL, 5.42 mmol) and TSTU (= N,N,N',N'-tetramethyl-O-(N-succinimidyl)uronium tetrafluoroborate) (212 mg, 0.71 mmol) to the reaction mixture, it was stirred for 2 hours at room temperature. The reaction mixture was diluted with ethyl acetate and water, and the organic layer was washed with 1 N hydrochloric acid and water in that order, and then dried over sodium sulfate and subsequently filtered. The filtrate was concentrated under reduced pressure to obtain a crude product of the title compound (449 mg).

ESI-MS (m/z): 683.33[M+H] +.

1H-NMR Spectrum (400 MHz, CDCl3) δ(ppm): 1.36 (6H, q, J=8.0 Hz), 2.82-2.89 (6H, m), 3.62-3.71 (6H, m), 3.75-3.78 (2H, m), 3.84 (2H, t, J=6.0 Hz), 4.34 (4H, q, J=8.0 Hz), 6.89-6.93 (4H, m), 7.86-7.90 (4H, m).

[Example 301-1] Creation of human anti-CEACAM6 monoclonal antibody For creation of a monoclonal antibody to bind with human CEACAM6 (UniProtKB P40199, SEQ ID NO: 1), a protein comprising secreted alkaline phosphatase (SEAP) and a histidine tag fused to the extracellular domain of human CEACAM6 (position 35 to 320) (SEQ ID NO: 2) (hereunder referred to as "human CEACAM6 extracellular domain-SEAP-His protein", SEQ ID NO: 3), and a protein comprising maltose-binding protein (MBP) and a histidine tag fused to the extracellular domain of human CEACAM6 (hereunder referred to as "human CEACAM6 extracellular domain-MBP-His protein", SEQ ID NO: 4), were prepared by the following steps.

First, a pcDNA3.4-human CEACAM6 extracellular domain-SEAP-His expression vector and a pcDNA3.4-human CEACAM6 extracellular domain-MBP-His expression vector were constructed. GenScript was used to synthesize a gene comprising the gene coding for the signal sequence (SEQ ID NO: 5) added to the gene coding for the human CEACAM6 extracellular domain. The synthesized gene was cloned in pcDNA3.4 vector having the DNA sequence coding for SEAP and the histidine tag (Invitrogen/LifeTechnologies), or pcDNA3.4 vector having the DNA sequence coding for MBP and the histidine tag, to construct pcDNA3.4-human CEACAM6 extracellular domain-SEAP-His expression vector and pcDNA3.4-human CEACAM6 extracellular domain-MBP-His expression vector. An Expi293 expression system (Thermo Scientific) was used for transfection of each expression vector into Expi293F cells (Thermo Scientific). After 4 days, the culture solution was collected and the cells were removed for clarification. TALON resin (TaKaRa) was used for purification, and the buffer was exchanged with PBS (FujiFilm-Wako Pure) by dialysis.

Screening was carried out using human CEACAM6 protein and a fully human antibody synthetic phage library, to obtain a human antibody fragment (scFv) specifically binding with human CEACAM6. Dynabeads magnetic beads (Thermo Scientific) were used to capture the human CEACAM6 extracellular domain-SEAP-His protein, the fully human antibody synthetic phage library was added, and after 1 hour the non-bound phage was removed in a series of wash cycles using PBS-Tween (0.1% v/v) or PBS. The bound phage particles were eluted out and amplified by infection in *E. coli* TG1 host cells. The infected TG1 cells were collected, spread on a plate and incubated at 30°C. The same panning procedure was carried out on the phage library prepared from the infected TG1 cells, using the Dynabeads magnetic beads that had captured the human CEACAM6 extracellular domain-MBP-His protein.

After the second panning procedure, a single colony from the concentrated phage-infected TG1 cells was transferred to culture medium on a 96-well plate. IPTG was added to induce expression of the FLAG-tagged scFv, and vibration culture was carried out overnight at 30°C. The TG1 cells were spinned down and the scFv-containing *E. coli* culture supernatant was used to pickup the wells that were reactive with human CEACAM6.

Reactivity with human CEACAM6 was evaluated by ELISA according to the following procedure, using either human CEACAM6 extracellular domain-SEAP-His protein or human CEACAM6 extracellular domain-MBP-His protein. The wells of a 96-well plate (Nunc) were coated with anti-FLAG antibody (Sigma). After incubation overnight at 4°C, the wells were blocked with 2% skim milk (BD) for 2 hours at room temperature. After rinsing 3 times with 0.02% Tween20/PBS (Nacalai Tesque, Inc.), human CEACAM6 extracellular domain-SEAP-His protein or human CEACAM6 extracellular domain-MBP-His protein (final concentration: 20 nM) and *E. coli* culture supernatant containing scFv were added to each well and incubation was continued for 2 hours at room temperature. After rinsing 3 times, horseradish peroxidase-labeled anti-His antibody (MBL) was added and the cells were incubated for 1 hour at room temperature. After rinsing 5 times, TMBZ (3,3',5,5'-tetramethylbenzidine, KPL) solution was added to the wells, and incubation was continued for 15 to 20 minutes at room temperature. An equivalent of stopping solution (1N $H_2SO_4$, FujiFilm Wako Pure) was added to each well, and the absorbance at 450 nm was read off using a microplate reader (Thermo Scientific). A human antibody fragment specifically binding to human CEACAM6 was selected out by screening and sequenced to determine the gene sequence of the fragment.

Amino acid sequences of the heavy chain and light chain variable regions included in the gene-sequenced human antibody fragment were combined with the amino acid sequence of the constant region to design a human CEACAM6-specific antibody (hereunder referred to as "antibody A", or alternatively "CEACAM6_#84.7"). Genes coding for the full lengths of the heavy chain and light chain of antibody A were synthesized using GenScript. The amino acid sequence of the heavy chain variable region of antibody A is the amino acid sequence listed as SEQ ID NO: 6, and the amino acid sequence of the light chain variable region is the amino acid sequence listed as SEQ ID NO: 7. The gene sequences coding for the amino acid sequences of antibody A were the nucleic acid sequence listed as SEQ ID NO: 8 for the heavy chain variable region and the nucleic acid sequence listed as SEQ ID NO: 9 for the light chain variable region. For the heavy chain constant region of antibody A there was used the constant region of human IgG1/2m (SEQ ID NO: 10), as the constant region of human IgG2 having human IgG1 at the CH1 portion and hinge portion, and V234A and G237A at the CH2 portion and CH3 portion. Here, "V234A" is a mutation where the valine at position 234 is replaced by alanine, and "G237A" is a mutation where the glycine at position 237 is replaced by alanine, according to Eu numbering. The CH1 portion is from position 118 to position 215 of the human IgG constant region, the hinge portion is from position 216 to position 230 of the human IgG constant region, and the CH2 portion is from position 231 to position 340 and the CH3 portion is from position 341 to position 446 of the human IgG constant region (Eu numbering). Human Igλ (SEQ ID NO: 11) was used for the light chain constant region of antibody A. The gene sequences coding for the amino acid sequences of antibody A were the nucleic acid sequence listed as SEQ ID NO: 12 for the heavy chain constant region and the nucleic acid sequence listed as SEQ ID NO: 13 for the light chain constant region. The amino acid sequence of the full length heavy chain of antibody A (without the signal sequence) is the amino acid sequence listed as SEQ ID NO: 14, and the amino acid sequence of the full length light chain (without the signal sequence) is the amino acid sequence listed as SEQ ID NO: 15. The nucleic acid sequence coding for the full length heavy chain of antibody A is the nucleic acid sequence listed as SEQ ID NO: 16, and the nucleic acid sequence coding for the full length light chain is the nucleic acid sequence listed as SEQ ID NO: 17.

A GS system (Lonza) expression vector was constructed for each synthesized gene. After incorporating the heavy chain into an expression vector (pEE6.4, Lonza) and the light chain into an expression vector (pEE12.4, Lonza), a double gene vector was constructed with the heavy chain and light chain loaded onto a single vector. This was introduced into CHO-K1SV cells, and selectively cultured with glutamine synthase inhibitor MSX (methionine sulfoximine) as a selective marker to obtain a stable expressing line. An F. Sight single cell printer (Cytena) was used for cell cloning to obtain a single cell line, and then a 3L Bioreactor was used for fed-batch culturing. The culture supernatant was collected and large-scale purification of human anti-CEACAM6_#84.7 monoclonal antibody was carried out using an Amsphere A3 (JSR) and Millistak+ Pod X0HC depth filter (Millipore). The purified antibody was exchanged with 25 mM histidine buffer and placed in refrigerated storage.

The CDR of antibody A was determined by IMGT numbering for CDR identification. The amino acid sequences

and nucleic acid sequences of the CDRs of antibody A are shown in Table 5 and Table 6.

[Table 5]

| Amino acid sequences of antibody A CDRs | |
|---|---|
| Name | Sequence |
| Heavy chain CDR1 | GFTFSSYA (SEQ ID NO: 18) |
| Heavy chain CDR2 | ISGSGGST (SEQ ID NO: 19) |
| Heavy chain CDR3 | ARYWIWWHYGFDV (SEQ ID NO: 20) |
| Light chain CDR1 | SLRSYY (SEQ ID NO: 21) |
| Light chain CDR2 | GKN (SEQ ID NO: 21-1) |
| Light chain CDR3 | QSYYSSSHYVV (SEQ ID NO: 22) |

CDR amino acid sequences of antibody A

[Table 6]

| Nucleic acid sequences of antibody A CDRs | |
|---|---|
| Name | Sequence |
| Heavy chain CDR1 | GGCTTCACCTTTTCCAGCTACGCT (SEQ ID NO: 23) |
| Heavy chain CDR2 | ATCAGCGGCTCTGGCGGCTCCACA (SEQ ID NO: 24) |
| Heavy chain CDR3 | GCTCGGTACTGGATCTGGTGGCACTATGGATTTGACGTG (SEQ ID NO: 25) |
| Light chain CDR1 | TCTCTGCGCTCCTACTAT (SEQ ID NO: 26) |
| Light chain CDR2 | GGCAAGAAC (SEQ ID NO: 26-1) |
| Light chain CDR3 | CAGTCTTACTATTCCAGCTCTCACTACGTGGTG (SEQ ID NO: 27) |

CDR nucleic acid sequences of antibody A

SEQ ID NO: 1 Human CEACAM6

MGPPSAPPCRLHVPWKEVLLTASLLTFWNPPTTAKLTIESTPFNVAEGKEVLLLAH
NLPQNRIGYSWYKGERVDGNSLIVGYVIGTQQATPGPAYSGRETIYPNASLLIQNV
TQNDTGFYTLQVIKSDLVNEEATGQFHVYPELPKPSISSNNSNPVEDKDAVAFTCE
PEVQNTTYLWWVNGQSLPVSPRLQLSNGNMTLTLLSVKRNDAGSYECEIQNPAS
ANRSDPVTLNVLYGPDVPTISPSKANYRPGENLNLSCHAASNPPAQYSWFINGTF
QQSTQELFIPNITVNNSGSYMCQAHNSATGLNRTTVTMITVSGSAPVLSAVATVGI
TIGVLARVALI

SEQ ID NO: 2 Human CEACAM6 extracellular domain

KLTIESTPFNVAEGKEVLLLAHNLPQNRIGYSWYKGERVDGNSLIVGYVIGTQQA
TPGPAYSGRETIYPNASLLIQNVTQNDTGFYTLQVIKSDLVNEEATGQFHVYPELP
KPSISSNNSNPVEDKDAVAFTCEPEVQNTTYLWWVNGQSLPVSPRLQLSNGNMT
LTLLSVKRNDAGSYECEIQNPASANRSDPVTLNVLYGPDGPTISPSKANYRPGENL
NLSCHAASNPPAQYSWFINGTFQQSTQELFIPNITVNNSGSYMCQAHNSATGLNR
TTVTMITVSG

SEQ ID NO: 3 Human CEACAM6 extracellular domain-SEAP-His protein

KLTIESTPFNVAEGKEVLLLAHNLPQNRIGYSWYKGERVDGNSLIVGYVIGTQQA
TPGPAYSGRETIYPNASLLIQNVTQNDTGFYTLQVIKSDLVNEEATGQFHVYPELP
KPSISSNNSNPVEDKDAVAFTCEPEVQNTTYLWWVNGQSLPVSPRLQLSNGNMT
LTLLSVKRNDAGSYECEIQNPASANRSDPVTLNVLYGPDGPTISPSKANYRPGENL
NLSCHAASNPPAQYSWFINGTFQQSTQELFIPNITVNNSGSYMCQAHNSATGLNR

TTVTMITVSGAAAIIPVEEENPDFWNREAAEALGAAKKLQPAQTAAKNLIIFLGD
GMGVSTVTAARILKGQKKDKLGPEIPLAMDRFPYVALSKTYNVDKHVPDSGATA
TAYLCGVKGNFQTIGLSAAARFNQCNTTRGNEVISVMNRAKKAGKSVGVVTTT
RVQHASPAGTYAHTVNRNWYSDADVPASARQEGCQDIATQLISNMDIDVILGGG
RKYMFRMGTPDPEYPDDYSQGGTRLDGKNLVQEWLAKRQGARYVWNRTELM
QASLDPSVTHLMGLFEPGDMKYEIHRDSTLDPSLMEMTEAALRLLSRNPRGFFLF
VEGGRIDHGHHESRAYRALTETIMFDDAIERAGQLTSEEDTLSLVTADHSHVFSFG
GYPLRGSSIFGLAPGKARDRKAYTVLLYGNGPGYVLKDGARPDVTESESGSPEY
RQQSAVPLDEETHAGEDVAVFARGPQAHLVHGVQEQTFIAHVMAFAACLEPYTA
CDLAPPAGTTDAAHPGHHHHHHHHHH

SEQ ID NO: 4 Human CEACAM6 extracellular domain-MBP-His protein

KLTIESTPFNVAEGKEVLLLAHNLPQNRIGYSWYKGERVDGNSLIVGYVIGTQQA
TPGPAYSGRETIYPNASLLIQNVTQNDTGFYTLQVIKSDLVNEEATGQFHVYPELP
KPSISSNNSNPVEDKDAVAFTCEPEVQNTTYLWWVNGQSLPVSPRLQLSNGNMT
LTLLSVKRNDAGSYECEIQNPASANRSDPVTLNVLYGPDGPTISPSKANYRPGENL
NLSCHAASNPPAQYSWFINGTFQQSTQELFIPNITVNNSGSYMCQAHNSATGLNR
TTVTMITVSGAAAEEGKLVIWINGDKGYNGLAEVGKKFEKDTGIKVTVEHPDKL
EEKFPQVAATGDGPDIIFWAHDRFGGYAQSGLLAEITPDKAFQDKLYPFTWDAVR
YNGKLIAYPIAVEALSLIYNKDLLPNPPKTWEEIPALDKELKAKGKSALMFNLQEP
YFTWPLIAADGGYAFKYENGKYDIKDVGVDNAGAKAGLTFLVDLIKNKHMNAD
TDYSIAEAAFNKGETAMTINGPWAWSNIDTSKVNYGVTVLPTFKGQPSKPFVGV
LSAGINAASPNKELAKEFLENYLLTDEGLEAVNKDKPLGAVALKSYEEELAKDPR
IAATMENAQKGEIMPNIPQMSAFWYAVRTAVINAASGRQTVDEALKDAQTHHHH
HHHHHH

SEQ ID NO: 5 Signal sequence
MEWSWVFLFFLSVTTGVHS

SEQ ID NO: 6 Antibody A heavy chain variable region amino acid sequence

EVQLLESGGGLVQPGGSLRLSCAASGFTFSSYAMSWVRQAPGKGLEWVSAISGS
GGSTYYADSVKGRFTISRDNSKNTLYLQMNSLRAEDTAVYYCARYWIWWHYGF
DVWGQGTLVTVSS

SEQ ID NO: 7 Antibody Alight chain variable region amino acid sequence

SSELTQDPAVSVALGQTVRITCQGDSLRSYYASWYQQKPGQAPVLVIYGKNNRPS
GIPDRFSGSSSGNTASLTITGAQAEDEADYYCQSYYSSSHYVVFGGGTKLTVL

SEQ ID NO: 8 Antibody A heavy chain variable region gene sequence

GAAGTGCAGCTGCTGGAATCCGGGGGAGGGCTGGTGCAGCCCGGCGGCTCT
CTGAGACTGTCCTGTGCTGCCTCCGGCTTCACCTTTTCCAGCTACGCTATGTCT
TGGGTGCGGCAGGCTCCTGGCAAGGGACTGGAGTGGGTGTCTGCTATCAGCG
GCTCTGGCGGCTCCACATACTATGCCGACAGCGTGAAGGGCAGGTTCACCATC
TCTCGGGATAACTCCAAGAATACACTGTATCTGCAGATGAACTCTCTGAGGGC
TGAGGACACCGCCGTGTACTATTGCGCTCGGTACTGGATCTGGTGGCACTATG
GATTTGACGTGTGGGGACAGGGCACCCTGGTGACAGTGTCTTCC

SEQ ID NO: 9 Antibody A light chain variable region gene sequence

TCTAGTGAACTGACCCAGGATCCCGCCGTCTCTGTGGCCCTGGGCCAGACAG TGAGAATCACCTGCCAGGGCGACTCTCTGCGCTCCTACTATGCCTCTTGGTAC CAGCAGAAGCCAGGCCAGGCTCCCGTGCTGGTCATCTACGGCAAGAACAATA GGCCTTCCGGCATCCCAGATCGGTTTAGCGGCTCCAGCTCTGGCAACACAGCC AGCCTGACAATTACAGGAGCTCAGGCTGAGGATGAGGCTGATTACTATTGCCA GTCTTACTATTCCAGCTCTCACTACGTGGTGTTCGGGGGAGGGACAAAGCTGA CCGTGCTG

SEQ ID NO: 10 Human IgG1/2m amino acid sequence

ASTKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKRVEPKSCDKTHTCPPC PAPPAAAPSVFLFPPKPKDTLMISRTPEVTCVVVDVSHEDPEVQFNWYVDGVEV HNAKTKPREEQFNSTFRVVSVLTVVHQDWLNGKEYKCKVSNKGLPAPIEKTISK TKGQPREPQVYTLPPSREEMTKNQVSLTCLVKGFYPSDIAVEWESNGQPENNYKT TPPMLDSDGSFFLYSKLTVDKSRWQQGNVFSCSVMHEALHNHYTQKSLSLSPGK

SEQ ID NO: 11 Human Igλ amino acid sequence

GQPKAAPSVTLFPPSSEELQANKATLVCLISDFYPGAVTVAWKADSSPVKAGVET TTPSKQSNNKYAASSYLSLTPEQWKSHRSYSCQVTHEGSTVEKTVAPTECS

SEQ ID NO: 12 Human IgG1/2m gene sequence

GCCTCCACAAAGGGACCATCCGTGTTCCCACTGGCCCCAAGCTCTAAGTCCA CCAGCGGCGGCACAGCCGCTCTGGGCTGTCTGGTGAAGGACTACTTCCCTGA

GCCAGTGACCGTGAGCTGGAACTCTGGCGCTCTGACCTCCGGCGTGCATACATTTCCAGCCGTGCTGCAGTCCAGCGGCCTGTACAGCCTGTCTTCCGTGGTGACCGTGCCCAGCTCTTCCCTGGGCACCCAGACATATATCTGCAACGTGAATCACAAGCCTAGCAATACAAAGGTGGACAAGAGAGTGGAGCCAAAGTCTTGTGATAAGACCCATACATGCCCTCCATGTCCTGCCCCACCCGCCGCTGCCCCTTCCGTGTTCCTGTTTCCTCCAAAGCCAAAGGACACCCTGATGATCTCTCGCACCCCTGAGGTGACATGCGTGGTGGTGGACGTGAGCCACGAGGATCCTGAGGTGCAGTTTAACTGGTACGTGGATGGCGTGGAGGTGCATAATGCTAAGACAAAGCCAAGAGAGGAGCAGTTCAACTCTACCTTTCGCGTGGTGTCCGTGCTGACAGTGGTGCACCAGGATTGGCTGAACGGCAAGGAGTATAAGTGCAAGGTGTCTAATAAGGGCCTGCCCGCCCCTATCGAGAAGACCATCTCCAAGACAAAGGGCCAGCCTAGAGAGCCACAGGTGTACACCCTGCCCCCTTCTCGCGAGGAGATGACCAAGAACCAGGTGTCCCTGACATGTCTGGTGAAGGGCTTCTATCCCTCCGACATCGCTGTGGAGTGGGAGAGCAATGGCCAGCCTGAGAACAATTACAAGACCACACCACCCATGCTGGACTCCGATGGCAGCTTCTTTCTGTATAGCAAGCTGACCGTGGATAAGTCTAGGTGGCAGCAGGGCAACGTGTTCTCCTGTTCCGTGATGCACGAAGCCCTGCACAACCATTATACTCAGAAGAGCCTGTCCCTGTCCCCTGGCAAA

SEQ ID NO: 13 Human Igλ gene sequence

GGACAGCCAAAGGCTGCTCCTTCTGTGACCCTGTTTCCCCCTTCCAGCGAGGAGCTGCAGGCCAATAAGGCCACCCTGGTGTGCCTGATCAGCGACTTCTATCCAGGAGCTGTGACAGTGGCTTGGAAGGCTGATTCTTCCCCAGTGAAGGCTGGCGTGGAGACCACAACCCCCAGCAAGCAGTCTAACAATAAGTACGCCGCCTCCTCTTATCTGTCTCTGACACCAGAGCAGTGGAAGTCCCATAGGTCCTACAGCTGCCAGGTGACCCACGAAGGTAGCACAGTCGAAAAAACCGTCGCCCCCACAGAGTGTTCT

SEQ ID NO: 14 Antibody A heavy chain full-length amino acid sequence (without signal sequence)

EVQLLESGGGLVQPGGSLRLSCAASGFTFSSYAMSWVRQAPGKGLEWVSAISGSGGSTYYADSVKGRFTISRDNSKNTLYLQMNSLRAEDTAVYYCARYWIWWHYGFDVWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKR

VEPKSCDKTHTCPPCPAPPAAAPSVFLFPPKPKDTLMISRTPEVTCVVVDVSHEDP
EVQFNWYVDGVEVHNAKTKPREEQFNSTFRVVSVLTVVHQDWLNGKEYKCKV
SNKGLPAPIEKTISKTKGQPREPQVYTLPPSREEMTKNQVSLTCLVKGFYPSDIAVE
WESNGQPENNYKTTPPMLDSDGSFFLYSKLTVDKSRWQQGNVFSCSVMHEALH
NHYTQKSLSLSPGK

SEQ ID NO: 15 Antibody Alight chain full-length amino acid sequence (without signal sequence)

SSELTQDPAVSVALGQTVRITCQGDSLRSYYASWYQQKPGQAPVLVIYGKNNRPS
GIPDRFSGSSSGNTASLTITGAQAEDEADYYCQSYYSSSHYVVFGGGTKLTVLGQ
PKAAPSVTLFPPSSEELQANKATLVCLISDFYPGAVTVAWKADSSPVKAGVETTTP
SKQSNNKYAASSYLSLTPEQWKSHRSYSCQVTHEGSTVEKTVAPTECS

SEQ ID NO: 16 Antibody A heavy chain full-length gene sequence (without signal sequence)

GAAGTGCAGCTGCTGGAATCCGGGGGAGGGCTGGTGCAGCCCGGCGGCTCT
CTGAGACTGTCCTGTGCTGCCTCCGGCTTCACCTTTTCCAGCTACGCTATGTCT
TGGGTGCGGCAGGCTCCTGGCAAGGGACTGGAGTGGGTGTCTGCTATCAGCG
GCTCTGGCGGCTCCACATACTATGCCGACAGCGTGAAGGGCAGGTTCACCATC
TCTCGGGATAACTCCAAGAATACACTGTATCTGCAGATGAACTCTCTGAGGGC
TGAGGACACCGCCGTGTACTATTGCGCTCGGTACTGGATCTGGTGGCACTATG
GATTTGACGTGTGGGGACAGGGCACCCTGGTGACAGTGTCTTCCGCCTCCAC
AAAGGGACCATCCGTGTTCCCACTGGCCCCAAGCTCTAAGTCCACCAGCGGC
GGCACAGCCGCTCTGGGCTGTCTGGTGAAGGACTACTTCCCTGAGCCAGTGA
CCGTGAGCTGGAACTCTGGCGCTCTGACCTCCGGCGTGCATACATTTCCAGCC
GTGCTGCAGTCCAGCGGCCTGTACAGCCTGTCTTCCGTGGTGACCGTGCCCA
GCTCTTCCCTGGGCACCCAGACATATATCTGCAACGTGAATCACAAGCCTAGC
AATACAAAGGTGGACAAGAGAGTGGAGCCAAAGTCTTGTGATAAGACCCATA
CATGCCCTCCATGTCCTGCCCCACCCGCCGCTGCCCCTTCCGTGTTCCTGTTTC
CTCCAAAGCCAAAGGACACCCTGATGATCTCTCGCACCCCTGAGGTGACATG
CGTGGTGGTGGACGTGAGCCACGAGGATCCTGAGGTGCAGTTTAACTGGTAC
GTGGATGGCGTGGAGGTGCATAATGCTAAGACAAAGCCAAGAGAGGAGCAG
TTCAACTCTACCTTTCGCGTGGTGTCCGTGCTGACAGTGGTGCACCAGGATTG

GCTGAACGGCAAGGAGTATAAGTGCAAGGTGTCTAATAAGGGCCTGCCCGCC
CCTATCGAGAAGACCATCTCCAAGACAAAGGGCCAGCCTAGAGAGCCACAGG
TGTACACCCTGCCCCCTTCTCGCGAGGAGATGACCAAGAACCAGGTGTCCCT
GACATGTCTGGTGAAGGGCTTCTATCCCTCCGACATCGCTGTGGAGTGGGAGA
GCAATGGCCAGCCTGAGAACAATTACAAGACCACACCACCCATGCTGGACTC
CGATGGCAGCTTCTTTCTGTATAGCAAGCTGACCGTGGATAAGTCTAGGTGGC
AGCAGGGCAACGTGTTCTCCTGTTCCGTGATGCACGAAGCCCTGCACAACCA
TTATACTCAGAAGAGCCTGTCCCTGTCCCCTGGCAAA

SEQ ID NO: 17 Antibody A light chain full-length gene sequence (without signal sequence)

TCTAGTGAACTGACCCAGGATCCCGCCGTCTCTGTGGCCCTGGGCCAGACAG
TGAGAATCACCTGCCAGGGCGACTCTCTGCGCTCCTACTATGCCTCTTGGTAC
CAGCAGAAGCCAGGCCAGGCTCCCGTGCTGGTCATCTACGGCAAGAACAATA
GGCCTTCCGGCATCCCAGATCGGTTTAGCGGCTCCAGCTCTGGCAACACAGCC
AGCCTGACAATTACAGGAGCTCAGGCTGAGGATGAGGCTGATTACTATTGCCA
GTCTTACTATTCCAGCTCTCACTACGTGGTGTTCGGGGGAGGGACAAAGCTGA
CCGTGCTGGGACAGCCAAAGGCTGCTCCTTCTGTGACCCTGTTTCCCCCTTCC
AGCGAGGAGCTGCAGGCCAATAAGGCCACCCTGGTGTGCCTGATCAGCGACT
TCTATCCAGGAGCTGTGACAGTGGCTTGGAAGGCTGATTCTTCCCCAGTGAAG
GCTGGCGTGGAGACCACAACCCCCAGCAAGCAGTCTAACAATAAGTACGCCG
CCTCCTCTTATCTGTCTCTGACACCAGAGCAGTGGAAGTCCCATAGGTCCTAC
AGCTGCCAGGTGACCCACGAAGGTAGCACAGTCGAAAAAACCGTCGCCCCC
ACAGAGTGTTCT

[Example 301-2] Preparation of anti-folate receptor α antibody

The anti-folate receptor α antibody was prepared by the method described in US Patent No. 4805848. Throughout the present specification, the prepared anti-folate receptor α antibody may be referred to as "anti-FRA_Farletuzumab-14AAS antibody" or "003". Sequence No. identification and encoded regions for the anti-folate receptor α are as follows.

[Table 7]

| Name | Sequence |
|---|---|
| Heavy chain CDR1 (amino acid sequence) | GFTFSGYG (SEQ ID NO: 28) |
| Heavy chain CDR2 (amino acid sequence) | ISSGGSYT (SEQ ID NO: 29) |
| Heavy chain CDR3 (amino acid sequence) | ARHGDDPAWFAY (SEQ ID NO: 30) |

(continued)

| Name | Sequence |
|---|---|
| Light chain CDR1 (amino acid sequence) | SSISSNN (SEQ ID NO: 31) |
| Light chain CDR2 (amino acid sequence) | GTS (SEQ ID NO: 31-2) |
| Light chain CDR3 (amino acid sequence) | QQWSSYPYMYT (SEQ ID NO: 32) |
| Heavy chain variable region (amino acid sequence) | EVQLVESGGGVVQPGRSLRLSCSASGFTFSGYGLSWVRQAPGKGLEWVAMISSGGSYTYYADSVKGRFAISRDNAKNTLFLQMDSLRPEDTGVYFCARHGDDPAWFAYWGQGTPVTVSS (SEQ ID NO: 33) |
| Light chain variable region (amino acid sequence) | DIQLTQSPSSLSASVGDRVTITCSVSSSISSNNLHWYQQKPGKAPKPWIYGTSNLASGVPSRFSGSGSGTDYTFTISSLQPEDIATYYCQQWSSYPYMYTFGQGTKVEIK (SEQ ID NO: 34) |
| Heavy chain full-length amino acid sequence (without signal sequence) | EVQLVESGGGVVQPGRSLRLSCSASGFTFSGYGLSWVRQAPGKGLEWVAMISSGGSYTYYADSVKGRFAISRDNAKNTLFLQMDSLRPEDTGVYFCARHGDDPAWFAYWGQGTPVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGSSVFLFPPKPKDTLMISRTPEVTCVVVDVSQEDPEVQFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK (SEQ ID NO: 35) |
| Light chain full-length amino acid sequence (without signal sequence) | DIQLTQSPSSLSASVGDRVTITCSVSSSISSNNLHWYQQKPGKAPKPWIYGTSNLASGVPSRFSGSGSGTDYTFTISSLQPEDIATYYCQQWSSYPYMYTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO: 36) |
| Signal sequence | MGWSCIILFLVATATGVHS (SEQ ID NO: 37) |

[Example 301-3] Creation of anti-mesothelin antibody

The anti-mesothelin antibody was prepared by the method described in International Patent Publication No. 2021/0900062. Throughout the present specification, the prepared anti-mesothelin antibody may be referred to as "anti-Mesothelin_345A12" or "345A12". Sequence No. identification and encoded regions for the antibody are as follows.

[Table 8]

| Name | Sequence |
|---|---|
| Heavy chain CDR1 (amino acid sequence) | GIDLSSYA (SEQ ID NO: 38) |
| Heavy chain CDR2 (amino acid sequence) | IDISGNR (SEQ ID NO: 39) |

(continued)

| Name | Sequence |
|---|---|
| Heavy chain CDR3 (amino acid sequence) | ARVDSRAWGPFNL (SEQ ID NO: 40) |
| Light chain CDR1 (amino acid sequence) | QSIFSY (SEQ ID NO: 41) |
| Light chain CDR2 (amino acid sequence) | DAS (SEQ ID NO: 41-1) |
| Light chain CDR3 (amino acid sequence) | QQGYTRSDVDNA (SEQ ID NO: 42) |
| Heavy chain variable region (amino acid sequence) | QVQLVESGGGVVQPGRSLRLSCAASGIDLSSYAMSWVRQAP GKGLEWIGVIDISGNRFYADWVKGRFTISRDNSKNTLYLQMS SLRAEDTAVYYCARVDSRAWGPFNLWGQGTLVTVSS (SEQ ID NO: 43) |
| Light chain variable region (amino acid sequence) | GATTACCAGATGACCCAGTCCCCCTCCAGCCTGTCCGCTTC TGTGGGCGACAGAGTGACCATCACCTGTCAGGCCTCCCAG TCCATCTTCTCCTACCTGGCCTGGTATCAGCAGAAGCCCGG CAAGGCCCCCAAGCTGCTGATCTACGACGCCTCTGATCTGG CCTCCGGCGTGCCCTCTAGATTCTCCGGCTCTGGCTCTGGC ACCGACTTTACCCTGACCATCAGCTCCCTCCAGCCCGAGGA TTTCGCCACCTACTACTGCCAGCAGGGCTACACCAGATCCG ACGTGGACAACGCCTTTGGCGGAGGCACCAAGGTGGAAAT CAAA (SEQ ID NO: 44) |
| Heavy chain full-length amino acid sequence (without signal sequence) | QVQLVESGGGVVQPGRSLRLSCAASGIDLSSYAMSWVRQAP GKGLEWIGVIDISGNRFYADWVKGRFTISRDNSKNTLYLQMS SLRAEDTAVYYCARVDSRAWGPFNLWGQGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNTK VDKKVEPKSCDKTHTCPPCPAPEAAGGSSVFLFPPKPKDTLMI SRTPEVTCVVVDVSQEDPEVQFNWYVDGVEVHNAKTKPREE QYNSTYRVVSVLTVLHQDWLNGKEYKCKVSNKALPAPIEKTI SKAKGQPREPQVYTLPPSRDELTKNQVSLTCLVKGFYPSDIAV EWESNGQPENNYKTTPPVLDSDGSFFLYSKLTVDKSRWQQGN VFSCSVMHEALHNHYTQKSLSLSPGK (SEQ ID NO: 45) |
| Light chain full-length amino acid sequence (without signal sequence) | DYQMTQSPSSLSASVGDRVTITCQASQSIFSYLAWYQQKPGK APKLLIYDASDLASGVPSRFSGSGSGTDFTLTISSLQPEDFATYY CQQGYTRSDVDNAFGGGTKVEIKRTVAAPSVFIFPPSDEQLKS GTASVVCLLNNFYPREAKVQWKVDNALQSGNSQESVTEQDS KDSTYSLSSTLTLSKADYEKHKVYACEVTHQGLSSPVTKSFNR GEC (SEQ ID NO: 46) |
| Signal sequence | MGWSCIILFLVATATGVHS (SEQ ID NO: 47) |

[Preparation of antibody-drug conjugate]

The abbreviations used have the following meanings.

ADC: Antibody-drug conjugate
DMA: Dimethyl acetamide
D-PBS(-): Dulbecco's Phosphate Buffered Saline (-)
EDTA: Ethylenediaminetetraacetic acid
TCEP: Tris(2-carboxyethyl)phosphine

The concentration of the antibody portion of the prepared antibody-drug conjugate was measured using a Pierce™ BCA Protein Assay Kit (Thermo Fisher Scientific Inc.), according to the manufacturer's instructions, and the absorbance of the antibody-drug conjugate at 562 nm was measured using a macro plate reader (SUNRISE Rainbow RC-R, Tecan). The antibody used in the conjugate of each antibody-drug conjugate was a standard sample, and the concentration of the antibody portion of the antibody-drug conjugate was calculated from a standard curve obtained using the standard sample.

[Example 302] Preparation of antibody-drug conjugate (1)

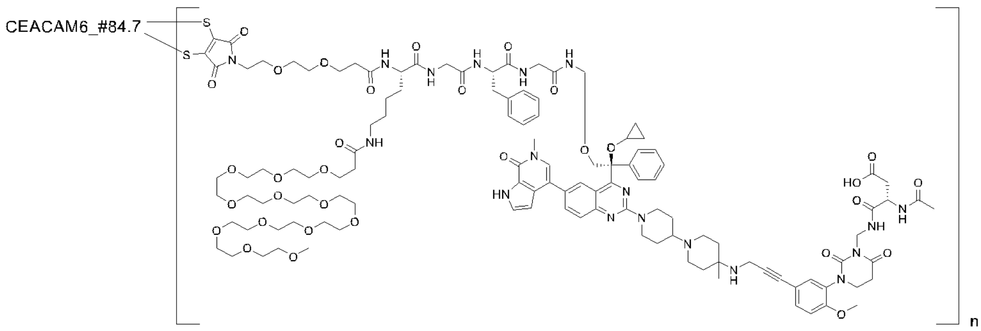

To a solution of the anti-CEACAM6_#84.7 antibody prepared in Example 301 (14.0 mg) in 25 mM histidine (5.0 mg/mL, pH 6.5) there was added a solution of TCEP hydrochloride in 2 mM EDTA/D-PBS(-) (600 μM, 955 μL; 6.0 eq. per antibody molecule), and a Microtube Rotator (MTR-103 by As One Corp.) was used for stirring overnight at room temperature. The reaction mixture was diluted with a 1 mM EDTA-50% propylene glycol/D-PBS(-) solution (3.76 mL), and then a solution of the (S)-3-acetamide-4-(((3-(5-(3-((1'-(4-((44S,50S,59S)-50-benzyl-59-cyclopropoxy-44-(3-(2-(2-(2,5-dioxo-3,4-bis(phenylthio)-2,5-dihydro-1H-pyrrol-1-yl)ethoxy)ethoxy)propanamido)-38,45,48,51,54-pentaoxo-59-phenyl-2,5,8,11,14,17,20,23,26,29,32,35,57-tridecaoxa-39,46,49,52,55-pentaazanonapentacontan-59-yl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid of Example 201 in DMA(12 mM, 47.8 μL; 6.0 eq. per antibody molecule) was added and a Microtube Rotator was used for stirring for 90 minutes at room temperature. The reaction mixture was evenly dispensed into Amicon Ultra-15 containers (4 containers, 30,000 MWCO, Millipore Corporation), and after dilution with a 25 mM histidine/250 mM sucrose solution (pH 5.5), the reaction mixture was concentrated to about 1/10 volume by centrifugation with a centrifuge (5000 G, 13 min). After repeating the same procedure two more times, the concentrated antibody-drug conjugate solution was sterilized using a syringe filter (MILLEX-GV, pore size: 0.22 μm, Millipore Corporation) to obtain the title antibody-drug conjugate.
Antibody concentration: 1.34 mg/mL, average number of bonded drug molecules per antibody molecule: 3.9

[Example 303] Preparation of antibody-drug conjugate (2)

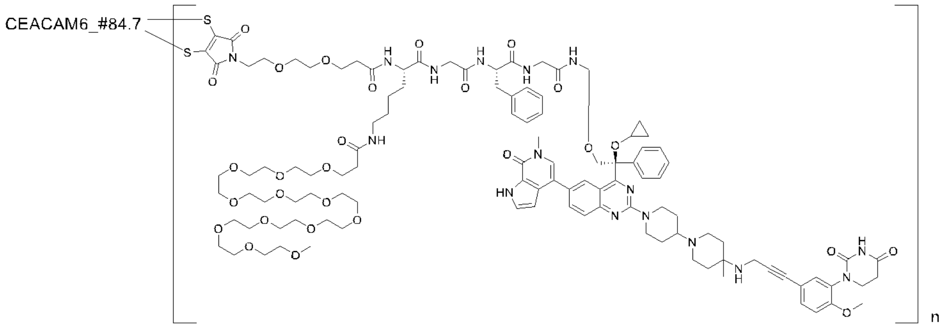

To a solution of the anti-CEACAM6_#84.7 antibody prepared in Example 301 (3.0 mg) in 25 mM histidine (5.0 mg/mL, pH 6.5) there was added a solution of TCEP hydrochloride in 2 mM EDTA/D-PBS(-) (600 μM, 205 μL; 6.0 eq. per antibody molecule), and a Microtube Rotator (MTR-103 by As One Corp.) was used for stirring overnight at room temperature. The reaction mixture was diluted with a 1 mM EDTA-50% propylene glycol/D-PBS(-) solution (805 μL), and then a solution of the N-((1S,10S, 16S)-10-benzyl-1-cyclopropoxy-16-(3-(2-(2-(2,5-dioxo-3,4-bis(phenylthio)-2,5-dihydro-1H-pyrrol-1-yl)ethoxy)ethoxy)propanamido)-1-(2-(4-((3-(3-(2,4-dioxotetrahydropyrimidine-1(2H)-yl)-4-methoxyphenyl)prop-2-yn-1-yl)amino)-4-methyl-[1,4'-bipiperidin]-1'-yl)-6-(6-methyl-7-oxo-6,7-dihydro-1H-pyrrolo[2,3-c]pyridin-4-yl)quinazolin-4-yl)-6,9,12,15-tetraoxo-1-phenyl-3-oxa-5,8,11,14-tetraazaeicosan-20-yl)-2,5,8,11,14,17,20,23,26,29,32,35-dodecaoxaoctatriacontan-38-amide of Example 202 in DMA(12 mM, 10.2 μL; 6.0 eq. per antibody molecule) was added and a Microtube Rotator was used for stirring for 90 minutes at room temperature. The reaction mixture was transferred into an Amicon Ultra-15 container (30,000 MWCO, Millipore Corporation), and after dilution with a 25 mM histidine/250 mM sucrose solution (pH 5.5), the reaction mixture was concentrated to about 1/10 volume by centrifugation with a centrifuge (5000 G, 10 min). After repeating the same procedure two more times, the concentrated antibody-drug conjugate solution was sterilized using a syringe filter (MILLEX-LG, pore size: 0.2 μm, Millipore Corporation) to obtain the title antibody-drug conjugate.
Antibody concentration: 1.25 mg/mL, average number of bonded drug molecules per antibody molecule: 3.9

[Example 304] Preparation of antibody-drug conjugate (3)

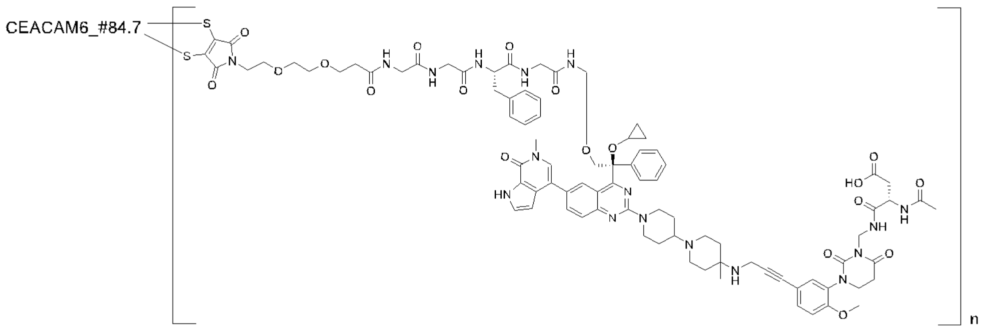

To a solution of the anti-CEACAM6_#84.7 antibody prepared in Example 301 (3.5 mg) in 25 mM histidine (5.0 mg/mL, pH 6.5) there was added a solution of TCEP hydrochloride in 2 mM EDTA/D-PBS(-) (600 μM, 239 μL; 6.0 eq. per antibody molecule), and a Microtube Rotator (MTR-103 by As One Corp.) was used for stirring overnight at room temperature. The reaction mixture was diluted with a 1 mM EDTA-50% propylene glycol/D-PBS(-) solution (939 μL), and then a solution of the (S)-3-acetamide-4-(((3-(5-(3-((1'-(4-((1S,10S)-10-benzyl-1-cyclopropoxy-26-(2,5-dioxo-3,4-bis(phenylthio)-2,5-dihydro-1H-pyrrol-1-yl)-6,9,12,15,18-pentaoxo-1-phenyl-3,21,24-trioxa-5,8,11,14,17-pentaazahexacosyl)-6-(6-methyl-7-oxo-6,7-dihydro-1H-pyrrolo[2,3-c]pyridin-4-yl)quinazoline-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid of Example 203 in DMA (12 mM, 23.9 μL; 12.0 eq. per antibody molecule) was added and a Microtube Rotator was used for stirring for 90 minutes at room temperature. The reaction mixture was transferred into an Amicon Ultra-15 container (30,000 MWCO, Millipore Corporation), and after dilution with a 25 mM histidine/250 mM sucrose solution (pH 5.5), the reaction mixture was concentrated to about 1/10 volume by centrifugation with a centrifuge (5000 G, 10 min). After repeating the same procedure two more times, the concentrated antibody-drug conjugate solution was sterilized using a syringe filter (MILLEX-LG, pore size: 0.2 μm, Millipore Corporation) to obtain the title antibody-drug conjugate.

Antibody concentration: 1.18 mg/mL, average number of bonded drug molecules per antibody molecule: 3.9

[Example 305] Preparation of antibody-drug conjugate (4)

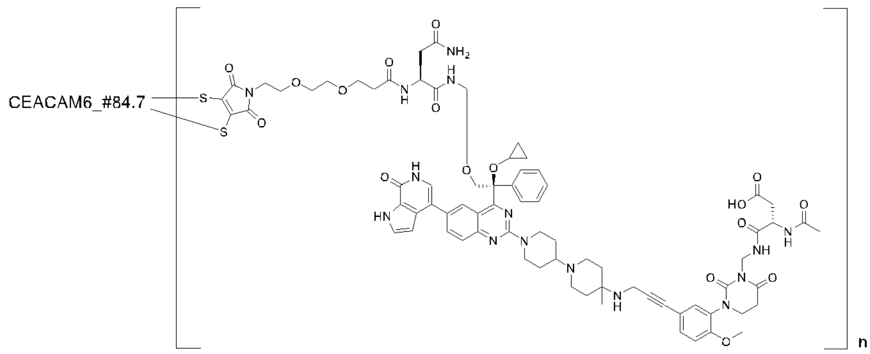

To a solution of the anti-CEACAM6_#84.7 antibody prepared in Example 301 (20.0 mg) in 25 mM histidine (5.0 mg/mL, pH 6.5) there was added a solution of TCEP hydrochloride in 2 mM EDTA/D-PBS(-) (600 μM, 1.36 mL; 6.0 eq. per antibody molecule), and a Microtube Rotator (MTR-103 by As One Corp.) was used for stirring overnight at room temperature. The reaction mixture was diluted with a 1 mM EDTA-50% propylene glycol/D-PBS(-) solution (5.36 mL), and then a solution of the (S)-3-acetamide-4-(((3-(5-(3-((1'-(4-((1S,7S)-7-(2-amino-2-oxoethyl)-1-cyclopropoxy-17-(2,5-dioxo-3,4-bis(phenylthio)-2,5-dihydro-1H-pyrrol-1-yl)-6,9-dioxo-1-phenyl-3,12,15-trioxa-5,8-diazaheptadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid of Example 204 in DMA (12 mM, 136 μL; 12.0 eq. per antibody molecule) was added and a Microtube Rotator was used for stirring for 90 minutes at room temperature. The reaction mixture was evenly dispensed into Amicon Ultra-15 containers (6 containers, 30,000 MWCO, Millipore Corporation), and after dilution with a 25 mM histidine/250 mM sucrose solution (pH 5.5), the reaction mixture was concentrated to about 1/10 volume by centrifugation with a centrifuge (5000 G, 10 min). After repeating the same procedure two more times, the concentrated antibody-drug conjugate solution was sterilized using a syringe filter (MILLEX-GV, pore size: 0.22 μm, Millipore Corporation) to obtain the title antibody-drug conjugate.
Antibody concentration: 1.40 mg/mL, average number of bonded drug molecules per antibody molecule: 3.8

[Example 306] Preparation of antibody-drug conjugate (69)

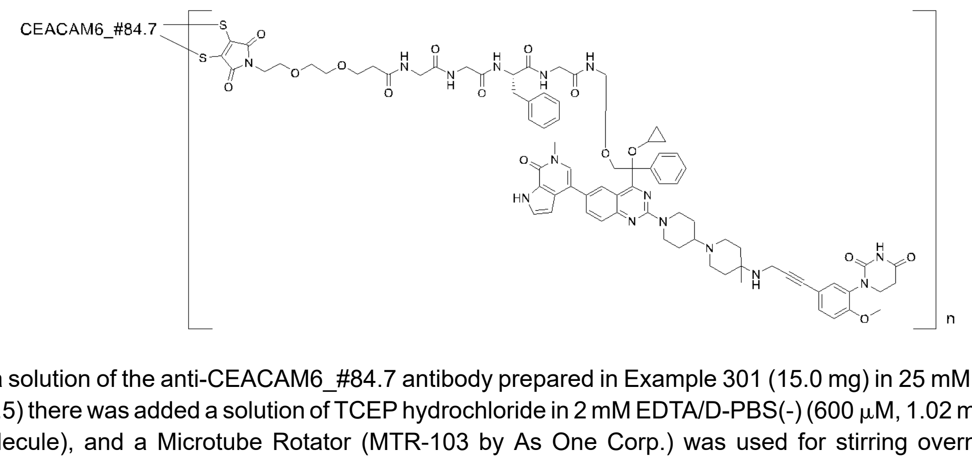

To a solution of the anti-CEACAM6_#84.7 antibody prepared in Example 301 (15.0 mg) in 25 mM histidine (5.0 mg/mL, pH 6.5) there was added a solution of TCEP hydrochloride in 2 mM EDTA/D-PBS(-) (600 μM, 1.02 mL; 6.0 eq. per antibody molecule), and a Microtube Rotator (MTR-103 by As One Corp.) was used for stirring overnight at room temperature. The reaction mixture was diluted with a 1 mM EDTA-50% propylene glycol/D-PBS(-) solution (4.02 mL), and then a solution of the (2S)-N-({[(2-cyclopropoxy-2-{2-[4-({3-[3-(2,4-dioxo-1,3-diazinane-1-yl)-4-methoxyphenyl]prop-2-yn-1-yl}amino)-4-methyl-[1,4'-bipiperidin]-1'-yl] -6-{6-methyl-7-oxo-1H,6H,7H-pyrrolo[2,3-c]pyridin-4-yl}quinazolin-4-yl}-2-phenylethoxy)methyl]carbamoyl}methyl)-2-(2-{2-[3-(2-{2-[2,5-dioxo-3,4-bis(phenylsulfanyl)-2,5-dihydro-1H-pyrrol-1-yl]ethoxy}ethoxy)propanamido]acetamide}acetamide)-3-phenylpropaneamide of Example 269 in DMA (12 mM,

102 μL; 12.0 eq. per antibody molecule) was added and a Microtube Rotator was used for stirring for 90 minutes at room temperature. The reaction mixture was evenly dispensed into Amicon Ultra-15 containers (5 containers, 30,000 MWCO, Millipore Corporation), and after dilution with a 25 mM histidine/250 mM sucrose solution (pH 5.5), the reaction mixture was concentrated to about 1/10 volume by centrifugation with a centrifuge (5000 G, 10 min). After repeating the same procedure two more times, the concentrated antibody-drug conjugate solution was sterilized using a syringe filter (MILLEX-GV, pore size: 0.22 μm, Millipore Corporation) to obtain the title antibody-drug conjugate.
Antibody concentration: 1.29 mg/mL, average number of bonded drug molecules per antibody molecule: 3.6

[Example 307] Preparation of antibody-drug conjugate (72-1)

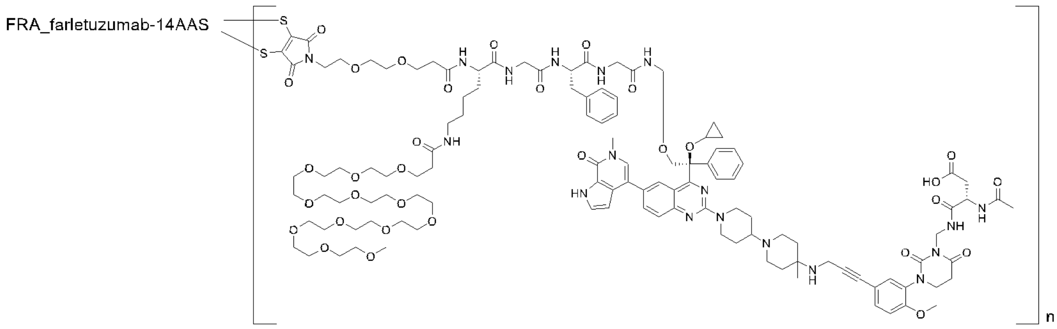

To a solution of anti-FRA_Farletuzumab-14AAS antibody (4.0 mg) in D-PBS (7.6 mg/mL) there was added a solution of TCEP hydrochloride in 2 mM EDTA/D-PBS(-) (600 μM, 270 μL; 6.0 eq. per antibody molecule), and a Microtube Rotator (MTR-103 by As One Corp.) was used for stirring overnight at room temperature. The reaction mixture was diluted with a 1 mM EDTA-50% propylene glycol/D-PBS(-) solution (796 μL), and then a solution of the (S)-3-acetamide-4-(((3-(5-(3-((1'-(4-((44S,50S,59S)-50-benzyl-59-cyclopropoxy-44-(3-(2-(2-(2,5-dioxo-3,4-bis(phenylthio)-2,5-dihydro-1H-pyrrol-1-yl)ethoxy)ethoxy)propanamido)-38,45,48,51,54-pentaoxo-59-phenyl-2,5,8,11,14,17,20,23,26,29,32,35,57-tridecaoxa-39,46,49,52,55-pentaazanonapentacontan-59-yl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid of Example 201 in DMA (12 mM, 13.5 μL; 6.0 eq. per antibody molecule) was added and a Microtube Rotator was used for stirring for 90 minutes at room temperature. The reaction mixture was transferred into an Amicon Ultra-15 container (30,000 MWCO, Millipore Corporation), and after dilution with a 25 mM histidine/250 mM sucrose solution (pH 5.5), the reaction mixture was concentrated to about 1/10 volume by centrifugation with a centrifuge (5000 G, 12 min). After repeating the same procedure two more times, the concentrated antibody-drug conjugate solution was sterilized using a syringe filter (MILLEX-LG, pore size: 0.2 μm, Millipore Corporation) to obtain the title antibody-drug conjugate.
Antibody concentration: 1.53 mg/mL, average number of bonded drug molecules per antibody molecule: 4.0

[Example 308] Preparation of antibody-drug conjugate (73)

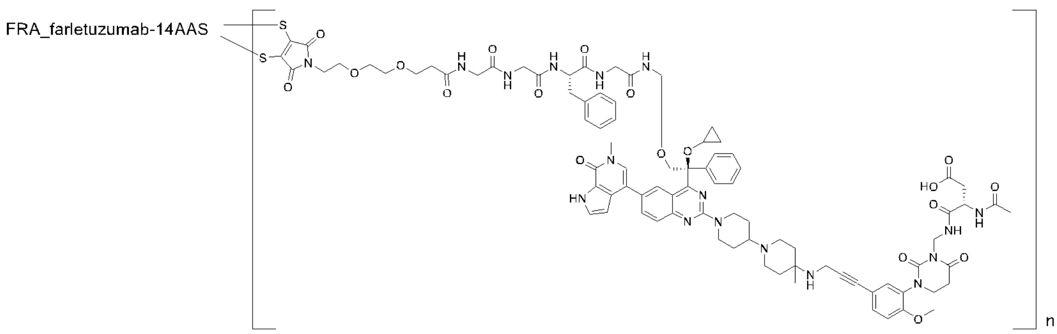

To a solution of anti-FRA_Farletuzumab-14AAS antibody (1.0 mg) in D-PBS (7.6 mg/mL) there was added a solution of TCEP hydrochloride in 2 mM EDTA/D-PBS(-) (600 μM, 67.5 μL; 6.0 eq. per antibody molecule), and a

Microtube Rotator (MTR-103 by As One Corp.) was used for stirring overnight at room temperature. The reaction mixture was diluted with a 1 mM EDTA-50% propylene glycol/D-PBS(-) solution (199 μL), and then a solution of the (S)-3-acetamide-4-(((3-(5-(3-((1'-(4-((1S,10S)-10-benzyl-1-cyclopropoxy-26-(2,5-dioxo-3,4-bis(phenylthio)-2,5-dihydro-1H-pyrrol-1-yl)-6,9,12,15,18-pentaoxo-1-phenyl-3,21,24-trioxa-5,8,11,14,17-pentaazahexacosyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid of Example 203 in DMA (12 mM, 6.8 μL; 12.0 eq. per antibody molecule) was added and a Microtube Rotator was used for stirring for 90 minutes at room temperature. The reaction mixture was transferred into an Amicon Ultra-4 container (30,000 MWCO, Millipore Corporation), and after dilution with a 25 mM histidine/250 mM sucrose solution (pH 5.5), the reaction mixture was concentrated to about 1/10 volume by centrifugation with a centrifuge (5000 G, 12 min). After repeating the same procedure two more times, the concentrated antibody-drug conjugate solution was sterilized using a syringe filter (MILLEX-LG, pore size: 0.2 μm, Millipore Corporation) to obtain the title antibody-drug conjugate.

Antibody concentration: 1.07 mg/mL, average number of bonded drug molecules per antibody molecule: 3.9

[Example 309] Preparation of antibody-drug conjugate (74)

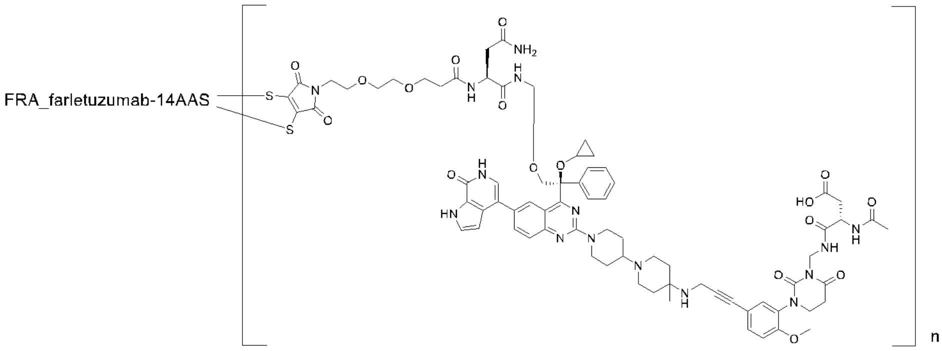


To a solution of anti-FRA_Farletuzumab-14AAS antibody (1.0 mg) in D-PBS (7.6 mg/mL) there was added a solution of TCEP hydrochloride in 2 mM EDTA/D-PBS(-) (600 μM, 67.5 μL; 6.0 eq. per antibody molecule), and a Microtube Rotator (MTR-103 by As One Corp.) was used for stirring overnight at room temperature. The reaction mixture was diluted with a 1 mM EDTA-50% propylene glycol/D-PBS(-) solution (199 μL), and then a solution of the (S)-3-acetamide-4-(((3-(5-(3-((1'-(4-((1S,7S)-7-(2-amino-2-oxoethyl)-1-cyclopropoxy-17-(2,5-dioxo-3,4-bis(phenylthio)-2,5-dihydro-1H-pyrrol-1-yl)-6,9-dioxo-1-phenyl-3,12,15-trioxa-5,8-diazaheptadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid of Example 204 in DMA (12 mM, 6.8 μL; 12.0 eq. per antibody molecule) was added and a Microtube Rotator was used for stirring for 90 minutes at room temperature. The reaction mixture was transferred into an Amicon Ultra-4 container (30,000 MWCO, Millipore Corporation), and after dilution with a 25 mM histidine/250 mM sucrose solution (pH 5.5), the reaction mixture was concentrated to about 1/10 volume by centrifugation with a centrifuge (5000 G, 12 min). After repeating the same procedure two more times, the concentrated antibody-drug conjugate solution was sterilized using a syringe filter (MILLEX-LG, pore size: 0.2 μm, Millipore Corporation) to obtain the title antibody-drug conjugate.

Antibody concentration: 1.03 mg/mL, average number of bonded drug molecules per antibody molecule: 4.0

[Example 310] Preparation of antibody-drug conjugate (75-1)

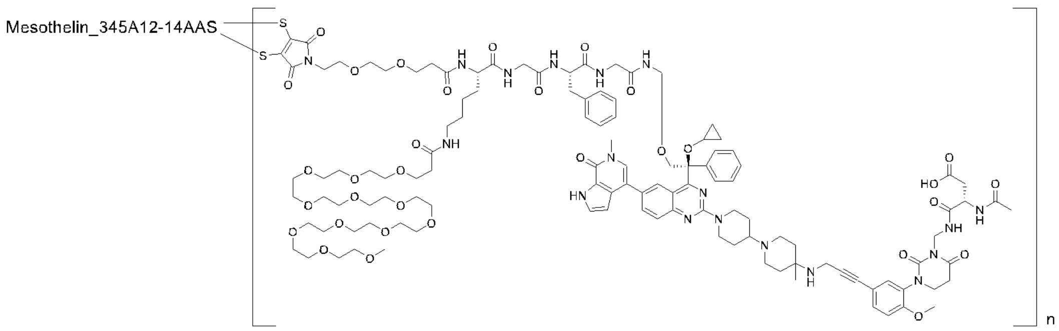

To a solution of anti-Mesothelin_345A12-14AAS antibody (4.0 mg) in D-PBS (9.8 mg/mL) there was added a solution of TCEP hydrochloride in 2 mM EDTA/D-PBS(-) (600 μM, 270 μL; 6.0 eq. per antibody molecule), and a Microtube Rotator (MTR-103 by As One Corp.) was used for stirring overnight at room temperature. The reaction mixture was diluted with a 1 mM EDTA-50% propylene glycol/D-PBS(-) solution (679 μL), and then a solution of the (S)-3-acetamide-4-(((3-(5-(3-((1'-(4-((44S,50S,59S)-50-benzyl-59-cyclopropoxy-44-(3-(2-(2-(2,5-dioxo-3,4-bis(phenylthio)-2,5-dihydro-1H-pyrrol-1-yl)ethoxy)ethoxy)propanamido)-38,45,48,51,54-pentaoxo-59-phenyl-2,5,8,11,14,17,20,23,26,29,32,35,57-tridecaoxa-39,46,49,52,55-pentaazanonapentacontan-59-yl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid of Example 201 in DMA (12 mM, 13.5 μL; 6.0 eq. per antibody molecule) was added and a Microtube Rotator was used for stirring for 90 minutes at room temperature. The reaction mixture was transferred into an Amicon Ultra-15 container (30,000 MWCO, Millipore Corporation), and after dilution with a 25 mM histidine/250 mM sucrose solution (pH 5.5), the reaction mixture was concentrated to about 1/10 volume by centrifugation with a centrifuge (5000 G, 12 min). After repeating the same procedure two more times, the concentrated antibody-drug conjugate solution was sterilized using a syringe filter (MILLEX-LG, pore size: 0.2 μm, Millipore Corporation) to obtain the title antibody-drug conjugate.
Antibody concentration: 1.22 mg/mL, average number of bonded drug molecules per antibody molecule: 4.0

[Example 311] Preparation of antibody-drug conjugate (76)

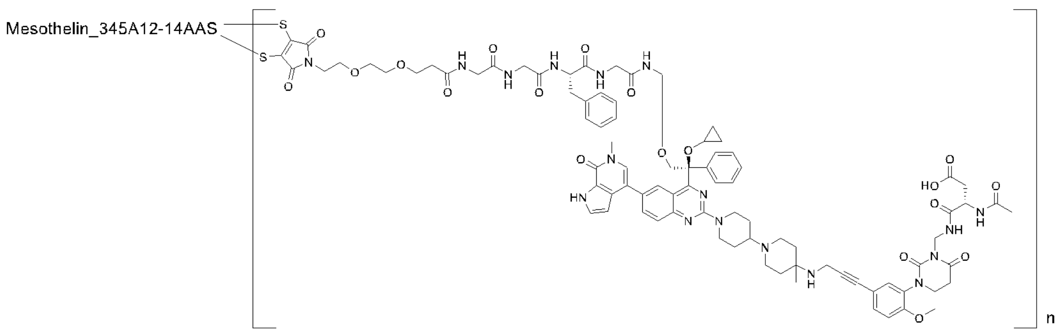

To a solution of anti-Mesothelin_345A12-14AAS antibody (1.0 mg) in D-PBS (9.8 mg/mL) there was added a solution of TCEP hydrochloride in 2 mM EDTA/D-PBS(-) (600 μM, 67.5 μL; 6.0 eq. per antibody molecule), and a Microtube Rotator (MTR-103 by As One Corp.) was used for stirring overnight at room temperature. The reaction mixture was diluted with a 1 mM EDTA-50% propylene glycol/D-PBS(-) solution (170 μL), and then a solution of the (S)-3-acetamide-4-(((3-(5-(3-((1'-(4-((1S,10S)-10-benzyl-1-cyclopropoxy-26-(2,5-dioxo-3,4-bis(phenylthio)-2,5-dihydro-1H-pyrrol-1-yl)-6,9,12,15,18-pentaoxo-1-phenyl-3,21,24-trioxa-5,8,11,14,17-pentaazahexacosyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid of Example 203 in DMA (12 mM, 6.8 μL; 12.0 eq. per antibody molecule) was added and a Microtube Rotator was used for stirring for 90 minutes at room temperature. The reaction mixture was transferred into an Amicon Ultra-4 container (30,000 MWCO, Millipore Corporation), and after dilution with a 25 mM histidine/250 mM sucrose solution (pH 5.5), the reaction mixture was concentrated to about 1/10 volume by centrifugation with a centrifuge (5000 G, 12 min). After repeating the same procedure two more times, the concentrated antibody-drug conjugate solution was sterilized using a syringe filter (MILLEX-LG, pore size: 0.2 μm, Millipore Corporation) to obtain the title antibody-drug conjugate.

Antibody concentration: 1.05 mg/mL, average number of bonded drug molecules per antibody molecule: 4.0

[Example 312] Preparation of antibody-drug conjugate (77)

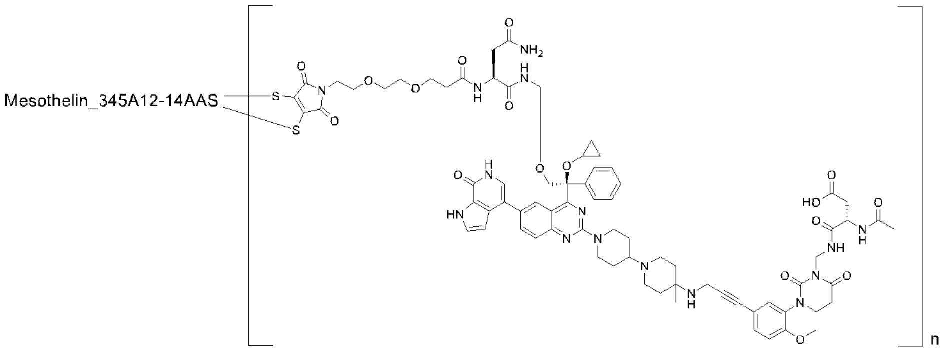

To a solution of anti-Mesothelin_ 345A12-14AAS antibody (1.0 mg) in D-PBS (9.8 mg/mL) there was added a solution of TCEP hydrochloride in 2 mM EDTA/D-PBS(-) (600 μM, 67.5 μL; 6.0 eq. per antibody molecule), and a Microtube Rotator (MTR-103 by As One Corp.) was used for stirring overnight at room temperature. The reaction mixture was diluted with a 1 mM EDTA-50% propylene glycol/D-PBS(-) solution (170 μL), and then a solution of the (S)-3-acetamide-4-(((3-(5-(3-((1'-(4-((1S,7S)-7-(2-amino-2-oxoethyl)-1-cyclopropoxy-17-(2,5-dioxo-3,4-bis(phenylthio)-2,5-dihydro-1H-pyrrol-1-yl)-6,9-dioxo-1-phenyl-3,12,15-trioxa-5,8-diazaheptadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidine-1(2H)-yl)methyl)amino)-4-oxobutanoic acid of Example 204 in DMA (12 mM, 6.8 μL; 12.0 eq. per antibody molecule) was added and a Microtube Rotator was used for stirring for 90 minutes at room temperature. The reaction mixture was transferred into an Amicon Ultra-4 container (30,000 MWCO, Millipore Corporation), and after dilution with a 25 mM histidine/250 mM sucrose solution (pH 5.5), the reaction mixture was concentrated to about 1/10 volume by centrifugation with a centrifuge (5000 G, 12 min). After repeating the same procedure two more times, the concentrated antibody-drug conjugate solution was sterilized using a syringe filter (MILLEX-LG, pore size: 0.2 μm, Millipore Corporation) to obtain the title antibody-drug conjugate.
Antibody concentration: 0.98 mg/mL, average number of bonded drug molecules per antibody molecule: 3.9

The same procedure as above was also carried out to prepare the ADCs other than the ADCs of Example 302 to Example 312. For ADC #55, however, the antibody-drug conjugate was obtained by the same procedure but with addition of a solution of TCEP hydrochloride in 2 mM EDTA/D-PBS(-) (250 μM) at 2.5 eq. per antibody molecule. For ADC #64 to 68, the antibody-drug conjugate was obtained by the same procedure but with addition of a solution of TCEP hydrochloride in 2 mM EDTA/D-PBS(-) (125 μM) at 1.25 eq. per antibody molecule.

The drug-antibody ratio (DAR) for the antibody drug conjugate (ADC) was calculated by either of the two following methods. The DARs for the ADCs using linkers with maleimide groups (ADC #55 and 64-68) in Table 10) were calculated based on i) hydrophobic interaction chromatography, and the DARs for the thio-bridged ADCs with bonding of two thiophenyl groups (the ADCs other than ADC #55 and 64-68 in Table 10) were calculated based on ii) LC/MS and c-SDS.

i) DAR calculation by hydrophobic interaction chromatography

Calculation was based on the area of absorbance (280 nm) of each separated DAR fraction.

[Hydrophobic interaction chromatography/HPLC conditions]

Column: TSKgel Butyl-NPR, 2.5 μm, 4.6 × 35 mm, Cat No.14947 (Tosoh)
Column temperature: 25°C
Detection wavelength: 280 nm
Mobile phase A: 1.5 mol/L ammonium sulfate/25 mmol/L sodium phosphate aqueous solution (pH 6.95 ±0.05)
Mobile phase B: 25 mmol/L sodium phosphate aqueous solution (pH 6.95 ±0.05)/2-propanol, 75/25, v/v
Flow rate: 0.8 mL/min
Measuring time: 45 min

Sampler temperature: 5°C
Injection rate: 25 μg
Gradient: B 0-100% (0-30 min), 100% (30-35 min), 100-0% (35-35.5 min), 0% (35.5-45 min)

[Drug-antibody ratio (DAR) calculation method]

**DAR** = $0 \times (A^{DAR0}280/A^{Total}280) + 1 \times (A^{DAR1}280/A^{Total}280) + 2 \times (A^{DAR2}280/A^{Total}280) + 3 \times (A^{DAR3}280/A^{Total}280) + 4 \times (A^{DAR4}280/A^{Total}280) + 5 \times (A^{DAR5}280/A^{Total}280) + 6 \times$ --> $(A^{DAR6}280/A^{Total}280) + 7 \times (A^{DAR7}280/A^{Total}280) + 8 \times (A^{DAR8}280/A^{Total}280)$

$A^{DAR0-8}280$: Area for each DAR (0 to 8) measured at 280 nm
$A^{Total}280$: Total area measured at 280 nm

ii) DAR calculation by LC/MS and c-SDS

Calculation was based on the DAR calculated for each isoform by LC/MS, and the isoform abundance ratio determined by c-SDS.

[LCMS conditions]

Detection mode: ESI(+), m/z = 500-4500
Column temperature: 80°C
Detection wavelength: 280 nm
Mobile phase A: 0.1 vol%-acetonitrile formate
Mobile phase B: 0.1 vol% formic acid-distilled water
Flow rate: 50 μL/min
Injection rate: 1-2 μL
Gradient (ex.): B 10% (0-0.5 min), 10% to 90% (0.5-4 min), 90% (4-5 min), 10% (5-8 min)

[c-SDS conditions]

Analyzer: PA800 plus Pharmaceutical Analysis System, Sciex
Separation conditions: Separate voltage 15 kV, duration 35 min
Detection wavelength: 220 nm

[Drug-antibody ratio (DAR) calculation method]

DAR = $R^{Inter} \times \{0 \times (I^{DAR0}/I^{Total}) + 1 \times (I^{DAR1}/I^{Total}) + 2 \times (I^{DAR2}/I^{Total}) + 3 \times (I^{DAR3}/I^{Total}) + 4 \times (I^{DAR4}/I^{Total})\} + 2 \times R^{Intra} \times \{(0 \times (N^{DAR0}/N^{Total}) + 1 \times (N^{DAR1}/N^{Total}) + 2 \times (N^{DAR2}/N^{Total})\}$

$$R^{Inter} = (HH + LHH + LHHL)/(H + LH + HH + LHH + LHHL)$$

: Abundance ratio of inter-chain isoforms of total ADCs

$$R^{Intra} = (H + LH)/(H + LH + HH + LHH + LHHL)$$

: Abundance ratio of intra-chain isoforms of total ADCs

H = H chain area determined by c-SDS
LH = LH chain area determined by c-SDS
HH = HH chain area determined by c-SDS
LHH = LHH chain area determined by c-SDS
LHHL = LHHL chain area determined by c-SDS
$I^{DAR0-4}$: Antibody-derived DAR 0 to 4 ionic strengths obtained by MS
$I^{Total}$: Antibody-derived total ionic strength obtained by MS
$N^{DAR0-2}$: Half antibody-derived DAR 0 to 2 ionic strengths obtained by MS
$N^{Total}$: Half antibody-derived total ionic strength obtained by MS

[Example 313: AsPC-1/Cas9: Growth inhibition assay]

Human pancreatic cancer cell line AsPC-1 (ATCC Number CRL1682) was infected with Edit-R Lentiviral hCMV-Blast-Cas9 Nuclease Particle (Dharmacon, VCAS10124) in the presence of 8 μg/ml of Hexadimethrine bromide (Sigma, H9268), and after 24 hours it was exposed to 10 μg/ml of Blastcidin S (Thermo, A11139) to remove the uninfected cells, thereby establishing an AsPC-1/Cas9 cell line. The cells were maintenance cultured in a 5% $CO_2$ incubator (37°C) using RPMI-1640 (FujiFilm-Wako Pure Chemical Industries) containing 10% fetal bovine serum (FBS: SIGMA 173012) and penicillin/streptomycin (FujiFilm-Wako Pure Chemical Industries) (hereunder referred to simply as "culture medium"). After adding 25 μL of AsPC-1/Cas9 cell suspension prepared to $3.2 \times 10^4$ cells/mL using culture medium into each well of a 384-well plate (Greiner 781091), the cells were cultured overnight in a 5% $CO_2$ incubator (37°C). On the following day, 25 μL of the test substance diluted with culture medium was added to each well, and culturing was continued for 5 days in a 5% $CO_2$ incubator (37°C). Next, 25 μL of Cell Titer Glo™ 2.0 (Promega) was added to each well, and the plate contents were mixed for 5 minutes using a plate mixer. The plate was stationed for 10 minutes at room temperature while shielded from light, and then the luminous intensity was measured with an ENVISION™ (Perkin-Elmer). The luminescence value of the wells with addition of test substance was determined as a relative value, with 100% as the luminescence value of the wells with cells but without addition of the test substance and 0% as the luminescence value of the wells without cells. A plot of test substance concentration against luminescence value % approximated a 4-parameter logistic curve, and the test substance concentration necessary for 50% inhibition of cell growth (Absolute $IC_{50}$) was calculated, and is shown in Table 10. In Table 10, the antibodies listed as "CEACAM6_#84.7" are the anti-CEACAM6 antibody of Example 301-1, the antibodies listed as "003" are the anti-folate receptor α antibody of Example 301-2, and the antibodies listed as "345A12" are the anti-mesothelin antibody of Example 301-3.

[Example 314: HEC-1-B Growth inhibition assay]

Cells of the human endometrial cancer cell line HEC-1-B (JCRB/HSRRB Number JCRB1193) were maintenance cultured in a 5% $CO_2$ incubator (37°C) using E-MEM (FujiFilm-Wako Pure Chemical Industries) containing 10% fetal bovine serum (FBS: SIGMA 173012) and penicillin/streptomycin (FujiFilm-Wako Pure Chemical Industries) (hereunder referred to simply as "culture medium"). After adding 45 μL of HEC-1-B cell suspension prepared to $1.8 \times 10^4$ cells/mL using culture medium into each well of a 384-well plate (Greiner 781091), the cells were cultured overnight in a 5% $CO_2$ incubator (37°C). On the following day, 5 μL of the test substance diluted with culture medium was added to each well, and culturing was continued for 5 days in a 5% $CO_2$ incubator (37°C). Next, 25 μL of Cell Titer Glo™ 2.0 (Promega) was added to each well, and the plate contents were mixed for 5 minutes using a plate mixer. The plate was stationed for 10 minutes at room temperature while shielded from light, and then the luminous intensity was measured with an ENVISION™ (Perkin-Elmer). The luminescence value of the wells with addition of test substance was determined as a relative value, with 100% as the luminescence value of the wells with cells but without addition of the test substance and 0% as the luminescence value of the wells without cells. A plot of test substance concentration against luminescence value % approximated a 4-parameter logistic curve, and the test substance concentration necessary for 50% inhibition of cell growth (Absolute $IC_{50}$) was calculated, and is shown in Table 10.

[Example 315: COV644 Growth inhibition assay]

Cells of the human ovarian mucinous cancer cell line COV644 (DS-pharma./KAC Number 07071908) were maintenance cultured in a 5% $CO_2$ incubator (37°C) using D-MEM (FujiFilm-Wako Pure Chemical Industries) containing 10% fetal bovine serum (FBS: SIGMA 173012) and penicillin/streptomycin (FujiFilm-Wako Pure Chemical Industries) (hereunder referred to as "culture medium"). After adding 45 μL of COV644 cell suspension prepared to $1.8 \times 10^4$ cells/mL using culture medium into each well of a 384-well plate (Greiner 781091), the cells were cultured overnight in a 5% $CO_2$

incubator (37°C). On the following day, 5 μL of the test substance diluted with culture medium was added to each well, and culturing was continued for 5 days in a 5% $CO_2$ incubator (37°C). Next, 25 μL of Cell Titer Glo™ 2.0 (Promega) was added to each well, and the plate contents were mixed for 5 minutes using a plate mixer. The plate was stationed for 10 minutes at room temperature while shielded from light, and then the luminous intensity was measured with an ENVISION™ (Perkin-Elmer). The luminescence value of the wells with addition of test substance was determined as a relative value, with 100% as the luminescence value of the wells with cells but without addition of the test substance and 0% as the luminescence value of the wells without cells. A plot of test substance concentration against luminescence value % approximated a 4-parameter logistic curve, and the test substance concentration necessary for 50% inhibition of cell growth (Absolute $IC_{50}$) was calculated, and is shown in Table 10.

[Example 316: Growth inhibition assay using different human cancer cell lines] Culture media for cells of the different human cancer cell lines shown in Table 9 were used for maintenance culture in a 5% $CO_2$ incubator (37°C). After adding 45 μL of cell suspension prepared to $1.8 \times 10^4$ cells/mL using culture medium into each well of a 384-well plate (Greiner 781091), the cells were cultured overnight in a 5% $CO_2$ incubator (37°C). On the following day, 5 μL of test substance diluted with RPMI-1640 medium (FujiFilm-Wako Pure Chemical Industries) containing 10% fetal bovine serum (FBS: SIGMA 173012) and penicillin/streptomycin (FujiFilm-Wako Pure Chemical Industries) was added to each well, and culturing was continued for 5 days in a 5% $CO_2$ incubator (37°C). Next, 25 μL of CellTiter Glo™ 2.0 (Promega) was added to each well, and the plate contents were mixed for 5 minutes using a plate mixer. The plate was stationed for 10 minutes at room temperature while shielded from light, and then the luminous intensity was measured with an ENVISION™ (Perkin-Elmer). The luminescence value of the wells with addition of test substance was determined as a relative value, with 100% as the luminescence value of the wells with cells but without addition of the test substance and 0% as the luminescence value of the wells without cells. A plot of test substance concentration against luminescence value % approximated a 4-parameter logistic curve, and the test substance concentration necessary for 50% inhibition of cell growth (Absolute $IC_{50}$) was calculated, and is shown in Table 10.

[Table 9]

| Cell line | Vendor/Cell No. | Culture medium |
|---|---|---|
| Human breast cancer cell line HCC1954 | ATCC CRL-2338 | RPMI-1640 Medium (FujiFilm-Wako Pure Chemical Ind.) containing 10% FBS (SIGMA173012), penicillin/streptomycin (FujiFilm-Wako Pure Chemical Ind.) |
| Human stomach cancer cell line NCI-N87 | ATCC CRL-5822 | |
| Human testicular cancer cell line NEC8 | JCRB JCRB0250 | |
| Human pancreatic cancer cell line HPAF-II | ATCC CRL-1997 | |
| Human osteosarcoma cell line HuO9 | JCRB/HSRRB JCRB0427 | |
| Human stomach cancer cell line SNU-216 | Creative-Bioarray CSC-C9651L | |

(continued)

| Cell line | Vendor/Cell No. | Culture medium |
|---|---|---|
| Human lung cancer cell line NCI-H1573 | ATCC CRL-5877 | RPMI-1640 (4,500 mg/L, glucose) medium (FujiFilm-Wako Pure Chemical Ind.) containing 10% FBS (SIGMA173012), penicillin/streptomycin (FujiFilm-Wako Pure Chemical Ind.) |
| Human pancreatic cancer cell line BxPC-3 | ATCC CRL-1687 | |
| Human prostate cancer cell LNCap.FGC | RIKEN BRORiken Cell Bank RCB2144 | |
| Human bladder cancer cell line HT-1376 | ATCC CRL-1472 | E-MEM (containing NEAA) medium (FujiFilm-Wako Pure Chemical Ind.) Containing 10% FBS (SIGMA173012), penicillin/streptomycin (FujiFilm-Wako Pure Chemical Ind.) |
| Human head and neck cancer cell line HSC-3 | RIKEN BRORiken Cell Bank RCB1975 | E-MEM medium (FujiFilm-Wako Pure Chemical Industries) Containing 10% FBS (SIGMA173012), penicillin/streptomycin (FujiFilm-Wako Pure Chemical Ind.) |
| Human endometrial cancer cell line HEC-6 | JCRB/HSRRB JCRB1118 | E-MEM medium (FujiFilm-Wako Pure Chemical Ind.) containing 15% FBS (SICN4A173012), penicillin/streptomycin (FujiFilm-Wako Pure Chemical Ind.) |
| Human lung cancer cell line Calu-1 | ATCC HTB-54 | McCoy's 5A culture medium (Thermo Fisher Scientific) containing 10% FBS (SIGMA173012), Penicillin/streptomycin (FujiFilm-Wako Pure Chemical Ind.) |
| Human pancreatic cancer cell line Capan-2 | ATCC HTB-80 | |
| Human leukemia cell line MV-4-11 | ATCC CRL-9591 | IMDM medium (FujiFilm-Wako Pure Chemical Ind.) Containing 10% FBS (SIGMA173012), penicillin/streptomycin (FujiFilm-Wako Pure Chemical Ind.) |
| Human lung cancer cell line NCI-H510A | ATCC HTB-184 | Ham's F12K medium (FujiFilm-Wako Pure Chemical Ind.) Containing 10% FBS (SIGMA173012), penicillin/streptomycin (FujiFilm-Wako Pure Chemical Ind.) |
| Human lung cancer cell line NCI-H841 | ATCC CRL-5845 | DMEM/F-12, GlutaMAX medium (ThermoFisher Scientific/Gibco) containing 5% FBS (SIGMA 173012), penicillin/streptomycin (FujiFilm-Wako Pure Chemical Ind.), 10 nM hydrocortisone (FujiFilm-Wako Pure Chemical Ind), 10 nM beta-estradiol (Sigma), Insulin-Transferrin-Selenium (ThermoFisher Scientific/Gibco), 1% Antibiotic-Antimycotic (ThemoFisher Scientific/Gibco) |
| Human ovary cancer cell line OV-90 | ATCC CRL-11732 | MCDB 105 medium (Sigma) and 199 medium (Sigma) at 1:1 mixture, containing 15% FBS (SIGMA173012), penicillin/streptomycin (FujiFilm-Wako Pure Chemical Ind.) |
| Human brain tumor cell line U-87MG | ATCC HTB-14 | D-MEM medium (FujiFilm-Wako Pure Chemical Ind.) Containing 10% FBS (SIGMA173012), penicillin/streptomycin (FujiFilm-Wako Pure Chemical Ind.) |

(continued)

| Cell line | Vendor/Cell No. | Culture medium |
|---|---|---|
| Human head and neck cancer cell line SCC-9 | ATCC CRL-1629 | DMEM-F12 and HEPES medium (ThermoFisher Scientific/Gibco) containing 10% FBS (SIGMA173012), penicillin/streptomycin (FujiFilm-Wako Pure Chemical Ind.) |

[Table 10]

| ADCNo. | Antibody | Linker-payload | ADC concentrati on (mg/mL) | DAR | Cell line | IC50 (nM) |
|---|---|---|---|---|---|---|
| 1 | CEACAM6_#84. 7 | Example 201 | 1.34 | 3.9 | AsPC-1 | 0.076 |
| 2 | CEACAM6_#84. 7 | Example 202 | 1.25 | 3.9 | AsPC-1 | <0.015 |
| 3 | CEACAM6_#84. 7 | Example 203 | 1.18 | 3.9 | AsPC-1 | 0.052 |
| 4 | CEACAM6_#84. 7 | Example 204 | 1.40 | 3.8 | AsPC-1 | 0.076 |
| 5 | CEACAM6_#84. 7 | Example 205 | 1.55 | 3.4 | AsPC-1 | 0.035 |
| 6 | CEACAM6_#84. 7 | Example 206 | 1.41 | 3.8 | AsPC-1 | 0.029 |
| 7 | CEACAM6_#84. 7 | Example 207 | 1.52 | 3.6 | AsPC-1 | 0.051 |
| 8 | CEACAM6_#84. 7 | Example 208 | 1.50 | 3.6 | AsPC-1 | 0.043 |
| 9 | CEACAM6_#84. 7 | Example 209 | 1.35 | 3.4 | AsPC-1 | 0.075 |
| 10 | CEACAM6_#84. 7 | Example 210 | 1.39 | 3.7 | AsPC-1 | 0.036 |
| 11 | CEACAM6_#84. 7 | Example 211 | 1.38 | 2.9 | AsPC-1 | 0.039 |
| 12 | CEACAM6_#84. 7 | Example 212 | 1.37 | 3.8 | AsPC-1 | 0.038 |
| 13 | CEACAM6_#84. 7 | Example 213 | 1.43 | 2.7 | AsPC-1 | 0.043 |
| 14 | CEACAM6_#84. 7 | Example 214 | 1.60 | 3.7 | AsPC-1 | 0.038 |
| 15 | CEACAM6_#84. 7 | Example 215 | 1.44 | 2.9 | AsPC-1 | 0.039 |
| 16 | CEACAM6_#84. 7 | Example 216 | 1.63 | 3.5 | AsPC-1 | 0.035 |
| 17 | CEACAM6_#84. 7 | Example 217 | 1.49 | 3.1 | AsPC-1 | 0.040 |
| 18 | CEACAM6_#84. 7 | Example 218 | 1.49 | 3.6 | AsPC-1 | 0.047 |
| 19 | CEACAM6_#84. 7 | Example 219 | 1.38 | 3.9 | AsPC-1 | 0.031 |
| 20 | CEACAM6_#84. 7 | Example 220 | 1.43 | 3.0 | AsPC-1 | 0.035 |
| 21 | CEACAM6_#84. 7 | Example 221 | 1.43 | 3.5 | AsPC-1 | 0.061 |
| 22 | CEACAM6_#84. 7 | Example 222 | 1.22 | 3.8 | AsPC-1 | 1.600 |
| 23 | CEACAM6_#84. 7 | Example 223 | 1.34 | 3.5 | AsPC-1 | 0.034 |
| 24 | CEACAM6_#84. 7 | Example 224 | 1.06 | 3.5 | AsPC-1 | 0.043 |
| 25 | CEACAM6_#84. 7 | Example 225 | 1.11 | 2.9 | AsPC-1 | 0.057 |
| 26 | CEACAM6_#84. 7 | Example 226 | 1.52 | 3.7 | AsPC-1 | 0.032 |
| 27 | CEACAM6_#84. 7 | Example 227 | 1.63 | 3.7 | AsPC-1 | <0.015 |
| 28 | CEACAM6_#84. 7 | Example 228 | 1.21 | 3.8 | AsPC-1 | 0.023 |
| 29 | CEACAM6_#84. 7 | Example 229 | 1.50 | 2.7 | AsPC-1 | 0.041 |
| 30 | CEACAM6_#84. 7 | Example 230 | 1.46 | 3.8 | AsPC-1 | <0.015 |
| 31 | CEACAM6_#84. 7 | Example 231 | 1.50 | 3.0 | AsPC-1 | 0.037 |
| 32 | CEACAM6_#84. 7 | Example 232 | 1.27 | 3.7 | AsPC-1 | 0.027 |
| 33 | CEACAM6_#84. 7 | Example 233 | 1.68 | 3.3 | AsPC-1 | <0.015 |

(continued)

| ADCNo. | Antibody | Linker-payload | ADC concentrati on (mg/mL) | DAR | Cell line | IC50 (nM) |
|---|---|---|---|---|---|---|
| 34 | CEACAM6_#84. 7 | Example 234 | 1.62 | 3.8 | AsPC-1 | 0.083 |
| 35 | CEACAM6_#84. 7 | Example 235 | 1.64 | 3.7 | AsPC-1 | 0.038 |
| 36 | CEACAM6_#84. 7 | Example 236 | 1.43 | 3.8 | AsPC-1 | 0.035 |
| 37 | CEACAM6_#84. 7 | Example 237 | 1.41 | 3.6 | AsPC-1 | 0.075 |
| 38 | CEACAM6_#84. 7 | Example 238 | 1.29 | 3.7 | AsPC-1 | 0.047 |
| 39 | CEACAM6_#84. 7 | Example 239 | 1.47 | 3.8 | AsPC-1 | 0.039 |
| 40 | CEACAM6_#84. 7 | Example 240 | 0.87 | 3.5 | AsPC-1 | 0.044 |
| 41 | CEACAM6_#84. 7 | Example 241 | 1.32 | 3.5 | AsPC-1 | 0.038 |
| 42 | CEACAM6_#84. 7 | Example 242 | 1.25 | 3.4 | AsPC-1 | 0.039 |
| 43 | CEACAM6_#84. 7 | Example 243 | 1.19 | 2.2 | AsPC-1 | 0.070 |
| 44 | CEACAM6_#84. 7 | Example 244 | 1.70 | 3.8 | AsPC-1 | 0.074 |
| 45 | CEACAM6_#84. 7 | Example 245 | 1.45 | 3.1 | AsPC-1 | 0.057 |
| 46 | CEACAM6_#84. 7 | Example 246 | 1.61 | 3.7 | AsPC-1 | 0.072 |
| 47 | CEACAM6_#84. 7 | Example 247 | 1.10 | 3.0 | AsPC-1 | 0.150 |
| 48 | CEACAM6_#84. 7 | Example 248 | 1.66 | 3.5 | AsPC-1 | 0.053 |
| 49 | CEACAM6_#84. 7 | Example 249 | 1.33 | 3.2 | AsPC-1 | 0.097 |
| 50 | CEACAM6_#84. 7 | Example 250 | 1.51 | 3.6 | AsPC-1 | 0.029 |
| 51 | CEACAM6_#84. 7 | Example 251 | 1.52 | 3.9 | AsPC-1 | 0.039 |
| 52 | CEACAM6_#84. 7 | Example 252 | 1.65 | 3.9 | AsPC-1 | 0.027 |
| 53 | CEACAM6_#84. 7 | Example 253 | 0.78 | 3.7 | AsPC-1 | 0.065 |
| 54 | CEACAM6_#84. 7 | Example 254 | 0.87 | 3.7 | AsPC-1 | 0.066 |
| 55 | CEACAM6_#84. 7 | Example 255 | 1.22 | 4.6 | AsPC-1 | 0.063 |
| 56 | CEACAM6_#84. 7 | Example 256 | 0.86 | 2.3 | AsPC-1 | 0.160 |
| 57 | CEACAM6_#84. 7 | Example 257 | 1.44 | 3.3 | AsPC-1 | 0.098 |
| 58 | CEACAM6_#84. 7 | Example 258 | 1.69 | 3.7 | AsPC-1 | 0.050 |
| 59 | CEACAM6_#84. 7 | Example 259 | 1.17 | 3.5 | AsPC-1 | 0.036 |
| 60 | CEACAM6_#84. 7 | Example 260 | 1.54 | 2.5 | AsPC-1 | 0.081 |
| 61 | CEACAM6_#84. 7 | Example 261 | 1.29 | 3.7 | AsPC-1 | 0.045 |
| 62 | CEACAM6_#84. 7 | Example 262 | 1.38 | 3.0 | AsPC-1 | 0.072 |
| 63 | CEACAM6_#84. 7 | Example 263 | 1.23 | 3.8 | AsPC-1 | 0.240 |
| 64 | CEACAM6_#84. 7 | Example 264 | 0.98 | 7.3 | AsPC-1 | 0.430 |
| 65 | CEACAM6_#84. 7 | Example 265 | 1.12 | 1.8 | AsPC-1 | N.T. |
| 66 | CEACAM6_#84. 7 | Example 266 | 1.41 | 2.1 | AsPC-1 | N.T. |
| 67 | CEACAM6_#84. 7 | Example 267 | 1.37 | 2.4 | AsPC-1 | 0.620 |
| 68 | CEACAM6_#84. 7 | Example 268 | 1.47 | 2.1 | AsPC-1 | N.T. |
| 69 | CEACAM6_#84. 7 | Example 269 | 1.29 | 3.6 | AsPC-1 | 0.037 |
| 70 | CEACAM6_#84. 7 | Example 270 | 1.15 | 3.6 | AsPC-1 | 0.550 |
| 71 | CEACAM6_#84. 7 | Example 271 | 0.97 | 3.9 | AsPC-1 | 0.350 |
| 72-1 | 003 | Example 201 | 1.53 | 4.0 | | |

(continued)

| ADCNo. | Antibody | Linker-payload | ADC concentrati on (mg/mL) | DAR | Cell line | IC50 (nM) |
|---|---|---|---|---|---|---|
| 72-2 | 003 | Example 201 | 1.46 | 3.9 | HEC 1B | 2.5 |
| 73 | 003 | Example 203 | 1.07 | 3.9 | HEC 1B | 4.2 |
| 74 | 003 | Example 204 | 1.03 | 4.0 | HEC 1B | 6.5 |
| 75-1 | 345A12 | Example 201 | 1.22 | 4.0 | | |
| 75-2 | 345A12 | Example 201 | 1.38 | 3.9 | COV644 | 0.11 |
| 75-3 | 345A12 | Example 201 | 2.36 | 4.0 | | |
| 76 | 345A12 | Example 203 | 1.05 | 4.0 | COV644 | 0.15 |
| 77 | 345A12 | Example 204 | 0.98 | 3.9 | COV644 | 0.14 |
| 78 | HER2 | Example 201 | 1.28 | 3.9 | HCC-1954<br>NCI-N87 | 0.15<br><0.015 |
| 79 | FLT3 | Example 201 | 1.50 | 3.5 | MV-4-11 | >100 |
| 80 | DLL3 | Example 201 | 1.60 | 4.0 | NCI-510A | 0.25 |
| 81 | Claudin6 | Example 201 | 1.53 | 4.0 | NCI-H841I<br>NEC8<br>HEC-6<br>OV-90 | <0.015<br><0.015<br><0.015<br>0.065 |
| 82 | EGFR | Example 201 | 1.01 | 4.0 | NCI-H1573 | 0.028 |
| 83 | Nectin4 | Example 201 | 1.46 | 3.9 | HT-1376 | 0.16 |
| 84 | Trop2 | Example 201 | 1.27 | 3.9 | HPAF-II<br>BxPC-3 | 0.019<br>0.027 |
| 87 | Claudin3/4 | Example 201 | 1.10 | 4.2 | SNU-216<br>Capan-2 | 0.032<br>0.034 |
| 88 | CA9 | Example 201 | 1.23 | 2.6 | U-87MG<br>SCC-9 | 51<br>>100 |

[Example 317: HPAF-II Growth inhibition assay]

Cells of the human pancreatic cancer cell line HPAF-II (ATCC Number CRL-1997) were maintenance cultured in a 5% $CO_2$ incubator (37°C) using RPMI 1640 (FujiFilm-Wako Pure Chemical Industries) containing 10% fetal bovine serum (FBS: SIGMA 173012) and penicillin/streptomycin (FujiFilm-Wako Pure Chemical Industries) (hereunder referred to simply as "culture medium"). After adding 25 μL of HPAF-II cell suspension prepared to $3.2 \times 10^4$ cells/mL using culture medium into each well of a 384-well plate (Greiner 781091), the cells were cultured overnight in a 5% $CO_2$ incubator (37°C). On the following day, 25 μL of the test substance diluted with culture medium was added to each well, and culturing was continued for 5 days in a 5% $CO_2$ incubator (37°C). Next, 25 μL of Cell Titer Glo™ 2.0 (Promega) was added to each well, and the plate contents were mixed for 5 minutes using a plate mixer. The plate was stationed for 10 minutes at room temperature while shielded from light, and then the luminous intensity was measured with an ENVISION™ (Perkin-Elmer). The luminescence value of the wells with addition of test substance was determined as a relative value, with 100% as the luminescence value of the wells with cells but without addition of the test substance and 0% as the luminescence value of the wells without cells. A plot of test substance concentration against luminescence value % approximated a 4-parameter logistic curve, and the test substance concentration necessary for 50% inhibition of cell growth (Absolute $IC_{50}$) was calculated, and is shown in Table 11.

[Table 11]

| ADC | Absolute IC50 (nM) |
|---|---|
| Example 310 | 1.3 |

(continued)

| ADC | Absolute IC50 (nM) |
|---|---|
| Example 312 | 2.1 |
| Example 311 | 2.2 |
| Compound of Example 135 | 2.9 |

[Example 318: Preparation of antibody-drug conjugate (89)]

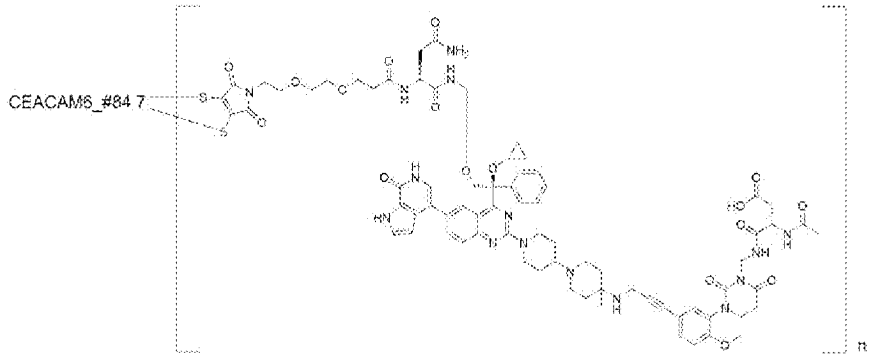

To a solution of the anti-CEACAM6_#84.7 antibody prepared in Example 301 (3.0 mg) in 25 mM histidine (5.0 mg/mL, pH 6.5) there was added a solution of TCEP hydrochloride in 2 mM EDTA/D-PBS(-) (600 μM, 0.205 mL; 6.0 eq. per antibody molecule), and a Microtube Rotator (MTR-103 by As One Corp.) was used for stirring overnight at room temperature. The reaction mixture was diluted with a 1 mM EDTA-50% propylene glycol/D-PBS(-) solution (0.805 mL), and then a solution of the (S)-3-acetamido-4-(((3-(5-(3-((1'-(4-((1S, 7S)-7-(2-amino-2-oxoethyl)-17-(3,4-bis(4-(ethoxycarbonyl)phenoxy)-2,5-dioxo-2,5-dihydro-1H-pyrrol-1-yl)-1-cyclopropoxy-6,9-dioxo-1-phenyl-3,12,15-trioxa-5,8-diazaheptadecyl)-6-(6-methyl-7-oxo-6,7-dihydro-1H-pyrrolo[2,3-c]pyridin-4-yl)quinazolin-2-yl)-4-methyl-[1,4'-bipiperidin]-4-yl)amino)prop-1-yn-1-yl)-2-methoxyphenyl)-2,6-dioxotetrahydropyrimidin-1(2H)-yl)methyl)amino)-4-oxobutanoic acid of Example 272 in DMA(12 mM, 20.5 μL; 12.0 eq. per antibody molecule) was added and a Microtube Rotator was used for stirring for 90 minutes at room temperature. The reaction mixture was transferred into an Amicon Ultra-15 container (30,000 MWCO, Millipore Corporation), and after dilution with a 25 mM histidine/250 mM sucrose solution (pH 5.5), the reaction mixture was concentrated to about 1/10 volume by centrifugation with a centrifuge (5000 G, 11 min). After repeating the same procedure two more times, the concentrated antibody-drug conjugate solution was filtered and sterilized using a syringe filter (MILLEX-LG, pore size: 0.22 μm, Millipore Corporation) to obtain the title antibody-drug conjugate.

Antibody concentration: 1.60 mg/mL, average number of bonded drug molecules per antibody molecule: 3.7

[Example 319: AsPC-1/Cas9: Growth inhibition assay]

According the same method as in the AsPC-1/Cas9 growth inhibition assay described in Example 313 above, the test substance concentration necessary for 50% inhibition of cell growth (Absolute IC50) in the ADC described in Example 318 was calculated and is shown in Table 12.

[Table 12]

| ADC No. | Antibody | Linker-payload | ADC concentration (mg/mL) | DAR | Cell line | IC50 (nM) |
|---|---|---|---|---|---|---|
| 89 | CEACAM6_#84.7 | Example 272 | 1.60 | 3.7 | AsPC-1 | 0.037 |

[Example 401: Antitumor effect against subcutaneous grafted tumor of the human-derived pancreatic cancer cell line AsPC-1, by ADC of Example 302, ADC of Example 304 and ADC of Example 305]

The antitumor effects of administering the ADC of Example 302, the ADC of Example 304 and the ADC of Example 305 were evaluated using BALB/c nude mice (CAnN.Cg-Foxn1/CrlCrlj, female, Charles River Japan), with 5

mice per group.

The human-derived pancreatic cancer cell line AsPC-1 (ATCC Number CRL-1682) was subcutaneously grafted on the right side of the mice, at 5 × $10^6$ cells/mouse. The long and short diameters of the tumors were measured using an electronic digital caliper (Super Caliper by Mitsutoyo Corp.). The tumor volumes were calculated by the following formula.

Tumor volume ($mm^3$) = Long diameter (mm) × short diameter (mm) × short diameter (mm)/2

On the 7th day after grafting the pancreatic cancer cell line, the mice were divided into groups having the same average tumor volume.

L-Histidine (Merck) and sucrose (Merck) were dissolved in ultrapure water and 1 mol/L hydrochloric acid was added to pH 5.5, after which the solution was adjusted upward to 2000 mL with ultrapure water (25 mM histidine + 250 mM sucrose, pH 5.5). The mixture was passed through a sterile filter unit (Thermo Fisher Scientific) and used as the specimen preparation mixture.

The drug was administered under the following conditions, with the beginning day of administration as day 1. On day 1, the mice in each group were injected through the caudal vein with the ADC of Example 302, the ADC of Example 304 and the ADC of Example 305 (doses of 1 mg/kg, 3 mg/kg and 6 mg/kg, respectively). For the control group, the specimen preparation mixture alone was injected through the caudal vein on day 1.

The tumor volume of each mouse was measured on day 1, 5, 8, 12, 15, 18, 22, 28, 31, 36, 39, 42, 46, 49, 53, 56, 59, 63, 67, 71, 74, 77, 81, 84 and 88. The results (mean) are shown in Fig. 1.

[Example 402: Antitumor effect against subcutaneous grafted tumor of the human-derived pancreatic cancer cell line AsPC-1, by ADC of Example 303]

The antitumor effect of administering the ADC of Example 303 was evaluated using BALB/c nude mice (CAnN.Cg-Foxn1$^{nu}$/CrlCrlj, female, Charles River Japan), with 3 mice per group.

The human-derived pancreatic cancer cell line AsPC-1 (ATCC Number CRL-1682) was subcutaneously grafted on the right side of the mice, at 5 × $10^6$ cells/mouse. The long and short diameters of the tumors were measured using an electronic digital caliper (Super Caliper by Mitsutoyo Corp.). The tumor volumes were calculated by the following formula.

Tumor volume ($mm^3$) = Long diameter (mm) × short diameter (mm) × short diameter (mm)/2

On the 8th day after grafting the pancreatic cancer cell line, the mice were divided into groups having the same average tumor volume.

L-Histidine (Merck) and sucrose (Merck) were dissolved in ultrapure water and 1 mol/L hydrochloric acid was added to pH 5.5, after which the solution was adjusted upward to 2000 mL with ultrapure water (25 mM histidine + 250 mM sucrose, pH 5.5). The mixture was passed through a sterile filter unit (Thermo Fisher Scientific) and used as the specimen preparation mixture.

The drug was administered under the following conditions, with the beginning day of administration as day 1. On day 1, the mice in each group were injected through the caudal vein with the ADC of Example 303 (doses of 1 mg/kg, 3 mg/kg and 6 mg/kg). For the control group, the specimen preparation mixture alone was injected through the caudal vein on day 1.

The tumor volume of each mouse was measured on day 1, 6, 8, 12, 15, 19, 22, 26 and 29. The results (mean) are shown in Fig. 2.

[Example 403: Antitumor effect against subcutaneous grafted tumor of the human uterine cancer cell line HEC-251, by ADC of Example 307]

The antitumor effect of administering the ADC of Example 307 was evaluated using BALB/c nude mice (CAnN.Cg-Foxn1$^{nu}$/CrlCrlj, female, Charles River Japan), with 5 mice per group.

The human-derived uterine cancer cell line HEC-251 (JCRB Number JCRB1141) was subcutaneously grafted on the right side of the mice, at 1 × $10^7$ cells/mouse. The long and short diameters of the tumors were measured using an electronic digital caliper (Super Caliper by Mitsutoyo Corp.). The tumor volumes were calculated by the following formula.

Tumor volume ($mm^3$) = Long diameter (mm) × short diameter (mm) × short diameter (mm)/2

On the sixth day after grafting the uterine cancer cell line, the mice were divided into groups having the same

average tumor volume.

L-Histidine (Merck) and sucrose (Merck) were dissolved in ultrapure water and 1 mol/L hydrochloric acid was added to pH 5.5, after which the solution was adjusted upward to 2000 mL with ultrapure water (25 mM histidine + 250 mM sucrose, pH 5.5). The mixture was passed through a sterile filter unit (Thermo Fisher Scientific) and used as the specimen preparation mixture. The ADC of Example 307 was dissolved in specimen preparation mixture to concentrations of 0.15 mg/mL, 0.3 mg/mL and 0.6 mg/mL.

The drug was administered under the following conditions, with the beginning day of administration as day 1. On day 1, the mice in each group were injected through the caudal vein with the ADC of Example 307 (doses of 1.5 mg/kg, 3 mg/kg or 6 mg/kg, at 10 mL/kg). For the control group, the specimen preparation mixture alone (10 mL/kg) was injected through the caudal vein on day 1.

The tumor volume of each mouse was measured on day 1, 4, 8, 11, 15, 18, 22, 25 and 29. The results (mean) are shown in Fig. 3.

[Example 404: Antitumor effect against subcutaneous grafted tumor of the human-derived lung cancer cell line NCI-H226 by ADC of Example 308]

The antitumor effect of administering the ADC of Example 308 was evaluated using BALB/c nude mice (CAnN.Cg-Foxn1$^{nu}$/CrlCrlj, female, Charles River Japan), with 5 mice per group.

The human-derived lung cancer cell line NCI-H226 (ATCC Number CRL-5826) was subcutaneously grafted on the right side of the mice, at $5 \times 10^6$ cells/mouse. The long and short diameters of the tumors were measured using an electronic digital caliper (Super Caliper by Mitsutoyo Corp.). The tumor volumes were calculated by the following formula.

Tumor volume ($mm^3$) = Long diameter (mm) $\times$ short diameter (mm) $\times$ short diameter (mm)/2

On the 9th day after grafting the lung cancer cell line, the mice were divided into groups having the same average tumor volume.

L-Histidine (Merck) and sucrose (Merck) were dissolved in ultrapure water and 1 mol/L hydrochloric acid was added to pH 5.5, after which the solution was adjusted upward to 2000 mL with ultrapure water (25 mM histidine + 250 mM sucrose, pH 5.5). The mixture was passed through a sterile filter unit (Thermo Fisher Scientific) and used as the specimen preparation mixture. The ADC of Example 308 was dissolved in specimen preparation mixture to concentrations of 0.15 mg/mL, 0.3 mg/mL and 0.6 mg/mL.

The drug was administered under the following conditions, with the beginning day of administration as day 0. On day 0, the mice in each group were injected through the caudal vein with the ADC of Example 308 (doses of 1.5 mg/kg, 3 mg/kg or 6 mg/kg, at 10 mL/kg). For the control group, the specimen preparation mixture alone (10 mL/kg) was injected through the caudal vein on day 0.

The tumor volume of each mouse was measured on day 0, 3, 6, 10, 13, 17, 20, 24 and 27. The results (mean) are shown in Fig. 4.

[Example 405: Antitumor effect against subcutaneous grafted tumor of human-derived stomach cancer cell line NCI-N87, by HER2-DTM-PEG2-K (PEG12)GFG-BLES-2113]

The antitumor effect of administering ADC #78 in Table 10 was evaluated using BALB/c nude mice (CAnN.Cg-Foxn1$^{nu}$/CrlCrlj, female, Charles River Japan), with 5 mice per group. The human-derived stomach cancer cell line NCI-N87 (ATCC Number CRL-5822) was subcutaneously grafted on the right side of the mice, at $1 \times 10^7$ cells/mouse. The long and short diameters of the tumors were measured using an electronic digital caliper (Super Caliper by Mitsutoyo Corp.). The tumor volumes were calculated by the following formula.

Tumor volume ($mm^3$) = Long diameter (mm) $\times$ short diameter (mm) $\times$ short diameter (mm)/2

On the 6th day after grafting the stomach cancer cell line, the mice were divided into groups having the same average tumor volume.

L-Histidine (Merck) and sucrose (Merck) were dissolved in ultrapure water and 1 mol/L hydrochloric acid was added to pH 5.5, after which the solution was adjusted upward to 2000 mL with ultrapure water (25 mM histidine + 250 mM sucrose, pH 5.5). The mixture was passed through a sterile filter unit (Thermo Fisher Scientific) and used as the specimen preparation mixture. ADC #78 of Table 10 was dissolved in specimen preparation mixture to concentrations of 0.15 mg/mL, 0.3 mg/mL and 0.6 mg/mL.

The drug was administered under the following conditions, with the beginning day of administration as day 1. On

day 1, the mice in each group were injected through the caudal vein with ADC #78 of Table 10 (doses of 1.5 mg/kg, 3 mg/kg or 6 mg/kg, at 10 mL/kg). For the control group, the specimen preparation mixture alone (10 mL/kg) was injected through the caudal vein on day 1.

The tumor volume of each mouse was measured on day 1, 4, 8, 11, 15, 18, 22, 25 and 29. The results (mean) are shown in Fig. 5.

[Example 406: Antitumor effect against subcutaneous grafted tumor of human-derived bladder cancer cell line HT-1376, by Nectin4-DTM-PEG2-K (PEG12)GFG-BLES-2113]

The antitumor effect of administering ADC #83 in Table 10 was evaluated using SCID mice (CB17/Icr-Prkdcscid/CrlCrlj, female, Charles River Japan), with 5 mice per group.

The human-derived bladder cancer cell line HT-1376 (ATCC Number CRL-1472) was subcutaneously grafted on the right side of the mice, at $3.5 \times 10^6$ cells/mouse. The long and short diameters of the tumors were measured using an electronic digital caliper (Super Caliper by Mitsutoyo Corp.). The tumor volumes were calculated by the following formula.

Tumor volume ($mm^3$) = Long diameter (mm) $\times$ short diameter (mm) $\times$ short diameter (mm)/2

On the 14th day after grafting the bladder cancer cell line, the mice were divided into groups having the same average tumor volume.

L-Histidine (Merck) and sucrose (Merck) were dissolved in ultrapure water and 1 mol/L hydrochloric acid was added to pH 5.5, after which the solution was adjusted upward to 2000 mL with ultrapure water (25 mM histidine + 250 mM sucrose, pH 5.5). The mixture was passed through a sterile filter unit (Thermo Fisher Scientific) and used as the specimen preparation mixture. ADC #83 of Table 10 was dissolved in specimen preparation mixture to concentrations of 0.15 mg/mL, 0.3 mg/mL and 0.6 mg/mL.

The drug was administered under the following conditions, with the beginning day of administration as day 0. On day 0, the mice in each group were injected through the caudal vein with ADC #83 of Table 10 (doses of 1.5 mg/kg, 3 mg/kg or 6 mg/kg, at 10 mL/kg). For the control group, the specimen preparation mixture alone (10 mL/kg) was injected through the caudal vein on day 0.

The tumor volume of each mouse was measured on day 0, 3, 7, 10, 14, 17, 21, 24 and 28. The results (mean) are shown in Fig. 6.

[Example 407: Antitumor effect against subcutaneous grafted tumor of human-derived pancreatic cancer cell line HPAF-II, by Trop2-DTM-PEG2-K (PEG12)GFG-BLES-2113]

The antitumor effect of administering ADC #84 in Table 10 was evaluated using BALB/c nude mice (CAnN.Cg-Foxn1$^{nu}$/CrlCrlj, female, Charles River Japan), with 5 mice per group. The human-derived pancreatic cancer cell line HPAF-II (ATCC Number CRL-1997) was subcutaneously grafted on the right side of the mice, at $4.4 \times 10^6$ cells/mouse. The long and short diameters of the tumors were measured using an electronic digital caliper (Super Caliper by Mitsutoyo Corp.). The tumor volumes were calculated by the following formula.

Tumor volume ($mm^3$) = Long diameter (mm) $\times$ short diameter (mm) $\times$ short diameter (mm)/2

On the 7th day after grafting the pancreatic cancer cell line, the mice were divided into groups having the same average tumor volume.

L-Histidine (Merck) and sucrose (Merck) were dissolved in ultrapure water and 1 mol/L hydrochloric acid was added to pH 5.5, after which the solution was adjusted upward to 2000 mL with ultrapure water (25 mM histidine + 250 mM sucrose, pH 5.5). The mixture was passed through a sterile filter unit (Thermo Fisher Scientific) and used as the specimen preparation mixture. ADC #84 of Table 10 was dissolved in specimen preparation mixture to concentrations of 0.15 mg/mL, 0.3 mg/mL and 0.6 mg/mL.

The drug was administered under the following conditions, with the beginning day of administration as day 0. On day 0, the mice in each group were injected through the caudal vein with ADC #84 of Table 10 (doses of 1.5 mg/kg, 3 mg/kg or 6 mg/kg, at 10 mL/kg). For the control group, the specimen preparation mixture alone (10 mL/kg) was injected through the caudal vein on day 0.

The tumor volume of each mouse was measured on day 0, 3, 7, 10, 14, 17, 21, 24 and 28. The results (mean) are shown in Fig. 7.

[Example 408: Antitumor effect against subcutaneous grafted tumor of human-derived ovarian cancer cell line COV644, by ADC of Example 311]

The antitumor effect of administering the ADC of Example 311 was evaluated using BALB/c nude mice (CAnN.Cg-Foxn1/CrlCrlj, female, Charles River Japan), with 5 mice per group.
The human-derived ovarian cancer cell line COV644 (DS Pharma Biomedical Co., Ltd., Number EC07071908) was subcutaneously grafted into NOD-SCID mice (NOD.CB17-Prkdcscid/J, female, Charles River Japan), as a vivo-conditioned line (COV644 vivo). The human-derived ovarian cancer cell line COV644 vivo was subcutaneously grafted on the right side of the mice, at $3 \times 10^6$ cells/mouse. The long and short diameters of the tumors were measured using an electronic digital caliper (Super Caliper by Mitsutoyo Corp.). The tumor volumes were calculated by the following formula.

Tumor volume ($mm^3$) = Long diameter (mm) $\times$ short diameter (mm) $\times$ short --> diameter (mm)/2

On the 15th day after grafting the ovarian cancer cell line, the mice were divided into groups having the same average tumor volume.
L-Histidine (Merck) and sucrose (Merck) were dissolved in ultrapure water and 1 mol/L hydrochloric acid was added to pH 5.5, after which the solution was adjusted upward to 2000 mL with ultrapure water (25 mM histidine + 250 mM sucrose, pH 5.5). The mixture was passed through a sterile filter unit (Thermo Fisher Scientific) and used as the specimen preparation mixture. The ADC of Example 311 was dissolved in specimen preparation mixture to concentrations of 0.15 mg/mL, 0.3 mg/mL and 0.6 mg/mL.
The drug was administered under the following conditions, with the beginning day of administration as day 0. On day 0, the mice in each group were injected through the caudal vein with the ADC of Example 311 (doses of 1.5 mg/kg, 3 mg/kg or 6 mg/kg, at 10 mL/kg). For the control group, the specimen preparation mixture alone (10 mL/kg) was injected through the caudal vein on day 0.
The tumor volume of each mouse was measured on day 0, 4, 7, 11, 14, 18, 22, 26 and 29. The results (mean) are shown in Fig. 8.

[Example 409: Antitumor effect against subcutaneous grafted tumor of human-derived pancreatic cancer cell line HPAF-II by ADC of Example 311]

The antitumor effect of administering the ADC of Example 311 was evaluated using BALB/c nude mice (CAnN.Cg-Foxn1/CrlCrlj, female, Charles River Japan), with 5 mice per group.
The human-derived pancreatic cancer cell line HPAF-II (ATCC Number CRL-1997) was subcutaneously grafted on the right side of the mice, at $5 \times 10^6$ cells/mouse. The long and short diameters of the tumors were measured using an electronic digital caliper (Super Caliper by Mitsutoyo Corp.). The tumor volumes were calculated by the following formula.

Tumor volume ($mm^3$) = Long diameter (mm) $\times$ short diameter (mm) $\times$ short diameter (mm)/2

On the 6th day after grafting the pancreatic cancer cell line, the mice were divided into groups having the same average tumor volume.
L-Histidine (Merck) and sucrose (Merck) were dissolved in ultrapure water and 1 mol/L hydrochloric acid was added to pH 5.5, after which the solution was adjusted upward to 2000 mL with ultrapure water (25 mM histidine + 250 mM sucrose, pH 5.5). The mixture was passed through a sterile filter unit (Thermo Fisher Scientific) and used as the specimen preparation mixture. The ADC of Example 311 was dissolved in specimen preparation mixture to concentrations of 0.025 mg/mL, 0.05 mg/mL, 0.15 mg/mL, 0.3 mg/mL and 0.6 mg/mL.
The drug was administered under the following conditions, with the beginning day of administration as day 0. On day 0, the mice in each group were injected through the caudal vein with the ADC of Example 311 (doses of 0.25 mg/kg, 0.5 mg/kg, 1.5 mg/kg, 3 mg/kg or 6 mg/kg, at 10 mL/kg). For the control group, the specimen preparation mixture alone (10 mL/kg) was injected through the caudal vein on day 0.
The tumor volume of each mouse was measured on day 0, 4, 7, 11, 14, 18, 21, 25, 29 and 33. The results (mean) are shown in Fig. 9.

[Example 410: Antitumor effect against subcutaneous grafted tumor of human-derived pancreatic cancer cell line HPAF-II by ADC of Example 318]

The antitumor effect of administering the ADC of Example 318 was evaluated using BALB/c nude mice (CAnN.Cg-Foxn1/CrlCrlj, female, Charles River Japan), with 5 mice per group.

The human-derived pancreatic cancer cell line HPAF-II (ATCC Number CRL-1997) was subcutaneously grafted on the right side of the mice, at 5 × 106 cells/mouse. The long and short diameters of the tumors were measured using an electronic digital caliper (DigimaticTM caliper by Mitsutoyo Corp.).
The tumor volumes were calculated by the following formula.

Tumor volume (mm3) = Long diameter (mm) × short diameter (mm) × short diameter (mm)/2

On the 6th day after grafting the pancreatic cancer cell line, the mice were divided into groups having the same average tumor volume.
L-Histidine (Merck) and sucrose (Merck) were dissolved in ultrapure water and 1 mol/L hydrochloric acid was added to pH 5.5, after which the solution was adjusted upward to 2000 mL with ultrapure water (25 mM histidine + 250 mM sucrose, pH 5.5). The mixture was passed through a sterile filter unit (Thermo Fisher Scientific) and used as the specimen preparation mixture. The ADC of Example 318 was dissolved in specimen preparation mixtures to concentrations of 0.025 mg/mL, 0.05 mg/mL and 0.1 mg/mL.
The drug was administered under the following conditions, with the beginning day of administration as day 0. On day 0, the mice in each group were injected through the caudal vein with the ADC of Example 318 (doses of 0.25 mg/kg, 0.5 mg/kg or 1 mg/kg, at 10 mL/kg). For the control group, the specimen preparation mixture alone (10 mL/kg) was injected through the caudal vein on day 0.
The tumor volume of each mouse was measured on day 0, 2, 6, 9, 13, 16, 20, 23, 27 and 30. The results (mean values) are shown in Fig. 10.

[Example 501: Preparation of Pan02/Pgp-KO/hCEACAM6 cell line]

The mouse pancreatic cancer cell line Pan02 (National Institutes of Health) was transfected with recombinant Cas9 protein (Alt-R S.p. HiFi Cas9 V3, Integrated DNA Technologies), and mouse Abcb1a and single guided RNA (Alt-R CRISPR sgRNA, Integrated DNA Technologies) for Abcb1b, using a NEPA21 Super Electroporator (Nepa Gene). The obtained cells were further transfected with human CEACAM6 using pLV[Exp]-Puro-EFS lentiviral vector (VectorBuilder) and MISSION Lentiviral Packaging Mix (Sigma-Aldrich). The transfected cells were stained with Rhodamine-123 (Sigma-Aldrich) and human CEACAM6 antibody (#84.7), and the strongly human CEACAM6-positive and strongly Rhodamine-123-positive cells were fractionated using a FACSARIAII cell sorter (BD). The established cells were designated as cell line Pan02/Pgp-KO/hCEACAM6, and were maintenance cultured in a 5% $CO_2$ incubator (37°C) using RPMI (FujiFilm-Wako Pure Chemical Industries) containing 10% fetal bovine serum (FBS: SIGMA 173012) and penicillin/streptomycin (FujiFilm-Wako Pure Chemical Industries) (hereunder referred to simply as "culture medium").

[Example 502: Antitumor effect against subcutaneous grafted tumor of mouse pancreatic cancer cell line Pan02/Pgp-KO/hCEACAM6, by combination of ADC of Example 304 and anti-mouse PD-1 antibody]

The antitumor effect of administering the ADC of Example 304 in combination with anti-mouse PD-1 antibody (trade name: In VivoMAb anti-mouse PD-1 (CD279), Bio X Cell) was evaluated using C57BL/6 J mice (C57BL/6 J, female, Charles River Japan), with 5 mice per group.
The mouse pancreatic cancer cell line Pan02/Pgp-KO/hCEACAM6 was subcutaneously grafted on the right side of the mice, at 5 × $10^6$ cells/mouse. The long and short diameters of the tumors were measured using an electronic digital caliper (Super Caliper by Mitsutoyo Corp.). The tumor volumes were calculated by the following formula.

Tumor volume ($mm^3$) = Long diameter (mm) × short diameter (mm) × short diameter (mm)/2

On the 5th day after grafting the pancreatic cancer cell line, the mice were divided into groups having the same average tumor volume.
L-Histidine (Merck) and sucrose (Merck) were dissolved in ultrapure water and 1 mol/L hydrochloric acid was added to pH 5.5, after which the solution was adjusted upward to 2000 mL with ultrapure water (25 mM histidine + 250 mM sucrose, pH 5.5). The mixture was passed through a sterile filter unit (Thermo Fisher Scientific) and used as the specimen preparation mixture. The ADC of Example 304 was dissolved in specimen preparation mixture to concentrations of 0.3 mg/mL and 0.6 mg/mL. The anti-mouse PD-1 antibody was dissolved in D-PBS (FujiFilm-Wako Pure Chemical Industries) to a concentration of 1 mg/mL.
The drug was administered under the following conditions, with the beginning day of administration as day 1. On day 1, the mice in each group were injected through the caudal vein with the ADC of Example 304 (doses of 3 mg/kg and 6 mg/kg, at 10 mL/kg). The anti-mouse PD-1 antibody-administered group was intraperitoneally injected with anti-mouse

PD-1 antibody (0.2 mg/mouse, 10 mL/kg) on days 1 and 8. For the control group, the specimen preparation mixture alone (10 mL/kg) was injected through the caudal vein on day 1.

The tumor volume of each mice was measured on day 1, 4, 8, 11, 15, 18, 22, 25, 29, 32, 39, 43, 46, 50, 53, 57, 60, 64, 67, 71, 74, 78, 81, 85, 88 and 92. The relative tumor volume of individuals reaching tumor volume of 0 during the measuring period was defined as 0.01 for graph visualization, and the results (mean) are shown in Fig. 11.

## Claims

1. An antibody-drug conjugate represented by formula (I):

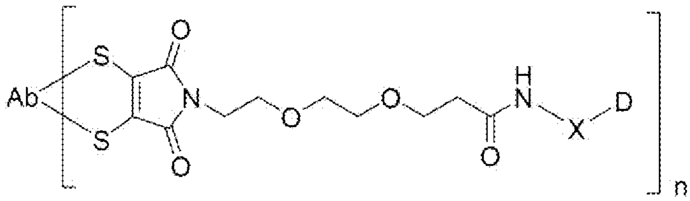

(I)

(where

Ab is an antibody,
X is a group represented by formula (X-1), formula (X-2) or formula (X-3):

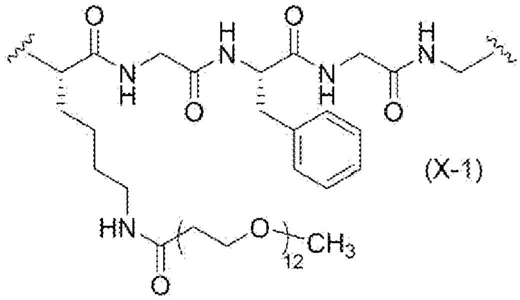

(X-1)

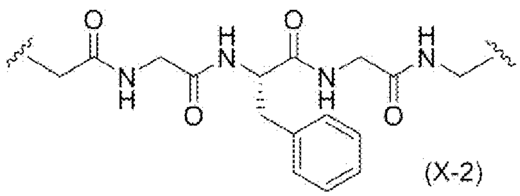

(X-2)

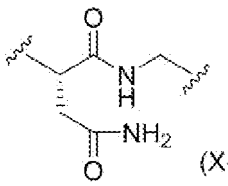

(X-3)

(where

at the left represents the binding site with NH and
at the right represents the binding site with D),
D is a group represented by formula (D-1) or formula (D-2):

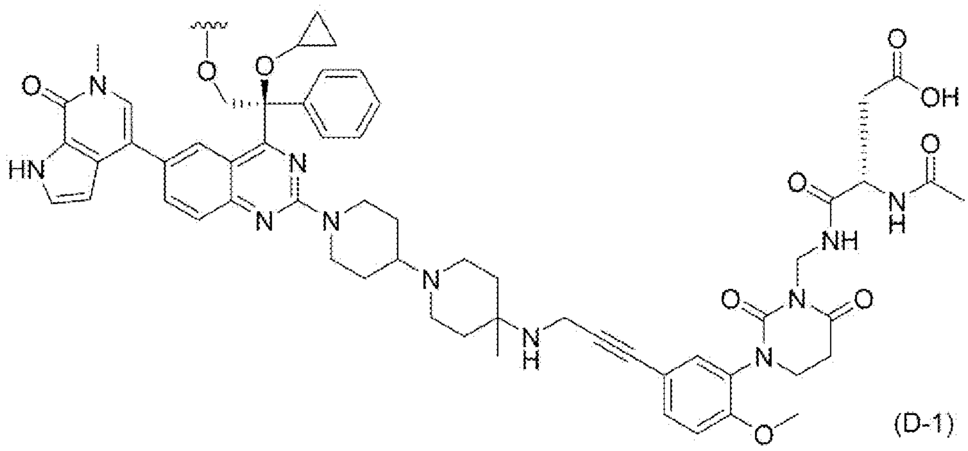

(D-1)

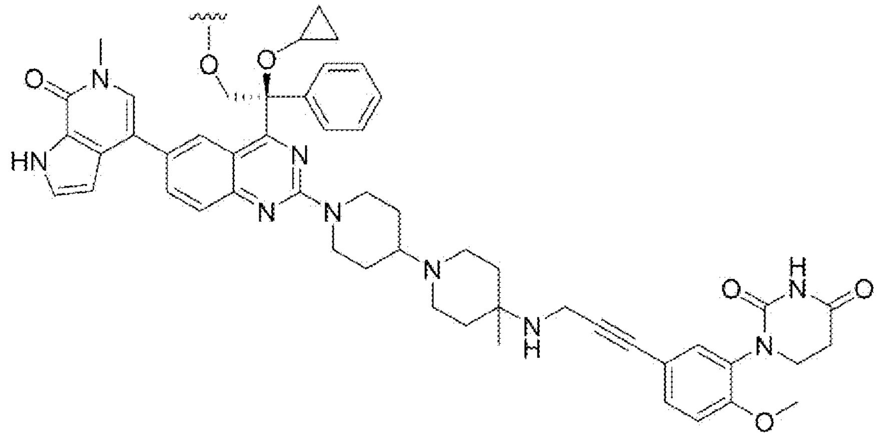

(D-2)

(where

represents the binding site with X), and
n is in the range of about 1 to about 8).

2. The antibody-drug conjugate according to claim 1, wherein the antibody is anti-CEACAM6 antibody, anti-folate receptor $\alpha$ antibody, anti-mesothelin antibody, anti-HER2 antibody, anti-FLT3 antibody, anti-DLL3 antibody, anti-CLDN6 antibody, anti-EGFR antibody, anti-Nectin4 antibody, anti-CA9 antibody or anti-TROP2 antibody.

3. A compound represented by formula (D'):

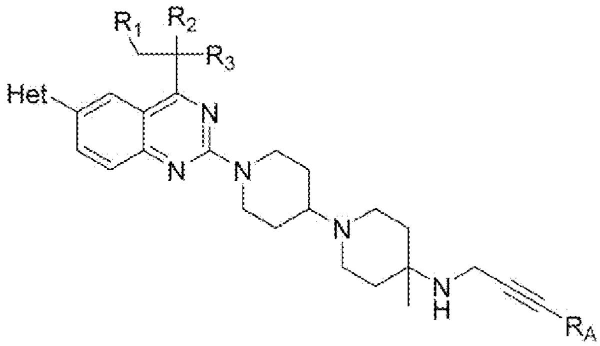

(D')

(where
Het is a group selected from the group consisting of the following formula:

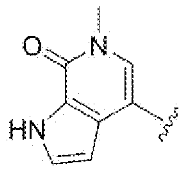 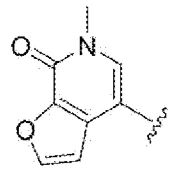 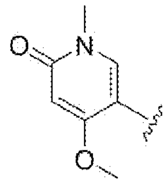 and 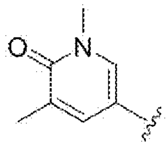

(where

represents a binding site),
$R_1$ is a hydroxyl, amino or optionally substituted $C_{1-6}$ alkylcarbamoyloxy group,
$R_2$ is a $C_{1-6}$ alkyl, $C_{3-6}$ cycloalkyl, $C_{1-6}$ alkoxy or $C_{3-6}$ cycloalkoxy group,
$R_3$ is a $C_{1-6}$ alkyl or $C_{6-10}$ aryl group, and
$R_A$ is group represented by formula (a) or formula (b):

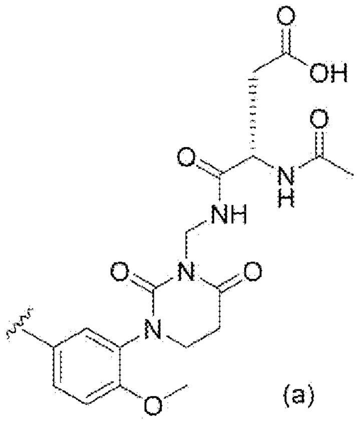
(a)

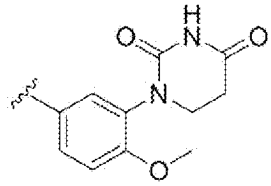
(b)

(where

represents a binding site),
or a pharmacologically acceptable salt thereof.

**4.** A compound represented by formula (D'-1) or formula (D'-2):

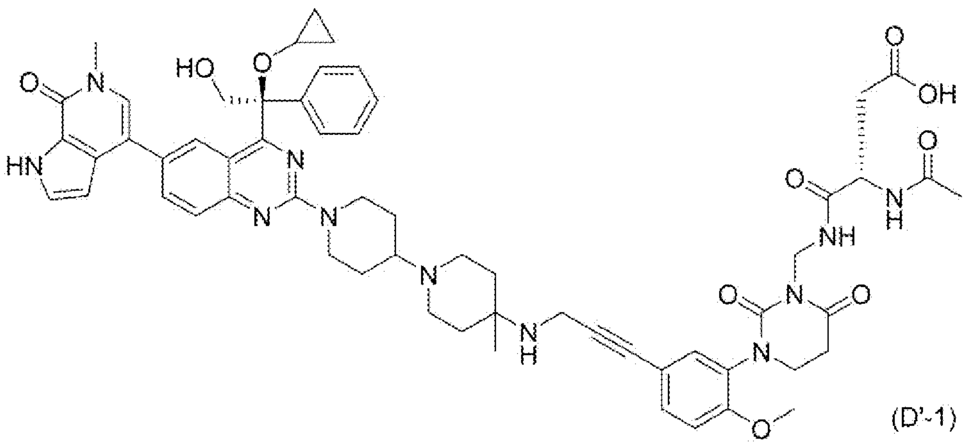
(D'-1)

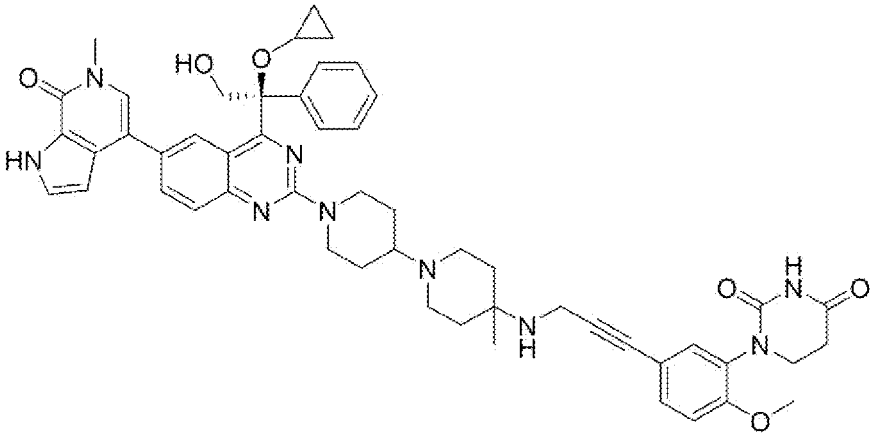
(D'-2)

or a pharmacologically acceptable salt thereof.

**5.** A compound represented by formula (M):

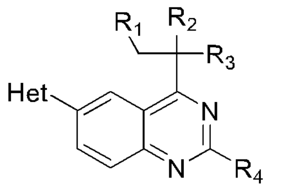
(M)

(where
Het is a group selected from the group consisting of the following formula:

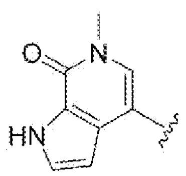
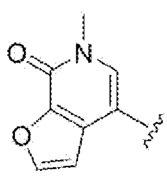
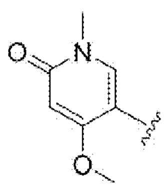
and
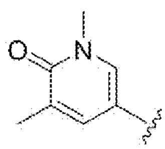

(where

represents a binding site),
$R_1$ is a hydroxyl, amino or optionally substituted $C_{1-6}$ alkylcarbamoyloxy group,
$R_2$ is a $C_{1-6}$ alkyl, $C_{3-6}$ cycloalkyl, $C_{1-6}$ alkoxy or $C_{3-6}$ cycloalkoxy group,
$R_3$ is a $C_{1-6}$ alkyl or $C_{6-10}$ aryl group, and
$R_4$ is an amino group or a group represented by the formula:

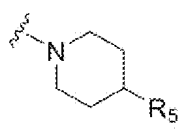

(where

$R_5$ is an ethynyl, piperidyl, piperazinyl, piperidinyloxy or piperazinylmethyl group, the piperidyl, piperazinyl, piperidyloxy, and piperazinylmethyl group being optionally substituted with one or more groups selected from the group consisting of methyl, ethynyl, hydroxyl, amino and propynylamino groups),
or a pharmacologically acceptable salt thereof.

Fig.1

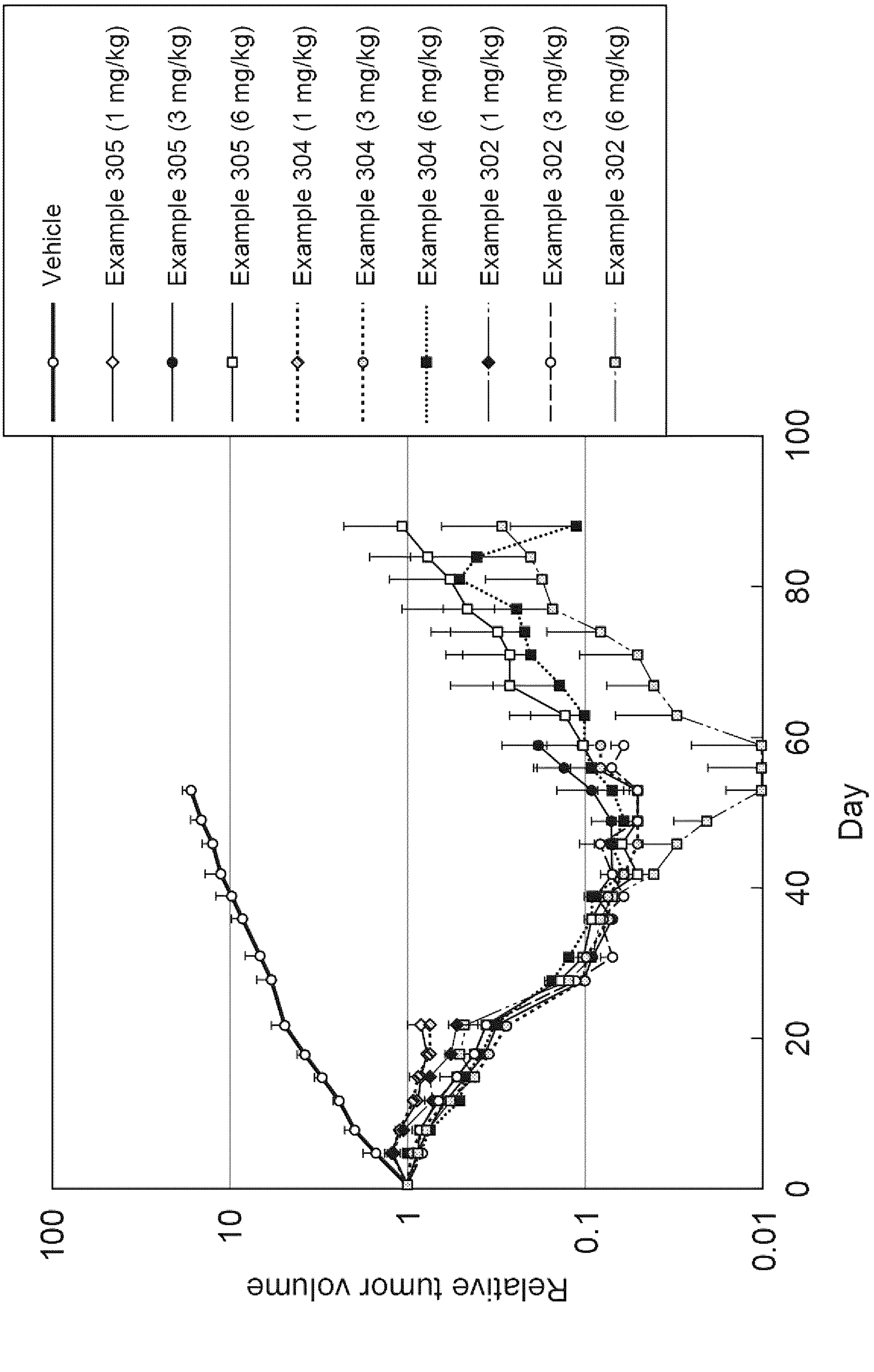
Vehicle
Example 305 (1 mg/kg)
Example 305 (3 mg/kg)
Example 305 (6 mg/kg)
Example 304 (1 mg/kg)
Example 304 (3 mg/kg)
Example 304 (6 mg/kg)
Example 302 (1 mg/kg)
Example 302 (3 mg/kg)
Example 302 (6 mg/kg)
Relative tumor volume
100
10
1
0.1
0.01
Day
0
20
40
60
80
100

Fig.2

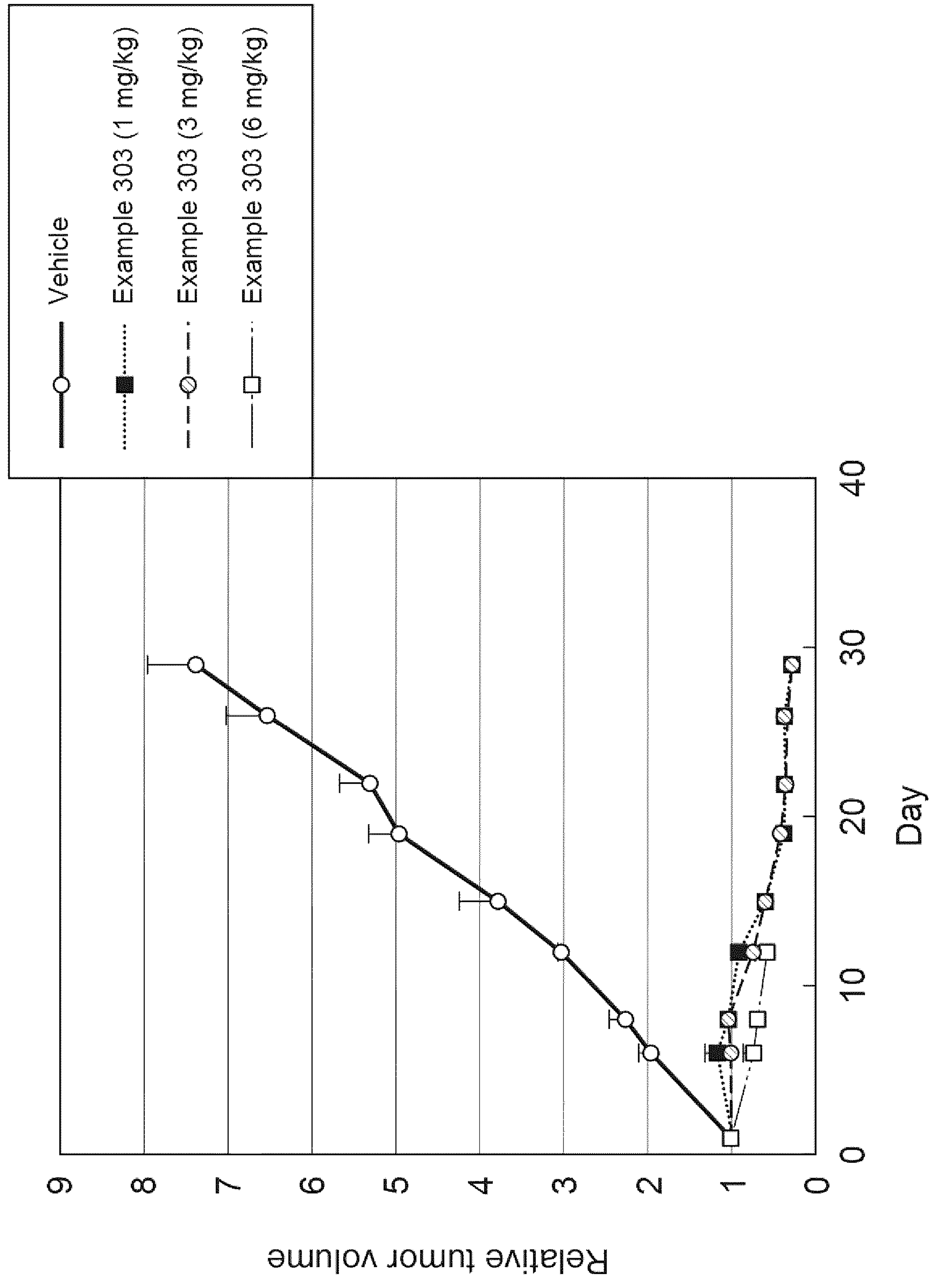
Vehicle
Example 303 (1 mg/kg)
Example 303 (3 mg/kg)
Example 303 (6 mg/kg)
Relative tumor volume
0
1
2
3
4
5
6
7
8
9
Day
0
10
20
30
40

Fig.3

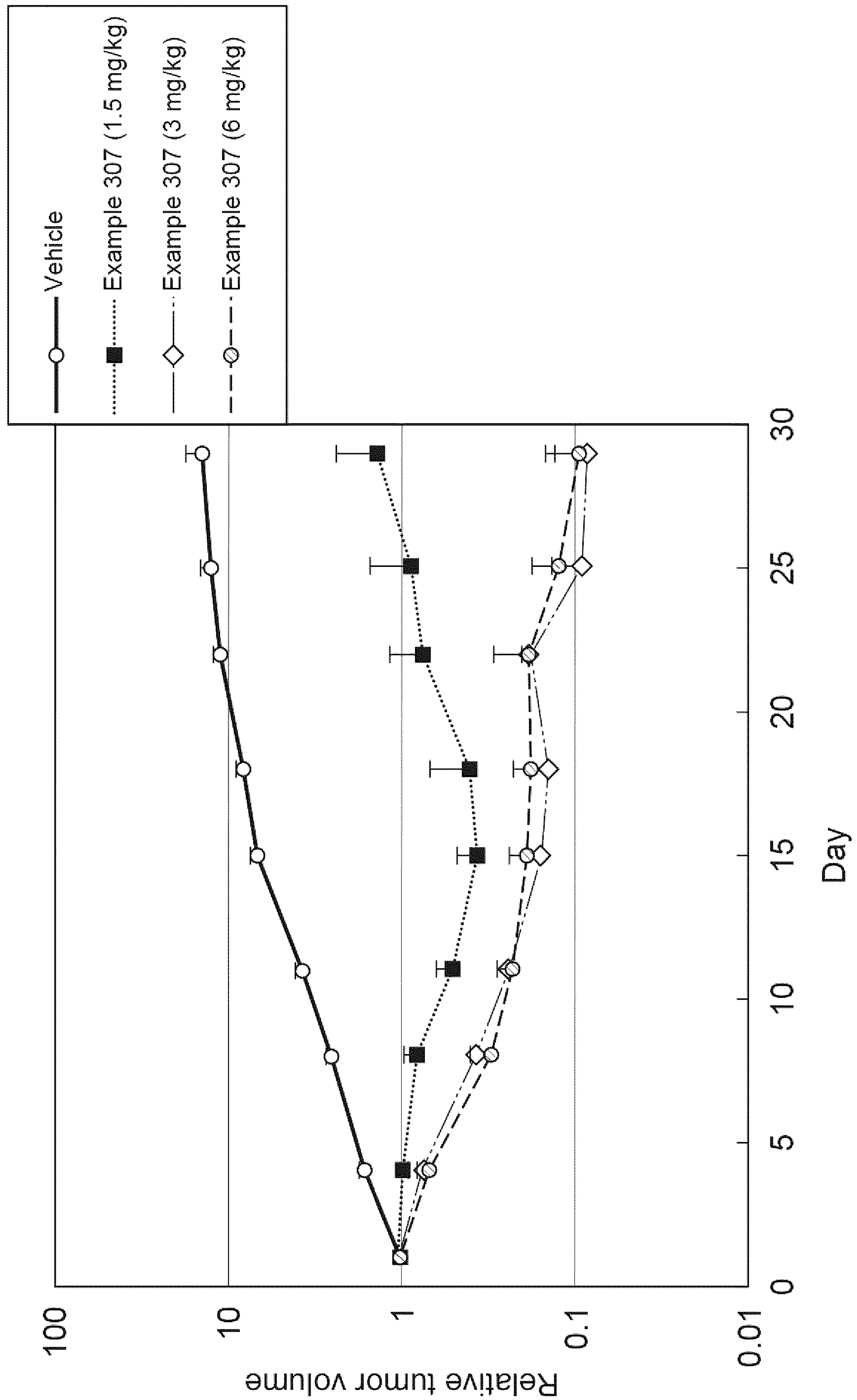
Vehicle
Example 307 (1.5 mg/kg)
Example 307 (3 mg/kg)
Example 307 (6 mg/kg)
Relative tumor volume
100
10
1
0.1
0.01
Day
0
5
10
15
20
25
30

Fig.4

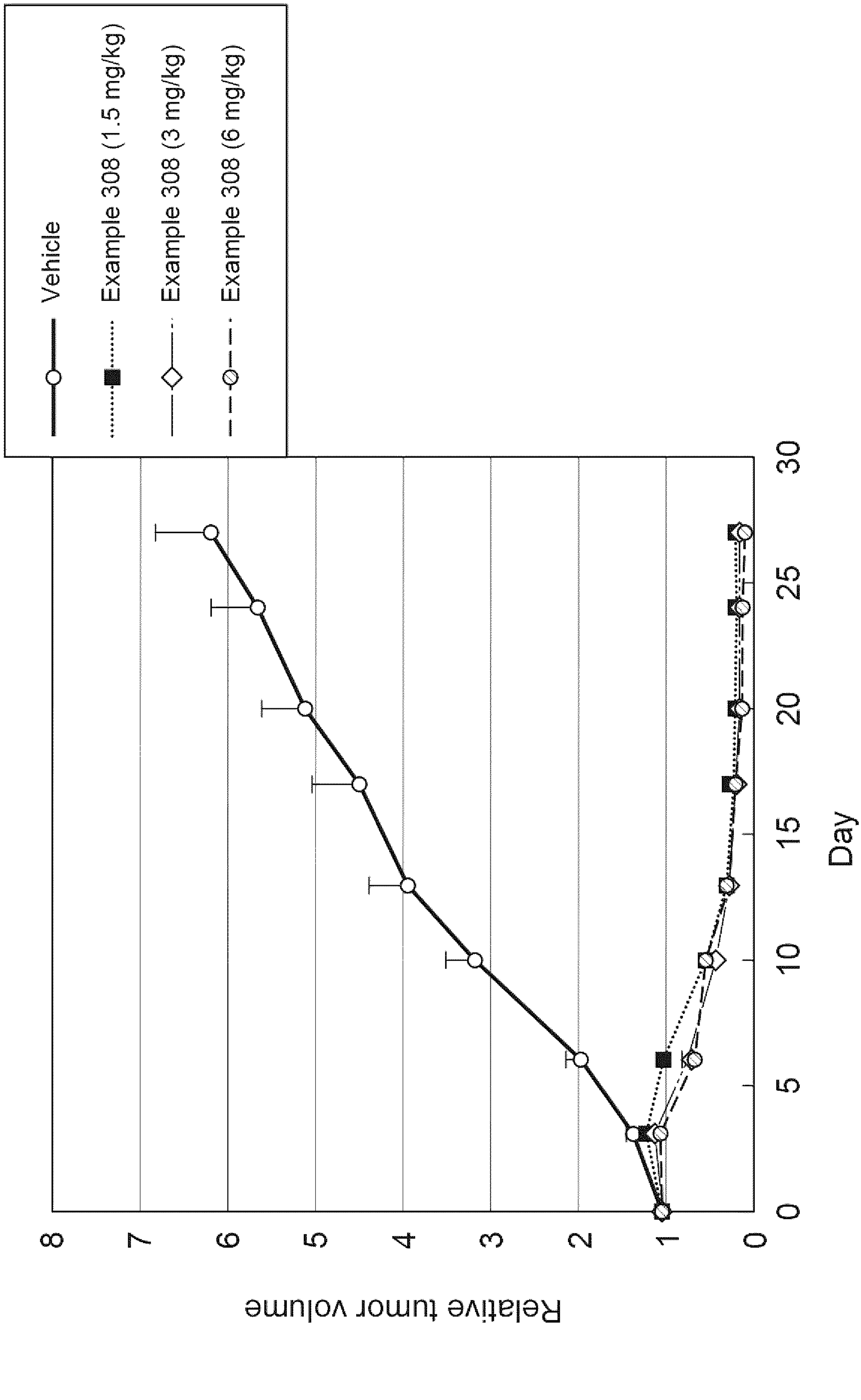
Vehicle
Example 308 (1.5 mg/kg)
Example 308 (3 mg/kg)
Example 308 (6 mg/kg)
Relative tumor volume
0
1
2
3
4
5
6
7
8
Day
0
5
10
15
20
25
30

*Fig.5*

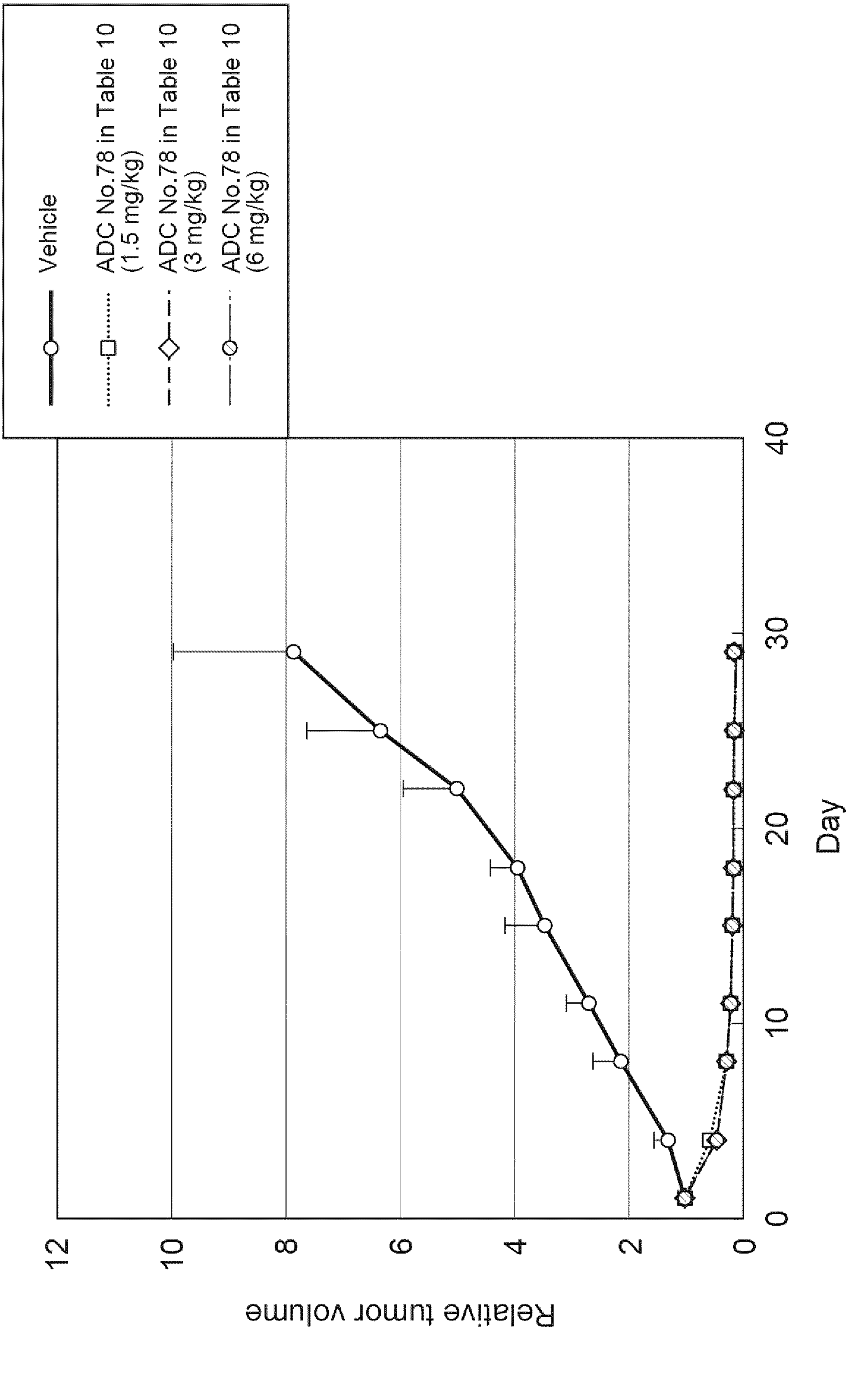
Vehicle
ADC No.78 in Table 10 (1.5 mg/kg)
ADC No.78 in Table 10 (3 mg/kg)
ADC No.78 in Table 10 (6 mg/kg)
Relative tumor volume
0
2
4
6
8
10
12
Day
0
10
20
30
40

Fig.6

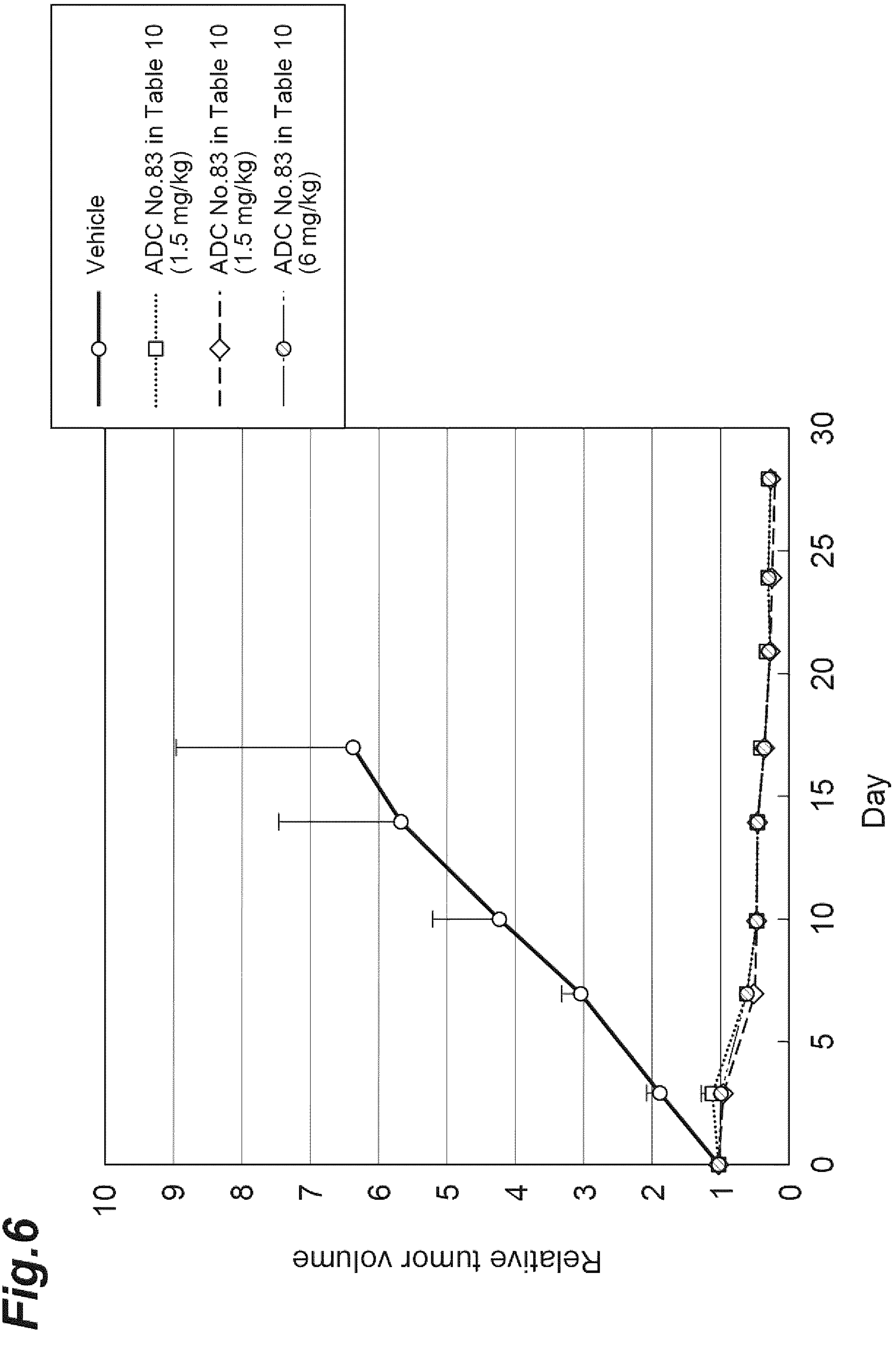
Vehicle
ADC No.83 in Table 10 (1.5 mg/kg)
ADC No.83 in Table 10 (1.5 mg/kg)
ADC No.83 in Table 10 (6 mg/kg)
Relative tumor volume
0
1
2
3
4
5
6
7
8
9
10
Day
0
5
10
15
20
25
30

*Fig.7*

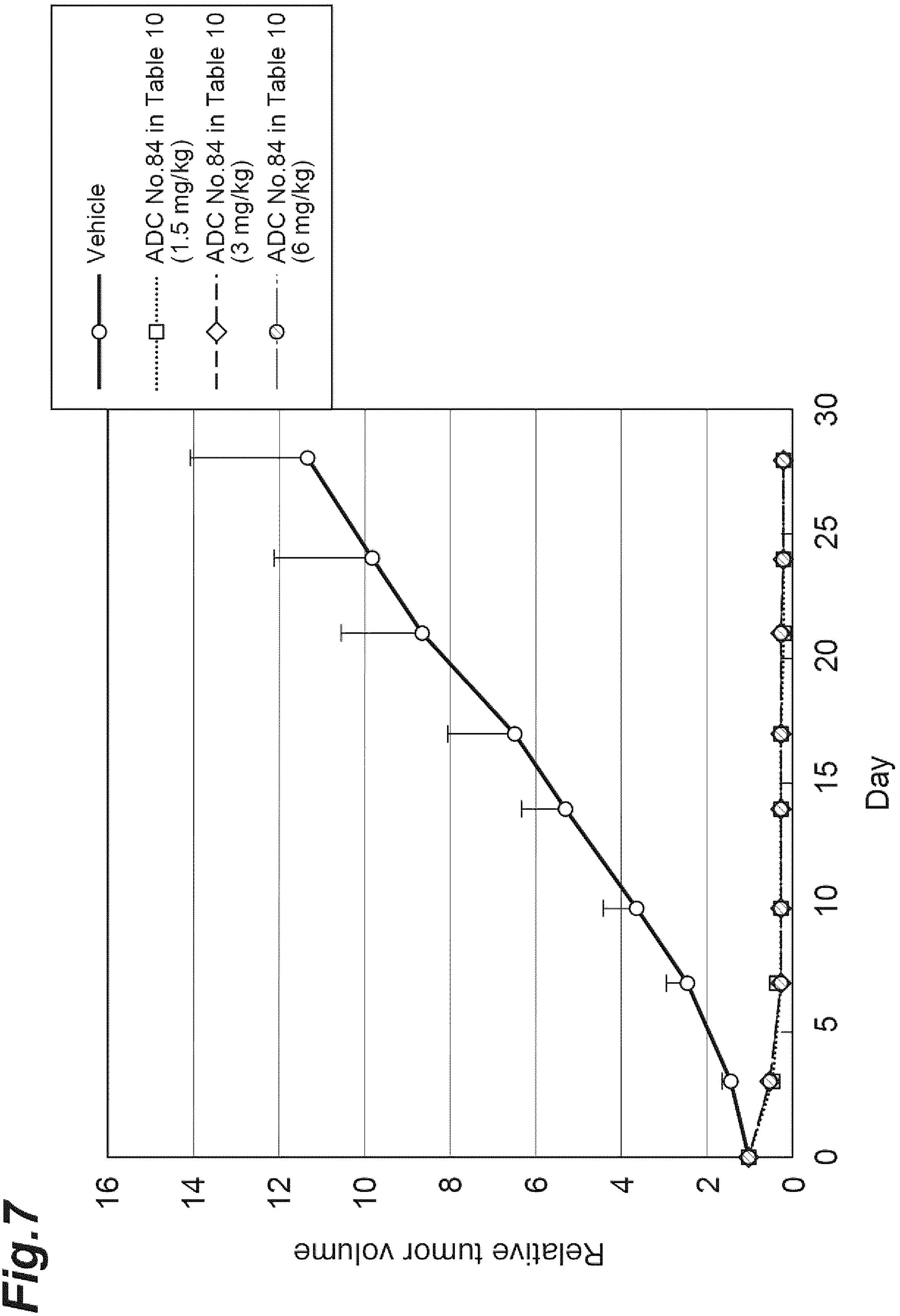
Vehicle
ADC No.84 in Table 10 (1.5 mg/kg)
ADC No.84 in Table 10 (3 mg/kg)
ADC No.84 in Table 10 (6 mg/kg)
Relative tumor volume
0
2
4
6
8
10
12
14
16
Day
0
5
10
15
20
25
30

Fig.8

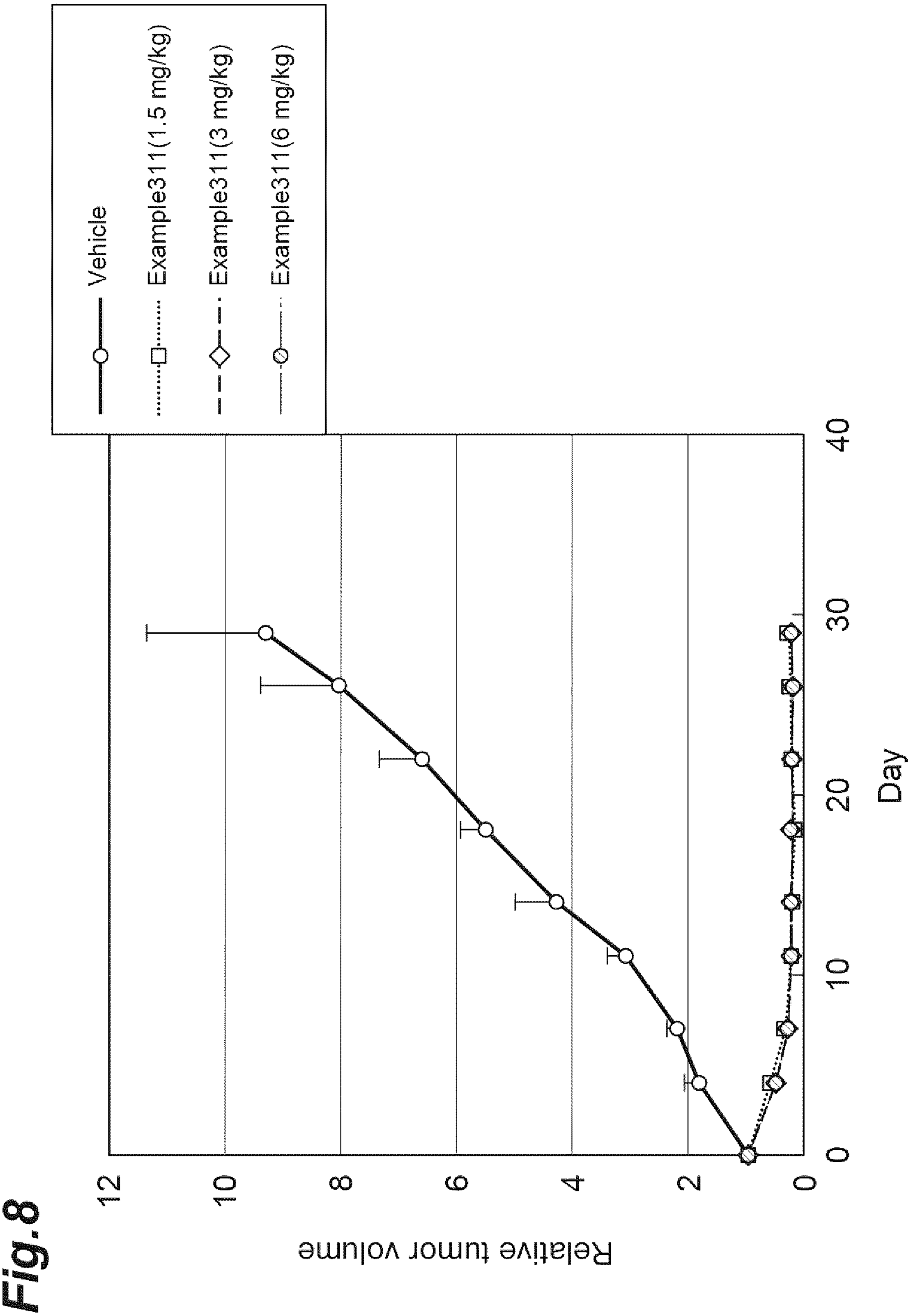
Vehicle
Example311(1.5 mg/kg)
Example311(3 mg/kg)
Example311(6 mg/kg)
Relative tumor volume
0
2
4
6
8
10
12
Day
0
10
20
30
40

Fig.9

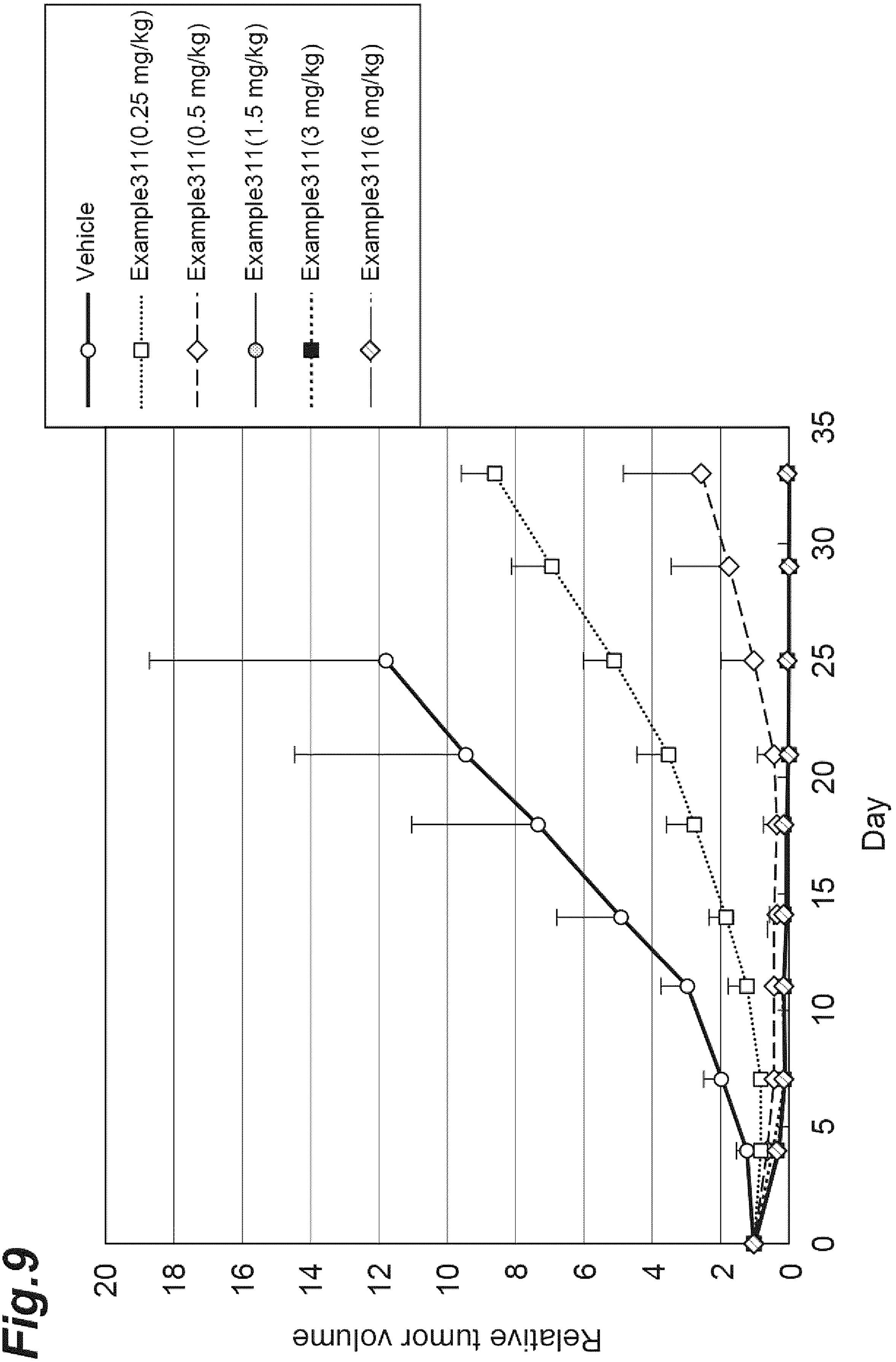
Vehicle
Example311(0.25 mg/kg)
Example311(0.5 mg/kg)
Example311(1.5 mg/kg)
Example311(3 mg/kg)
Example311(6 mg/kg)
Relative tumor volume
Day
0
2
4
6
8
10
12
14
16
18
20
5
15
25
30
35

*Fig.10*

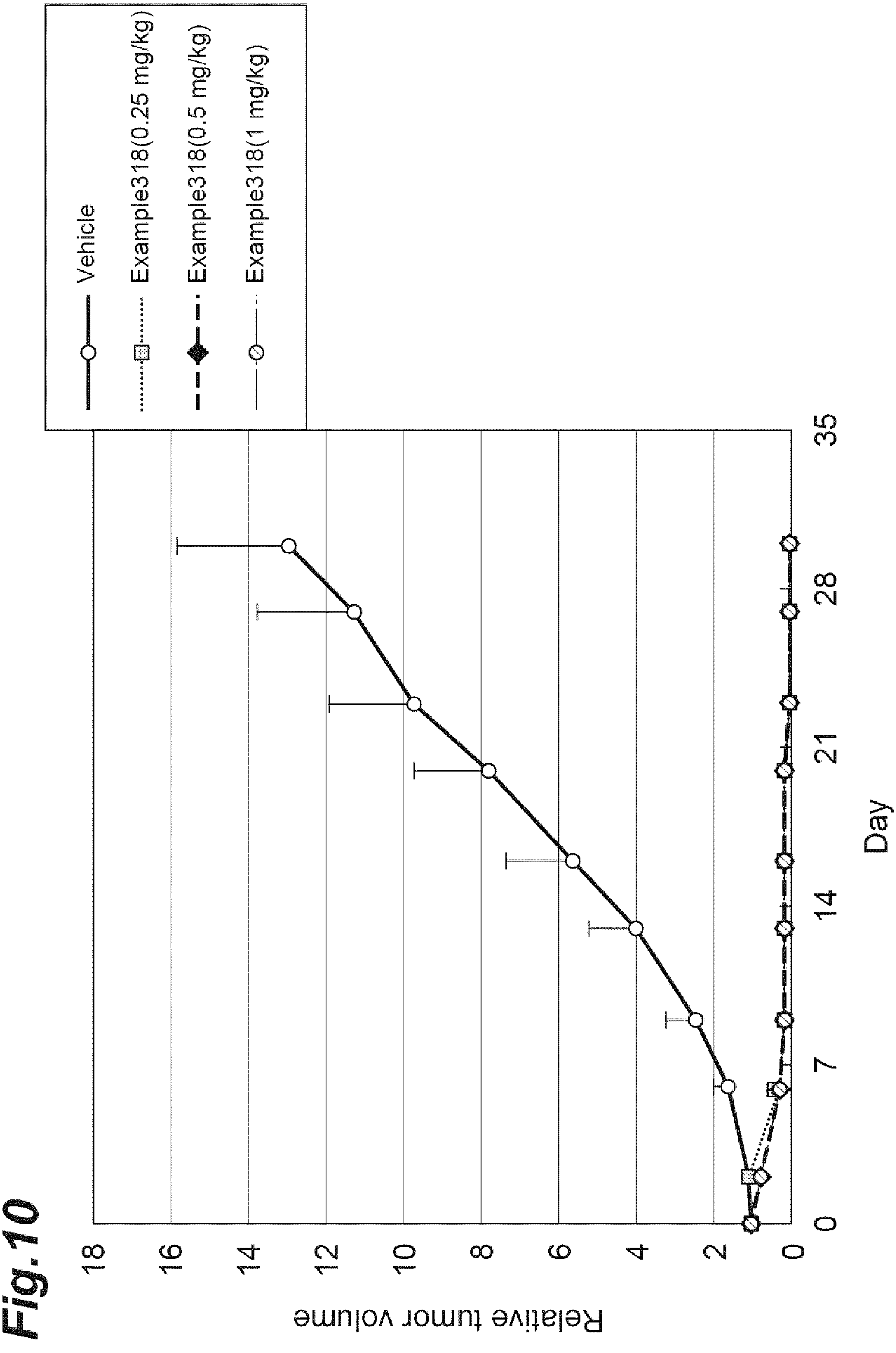
Vehicle
Example318(0.25 mg/kg)
Example318(0.5 mg/kg)
Example318(1 mg/kg)
Relative tumor volume
18
16
14
12
10
8
6
4
2
0
0
7
14
21
28
35
Day

*Fig.11*

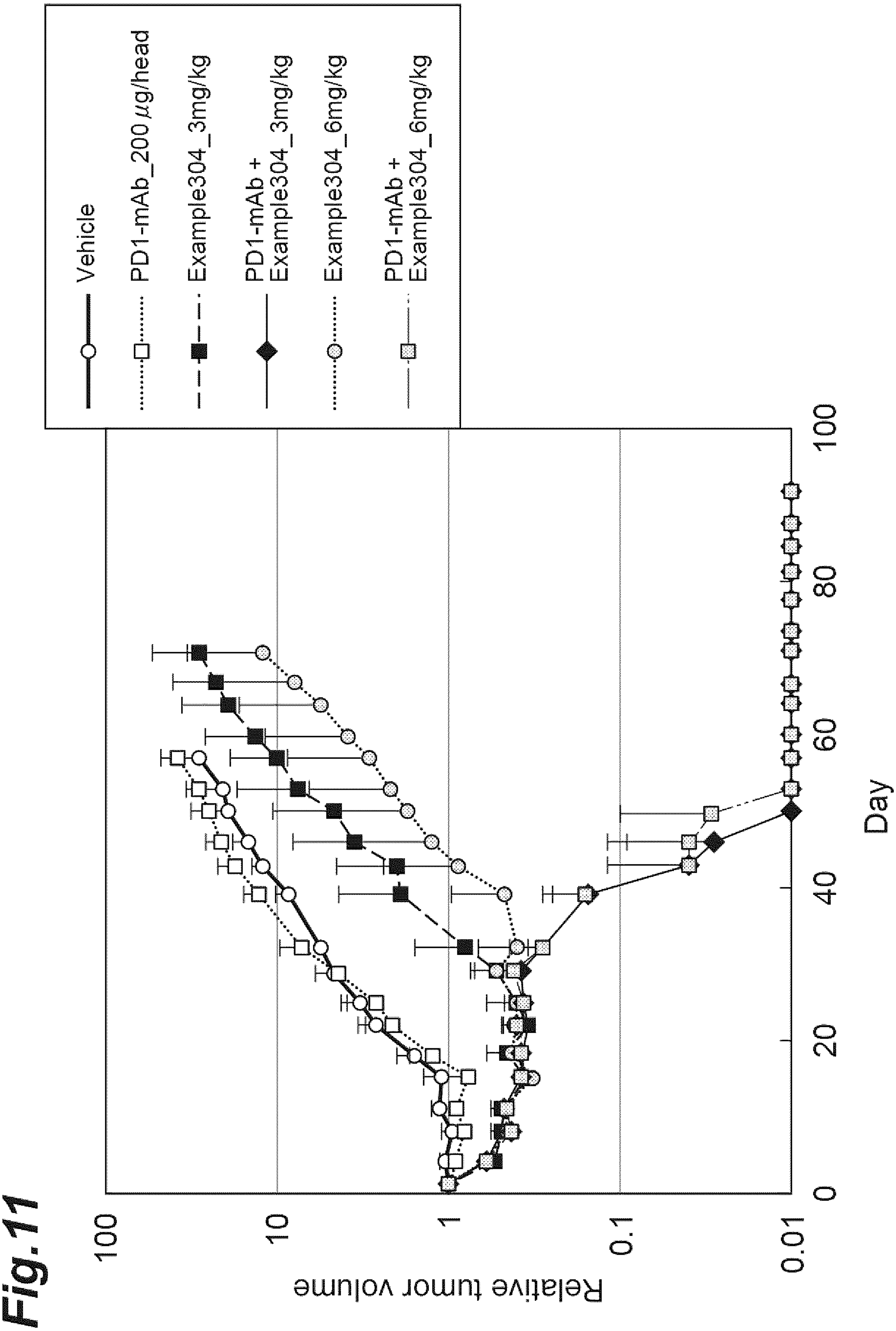
Vehicle
PD1-mAb_200 μg/head
Example304_3mg/kg
PD1-mAb + Example304_3mg/kg
Example304_6mg/kg
PD1-mAb + Example304_6mg/kg
Relative tumor volume
100
10
1
0.1
0.01
0
20
40
60
80
100
Day

**INTERNATIONAL SEARCH REPORT**

International application No. **PCT/JP2023/030603**

**A. CLASSIFICATION OF SUBJECT MATTER**

***A61K 47/68***(2017.01)i; ***A61K 31/498***(2006.01)i; ***A61K 47/54***(2017.01)i; ***C07D 401/04***(2006.01)i; ***C07D 401/14***(2006.01)i; ***C07D 405/14***(2006.01)i; ***C07D 471/04***(2006.01)i; ***C07D 491/048***(2006.01)i

FI: A61K47/68 ZNA; A61K47/54; A61K31/498; C07D401/04 CSP; C07D401/14; C07D405/14; C07D471/04 104Z; C07D491/048

According to International Patent Classification (IPC) or to both national classification and IPC

**B. FIELDS SEARCHED**

Minimum documentation searched (classification system followed by classification symbols)

A61K47/68; A61K31/498; A61K47/54; C07D401/04; C07D401/14; C07D405/14; C07D471/04; C07D491/048

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | YANG, Shyh-Ming et al. Discovery and lead identification of quinazoline-based BRD4 inhibitors. Bioorganic & Medicinal Chemistry Letters. 2018, vol. 28, pp. 3483-3488<br>table 2, fig. 2, p. 3438, left column, line 11 to right column, line 6 | 1-5 |
| A | JP 2019-501140 A (CONVERGENE LLC) 17 January 2019 (2019-01-17)<br>claims, examples, biological assay, compounds 106-111, 121-129, etc. | 1-5 |
| A | JP 2022-534472 A (CONVERGENE LLC) 01 August 2022 (2022-08-01)<br>claims, examples, etc. | 1-5 |
| A | LIN, Songwen et al. Identification of novel 7-amino-5-methyl-1,6-naphthyridin-2(1H) -one derivatives as potent PI3K/mTOR dual inhibitors. Bioorganic & Medicinal Chemistry Letters. 2014, vol. 24, pp. 790-793<br>abstract, compound 14, p. 793, left column, line 5 from the bottom to right column, line 5, etc. | 1-5 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 November 2023** | **21 November 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
PCT/JP2023/030603

C. DOCUMENTS CONSIDERED TO BE RELEVANT

| Category* | Citation of document, with indication, where appropriate, of the relevant passages | Relevant to claim No. |
|---|---|---|
| A | JP 2020-503321 A (INSTITUTE OF MATERIA MEDICA, CHINESE ACADEMY OF MEDICAL SCIENCES) 30 January 2020 (2020-01-30)<br>examples 1-3, claims, etc. | 1-5 |
| A | WO 2021/053495 A1 (NOVARTIS AG) 25 March 2021 (2021-03-25)<br>claims, examples | 1-5 |
| A | WO 2021/077010 A1 (ARVINAS OPERATIONS, INC.) 22 April 2021 (2021-04-22)<br>claims, examples | 1-5 |

Form PCT/ISA/210 (second sheet) (January 2015)

**INTERNATIONAL SEARCH REPORT**

International application No.
**PCT/JP2023/030603**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

   "Annex C/ST.25 text file format" should be read as 'ST.26 format"

Form PCT/ISA/210 (continuation of first sheet) (January 2015)

**INTERNATIONAL SEARCH REPORT**
**Information on patent family members**

International application No.
**PCT/JP2023/030603**

| Patent document cited in search report | Publication date (day/month/year) | Patent family member(s) | Publication date (day/month/year) |
|---|---|---|---|
| JP 2019-501140 A | 17 January 2019 | US 2018/0305344 A1<br>claims, examples, biological assay, compounds 106-111, 121-129<br>EP 3380469 A1<br>CN 109641886 A | |
| JP 2022-534472 A | 01 August 2022 | US 2022/0259200 A1<br>claims, examples<br>EP 3958867 A1<br>CN 114786675 A | |
| JP 2020-503321 A | 30 January 2020 | US 2021/0128559 A1<br>experimental examples 1-3, claims<br>EP 3560921 A1<br>CN 108239075 A | |
| WO 2021/053495 A1 | 25 March 2021 | EP 4031247 A1<br>CN 114521196 A | |
| WO 2021/077010 A1 | 22 April 2021 | EP 4045496 A1<br>KR 10-2022-0102156 A<br>CN 115397821 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020086858 A
- US 4805848 A
- WO 20210900062 A
- US 7488802 B
- US 7521051 B
- US 8008449 B
- US 8354509 B
- US 8168757 B
- WO 2004004771 A
- WO 2004072286 A
- WO 2004056875 A
- US 20110271358


### Non-patent literature cited in the description


- *World Cancer Report*, 2014
- **THOMAS et al.** Antibody Conjugation of a Chimeric BET Degrader Enables in vivo Activity. *ChemMedChem*, 2020, vol. 15, 17-25
- **KABAT et al.** Sequences of Proteins of Immunological Interest. National Institutes of Health, 1991
- **AL-LAZIKANI et al.** *J. Molec. Biol.*, 1997, vol. 273, 927-948
- **KONTERMANN** ; **DUBEL**. Antibody Engineering, Springer Lab Manual. 2001
- **TSURUSHITA et al.** *Methods*, 2005, vol. 36, 69-83
- **JONES et al.** *Nature*, 1986, vol. 321, 522-525
- **RIECHMANN et al.** *Nature*, 1988, vol. 332, 323-327
- **VERHOEYEN et al.** *Science*, 1988, vol. 239, 1534-1536
- **KONTERMANN**. *mAbs*, 2012, vol. 4, 182-97
- **AKAGI T.** *PNAS*, 2003, vol. 100, 13567-72